# EUROPÄISCHE PATENTSCHRIFT

(11) **EP 3 019 493 B1**
(45) Veröffentlichungstag und Bekanntmachung des Hinweises auf die Patenterteilung: **14.06.2017**
(21) Anmeldenummer: 14737225.4
(22) Anmeldetag: 07.07.2014
(51) Int. Cl.: C07D 405/04, C07D 241/44, C07D 471/04, A61K 31/4985, A61P 35/00, C07D 401/04, C07D 519/00, C07D 405/14, A61K 31/498, A61K 31/5377

(54) **MODIFIZIERTE BET-PROTEININHIBITORISCHE DIHYDROCHINOXALINONE UND DIHYDROPYRIDOPYRAZINONE**
MODIFIED BET-PROTEIN INHIBITING DIHYDROCHINOXALINONES AND DIHYDROPYRIDOPYRAZINONES
DIHYDROCHINOXALINONE ET DIHYDROPYRIDOPYRAZINONE MODIFIÉES INHIBITRICES DE LA PROTÉINE BET

(30) Priorität: 09.07.2013 EP 13175767
(43) Veröffentlichungstag der Anmeldung: 18.05.2016
(73) Patentinhaber: Bayer Pharma Aktiengesellschaft, 13353 Berlin (DE)
(72) Erfinder: SCHMEES, Norbert, 13469 Berlin (DE); HAENDLER, Bernard, 13465 Berlin (DE); STÖCKIGT, Detlef, 14471 Potsdam (DE); GALLENKAMP, Daniel, 42109 Wuppertal (DE); BISSELL, Richard, Alexander, Stockport SK6 5AF (GB); BOUGLAS, Richard, Alexander, Buxton SK17 7AR (GB)
(74) Vertreter: BIP Patents
(86) Internationale Anmeldenummer: PCT/EP2014/064486
(87) Internationale Veröffentlichungsnummer: WO 2015/004075

(56) Entgegenhaltungen:
- WO-A1-2013/027168
- WO-A2-2009/071480

## Beschreibung

Die vorliegende Erfindung betrifft BET-proteininhibitorische, insbesondere BRD4-inhibitorische Dihydrochinoxalinone und Dihydropyridopyrazinone, Intermediate zur Herstellung der erfindungsgemäßen Verbindungen, pharmazeutische Mittel enthaltend die erfindungsgemäßen Verbindungen sowie deren prophylaktische und therapeutische Verwendung bei hyperproliferativen Erkrankungen, insbesondere bei Tumorerkrankungen. Desweiteren betrifft diese Erfindung die Verwendung von BET-Proteininhibitoren in viralen Infektionen, in neurodegenerativen Erkrankungen, in inflammatorischen Krankheiten, in atherosklerotischen Erkrankungen und in der männlichen Fertilitätskontrolle.

Die humane BET-Familie (bromodomain and extra C-terminal domain family) hat vier Mitglieder (BRD2, BRD3, BRD4 und BRDT), die zwei verwandte Bromodomänen und eine extraterminale Domäne enthalten (Wu und Chiang, J. Biol. Chem., 2007, 282:13141-13145). Die Bromodomänen sind Proteinregionen, die acetylierte Lysinreste erkennen. Solche acetylierten Lysine findet man oft amN-terminalen Ende von Histonen (z. B. Histon 3 oder Histon 4) und sind Merkmale für eine offene Chromatin-Struktur und aktive Gentranskription (Kuo und Allis, Bioessays, 1998, 20:615-626). Die verschiedenen Acetylierungsmuster, die durch BET Proteine in Histonen erkannt werden, wurden genau untersucht (Umehara et al., J. Biol. Chem., 2010, 285:7610-7618; Filippakopoulos et al., Cell, 2012, 149:214-231). Zusätzlich können Bromodomänen weitere acetylierte Proteine erkennen. Zum Beispiel bindet BRD4 an RelA, was zur Stimulierung von NF-κB und transkriptioneller Aktivität von inflammatorischen Genen führt (Huang et al., Mol. Cell. Biol., 2009, 29:1375-1387; Zhang et al., J. Biol. Chem., 2012, 287: 28840-28851; Zou et al., Oncogene, 2013, doi:10.1038/onc.2013.179). BRD4 bindet auch an Cyclin T1 und bildet einen aktiven Komplex, der für die Transkriptionselongation wichtig ist (Schröder et al., J. Biol. Chem., 2012, 287:1090-1099). Die extraterminale Domäne von BRD2, BRD3 und BRD4 interagiert mit mehreren Proteinen, die eine Rolle in der Chromatinmodulierung und der Regulation der Genexpression haben (Rahman et al., Mol. Cell. Biol., 2011, 31:2641-2652).
Mechanistisch spielen BET-Proteine eine wichtige Rolle im Zellwachstum und im Zellzyklus. Sie sind mit mitotischen Chromosomen assoziiert, was eine Rolle im epigenetischen Gedächtnis nahelegt (Dey et al., Mol. Biol. Cell, 2009, 20:4899-4909; Yang et al., Mol. Cell. Biol., 2008, 28:967-976). Eine Rolle von BRD4 in der post-mitotischen Reaktivierung der Gentranskription wurde nachgewiesen (Zhao et al., Nat. Cell. Biol., 2011, 13:1295-1304). BRD4 ist essentiell für die Transkriptionselongation und rekrutiert den Elongationskomplex P-TEFb, der aus CDK9 und Cyclin T1 besteht, was zur Aktivierung der RNA Polymerase II führt (Yang et al., Mol. Cell, 2005, 19:535-545; Schröder et al., J. Biol. Chem., 2012, 287:1090-1099). Folglich wird die Expression von Genen stimuliert, die in der Zellproliferation involviert sind, wie zum Beispiel c-Myc, Cyclin D1 und Aurora B (You et al., Mol. Cell. Biol., 2009, 29:5094-5103; Zuber et al., Nature, 2011, doi:10.1038). BRD2 ist in der Regulation von Targetgene des Androgenrezeptors beteiligt (Draker et al., PLOS Genetics, 2012, 8, e1003047). BRD2 und BRD3 binden an transkribierte Gene in hyperacetylierten Chromatinbereichen und fördern die Transkription durch RNA Polymerase II (LeRoy et al., Mol. Cell, 2008, 30:51-60).
Der Knock-down von BRD4 bzw. die Hemmung der Interaktion mit acetylierten Histonen in verschiedenen Zelllinien führt zu einem G1-Arrest (Mochizuki et al., J. Biol. Chem., 2008, 283:9040-9048; Mertz et al., Proc. Natl. Acad. Sci. USA, 2011, 108:16669-16674). Es wurde auch gezeigt, dass BRD4 an Promotorregionen von mehreren Genen, die in der G1-Phase aktiviert werden wie zum Beispiel Cyclin D1 und D2, bindet (Mochizuki et al., J. Biol. Chem., 2008, 283:9040-9048). Zusätzlich wurde eine Hemmung der Expression von c-Myc, ein essentieller Faktor in der Zellproliferation, nach BRD4-Inhibition nachgewiesen (Dawson et al., Nature, 2011, 478:529-533; Delmore et al., Cell, 2011, 146:1-14; Mertz et al., Proc. Natl. Acad. Sci. USA, 2011, 108:16669-16674). Eine Hemmung der Expression von androgenregulierten Genen und eine Bindung von BRD2 an entsprechende regulatorischen Regionen wurde auch nachgewiesen (Draker et al., PLOS Genetics, 2012, 8, e1003047).
BRD2 und BRD4 Knockout-Mäuse sterben früh während der Embryogenese (Gyuris et al., Biochim. Biophys. Acta, 2009, 1789:413-421; Houzelstein et al., Mol. Cell. Biol., 2002, 22:3794-3802). Heterozygote BRD4 Mäuse haben verschiedene Wachstumsdefekte, die auf eine reduzierte Zellproliferation zurückzuführen sind (Houzelstein et al., Mol. Cell. Biol., 2002, 22:3794-3802). BET-Proteine spielen eine wichtige Rolle in verschiedenen Tumorarten. Die Fusion zwischen den BET-Proteinen BRD3 oder BRD4 und NUT, einem Protein, das normalerweise nur im Hoden exprimiert wird, führt zu einer aggressiven Form des Plattenepithelkarzinoms, genannt NUT midline carcinoma (French, Cancer Genet. Cytogenet., 2010, 203:16-20). Das Fusionsprotein verhindert Zelldifferenzierung und fördert Proliferation (Yan et al., J. Biol. Chem., 2011, 286:27663-27675, Grayson et al., 2013, doi:10-1038/onc.2013.126). Das Wachstum von davon abgeleiteten *in vivo* Modellen wird durch einen BRD4-Inhibitor gehemmt (Filippakopoulos et al., Nature, 2010, 468:1067-1073). Ein Screening für therapeutische Targets in einer akuten myeloiden Leukämiezelllinie (AML) zeigte, dass BRD4 eine wichtige Rolle in diesem Tumor spielt (Zuber et al., Nature, 2011, 478, 524-528). Die Reduktion der BRD4-Expression führt zu einem selektiven Arrest des Zellzyklus und zur Apoptose. Die Behandlung mit einem BRD4-Hemmer verhindert die Proliferation eines AML-Xenografts *in vivo.* Weitere Versuche mit einem BRD4-Hemmer zeigen, dass BRD4 eine Rolle in verschiedenen hämatologischen Tumoren spielt, wie zum Beispiel Multiples Myelom (Delmore et al., Cell, 2011, 146, 904-917) und Burkitt's Lymphom (Mertz et al., Proc. Natl. Acad. Sci. USA, 2011, 108, 16669-16674). Auch in soliden Tumoren, wie zum Beispiel Lungenkrebs spielt BRD4 eine wichtige Rolle (Lockwood et al., Proc. Natl. Acad. Sci. USA, 2012, 109, 19408-19413). Eine erhöhte Expression von BRD4 wurde im Multiplen Myelom festgestellt, und auch eine Amplifizierung des BRD4-Gens wurde in Patienten mit Multiplem Myelom festgestellt (Delmore et al., Cell, 2011, 146, 904-917). Eine Amplifizierung der DNA-Region, die das BRD4-Gen enthält, wurde in primären Brusttumoren nachgewiesen (Kadota et al., Cancer Res, 2009, 69:7357-7365). Auch für BRD2 gibt es Daten bezüglich einer Rolle in Tumoren. Eine transgene Maus, die BRD2 selektiv in B-Zellen hochexprimiert, entwickelt B-Zell Lymphome und Leukämien (Greenwall et al., Blood, 2005, 103:1475-1484).
BET-Proteine sind auch an viralen Infektionen beteiligt. BRD4 bindet an das E2 Protein von verschiedenen Papillomaviren und ist wichtig für das Überleben der Viren in latent infizierten Zellen (Wu et al., Genes Dev., 2006, 20:2383-2396; Vosa et al., J. Virol., 2006, 80:8909-8919). Auch das Herpesvirus, das für das Kaposi-Sarkom verantwortlich ist, interagiert mit verschiedenen BET-Proteinen, was für die Krankheitsbeständigkeit wichtig ist (Viejo-Borbolla et al., J. Virol., 2005, 79:13618-13629; You et al., J. Virol., 2006, 80:8909-8919). Durch Bindung an P-TEFb spielt BRD4 auch eine wichtige Rolle in der Replikation von HIV-1 (Bisgrove et al., Proc. Natl Acad. Sci. USA, 2007, 104:13690-13695). Die Behandlung mit einem BRD4-Hemmer führt zu einer Stimulierung des ruhenden, nicht behandelbaren Reservoirs von HIV-1 Viren in T-Zellen (Banerjee et al., J. Leukoc. Biol., 2012, 92, 1147-1154). Diese Reaktivierung könnte neue Therapiewege für AIDS-Behandlung ermöglichen (Zinchenko et al., J. Leukoc. Biol., 2012, 92, 1127-1129). Eine kritische Rolle von BRD4 in der DNA Replikation von Polyomaviren wurde auch berichtet (Wang et al., PLoS Pathog., 2012, 8, doi:10.1371).
BET-Proteine sind zusätzlich an Inflammationsprozessen beteiligt. BRD2-hypomorphe Mäuse zeigen eine reduzierte Inflammation im Fettgewebe (Wang et al., Biochem. J., 2009, 425:71-83). Auch die Infiltration von Makrophagen in weißem Fettgewebe ist in BRD2-defizienten Mäusen reduziert (Wang et al., Biochem. J., 2009, 425:71-83). Es wurde auch gezeigt, dass BRD4 eine Reihe von Genen reguliert, die in der Inflammation involviert sind. In LPS-stimulierten Makrophagen verhindert ein BRD4-Inhibitor die Expression von inflammatorischen Genen, wie zum Beispiel IL-1 oder IL-6 (Nicodeme et al., Nature, 2010, 468:1119-1123).
BET-Proteine sind auch in der Regulierung des ApoA1-Gens involviert (Mirguet et al., Bioorg. Med. Chem. Lett., 2012, 22:2963-2967). Das entsprechende Protein ist Bestandteil des Lipoproteins höherer Dichte (HDL), das bei Atherosklerose eine wichtige Rolle spielt (Smith, Arterioscler. Thromb. Vasc. Biol., 2010, 30:151-155). Durch die Stimulierung der ApoA1-Expression, können BET-Proteininhibitoren die Konzentrationen an Cholesterin HDL erhöhen und somit für die Behandlung von Atherosklerose potentiell nützlich sein (Mirguet el al., Bioorg. Med. Chem. Lett., 2012, 22:2963-2967).
Das BET-Protein BRDT spielt eine wesentliche Rolle in der Spermatogenese durch die Regulierung der Expression mehreren Genen, die während und nach der Meiose wichtig sind (Shang et al., Development, 2007, 134:3507-3515; Matzuk et al., Cell, 2012, 150:673-684).

Desweiteren ist BRDT in der post-meiotischen Organisation des Chromatins involviert (Dhar et al., J. Biol. Chem., 2012, 287:6387-6405). *In vivo* Versuche in der Maus zeigen, dass die Behandlung mit einem BET-Hemmer, der auch BRDT inhibiert, zu einer Abnahme der Spermienproduktion und Infertilität führt (Matzuk et al., Cell, 2012, 150:673-684).

Alle diese Untersuchungen zeigen, dass die BET-Proteine eine essentielle Rolle in verschiedenen Pathologien und auch in der männlichen Fertilität spielen. Es wäre deshalb wünschenswert, potente und selektive Inhibitoren zu finden, die die Interaktion zwischen den BET-Proteinen und acetylierten Proteinen verhindern. Diese neuen Inhibitoren sollten auch geeignete pharmakokinetische Eigenschaften haben, die es erlauben *in vivo*, also im Patienten, diese Interaktionen zu hemmen.

Es wurde nun gefunden, dass substituierte Dihydrochinoxalinone und Pyridopyrazinone die erwünschten Eigenschaften aufweisen, d.h. eine BET-inhibitorische, insbesondere eine BRD4-inhibitorische Wirkung zeigen. Die erfindungsgemäßen Verbindungen stellen somit wertvolle Wirkstoffe zur prophylaktischen und therapeutischen Verwendung bei hyper-proliferativen Erkrankungen, insbesondere bei Tumorerkrankungen dar. Desweiteren können die erfindungsgemäßen Verbindungen bei viralen Infektionen, bei neurodegenerativen Erkrankungen, bei inflammatorischen Krankheiten, bei atherosklerotischen Erkrankungen und in der männlichen Fertilitätskontrolle zur Anwendung kommen.

### Stand der Technik

Die bei der Betrachtung des Standes der Technik angewandte Nomenklatur (abgeleitet aus der Nomenklatursoftware ACD Name batch, Version 12.01, von Advanced Chemical Development, Inc.) wird durch die nachfolgenden Abbildungen verdeutlicht:

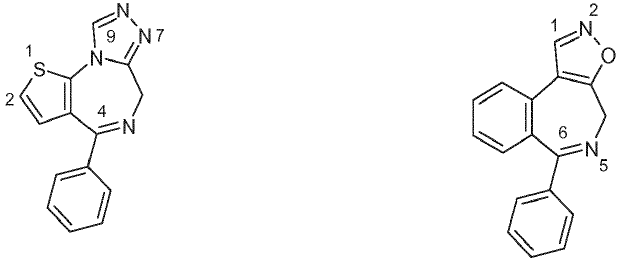

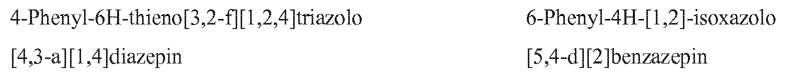

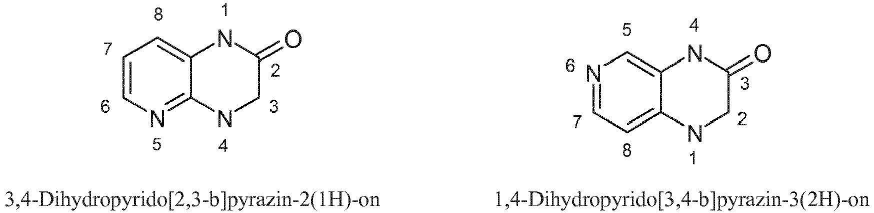

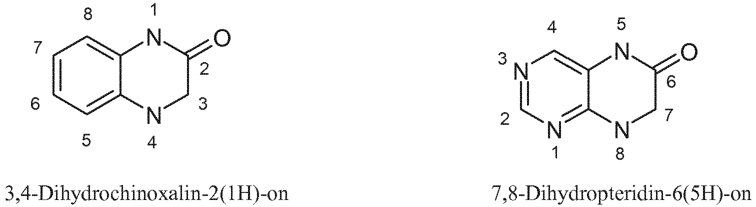

Bezogen auf die chemische Struktur wurden bisher nur sehr wenige Typen von BRD4-Inhibitoren beschrieben (Chun-Wa Chung et al., Progress in Medicinal Chemistry 2012, 51, 1-55).
Die ersten publizierten BRD4-Inhibitoren waren Diazepine. So werden z. B. Phenyl-thieno-triazolo-1,4-diazepine (4-Phenyl-6*H*-thieno[3,2-*f*][1,2,4]triazolo[4,3-*a*][1,4]diazepine) in WO2009/084693 (Mitsubishi Tanabe Pharma Corporation) und als Verbindung JQ1 in WO2011/143669 (Dana Farber Cancer Institute) beschrieben.

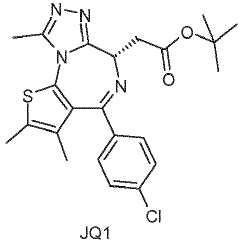

Der Ersatz der Thieno- durch eine Benzo-Einheit führt ebenfalls zu aktiven Inhibitoren (J. Med. Chem. 2011, 54, 3827 - 3838; E. Nicodeme et al., Nature 2010, 468, 1119). Weitere 4-Phenyl-6H-thieno[3,2-*f*][1,2,4]triazolo[4,3-*α*][1,4]diazepine und verwandte Verbindungen mit alternativen Ringen als Fusionspartner anstelle der Benzo-Einheit werden generisch beansprucht oder explizit beschrieben in WO2012/075456 (Constellation Pharmaceuticals).
Azepine als BRD4-Inhibitoren werden in der WO2012/075383 (Constellation Pharmaceuticals) beschrieben. Diese Anmeldung betrifft 6-substituierte 4*H*-Isoxazolo[5,4-*d*][2]benzazepine und 4*H-*Isoxazolo[3,4-*d*][2]benzazepine einschließlich solcher Verbindungen, die an Position 6 optional substituiertes Phenyl aufweisen und auch Analoga mit alternativen heterocyclischen Fusionspartnern anstelle der Benzo-Einheit, wie z.B. Thieno- oder Pyridoazepine. Als eine andere strukturelle Klasse von BRD4-Inhibitoren werden 7-Isoxazolochinoline und verwandte Chinolon-Derivate beschrieben (Bioorganic & Medicinal Chemistry Letters 22 (2012) 2963-2967). In WO2011/054845 (GlaxoSmithKline) werden weitere Benzodiazepine als BRD4-Inhibitoren beschrieben.

Bei den erfindungsgemäßen Verbindungen handelt es sich hingegen um substituierte 3,4-Dihydrochinoxalin-2(1H)-on-Derivate sowie 3,4-Dihydropyrido[2,3-b]pyrazin-2(1H)-on-Derivate, die sich strukturell in vielfältiger Form von den oben diskutierten Chemotypen von BRD4-Inhibitoren unterscheiden. Aufgrund der wesentlichen Strukturunterschiede war nicht davon auszugehen, dass die hier beanspruchten Verbindungen auch BRD4-inhibitorisch wirksam sind. Es ist deshalb überraschend, dass die erfindungsgemäßen Verbindungen trotz der erheblichen Strukturunterschiede eine gute inhibitorische Wirkung aufweisen.

Einige Schriften beinhalten strukturell ähnliche, aber auf völlig andere Wirkmechanismen und teilweise auch auf andere Indikationen gerichtete Verbindungen. Dihydrochinoxalinone und Dihydropyridopyrazinone, sowie verwandte bicyclische Systeme sind in einer Reihe von Patentanmeldungen beschrieben.

WO 2010/085570 (Takeda Pharmaceutical Company) beschreibt Hemmer der Poly-ADP-Ribose-Polymerase (PARP), die aus einer Reihe bi- und tricyclischer Gerüste abgeleitet sind, und welche 3,4-Dihydropyrido[2,3-b]pyrazin-2(1H)-on-Derivate einschließen, als Arzneimittel zur Behandlung verschiedener Krankheiten. Die darin offenbarten Beispielverbindungen unterscheiden sich von den erfindungsgemäßen Verbindungen beispielsweise durch Art und Position der Substitution am Pyrido-Teil des Dihydropyridopyrazinon-Gerüstes.

WO 2006/005510 (Boehringer Ingelheim) beschreibt 1,4-Dihydropyrido[3,4-b]pyrazin-3(2H)-on-Derivate als Inhibitoren von PLK-1 zur Behandlung hyperproliferativer Erkrankungen. Die Position des Pyrido-Stickstoffes unterscheidet die hier offenbarten Substanzen von den erfindungsgemäßen Verbindungen. Die beanspruchten Substanzen sind durch eine anilinische Gruppe gekennzeichnet, die via -NH- an C-7 des Dihydropyridopyrazinon-Gerüstes gebunden ist, und seinerseits in *para-*Position mit einem Carboxamid substituiert ist.

WO 2008/117061 (Sterix Ltd) beschreibt eine Reihe bicyclischer Chemotypen, darunter 3,4-Dihydrochinoxalin-2(1H)-on-Derivate, als Inhibitoren der Steroid-Sulfatase, unter anderem zur Verwendung zur Hemmung des Wachstums von Tumoren. Die in der genannten Anmeldung beanspruchten Substanzen unterscheiden sich von den in der hier vorliegenden Erfindung offenbarten Substanzen beispielsweise durch die Substitution an N-1.

US 2006/0019961 (P. E. Mahaney et al.) beschreibt substituierte 3,4-Dihydrochinoxalin-2(1H)-on-Derivate als Modulatoren des Estrogen-Rezeptors zur Behandlung verschiedener entzündlicher, kardiovaskulärer, sowie Autoimmun-Erkrankungen. Die in dieser Anmeldung offenbarten Beispielsubstanzen weisen an C-6 nur kleine Substituenten (wie Halogen oder Methyl) auf, dagegen an N-4 einen Substituenten, der verpflichtend einen hydroxylierten Aromaten aufweist, wodurch sich die Substanzen von den Verbindungen der hier vorliegenden Erfindung unterscheiden.

WO 2006/050054, WO 2007/134169 und US 2009/0264384 (Nuada LLC) beschreiben eine Reihe bicyclischer Chemotypen, darunter 3,4-Dihydrochinoxalin-2(1H)-on-Derivate, als Hemmer von Tumor-Nekrose-Faktor alpha (TNF-α) sowie verschiedener Isoformen der Phosphodiesterase zur Behandlung unter anderem von entzündlichen Erkrankungen. N-1 ist bei den beanspruchten Strukturen durch eine Gruppe substituiert, die gekennzeichnet ist beispielsweise durch ein Carboxamid oder eine von der Boronsäure abgeleitete terminale Gruppe, die sie von den Verbindungen der hier vorliegenden Erfindung unterscheiden.

WO 2003/020722 und WO 2004/076454 (Boehringer Ingelheim) offenbaren 7,8-Dihydropteridin-6(5H)-one als Hemmer spezifischer Zellcyclus-Kinasen zur Therapie hyperproliferativer Erkrankungen.

WO 2006/018182 (Boehringer Ingelheim) beschreibt pharmazeutische Zubereitungen von 7,8-Dihydropteridin-6(5H)-onen in Kombination unter anderem mit verschiedenen Zytostatika zur Therapie von Tumorerkrankungen.

WO 2006/018185 (Boehringer Ingelheim) beschreibt die Verwendung von 7,8-Dihydropteridin-6(5H)-onen zur Therapie verschiedener Tumorerkrankungen.

WO 2011/101369 (Boehringer Ingelheim), WO 2011/113293 (Jiangsu Hengrui Medicine), WO 2009/141575 (Chroma Therapeutics) WO 2009/071480 (Nerviano Medical Sciences), sowie WO 2006/021378, WO 2006/021379 und WO 2006/021548 (ebenfalls (Boehringer Ingelheim) offenbaren weitere 7,8-Dihydropteridin-6(5H)-on-Derivate als Hemmer von PLK-1 zur Behandlung hyperproliferativer Erkrankungen.

US 6,369,057 beschreibt verschiedene Chinoxalin- und Chinoxalinon-Derivate als antivirale Wirkstoffe; EP 0657166 und EP 728481 beschreiben Kombinationen solcher Verbindungen mit Nucleosiden beziehungsweise Protease-Hemmern mit antiviraler Wirkung.

WO 2007/022638 (Methylgene Inc.) offenbart ganz allgemein HDAC-Inhibitoren mehrerer Chemotypen, unter anderem Dihydrochinoxalinon-Derivate, jedoch unterscheiden sich die Strukturen der offenbarten Beispielverbindungen deutlich von den Verbindungen der vorliegenden Erfindung.

WO 1999/050254 (Pfizer) beschreibt unter anderem Chinoxalinone und Dihydrochinoxalinone als Hemmer von Serinproteasen zur antithrombotischen Therapie, jedoch unterscheiden sich diese Verbindungen deutlich durch Art und Position der Substituenten von den erfindungsgemässen Verbindungen.

Einige an C-6 mit einer aromatischen Aminogruppe, deren Phenylgruppe ihrerseits mit einer *para*ständigen Amid-Gruppe substituiert ist, substituierte 3,4-Dihydrochinoxalin-2(1H)-on-Derivate (entsprechend 2-Oxo-1,2,3,4-Tetrahydrochinoxalin-Derivaten) sind von *Chemical Abstracts* als "Chemical Library"-Substanzen ohne Literaturreferenz indexiert [siehe 4-{[(3*R*)-4-Cyclopentyl-3-ethyl-1-methyl-2-oxo-1,2,3,4-tetrahydrochinoxalin-6-yl]amino}-3-methoxy-*N*-[2-methyl-1-(pyrrolidin-1-yl)propan-2-yl]benzamid, *CAS Registry-Nr. 1026451-60-4, N*-(1-Benzylpiperidin-4-yl)-4-{[(3*R*)-4-cyclopentyl-1,3-dimethyl-2-oxo-1,2,3,4-tetrahydrochinoxalin-6-yl]amino}-3-methoxybenzamid, *CAS Registry*-*Nr*. *1026961-36-3*, 4-{[(3*R*)-4*-*Cyclohexyl-1,3-dimethyl-2-oxo-1,2,3,4-tetrahydrochinoxalin-6-yl]amino}-*N-*[1-(dimethylamino)-2-methylpropan-2-yl]-3-methoxybenzamid, *CAS Registry-Nr. 1025882-57-8*]*.* Eine therapeutische Anwendung ist für diese Verbindungen bisher nicht beschrieben.

Dennoch besteht nach wie vor ein großes Bedürfnis nach wirksamen Verbindungen zur Prophylaxe und Therapie von Erkrankungen, insbesondere von hyperproliferativen Erkrankungen, und ganz besonders von Tumorerkrankungen.

Es wurde nun gefunden, dass Verbindungen der allgemeinen Formel (I) 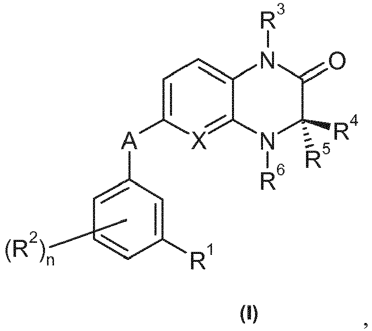 in der
- A: für -NH-, -N(C₁-C₃-Alkyl)- oder -O- steht,
- X: für -N- oder -CH- steht,
- n: für 0,1 oder 2 steht,
- R¹: für eine Gruppe -C(=O)NR⁷R⁸ oder -S(=O)₂NR⁷R⁸ steht,
oder
für 5-gliedriges monocyclisches Heteroaryl- steht, das gegebenenfalls ein-, zwei- oder dreifach, gleich oder verschieden substituiert sein kann mit Halogen, Cyano, C₁-C₄-lkyl-, C₂-C₄-Alkenyl-, C₂-C₄-Alkinyl-, Halogen-C₁-C₄-alkyl-, C₁-C₄-Alkoxy-, Halogen-C₁-C₄-alkoxy-, C₁-C₄-Alkylthio-, Halogen-C₁-C₄-Alkylthio-, -NR⁹R¹⁰, -C(=O)OR¹¹, -C(=O)N⁹R¹⁰, -C(=O)R¹¹, -S(=O)₂R¹¹, -S(=O)₂NR⁹R¹⁰,
- R²: für Wasserstoff, Halogen, Cyano, C₁-C₃-Alkyl-, C₂-C₄-Alkenyl-, C₂-C₄-Alkinyl-, Halogen-C₁-C₄-alkyl-, C₁-C₄-Alkoxy-, Halogen-C₁-C₄-alkoxy-, C₁-C₄-Alkylthio- oder Halogen-C₁-C₄-Alkylthio- steht, und falls n für 2 steht, kann R² gleich oder verschieden sein,
oder
- R¹ und R²: gemeinsam für eine Gruppe *-S(=O)₂-NR⁸-CH₂-**, *-S(=O)₂-NR⁸-CH₂-CH₂-**, *-C(=O)-NR⁸-CH₂-** oder *-C(=O)-NR⁸-CH₂-CH₂-** stehen, worin "*" den Anknüpfungspunkt von R¹ am in Formel (I) enthaltenen Phenylring bedeutet, und worin "**" ein diesem Anknüfungspunkt benachbartes Kohlenstoffatom dieses Phenylringes bedeutet,
- R³: für Methyl- oder Ethyl- steht
- R⁴: für Wasserstoff oder C₁-C₃-Alkyl- steht,
- R⁵: für Wasserstoff oder C₁-C₃-Alkyl- steht,
oder
- R⁴ und R⁵: gemeinsam mit dem Kohlenstoffatom, an das sie gebunden sind, für C₃-C₆-Cycloalkylen stehen,
- R⁶: für C₁-C₆-Alkyl- steht, das gegebenenfalls einfach substituiert sein kann mit C₁-C₃-Alkoxy-, Phenyl-, C₃-C₈-Cycloalkyl-, oder 4-bis 8-gliedrigem Heterocycloalkyl-,
worin Phenyl- seinerseits gegebenenfalls ein-, zwei- oder dreifach, gleich oder verschieden substituiert sein kann mit: Halogen, Cyano, C₁-C₄-Alkyl-, C₂-C₄-Alkenyl-, C₂-C₄-Alkinyl-, C₁-C₄-Alkoxy-, Halogen-C₁-C₄-Alkyl-, Halogen-C₁-C₄-Alkoxy-, und worin C₃-C₈-Cycloalkyl- und 4-bis 8-gliedriges Heterocycloalkyl- ihrerseits gegebenenfalls ein- oder zweifach, gleich oder verschieden substituiert sein können mit C₁-C₃-Alkyl-,
oder
für C₃-C₈-Cycloalkyl- oder 4-bis 8-gliedriges Heterocycloalkyl- steht, die gegebenenfalls ein- oder zweifach, gleich oder verschieden substituiert sein können mit C₁-C₃-Alkyl- oder C₁-C₄-Alkoxycarbonyl-,
oder
für Phenyl steht, das gegebenenfalls ein- oder zweifach, gleich oder verschieden substituiert sein kann mit Halogen, C₁-C₃-Alkyl- oder 4-8-gliedrigem Heterocycloalkyl-,
worin das 4-8-gliedrige Heterocycloalkyl- seinerseits gegebenenfalls ein- oder zweifach, gleich oder verschieden substituiert sein kann mit C₁-C₃-Alkyl- oder C₁-C₄-Alkoxycarbonyl-,
- R⁷: für Wasserstoff steht,
oder
für C₁-C₆-Alkyl- steht, das gegebenenfalls ein-, zwei-, oder dreifach, gleich oder verschieden substituiert sein kann mit: Hydroxy, Oxo, Fluor, Cyano, C₁-C₄-Alkoxy-, Halogen-C₁-C₄-alkoxy-, -NR⁹R¹⁰, C₃-C₈-Cycloalkyl-, C₄-C₈-Cycloalkenyl-, 4- bis 8-gliedrigem Heterocycloalkyl-, 4- bis 8-gliedrigem Heterocycloalkenyl-, C₅-C₁₁-Spirocycloalkyl-, C₅-C₁₁-Heterospirocycloalkyl-, verbrücktem C₆-C₁₂-Cycloalkyl-, verbrücktem C₆-C₁₂-Heterocycloalkyl-, C₆-C₁₂-Bicycloalkyl-, C₆-C₁₂-Heterobicycloalkyl-, Phenyl-, 5- bis 6-gliedrigem Heteroaryl-,
worin C₃-C₈-Cycloalkyl-, C₄-C₈-Cycloalkenyl-, 4- bis 8-gliedriges Heterocycloalkyl-, 4- bis 8-gliedriges Heterocycloalkenyl-, C₅-C₁₁-Spirocycloalkyl-, C₅-C₁₁-Heterospirocycloalkyl-, verbrücktes C₆-C₁₂-Cycloalkyl-, verbrücktes C₆-C₁₂-Heterocycloalkyl-, C₆-C₁₂-Bicycloalkyl-, C₆-C₁₂-Heterobicycloalkyl- ihrerseits gegebenenfalls ein- oder zweifach, gleich oder verschieden substituiert sein können mit: Hydroxy, Fluor, Oxo, Cyano, C₁-C₃-Alkyl-, Fluor-C₁-C₃-Alkyl-, C₃-C₆-Cycloalkyl-, Cyclopropylmethyl-, C₁-C₃-Alkylcarbonyl-, C₁-C₄-Alkoxycarbonyl- oder -NR⁹R¹⁰, und
worin Phenyl- und 5- bis 6-gliedriges Heteroaryl- gegebenenfalls ein- oder zweifach, gleich oder verschieden substituiert sein können mit: Halogen, Cyano, Trifluormethyl-, C₁-C₃-Alkyl-, C₁-C₃-Alkoxy-,
oder
für C₃-C₆-Alkenyl- oder C₃-C₆-Alkinyl- steht,
oder
für C₃-C₈-Cycloalkyl-, C₄-C₈-Cycloalkenyl-, C₅-C₁₁-Spirocycloalkyl-, verbrücktes C₆-C₁₂-Cycloalkyl- oder C₆-C₁₂-Bicycloalkyl- steht, die gegebenenfalls ein- oder zweifach, gleich oder verschieden substituiert sein können mit: Hydroxy, Oxo, Cyano, Fluor, C₁-C₃-Alkyl, C₁-C₃₋Alkoxy, Trifluormethyl, -NR⁹R¹⁰,
oder
für 4- bis 8-gliedriges Heterocycloalkyl-, 4- bis 8-gliedriges Heterocycloalkenyl-, C₅-C₁₁-Heterospirocycloalkyl-, verbrücktes C₆-C₁₂-Heterocycloalkyl- oder C₆-C₁₂-Heterobicycloalkyl- steht, die gegebenenfalls ein- oder zweifach, gleich oder verschieden substituiert sein können mit: Hydroxy, Fluor, Oxo, Cyano, C₁-C₃-Alkyl-, Fluor-C₁-C₃-Alkyl-, C₃-C₆-Cycloalkyl-, Cyclopropylmethyl-, C₁-C₃-Alkylcarbonyl-, C₁-C₄-Alkoxycarbonyl- oder -NR⁹R¹⁰,
- R⁸: für Wasserstoff oder für gegebenenfalls ein- oder zweifach, gleich oder verschieden mit Hydroxy, Oxo oder C₁-C₃-Alkoxy- substituiertes C₁-C₃-Alkyl-, oder für Fluor-C₁-C₃-Alkyl steht,
oder
- R⁷ und R⁸: gemeinsam mit dem Stickstoffatom, an welches sie gebunden sind, für 4-bis 8-gliedriges Heterocycloalkyl, 4-bis 8-gliedriges Heterocycloalkenyl-, C₅-C₁₁-Heterospirocycloalkyl-, verbrücktes C₆-C₁₂-Heterocycloalkyl- oder C₆-C₁₂-Heterobicycloalkyl- stehen, die gegebenenfalls ein- oder zweifach, gleich oder verschieden substituiert sein können mit: Hydroxy, Fluor, Oxo, Cyano, C₁-C₃-Alkyl-, Fluor-C₁-C₃-Alkyl-, C₃-C₆-Cycloalkyl-, Cyclopropylmethyl-, C₁-C₃-Alkylcarbonyl-, C₁-C₄-Alkoxycarbonyl- oder -NR⁹R¹⁰,
- R⁹ und R¹⁰: unabhängig voneinander für Wasserstoff oder für gegebenenfalls ein- oder zweifach, gleich oder verschieden mit Hydroxy, Oxo, C₁-C₃-Alkoxy- substituiertes C₁-C₃-Alkyl, Fluor-C₁-C₃-Alkyl, oder für 4-bis 8-gliedriges Heterocycloalkylstehen,
worin das 4-bis 8-gliedrige Heterocycloalkyl- seinerseits gegebenenfalls ein- oder zweifach, gleich oder verschieden substituiert sein kann mit C₁-C₃-Alkyl-,
oder
- R⁹ und R¹⁰: gemeinsam mit dem Stickstoffatom, an das sie gebunden sind, für 4- bis 8-gliedriges Heterocycloalkyl- stehen, das gegebenenfalls ein- oder zweifach, gleich oder verschieden substituiert sein kann mit: Hydroxy, Fluor, Oxo, Cyano, C₁-C₃-Alkyl-, Fluor-C₁-C₃-Alkyl-, C₃-C₆-Cycloalkyl-, Cyclopropylmethyl-, C₁-C₃-Alkylcarbonyl-oder C₁-C₄-Alkoxycarbonyl-,
- R¹¹: für C₁-C₆-Alkyl- oder Phenyl-C₁-C₃-Alkyl- steht,

sowie deren Diastereomere, Racemate, Polymorphe und physiologisch verträglichen Salze, überraschenderweise die Interaktion zwischen BET-Proteinen, insbesondere BRD4, und einem acetylierten Histon 4-Peptid inhibieren und somit das Wachstum von Krebszellen hemmen.

Bevorzugt sind solche Verbindungen der allgemeinen Formel (I),

in der
- A: für -NH- oder -N(C₁-C₃-Alkyl)- steht,
- X: für -N- oder -CH- steht,
- n: für 0,1 oder 2 steht,
- R¹: für eine Gruppe -C(=O)NR⁷R⁸ oder -S(=O)₂NR⁷R⁸ steht,
oder
für Oxazolyl-, Thiazolyl-, Oxadiazolyl- oder Thiadiazolyl- steht, die gegebenenfalls ein- oder zweifach, gleich oder verschieden substituiert sein können mit Halogen, Cyano, C₁-C₃-Alkyl-, Trifluormethyl-, C₁-C₃-Alkoxy-, Trifluormethoxy- oder -NR⁹R¹⁰,
- R²: für Wasserstoff, Fluor, Chlor, Cyano, Methyl-, Methoxy-, Ethyl- oder Ethoxysteht, und falls n für 2 steht, kann R² gleich oder verschieden sein, oder
- R¹ und R²: gemeinsam für eine Gruppe *-S(=O)₂-NR⁸-CH₂-** oder *-C(=O)-NR⁸-CH₂-** stehen, worin "*" den Anknüpfungspunkt von R¹ am in Formel (I) enthaltenen Phenylring bedeutet, und worin "**" ein diesem Anknüfungspunkt benachbartes Kohlenstoffatom dieses Phenylringes bedeutet,
- R³: für Methyl- oder Ethyl- steht,
- R⁴: für Wasserstoff, Methyl- oder Ethyl- steht,
- R⁵: für Wasserstoff, Methyl- oder Ethyl- steht,
- R⁶: für C₂-C₅-Alkyl- steht,
oder
für Methyl- oder Ethyl- steht, das einfach substituiert ist mit C₁-C₃-Alkoxy-, Phenyl- oder 4-bis 8-gliedrigem Heterocycloalkyl-,
worin Phenyl- seinerseits gegebenenfalls ein-, zwei- oder dreifach, gleich oder verschieden substituiert sein kann mit: Fluor, Chlor, Brom, Cyano, C₁-C₃-Alkyl-, C₁-C₃-Alkoxy-, und
worin das 4-bis 8-gliedrige Heterocycloalkyl- seinerseits gegebenenfalls ein- oder zweifach, gleich oder verschieden substituiert sein kann mit Methyl-,
oder
für C₃-C₈-Cycloalkyl- oder 4-bis 8-gliedriges Heterocycloalkyl- steht, die gegebenenfalls ein- oder zweifach, gleich oder verschieden substituiert sein können mit C₁-C₃-Alkyl- oder C₁-C₄-Alkoxycarbonyl-,
oder
für Phenyl steht, das gegebenenfalls ein- oder zweifach, gleich oder verschieden substituiert sein kann mit Fluor, Chlor, Methyl- oder 6-gliedrigem Heterocycloalkyl-,
worin das 6-gliedrige Heterocycloalkyl- seinerseits gegebenenfalls einfach substituiert sein kann mit Methyl- oder *tert*-Butoxycarbonyl-,
- R⁷: für Wasserstoff steht,
oder
für C₁-C₆-Alkyl- steht, das gegebenenfalls ein-, zwei-, oder dreifach, gleich oder verschieden substituiert sein kann mit: Hydroxy, Oxo, Fluor, Cyano, C₁-C₃-Alkoxy-, Fluor-C₁-C₃-alkoxy-, -NR⁹R¹⁰, 4- bis 8-gliedrigem Heterocycloalkyl-, Phenyl-, 5- bis 6-gliedrigem Heteroaryl-,
worin das 4- bis 8-gliedrige Heterocycloalkyl- seinerseits gegebenenfalls einfach substituiert sein kann mit: Hydroxy, Oxo, C₁-C₃-Alkyl-, Fluor-C₁-C₃-Alkyl-, Cyclopropyl-, Cyclopropylmethyl-, Acetyl- oder *tert-*Butoxycarbonyl-,
oder
für C₃-C₆-Cycloalkyl- steht, das gegebenenfalls ein- oder zweifach, gleich oder verschieden substituiert sein kann mit: Hydroxy, Oxo, Cyano, Fluor, -NR⁹R¹⁰,
oder
für 4- bis 8-gliedriges Heterocycloalkyl-, C₆-C₈-Heterospirocycloalkyl-, verbrücktes C₆-C₁₀-Heterocycloalkyl- oder C₆-C₁₀-Heterobicycloalkyl- steht, die gegebenenfalls ein- oder zweifach, gleich oder verschieden substituiert sein können mit: Hydroxy, Oxo, C₁-C₃-Alkyl-, Fluor-C₁-C₃-Alkyl-, Cyclopropyl-, Cyclopropylmethyl-, Acetyl- oder *tert*-Butoxycarbonyl-,
- R⁸: für Wasserstoff oder C₁-C₃-Alkyl- steht,
oder
- R⁷ und R⁸: gemeinsam mit dem Stickstoffatom, an welches sie gebunden sind, für 4-bis 8-gliedriges Heterocycloalkyl-, C₆-C₈-Heterospirocycloalkyl-, verbrücktes C₆-C₁₀-Heterocycloalkyl- oder C₆-C₁₀-Heterobicycloalkyl- stehen, die gegebenenfalls ein- oder zweifach, gleich oder verschieden substituiert sein können mit: Hydroxy, Fluor, Oxo, C₁-C₃-Alkyl-, Fluor-C₁-C₃₋Alkyl-, Cyclopropyl-, Cyclopropylmethyl-, Acetyl- oder *tert*-Butoxycarbonyl-,
- R⁹ und R¹⁰: unabhängig voneinander für Wasserstoff oder für gegebenenfalls einfach mit Hydroxy oder Oxo substituiertes C₁-C₃-Alkyl- oder für Trifluormethyl-, oder für 6-gliedriges Heterocycloalkyl- stehen,
worin das 6-gliedrige Heterocycloalkyl- seinerseits gegebenenfalls ein- oder zweifach, gleich oder verschieden substituiert sein kann mit C₁-C₃-Alkyl-,
oder
- R⁹ und R¹⁰: gemeinsam mit dem Stickstoffatom, an das sie gebunden sind, für 4- bis 7-gliedriges Heterocycloalkyl- stehen, das gegebenenfalls ein- oder zweifach, gleich oder verschieden substituiert sein kann mit: Hydroxy, Fluor, Oxo, C₁-C₃-Alkyl-, Fluor-C₁-C₃-Alkyl-, Cyclopropyl-, Cyclopropylmethyl-, Acetyl- oder *tert-*Butoxycarbonyl-,
sowie deren Diastereomere, Racemate, Polymorphe und physiologisch verträglichen Salze.

Besonders bevorzugt sind solche Verbindungen der allgemeinen Formel (I),
in der
- A: für -NH- oder -N(Methyl)- steht,
- X: für -N- oder -CH- steht,
- n: für 0 oder 1 steht,
- R¹: für eine Gruppe -C(=O)NR⁷R⁸ oder -S(=O)₂NR⁷R⁸ steht,
oder
für Oxazolyl- oder Oxadiazolyl-, die gegebenenfalls ein- oder zweifach, gleich oder verschieden substituiert sein können mit C₁-C₃-Alkyl-,
- R²: für Wasserstoff, Fluor, Chlor, Methyl- oder Methoxy- steht, oder
- R¹ und R²: gemeinsam für eine Gruppe *-S(=O)₂-NR⁸-CH₂-** stehen, worin "*" den Anknüpfungspunkt von R¹ am in Formel (I) enthaltenen Phenylring bedeutet, und worin "**" ein diesem Anknüfungspunkt benachbartes Kohlenstoffatom dieses Phenylringes bedeutet,
- R³: für Methyl- steht,
- R⁴: für Methyl- oder Ethyl- steht,

- R⁵: für Wasserstoff steht,
- R⁶: für C₃-C₅-Alkyl- oder für 2-Methoxyethyl- steht,
oder
für Methyl- steht, das einfach substituiert ist mit Phenyl- oder 4-bis 6-gliedrigem Heterocycloalkyl-,
worin Phenyl- seinerseits gegebenenfalls ein-, oder zweifach, gleich oder verschieden substituiert sein kann mit: Fluor, Chlor, Cyano, Methyl-, Methoxy-, und
worin das 4-bis 6-gliedrige Heterocycloalkyl- seinerseits gegebenenfalls einfach substituiert sein kann mit Methyl-,
oder
für C₃-C₈-Cycloalkyl- oder für 4-bis 6-gliedriges Heterocycloalkyl- steht, die gegebenenfalls ein- oder zweifach, gleich oder verschieden substituiert sein können mit C₁-C₃-Alkyl- oder C₁-C₄-Alkoxycarbonyl-,
oder
für Phenyl steht, das gegebenenfalls ein- oder zweifach, gleich oder verschieden substituiert sein kann mit Fluor, Chlor, Methyl- oder *N-tert-*Butoxycarbonylpiperazinyl-,
- R⁷: für Wasserstoff steht,
oder
für C₁-C₄-Alkyl- steht, das gegebenenfalls einfach substituiert sein kann mit -NR⁹R¹⁰ oder 4- bis 8-gliedrigem Heterocycloalkyl-,
worin das 4- bis 8-gliedrige Heterocycloalkyl- seinerseits gegebenenfalls einfach substituiert sein kann mit: Oxo, C₁-C₃-Alkyl-, Fluor-C₁-C₃₋Alkyl-, Cyclopropyl- oder Cyclopropylmethyl-,
oder für C₃-C₆-Cycloalkyl- steht, das gegebenenfalls einfach substituiert sein kann mit Hydroxy, Fluor oder -NR⁹R¹⁰,
oder
für 4- bis 8-gliedriges Heterocycloalkyl- steht, das gegebenenfalls ein- oder zweifach, gleich oder verschieden substituiert sein kann mit: Oxo, C₁-C₃-Alkyl-, Fluor-C₁-C₃-Alkyl-, Cyclopropyl- oder Cyclopropylmethyl-,
- R⁸: für Wasserstoff, Methyl- oder Ethyl- steht,
oder
- R⁷ und R⁸: gemeinsam mit dem Stickstoffatom, an welches sie gebunden sind, für 4-bis 6-gliedriges Heterocycloalkyl- oder C₆-C₈-Heterospirocycloalkyl- stehen, die gegebenenfalls ein- oder zweifach, gleich oder verschieden substituiert sein können mit: Fluor, Oxo, C₁-C₃-Alkyl-, Fluor-C₁-C₃-Alkyl-, Cyclopropyl- oder Cyclopropylmethyl-,
- R⁹ und R¹⁰: unabhängig voneinander für Wasserstoff oder für gegebenenfalls einfach mit Hydroxy oder Oxo substituiertes C₁-C₃-Alkyl-, Trifluormethyl-, oder für *N-*Methylpiperidinyl- stehen,
oder
- R⁹ und R¹⁰: gemeinsam mit dem Stickstoffatom, an das sie gebunden sind, für 4- bis 7-gliedriges Heterocycloalkyl- stehen, das gegebenenfalls ein- oder zweifach, gleich oder verschieden substituiert sein kann mit: Fluor, Oxo, C₁-C₃-Alkyl-, Fluor-C₁-C₃-Alkyl-, Cyclopropyl- oder Cyclopropylmethyl-,
sowie deren Diastereomere, Racemate, Polymorphe und physiologisch verträglichen Salze.

Besonders bevorzugt sind weiterhin solche Verbindungen der allgemeinen Formel (I), in der
- A: für -NH- oder -N(Methyl)- steht,
- X: für -N- steht,
- n: für 0 oder 1 steht,
- R¹: für eine Gruppe -C(=O)NR⁷R⁸ oder -S(=O)₂NR⁷R⁸ steht,
oder
für Oxazolyl- oder Oxadiazolyl-, die gegebenenfalls ein- oder zweifach, gleich oder verschieden substituiert sein können mit C₁-C₃-Alkyl-,
- R²: für Wasserstoff, Fluor, Chlor, Methyl- oder Methoxy- steht, oder
- R¹ und R²: gemeinsam für eine Gruppe *-S(=O)₂-NR⁸-CH₂-** stehen, worin "*" den Anknüpfungspunkt von R¹ am in Formel (I) enthaltenen Phenylring bedeutet, und worin "**" ein diesem Anknüpfungspunkt benachbartes Kohlenstoffatom dieses Phenylringes bedeutet,
- R³: für Methyl- steht,
- R⁴: für Methyl- oder Ethyl- steht,
- R⁵: für Wasserstoff steht,
- R⁶: für C₃-C₅-Alkyl- oder für 2-Methoxyethyl- steht,
oder
für Methyl- steht, das einfach substituiert ist mit Phenyl- oder 4-bis 6-gliedrigem Heterocycloalkyl-,
worin Phenyl- seinerseits gegebenenfalls ein-, oder zweifach, gleich oder verschieden substituiert sein kann mit: Fluor, Chlor, Cyano, Methyl-, Methoxy-, und
worin das 4-bis 6-gliedrige Heterocycloalkyl- seinerseits gegebenenfalls einfach substituiert sein kann mit Methyl-,
oder
für C₃-C₈-Cycloalkyl- oder für 4-bis 6-gliedriges Heterocycloalkyl- steht, die gegebenenfalls ein- oder zweifach, gleich oder verschieden substituiert sein können mit C₁-C₃-Alkyl- oder C₁-C₄-Alkoxycarbonyl-,
oder
für Phenyl steht, das gegebenenfalls ein- oder zweifach, gleich oder verschieden substituiert sein kann mit Fluor, Chlor, Methyl- oder *N-tert-*Butoxycarbonylpiperazinyl-,
- R⁷: für Wasserstoff steht,
oder
für C₁-C₄-Alkyl- steht, das gegebenenfalls einfach substituiert sein kann mit -NR⁹R¹⁰ oder 4- bis 8-gliedrigem Heterocycloalkyl-,
worin das 4- bis 8-gliedrige Heterocycloalkyl- seinerseits gegebenenfalls einfach substituiert sein kann mit: Oxo, C₁-C₃-Alkyl-, Fluor-C₁-C₃-Alkyl-, Cyclopropyl- oder Cyclopropylmethyl-,
oder
für C₃-C₆-Cycloalkyl- steht, das gegebenenfalls einfach substituiert sein kann mit Hydroxy, Fluor oder -NR⁹R¹⁰,
oder
für 4- bis 8-gliedriges Heterocycloalkyl-, das gegebenenfalls ein- oder zweifach, gleich oder verschieden substituiert sein kann mit: Oxo, C₁-C₃-Alkyl-, Fluor-C₁-C₃-Alkyl-, Cyclopropyl- oder Cyclopropylmethyl-,
- R⁸: für Wasserstoff, Methyl- oder Ethyl- steht,
oder
- R⁷ und R⁸: gemeinsam mit dem Stickstoffatom, an welches sie gebunden sind, für 4-bis 6-gliedriges Heterocycloalkyl- oder C₆-C₈-Heterospirocycloalkyl- stehen, die gegebenenfalls ein- oder zweifach, gleich oder verschieden substituiert sein können mit: Fluor, Oxo, C₁-C₃-Alkyl-, Fluor-C₁-C₃₋Alkyl-, Cyclopropyl- oder Cyclopropylmethyl-,
- R⁹ und R¹⁰: unabhängig voneinander für Wasserstoff oder für gegebenenfalls einfach mit Hydroxy oder Oxo substituiertes C₁-C₃-Alkyl-, Trifluormethyl-, oder für *N-*Methylpiperidinyl- stehen,
oder
- R⁹ und R¹⁰: gemeinsam mit dem Stickstoffatom, an das sie gebunden sind, für 4- bis 7-gliedriges Heterocycloalkyl- stehen, das gegebenenfalls ein- oder zweifach, gleich oder verschieden substituiert sein kann mit: Fluor, Oxo, C₁-C₃-Alkyl-, Fluor-C₁-C₃-Alkyl-, Cyclopropyl- oder Cyclopropylmethyl-,
sowie deren Diastereomere, Racemate, Polymorphe und physiologisch verträglichen Salze.

Besonders bevorzugt sind weiterhin solche Verbindungen der allgemeinen Formel (I), in der
- A: für -NH- oder -N(Methyl)- steht,
- X: für -CH- steht,
- n: für 0 oder 1 steht,
- R¹: für eine Gruppe -C(=O)NR⁷R⁸ oder -S(=O)₂NR⁷R⁸ steht,
oder
für Oxazolyl- oder Oxadiazolyl-, die gegebenenfalls ein- oder zweifach, gleich oder verschieden substituiert sein können mit C₁-C₃-Alkyl-,
- R²: für Wasserstoff, Fluor, Chlor, Methyl- oder Methoxy- steht, oder
- R¹ und R²: gemeinsam für eine Gruppe *-S(=O)₂-NR⁸-CH₂-** stehen, worin "*" den Anknüpfungspunkt von R¹ am in Formel (I) enthaltenen Phenylring bedeutet, und worin "**" ein diesem Anknüfungspunkt benachbartes Kohlenstoffatom dieses Phenylringes bedeutet,
- R³: für Methyl- steht,
- R⁴: für Methyl- oder Ethyl- steht,
- R⁵: für Wasserstoff steht,
- R⁶: für C₃-C₅-Alkyl- oder für 2-Methoxyethyl- steht,
oder
für Methyl- steht, das einfach substituiert ist mit Phenyl- oder 4-bis 6-gliedrigem Heterocycloalkyl-,
worin Phenyl- seinerseits gegebenenfalls ein-, oder zweifach, gleich oder verschieden substituiert sein kann mit: Fluor, Chlor, Cyano, Methyl-, Methoxy-, und
worin das 4-bis 6-gliedrige Heterocycloalkyl- seinerseits gegebenenfalls einfach substituiert sein kann mit Methyl-,
oder
für C₃-C₈-Cycloalkyl- oder für 4-bis 6-gliedriges Heterocycloalkyl- steht, die gegebenenfalls ein- oder zweifach, gleich oder verschieden substituiert sein können mit C₁-C₃-Alkyl- oder C₁-C₄-Alkoxycarbonyl-, oder
für Phenyl steht, das gegebenenfalls ein- oder zweifach, gleich oder verschieden substituiert sein kann mit Fluor, Chlor, Methyl- oder *N-tert-*Butoxycarbonylpiperazinyl-,
- R⁷: für Wasserstoff steht,
oder
für C₁-C₄-Alkyl- steht, das gegebenenfalls einfach substituiert sein kann mit -NR⁹R¹⁰ oder 4- bis 8-gliedrigem Heterocycloalkyl-,
worin das 4- bis 8-gliedrige Heterocycloalkyl- seinerseits gegebenenfalls einfach substituiert sein kann mit: Oxo, C₁-C₃-Alkyl-, Fluor-C₁-C₃-Alkyl-, Cyclopropyl- oder Cyclopropylmethyl-,
oder
für C₃-C₆-Cycloalkyl- steht, das gegebenenfalls einfach substituiert sein kann mit Hydroxy, Fluor oder -NR⁹R¹⁰,
oder
für 4- bis 8-gliedriges Heterocycloalkyl-, das gegebenenfalls ein- oder zweifach, gleich oder verschieden substituiert sein kann mit: Oxo, C₁-C₃-Alkyl-, Fluor-C₁-C₃-Alkyl-, Cyclopropyl- oder Cyclopropylmethyl-,
- R⁸: für Wasserstoff, Methyl- oder Ethyl- steht,
oder
- R⁷ und R⁸: gemeinsam mit dem Stickstoffatom, an welches sie gebunden sind, für 4-bis 6-gliedriges Heterocycloalkyl- oder C₆-C₈-Heterospirocycloalkyl- stehen, die gegebenenfalls ein- oder zweifach, gleich oder verschieden substituiert sein können mit: Fluor, Oxo, C₁-C₃-Alkyl-, Fluor-C₁-C₃-Alkyl-, Cyclopropyl- oder Cyclopropylmethyl-,
- R⁹ und R¹⁰: unabhängig voneinander für Wasserstoff oder für gegebenenfalls einfach mit Hydroxy oder Oxo substituiertes C₁-C₃₋Alkyl-, Trifluormethyl-, oder für *N-*Methylpiperidinyl- stehen,
oder
- R⁹ und R¹⁰: gemeinsam mit dem Stickstoffatom, an das sie gebunden sind, für 4- bis 7-gliedriges Heterocycloalkyl- stehen, das gegebenenfalls ein- oder zweifach, gleich oder verschieden substituiert sein kann mit: Fluor, Oxo, C₁-C₃-Alkyl-, Fluor-C₁-C₃-Alkyl-, Cyclopropyl- oder Cyclopropylmethyl-,
sowie deren Diastereomere, Racemate, Polymorphe und physiologisch verträglichen Salze.

Ganz besonders bevorzugt sind solche Verbindungen der allgemeinen Formel (I),
in der
- A: für -NH- oder -N(Methyl)- steht,
- X: für -N- oder -CH- steht,
- n: für 0 oder 1 steht,
- R¹: für eine Gruppe -C(=O)NR⁷R⁸ oder -S(=O)₂NR⁷R⁸ steht,
oder
für Oxazolyl- oder Oxadiazolyl-, die gegebenenfalls ein- oder zweifach substituiert sein können mit Methyl-,
- R²: für Wasserstoff, Methyl- oder Methoxy- steht, oder
- R¹ und R²: gemeinsam für eine Gruppe *-S(=O)₂-NH-CH₂-** stehen, worin "*" den Anknüpfungspunkt von R¹ am in Formel (I) enthaltenen Phenylring bedeutet, und worin "**" ein diesem Anknüfungspunkt benachbartes Kohlenstoffatom dieses Phenylringes bedeutet,
- R³: für Methyl- steht,
- R⁴: für Methyl- steht,
- R⁵: für Wasserstoff steht,
- R⁶: für Isopropyl-, Isobutyl- oder 2-Methoxyethyl- steht,
oder
für Benzyl- steht, dessen Phenylteil gegebenenfalls ein- oder zweifach, gleich oder verschieden substituiert sein kann mit: Fluor, Methoxy-,
oder
für C₅-C₇-Cycloalkyl- steht, das gegebenenfalls ein- oder zweifach substituiert sein kann mit Methyl-,
oder
für Tetrahydrofuranyl-, Tetrahydropyranyl- oder Piperidinyl- steht,
worin Piperidinyl- gegebenenfalls einfach substitiert sein kann mit Methyl- oder *tert*-Butoxycarbonyl-,
oder
für Phenyl steht, dass gegebenenfalls einfach substituiert sein kann mit Fluor, Methyl- oder *N*-*tert*-Butoxycarbonylpiperazinyl-,
- R⁷: für Wasserstoff steht,
oder
für C₁-C₃-Alkyl- steht, das gegebenenfalls einfach substituiert sein kann mit -NR⁹R¹⁰ oder *N-*Methylpiperidinyl-,
oder
für Cyclopropyl- steht, oder für Cyclohexyl-,
worin Cyclohexyl- gegebenenfalls einfach substituiert sein kann mit Hydroxy- oder -NR⁹R¹⁰,
oder
für 4- bis 6-gliedriges Heterocycloalkyl- steht, das gegebenenfalls einfach substituiert sein kann mit Methyl-,
- R⁸: für Wasserstoff, Methyl- oder Ethyl- steht,
oder
- R⁷ und R⁸: gemeinsam mit dem Stickstoffatom, an das sie gebunden sind, für 4- bis 6-gliedriges Heterocycloalkyl- stehen, das gegebenenfalls ein- oder zweifach substituiert sein kann mit Fluor, oder das gegebenenfalls einfach substituiert sein kann mit Methyl-, Isopropyl-, 2,2,2-Trifluoroethyl- oder Cyclopropylmethyl-, oder für 6-Azaspiro[3.3]heptyl-oder für 2-Oxa-6-azaspiro[3.3]heptyl- stehen,
- R⁹ und R¹⁰: unabhängig voneinander für Wasserstoff, C₁-C₃-Alkyl- oder für *N-*Methylpiperidinyl- stehen,
oder
- R⁹ und R¹⁰: gemeinsam mit dem Stickstoffatom, an das sie gebunden sind, für 6-gliedriges Heterocycloalkyl- stehen, das gegebenenfalls ein- oder zweifach substituiert sein kann mit Fluor, oder das gegebenenfalls einfach substituiert sein kann mit Methyl-, 2,2,2-Trifluorethyl-, Cyclopropyl- oder Cyclopropylmethyl-,
sowie deren Diastereomere, Racemate, Polymorphe und physiologisch verträglichen Salze.

Überaus bevorzugt sind solche Verbindungen der allgemeinen Formel (I),
in der
- A: für -NH- oder -N(Methyl)- steht,
- X: für -N- oder -CH- steht,
- n: für 0 oder 1 steht,
- R¹: für eine Gruppe -C(=O)NR⁷R⁸ oder -S(=O)₂NR⁷R⁸ steht,
oder für 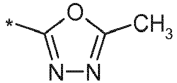 steht, worin "*" den Anknüpfungspunkt an den Rest des Moleküls bedeutet,
- R²: für Wasserstoff, Methyl- oder Methoxy- steht,
oder
- R¹ und R²: gemeinsam mit dem Phenylring, an den sie gebunden sind, für 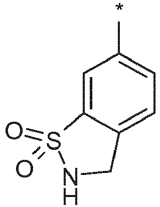 stehen, worin "*" den Anknüpfungspunkt an den Rest des Moleküls bedeutet,
- R³: für Methyl- steht,
- R⁴: für Methyl- steht,
- R⁵: für Wasserstoff steht,
- R⁶: für Isopropyl-, Isobutyl-, 2-Methoxyethyl-, Benzyl-, 4-Methoxybenzyl-, 2,6-Difluorbenzyl-, Cyclopentyl-, Cyclohexyl-, Cycloheptyl-, Tetrahydropyran-4-yl-, Phenyl-, 3-Methylphenyl- oder 4-Fluorphenyl- steht,
oder für 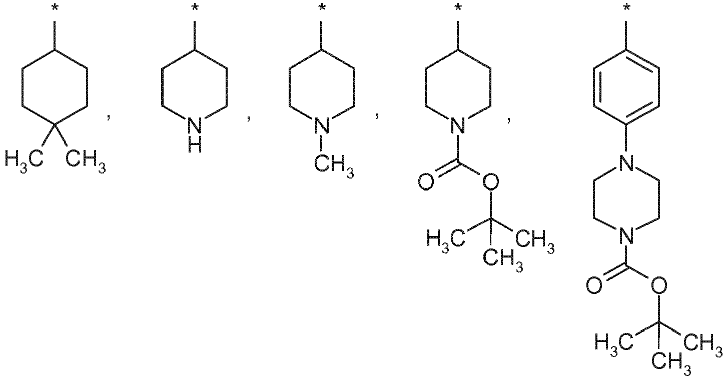 steht, worin "*" jeweils den Anknüpfungspunkt an den Rest des Moleküls bedeutet,
- R⁷: für Wasserstoff, Methyl-, Ethyl-, Isopropyl- oder Cyclopropyl- steht, oder für 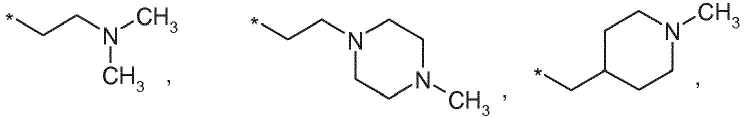 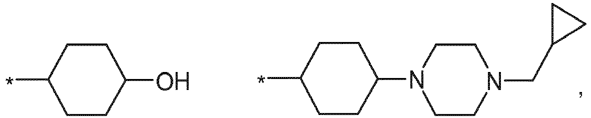 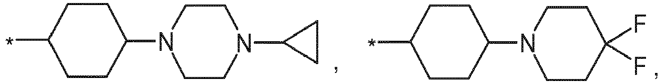 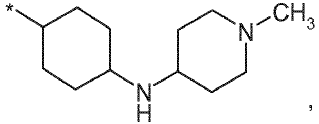 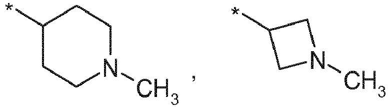 steht,
wobei "*" jeweils den Anknüpfungspunkt an den Rest des Moleküls bedeutet,
- R⁸: für Wasserstoff, Methyl- oder Ethyl- steht,
oder
- R⁷ und R⁸: gemeinsam mit dem Stickstoffatom, an welches sie gebunden sind, für 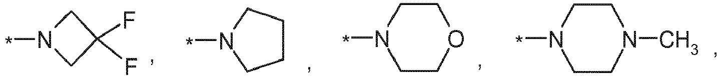 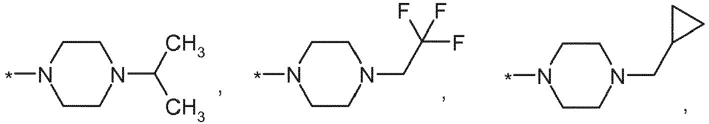 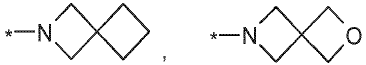
stehen,
wobei "*" jeweils den Anknüpfungspunkt an den Rest des Moleküls bedeutet, sowie deren Diastereomere, Racemate, Polymorphe und physiologisch verträglichen Salze.

Ebenfalls von Interesse sind solche Verbindungen der allgemeinen Formel I, in der
- A: für -NH- oder -O- steht,
- X: für -N- oder -CH- steht,
- n: für 0,1 oder 2 steht,
- R¹: für eine Gruppe -C(=O)NR⁷R⁸ oder -S(=O)₂NR⁷R⁸ steht,
oder
für 5-gliedriges monocyclisches Heteroaryl- steht, das gegebenenfalls ein-, zwei- oder dreifach, gleich oder verschieden substituiert sein kann mit Halogen, Cyano, C₁-C₄-Alkyl-, C₂-C₄-Alkenyl-, C₂-C₄-Alkinyl-, Halogen-C₁-C₄-alkyl-, C₁-C₄-Alkoxy-, Halogen-C₁-C₄-alkoxy-, C₁-C₄-Alkylthio-, Halogen-C₁-C₄-alkylthio-, -NR⁹R¹⁰, -C(=O)OR¹¹, -C(=O)N⁹R¹⁰, -C(=O)R¹¹, -S(=O)₂R¹¹, -S(=O)₂NR⁹R¹⁰,
- R²: für Wasserstoff, Halogen, Cyano, C₁-C₃-Alkyl-, C₂-C₄-Alkenyl-, C₂-C₄-Alkinyl-, Halogen-C₁-C₄-alkyl-, C₁-C₄-Alkoxy-, Halogen-C₁-C₄-alkoxy-, C₁-C₄-Alkylthio- oder Halogen-C₁-C₄-alkylthio- steht, und falls n für 2 steht, kann R² gleich oder verschieden sein,
- R³: für Methyl- oder Ethyl- steht,
- R⁴: für Wasserstoff oder C₁-C₃-Alkyl- steht,
- R⁵: für Wasserstoff oder C₁-C₃-Alkyl- steht,
oder
- R⁴ und R⁵: gemeinsam mit dem Kohlenstoffatom, an das sie gebunden sind, für C₃-C₆-Cycloalkylen stehen,
- R⁶: für C₁-C₆-Alkyl- steht, das gegebenenfalls einfach substituiert sein kann mit Phenyl-, C₃-C₈-Cycloalkyl-, oder 4-bis 8-gliedrigem Heterocycloalkyl-, worin Phenyl- seinerseits gegebenenfalls ein-, zwei- oder dreifach, gleich oder verschieden substituiert sein kann mit: Halogen, Cyano, C₁-C₄-Alkyl-, C₂-C₄-Alkenyl-, C₂-C₄-Alkinyl-, C₁-C₄-Alkoxy-, Halogen-C₁-C₄-Alkyl-, Halogen-C₁-C₄-Alkoxy-,
und
worin C₃-C₈-Cycloalkyl- und 4-bis 8-gliedriges Heterocycloalkyl- ihrerseits gegebenenfalls ein- oder zweifach, gleich oder verschieden substituiert sein können mit C₁-C₃-Alkyl-,
oder
für C₃-C₈-Cycloalkyl- oder 4-bis 8-gliedriges Heterocycloalkyl- steht, das gegebenenfalls ein- oder zweifach, gleich oder verschieden substituiert sein kann mit C₁-C₃-Alkyl-,
- R⁷: für C₁-C₆-Alkyl- steht, das gegebenenfalls ein-, zwei-, oder dreifach, gleich oder verschieden substituiert sein kann mit: Hydroxy, Oxo, Fluor, Cyano, C₁-C₄-Alkoxy-, Halogen-C₁-C₄-alkoxy-, -NR⁹R¹⁰, C₃-C₈-Cycloalkyl-, C₄-C₈-Cycloalkenyl-, 4- bis 8-gliedrigem Heterocycloalkyl-, 4- bis 8-gliedrigem Heterocycloalkenyl-, C₅-C₁₁-Spirocycloalkyl-, C₅-C₁₁-Heterospirocycloalkyl-, verbrücktem C₆-C₁₂-Cycloalkyl-, verbrücktem C₆-C₁₂-Heterocycloalkyl-, C₆-C₁₂-Bicycloalkyl-, C₆-C₁₂-Heterobicycloalkyl-, Phenyl-, 5- bis 6-gliedrigem Heteroaryl-,
und worin C₃-C₈-Cycloalkyl-, C₄-C₈-Cycloalkenyl-, 4- bis 8-gliedriges Heterocycloalkyl-, 4- bis 8-gliedriges Heterocycloalkenyl-, C₅-C₁₁-Spirocycloalkyl-, C₅-C₁₁-Heterospirocycloalkyl-, verbrücktes C₆-C₁₂-Cycloalkyl-, verbrücktes C₆-C₁₂-Heterocycloalkyl-, C₆-C₁₂-Bicycloalkyl-, C₆-C₁₂-Heterobicycloalkyl- gegebenenfalls ein- oder zweifach, gleich oder verschieden substituiert sein können mit: Hydroxy, Fluor, Oxo, Cyano, C₁-C₃-Alkyl-, Fluor-C₁-C₃-Alkyl-, C₃-C₆-Cycloalkyl-, Cyclopropylmethyl-, C₁-C₃-Alkylcarbonyl-, C₁-C₄-Alkoxycarbonyl- oder -NR⁹R¹⁰,
und
worin Phenyl- und 5- bis 6-gliedriges Heteroaryl- gegebenenfalls ein- oder zweifach, gleich oder verschieden substituiert sein können mit: Halogen, Cyano, Trifluormethyl-, C₁-C₃-Alkyl-, C₁-C₃-Alkoxy-,
oder für C₃-C₆-Alkenyl- oder C₃-C₆-Alkinyl- steht,
oder für C₃-C₈-Cycloalkyl-, C₄-C₈-Cycloalkenyl-, C₅-C₁₁-Spirocycloalkyl-, verbrücktes C₆-C₁₂-Cycloalkyl- oder C₆-C₁₂-Bicycloalkyl- steht, die gegebenenfalls ein- oder zweifach, gleich oder verschieden substituiert sein können mit: Hydroxy, Oxo, Cyano, Fluor, C₁-C₃-Alkyl, C₁-C₃-Alkoxy, Trifluormethyl, -NR⁹R¹⁰, oder für 4- bis 8-gliedriges Heterocycloalkyl-, 4- bis 8-gliedriges Heterocycloalkenyl-, C₅-C₁₁-Heterospirocycloalkyl-, verbrücktes C₆-C₁₂-Heterocycloalkyl- oder C₆-C₁₂-Heterobicycloalkyl- steht, die gegebenenfalls ein- oder zweifach, gleich oder verschieden substituiert sein können mit: Hydroxy, Fluor, Oxo, Cyano, C₁-C₃-Alkyl-, Fluor-C₁-C₃-Alkyl-, C₃-C₆-Cycloalkyl-, Cyclopropylmethyl-, C₁-C₃-Alkylcarbonyl-, C₁-C₄-Alkoxycarbonyl- oder -NR⁹R¹⁰,
- R⁸: für Wasserstoff oder für gegebenenfalls ein- oder zweifach, gleich oder verschieden mit Hydroxy, Oxo oder C₁-C₃-Alkoxy- substituiertes C₁-C₃-Alkyl-, oder für Fluor-C₁-C₃-Alkyl steht,
oder
- R⁷ und R⁸: gemeinsam mit dem Stickstoffatom, an welches sie gebunden sind, für 4-bis 8-gliedriges Heterocycloalkyl, 4-bis 8-gliedriges Heterocycloalkenyl-, C₅-C₁₁-Heterospirocycloalkyl-, verbrücktes C₆-C₁₂-Heterocycloalkyl- oder C₆-C₁₂-Heterobicycloalkyl- stehen, die gegebenenfalls ein- oder zweifach, gleich oder verschieden substituiert sein können mit: Hydroxy, Fluor, Oxo, Cyano, C₁-C₃-Alkyl-, Fluor-C₁-C₃-Alkyl-, C₃-C₆-Cycloalkyl-, Cyclopropylmethyl-, C₁-C₃-Alkylcarbonyl-, C₁-C₄-Alkoxycarbonyl- oder -NR⁹R¹⁰,
- R⁹ und R¹⁰: unabhängig voneinander für Wasserstoff oder für gegebenenfalls ein- oder zweifach, gleich oder verschieden mit Hydroxy, Oxo, C₁-C₃-Alkoxy- substituiertes C₁-C₃-Alkyl, oder für Fluor-C₁-C₃-Alkyl stehen,
oder
- R⁹ und R¹⁰: gemeinsam mit dem Stickstoffatom, an das sie gebunden sind, für 4-8-gliedriges Heterocycloalkyl- stehen, das gegebenenfalls ein- oder zweifach, gleich oder verschieden substituiert sein kann mit: Hydroxy, Fluor, Oxo, Cyano, C₁-C₃-Alkyl-, Fluor-C₁-C₃-Alkyl-, C₃-C₆-Cycloalkyl-, Cyclopropylmethyl-, C₁-C₃-Alkylcarbonyl- oder C₁-C₄-Alkoxycarbonyl- und
- R¹¹: für C₁-C₆-Alkyl- oder Phenyl-C₁-C₃-Alkyl- steht,
sowie deren Diastereomere, Racemate, Polymorphe und physiologisch verträglichen Salze, überraschenderweise die Interaktion zwischen BET-Proteinen, insbesondere BRD4, und einem acetylierten Histon 4-Peptid inhibieren und somit das Wachstum von Krebszellen hemmen.

Bevorzugt sind solche Verbindungen der allgemeinen Formel (I) von Interesse,
in der
- A: für -NH- steht,
- X: für -N- oder -CH- steht,
- n: für 0,1 oder 2 steht,
- R¹: für eine Gruppe -C(=O)NR⁷R⁸ oder -S(=O)₂NR⁷R⁸ steht,
oder
für Oxazolyl-, Thiazolyl-, Oxadiazolyl- oder Thiadiazolyl- steht, die gegebenenfalls ein- oder zweifach, gleich oder verschieden substituiert sein können mit Halogen, Cyano, C₁-C₃-Alkyl-, Trifluormethyl-, C₁-C₃-Alkoxy-, Trifluormethoxy- oder -NR⁹R¹⁰,
- R²: für Wasserstoff, Fluor, Chlor, Cyano, Methoxy- oder Ethoxy- steht, und falls n für 2 steht, kann R² gleich oder verschieden sein,
- R³: für Methyl- oder Ethyl- steht,
- R⁴: für Wasserstoff, Methyl- oder Ethyl- steht,
- R⁵: für Wasserstoff, Methyl- oder Ethyl- steht,
- R⁶: für unsubstituiertes C₂-C₅-Alkyl- steht,
oder
für Methyl- oder Ethyl- steht, das einfach substituiert ist mit Phenyl- oder 4-bis 8-gliedrigem Heterocycloalkyl-,
worin Phenyl- seinerseits gegebenenfalls ein-, oder zweifach, gleich oder verschieden substituiert sein kann mit: Fluor, Chlor, Brom, Cyano, C₁-C₃-Alkyl-, C₁-C₃-Alkoxy-, Trifluoromethyl-,
und
worin 4-bis 8-gliedriges Heterocycloalkyl- seinerseits gegebenenfalls ein- oder zweifach substituiert sein kann mit Methyl-, oder
für C₃-C₆-Cycloalkyl- oder 4-bis 8-gliedriges Heterocycloalkyl-, die gegebenenfalls ein- oder zweifach substituiert sein können mit Methyl-,
- R⁷: für C₁-C₆-Alkyl- steht, das gegebenenfalls ein-, zwei-, oder dreifach, gleich oder verschieden substituiert sein kann mit: Hydroxy, Oxo, Fluor, Cyano, C₁-C₃-Alkoxy-, Fluor-C₁-C₃-alkoxy-, -NR⁹R¹⁰, 4- bis 8-gliedrigem Heterocycloalkyl-, Phenyl-, 5- bis 6-gliedrigem Heteroaryl-,
und worin das 4- bis 8-gliedrige Heterocycloalkyl- gegebenenfalls einfach substituiert sein kann mit: Hydroxy, Oxo, C₁-C₃-Alkyl-, Fluor-C₁-C₃-Alkyl-, Cyclopropyl-, Cyclopropylmethyl-, Acetyl- oder *tert*-Butoxycarbonyl-,
oder für C₃-C₆-Cycloalkyl- steht, das gegebenenfalls ein- oder zweifach, gleich oder verschieden substituiert sein kann mit: Hydroxy, Oxo, Cyano, Fluor, -NR⁹R¹⁰, oder für 4- bis 8-gliedriges Heterocycloalkyl-, C₆-C₈-Heterospirocycloalkyl-, verbrücktes C₆-C₁₀-Heterocycloalkyl- oder C₆-C₁₀-Heterobicycloalkyl- steht, die gegebenenfalls ein- oder zweifach, gleich oder verschieden substituiert sein können mit: Hydroxy, Oxo, C₁-C₃-Alkyl-, Fluor-C₁-C₃-Alkyl-, Cyclopropyl-, Cyclopropylmethyl-, Acetyl- oder *tert*-Butoxycarbonyl-,
- R⁸: für Wasserstoff oder C₁-C₃-Alkyl steht,
oder
- R⁷ und R⁸: gemeinsam mit dem Stickstoffatom, an welches sie gebunden sind, für 4-bis 8-gliedriges Heterocycloalkyl-, C₆-C₈-Heterospirocycloalkyl-, verbrücktes C₆-C₁₀-Heterocycloalkyl- oder C₆-C₁₀-Heterobicycloalkyl- stehen,
die gegebenenfalls ein- oder zweifach, gleich oder verschieden substituiert sein können mit: Hydroxy, Oxo, C₁-C₃-Alkyl-, Fluor-C₁-C₃-Alkyl-, Cyclopropyl-, Cyclopropylmethyl-, Acetyl- oder *tert-*Butoxycarbonyl-,
- R⁹ und R¹⁰: unabhängig voneinander für Wasserstoff oder für gegebenenfalls einfach mit Hydroxy oder Oxo substituiertes C₁-C₃-Alkyl- oder für Trifluormethyl- stehen, oder
- R⁹ und R¹⁰: gemeinsam mit dem Stickstoffatom, an das sie gebunden sind, für 4-7-gliedriges Heterocycloalkyl- stehen, das gegebenenfalls ein- oder zweifach, gleich oder verschieden substituiert sein kann mit: Hydroxy, Oxo, C₁-C₃-Alkyl-, Fluor-C₁-C₃-Alkyl-, Cyclopropyl-, Cyclopropylmethyl-, Acetyl- oder *tert*-Butoxycarbonyl-,
sowie deren Diastereomere, Racemate, Polymorphe und physiologisch verträglichen Salze.

Von besonders bevorzugtem Interesse sind auch solche Verbindungen der allgemeinen Formel (I), in der
- A: für -NH- steht,
- X: für -N- oder -CH- steht,
- n: für 0 oder 1 steht,
- R¹: für eine Gruppe -C(=O)NR⁷R⁸ oder -S(=O)₂NR⁷R⁸ steht,
- R²: für Fluor, Chlor oder Methoxy- steht,
- R³: für Methyl- steht,
- R⁴: für Methyl- oder Ethyl- steht,
- R⁵: für Wasserstoff steht,
- R⁶: für unsubstituiertes C₃-C₅₋Alkyl- steht,
oder
für Methyl- steht, das einfach substituiert ist mit Phenyl- oder 4-bis 6-gliedrigem Heterocycloalkyl-,
worin Phenyl- seinerseits gegebenenfalls ein-, oder zweifach, gleich oder verschieden substituiert sein kann mit: Fluor, Chlor, Cyano, Methyl-, Methoxy-, und
worin 4-bis 6-gliedriges Heterocycloalkyl- seinerseits gegebenenfalls einfach substituiert sein kann mit Methyl-,
oder
für C₃-C₆-Cycloalkyl- steht, oder für 4-bis 6-gliedriges Heterocycloalkyl-,
- R⁷: für C₁-C₄-Alkyl- steht, das gegebenenfalls einfach substituiert sein kann mit-NR⁹R¹⁰ oder 4- bis 8-gliedrigem Heterocycloalkyl-,
worin das 4- bis 8-gliedrige Heterocycloalkyl- gegebenenfalls einfach substituiert sein kann mit: Oxo, C₁-C₃-Alkyl-, Fluor-C₁-C₃-Alkyl-, Cyclopropyl- oder Cyclopropylmethyl-,
oder für C₃-C₆-Cycloalkyl- steht, das gegebenenfalls einfach substituiert sein kann mit Hydroxy, Fluor oder -NR⁹R¹⁰,
oder für 4- bis 8-gliedriges Heterocycloalkyl-, das gegebenenfalls ein- oder zweifach, gleich oder verschieden substituiert sein kann mit: Oxo, C₁-C₃-Alkyl-, Fluor-C₁-C₃-Alkyl-, Cyclopropyl- oder Cyclopropylmethyl-,
- R⁸: für Wasserstoff oder Methyl- steht,
oder
- R⁷ und R⁸: gemeinsam mit dem Stickstoffatom, an welches sie gebunden sind, für 5-bis 6-gliedriges Heterocycloalkyl- oder C₆-C₈-Heterospirocycloalkyl- stehen, die gegebenenfalls ein- oder zweifach, gleich oder verschieden substituiert sein können mit: Oxo, C₁-C₃-Alkyl-, Fluor-C₁-C₃-Alkyl-, Cyclopropyl- oder Cyclopropylmethyl-,
- R⁹ und R¹⁰: unabhängig voneinander für Wasserstoff oder für gegebenenfalls einfach mit Hydroxy oder Oxo substituiertes C₁-C₃-Alkyl- oder für Trifluormethyl- stehen,
oder
- R⁹ und R¹⁰: gemeinsam mit dem Stickstoffatom, an das sie gebunden sind, für 4-7-gliedriges Heterocycloalkyl- stehen, das gegebenenfalls ein- oder zweifach, gleich oder verschieden substituiert sein kann mit: Oxo, C₁-C₃-Alkyl-, Fluor-C₁-C₃-Alkyl-, Cyclopropyl- oder Cyclopropylmethyl-,
sowie deren Diastereomere, Racemate, Polymorphe und physiologisch verträglichen Salze.

Von besonders bevorzugtem Interesse sind weiterhin auch solche Verbindungen der allgemeinen Formel (I),
in der
- A: für -NH- steht,
- X: für -N- steht,
- n: für 0 oder 1 steht,
- R¹: für eine Gruppe -C(=O)NR⁷R⁸ oder -S(=O)₂NR⁷R⁸ steht,
- R²: für Fluor, Chlor oder Methoxy- steht,
- R³: für Methyl- steht,
- R⁴: für Methyl- oder Ethyl- steht,
- R⁵: für Wasserstoff steht,
- R⁶: für unsubstituiertes C₃-C₅-Alkyl- steht,
oder
für Methyl- steht, das einfach substituiert ist mit Phenyl- oder 4-bis 6-gliedrigem Heterocycloalkyl-,
worin Phenyl- seinerseits gegebenenfalls ein-, oder zweifach, gleich oder verschieden substituiert sein kann mit: Fluor, Chlor, Cyano, Methyl-, Methoxy-, und
worin 4-bis 6-gliedriges Heterocycloalkyl- seinerseits gegebenenfalls einfach substituiert sein kann mit Methyl-,
oder
für C₃-C₆-Cycloalkyl- steht, oder 4-bis 6-gliedriges Heterocycloalkyl-,
- R⁷: für C₁-C₄-Alkyl- steht, das gegebenenfalls einfach substituiert sein kann mit-NR⁹R¹⁰ oder 4- bis 8-gliedrigem Heterocycloalkyl-,
worin das 4- bis 8-gliedrige Heterocycloalkyl- gegebenenfalls einfach substituiert sein kann mit: Oxo, C₁-C₃-Alkyl-, Fluor-C₁-C₃-Alkyl-, Cyclopropyl- oder Cyclopropylmethyl-,
oder für C₃-C₆-Cycloalkyl- steht, das gegebenenfalls einfach substituiert sein kann mit Hydroxy, Fluor oder -NR⁹R¹⁰,
oder für 4- bis 8-gliedriges Heterocycloalkyl-, das gegebenenfalls ein- oder zweifach, gleich oder verschieden substituiert sein kann mit: Oxo, C₁-C₃₋Alkyl-, Fluor-C₁-C₃-Alkyl-, Cyclopropyl- oder Cyclopropylmethyl-,
- R⁸: für Wasserstoff oder Methyl- steht,
oder
- R⁷ und R⁸: gemeinsam mit dem Stickstoffatom, an welches sie gebunden sind, für 5-bis 6-gliedriges Heterocycloalkyl- oder C₆-C₈-Heterospirocycloalkyl- stehen, die gegebenenfalls ein- oder zweifach, gleich oder verschieden substituiert sein können mit: Oxo, C₁-C₃-Alkyl-, Fluor-C₁-C₃-Alkyl-, Cyclopropyl- oder Cyclopropylmethyl-,
- R⁹ und R¹⁰: unabhängig voneinander für Wasserstoff oder für gegebenenfalls einfach mit Hydroxy oder Oxo substituiertes C₁-C₃-Alkyl- oder für Trifluormethyl- stehen,
oder
- R⁹ und R¹⁰: gemeinsam mit dem Stickstoffatom, an das sie gebunden sind, für 4-7-gliedriges Heterocycloalkyl- stehen, das gegebenenfalls ein- oder zweifach, gleich oder verschieden substituiert sein kann mit: Oxo, C₁-C₃-Alkyl-, Fluor-C₁-C₃-Alkyl-, Cyclopropyl- oder Cyclopropylmethyl-,
sowie deren Diastereomere, Racemate, Polymorphe und physiologisch verträglichen Salze.

Von besonders bevorzugtem Interesse sind weiterhin auch solche Verbindungen der allgemeinen Formel (I),
in der
- A: für -NH- steht,
- X: für -CH- steht,
- n: für 0 oder 1 steht,
- R¹: für eine Gruppe -C(=O)NR⁷R⁸ oder -S(=O)₂NR⁷R⁸ steht,
- R²: für Fluor, Chlor oder Methoxy- steht,
- R³: für Methyl- steht,
- R⁴: für Methyl- oder Ethyl- steht,
- R⁵: für Wasserstoff steht,
- R⁶: für unsubstituiertes C₃-C₅-Alkyl- steht,
oder
für Methyl- steht, das einfach substituiert ist mit Phenyl- oder 4-bis 6-gliedrigem Heterocycloalkyl-,
worin Phenyl- seinerseits gegebenenfalls ein-, oder zweifach, gleich oder verschieden substituiert sein kann mit: Fluor, Chlor, Cyano, Methyl-, Methoxy-, und
worin 4-bis 6-gliedriges Heterocycloalkyl- seinerseits gegebenenfalls einfach substituiert sein kann mit Methyl-,
oder
für C₃-C₆-Cycloalkyl- steht, oder 4-bis 6-gliedriges Heterocycloalkyl-,
- R⁷: für C₁-C₄-Alkyl- steht, das gegebenenfalls einfach substituiert sein kann mit-NR⁹R¹⁰ oder 4- bis 8-gliedrigem Heterocycloalkyl-,
worin das 4- bis 8-gliedrige Heterocycloalkyl- gegebenenfalls einfach substituiert sein kann mit: Oxo, C₁-C₃-Alkyl-, Fluor-C₁-C₃-Alkyl-, Cyclopropyl- oder Cyclopropylmethyl-,
oder für C₃-C₆-Cycloalkyl- steht, das gegebenenfalls einfach substituiert sein kann mit Hydroxy, Fluor oder -NR⁹R¹⁰,
oder für 4- bis 8-gliedriges Heterocycloalkyl-, das gegebenenfalls ein- oder zweifach, gleich oder verschieden substituiert sein kann mit: Oxo, C₁-C₃-Alkyl-, Fluor-C₁-C₃-Alkyl-, Cyclopropyl- oder Cyclopropylmethyl-,
- R⁸: für Wasserstoff oder Methyl- steht,
oder
- R⁷ und R⁸: gemeinsam mit dem Stickstoffatom, an welches sie gebunden sind, für 5-bis 6-gliedriges Heterocycloalkyl- oder C₆-C₈-Heterospirocycloalkyl- stehen, die gegebenenfalls ein- oder zweifach, gleich oder verschieden substituiert sein können mit: Oxo, C₁-C₃-Alkyl, Fluor-C₁-C₃-Alkyl-, Cyclopropyl- oder Cyclopropylmethyl-,
- R⁹ und R¹⁰: unabhängig voneinander für Wasserstoff oder für gegebenenfalls einfach mit Hydroxy oder Oxo substituiertes C₁-C₃-Alkyl- oder für Trifluormethyl- stehen,
oder
- R⁹ und R¹⁰: gemeinsam mit dem Stickstoffatom, an das sie gebunden sind, für 4-7-gliedriges Heterocycloalkyl- stehen, das gegebenenfalls ein- oder zweifach, gleich oder verschieden substituiert sein kann mit: Oxo, C₁-C₃-Alkyl-, Fluor-C₁-C₃-Alklyl-, Cyclopropyl- oder Cyclopropylmethyl-,
sowie deren Diastereomere, Racemate, Polymorphe und physiologisch verträglichen Salze.

Von ganz besonders bevorzugtem Interesse sind solche Verbindungen der allgemeinen Formel (I), in der
- A: für -NH- steht,
- X: für -N- oder -CH- steht,
- n: für 0 oder 1 steht,
- R¹: für eine Gruppe -C(=O)NR⁷R⁸ oder -S(=O)₂NR⁷R⁸ steht,
- R²: für Methoxy- steht,
- R³: für Methyl- steht,
- R⁴: für Methyl- steht,
- R⁵: für Wasserstoff steht,
- R⁶: für Isopropyl- steht,
oder
für Benzyl steht, dessen Phenylteil gegebenenfalls ein- oder zweifach, gleich oder verschieden substituiert sein kann mit: Fluor, Chlor, Methoxy-,
oder
für Cyclopentyl- oder Cyclohexyl- steht,
oder
für Tetrahydrofuranyl- oder Tetrahydropyranyl- steht,
- R⁷: für C₁-C₃-Alkyl- steht, das gegebenenfalls einfach substituiert sein kann mit -NR⁹R¹⁰,
oder für C₅-C₆-Cycloalkyl- steht, das gegebenenfalls einfach substituiert sein kann mit -NR⁹R¹⁰, o
der für 4- bis 6-gliedriges Heterocycloalkyl-, das gegebenenfalls einfach substituiert sein kann mit Methyl-,
- R⁸: für Wasserstoff oder Methyl- steht,
oder
- R⁷ und R⁸: gemeinsam mit dem Stickstoffatom, an das sie gebunden sind, für 6-gliedriges Heterocycloalkyl- stehen, das gegebenenfalls einfach substituiert sein kann mit Methyl-,
- R⁹ und R¹⁰: unabhängig voneinander für Wasserstoff oder für C₁-C₃-Alkyl- stehen,
oder
- R⁹ und R¹⁰: gemeinsam mit dem Stickstoffatom, an das sie gebunden sind, für 6-gliedriges Heterocycloalkyl- stehen, das gegebenenfalls einfach substituiert sein kann mit Methyl-, 2,2,2-Trifluorethyl- oder Cyclopropylmethyl-,
sowie deren Diastereomere, Racemate, Polymorphe und physiologisch verträglichen Salze.

Von überaus bevorzugtem Interessse sind auch solche Verbindungen der allgemeinen Formel (I), in der
- A: für -NH- steht,
- X: für -N- oder -CH- steht,
- n: für 0 oder 1 steht,
- R¹: für eine Gruppe -C(=O)NR⁷R⁸ oder -S(=O)₂NR⁷R⁸ steht,
- R²: für Methoxy- steht,
- R³: für Methyl- steht,
- R⁴: für Methyl- steht,
- R⁵: für Wasserstoff steht,
- R⁶: für 4-Methoxybenzyl-, 2,6-Difluorbenzyl-, Cyclopentyl- oder Tetrahydropyran-4-yl- steht,
- R⁷: für 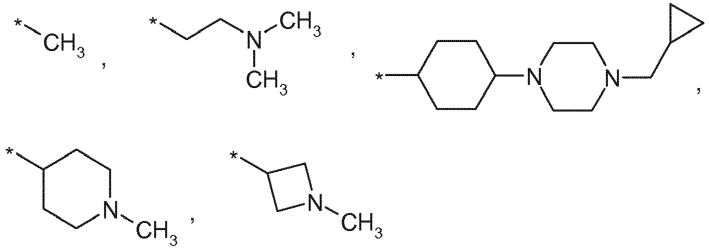 steht,
wobei "*" jeweils den Anknüpfungspunkt an den Rest des Moleküls bedeutet, und
- R⁸: für Wasserstoff oder Methyl- steht,
oder
- R⁷ und R⁸: gemeinsam mit dem Stickstoffatom, an welches sie gebunden sind, für 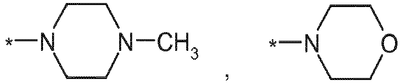
stehen,
wobei "*" jeweils den Anknüpfungspunkt an den Rest des Moleküls bedeutet, sowie deren Diastereomere, Racemate, Polymorphe und physiologisch verträglichen Salze.

Bevorzugt sind Verbindungen der allgemeinen Formel (I), in denen A für -NH- steht.

Bevorzugt sind Verbindungen der allgemeinen Formel (I), in denen A für -O- steht.

Bevorzugt sind Verbindungen der allgemeinen Formel (I), in denen A für -NH- oder für -N(C₁-C₃-Alkyl)- steht.

Bevorzugt sind Verbindungen der allgemeinen Formel (I), in denen A für -N(C₁-C₃-Alkyl)- steht.

Besonders bevorzugt sind Verbindungen der allgemeinen Formel (I), in denen A für -NH- oder für -N(Methyl)- steht.

Besonders bevorzugt sind Verbindungen der allgemeinen Formel (I), in denen A für -N(Methyl)-steht.

Bevorzugt sind Verbindungen der allgemeinen Formel (I), in denen X für -N- steht.

Bevorzugt sind Verbindungen der allgemeinen Formel (I), in denen X für -CH- steht.

Bevorzugt sind Verbindungen der allgemeinen Formel (I), in denen n für die Zahl 0 oder die Zahl 1 steht.

Bevorzugt sind Verbindungen der allgemeinen Formel (I), in denen n für die Zahl 0 steht.

Bevorzugt sind Verbindungen der allgemeinen Formel (I), in denen n für die Zahl 1 steht.

Bevorzugt sind Verbindungen der allgemeinen Formel (I), in denen R¹ für -C(=O)NR⁷R⁸ steht.

Bevorzugt sind Verbindungen der allgemeinen Formel (I), in denen R¹ für -S(=O)₂NR⁷R⁸ steht.

Bevorzugt sind Verbindungen der allgemeinen Formel (I), in denen R¹ für 5-gliedriges monocyclisches Hetaryl- steht, das gegebenenfalls ein-, zwei- oder dreifach, gleich oder verschieden substituiert sein kann mit Halogen, Cyano, C₁-C₄-Alkyl-, C₂-C₄-Alkenyl-, C₂-C₄-Alkinyl-, Halogen-C₁-C₄-alkyl-, C₁-C₄-Alkoxy-, Halogen-C₁-C₄-alkoxy-, C₁-C₄-Alkylthio-, Halogen-C₁-C₄-alkylthio-, -NR⁹R¹⁰), -C(=O)OR¹¹, -C(=O)N⁹R¹⁰, -C(=O)R¹¹, -S(=O)₂R¹¹,-S(=O)₂NR⁹R¹⁰.

Bevorzugt sind Verbindungen der allgemeinen Formel (I), in denen R¹ für Oxazolyl-, Thiazolyl-, Oxadiazolyl- oder Thiadiazolyl- steht, die gegebenenfalls ein- oder zweifach, gleich oder verschieden substituiert sein können mit Halogen, Cyano, C₁-C₃-Alkyl-, Trifluormethyl-, C₁-C₃-Alkoxy-, Trifluormethoxy- oder -NR⁹R¹⁰.

Bevorzugt sind Verbindungen der allgemeinen Formel (I), in denen R¹ für eine Gruppe -C(=O)NR⁷R⁸ oder -S(=O)₂NR⁷R⁸ steht, oder
für Oxazolyl-, Thiazolyl-, Oxadiazolyl- oder Thiadiazolyl- steht, die gegebenenfalls ein- oder zweifach, gleich oder verschieden substituiert sein können mit Halogen, Cyano, C₁-C₃-Alkyl-, Trifluormethyl-, C₁-C₃-Alkoxy-, Trifluormethoxy- oder -NR⁹R¹⁰.

Bevorzugt sind Verbindungen der allgemeinen Formel (I), in denen R¹ für Oxazolyl-, Thiazolyl-, Oxadiazolyl- oder Thiadiazolyl- steht, die gegebenenfalls ein- oder zweifach, gleich oder verschieden substituiert sein können mit Halogen, Cyano, C₁-C₃-Alkyl-, Trifluormethyl-, C₁-C₃-Alkoxy-, Trifluormethoxy- oder -NR⁹R¹⁰.

Besonders bevorzugt sind Verbindungen der allgemeinen Formel (I), in denen R¹ für eine Gruppe -C(=O)NR⁷R⁸ oder -S(=O)₂NR⁷R⁸ steht, oder
für Oxazolyl- oder Oxadiazolyl-, die gegebenenfalls ein- oder zweifach, gleich oder verschieden substituiert sein können mit C₁-C₃-Alkyl-.

Besonders bevorzugt sind Verbindungen der allgemeinen Formel (I), in denen R¹ für
Oxazolyl- oder Oxadiazolyl- steht, die gegebenenfalls ein- oder zweifach, gleich oder verschieden substituiert sein können mit C₁-C₃-Alkyl-.

Ganz besonders bevorzugt sind Verbindungen der allgemeinen Formel (I), in denen R¹ für eine Gruppe -C(=O)NR⁷R⁸ oder -S(=O)₂NR⁷R⁸ steht, oder
für Oxazolyl- oder Oxadiazolyl-, die gegebenenfalls ein- oder zweifach substituiert sein können mit Methyl-.

Ganz besonders bevorzugt sind Verbindungen der allgemeinen Formel (I), in denen R¹ für Oxazolyl- oder Oxadiazolyl- steht, die gegebenenfalls ein- oder zweifach substituiert sein können mit Methyl-.

Überaus bevorzugt sind Verbindungen der allgemeinen Formel (I), in denen R¹ für eine Gruppe -C(=O)NR⁷R⁸ oder -S(=O)₂NR⁷R⁸ steht, oder für 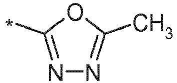 steht, worin "*" den Anknüpfungspunkt an den Rest des Moleküls bedeutet.

Überaus bevorzugt sind Verbindungen der allgemeinen Formel (I), in denen R¹ für 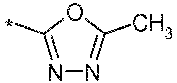 steht, worin "*" den Anknüpfungspunkt an den Rest des Moleküls bedeutet.

Bevorzugt sind Verbindungen der allgemeinen Formel (I), in denen R² für Wasserstoff, Fluor, Chlor, Cyano, Methyl-, Methoxy-, Ethyl- oder Ethoxy- steht.

Bevorzugt sind Verbindungen der allgemeinen Formel (I), in denen R² für C₁-C₃-Alkoxy- steht.

Bevorzugt sind Verbindungen der allgemeinen Formel (I), in denen R² für Ethoxy- steht.

Bevorzugt sind Verbindungen der allgemeinen Formel (I), in denen R² für Fluor steht.

Bevorzugt sind Verbindungen der allgemeinen Formel (I), in denen R² für Chlor steht.

Besonders bevorzugt sind Verbindungen der allgemeinen Formel (I), in denen R² für Wasserstoff, Fluor, Chlor, Methyl- oder Methoxy- steht.

Besonders bevorzugt sind Verbindungen der allgemeinen Formel (I), in denen R² für Wasserstoff, Methyl- oder Methoxy- steht.

Besonders bevorzugt sind Verbindungen der allgemeinen Formel (I), in denen R² für Methoxysteht.

Besonders bevorzugt sind Verbindungen der allgemeinen Formel (I), in denen R² für Methyl- steht.

Besonders bevorzugt sind Verbindungen der allgemeinen Formel (I), in denen R² für Wasserstoff steht.

Bevorzugt sind Verbindungen der allgemeinen Formel (I), in denen R¹ und R² gemeinsam für eine Gruppe *-S(=O)₂-NR⁸-CH₂-** oder *-C(=O)-NR⁸-CH₂-** stehen, worin "*" den Anknüpfungspunkt von R¹ am in Formel (I) enthaltenen Phenylring bedeutet, und worin "**" ein diesem Anknüfungspunkt benachbartes Kohlenstoffatom dieses Phenylringes bedeutet.

Besonders bevorzugt sind Verbindungen der allgemeinen Formel (I), in denen R¹ und R² gemeinsam für eine Gruppe *-S(=O)₂-NR⁸-CH₂-** stehen, worin "*" den Anknüpfungspunkt von R¹ am in Formel (I) enthaltenen Phenylring bedeutet, und worin "**" ein diesem Anknüfungspunkt benachbartes Kohlenstoffatom dieses Phenylringes bedeutet.

Ganz besonders bevorzugt sind Verbindungen der allgemeinen Formel (I), in denen R¹ und R² gemeinsam für eine Gruppe *-S(=O)₂-NH-CH₂-** stehen, worin "*" den Anknüpfungspunkt von R¹ am in Formel (I) enthaltenen Phenylring bedeutet, und worin "**" ein diesem Anknüfungspunkt benachbartes Kohlenstoffatom dieses Phenylringes bedeutet.

Besonders bevorzugt sind Verbindungen der allgemeinen Formel (I), in denen R¹ für eine Gruppe -C(=O)NR⁷R⁸ oder -S(=O)₂NR⁷R⁸ steht,
oder
für Oxazolyl- oder Oxadiazolyl-, die gegebenenfalls ein- oder zweifach, gleich oder verschieden substituiert sein können mit C₁-C₃-Alkyl-,
und in denen R² für Wasserstoff, Fluor, Chlor, Methyl- oder Methoxy- steht,
oder in denen R¹ und R² gemeinsam für eine Gruppe *-S(=O)₂-NR⁸-CH₂-** stehen, worin "*" den Anknüpfungspunkt von R¹ am in Formel (I) enthaltenen Phenylring bedeutet, und worin "**" ein diesem Anknüfungspunkt benachbartes Kohlenstoffatom dieses Phenylringes bedeutet.

Ganz besonders bevorzugt sind Verbindungen der allgemeinen Formel (I), in denen R¹ für eine Gruppe -C(=O)NR⁷R⁸ oder -S(=O)₂NR⁷R⁸ steht,
oder
für Oxazolyl- oder Oxadiazolyl-, die gegebenenfalls ein- oder zweifach, gleich oder verschieden substituiert sein können mit Methyl-,
und in denen R² für Wasserstoff, Methyl- oder Methoxy- steht,
oder in denen R¹ und R² gemeinsam für eine Gruppe *-S(=O)₂-NH-CH₂-** stehen, worin "*" den Anknüpfungspunkt von R¹ am in Formel (I) enthaltenen Phenylring bedeutet, und worin "**" ein diesem Anknüpfungspunkt benachbartes Kohlenstoffatom dieses Phenylringes bedeutet.

Überaus bevorzugt sind Verbindungen der allgemeinen Formel (I), in denen R¹ für eine Gruppe -C(=O)NR⁷R⁸ oder -S(=O)₂NR⁷R⁸ steht,
oder
oder für 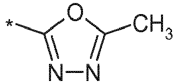 steht, worin "*" den Anknüpfungspunkt an den Rest des Moleküls bedeutet,
und in denen R² für Wasserstoff, Methyl- oder Methoxy- steht,
oder in denen R¹ und R² gemeinsam mit dem Phenylring, an den sie gebunden sind, für 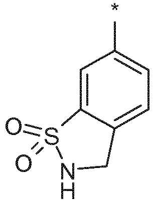 stehen, worin "*" den Anknüpfungspunkt an den Rest des Moleküls bedeutet.

Bevorzugt sind Verbindungen der allgemeinen Formel (I), in denen R³ für Methyl- oder Ethylsteht.

Bevorzugt sind Verbindungen der allgemeinen Formel (I), in denen R³ für Ethyl- steht.

Besonders bevorzugt sind Verbindungen der allgemeinen Formel (I), in denen R³ für Methyl- steht.

Bevorzugt sind Verbindungen der allgemeinen Formel (I), in denen R⁴ für Wasserstoff, Methyl- oder Ethyl- steht.

Bevorzugt sind Verbindungen der allgemeinen Formel (I), in denen R⁴ für Methyl- oder Ethylsteht.

Bevorzugt sind Verbindungen der allgemeinen Formel (I), in denen R⁴ für Ethyl- steht.

Besonders bevorzugt sind Verbindungen der allgemeinen Formel (I), in denen R⁴ für Methyl- steht.

Bevorzugt sind Verbindungen der allgemeinen Formel (I), in denen R⁴ für Ethyl- und R⁵ für Wasserstoff steht.

Bevorzugt sind Verbindungen der allgemeinen Formel (I), in denen je ein Substituent aus R⁴ und R⁵ für Methyl- und einer für Wasserstoff steht, so dass bezüglich des aus R⁴, R⁵ und dem an R⁴ und R⁵ gebundenen Kohlenstoffatom gebildeten Stereozentrums ein Racemat resultiert.

Besonders bevorzugt sind Verbindungen der allgemeinen Formel (I), in denen je ein Substituent aus R⁴ und R⁵ für Methyl- und einer für Wasserstoff steht, so dass bezüglich des aus R⁴, R⁵ und dem an R⁴ und R⁵ gebundenen Kohlenstoffatom gebildeten Stereozentrums ein Isomerengemisch resultiert, in welchem die (*R*)-Form überwiegt.

Besonders bevorzugt sind Verbindungen der allgemeinen Formel (I), in denen R⁴ für Methyl- und R⁵ für Wasserstoff steht.

Bevorzugt sind Verbindungen der allgemeinen Formel (I), in denen R⁶ für unsubstituiertes C₃-C₅-Alkyl- steht,
oder
für Methyl- steht, das einfach substituiert ist mit Phenyl- oder 4- bis 6-gliedrigem Heterocycloalkyl-,
worin Phenyl- seinerseits gegebenenfalls ein-, oder zweifach, gleich oder verschieden substituiert sein kann mit: Fluor, Chlor, Cyano, Methyl-, Methoxy-,
und
worin 4-bis 6-gliedriges Heterocycloalkyl- seinerseits gegebenenfalls einfach substituiert sein kann mit Methyl-,
oder
für C₃-C₆-Cycloalkyl- steht, oder für 4-bis 6-gliedriges Heterocycloalkyl-.

Bevorzugt sind Verbindungen der allgemeinen Formel (I), in denen R⁶ für unsubstituiertes C₃-C₅-Alkyl steht.

Bevorzugt sind Verbindungen der allgemeinen Formel (I), in denen R⁶ für Methyl- steht, das einfach substituiert ist mit Phenyl-,
worin Phenyl- seinerseits gegebenenfalls ein-, oder zweifach, gleich oder verschieden substituiert sein kann mit: Fluor, Chlor, Cyano, Methyl-, Methoxy-.

Bevorzugt sind Verbindungen der allgemeinen Formel (I), in denen R⁶ für Methyl- steht, das einfach substituiert ist mit 4-bis 6-gliedrigem Heterocycloalkyl-,
worin 4-bis 6-gliedriges Heterocycloalkyl- seinerseits gegebenenfalls einfach substituiert sein kann mit Methyl-.

Bevorzugt sind Verbindungen der allgemeinen Formel (I), in denen R⁶ für C₃-C₆-Cycloalkyl- steht.

Bevorzugt sind Verbindungen der allgemeinen Formel (I), in denen R⁶ für 4-bis 6-gliedriges Heterocycloalkyl- steht.

Bevorzugt sind ferner Verbindungen der allgemeinen Formel (I), in denen R⁶ für C₂-C₅-Alkyl-steht, oder
für Methyl- oder Ethyl- steht, das einfach substituiert ist mit C₁-C₃-Alkoxy-, Phenyl- oder 4-bis 8-gliedrigem Heterocycloalkyl-,
worin Phenyl- seinerseits gegebenenfalls ein-, zwei- oder dreifach, gleich oder verschieden substituiert sein kann mit: Fluor, Chlor, Brom, Cyano, C₁-C₃-Alkyl-, C₁-C₃-Alkoxy-, und worin das 4-bis 8-gliedrige Heterocycloalkyl- seinerseits gegebenenfalls ein- oder zweifach, gleich oder verschieden substituiert sein kann mit Methyl-,
oder
für C₃-C₈-Cycloalkyl- oder 4-bis 8-gliedriges Heterocycloalkyl- steht, die gegebenenfalls ein- oder zweifach, gleich oder verschieden substituiert sein können mit C₁-C₃-Alkyl- oder C₁-C₄-Alkoxycarbonyl-,
oder
für Phenyl steht, das gegebenenfalls ein- oder zweifach, gleich oder verschieden substituiert sein kann mit Fluor, Chlor, Methyl- oder 6-gliedrigem Heterocycloalkyl-,
worin das 6-gliedrige Heterocycloalkyl- seinerseits gegebenenfalls einfach substituiert sein kann mit Methyl- oder *tert*-Butoxycarbonyl-,

Bevorzugt sind Verbindungen der allgemeinen Formel (I), in denen R⁶ für C₂-C₅-Alkyl- steht, oder für Methyl- oder Ethyl- steht, das einfach substituiert ist mit C₁-C₃-Alkoxy-, Phenyl- oder 4-bis 8-gliedrigem Heterocycloalkyl-,
worin Phenyl- seinerseits gegebenenfalls ein-, zwei- oder dreifach, gleich oder verschieden substituiert sein kann mit: Fluor, Chlor, Brom, Cyano, C₁-C₃-Alkyl-, C₁-C₃-Alkoxy-, und worin das 4-bis 8-gliedrige Heterocycloalkyl- seinerseits gegebenenfalls ein- oder zweifach, gleich oder verschieden substituiert sein kann mit Methyl-.

Bevorzugt sind Verbindungen der allgemeinen Formel (I), in denen R⁶ für C₂-C₅-Alkyl- steht.

Bevorzugt sind Verbindungen der allgemeinen Formel (I), in denen R⁶ für Methyl- oder Ethylsteht, das einfach substituiert ist mit C₁-C₃-Alkoxy-, Phenyl- oder 4-bis 8-gliedrigem Heterocycloalkyl-,
worin Phenyl- seinerseits gegebenenfalls ein-, zwei- oder dreifach, gleich oder verschieden substituiert sein kann mit: Fluor, Chlor, Brom, Cyano, C₁-C₃-Alkyl-, C₁-C₃-Alkoxy-, und worin das 4-bis 8-gliedrige Heterocycloalkyl- seinerseits gegebenenfalls ein- oder zweifach, gleich oder verschieden substituiert sein kann mit Methyl-.

Bevorzugt sind Verbindungen der allgemeinen Formel (I), in denen R⁶ für C₃-C₈-Cycloalkyl- oder 4-bis 8-gliedriges Heterocycloalkyl- steht, die gegebenenfalls ein- oder zweifach, gleich oder verschieden substituiert sein können mit C₁-C₃-Alkyl- oder C₁-C₄-Alkoxycarbonyl-.

Bevorzugt sind Verbindungen der allgemeinen Formel (I), in denen R⁶ für Phenyl steht, das gegebenenfalls ein- oder zweifach, gleich oder verschieden substituiert sein kann mit Fluor, Chlor, Methyl- oder 6-gliedrigem Heterocycloalkyl-,
worin das 6-gliedrige Heterocycloalkyl- seinerseits gegebenenfalls einfach substituiert sein kann mit Methyl- oder *tert*-Butoxycarbonyl-.

Besonders bevorzugt sind Verbindungen der allgemeinen Formel (I), in denen R⁶ für Benzyl- steht, dessen Phenylteil gegebenenfalls ein-, oder zweifach, gleich oder verschieden substituiert sein kann mit: Fluor, Chlor, Methoxy-.

Besonders bevorzugt sind Verbindungen der allgemeinen Formel (I), in denen R⁶ für Cyclopentyl- oder Cyclohexyl- steht.

Besonders bevorzugt sind Verbindungen der allgemeinen Formel (I), in denen R⁶ für Tetrahydrofuranyl- oder Tetrahydropyranyl- steht.

Besonders bevorzugt sind ferner Verbindungen der allgemeinen Formel (I), in denen R⁶ für C₃-C₅-Alkyl- oder für 2-Methoxyethyl- steht,
oder für Methyl- steht, das einfach substituiert ist mit Phenyl- oder 4-bis 6-gliedrigem Heterocycloalkyl-,
worin Phenyl- seinerseits gegebenenfalls ein-, oder zweifach, gleich oder verschieden substituiert sein kann mit: Fluor, Chlor, Cyano, Methyl-, Methoxy-, und worin das 4-bis 6-gliedrige Heterocycloalkyl- seinerseits gegebenenfalls einfach substituiert sein kann mit Methyl-,
oder für C₃-C₈-Cycloalkyl- oder für 4-bis 6-gliedriges Heterocycloalkyl- steht, die gegebenenfalls ein- oder zweifach, gleich oder verschieden substituiert sein können mit C₁-C₃-Alkyl- oder C₁-C₄-Alkoxycarbonyl-,
oder für Phenyl steht, das gegebenenfalls ein- oder zweifach, gleich oder verschieden substituiert sein kann mit Fluor, Chlor, Methyl- oder *N-tert*-Butoxycarbonylpiperazinyl-.

Besonders bevorzugt sind Verbindungen der allgemeinen Formel (I), in denen R⁶ für C₃-C₅-Alkyl- oder für 2-Methoxyethyl- steht,
oder für Methyl- steht, das einfach substituiert ist mit Phenyl- oder 4-bis 6-gliedrigem Heterocycloalkyl-,
worin Phenyl- seinerseits gegebenenfalls ein-, oder zweifach, gleich oder verschieden substituiert sein kann mit: Fluor, Chlor, Cyano, Methyl-, Methoxy-, und worin das 4-bis 6-gliedrige Heterocycloalkyl- seinerseits gegebenenfalls einfach substituiert sein kann mit Methyl-.

Besonders bevorzugt sind Verbindungen der allgemeinen Formel (I), in denen R⁶ für C₃-C₅-Alkyl- oder für 2-Methoxyethyl- steht.

Besonders bevorzugt sind Verbindungen der allgemeinen Formel (I), in denen R⁶ für Methyl- steht, das einfach substituiert ist mit Phenyl- oder 4-bis 6-gliedrigem Heterocycloalkyl-,
worin Phenyl- seinerseits gegebenenfalls ein-, oder zweifach, gleich oder verschieden substituiert sein kann mit: Fluor, Chlor, Cyano, Methyl-, Methoxy-, und worin das 4-bis 6-gliedrige Heterocycloalkyl- seinerseits gegebenenfalls einfach substituiert sein kann mit Methyl-.

Besonders bevorzugt sind Verbindungen der allgemeinen Formel (I), in denen R⁶ für C₃-C₈-Cycloalkyl- oder für 4-bis 6-gliedriges Heterocycloalkyl- steht, die gegebenenfalls ein- oder zweifach, gleich oder verschieden substituiert sein können mit C₁-C₃-Alkyl- oder C₁-C₄-Alkoxycarbonyl-.

Besonders bevorzugt sind Verbindungen der allgemeinen Formel (I), in denen R⁶ für Phenyl steht, das gegebenenfalls ein- oder zweifach, gleich oder verschieden substituiert sein kann mit Fluor, Chlor, Methyl- oder *N- tert*-Butoxycarbonylpiperazinyl-.

Ganz besonders bevorzugt sind Verbindungen der allgemeinen Formel (I), in denen R⁶ für Isopropyl-, Isobutyl- oder 2-Methoxyethyl- steht, oder
für Benzyl- steht, dessen Phenylteil gegebenenfalls ein- oder zweifach, gleich oder verschieden substituiert sein kann mit: Fluor, Methoxy-, oder
für C₅-C₇-Cycloalkyl- steht, das gegebenenfalls ein- oder zweifach substituiert sein kann mit Methyl-, oder
für Tetrahydrofuranyl-, Tetrahydropyranyl- oder Piperidinyl- steht,
worin Piperidinyl- gegebenenfalls einfach substitiert sein kann mit Methyl- oder *test-*Butoxycarbonyl-, oder
für Phenyl steht, dass gegebenenfalls einfach substituiert sein kann mit Fluor, Methyl- oder *N-tert-*Butoxycarbonylpiperazinyl-.

Ganz besonders bevorzugt sind Verbindungen der allgemeinen Formel (I), in denen R⁶ für Isopropyl-, Isobutyl- oder 2-Methoxyethyl- steht, oder
für Benzyl- steht, dessen Phenylteil gegebenenfalls ein- oder zweifach, gleich oder verschieden substituiert sein kann mit: Fluor, Methoxy-.

Ganz besonders bevorzugt sind Verbindungen der allgemeinen Formel (I), in denen R⁶ für Isopropyl-, Isobutyl- oder 2-Methoxyethyl- steht.

Ganz besonders bevorzugt sind Verbindungen der allgemeinen Formel (I), in denen R⁶ für Benzylsteht, dessen Phenylteil gegebenenfalls ein- oder zweifach, gleich oder verschieden substituiert sein kann mit: Fluor, Methoxy-.

Ganz besonders bevorzugt sind Verbindungen der allgemeinen Formel (I), in denen R⁶ für Tetrahydrofuranyl-, Tetrahydropyranyl- oder Piperidinyl- steht,
worin Piperidinyl- gegebenenfalls einfach substitiert sein kann mit Methyl- oder *tert-*Butoxycarbonyl-.

Ganz besonders bevorzugt sind Verbindungen der allgemeinen Formel (I), in denen R⁶ für Phenyl steht, dass gegebenenfalls einfach substituiert sein kann mit Fluor, Methyl- oder *N-tert-*Butoxycarbonylpiperazinyl-.

Überaus bevorzugt sind Verbindungen der allgemeinen Formel (I), in denen R⁶ für Isopropyl-, Isobutyl- oder 2-Methoxyethyl-, Benzyl-, 4-Methoxybenzyl-, 2,6-Difluorbenzyl-, Cyclopentyl-, Cyclohexyl-, Cycloheptyl-, Tetrahydropyran-4-yl-, Phenyl-, 3-Methylphenyl- oder 4-Fluorphenylsteht,
oder für 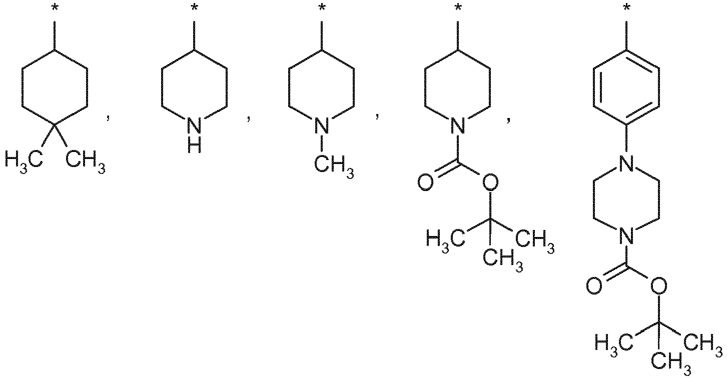 steht, worin "*" jeweils den Anknüpfungspunkt an den Rest des Moleküls bedeutet.

Bevorzugt sind Verbindungen der allgemeinen Formel (I), in denen R⁷ für C₁-C₄-Alkyl- steht, das gegebenenfalls einfach substituiert sein kann mit -NR⁹R¹⁰ oder 4- bis 8-gliedrigem Heterocycloalkyl-,
worin das 4- bis 8-gliedrige Heterocycloalkyl- gegebenenfalls einfach substituiert sein kann mit: Oxo, C₁-C₃-Alkyl-, Fluor-C₁-C₃-Alkyl-, Cyclopropyl- oder Cyclopropylmethyl-,
oder für C₃-C₆-Cycloalkyl- steht, das gegebenenfalls einfach substituiert sein kann mit Hydroxy, Fluor oder -NR⁹R¹⁰,
oder für 4- bis 8-gliedriges Heterocycloalkyl-, das gegebenenfalls ein- oder zweifach, gleich oder verschieden substituiert sein kann mit: Oxo, C₁-C₃-Alkyl, Fluor-C₁-C₃-Alkyl-, Cyclopropyl- oder Cyclopropylmethyl-.

Bevorzugt sind Verbindungen der allgemeinen Formel (I), in denen R⁷ für C₁-C₄-Alkyl- steht, das gegebenenfalls einfach substituiert sein kann mit -NR⁹R¹⁰ oder 4- bis 8-gliedrigem Heterocycloalkyl-,
worin das 4- bis 8-gliedrige Heterocycloalkyl- gegebenenfalls einfach substituiert sein kann mit: Oxo, C₁-C₃-Alkyl-, Fluor-C₁-C₃-Alkyl-, Cyclopropyl- oder Cyclopropylmethyl-.

Bevorzugt sind Verbindungen der allgemeinen Formel (I), in denen R⁷ für C₃-C₆-Cycloalkyl- steht, das gegebenenfalls einfach substituiert sein kann mit Hydroxy, Fluor oder -NR⁹R¹⁰.

Bevorzugt sind Verbindungen der allgemeinen Formel (I), in denen R⁷ für 4- bis 8-gliedriges Heterocycloalkyl-, das gegebenenfalls ein- oder zweifach, gleich oder verschieden substituiert sein kann mit: Oxo, C₁-C₃-Alkyl, Fluor-C₁-C₃-Alkyl-, Cyclopropyl- oder Cyclopropylmethyl-. Bevorzugt sind ferner Verbindungen der allgemeinen Formel (I), in denen R⁷ für Wasserstoff steht, oder für C₁-C₆-Alkyl- steht, das gegebenenfalls ein-, zwei-, oder dreifach, gleich oder verschieden substituiert sein kann mit: Hydroxy, Oxo, Fluor, Cyano, C₁-C₃-Alkoxy-, Fluor-C₁-C₃-alkoxy-, -NR⁹R¹⁰, 4- bis 8-gliedrigem Heterocycloalkyl-, Phenyl-, 5- bis 6-gliedrigem Heteroaryl-,
worin das 4- bis 8-gliedrige Heterocycloalkyl- seinerseits gegebenenfalls einfach substituiert sein kann mit: Hydroxy, Oxo, C₁-C₃-Alkyl-, Fluor-C₁-C₃-Alkyl-, Cyclopropyl-, Cyclopropylmethyl-, Acetyl- oder *tert*-Butoxycarbonyl-,
oder für C₃-C₆-Cycloalkyl- steht, das gegebenenfalls ein- oder zweifach, gleich oder verschieden substituiert sein kann mit: Hydroxy, Oxo, Cyano, Fluor, -NR⁹R¹⁰,
oder für 4- bis 8-gliedriges Heterocycloalkyl-, C₆-C₈-Heterospirocycloalkyl-, verbrücktes C₆-C₁₀-Heterocycloalkyl- oder C₆-C₁₀-Heterobicycloalkyl- steht, die gegebenenfalls ein- oder zweifach, gleich oder verschieden substituiert sein können mit: Hydroxy, Oxo, C₁-C₃-Alkyl-, Fluor-C₁-C₃-Alkyl-, Cyclopropyl-, Cyclopropylmethyl-, Acetyl- oder *tert*-Butoxycarbonyl-.

Bevorzugt sind Verbindungen der allgemeinen Formel (I), in denen R⁷ für Wasserstoff steht.

Bevorzugt sind Verbindungen der allgemeinen Formel (I), in denen R⁷ für C₁-C₆-Alkyl- steht, das gegebenenfalls ein-, zwei-, oder dreifach, gleich oder verschieden substituiert sein kann mit: Hydroxy, Oxo, Fluor, Cyano, C₁-C₃-Alkoxy-, Fluor-C₁-C₃-alkoxy-, -NR⁹R¹⁰, 4- bis 8-gliedrigem Heterocycloalkyl-, Phenyl-, 5- bis 6-gliedrigem Heteroaryl-,
worin das 4- bis 8-gliedrige Heterocycloalkyl- seinerseits gegebenenfalls einfach substituiert sein kann mit: Hydroxy, Oxo, C₁-C₃-Alkyl-, Fluor-C₁-C₃-Alkyl-, Cyclopropyl-, Cyclopropylmethyl-, Acetyl- oder *tert*-Butoxycarbonyl-.

Bevorzugt sind Verbindungen der allgemeinen Formel (I), in denen R⁷ für C₃-C₆-Cycloalkyl- steht, das gegebenenfalls ein- oder zweifach, gleich oder verschieden substituiert sein kann mit: Hydroxy, Oxo, Cyano, Fluor, -NR⁹R¹⁰.

Bevorzugt sind Verbindungen der allgemeinen Formel (I), in denen R⁷ für 4- bis 8-gliedriges Heterocycloalkyl-, C₆-C₈-Heterospirocycloalkyl-, verbrücktes C₆-C₁₀-Heterocycloalkyl- oder C₆-C₁₀-Heterobicycloalkyl- steht, die gegebenenfalls ein- oder zweifach, gleich oder verschieden substituiert sein können mit: Hydroxy, Oxo, C₁-C₃-Alkyl-, Fluor-C₁-C₃-Alkyl-, Cyclopropyl-, Cyclopropylmethyl-, Acetyl- oder *tert*-Butoxycarbonyl-.

Besonders bevorzugt sind Verbindungen der allgemeinen Formel (I), in denen R⁷ für C₁-C₃-Alkylsteht, das gegebenenfalls einfach substituiert sein kann mit -NR⁹R¹⁰, oder für C₅-C₆-Cycloalkyl- steht, das gegebenenfalls einfach substituiert sein kann mit -NR⁹R¹⁰, oder für 4- bis 6-gliedriges Heterocycloalkyl-, das gegebenenfalls einfach substituiert sein kann mit Methyl-.

Besonders bevorzugt sind Verbindungen der allgemeinen Formel (I), in denen R⁷ für für C₁-C₃-Alkyl- steht, das gegebenenfalls einfach substituiert sein kann mit -NR⁹R¹⁰.

Besonders bevorzugt sind Verbindungen der allgemeinen Formel (I), in denen R⁷ für C₅-C₆-Cycloalkyl- steht, das gegebenenfalls einfach substituiert sein kann mit -NR⁹R¹⁰.

Besonders bevorzugt sind Verbindungen der allgemeinen Formel (I), in denen R⁷ für 4- bis 6-gliedriges Heterocycloalkyl- steht, das gegebenenfalls einfach substituiert sein kann mit Methyl-.

Besonders bevorzugt sind ferner Verbindungen der allgemeinen Formel (I), in denen R⁷ für Wasserstoff steht,
oder für C₁-C₄-Alkyl- steht, das gegebenenfalls einfach substituiert sein kann mit -NR⁹R¹⁰ oder 4- bis 8-gliedrigem Heterocycloalkyl-,
worin das 4- bis 8-gliedrige Heterocycloalkyl- seinerseits gegebenenfalls einfach substituiert sein kann mit: Oxo, C₁-C₃-Alkyl-, Fluor-C₁-C₃-Alkyl-, Cyclopropyl- oder Cyclopropylmethyl-,
oder für C₃-C₆-Cycloalkyl- steht, das gegebenenfalls einfach substituiert sein kann mit Hydroxy, Fluor oder -NR⁹R¹⁰,
oder für 4- bis 8-gliedriges Heterocycloalkyl- steht, das gegebenenfalls ein- oder zweifach, gleich oder verschieden substituiert sein kann mit: Oxo, C₁-C₃-Alkyl-, Fluor-C₁-C₃-Alkyl-, Cyclopropyl- oder Cyclopropylmethyl-.

Besonders bevorzugt sind Verbindungen der allgemeinen Formel (I), in denen R⁷ für C₁-C₄-Alkylsteht, das gegebenenfalls einfach substituiert sein kann mit -NR⁹R¹⁰ oder 4- bis 8-gliedrigem Heterocycloalkyl-,
worin das 4- bis 8-gliedrige Heterocycloalkyl- seinerseits gegebenenfalls einfach substituiert sein kann mit: Oxo, C₁-C₃-Alkyl-, Fluor-C₁-C₃-Alkyl-, Cyclopropyl- oder Cyclopropylmethyl-.

Besonders bevorzugt sind Verbindungen der allgemeinen Formel (I), in denen R⁷ für C₃-C₆-Cycloalkyl- steht, das gegebenenfalls einfach substituiert sein kann mit Hydroxy, Fluor oder -NR⁹R¹⁰.

Besonders bevorzugt sind Verbindungen der allgemeinen Formel (I), in denen R⁷ für 4- bis 8-gliedriges Heterocycloalkyl- steht, das gegebenenfalls ein- oder zweifach, gleich oder verschieden substituiert sein kann mit: Oxo, C₁-C₃-Alkyl-, Fluor-C₁-C₃-Alkyl-, Cyclopropyl- oder Cyclopropylmethyl-.

Ganz besonders bevorzugt sind Verbindungen der allgemeinen Formel (I), in denen R⁷ für 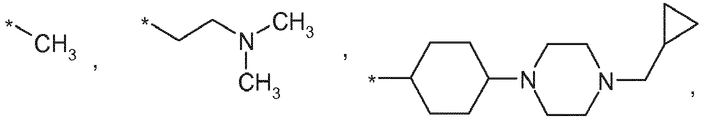 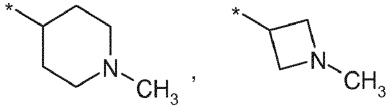 steht,
wobei "*" jeweils den Anknüpfungspunkt an den Rest des Moleküls bedeutet.

Ganz besonders bevorzugt sind ferner Verbindungen der allgemeinen Formel (I), in denen R⁷ für Wasserstoff steht,
oder für C₁-C₃-Alkyl- steht, das gegebenenfalls einfach substituiert sein kann mit -NR⁹R¹⁰ oder *N*-Methylpiperidinyl-,
oder für Cyclopropyl- steht, oder für Cyclohexyl-,
worin Cyclohexyl- gegebenenfalls einfach substituiert sein kann mit Hydroxy- oder -NR⁹R¹⁰,
oder für 4- bis 6-gliedriges Heterocycloalkyl- steht, das gegebenenfalls einfach substituiert sein kann mit Methyl-.

Ganz besonders bevorzugt sind Verbindungen der allgemeinen Formel (I), in denen R⁷ für C₁-C₃-Alkyl- steht, das gegebenenfalls einfach substituiert sein kann mit -NR⁹R¹⁰ oder *N*-Methylpiperidinyl-.

Ganz besonders bevorzugt sind Verbindungen der allgemeinen Formel (I), in denen R⁷ für für Cyclopropyl- steht, oder für Cyclohexyl-,
worin Cyclohexyl- gegebenenfalls einfach substituiert sein kann mit Hydroxy- oder -NR⁹R¹⁰.

Ganz besonders bevorzugt sind ferner Verbindungen der allgemeinen Formel (I), in denen R⁷ für 4- bis 6-gliedriges Heterocycloalkyl- steht, das gegebenenfalls einfach substituiert sein kann mit Methyl-.

Überaus bevorzugt sind ferner Verbindungen der allgemeinen Formel (I), in denen R⁷ für Wasserstoff, Methyl-, Ethyl-, Isopropyl- oder Cyclopropyl- steht,
oder für 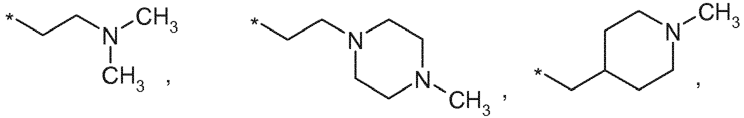 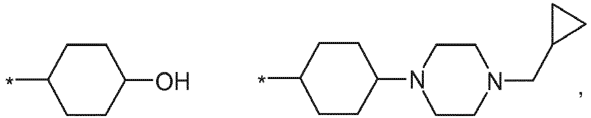 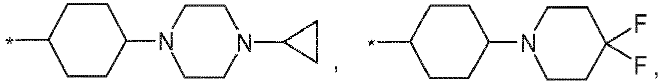 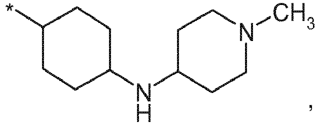 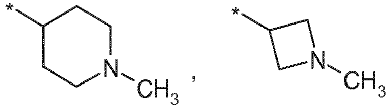 steht,
wobei "*" jeweils den Anknüpfungspunkt an den Rest des Moleküls bedeutet.

Bevorzugt sind Verbindungen der allgemeinen Formel (I), in denen R⁸ für Wasserstoff oder Methyl- steht.

Bevorzugt sind Verbindungen der allgemeinen Formel (I), in denen R⁸ für Wasserstoff, Methyl- oder Ethyl- steht.

Bevorzugt sind Verbindungen der allgemeinen Formel (I), in denen R⁸ für Wasserstoff steht.

Bevorzugt sind Verbindungen der allgemeinen Formel (I), in denen R⁸ für Methyl- steht.

Bevorzugt sind Verbindungen der allgemeinen Formel (I), in denen R⁸ für Ethyl- steht.

Bevorzugt sind Verbindungen der allgemeinen Formel (I), in denen R⁷ und R⁸, gemeinsam mit dem Stickstoffatom, an das sie gebunden sind, für 4-bis 8-gliedriges Heterocycloalkyl-, C₆-C₈-Heterospirocycloalkyl-, verbrücktes C₆-C₁₀-Heterocycloalkyl- oder C₆-C₁₀-Heterobicycloalkylstehen,
die gegebenenfalls ein- oder zweifach, gleich oder verschieden substituiert sein können mit: Hydroxy, Oxo, C₁-C₃-Alkyl-, Fluor-C₁-C₃-Alkyl-, Cyclopropyl-, Cyclopropylmethyl-, Acetyl- oder *tert*-Butoxycarbonyl-.

Bevorzugt sind ferner Verbindungen der allgemeinen Formel (I), in denen R⁷ und R⁸, gemeinsam mit dem Stickstoffatom, an das sie gebunden sind, für 4-bis 8-gliedriges Heterocycloalkyl-, C₆-C₈-Heterospirocycloalkyl-, verbrücktes C₆-C₁₀-Heterocycloalkyl- oder C₆-C₁₀-Heterobicycloalkylstehen,
die gegebenenfalls ein- oder zweifach, gleich oder verschieden substituiert sein können mit: Hydroxy, Fluor, Oxo, C₁-C₃-Alkyl-, Fluor-C₁-C₃-Alkyl-, Cyclopropyl-, Cyclopropylmethyl-, Acetyl- oder *tert*-Butoxycarbonyl-.

Bevorzugt sind Verbindungen der allgemeinen Formel (I), in denen R⁷ und R⁸, gemeinsam mit dem Stickstoffatom, an das sie gebunden sind, für 4-bis 8-gliedriges Heterocycloalkyl- stehen,
das gegebenenfalls ein- oder zweifach, gleich oder verschieden substituiert sein kann mit: Hydroxy, Oxo, C₁-C₃-Alkyl-, Fluor-C₁-C₃-Alkyl-, Cyclopropyl-, Cyclopropylmethyl-, Acetyl- oder *tert-*Butoxycarbonyl-.

Besonders bevorzugt sind Verbindungen der allgemeinen Formel (I), in denen R⁷ und R⁸, gemeinsam mit dem Stickstoffatom, an das sie gebunden sind, für 5-bis 6-gliedriges Heterocycloalkyl- oder C₆-C₈-Heterospirocycloalkyl- stehen, die gegebenenfalls ein- oder zweifach, gleich oder verschieden substituiert sein können mit: Oxo, C₁-C₃-Alkyl, Fluor-C₁-C₃-Alkyl-, Cyclopropyl- oder Cyclopropylmethyl-.

Besonders bevorzugt sind Verbindungen der allgemeinen Formel (I), in denen R⁷ und R⁸, gemeinsam mit dem Stickstoffatom, an das sie gebunden sind, für 5-bis 6-gliedriges Heterocycloalkyl- stehen, das gegebenenfalls ein- oder zweifach, gleich oder verschieden substituiert sein kann mit: Oxo, C₁-C₃-Alkyl, Fluor-C₁-C₃-Alkyl-, Cyclopropyl- oder Cyclopropylmethyl-.

Besonders bevorzugt sind ferner Verbindungen der allgemeinen Formel (I), in denen R⁷ und R⁸, gemeinsam mit dem Stickstoffatom, an das sie gebunden sind, für 4-bis 6-gliedriges Heterocycloalkyl- oder C₆-C₈-Heterospirocycloalkyl- stehen, die gegebenenfalls ein- oder zweifach, gleich oder verschieden substituiert sein können mit: Fluor, Oxo, C₁-C₃-Alkyl-, Fluor-C₁-C₃-Alkyl-, Cyclopropyl- oder Cyclopropylmethyl-.

Ganz besonders bevorzugt sind Verbindungen der allgemeinen Formel (I), in denen R⁷ und R⁸, gemeinsam mit dem Stickstoffatom, an das sie gebunden sind, für 6-gliedriges Heterocycloalkylstehen, das gegebenenfalls einfach substituiert sein kann mit Methyl-.

Ganz besonders bevorzugt sind weiterhin Verbindungen der allgemeinen Formel (I), in denen R⁷ und R⁸, gemeinsam mit dem Stickstoffatom, an das sie gebunden sind, für 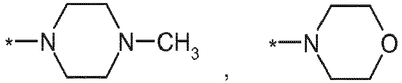 stehen,
wobei "*" jeweils den Anknüpfungspunkt an den Rest des Moleküls bedeutet.

Ganz besonders bevorzugt sind ferner Verbindungen der allgemeinen Formel (I), in denen R⁷ und R⁸, gemeinsam mit dem Stickstoffatom, an das sie gebunden sind, für 4- bis 6-gliedriges Heterocycloalkyl- stehen, das gegebenenfalls ein- oder zweifach substituiert sein kann mit Fluor, oder das gegebenenfalls einfach substituiert sein kann mit Methyl-, Isopropyl-, 2,2,2-Trifluoroethyl- oder Cyclopropylmethyl-,
oder für 6-Azaspiro[3.3]heptyl-oder für 2-Oxa-6-azaspiro[3.3]heptyl- stehen.

Überaus bevorzugt sind Verbindungen der allgemeinen Formel (I), in denen R⁷ und R⁸, gemeinsam mit dem Stickstoffatom, an das sie gebunden sind, für 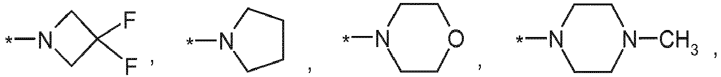 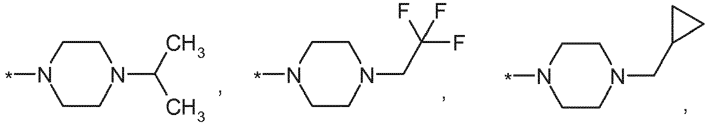 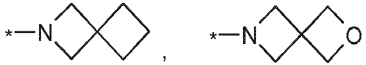 stehen,
wobei "*" jeweils den Anknüpfungspunkt an den Rest des Moleküls bedeutet.

Bevorzugt sind ferner Verbindungen der allgemeinen Formel (I), in denen R⁷ für Wasserstoff steht, oder für C₁-C₆-Alkyl- steht, das gegebenenfalls ein-, zwei-, oder dreifach, gleich oder verschieden substituiert sein kann mit: Hydroxy, Oxo, Fluor, Cyano, C₁-C₃-Alkoxy-, Fluor-C₁-C₃-alkoxy-, -NR⁹R¹⁰, 4- bis 8-gliedrigem Heterocycloalkyl-, Phenyl-, 5- bis 6-gliedrigem Heteroaryl-,
worin das 4- bis 8-gliedrige Heterocycloalkyl- seinerseits gegebenenfalls einfach substituiert sein kann mit: Hydroxy, Oxo, C₁-C₃-Alkyl-, Fluor-C₁-C₃-Alkyl-, Cyclopropyl-, Cyclopropylmethyl-, Acetyl- oder *tert*-Butoxycarbonyl-,
oder für C₃-C₆-Cycloalkyl- steht, das gegebenenfalls ein- oder zweifach, gleich oder verschieden substituiert sein kann mit: Hydroxy, Oxo, Cyano, Fluor, -NR⁹R¹⁰,
oder für 4- bis 8-gliedriges Heterocycloalkyl-, C₆-C₈-Heterospirocycloalkyl-, verbrücktes C₆-C₁₀-Heterocycloalkyl- oder C₆-C₁₀-Heterobicycloalkyl- steht, die gegebenenfalls ein- oder zweifach, gleich oder verschieden substituiert sein können mit: Hydroxy, Oxo, C₁-C₃-Alkyl-, Fluor-C₁-C₃-Alkyl-, Cyclopropyl-, Cyclopropylmethyl-, Acetyl- oder *tert*-Butoxycarbonyl-,
und in denen R⁸ für Wasserstoff oder C₁-C₃-Alkyl- steht,
oder in denen R⁷ und R⁸ gemeinsam mit dem Stickstoffatom, an welches sie gebunden sind, für 4-bis 8-gliedriges Heterocycloalkyl-, C₆-C₈-Heterospirocycloalkyl-, verbrücktes C₆-C₁₀-Heterocycloalkyl- oder C₆-C₁₀-Heterobicycloalkyl- stehen,
die gegebenenfalls ein- oder zweifach, gleich oder verschieden substituiert sein können mit: Hydroxy, Fluor, Oxo, C₁-C₃-Alkyl-, Fluor-C₁-C₃-Alkyl-, Cyclopropyl-, Cyclopropylmethyl-, Acetyl- oder *tert*-Butoxycarbonyl-.

Besonders bevorzugt sind Verbindungen der allgemeinen Formel (I), in denen R⁷ für Wasserstoff steht, oder für C₁-C₄-Alkyl- steht, das gegebenenfalls einfach substituiert sein kann mit -NR⁹R¹⁰ oder 4- bis 8-gliedrigem Heterocycloalkyl-,
worin das 4- bis 8-gliedrige Heterocycloalkyl- seinerseits gegebenenfalls einfach substituiert sein kann mit: Oxo, C₁-C₃-Alkyl-, Fluor-C₁-C₃-Alkyl-, Cyclopropyl- oder Cyclopropylmethyl-,
oder für C₃-C₆-Cycloalkyl- steht, das gegebenenfalls einfach substituiert sein kann mit Hydroxy, Fluor oder -NR⁹R¹⁰,
oder für 4- bis 8-gliedriges Heterocycloalkyl- steht, das gegebenenfalls ein- oder zweifach, gleich oder verschieden substituiert sein kann mit: Oxo, C₁-C₃-Alkyl-, Fluor-C₁-C₃-Alkyl-, Cyclopropyl- oder Cyclopropylmethyl-,
und in denen R⁸ für Wasserstoff, Methyl- oder Ethyl- steht,
oder in denen R⁷ und R⁸ gemeinsam mit dem Stickstoffatom, an welches sie gebunden sind, für 4-bis 6-gliedriges Heterocycloalkyl- oder C₆-C₈-Heterospirocycloalkyl- stehen, die gegebenenfalls ein- oder zweifach, gleich oder verschieden substituiert sein können mit: Fluor, Oxo, C₁-C₃-Alkyl-, Fluor-C₁-C₃-Alkyl-, Cyclopropyl- oder Cyclopropylmethyl-.

Ganz besonders bevorzugt sind Verbindungen der allgemeinen Formel (I), in denen R⁷ (für Wasserstoff steht, oder für C₁-C₃-Alkyl- steht, das gegebenenfalls einfach substituiert sein kann mit -NR⁹R¹⁰ oder *N*-Methylpiperidinyl-,
oder für Cyclopropyl- steht, oder für Cyclohexyl-,
worin Cyclohexyl- gegebenenfalls einfach substituiert sein kann mit Hydroxy- oder-NR⁹R¹⁰,
oder für 4- bis 6-gliedriges Heterocycloalkyl- steht, das gegebenenfalls einfach substituiert sein kann mit Methyl-,
und in denen R⁸ für Wasserstoff, Methyl- oder Ethyl- steht,
oder in denen R⁷ und R⁸ gemeinsam mit dem Stickstoffatom, an das sie gebunden sind, für 4- bis 6-gliedriges Heterocycloalkyl- stehen, das gegebenenfalls ein- oder zweifach substituiert sein kann mit Fluor, oder das gegebenenfalls einfach substituiert sein kann mit Methyl-, Isopropyl-, 2,2,2-Trifluoroethyl- oder Cyclopropylmethyl-, oder für 6-Azaspiro[3.3]heptyl-oder für 2-Oxa-6-azaspiro[3.3]heptyl- stehen.

Bevorzugt sind Verbindungen der allgemeinen Formel (I), in denen R⁹ und R¹⁰ unabhängig voneinander für Wasserstoff oder für gegebenenfalls einfach mit Hydroxy oder Oxo substituiertes C₁-C₃-Alkyl- oder für Trifluormethyl- stehen.

Bevorzugt sind ferner Verbindungen der allgemeinen Formel (I), in denen R⁹ und R¹⁰ unabhängig voneinander für Wasserstoff oder für gegebenenfalls einfach mit Hydroxy oder Oxo substituiertes C₁-C₃-Alkyl- oder für Trifluormethyl-, oder für 6-gliedriges Heterocycloalkyl- stehen,
worin das 6-gliedrige Heterocycloalkyl- seinerseits gegebenenfalls ein- oder zweifach, gleich oder verschieden substituiert sein kann mit C₁-C₃-Alkyl-.

Besonders bevorzugt sind Verbindungen der allgemeinen Formel (I), in denen R⁹ und R¹⁰ unabhängig voneinander für Wasserstoff oder für C₁-C₃-Alkyl- stehen.

Besonders bevorzugt sind Verbindungen der allgemeinen Formel (I), in denen R⁹ und R¹⁰ unabhängig voneinander für C₁-C₃-Alkyl- stehen.

Besonders bevorzugt sind Verbindungen der allgemeinen Formel (I), in denen R⁹ und R¹⁰ jeweils für Methyl- stehen.

Besonders bevorzugt sind ferner Verbindungen der allgemeinen Formel (I), in denen R⁹ und R¹⁰ unabhängig voneinander für Wasserstoff oder für gegebenenfalls einfach mit Hydroxy oder Oxo substituiertes C₁-C₃-Alkyl-, Trifluormethyl-, oder für *N*-Methylpiperidinyl- stehen.

Besonders bevorzugt sind Verbindungen der allgemeinen Formel (I), in denen R⁹ für gegebenenfalls einfach mit Hydroxy oder Oxo substituiertes C₁-C₃-Alkyl-, Trifluormethyl-, oder für *N*-Methylpiperidinyl- steht, und in denen R¹⁰ für Wasserstoff steht.

Ganz besonders bevorzugt sind Verbindungen der allgemeinen Formel (I), in denen R⁹ und R¹⁰ unabhängig voneinander für Wasserstoff, C₁-C₃-Alkyl- oder für *N*-Methylpiperidinyl- stehen.

Ganz besonders bevorzugt sind Verbindungen der allgemeinen Formel (I), in denen R⁹ für C₁-C₃-Alkyl- oder für *N*-Methylpiperidinyl- steht, und in denen R¹⁰ für Wasserstoff steht.

Bevorzugt sind Verbindungen der allgemeinen Formel (I), in denen R⁹ und R¹⁰, gemeinsam mit dem Stickstoffatom, an das sie gebunden sind, für 4-7-gliedriges Heterocycloalkyl- stehen, das gegebenenfalls ein- oder zweifach, gleich oder verschieden substituiert sein kann mit: Oxo, C₁-C₃-Alkyl-, Fluor-C₁-C₃-Alkyl-, Cyclopropyl- oder Cyclopropylmethyl-.

Bevorzugt sind ferner Verbindungen der allgemeinen Formel (I), in denen R⁹ und R¹⁰, gemeinsam mit dem Stickstoffatom, an das sie gebunden sind, für 4- bis 7-gliedriges Heterocycloalkyl- stehen, das gegebenenfalls ein- oder zweifach, gleich oder verschieden substituiert sein kann mit: Hydroxy, Fluor, Oxo, C₁-C₃-Alkyl-, Fluor-C₁-C₃-Alkyl-, Cyclopropyl-, Cyclopropylmethyl-, Acetyl- oder *tert*-Butoxycarbonyl-.

Besonders bevorzugt sind Verbindungen der allgemeinen Formel (I), in denen R⁹ und R¹⁰, gemeinsam mit dem Stickstoffatom, an das sie gebunden sind, für 6-gliedriges Heterocycloalkylstehen, das gegebenenfalls einfach substituiert sein kann mit Methyl-, 2,2,2-Trifluorethyl- oder Cyclopropylmethyl-.

Besonders bevorzugt sind ferner Verbindungen der allgemeinen Formel (I), in denen R⁹ und R¹⁰, gemeinsam mit dem Stickstoffatom, an das sie gebunden sind, für 4- bis 7-gliedriges Heterocycloalkyl- stehen, das gegebenenfalls ein- oder zweifach, gleich oder verschieden substituiert sein kann mit: Fluor, Oxo, C₁-C₃-Alkyl-, Fluor-C₁-C₃-Alkyl-, Cyclopropyl- oder Cyclopropylmethyl-.

Besonders bevorzugt sind Verbindungen der allgemeinen Formel (I), in denen R⁹ und R¹⁰, gemeinsam mit dem Stickstoffatom, an das sie gebunden sind, für *N*-Cyclopropylmethylpiperazinyl- stehen.

Ganz besonders bevorzugt sind Verbindungen der allgemeinen Formel (I), in denen R⁹ und R¹⁰, gemeinsam mit dem Stickstoffatom, an das sie gebunden sind, für 6-gliedriges Heterocycloalkylstehen, das gegebenenfalls ein- oder zweifach substituiert sein kann mit Fluor, oder das gegebenenfalls einfach substituiert sein kann mit Methyl-, 2,2,2-Trifluorethyl-, Cyclopropyl- oder Cyclopropylmethyl-.

Bevorzugt sind Verbindungen der allgemeinen Formel (I), in denen R⁹ und R¹⁰ unabhängig voneinander für Wasserstoff oder für gegebenenfalls einfach mit Hydroxy oder Oxo substituiertes C₁-C₃-Alkyl- oder für Trifluormethyl-, oder für 6-gliedriges Heterocycloalkyl- stehen,
worin das 6-gliedrige Heterocycloalkyl- seinerseits gegebenenfalls ein- oder zweifach, gleich oder verschieden substituiert sein kann mit C₁-C₃-Alkyl-,
oder in denen R⁹ und R¹⁰ gemeinsam mit dem Stickstoffatom, an das sie gebunden sind, für 4- bis 7-gliedriges Heterocycloalkyl- stehen, das gegebenenfalls ein- oder zweifach, gleich oder verschieden substituiert sein kann mit: Hydroxy, Fluor, Oxo, C₁-C₃-Alkyl-, Fluor-C₁-C₃-Alkyl-, Cyclopropyl-, Cyclopropylmethyl-, Acetyl- oder *tert*-Butoxycarbonyl-.

Besonders bevorzugt sind Verbindungen der allgemeinen Formel (I), in denen R⁹ und R¹⁰ unabhängig voneinander für Wasserstoff oder für gegebenenfalls einfach mit Hydroxy oder Oxo substituiertes C₁-C₃-Alkyl-, Trifluormethyl-, oder für *N*-Methylpiperidinyl- stehen,
oder in denen R⁹ und R¹⁰ gemeinsam mit dem Stickstoffatom, an das sie gebunden sind, für 4- bis 7-gliedriges Heterocycloalkyl- stehen, das gegebenenfalls ein- oder zweifach, gleich oder verschieden substituiert sein kann mit: Fluor, Oxo, C₁-C₃-Alkyl-, Fluor-C₁-C₃-Alkyl-, Cyclopropyl- oder Cyclopropylmethyl-.

Ganz besonders bevorzugt sind Verbindungen der allgemeinen Formel (I), in denen R⁹ und R¹⁰ unabhängig voneinander für Wasserstoff, C₁-C₃-Alkyl- oder für *N*-Methylpiperidinyl- stehen,
oder in denen R⁹ und R¹⁰ gemeinsam mit dem Stickstoffatom, an das sie gebunden sind, für 6-gliedriges Heterocycloalkyl- stehen, das gegebenenfalls ein- oder zweifach substituiert sein kann mit Fluor, oder das gegebenenfalls einfach substituiert sein kann mit Methyl-, 2,2,2-Trifluorethyl-, Cyclopropyl- oder Cyclopropylmethyl-.

Besonders bevorzugt sind Verbindungen der allgemeinen Formel (I), in denen A für -NH- oder -N(Methyl)- steht, n für 0 oder 1 steht, R² für Wasserstoff, Methyl- oder Methoxy- steht, R³ für Methyl- steht, R⁴ für Methyl- steht und R⁵ für Wasserstoff steht.

Besonders bevorzugt sind Verbindungen der allgemeinen Formel (I), in denen A für -NH- oder -N(Methyl)- steht, X für -N- steht, n für 0 oder 1 steht, R² für Wasserstoff, Methyl- oder Methoxysteht, R³ für Methyl- steht, R⁴ für Methyl- steht und R⁵ für Wasserstoff steht.

Besonders bevorzugt sind Verbindungen der allgemeinen Formel (I), in denen A für -NH- oder -N(Methyl)- steht, X für -CH- steht, n für 0 oder 1 steht, R² für Wasserstoff, Methyl- oder Methoxy- steht, R³ für Methyl- steht, R⁴ für Methyl- steht und R⁵ für Wasserstoff steht.

Die in den jeweiligen Kombinationen bzw. bevorzugten Kombinationen von Resten im Einzelnen angegebenen Restedefinitionen werden unabhängig von den jeweiligen angegebenen Kombinationen der Reste beliebig auch durch Restedefinitionen anderer Kombination ersetzt.

Ganz besonders bevorzugt sind Kombinationen von zwei oder mehreren der oben genannten Vorzugsbereiche.

Ganz besonders bevorzugt sind die nachfolgenden Verbindungen der allgemeinen Formel (I):
(3*R*)-4-Cyclopentyl-1,3-dimethyl-6-({3-[(4-methylpiperazin-1-yl)carbonyl]phenyl}amino)-3,4-dihydropyrido[2,3-*b*]pyrazin-2(1*H*)-on;
3-{[(3*R*)-4-Cyclopentyl-1,3-dimethyl-2-oxo-1,2,3,4-tetrahydropyrido[2,3-*b*]pyrazin-6-yl]amino}-*N*-(1- methylpiperidin-4-yl)benzamid;
3-{[(3*R*)-4-Cyclopentyl-1,3-dimethyl-2-oxo-1,2,3,4-tetrahydropyrido[2,3-*b*]pyrazin-6-yl]amino}-*N*-(1-methylazetidin-3-yl)benzamid;
3-{[(3*R*)-4-Cyclopentyl-1,3-dimethyl-2-oxo-1,2,3,4-tetrahydropyrido[2,3-*b*]pyrazin-6-yl]amino}-*N*-[2-(dimethylamino)ethyl]benzamid;
3-{[(3*R*)-4-Cyclopentyl-1,3-dimethyl-2-oxo-1,2,3,4-tetrahydropyrido[2,3-*b*]pyrazin-6-yl]amino}-*N*-{*trans*-4-[4-(cyclopropylmethyl)piperazin-1-yl]cyclohexyl}benzamid;
3-{[(3*R*)-1,3-Dimethyl-2-oxo-4-(tetrahydro-2*H*-pyran-4-yl)-1,2,3,4-tetrahydropyrido[2,3-*b*]-pyrazin-6-yl]amino}-*N,N*-dimethylbenzolsulfonamid;
3-{[(3*R*)-1,3-Dimethyl-2-oxo-4-(tetrahydro-2*H*-pyran-4-yl)-1,2,3,4-tetrahydrochinoxalin-6-yl]-amino} -*N,N*-dimethylbenzolsulfonamid;
(3*R*)-1,3-Dimethyl-6-{[3-(morpholin-4-ylsulfonyl)phenyl]amino}-4-(tetrahydro-2*H*-pyran-4-yl)-3,4-dihydrochinoxalin-2(1*H*)-on;
(3*R*)*-*1,3-Dimethyl-6-{[3-(morpholin-4-ylsulfonyl)phenyl]amino}-4-(tetrahydro-2*H-*pyran-4-yl)-3,4-dihydropyrido[2,3-*b*]pyrazin-2(1*H*)-on;
3-{[(3*R*)-4-(4-Methoxybenzyl)-1,3-dimethyl-2-oxo-1,2,3,4-tetrahydrochinoxalin-6-yl]amino*-N,N-*dimethylbenzolsulfonamid;
(3*R*)-4-(4-Methoxybenzyl)-1,3-diethyl-6-{[3-(morpholin-4-ylsulfonyl)phenyl]amino}-3,4-dihydrochinoxalin-2(1*H*)-on;
(3*R*)-1,3-Dimethyl-6-({3-[(4-methylpiperazin-1-yl)sulfonyl]phenyl}amino)-4-(tetrahydro-2*H-*pyran-4-yl)-3,4-dihydropyrido[2,3-*b*]pyrazin-2(1*H*)-on;
3-{[(3*R*)-1,3-Dimethyl-2-oxo-4-(tetrahydro-2*H*-pyran-4-yl)-1,2,3,4-tetrahydropyrido[2,3-*b*]-pyrazin-6-yl]amino}-*N*-(1-methylpiperidin-4-yl)benzamid;
*N*-{*trans*-4-[4-(Cyclopropylmethyl)piperazin-1-yl]cyclohexyl}-3-{[(3*R*)-1,3-dimethyl-2-oxo-4-(tetrahydro-2*H*-pyran-4-yl)-1,2,3,4-tetrahydropyrido[2,3-*b*]pyrazin-6-yl]amino}benzamid;
(3*R*)-1,3-Dimethyl-6-({3-[(4-methylpiperazin-1-yl)carbonyl]phenyl}amino)-4-(tetrahydro-2*H-*pyran-4-yl)-3,4-dihydropyrido[2,3-*b*]pyrazin-2(1*H*)-on;
3-{[(3*R*)-1,3-Dimethyl-2-oxo-4-(tetrahydro-2*H*-pyran-4-yl)-1,2,3,4-tetrahydropyrido [2,3-*b*]-pyrazin-6-yl]amino}-4-methoxy-*N*-(1-methylpiperidin-4-yl)benzamid;
(3*R*)-6-({2-Methoxy-5-[(4-methylpiperazin-1-yl)carbonyl]phenyl}amino)-1,3-dimethyl-4-(tetrahydro-2*H*-pyran-4-yl)-3,4-dihydropyrido[2,3-*b*]pyrazin-2(1*H*)-on;
*N*-{*trans*-4-[4-(Cyclopropylmethyl)piperazin-1-yl]cyclohexyl}-3-{[(3*R*)-1,3-dimethyl-2-oxo-4-(tetrahydro-2*H*-pyran-4-yl)-1,2,3,4-tetrahydropyrido[2,3-*b*]pyrazin-6-yl]amino}-4-methoxybenzamid;
*N*-[2-(Dimethylamino)ethyl]-3-{[(3*R*)-1,3-dimethyl-2-oxo-4-(tetrahydro-2*H*-pyran-4-yl)-1,2,3,4-tetrahydropyrido[2,3-*b*]pyrazin-6-yl]amino}-4-methoxybenzamid;
3-{[(3*R*)-1,3-Dimethyl-2-oxo-4-(tetrahydro-2*H*-pyran-4-yl)-1,2,3,4-tetrahydropyrido[2,3-*b*]-pyrazin-6-yl]amino}-*N*-(1-methylpiperidin-4-yl)benzolsulfonamid;
*N*-[2-(Dimethylamino)ethyl]-3-{[(3*R*)-1,3-dimethyl-2-oxo-4-(tetrahydro-2*H*-pyran-4-yl)-1,2,3,4-tetrahydropyrido [2,3-*b*]pyrazin-6-yl]amino}benzolsulfonamid;
*N*-{*trans*-4-[4-(Cyclopropylmethyl)piperazin-1-yl]cyclohexyl}-3-{[4-(2,6-difluorbenzyl)-1,3-dimethyl-2-oxo-1,2,3,4-tetrahydrochinoxalin-6-yl]amino}benzamid;
3-{[(3*R*)-1,3-Dimethyl-2-oxo-4-(tetrahydro-2H-pyran-4-yl)-1,2,3,4-tetrahydropyrido[2,3-b]pyrazin-6-yl]amino}-*N*-ethylbenzolsulfonamid;
(3*R*)*-*1,3-Dimethyl-4-(1-methylpiperidin-4-yl)-6-{[3-(pyrrolidin-1-ylsulfonyl)phenyl]amino}-3,4-dihydropyrido[2,3-b]pyrazin-2(1H)-on;
(3*R*)-6-{[2-Methoxy-5-(morpholin-4-ylsulfonyl)phenyl]amino}-1,3-dimethyl-4-(1-methylpiperidin-4-yl)-3,4-dihydropyrido[2,3-b]pyrazin-2(1H)-on;
*N*-Cyclopropyl-3-{[(3*R*)-1,3-dimethyl-2-oxo-4-(tetrahydro-2H-pyran-4-yl)-1,2,3,4-tetrahydropyrido[2,3-b]pyrazin-6-yl]amino}benzolsulfonamid;
(3*R*)-1,3-Dimethyl-6-{[3-(Pyrrolidin-1-ylsulfonyl)phenyl]amino}-4-(tetrahydro-2H-pyran-4-yl)-3,4-dihydropyrido[2,3-b]pyrazin-2(1H)-on;
(3*R*)-6-{{[2-Methoxy-5-(morpholin-4-ylsulfonyl)phenyl]amino}-1,3-dimethyl-4-(tetrahydro-2H-pyran-4-yl)-3,4-dihydropyrido[2,3-b]pyrazin-2(1H)-on;
(3*R*)-6-({3-[(3,3-Difluorazetidin-1-yl)sulfonyl]phenyl}amino)-1,3-dimethyl-4-(1-methylpiperidin-4-yl)-3,4-dihydropyrido[2,3-b]pyrazin-2(1H)-on;
(3*R*)-6-{[2-Methoxy-5-(2-oxa-6-azaspiro[3.3]hept-6-ylsulfonyl)phenyl]amino}-1,3-dimethyl-4-(tetrahydro-2H-pyran-4-yl)-3,4-dihydropyrido [2,3-b]pyrazin-2(1H)-on;
(3*R*)-6-({3-[(3,3-Difluorazetidin-1-yl)sulfonyl]phenyl}amino)-1,3-dimethyl-4-(tetrahydro-2H-pyran-4-yl)-3,4-dihydropyrido [2,3-b]pyrazin-2(1H)-on;
3-{[(3*R*)-1,3-Dimethyl-2-oxo-4-(tetrahydro-2H-pyran-4-yl)-1,2,3,4-tetrahydropyrido[2,3-b]pyrazin-6-yl]amino}-N,N-diethylbenzol sulfonamid;
3-{[(3*R*)-4-Benzyl-1,3-dimethyl-2-oxo-1,2,3,4-tetrahydropyrido[2,3-b]pyrazin-6-yl]amino}-*N*-(1-methylpiperidin-4-yl)benzolsulfonamid;
*N*-{*trans*-4-[4-(Cyclopropylmethyl)piperazin-1-yl]cyclohexyl}-3-}[(3*R*)-4-isopropyl-1,3-dimethyl-2-oxo-1,2,3,4-tetrahydropyrido[2,3-b]pyrazin-6-yl]amino}benzolsulfonamid;
*N-*{*trans*-4-[4-(Cyclopropylmethyl)piperazin-1-yl]cyclohexyl}-3-{[(3*R*)-1,3-dimethyl-2-oxo-4-(tetrahydro-2H-pyran-4-yl)-1,2,3,4-tetrahydropyrido[2,3-b]pyrazin-6-yl]amino}benzolsulfonamid;
*N*-{*trans*-4-[4-(Cyclopropylmethyl)piperazin-1-yl]cyclohexyl}-3-{[(3*S*)-1,3-dimethyl-2-oxo-4-(tetrahydro-2H-pyran-4-yl)-1,2,3,4-tetrahydropyrido[2,3-b]pyrazin-6-yl]amino}benzolsulfonamid;
(3*R*)-1,3-Dimethyl-4-(tetrahydro-2H-pyran-4-yl)-6-[(3-{[4-(2,2,2-trifluorethyl)piperazin-1-yl]sulfonyl}phenyl)amino]-3,4-dihydropyrido[2,3-b]pyrazin-2(1H)-on;
3-{[(3*R*)-4-Benzyl-1,3-dimethyl-2-oxo-1,2,3,4-tetrahydropyrido[2,3-b]pyrazin-6-yl]amino}-*N-*{*trans*-4-[4-(cyclopropyl-methyl)piperazin-1-yl]cyclohexyl}benzolsulfonamid;
3-{[(3*R*)-1,3-Dimethyl-2-oxo-4-(tetrahydro-2H-pyran-4-yl)-1,2,3,4-tetrahydropyrido [2,3-b]pyrazin-6-yl]amino}-*N*-methylbenzolsulfonamid;
(3*R*)-6-({2-Methoxy-5-[(4-methylpiperazin-1-yl)sulfonyl]phenyl}amino)-1,3-dimethyl-4-(tetrahydro-2H-pyran-4-yl)-3,4-dihydropyrido [2,3-b]pyrazin-2(1H)-on;
3-{[(3*R*)-1,3-Dimethyl-4-(1-methylpiperidin-4-yl)-2-oxo-1,2,3,4-tetra-hydropyrido[2,3-b]pyrazin-6-yl]amino}-*N,N*-dimethylbenzolsulfonamid;
3-{[(3*R*)-1,3-Dimethyl-2-oxo-4-(tetrahydro-2H-pyran-4-yl)-1,2,3,4-tetrahydropyrido[2,3-b]pyrazin-6-yl]amino}-*N*-isopropylbenzolsulfonamid;
(3*R*)-4-Isopropyl-1,3-dimethyl-6-({3-[(4-methylpiperazin-1-yl)sulfonyl]phenyl}amino)-3,4-dihydropyrido[2,3-b]pyrazin-2(1H)-on;
3-{[(3*R*)-1,3-Dimethyl-2-oxo-4-(tetrahydro-2H-pyran-4-yl)-1,2,3,4-tetrahydropyrido[2,3-b]pyrazin-6-yl]amino}-*N*-[(1-methylpiperidin-4-yl)methyl]benzolsulfonamid;
*N*-{*trans*-4-[4-(Cyclopropylmethyl)piperazin-1-yl]cyclohexyl}-3-{[(3*R*)-4-isobutyl-1,3-dimethyl-2-oxo-1,2,3,4-tetrahydropyrido[2,3-b]pyrazin-6-yl]amino}benzolsulfonamid;
(3*R*)-6-({2-Methoxy-5-[(4-methylpiperazin-1-yl)sulfonyl]phenyl}amino)-1,3-dimethyl-4-(1-methylpiperidin-4-yl)-3,4-dihydropyrido[2,3-b]pyrazin-2(1H)-on;
(3*R*)-1,3-Dimethyl-4-(1-methylpiperidin-4-yl)-6-[3-(morpholin-4-ylsulfonyl)phenyl]amino}-3,4-dihydropyrido[2,3-b]pyrazin-2(1H)-on;
3-{[(3*R*)-4-Cycloheptyl-1,3-dimethyl-2-oxo-1,2,3,4-tetrahydropyrido[2,3-b]pyrazin-6-yl]amino}-*N*-(1-methyl-piperidin-4-yl)benzol-sulfonamid;
4-(2-Methoxyethyl)-1,3-dimethyl-6-{[3-(morpholin-4-ylsulfonyl)phenyl]amino}-3,4-dihydropyrido[2,3-b]pyrazin-2(1H)-on;
(3*R*)-1,3-Dimethyl-6-({3-[(4-methylpiperazin-1-yl)sulfonyl]phenyl}amino)-4-(1-methylpiperidin-4-yl)-3,4-dihydropyrido[2,3-b]pyrazin-2(1H)-on;
3-{[(3*R*)-4-Isopropyl-1,3-dimethyl-2-oxo-1,2,3,4-tetrahydropyrido[2,3-b]pyrazin-6-yl]amino}-*N-*(1-methylpiperidin-4-yl)benzolsulfonamid;
*tert-*Butyl-4-[(3*R*)-6-{[3-({*trans*-4-[4-(cyclopropyl-methyl)piperazin-1-yl]cyclohexyl}sulfamoyl) phenyl]amino}-1,3-dimethyl-2-oxo-2,3-dihydropyrido[2,3-b]pyrazin-4(1H)-yl]piperidin-1-carboxylat;
4-(2-Methoxyethyl)-1,3-dimethyl-6-({3-[(4-methylpiperazin-1-yl)sulfonyl]phenyl}amino)-3,4-dihydropyrido[2,3-b]pyrazin-2(1H)-on;
3-{[(3*R*)-1,3-Dimethyl-2-oxo-4-(tetrahydro-2H-pyran-4-yl)-1,2,3,4-tetrahydropyrido[2,3-b]pyrazin-6-yl](methyl)amino}-*N,N*-dimethyl-benzolsulfonamid;
*N*-[2-(Dimethylamino)ethyl]-3-{[(3*R*)-4-isopropyl-1,3-dimethyl-2-oxo-1,2,3,4-tetrahydropyrido[2,3-b]pyrazin-6-yl]amino}benzolsulfonamid;
(3*R*)-6-[(1,1-Dioxido-2,3-dihydro-1,2-benzothiazol-6-yl)amino]-1,3-dimethyl-4-(tetrahydro-2H-pyran-4-yl)-3,4-dihydropyrido [2,3-b]pyrazin-2(1H)-on;
*tert*-Butyl-4-[(3*R*)-1,3-dimethyl-6-({3-[(1-methylpiperidin-4-yl)sulfamoyl]phenyl}amino)-2-oxo-2,3-dihydro-pyrido[2,3-b]pyrazin-4(1H)-yl]piperidin-1-carboxylat;
3-{[(3*R*)-1,3-Dimethyl-2-oxo-4-(tetrahydro-2H-pyran-4-yl)-1,2,3,4-tetrahydropyrido [2,3-b]pyrazin-6-yl]amino} benzolsulfonamid;
*tert*-Butyl-4-[(3*R*)-6-[(3-{[2-(dimethylamino)ethyl]sulfamoyl}phenyl)amino]-1,3-dimethyl-2-oxo-2,3-dihydropyrido[2,3-b]pyrazin-4(1H)-yl] piperidin-1-carboxylat;
*tert-*Butyl-4-[(3*R*)-6-{[3-(2-azaspiro[3.3]hept-2-ylsulfonyl)phenyl]amino}-1,3-dimethyl-2-oxo-2,3-dihydropyrido[2,3-b]pyrazin-4(1H)-yl]piperidin-1-carboxylat;
*tert-*Butyl-4-[(3*R*)-6-{[3-(dimethylsulfamoyl)phenyl]amino}-1,3-dimethyl-2-oxo-2,3-dihydropyrido [2,3-b]pyrazin-4(1H)-yl]piperidin-1-carboxylat;
*tert*-Butyl-4-[(3*R*)-1,3-dimethyl-6-({3-[(4-methylpiperazin-1-yl)sulfonyl]phenyl}amino)-2-oxo-2,3-dihydropyrido [2,3-b]pyrazin-4(1H)-yl]piperidin-1-carboxylat;
5-{[(3*R*)-1,3-Dimethyl-2-oxo-4-(tetrahydro-2H-pyran-4-yl)-1,2,3,4-tetrahydropyrido[2,3-b]pyrazin-6-yl]amino}-2-methylbenzolsulfonamid;
1,3-Dimethyl-4-(3-methylphenyl)-6-{[3-(morpholin-4-ylsulfonyl)phenyl]amino}-3,4-dihydropyrido [2,3-b]pyrazin-2(1H)-on;
3-{[1,3-Dimethyl-4-(3-methylphenyl)-2-oxo-1,2,3,4-tetrahydro-pyrido[2,3-b]pyrazin-6-yl]amino}-*N*-(1-methylpiperidin-4-yl)benzolsulfonamid;
*tert*-Butyl-4-[(3*R*)-1,3-dimethyl-2-oxo-6-[(3-{[4-(2,2,2-trifluorethyl) piperazin-1-yl]sulfonyl} phenyl)amino]-2,3-dihydropyrido[2,3-b]pyrazin-4(1H)-yl]piperidin-1-carboxylat;
*tert*-Butyl-4-{4-[1,3-dimethyl-6-({3-[(4-methylpiperazin-1-yl)sulfonyl]phenyl}amino)-2-oxo-2,3-dihydropyrido[2,3-b]pyrazin-4(1H)-yl]phenyl}piperazin-1-carboxylat;
*tert-*Butyl-4-[(2*R*)-7-{[3-(dimethylsulfamoyl)phenyl]amino}-2,4-dimethyl-3-oxo-3,4-dihydrochinoxalin-1 (2H)-yl]piperidin-1-carboxylat;
3-{[(3*R*)-4-Benzyl-1,3-dimethyl-2-oxo-1,2,3,4-tetrahydrochinoxalin-6-yl]amino}-*N,N-*dimethylbenzolsulfonamid;
*tert*-Butyl-4-[(2*R*)-7-{[3-(dimethylsulfamoyl)phenyl](methyl)amino}-2,4-dimethyl-3-oxo-3,4-dihydrochinoxalin-1 (2H)-yl]piperidin-1-carboxylat;
(3*R*)-1,3-Dimethyl-6-({3-[(4-methylpiperazin-1-yl)sulfonyl]phenyl}amino)-4-(tetrahydro-2H-pyran-4-yl)-3,4-dihydrochinoxalin-2(1H)-on;
3-{[(3*R*)-4-Benzyl-1,3-dimethyl-2-oxo-1,2,3,4-tetrahydropyrido[2,3-b]pyrazin-6-yl]amino}-*N-*{trans-4-[4-(cyclopropyl-methyl)piperazin-1-yl]cyclohexyl}benzamid;
3-{[(3*R*)-4-Benzyl-1,3-dimethyl-2-oxo-1,2,3,4-tetrahydropyrido[2,3-b]pyrazin-6-yl]amino}-*N*-(1-methylpiperidin-4-yl)benzamid;
3-{[(3*R*)-4-Benzyl-1,3-dimethyl-2-oxo-1,2,3,4-tetrahydropyrido[2,3-b]pyrazin-6-yl]amino}-*N*-[2-(4-methylpiperazin-1-yl)ethyl]benzamid;
(3*R*)-4-Benzyl-1,3-dimethyl-6-({3-[(4-methylpiperazin-1-yl)carbonyl]phenyl}amino)-3,4-dihydropyrido[2,3-b]pyrazin-2(1H)-on;
(3*R*)-4-Benzyl-6-({3-[(4-isopropylpiperazin-1-yl)carbonyl]phenyl}amino)-1,3-dimethyl-3,4-dihydropyrido[2,3-b]pyrazin-2(1H)-on;
(3*R*)-4-Isopropyl-1,3-dimethyl-6-({3-[(4-methylpiperazin-1-yl)carbonyl]phenyl}amino)-3,4-dihydropyrido[2,3-b]pyrazin-2(1H)-on;
*tert*-Butyl-4-[(3*R*)-1,3-dimethyl-6-({3-[(4-methylpiperazin-1-yl)carbonyl]phenyl}amino)-2-oxo-2,3-dihydro-pyrido [2,3-b]pyrazin-4(1H)-yl]piperidin-1-carboxylat;
*N*-{*trans*-4-[4-(Cyclopropylmethyl)piperazin-1-yl]cyclohexyl}-3-{[(3*R*)-4-isopropyl-1,3-dimethyl-2-oxo-1,2,3,4-tetrahydropyrido[2,3-b]pyrazin-6-yl]amino} benzamid;
3-{[(3*R*)-1,3-Dimethyl-2-oxo-4-(tetrahydro-2H-pyran-4-yl)-1,2,3,4-tetrahydropyrido[2,3-b]pyrazin-6-yl]amino}-*N*-{4-[(1-methylpiperidin-4-yl)amino]cyclohexyl}benzamid;
5-{[(3*R*)-1,3-Dimethyl-2-oxo-4-(tetrahydro-2H-pyran-4-yl)-1,2,3,4-tetrahydropyrido[2,3-b]pyrazin-6-yl]amino}-2-methoxy-*N*-(1-methylpiperidin-4-yl)benzamid;
*N*-{*trans*-4-[4-(Cyclopropylmethyl)piperazin-1-yl]cyclohexyl}-5-{[(3*R*)-1,3-dimethyl-2-oxo-4-(tetrahydro-2H-pyran-4-yl)-1,2,3,4-tetrahydropyrido [2,3-b]pyrazin-6-yl]amino}-2-methoxybenzamid;
(3*R*)-6-({4-Methoxy-3-[(4-methylpiperazin-1-yl)carbonyl]phenyl}amino)-1,3-dimethyl-4-(tetrahydro-2H-pyran-4-yl)-3,4-dihydropyrido [2,3-b]pyrazin-2(1H)-on;
3-{[(3*R*)-4-Cyclohexyl-1,3-dimethyl-2-oxo-1,2,3,4-tetrahydro-pyrido[2,3-b]pyrazin-6-yl]amino}-*N*-[2-(4-methylpiperazin-1-yl)ethyl]benzamid;
(3*R*)-4-Cyclohexyl-6-(3-[(4-isopropylpiperazin-1-yl)carbonyl]phenyl}amino)-1,3-dimethyl-3,4-dihydropyrido[2,3-b]pyrazin-2(1H)-on;
3-{[(3*R*)-4-Cyclohexyl-1,3-dimethyl-2-oxo-1,2,3,4-tetrahydro-pyrido[2,3-b]pyrazin-6-yl]amino}-*N*-(4-hydroxycyclohexyl)benzamid;
*N*-{*trans*-4-[4-(Cyclopropylmethyl)piperazin-1-yl]cyclohexyl}-3-{[(3*R*)-1,3-dimethyl-2-oxo-4-(tehahydro-2H-pyran-4-yl)-1,2,3,4-tetrahydropyudo[2,3-b]pyrazin-6-yl]amino}-4-methoxybenzamid;
(3*R*)-6-[(3-{[4-(Cyclopropylmethyl)piperazin-1-yl]carbonyl}phenyl)amino]-4-isopropyl-1,3)-dimethyl-3,4-dihydropyrido [2,3-b]pyrazin-2(1H)-on;
*N*-[4-(4,4-Difluorpiperidin-1-yl)cyclohexyl]-5-{[(3*R*)-1,3-dimethyl-2-oxo-4-(tetrahydro-2H-pyran-4-yl)-1,2,3,4-tetrahydro-pyrido [2,3-b]pyrazin-6-yl]amino}-2-methoxy-benzamid;
*N*-[*cis*-4-(4-Cyclopropylpiperazin-1-yl)cyclohexyl]-3-{[(3*R*)-1,3-dimethyl-2-oxo-4-(tetrahydro-2H-pyran-4-yl)-1,2,3,4-tetrahydro-pyrido[2,3-b]pyrazin-6-yl]amino}-4-methoxy-benzamid;
*N*-{*trans*-4-[4-(Cyclopropylmethyl)piperazin-1-yl]cyclohexyl}-3-{[(3*R*)-1,3-dimethyl-2-oxo-4-(tetrahydro-2H-pyran-4-yl)-1,2,3,4-tetrahydropyrido [2,3-b]pyrazin-6-yl]amino}-2-methoxybenzamid;
(3*R*)-6-({2-Methoxy-3-[(4-methylpiperazin-1-yl)carbonyl]phenyl}amino)-1,3-dimethyl-4-(tetrahydro-2H-pyran-4-yl)-3,4-dihydropyrido [2,3-b]pyrazin-2(1H)-on;
*N*-{*trans*-4-[4-(Cyclopropylmethyl)piperazin-1-yl]cyclohexyl}-3-{[(3*R*)-4-(4-fluorphenyl)-1,3-dimethyl-2-oxo-1,2,3,4-tetrahydropyrido [2,3-b]pyrazin-6-yl]amino}benzamid;
3-{[(3*R*)-4-Isopropyl-1,3-dimethyl-2-oxo-1,2,3,4-tetrahydropyrido[2,3-b]pyrazin-6-yl]amino}-*N-*(1-methylpiperidin-4-yl)benzamid;
3-{[(3*R*)-4-Cyclohexyl-1,3-dimethyl-2-oxo-1,2,3,4-tetrahydropyrido[2,3-b]pyrazin-6-yl]amino}-*N*-(1-methylpiperidin-4-yl)benzamid;
3- {[(3*R*)-4-(4,4-Dimethylcyclohexyl)-1,3-dimethyl-2-oxo-1,2,3,4-tetrahydropyrido[2,3-b]pyrazin-6-yl]amino}-*N*-(1-methylpiperidin-4-yl)benzamid;
*N*-[2-(Dimethylamino)ethyl]-3-{[(3R)-4-isopropyl-1,3-dimethyl-2-oxo-1,2,3,4-tetrahydropyrido[2,3-b]pyrazin-6-yl]amino}benzamid;
3-{[4-(2-Methoxyethyl)-1,3-dimethyl-2-oxo-1,2,3,4-tetrahydropyrido[2,3-b]pyrazin-6-yl]amino}-*N*-(1-methylpiperidin-4-yl)benzamid;
3-{[(3*R*)-4-Cyclohexyl-1,3-dimethyl-2-oxo-1,2,3,4-tetrahydropyrido[2,3-b]pyrazin-6-yl]amino}-*N*-{*trans*-4-[4-(cyclopropylmethyl)piperazin-1-yl]cyclohexyl}benzamid;
*tert*-Butyl-4-[(3*R*)-1,3-dimethyl-6-({3-[(1-methylpiperidin-4-yl)carbamoyl]phenyl}amino)-2-oxo-2,3-dihydropyrido[2,3-b]pyrazin-4(1H)-yl]piperidin-1-carboxylat;
*N*-{*trans*-4-[4-(Cyclopropylmethyl)piperazin-1-yl]cyclohexyl}-3-{[(3*R*)-4-(2-methoxyethyl)-1,3-dimethyl-2-oxo-1,2,3,4-tetrahydropyrido[2,3-b]pyrazin-6-yl]amino}benzamid;
*tert*-Butyl-4-[(3*R*)-6-{[3-({*trans*-4-[4-(cyclopropyl-methyl)piperazin-1-yl]cyclohexyl}carbamoyl)phenyl]amino}-1,3-dimethyl-2-oxo-2,3-dihydropyrido[2,3-b]pyrazin-4(1H)-yl]piperidin-1-carboxylat;
3-{[(3*R*)-1,3-Dimethyl-2-oxo-4-(tetrahydro-2H-pyran-4-yl)-1,2,3,4-tetrahydropyrido[2,3-b]pyrazin-6-yl]amino} -2-methoxy-*N*-(1-methyl-piperidin-4-yl)benzamid;
3-[(1,3-Dimethyl-2-oxo-4-phenyl-1,2,3,4-tetrahydropyrido[2,3-b]pyrazin-6-yl)amino]-*N*-(1-methylpiperidin-4-yl)benzamid;
*tert*-Butyl-4-[(2*R*)-7-{[3-(dimethylcarbamoyl)phenyl]amino}-2,4-dimethyl-3-oxo-3,4-dihydrochinoxalin-1 (2H)-yl]piperidin-1-carboxylat;
(3*R*)-1,3-Dimethyl-6-({3-[(4-methylpiperazin-1-yl)carbonyl]phenyl}amino)-4-(tetrahydro-2H-pyran-4-yl)-3,4-dihydrochinoxalin-2(1H)-on;
3-{[(3*R*)-4-Benzyl-1,3-dimethyl-2-oxo-1,2,3,4-tetrahydrochinoxalin-6-yl]amino}-*N,N-*dimethylbenzamid;
*N-*{*trans*-4-[4-(Cyclopropylmethyl)piperazin-1-yl]cyclohexyl}-3-{[(3*R*)-1,3-dimethyl-2-oxo-4-(tetrahydro-2H-pyran-4-yl)-1,2,3,4-tetrahydrochinoxalin-6-yl]amino}benzamid;
3-{[(3*R*)-1,3-Dimethyl-2-oxo-4-(tetrahydro-2H-pyran-4-yl)-1,2,3,4-tetrahydrochinoxalin-6-yl]amino}-*N*-(1-methylpiperidin-4-yl)benzamid;
3-[(4-Benzyl-1,3-dimethyl-2-oxo-1,2,3,4-tetrahydrochinoxalin-6-yl)(methyl)amino]-*N,N-*dimethylbenzolsulfonamid;
(3*R*)-4-(2-Methoxyethyl)-1,3-dimethyl-6-{[3-(morpholin-4-ylsulfonyl) phenyl]amino}-3,4-dihydropyrido[2,3-b]pyrazin-2(1H)-on;
(3*S*)-4-(2-Methoxyethyl)-1,3-dimethyl-6-{[3-(morpholin-4-ylsulfonyl)phenyl]amino}-3,4-dihydropyrido[2,3-b]pyrazin-2(1H)-on;
(3*R*)-4-(2-Methoxyethyl)-1,3-dimethyl-6-({3-[(4-methylpiperazin-1-yl)sulfonyl]phenyl}amino)-3,4-dihydropyrido [2,3-b]pyrazin-2(1H)-on;
(3*R*)-4-(4-Fluorphenyl)-1,3-dimethyl-6-{[3-(morpholin-4-ylsulfonyl)phenyl]amino}-3,4-dihydropyrido[2,3-b]pyrazin-2(1H)-on;
3-{[(3*R*)-4-(4-Fluorphenyl)-1,3-dimethyl-2-oxo-1,2,3,4-tetrahydropyrido[2,3-b]pyrazin-6-yl]amino}-*N*-(1-methylpiperidin-4-yl)benzamid;
3-{[(3*S*)-4-(4-Fluorphenyl)-1,3-dimethyl-2-oxo-1,2,3,4-tetrahydropyrido[2,3-b]pyrazin-6-yl]amino}-*N*-(1-methylpiperidin-4-yl)benzamid;
(3*R*)-4-(2-Methoxyethyl)-1,3-dimethyl-6-({3-[(4-methylpiperazin-1-yl)carbonyl]phenyl}amino)-3,4-dihydropyrido[2,3-b]pyrazin-2(1H)-on;
(3*S*)-4-(2-Methoxyethyl)-1,3-dimethyl-6-({3-[(4-methylpiperazin-1-yl)carbonyl]phenyl}amino)-3,4-dihydropyrido [2,3-b]pyrazin-2(1H)-on;
3-{[(3*R*)-4-(2-Methoxy-ethyl)-1,3-dimethyl-2-oxo-1,2,3,4-tetrahydropyrido [2,3-b]pyrazin-6-yl] amino}-*N*-(1-methyl piperidin-4-yl)benzamid;
3-{[(3*S*)-4-(2-Methoxy-ethyl)-1,3-dimethyl-2-oxo-1,2,3,4-tetrahydropyrido [2,3-b]pyrazin-6-yl] amino}-*N-*(1-methyl piperidin-4-yl)benzamid;
3-{[(3*R*)-1,3-Dimethyl-2-oxo-4-phenyl-1,2,3,4-tetrahydropyrido[2,3-b]pyrazin-6-yl]amino}*-N-*(1-methylpiperidin-4-yl)benzamid;
(3*R*)-6-{[3-(2-Azaspiro[3.3]hept-2-ylsulfonyl)phenyl]amino}-1,3-dimethyl-4-(piperidin-4-yl)-3,4-dihydropyrido[2,3-b]pyrazin-2(1H)-on;
3-{[(3*R*)-1,3-Dimethyl-2-oxo-4-(piperidin-4-yl)-1,2,3,4-tetrahydropyrido[2,3-b]pyrazin-6-yl]amino}-*N,N*-dimethyl benzolsulfonamid;
3-{[(3*R*)-1,3-Dimethyl-2-oxo-4-(piperidin-4-yl)-1,2,3,4-tetrahydropyrido [2,3-b]pyrazin-6-yl]amino}-*N*-(1-methyl piperidin-4-yl)benzol sulfonamid;
(3*R*)-1,3-Dimethyl-6-({3-[(4-methylpiperazin-1-yl)sulfonyl]phenyl}amino)-4-(piperidin-4-yl)-3,4-dihydropyrido[2,3-b]pyrazin-2(1H)-on;
(3*R*)-1,3-Dimethyl-4-(piperidin-4-yl)-6-[(3-{[4-(2,2,2-trifluorethyl)piperazin-1-yl]sulfonyl} phenyl)amino]-3,4-dihydropyrido [2,3-b]pyrazin-2(1H)-on;
*N*-{*trans*-4-[4-(Cyclopropylmethyl)piperazin-1-yl]cyclohexyl}-3-{[(3*R*)-1,3-dimethyl-2-oxo-4-(piperidin-4-yl)-1,2,3,4-tetra-hydropyrido[2,3-b]pyrazin-6-yl]amino} benzolsulfonamid;
*N*-[2-(Dimethylamino)ethyl]-3-{[(3*R*)-1,3-dimethyl-2-oxo-4-(piperidin-4-yl)-1,2,3,4-tetrahydropyrido[2,3-b]pyrazin-6-yl]amino}benzolsulfonamid;
3-{[(3*R*)-1,3-Dimethyl-2-oxo-4-(piperidin-4-yl)-1,2,3,4-tetrahydro-chinoxalin-6-yl]amino}-*N,N-*dimethylbenzol-sulfonamid;
(3*R*)-1,3-Dimethyl-6-({3-[(4-methylpiperazin-1-yl)carbonyl]phenyl} amino)-4-(piperidin-4-yl)-3,4-dihydropyrido[2,3-b]pyrazin-2(1H)-on;
N-{*trans*-4-[4-(Cyclopropylmethyl)piperazin-1-yl]cyclohexyl}-3-{[(3*R*)-1,3-dimethyl-2-oxo-4-(piperidin-4-yl)-1,2,3,4-tetrahydro-pyrido[2,3-b]pyrazin-6-yl]amino}benzamid;
3-{[(3*R*)-1,3-Dimethyl-2-oxo-4-(piperidin-4-yl)-1,2,3,4-tetrahydro-pyrido[2,3-b]pyrazin-6-yl]amino}-*N*-(1-methylpiperidin-4-yl)benzamid;
3-{[(3*R*)-1,3-Dimethyl-2-oxo-4-(piperidin-4-yl)-1,2,3,4-tetrahydro-chinoxalin-6-yl]amino}-*N,N-*dimethylbenzamid;
(3*R*)-1,3-Dimethyl-6-{[3-(5-methyl-1,3,4-oxadiazol-2-yl)phenyl]amino}-4-(tetrahydro-2H-pyran-4-yl)-3,4-dihydropyrido[2,3-b]pyrazin-2(1H)-on;
4-(4-Fluorphenyl)-1,3-dimethyl-6-{[3-(morpholin-4-ylsulfonyl)phenyl]amino}-3,4-dihydropyrido[2,3-b]pyrazin -2(1H)-on;
3-{[4-(4-Fluorphenyl)-1,3-dimethyl-2-oxo-1,2,3,4-tetrahydropyrido[2,3-b]pyrazin-6-yl]amino}-*N-*(1-methylpiperidin-4-yl)benzamid
   und
4-(2-Methoxyethyl)-1,3-dimethyl-6-({3-[(4-methylpiperazin-1-yl)carbonyl]phenyl}amino)-3,4-dihydropyrido[2,3-b]pyrazin-2(1H)-on.

### Definitionen:

Unter C₁-C₆-Alkyl-, bzw. einer C₁-C₆-Alkyl-Gruppe ist ein linearer oder verzweigter, gesättigter, monovalenter Kohlenwasserstoffrest zu verstehen, wie z.B. ein Methyl-, Ethyl-, Propyl-, Butyl-, Pentyl-, Hexyl-, *iso*-Propyl-, *iso*-Butyl-, *sec*-Butyl, *tert*-Butyl*-, iso*-Pentyl-, 2-Methylbutyl-, 1-Methylbutyl-, 1-Ethylpropyl-, 1,2-Dimethylpropyl-, *neo*-Pentyl-, 1,1-Dimethylpropyl-, 4-Methylpentyl-, 3-Methylpentyl-, 2-Methylpentyl-, 1-Methylpentyl-, 2-Ethylbutyl-, 1-Ethylbutyl-, 3,3-Dimethylbutyl-, 2,2-Dimethylbutyl-, 1,1-Dimethylbutyl-, 2,3-Dimethylbutyl-, 1,3-Dimethylbutyl- oder 1,2-Dimethylbutyl-Rest.
Vorzugsweise ist unter C₁-C₆-Alkyl- bzw. einer C₁-C₆-Alkyl-Gruppe C₁-C₄-Alkylbeziehungsweise C₂-C₅-Alkyl-, besonders bevorzugt C₁-C₃-Alkyl- beziehungsweise ein Methyl-, Ethyl-, Propyl- oder Isopropyl-Rest zu verstehen.

Unter C₂-C₆-Alkenyl-, bzw. einer C₂-C₆-Alkenyl-Gruppe ist ein linearer oder verzweigter, monovalenter Kohlenwasserstoffrest mit einer oder zwei C=C-Doppelbindungen zu verstehen, wie z.B. ein Ethenyl-, (*E*)-Prop-2-enyl-, (*Z*)-Prop-2-enyl-, Allyl- (Prop-1-enyl-), Allenyl- Buten-1-yl-, oder Buta-1,3-dienyl-Rest. Bevorzugt sind C₃-C₆-Alkenyl- und C₂-C₄-Alkenyl-; besonders bevorzugt sind Ethenyl- und Allyl-.

Unter C₂-C₆-Alkinyl-, bzw. einer C₂-C₆-Alkinyl-Gruppe ist ein linearer oder verzweigter, monovalenter Kohlenwasserstoffrest mit einer C≡C-Dreifachbindung zu verstehen, wie z.B. ein Ethinyl-, Propargyl- (Prop-1-inyl-), oder Butin-1-yl-Rest. Bevorzugt sind C₃-C₆-Alkinyl- und C₂-C₄-Alkinyl-; besonders bevorzugt sind Ethinyl und Propargyl.

Unter C₁-C₄-Alkoxy-, bzw. einer C₁-C₄-Alkoxy-Gruppe ist ein linearer oder verzweigter, gesättigter Alkyletherrest -O-Alkyl zu verstehen, wie z.B. ein Methoxy-, Ethoxy-, n-Propoxy-, Isopropoxy- oder *tert*.-Butoxy-Rest.
Vorzugsweise ist unter C₁-C₄-Alkoxy-, bzw. einer C₁-C₄-Alkoxy-Gruppe C₁-C₃-Alkoxy-, besonders bevorzugt ein Methoxy- oder Ethoxy-Rest zu verstehen.

Unter C₁-C₄-Alkylthio-, bzw. einer C₁-C₄-Alkylthio-Gruppe ist ein linearer oder verzweigter, gesättigter Alkylthioetherrest -S-Alkyl zu verstehen, wie z.B. ein Methylthio-, Ethylthio-, n-Propylthio-, Isopropylthio-, oder tert.-Butylthio-Rest.
Vorzugsweise ist unter C₁-C₄-Alkylthio-, bzw. einer C₁-C₄-Alkylthio-Gruppe C₁-C₃-Alkylthio-, besonders bevorzugt ein ein Methylthio- oder Ethylthio-Rest zu verstehen.

Unter einem Heteroatom ist zu verstehen -O-, NH-, =N- oder -S-. Das Heteroatom -NH- kann gegebenenfalls substituiert sein durch C₁-C₃-Alkyl, C₁-C₃-Alkylcarbonyl, C₁-C₄-Alkoxycarbonyl, oder -S(=O)₂-C₁-C₃-Alkyl.
Bevorzugt sind ein Sauerstoff- oder ein Stickstoffatom.

Unter Oxo, beziehungsweise einem Oxo-Substituenten ist ein doppelt gebundenes Sauerstoff-Atom =O zu verstehen. Oxo kann an Atome geeigneter Valenz gebunden sein, beispielsweise an ein gesättigtes Kohlenstoff-Atom oder an Schwefel.
Bevorzugt ist die Bindung an Kohlenstoff unter Bildung einer Carbonyl-Gruppe -(C=O)-. Bevorzugt ist weiterhin die Bindung zweier doppelt gebundener Sauerstoffatome an Schwefel unter Bildung einer Sulfonyl-Gruppe -(S=O)₂-.

Unter Halogen ist Fluor, Chlor Brom oder Iod zu verstehen.

Unter einem Halogen-C₁-C₄-Alkylrest beziehungsweise Halogen-C₁-C₄-Alkyl- ist ein C₁-C₄-Alkylrest, substituiert mit mindestens einem Halogensubstituenten, vorzugsweise mit mindestens einem Fluorsubstituenten, zu verstehen.
Bevorzugt sind Fluor-C₁-C₃-Alkyl-Reste, beispielsweise Difluormethyl-, Trifluormethyl-, 2,2,2-Trifluorethyl- oder Pentafluorethyl-.
Besonders bevorzugt sind perfluorierte Alkylreste wie Trifluormethyl- oder Pentafluorethyl-.

Unter Phenyl-C₁-C₃-alkyl- ist eine Gruppe zu verstehen, die zusammengesetzt ist aus einem gegebenenfalls substituierten Phenylrest und einer C₁-C₃-Alkyl-Gruppe, und die über die C₁-C₃-AlkylGruppe an den Rest des Moleküls gebunden ist.

Unter einem Halogen-C₁-C₄-Alkoxyrest beziehungsweise Halogen-C₁-C₄-Alkoxy- ist ein C₁-C₄-Alkoxyrest, substituiert mit mindestens einem Halogensubstituenten, vorzugsweise mit mindestens einem Fluorsubstituenten, zu verstehen.
Bevorzugt sind Fluor-C₁-C₃-Alkoxy-Reste, beispielsweise Difluormethoxy-, Trifluormethoxy- oder 2,2,2-Trifluorethoxy-.

Unter einem Halogen-C₁-C₄-Alkylthio-Rest beziehungsweise Halogen-C₁-C₄-Alkylthio- ist ein C₁-C₄-Alkylthio-Rest substituiert mit mindestens einem Halogensubstituenten, vorzugsweise mit mindestens einem Fluorsubstituenten, zu verstehen.
Bevorzugt sind Fluor-C₁-C₃-Alkylthio-Reste, insbesondere Trifluormethylthio-.

Unter einem C₁-C₄-Alkylcarbonylrest ist eine C₁-C₄-Alkyl-C(=O)-Gruppe zu verstehen. Bevorzugt ist C₁-C₃-Alkylcarbonyl-, besonders bevorzugt Acetyl- oder Propanoyl-.

Unter einem C₁-C₄-Alkoxycarbonylrest ist eine C₁-C₄-Alkoxy-C(=O)-Gruppe zu verstehen. Bevorzugt ist Methoxycarbonyl-, Ethoxycarbonyl-, oder *tert*.-Butoxycarbonyl-.

Unter einem C₁-C₄-Alkoxy-C₁-C₄-Alkylrest ist ein mit C₁-C₄-Alkoxy substituierter C₁-C₄-Alkylrest zu verstehen, wie z. B.Methoxymethyl-, Methoxyethyl-, Ethoxymethyl- und Ethoxyethyl-.

Unter Aryl ist ein aus Kohlenstoffatomen aufgebautes ungesättigtes vollständig konjugiertes System zu verstehen, welches über 3, 5 oder 7 konjugierte Doppelbindungen verfügt, wie z.B. Phenyl-, Naphthyl- oder Phenantryl-. Bevorzugt ist Phenyl.

Unter Heteroaryl- sind Ringsysteme zu verstehen, die über ein aromatisch konjugiertes Ringsystem verfügen sowie mindestens ein und bis zu fünf Heteroatomen wie voranstehend definiert enthalten, enthalten. Diese Ringsysteme können über 5, 6 oder 7 Ringatome verfügen, oder im Fall von kondensierten beziehungsweise benzokondensierten Ringsystemen auch über Kombinationen aus 5- und 6-gliedrigen Ringsystemen, 5- und 5-gliedrigen Ringsystemen oder auch aus 6- und 6-gliedrigen Ringsystemen verfügen. Als Beispiel seien aufgeführt Ringsysteme wie Pyrrolyl-, Pyrazolyl-, Imidazolyl-, Triazolyl-, Tetrazolyl-, Furanyl-, Thienyl-, Oxazolyl-, Thiazolyl-, Isoxazolyl-, Oxadiazolyl-, Thiadiazolyl-, Pyridinyl-, Pyrimidinyl-, Pyrazinyl-, Triazinyl-, Oxazinyl-, Indolyl-, Benzimidazolyl-, Indazolyl-, Benzotriazolyl-, Benzothiazolyl-, Benzoxazolyl-, Benzofuranyl-, Benzothienyl-, Chinolinyl-, Isochinolinyl-, Cinnolinyl-, Chinazolinyl-, Chinoxalinyl-, Imidazopyridylyl- oder auch Benzoxazinyl-.
Bevorzugt ist 5- bis 6-gliedriges, monocyclisches Heteroaryl-, beispielsweise Pyrrolyl-, Pyrazolyl-, Imidazolyl-, Triazolyl-, Tetrazolyl-, Furanyl-, Thienyl-, Oxazolyl-, Thiazolyl-, Isoxazolyl-, Oxadiazolyl-, Thiadiazolyl-, Pyridinyl-, Pyrimidinyl-, Pyrazinyl-, Triazinyl-.

Unter C₃-C₆-Cycloalkyl, C₃-C₈-Cycloalkyl, bzw. C₅-C₈-Cycloalkyl ist ein monocyclisches, ausschließlich aus Kohlenstoffatomen aufgebautes, gesättigtes Ringsystem mit 3 bis 6, 3 bis 8 Atomen, bzw. 5 bis 8 Atomen zu verstehen. Beispiele sind Cyclopropyl-, Cyclobutyl-, Cyclopentyl-, Cyclohexyl-, Cycloheptyl- oder Cyclooctyl-.
Unter Cycloalkylen, beispielsweise C₃-C₆-Cycloalkylen, ist ein bivalenter Cycloalkyl-Rest zu verstehen; bevorzugt sind solche C₃-C₆-Cycloalkylen-Systeme, bei denen beide Bindungen vom selben Ringkohlenstoffatom ausgehen.

Unter C₄-C₆-Cycloalkenyl, C₄-C₈-Cycloalkenyl, bzw. C₅-C₈-Cycloalkenyl ist ein monocyclisches, ausschließlich aus Kohlenstoffatomen aufgebautes, ein- oder mehrfach ungesättigtes, nicht-aromatisches Ringsystem mit 3 bis 6, 3 bis 8 Atomen, bzw. 5 bis 8 Atomen zu verstehen. Beispiele sind Cyclobuten-1-yl-, Cyclopenten-1-yl-, Cyclohexen-2-yl-, Cyclohexen-1-yl- oder Cycloocta-2,5-dienyl-.

Unter Heterocycloalkyl- ist ein 4- bis 8-gliedriges monocyclisches, gesättigtes Ringsystem zu verstehen, welches über 1 bis 3 Heteroatome wie voranstehend definiert, in beliebiger Kombination verfügt. Bevorzugt sind 4-7-gliedrige Heterocycloalkyl-Gruppen, besonders bevorzugt sind 5-6-gliedrige Heterocycloalkyl-Gruppen. Beispielhaft zu nennen sind Pyrrolidinyl-, Piperidinyl-, Tetrahydrofuranyl-, Tetrahydropyranyl-, Oxetanyl-, Azetidinyl-, Azepanyl-, Morpholinyl-, Thiomorpholinyl- oder Piperazinyl-.

Unter Heterocycloalkenyl ist ein 4- bis 8-gliedriges monocyclisches, ein- oder mehrfach ungesättigtes, nicht-aromatisches Ringsystem zu verstehen, welches über 1 bis 3 Heteroatome wie voranstehend definiert, in beliebiger Kombination verfügt. Bevorzugt sind 4-7-gliedrige Heterocycloalkenyl-Gruppen, besonders bevorzugt sind 5-6-gliedrige Heterocycloalkenyl-Gruppen. Beispielhaft zu nennen sind 4H-Pyranyl-, 2H-Pyranyl-, 2,5-Dihydro-1H-pymolyl-, [1,3]Dioxolyl, 4H-[1,3,4]Thiadiazinyl, 2,5-Dihydrofuranyl, 2,3-Dihydrofuranyl, 2,5-Dihydrothiophenyl-, 2,3-Dihydrothiophenyl-, 4,5-Dihydrooxazolyl-, or 4H-[1,4]Thiazinyl-.

Unter C₅-C₁₁ -Spirocycloalkyl bzw. C₅-C₁₁-Heterospirocycloalkyl mit einem Ersatz von 1-4 Kohlenstoffatomen durch Heteroatome wie voranstehend definiert in beliebiger Kombination, ist eine Fusion aus zwei gesättigten Ringsystemen zu verstehen, die sich ein gemeinsames Atom teilen. Beispiele sind Spiro[2.2]pentyl-, Spiro[2.3]hexyl-, Azaspiro[2.3]hexyl-, Spiro[3.3]heptyl-, Azaspiro[3.3]heptyl-, Oxaazaspiro[3.3]heptyl-, Thiaazaspiro[3.3]heptyl-, Oxaspiro[3.3]heptyl-, Oxazaspiro[5.3]nonyl-, Oxazaspiro[4.3]octyl-, Oxazaspiro[5.5]undecyl-, Diazaspiro[3.3]heptyl-, Thiazaspiro[3.3]heptyl-, Thiazaspiro[4.3]octyl-, Azaspiro[5.5]decyl-, sowie die weiteren homologen Spiro[3.4]-, Spiro[4.4]-, Spiro[5.5]-, Spiro[6.6]-, Spiro[2.4]-, Spiro[2.5]-, Spiro[2.6]-, Spiro[3.5]-, Spiro[3.6]-, Spiro[4.5]-, Spiro[4.6]- und Spiro[5.6]-Systeme inklusive der durch Heteroatome modifizierten Varianten gemäß Definition. Bevorzugt ist C₆-C₈-Heterospirocycloalkyl.

Unter C₆-C₁₂-Bicycloalkyl bzw. C₆-C₁₂-Heterobicycloalkyl mit einem Ersatz von 1-4 Kohlenstoffatomen durch Heteroatome wie voranstehend definiert in beliebiger Kombination, ist eine Fusion aus zwei gesättigten Ringsystemen zu verstehen, die sich gemeinsam zwei direkt benachbarte Atome teilen. Beispiele sind Bicyclo[2.2.0]hexyl-, Bicyclo[3.3.0]octyl-, Bicyclo[4.4.0]decyl-, Bicyclo[5.4.0]undecyl-, Bicyclo[3.2.0]heptyl-, Bicyclo[4.2.0]octyl-, Bicyclo[5.2.0]nonyl-, Bicyclo[6.2.0]decyl-, Bicyclo[4.3.0]nonyl-, Bicyclo[5.3.0]decyl-, Bicyclo[6.3.0]undecyl- und Bicyclo[5.4.0]undecyl-, inklusive der durch Heteroatome modifizierten Varianten wie z.B. Azabicyclo[3.3.0]octyl-, Azabicyclo[4.3.0]nonyl-, Diazabicyclo[4.3.0]nonyl-, Oxazabicyclo[4.3.0]nonyl-, Thiazabicyclo[4.3.0]nonyl- oder Azabicyclo[4.4.0]decyl- sowie die weiteren möglichen Kombinationen gemäß Definition. Bevorzugt ist C₆-C₁₀-Heterobicycloalkyl.

Unter einem verbrückten C₆-C₁₂-Ringsystem wie verbrücktes C₆-C₁₂-Cycloalkyl oder verbrücktes C₆-C₁₂-Heterocycloalkyl ist eine Fusion aus mindestens zwei gesättigten Ringen zu verstehen, die sich zwei Atome teilen, die nicht direkt benachbart zueinander sind. Dabei kann sowohl ein verbrücktes Carbocyclus (verbrücktes Cycloalkyl) entstehen als auch ein verbrückter Heterocyclus (verbrücktes Heterocycloalkyl) mit einem Ersatz von 1-4 Kohlenstoffatomen durch Heteroatome wie voranstehend definiert in beliebiger Kombination. Beispiele sind Bicyclo[2.2.1]heptyl-, Azabicyclo[2.2.1]heptyl-, Oxazabicyclo[2.2.1]heptyl-, Thiazabicyclo[2.2.1]heptyl-, Diazabicyclo[2.2.1]heptyl-, Bicyclo[2.2.2]octyl-, Azabicyclo[2.2.2]octyl-, Diazabicyclo[2.2.2]octyl-, Oxazabicyclo[2.2.2]octyl-, Thiazabicyclo[2.2.2]octyl-, Bicyclo[3.2.1]octyl-, Azabicyclo[3.2.1]octyl-, Diazabicyclo[3.2.1]octyl-, Oxazabicyclo[3.2.1]octyl-, Thiazabicyclo[3.2.1]octyl-, Bicyclo[3.3.1]nonyl-, Azabicyclo[3.3.1]nonyl-, Diazabicyclo[3.3.1]nonyl-, Oxazabicyclo[3.3.1]nonyl-, Thiazabicyclo[3.3.1]nonyl-, Bicyclo[4.2.1]nonyl-, Azabicyclo[4.2.1]nonyl-, Diazabicyclo[4.2.1]nonyl-, Oxazabicyclo[4.2.1]nonyl-, Thiazabicyclo[4.2.1]nonyl-, Bicyclo[3.3.2]decyl-, Azabicyclo[3.3.2]decyl-, Diazabicyclo[3.3.2]decyl-, Oxazabicyclo[3.3.2]decyl-, Thiazabicyclo[3.3.2]decyl- oder Azabicyclo[4.2.2]decyl- sowie die weiteren möglichen Kombinationen gemäß Definition. Bevorzugt ist verbrücktes C₆-C₁₀-Heterocycloalkyl.

Erfindungsgemäße Verbindungen sind die Verbindungen der allgemeinen Formel (I) und deren Salze, Solvate und Solvate der Salze, die von der allgemeinen Formel (I) umfassten Verbindungen der nachfolgend genannten Formeln und deren Salze, Solvate und Solvate der Salze sowie die von der allgemeinen Formel (I) umfassten, nachfolgend als Ausführungsbeispiele genannten Verbindungen und deren Salze, Solvate und Solvate der Salze, soweit es sich bei den von der allgemeinen Formel (I) umfassten, nachfolgend genannten Verbindungen nicht bereits um Salze, Solvate und Solvate der Salze handelt.

Ebenfalls als von der vorliegenden Erfindung als umfasst anzusehen ist die Verwendung der Salze der erfindungsgemäßen Verbindungen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Ein weiterer Gegenstand der vorliegenden Erfindung sind alle möglichen kristallinen und polymorphen Formen der erfindungsgemäßen Verbindungen, wobei die Polymorphe entweder als einzelne Polymorphe oder als Gemisch mehrerer Polymorphe in allen Konzentrationsbereichen vorliegen können.

Die vorliegende Erfindung betrifft auch Arzneimittel enthaltend die erfindungsgemäßen Verbindungen zusammen mit mindestens einem oder mehreren weiteren Wirkstoffen, insbesondere zur Prophylaxe und/oder Therapie von Tumorerkrankungen.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere. Die erfindungsgemäßen Verbindungen können am Kohlenstoffatom, an welches R⁴ und R⁵ gebunden sind, ein Asymmetriezentrum aufweisen. Sie können daher als reine Enantiomere, Racemate aber auch als Diastereomere oder deren Gemische vorliegen, wenn einer oder mehrere der in der Formel (I) beschriebenen Substituenten ein weiteres Asymmetrieelement enthält, beispielsweise ein chirales Kohlenstoffatom. Die vorliegende Erfindung umfasst deshalb auch Diastereomere und ihre jeweiligen Mischungen. Aus solchen Mischungen lassen sich die reinen Stereoisomere in bekannter Weise isolieren; vorzugsweise werden hierfür chromatographische Verfahren verwendet, insbesondere die HPLC-Chromatographie an chiraler bzw. achiraler Phase.

In der Regel inhibieren die erfindungsgemäßen Enantiomere unterschiedlich stark das Target und sind unterschiedlich aktiv in den untersuchten Krebszelllinien. Das aktivere Enantiomer ist bevorzugt, welches oft dasjenige ist, an dem das durch das an R⁴ und R⁵ gebundene Kohlenstoffatom repräsentierte Asymmetriezentrum (*R*)-konfiguriert ist.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹¹C, ¹³C, ¹⁴C, ¹⁵N, ¹⁷O, ¹80, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagenzien und/oder Ausgangsverbindungen eingesetzt werden.

Die erfindungsgemäßen Verbindungen können systemisch und/oder lokal wirken. Zu diesem Zweck kann sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.
Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophilisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subkutan, intrakutan, perkutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die die erfindungsgemäßen Verbindungen, üblicherweise zusammen mit einem oder mehreren inerten, nichttoxischen, pharmazeutisch geeigneten Hilfsstoffen enthalten, sowie deren Verwendung zu den zuvor genannten Zwecken.

Die Formulierung der erfindungsgemäßen Verbindungen zu pharmazeutischen Präparaten erfolgt in an sich bekannter Weise, indem man den oder die Wirkstoffe mit den in der Galenik gebräuchlichen Hilfsstoffen in die gewünschte Applikationsform überführt.

Als Hilfsstoffe können dabei beispielsweise Trägersubstanzen, Füllstoffe, Sprengmittel, Bindemittel, Feuchthaltemittel, Gleitmittel, Ab- und Adsorptionsmittel, Verdünnungsmittel, Lösungsmittel, Cosolventien, Emulgatoren, Lösungsvermittler, Geschmackskorrigentien, Färbemittel, Konservierungs-, Stabilisierungs-, Netzmittel, Salze zur Veränderung des osmotischen Drucks oder Puffer zum Einsatz kommen. Dabei ist auf Remington's Pharmaceutical Science, 15th ed. Mack Publishing Company, East Pennsylvania (1980) hinzuweisen.

Die pharmazeutischen Formulierungen können in fester Form, zum Beispiel als Tabletten, Dragees,

Pillen, Suppositorien, Kapseln, transdermale Systeme oder in halbfester Form , zum Beispiel als Salben, Cremes, Gele, Suppositorien, Emulsionen oder in flüssiger Form, zum Beispiel als Lösungen, Tinkturen, Suspensionen oder Emulsionen vorliegen.

Hilfsstoffe im Sinne der Erfindung können beispielsweise Salze, Saccharide (Mono-, Di-, Tri-, Oligo-, und/oder Polysaccharide), Proteine, Aminosäuren, Peptide, Fette, Wachse, Öle, Kohlenwasserstoffe sowie deren Derivate sein, wobei die Hilfsstoffe natürlichen Ursprungs sein können oder synthetisch bzw. partial synthetisch gewonnen werden können.

Für die orale oder perorale Applikation kommen insbesondere Tabletten, Dragees, Kapseln, Pillen, Pulver, Granulate, Pastillen, Suspensionen, Emulsionen oder Lösungen in Frage.
Für die parenterale Applikation kommen insbesondere Suspensionen, Emulsionen und vor allem Lösungen in Frage.

Die erfindungsgemäßen Verbindungen eignen sich zur Prophylaxe und/oder Therapie von hyperproliferativen Erkrankungen wie beispielsweise Psoriasis, Keloide und andere Hyperplasien, die die Haut betreffen, gutartige Prostathyperplasien (BPH), solide Tumore und hämatologische Tumore

Als solide Tumore sind erfindungsgemäß beispielsweise Tumore behandelbar der Brust, des Respirationstraktes, des Gehirns , der Fortpflanzungsorgane, des Magen-Darmtraktes, des Urogenitaltraktes, des Auges, der Leber, der Haut, des Kopfes und des Halses, der Schilddrüse, der Nebenschilddrüse, der Knochen sowie des Bindegewebes und Metastasen dieser Tumore.

Als hämatologischeTumore sind beispielsweise behandelbar multiple Myelome, Lymphome oder Leukämien.

Als Brusttumore sind beispielsweise behandelbar Mammakarzinome mit positivem Hormonrezeptorstatus, Mammakarzinome mit negativem Hormonrezeptorstatus, Her-2 positive Mammakarzinome, Hormonrezeptor- und Her-2 negative Mammakarzinome, BRCA -assoziierte Mammakarzinome und entzündliches Mammakarzinom.

Als Tumore des Respirationstraktes sind beispielsweise behandelbar nicht-kleinzellige Bronchialkarzinome und kleinzellige Bronchialkarzinome.

Als Tumore des Gehirns sind beispielsweise behandelbar Gliome, Glioblastome, Astrozytome, Meningiome und Medulloblastome.

Als Tumore der männlichen Fortpflanzungsorgane sind beispielsweise behandelbar Prostatakarzinome, Maligne Nebenhodentumore, Maligne Hodentumore und Peniskarzinome.

Als Tumore der weiblichen Fortpflanzungsorgane sind beispielsweise behandelbar Endometriumkarzinome, Zervixkarzinome, Ovarialkarzinome, Vaginalkarzinome und Vulvarkarzinome.

Als Tumore des Magen- Darm-Traktes sind beispielsweise behandelbar Kolorektale Karzinome, Analkarzinome, Magenkarzinome, Pankreaskarzinome, Ösophagukarzinome, Gallenblasenkarzinome, Dünndarmkarzinome, Speicheldrüsenkarzinome, Neuroendokrine Tumore und Gastrointestinale Stromatumore.

Als Tumore des Urogenital-Traktes sind beispielsweise behandelbar Harnblasenkarzinome, Nierenzellkarzinome, und Karzinome des Nierenbeckens und der ableitenden Harnwege.

Als Tumore des Auges sind beispielsweise behandelbar Retinoblastome und Intraokulare Melanome

Als Tumore der Leber sind beispielsweise behandelbar Hepatozelluläre Karzinome und Cholangiozelluläre Karzinome.

Als Tumore der Haut sind beispielsweise behandelbar Maligne Melanome, Basaliome, Spinaliome, Kaposi-Sarkome und Merkelzellkarzinome.

Als Tumore des Kopfes und Halses sind beispielsweise behandelbar Larynxkarzinome und Karzinome des Pharynx und der Mundhöhle.

Als Sarkome sind beispielsweise behandelbar Weichteilsarkome und Osteosarkome.

Als Lymphome sind beispielsweise behandelbar Non-Hodgkin-Lymphome, Hodgkin-Lymphome, Kutane Lymphome, Lymphome des zentralen Nervensystems und AIDS-assoziierte Lymphome.

Als Leukämien sind beispielsweise behandelbar Akute myeloische Leukämien, Chronische myeloische Leukämien, Akute lymphatische Leukämien, Chronische lymphatische Leukämien und Haarzellleukämien.

Vorteilhaft können die erfindungsgemäßen Verbindungen verwendet werden zur Prophylaxe und/oder Therapie von Leukämien, insbesondere akuten myeolischen Leukämien, Prostatakarzinomen, insbesondere Androgenrezeptor-positiven Prostatakarzinomen, Zervixkarzinomen, Mammakarzinomen, insbesondere von Hormonrezeptor-negativen, Hormonrezeptor-positiven oder BRCA-assoziierten Mammakarzinomen, Pankreaskarzinomen, Nierenzellkarzinomen, Hepatozellulären Karzinomen, Melanomen und anderen Hauttumoren, Nicht-Kleinzelligen Bronchialkarzinomen, Endometriumkarzinomen und Kolorektalen Karzinomen.

Besonders vorteilhaft können die erfindungsgemäßen Verbindungen verwendet werden zur Prophylaxe und/oder Therapie von Leukämien, insbesondere akuten myeloischen Leukämien, Prostatakarzinomen, insbesondere Androgenrezeptor-positiven Prostatakarzinomen, Mammakarzinomen, insbesondere Estrogenrezeptor-alpha negativen Mammakarzinomen, Melanomen oder Multiplen Myelomen.

Die erfindungsgemäßen Verbindungen eignen sich auch zur Prophylaxe und/oder Therapie von benignen hyperproliferativen Krankheiten wie zum Beispiel Endometriose, Leiomyom und benigne Prostatahyperplasie.

Die erfindungsgemäßen Verbindungen eignen sich auch zur Prophylaxe und/oder Therapie von systemischen inflammatorischen Krankheiten, insbesondere LPS-induzierter endotoxischer Schock und/oder Bakterien-induzierte Sepsis.

Die erfindungsgemäßen Verbindungen eignen sich auch zur Prophylaxe und/oder Therapie von inflammatorischen oder Autoimmunerkrankungen wie zum Beispiel:
- Lungenerkrankungen, die mit entzündlichen, allergischen und/oder proliferativen Prozessen einhergehen: chronisch obstruktive Lungenerkrankungen jeglicher Genese, vor allem Asthma bronchiale; Bronchitis unterschiedlicher Genese; alle Formen der restriktiven Lungenerkrankungen, vor allem allergische Alveolitis; alle Formen des Lungenödems, vor allem toxisches Lungenödem; Sarkoidosen und Granulomatosen, insbesondere Morbus Boeck
- Rheumatische Erkrankungen/Autoimmunerkrankungen/Gelenkerkrankungen, die mit entzündlichen, allergischen und/oder proliferativen Prozessen einhergehen: alle Formen rheumatischer Erkrankungen, insbesondere rheumatoide Arthritis, akutes rheumatisches Fieber, Polymyalgia rheumatica; reaktive Arthritis; entzündliche Weichteilerkrankungen sonstiger Genese; arthritische Symptome bei degenerativen Gelenkerkankungen (Arthrosen); traumatische Arthritiden; Kollagenosen jeglicher Genese, z.B. systemischer Lupus erythematodes, Sklerodermie, Polymyositis, Dermatomyositis, Sjögren-Syndrom, Still-Syndrom, Felty-Syndrom
- Allergien, die mit entzündlichen und/oder proliferativen Prozessen einhergehen: alle Formen allergischer Reaktionen, z.B. Quincke Ödem, Heuschnupfen, Insektenstich, allergische Reaktionen auf Arzneimittel, Blutderivate, Kontrastmittel etc., anaphylaktischer Schock, Urtikaria, Kontaktdermatitis
- Gefäßentzündugen (Vaskulitiden): Panarterilitis nodosa, Arterilitis temporalis, Erythema nodosum
- Dermatologische Erkrankungen, die mit entzündlichen, allergischen und/oder proliferativen Prozessen einhergehen: atopische Dermatitis; Psoriasis; Pityriasis rubra pilaris; erythematöse Erkrankungen, ausgelöst durch unterschiedliche Noxen, z.B. Strahlen, Chemikalien, Verbrennungen etc.; bullöse Dermatosen; Erkrankungen des lichenoiden Formenkreises; Pruritus; Seborrhoisches Ekzem; Rosacea; Pemphigus vulgaris; Erythema exsudativum multiforme; Balanitis; Vulvitis; Haarausfall wie Alopecia areata; kutane T-Zell Lymphome
- Nierenerkrankungen, die mit entzündlichen, allergischen und/oder proliferativen Prozessen einhergehen: nephrotisches Syndrom; alle Nephritiden
- Lebererkrankungen, die mit entzündlichen, allergischen und/oder proliferativen Prozessen einhergehen: akuter Leberzellzerfall; akute Hepatitis unterschiedlicher Genese, z.B. viral, toxisch, arneimittelinduziert; chronisch aggressive und/oder chronisch intermittierende Hepatitis
- Gastrointestinale Erkrankungen, die mit entzündlichen, allergischen und/oder proliferativen Prozessen einhergehen: regionale Enteritis (Morbus Crohn); Colitis ulcerosa; Gastritis; Refluxoesophagitis; Gastroenteritiden anderer Genese, z.B. einheimische Sprue
- Proktologische Erkrankungen, die mit entzündlichen, allergischen und/oder proliferativen Prozessen einhergehen: Analekzem; Fissuren; Hämorrhoiden; idiopatische Proktitis
- Augenerkrankungen, die mit entzündlichen, allergischen und/oder proliferativen Prozessen einhergehen: allergische Keratitis, Uveitis, Iritis; Konjuktivitis; Blepharitis; Neuritis nervi optici; Chlorioditis; Opthalmia sympathica
- Erkrankungen des Hals-Nasen-Ohren-Bereiches, die mit entzündlichen, allergischen und/oder proliferativen Prozessen einhergehen: allergische Rhinitis, Heuschnupfen; Otitis externa, z.B. bedingt durch Kontaktexem, Infektion etc.; Otitis media
- Neurologische Erkrankungen, die mit entzündlichen, allergischen und/oder proliferativen Prozessen einhergehen: Hirnödem, vor allem Tumor-bedingtes Hirnödem; Multiple Sklerose; akute Encephalomyelitis; Meningitis; verschiedene Formen von Krampfanfällen, z.B. BNS-Krämpfe
- Bluterkrankungen, die mit entzündlichen, allergischen und/oder proliferativen Prozessen einhergehen: erworbene hämolytische Anämie; idiopathische Thrombozytopenie
- Tumorerkrankungen, die mit entzündlichen, allergischen und/oder proliferativen Prozessen einhergehen: akute lymphatische Leukämie; maligne Lymphome; Lymphogranulomatosen; Lymphosarkome; ausgedehnte Metastasierungen, vor allem bei Mamma-, Bronchial- und Prostatakarzinom
- Endokrine Erkrankungen, die mit entzündlichen, allergischen und/oder proliferativen Prozessen einhergehen: endokrine Orbitopathie; thyreotoxische Krise; Thyreoditis de Quervain; Hashimoto Thyreoditis; Morbus Basedow
- Organ- und Gewebstransplantationen, Graft-versus-Host disease
- Schwere Schockzuständen, z.B. anaphylaktischer Schock, systemic inflammatory response syndrome (SIRS)
- Substitutionstherapie bei: angeborene primäre Nebenniereninsuffizienz, z.B. kongenitales adrenogenitales Syndrom; erworbene primäre Nebenniereninsuffizienz, z.B. Morbus Addison, autoimmune Adrenalitis, postinfektiös, Tumoren, Metastasen, etc; angeborene sekundäre Nebenniereninsuffizienz, z.B. kongenitaler Hypopituitarismus; erworbene sekundäre Nebenniereninsuffizenz, z.B. postinfektiös, Tumoren, etc
- Emesis, die mit entzündlichen, allergischen und/oder proliferativen Prozessen einhergehen, z.B. in Kombination mit einem 5-HT3-Antagonisten bei Zytostatika-bedingten Erbrechen
- Schmerzen bei entzündlicher Genese, z.B. Lumbago

Die erfindungsgemäßen Verbindungen eignen sich auch für die Behandlung von viralen Erkrankungen, wie zum Beispiel Infektionen die verursacht sind durch Papilloma-Viren, Herpes-Viren, Epstein-Barr-Viren, Hepatitis B- oder C-Viren, und humane Immunschwäche-Viren.

Die erfindungsgemäßen Verbindungen eignen sich auch für die Behandlung von Atherosklerose, Dyslipidemie, Hypercholesterolemie, Hypertriglyceridämie, perifere Gefäßerkrankungen, kardiovaskuläre Erkrankungen, Angina, pectoris, Ischemie, Schlaganfall, Myokardinfarkt, angioplastische Restenose, Bluthochdruck, Thrombose, Adipositas, Endotoxemie.

Die erfindungsgemäßen Verbindungen eignen sich auch für die Behandlung von neurodegenerativen Krankheiten wie zum Beispiel multiple Sklerose, Alzheimer's Krankheit und Parkinson's Krankheit.

Diese Erkrankungen sind gut charakterisiert im Menschen, existieren aber auch bei anderen Säugetieren.

Ein weiterer Gegenstand der vorliegenden Anmeldung sind die erfindungsgemäßen Verbindungen zur Verwendung als Arzneimittel, insbesondere zur Prophylaxe und/oder Therapie von Tumorerkrankungen.

Ein weiterer Gegenstand der vorliegenden Anmeldung sind die erfindungsgemäßen Verbindungen zur Prophylaxe und/oder Therapie von Leukämien, insbesondere akuten myeolischen Leukämien, Prostatakarzinomen, insbesondere Androgenrezeptor-positiven Prostatakarzinomen, Zervixkarzinomen, Mammakarzinomen, insbesondere von Hormonrezeptor-negativen, Hormonrezeptor-positiven oder BRCA-assoziierten Mammakarzinomen, Pankreaskarzinomen, Nierenzellkarzinomen, Hepatozellulären Karzinomen, Melanomen und anderen Hauttumoren, Nicht-Kleinzelligen Bronchialkarzinomen, Endometriumkarzinomen und Kolorektalen Karzinomen.

Ein weiterer Gegenstand der vorliegenden Anmeldung sind die erfindungsgemäßen Verbindungen zur Prophylaxe und/oder Therapie von Leukämien, insbesondere akuten myeloischen Leukämien, Prostatakarzinomen, insbesondere Androgenrezeptor-positiven Prostatakarzinomen, Mammakarzinomen, insbesondere Estrogenrezeptor-alpha negativen Mammakarzinomen, Melanomen oder Multiplen Myelomen.

Ein weiterer Gegenstand der Erfindung ist die Verwendung der erfindungsgemäßen Verbindungen zur Herstellung eines Arzneimittels.

Ein weiterer Gegenstand der vorliegenden Anmeldung ist die Verwendung der erfindungsgemäßen Verbindungen zur Herstellung eines Arzneimittels zur Prophylaxe und/oder Therapie von Tumorerkrankungen.

Ein weiterer Gegenstand der vorliegenden Anmeldung ist die Verwendung der erfindungsgemäßen Verbindungen zur Herstellung eines Arzneimittels zur Prophylaxe und/oder Therapie von Leukämien, insbesondere akuten myeolischen Leukämien, Prostatakarzinomen, insbesondere Androgenrezeptor-positiven Prostatakarzinomen, Zervixkarzinomen, Mammakarzinomen, insbesondere von Hormonrezeptor-negativen, Hormonrezeptor-positiven oder BRCA-assoziierten Mammakarzinomen, Pankreaskarzinomen, Nierenzellkarzinomen, Hepatozellulären Karzinomen, Melanomen und anderen Hauttumoren, Nicht-Kleinzelligen Bronchialkarzinomen, Endometriumkarzinomen und Kolorektalen Karzinomen.

Ein weiterer Gegenstand der vorliegenden Anmeldung ist die Verwendung der erfindungsgemäßen Verbindungen zur Herstellung eines Arzneimittels zur Prophylaxe und/oder Therapie von Leukämien, insbesondere akuten myeloischen Leukämien, Prostatakarzinomen, insbesondere Androgenrezeptor-positiven Prostatakarzinomen, Mammakarzinomen, insbesondere Estrogenrezeptor-alpha negativen Mammakarzinomen, Melanomen oder Multiplen Myelomen.

Ein weiterer Gegenstand der vorliegenden Anmeldung ist die Verwendung der erfindungsgemäßen Verbindungen zur Prophylaxe und/oder Therapie von Tumorerkrankungen.

Ein weiterer Gegenstand der vorliegenden Anmeldung ist die Verwendung der erfindungsgemäßen Verbindungen zur Prophylaxe und/oder Therapie von Leukämien, insbesondere akuten myeolischen Leukämien, Prostatakarzinomen, insbesondere Androgenrezeptor-positiven Prostatakarzinomen, Zervixkarzinomen, Mammakarzinomen, insbesondere von Hormonrezeptornegativen, Hormonrezeptor-positiven oder BRCA-assoziierten Mammakarzinomen, Pankreaskarzinomen, Nierenzellkarzinomen, Hepatozellulären Karzinomen, Melanomen und anderen Hauttumoren, Nicht-Kleinzelligen Bronchialkarzinomen, Endometriumkarzinomen und Kolorektalen Karzinomen.

Ein weiterer Gegenstand der vorliegenden Anmeldung ist die Verwendung der erfindungsgemäßen Verbindungen zur Prophylaxe und/oder Therapie von Leukämien, insbesondere akuten myeloischen Leukämien, Prostatakarzinomen, insbesondere Androgenrezeptor-positiven Prostatakarzinomen, Mammakarzinomen, insbesondere Estrogenrezeptor-alpha negativen Mammakarzinomen, Melanomen oder Multiplen Myelomen.

Ein weiterer Gegenstand der vorliegenden Anmeldung sind pharmazeutische Formulierungen in Form von Tabletten enthaltend eine der erfindungsgemäßen Verbindungen zur Prophylaxe und/oder Therapie von Leukämien, insbesondere akuten myeolischen Leukämien, Prostatakarzinomen, insbesondere Androgenrezeptor-positiven Prostatakarzinomen, Zervixkarzinomen, Mammakarzinomen, insbesondere von Hormonrezeptor-negativen, Hormonrezeptor-positiven oder BRCA-assoziierten Mammakarzinomen, Pankreaskarzinomen, Nierenzellkarzinomen, Hepatozellulären Karzinomen, Melanomen und anderen Hauttumoren, Nicht-Kleinzelligen Bronchialkarzinomen, Endometriumkarzinomen und Kolorektalen Karzinomen.

Ein weiterer Gegenstand der vorliegenden Anmeldung sind pharmazeutische Formulierungen in Form von Tabletten enthaltend eine der erfindungsgemäßen Verbindungen zur Prophylaxe und/oder Therapie von Leukämien, insbesondere akuten myeloischen Leukämien, Prostatakarzinomen, insbesondere Androgenrezeptor-positiven Prostatakarzinomen, Mammakarzinomen, insbesondere Estrogenrezeptor-alpha negativen Mammakarzinomen, Melanomen oder Multiplen Myelomen.

Ein weiterer Gegenstand der Erfindung ist die Verwendung der erfindungsgemäßen Verbindungen zur Behandlung von Erkrankungen, die mit proliferativen Prozessen einhergehen.

Ein weiterer Gegenstand der Erfindung ist die Verwendung der erfindungsgemäßen Verbindungen zur Behandlung von benignen Hyperplasien, inflammatorischen Erkrankungen, autoimmunen Erkrankungen, Sepsis, viralen Infektionen, Gefäßerkrankungen und neurodegenerativen Erkrankungen.

Die erfindungsgemäßen Verbindungen können allein oder bei Bedarf in Kombination mit einer oder mehreren weiteren pharmakologisch wirksamen Substanzen eingesetzt werden, solange diese Kombination nicht zu unerwünschten und inakzeptablen Nebenwirkungen führt. Ein weiterer Gegenstand der vorliegenden Erfindung sind daher Arzneimittel, enthaltend eine erfindungsgemäße Verbindung und einen oder mehrere weitere Wirkstoffe, insbesondere zur Prophylaxe und/oder Therapie der zuvor genannten Erkrankungen.

Beispielsweise können die erfindungsgemäßen Verbindungen mit bekannten anti-hyperproliferativen, zytostatischen oder zytotoxischen chemischen und biologischen Substanzen zur Behandlung von Krebserkrankungen kombiniert werden. Die Kombination der erfindungsgemäßen Verbindungen mit anderen für die Krebstherapie gebräuchlichen Substanzen oder auch mit der Strahlentherapie ist besonders angezeigt.

Als geeignete Kombinationswirkstoffe seien beispielhaft genannt, ohne dass diese Aufzählung abschließend wäre:
Abiraterone acetate, Abraxane, Acolbifen, Actimmun, Actinomycin D (Dactinomycin), Afatinib, Affinitak,Afinitor, Aldesleukin, Alendronsäure, Alfaferon, Alitretinoin, Allopurinol, Aloprim, Aloxi, Alpharadin, Altretamin, Amino¬glutethimid, Aminopterin, Amifostin, Amrubicin, Amsacrin, Anastrozol, Anzmet, Apatinib, Aranesp, Arglabin, Arsen-trioxid, Aromasin, Arzoxifen, Asoprisnil, L-Asparaginase, Atamestan, Atrasentan, Avastin, Axitinib, 5-Azacytidin, Azathioprin, BCG oder tice-BCG, Bendamustin, Bestatin, Beta-methason-Acetat, Betamethason-Natriumphosphat, Bexaroten, Bicalutamid, Bleomycin-Sulfat, Broxuridin, Bortezomib, Bosutinib, Busulfan, Cabazitaxel, Calcitonin, Campath, Camptothecin, Capecitabin, Carboplatin, Carfilzomib, Carmustin, Casodex,CCI-779, CDC-501, Cediranib, Cefeson, Celebrex, Celmoleukin, Cerubidin, Cediranib, Chlorambucil, Cisplatin, Cladribin, Clodronsäure, Clofarabin, Colaspase, Copanlisib, Corixa, Crisnatol, Crizotinib, Cyclophosphamid, Cyproterone-Acetat, Cytarabin, Dacarbazin, Dactinomycin, Dasatinib, Daunorubicin, DaunoXome, Decadron, Decadron-Phosphat, Decitabin, Degarelix, Delestrogen, Denileukin Diftitox, Depomedrol, Deslorelin, Dexrazoxan, Diethylstilbestrol, Diflucan, 2',2'-Difluordeoxycytidin, DN-101, Docetaxel, Doxifluridin, Doxo-¬rubicin (Adriamycin), Dronabinol, dSLIM, Dutasterid, DW-166HC, Edotecarin, Eflornithin, Eligard, Elitek, Ellence, Emend, Enzalutamide, Epirubicin, Epoetin-alfa, Epogen, Epothilon und seine Derivate, Eptaplatin, Ergamisol, Erlotinib, Erythro-Hydroxynonyladenin, Estrace, Estradiol, Estramustin-Natriumphosphat, Ethinylestradiol, Ethyol, Etidronsäure, Etopophos, Etoposid, Everolimus, Exatecan, Exemestan, Fadrozol, Farston, Fenretinid, Filgrastim, Finasterid, Fligrastim, Floxuridin, Fluconazol, Fludarabin, 5-Fluordeoxyuridin-Monophosphat, 5-Fluoruracil (5-FU), Flu¬oxy¬mesteron, Flutamid, Folotin, Formestan, Fosteabin, Fotemustin, Fulvestrant, Gammagard, Gefitinib, Gemcitabin, Gemtuzumab, Gleevec, Gliadel, Goserelin, Gossypol, Granisetron-Hydrochlorid, Hexamethylmelamin, Histamin-Dihydrochlorid, Histrelin, Holmium-166-DOTPM, Hycamtin, Hydrocorton, erythro-Hydroxynonyladenin, Hydroxyharnstoff, Hydroxyprogesteronecaproat, Ibandronsäure, Ibritumomab Tiuxetan, Idarubicin, Ifosfamid, Imatinib, Iniparib, Interferon-alpha, Interferon-alpha-2, Interferon-alpha-2α, Interferon-alpha-2β, Interferon-alpha-n1, Interferon-alpha-n3, Interferon-beta, Interferon-gamma-1α, Interleukin-2, Intron A, Iressa, Irinotecan, Ixabepilon, Keyhole limpet Hemocyanin, Kytril, Lanreotid, Lapatinib, Lasofoxifen, Lenalidomid, Lentinan-Sulfat, Lestaurtinib, Letrozol, Leucovorin, Leuprolid, Leuprolid-Acetat, Levamisol, Levofolinsäure-Calciumsalz, Levothroid, Levoxyl, Libra, liposomales MTP-PE, Lomustin, Lonafarnib, Lonidamin, Marinol, Mechlorethamin, Mecobalamin, Medroxyprogesteron-Acetat, Megestrol-Acetat, Melphalan, Menest, 6-Mercaptopurin, Mesna, Methotrexat, Metvix, Miltefosin, Minocyclin, Minodronat, Miproxifen, Mitomycin C, Mitotan, Mitoxantron, Modrenal, MS-209, MX-6, Myocet, Nafarelin, Nedaplatin, Nelarabine, Nemorubicin, Neovastat, Neratinib, Neulasta, Neumega, Neupogen, Nilotimib, Nilutamid, Nimustin, Nolatrexed, Nolvadex, NSC-631570, Obatoclax, Oblimersen, OCT-43, Octreotid, Olaparib, Ondansetron-Hydrochlorid, Onko-TCS, Orapred, Osidem, Oxaliplatin, Paclitaxel, Pamidronat-Dinatrium, Pazopanib, Pediapred, Pegaspargase, Pegasys, Pemetrexed, Pentostatin, N-Phosphono-acetyl-L-Aspartat, Picibanil, Pilocarpin-Hydrochlorid, Pirarubicin, Plerixafor, Plicamycin, PN-401, Porfimer-Natrium, Prednimustin, Prednisolon, Prednison, Premarin, Procarbazin, Procrit, QS-21, Quazepam, R-1589, Raloxifene, Raltitrexed, Ranpirnas, RDEA119, Rebif, Regorafenib, 13-cis-Retinsäure, Rhenium-186-Etidronat, Rituximab, Roferon-A, Romidepsin, Romurtid, Ruxolitinib, Salagen, Salinomycin, Sandostatin, Sargramostim, Satraplatin, Semaxatinib, Semustin, Seocalcitol, Sipuleucel-T, Sizofiran, Sobuzoxan, Solu-Medrol, Sorafenib, Streptozocin, Strontium-89-chlorid, Sunitinib, Synthroid, T-138067, Tamoxifen, Tamsulosin, Tarceva, Tasonermin, Tastolacton, Taxoprexin, Taxoter, Teceleukin, Temozolomid, Temsirolimus, Teniposid, Testosteron-Propionat, Testred, Thalidomid, Thymosin-alpha-1, Thioguanin, Thiotepa, Thyrotropin, Tiazorufin, Tiludronsäure, Tipifarnib, Tirapazamin, TLK-286, Toceranib, Topotecan, Toremifen, Tositumomab, Tastuzumab, Teosulfan, TransMID-107R, Tretinoin, Trexall, Trimethylmelamin, Trimetrexat, Triptorelin-Acetat, Triptorelin-Pamoat, Trofosfamid, UFT, Uridin, Valrubicin, Valspodar, Vandetanib, Vapreotid, Vatalanib, Vemurafinib, Verte-porfin, Vesnarinon, Vinblastin, Vincristin, Vindesin, Vinflumin, Vinorelbin, Virulizin, Vismodegib, Xeloda, Z-100, Zinecard, Zinostatin-Stimalamer, Zofran, Zoledronsäure

Insbesondere lassen sich die erfindungsgemäßen Verbindungen mit Antikörpern wie z.B. Aflibercept, Alemtuzumab, Bevacizumab, Brentuximumab, Catumaxomab, Cetuximab, Denosumab, Edrecolomab, Gemtuzumab, Ibritumomab, Ipilimumab, Ofatumumab, Panitumumab, Pertuzumab, Rituximab, Tositumumab oder Trastuzumabsowie mit rekombinanten Proteinen kombinieren.

Insbesondere können die erfindungsgemäßen Verbindungen in Kombination mit gegen die Angiogenese gerichtete Therapien wie z.B. Bevacizumab, Axitinib, Regorafenib, Cediranib, Sorafenib, Sunitinib, Lenalidomid, Copanlisib oder Thalidomid zum Einsatz kommen.

Kombinationen mit Antihormonen und steroidalen metabolischen Enzyminhibitoren sind wegen ihres günstigen Nebenwirkungsprofils besonders geeignet.

Kombinationen mit P-TEFb- und CDK9-Inhibitoren sind wegen der möglichen synergistischen Effekte ebenfalls besonders geeignet.

Generell können mit der Kombination der erfindungsgemäßen Verbindungen mit anderen, zytostatisch oder zytotoxisch wirksamen Agentien folgende Ziele verfolgt werden:
- eine verbesserte Wirksamkeit bei der Verlangsamung des Wachstums eines Tumors, bei der Reduktion seiner Größe oder sogar bei seiner völligen Eliminierung im Vergleich zu einer Behandlung mit einem einzelnen Wirkstoff;
- die Möglichkeit, die verwendeten Chemotherapeutika in geringerer Dosierung als bei der Monotherapie einzusetzen;
- die Möglichkeit einer verträglicheren Therapie mit weniger Nebeneffekten im Vergleich zur Einzelgabe;
- die Möglichkeit zur Behandlung eines breiteren Spektrums von Tumorerkrankungen;
- das Erreichen einer höheren Ansprechrate auf die Therapie;
- eine längere Überlebenszeit der Patienten im Vergleich zur heutigen Standardtherapie.

Darüber hinaus können die erfindungsgemäßen Verbindungen auch in Verbindung mit einer Strahlentherapie und/oder einer chirurgischen Intervention eingesetzt werden.

### Herstellung der erfindungsgemäßen Verbindungen:

In der vorliegenden Beschreibung bedeuten:
NMR-Signale werden mit ihrer jeweils erkennbaren Multiplizität bzw. deren Kombinationen angegeben. Dabei bedeutet s = Singulett, d = Dublett, t = Triplett, q = Quartett, qi = Quintett, sp = Septett, m = Multiplett, b = breites Signal. Signale mit kombinierter Multiplizität werden beispielsweise angegeben als dd = Dublett vom Dublett.
   - CDCl₃: Deuterochloroform
   - dba: Dibenzylidenaceton
   - DMA: N,N-Dimethylacetamid
   - DMF: N,N-Dimethylformamid
   - DMSO-d6: deuteriertes Dimethylsulfoxid
   - DMSO: Dimethylsulfoxid
   - HATU: (7-Aza-1 H-benzotriazole-1-yl)-1,1,3,3-tetramethyluronium hexafluorophosphat
   - RP-HPLC: Reversed Phase Hochdruckflüssigkeitschromatographie
   - RT: Raumtemperatur
   - THF: Tetrahydrofuran
   - HBTU: O-Benzotriazol-N,N,N',N'-tetramethyl-uronium-hexafluor-phosphat
   - PyBOB: (Benzotriazol-1-yl)-oxytripyrrolidinophosphonium hexafluorophosphat)
   - T3P: 2,4,6-Tripropyl-1,3,5,2,4,6-trioxatriphosphorinan-2,4,6-trioxid
   - LCMS: Flüssigchromatographie gekoppelt mit Massenspektronmetrie
   - CHAPS: 3-{Dimethyl[3-(4-{5,9,16-trihydroxy-2,15-dimethyltetracyclo [8.7.0.0^{2,7}.0^{11,15}]heptadecan-14-yl}pentanamido)propyl]-azaniumyl}propan-1-sulfonat
   - (+)-BINAP: (R)-(+)-2,2'-Bis(diphenylphosphino)-1,1'-binaphthyl
   - (±)-BINAP: 2,2'-Bis(diphenylphosphino)-1,1'-binaphthyl (racemisch)
   - TBTU: (Benzotriazol-1-yloxy)bisdimethylaminomethyliumfluoroborat
   - DCC: Dicyclohexylcarbodiimid
   - Xanthphos: (9,9-Dimethyl-9H-xanthene-4,5-diyl)bis(diphenylphosphan)

### Allgemeine Beschreibung der Herstellung der erfindungsgemäßen Verbindungen der allgemeinen Formel I

Die in Schema 1 gezeigten erfindungsgemäßen Verbindungen der Formeln (Ia), (Ib),(Ic) und (Id) lassen sich herstellen über Synthesewege, die im Folgenden beschrieben werden. Die genannten Formeln stellen verschiedene Teilmengen der allgemeinen Formel (I) dar, in denen A, R², R³, R⁴, R⁵, R⁶, R⁷, R⁸ und n definiert sind wie für die allgemeine Formel (I). In Verbindungen der Formel (Ia) vom Typ der Dihydropyridopyrazinone steht -N- an der Stelle von X und eine Gruppe - C(=O)NR⁷R⁸ steht an der Stelle von R¹; in Verbindungen der Formel (Ib) vom Typ der Dihydrochinoxalinone steht -CH- an der Stelle von X und eine Gruppe -C(=O)NR⁷R⁸ steht an der Stelle von R¹; in Verbindungen der Formel (Ic) ist X definiert wie für die allgemeine Formel (I) und eine Gruppe -S(=O)₂NR⁷R⁸ steht an der Stelle von R¹, und in Verbindungen (Id) schließlich steht HetAr, das 5-gliedriges, monocyclisches Heteroaryl- wie in Formel (I) für R¹ definiert bedeutet, an der Stelle von R¹.

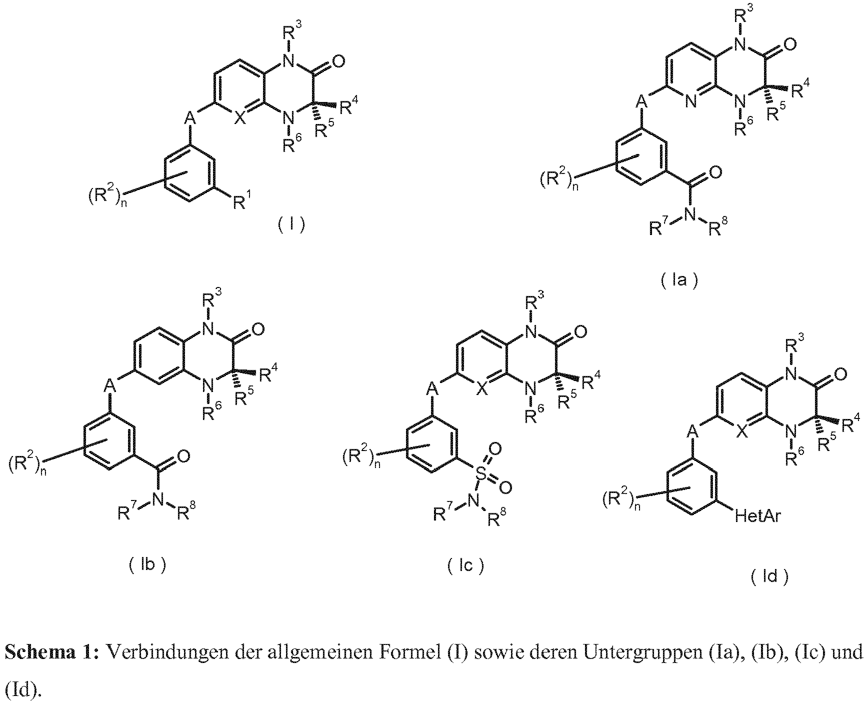

Zusätzlich zu den nachfolgend besprochenen Synthesequenzen können, entsprechend den allgemeinen Kenntnissen des Fachmannes in der Organischen Chemie, auch weitere Synthesewege für die Synthese von erfindungsgemäßen Verbindungen der allgemeinen Formel (I) beschritten werden. Die Reihenfolge der in den nachfolgenden Schemata gezeigten Syntheseschritte ist nicht bindend, und Syntheseschritte aus verschiedenen der nachfolgend gezeigten Schemata können gegebenenfalls zu neuen Sequenzen kombiniert werden. Zusätzlich können Interkonversionen der Substituenten R², R³, R⁴, R⁵, R⁶, R⁷, R⁸ vor oder nach den gezeigten Synthesestufen durchgeführt werden. Beispiele für solche Umwandlungen sind die Einführung oder Abspaltung von Schutzgruppen, Reduktion oder Oxidation funktioneller Gruppen, Halogenierung, Metallierung, metallkatalysierte Kupplungsreaktionen, Substitutionsreaktionen oder weitere dem Fachmann bekannte Umsetzungen. Diese Reaktionen schließen Umsetzungen ein, welche eine funktionelle Gruppe einführen, die weitere Umwandlung von Substituenten ermöglicht. Geeignete Schutzgruppen sowie Methoden zu ihrer Einführung und Abspaltung sind dem Fachmann bekannt (siehe z.B. T.W. Greene und P.G.M. Wuts in: Protective Groups in Organic Synthesis, 3. Auflage, Wiley 1999). Weiterhin ist die Zusammenfassung zweier oder mehrerer Reaktionsschritte ohne zwischenzeitliche Aufarbeitung in einer dem Fachmann bekannten Weise möglich (z.B. in sogenannten "Eintopf "-Reaktionen).

Schema 2 illustriert den Aufbau von Amiden der Formel (V) aus einfachen Pyridinderivaten der Formel (II), worin die Gruppen R^{Hal} gleich oder verschieden sein können und jeweils für Halogen, bevorzugt Fluor oder Chlor, stehen, wie zum Beispiel 3-Amino-2,6-dichlorpyridin (CAS-Nr. 62476-56-6) oder 3-Amino-2,6-difluorpyridin (CAS-Nr. 108118-69-0). Für die Herstellung von (III) aus (II) kann eine Vielzahl an Methoden zur Herstellung von Amiden ausgehend von den Azido-Carbonsäuren der Formel (IIa), in denen R⁴ und R⁵ definiert sind wie für die allgemeine Formel (I), verwendet werden. So können für den Fachmann bekannte Kopplungsreagenzien wie TBTU, HATU, T3P oder DCC eingesetzt werden. Ebenfalls eignet sich die Umsetzung der zum Einsatz kommenden Azido-Carbonsäuren mit einem anorganischen Säurechlorid wie Thionylchlorid, Phosphoroxychlorid oder Oxalylchlorid und anschließender Zugabe des Pyridinamins. Die Herstellung der benötigten Azido-Carbonsäuren ist in der Literatur beschrieben (Chem Eur J (2010), 16, p7572 ff, D. Tietze et al.; J Org Chem (2010), 75, p6532ff, Katritzky et al.). Die Handhabung der Azido-Carbonsäuren ist mit größter Vorsicht durchzuführen, da diese sich explosionsartig zersetzten können. Ebenso sollte auf Lagerung der zur Azid-Einführung benötigten Reagenzien verzichtet werden. Diese Aspekte werden bei Katritzky et al. diskutiert. Zur Reduktion der Azidogruppe in (III), die zu Aminen der Formel (IV) führt, kann man die Reaktion mit Trialkyl- oder Triarylphosphinen nach Staudinger durchführen (Tetrahedron (2012), 68, p697ff, Laschat et al.). Geeignet ist z.B. Trimethylphosphin. Die Isolation der Amine (IV) kann als freie Base oder, vorteilhaft, in Salzform, etwa als Hydrochlorid, erfolgen. Dazu wird das rohe Amin der Formel (IV) in einem unpolaren Lösungsmittel, beispielsweise Diethylether gelöst, und durch Zugabe einer Säure, beispielsweise von Chlorwasserstoff, als Salz ausgefällt. Die weitere Umsetzung zu Verbindungen der Formel (V) unter Einführung des Restes R⁶, der definiert ist wie für die allgemeine Formel (I), kann bevorzugt durch die für den Fachmann bekannte reduktive Aminierung durchgeführt werden (für repräsentative Vorschriften siehe z.B. US2010/105906 A1). Dabei wird das primäre Amin (IV), als freie Base oder in Salzform, mit einem für die Einführung von R⁶ geeigneten Aldehyd oder Keton *in situ* zu einem Imin umgesetzt und dieses anschließend durch Zugabe eines geeigneten Reduktionsmittels wie Natriumtriacetoxyborhydrid zum sekundären Amin der Formel (V) transformiert.

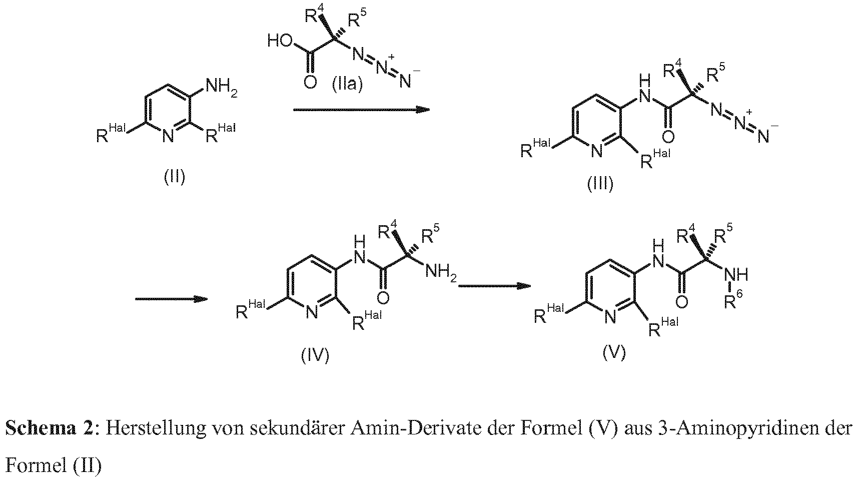

Alternativ können Intermediate der Formel (IV), als freie Base oder in Salzform, durch Umsetzung von einfachen Pyridinderivaten der Formel (II), worin die Gruppen R^{Hal} gleich oder verschieden sein können und jeweils für Halogen, bevorzugt Fluor oder Chlor, stehen, wie zum Beispiel 3-Amino-2,6-dichlorpyridin (CAS-Nr. 62476-56-6) oder 3-Amino-2,6-difluorpyridin (CAS-Nr. 108118-69-0), mit einer entsprechenden *N*-geschützten Aminosäure der Formel (IIb), in der R⁴ und R⁵ definiert ist wie für die allgemeine Formel (I), und in der SG für eine geeignete Schutzgruppe SG, wie beispielsweise BOC, Fmoc oder Cbz steht, hergestellt werden (Schema 3). *N-*Geschützte Aminosäuren sind üblicherweise kommerziell erhältlich. Es können für den Fachmann bekannte Kopplungsreagenzien wie TBTU, HATU, T3P oder DCC eingesetzt werden. Ebenfalls eignet sich die Umsetzung der zum Einsatz kommenden *N*-geschützten Aminosäure der Formel (IIb) mit einem anorganischen Säurechlorid wie Thionylchlorid, Phosphoroxychlorid oder Oxalylchlorid und anschließender Zugabe des Pyridinamins. Dabei werden Verbindungen gemäß Formel (VI) erhalten, die nach dem für den Fachmann bekannten Methoden zur Abspaltung von Schutzgruppen zu Verbindungen der Formel (IV) umgesetzt werden können.

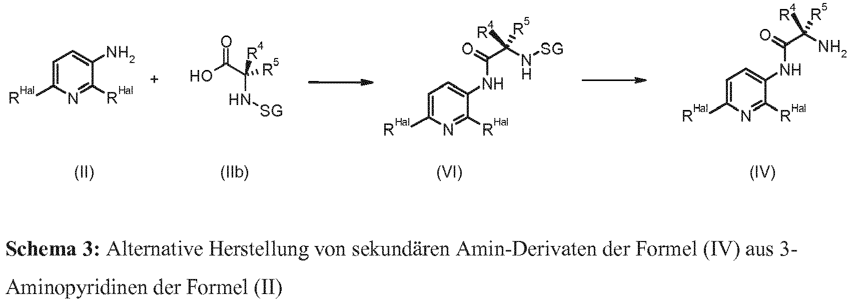

Wie in Schema 4 gezeigt, lassen sich die sekundären Amine der Formel (V) durch Cyclisierung zu Dihydropyridopyrazinonen der Formel (VII) umsetzen. Dazu kann man Verbindungen der Formel (V) in Anwesenheit einer geeigneten Base unter erhöhter Temperatur umsetzen (siehe dazu auch WO2010/9642 A2, Example 16). Die nachfolgende Alkylierung zu Verbindungen (VIII) kann erfolgen durch Umsetzung mit R³ -LG, worin R³ definiert ist wie in der allgemeinen Formel (I) und LG für eine Abgangsgruppe, bevorzugt Iodid, steht, in Gegenwart einer geeigneten Base wie Natriumhydrid, nach für den Fachmann bekannten Bedingungen. Die weitere Umsetzung der resultierenden Verbindungen der Formel (VIII) zu den Esterderivaten (IX) kann erfolgen durch Umsetzung mit Verbindungen der Formel (VIIIa), in denen A, R² und n definiert sind wie in der allgemeinen Formel I, und in denen R^{E} für C₁-C₆-Alkyl steht, in einer Palladium-katalysierten Kupplungsreaktion nach Buchwald und Hartwig (siehe beispielsweise J. Organomet. Chem. (1999), 576, p125ff). Als Palladiumquelle eignen sich hier z.B. Palladiumacetat oder Palladium-(dba)-Komplexe, wie zum Beispiel Pd₂(dba)₃ (CAS-Nr. 51364-51-3 bzw. 52409-22-0). Der Umsatz hängt dabei stark von den verwendeten Liganden ab. Die im Experimentalteil ausgeführte Beispiele ließen sich so z.B. durch die Verwendung von (+)-BINAP erhalten (vgl. auch US2006/009457 A1).

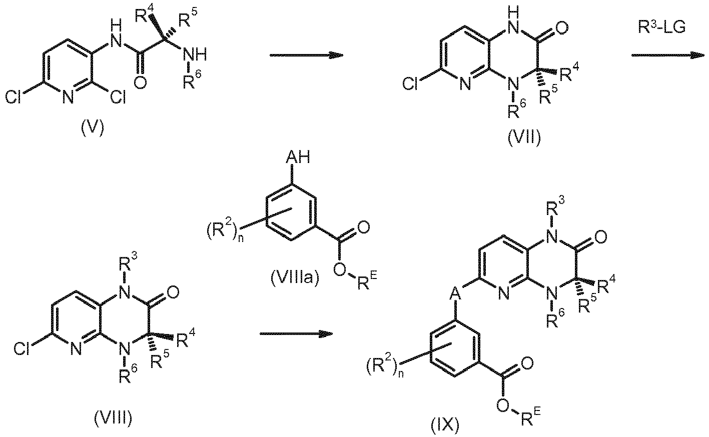

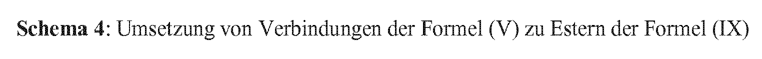

Die Herstellung von Carboxamiden der allgemeinen Formel (Ia) kann entsprechend Schema 5 mittels Hydrolyse der jeweiligen Ester der Formel (IX) zu den entsprechenden Carbonsäuren der Formel (X) nach für den Fachmann bekannten Methoden erfolgen. Diese Umsetzungen lassen sich bevorzugt unter Verwendung von Alkalihydroxiden wie Lithiumhydroxid, Natriumhydroxid oder Kaliumhydroxid in wässrigen alkoholischen Lösungen durchführen.

Die so erhaltenen Carbonsäuren (X) lassen sich in die erfindungsgemäßen Carboxamide der allgemeinen Formel (Ia) überführen durch Umsetzung mit den in der Regel kommerziell erhältlichen Aminen der Formel R⁷R⁸NH, in denen R⁷ und R⁸ definiert sind wie für die allgemeine Formel (I), unter zusätzlicher Aktivierung mit einer Methode, wie sie dem Fachmann allgemein bekannt ist. Als mögliche Methoden seien hier genannt die Verwendung von HATU, HBTU, PyBOB oder T3P unter Zusatz einer geeigneten Base. Die Umwandlung der Carbonsäuren in ihre Amide wird allgemein beschrieben in Referenzbüchern wie "Compendium of Organic Synthetic Methods", Band I-VI (Wiley Interscience) oder "The Practice of Peptide Synthesis", Bodansky (Springer Verlag).

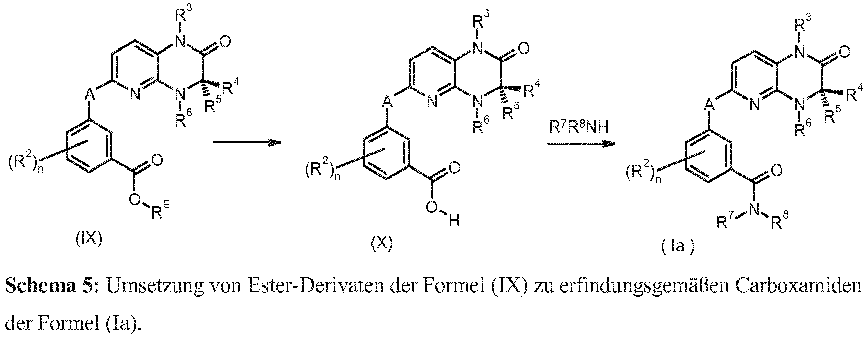

Dihydrochinoxalinone der Formel (Ib) können erhalten werden wie in Schema 6 beschrieben. Dazu lassen sich geeignete *ortho*-Fluornitrobenzolderivate, wie zum Beispiel 4-Brom-2-fluornitrobenzol ((XI); CAS-Nr. 321-23-3) durch nucleophile *ipso*-Substitution mit Aminosäuren der Struktur (XIa), in denen R⁴ und R⁵ definiert sind wie für die allgemeine Formel (I), zu Verbindungen der Struktur (XII) umsetzen. Durch selektive Reduktion der Nitro-Gruppe mit einem geeigneten Reduktionsmittel und anschließender Aufarbeitung im sauren Milieu erhält man direkt die bicyclischen Verbindungen der Formel (XIII). Als geeignete Reduktionsmittel können verwendet werden z.B. Alkalidithionite (J Heterocyclic Chem. (1992), 29, P1859-61, Shafiee et al.), oder Zinn(II)chlorid (J. Org. Chem. (1983), 48, p2515ff, Xing et al.). Die gesamte Reaktionsfolge von Reduktion und Cyclisierung ist ebenfalls beschrieben (WO2010/116270 A1, L.1.b). Zur Herstellung der am basischen Stickstoff substituierten Verbindungen (XIV), in denen R⁶ definiert ist wie in der allgemeinen Formel (I), können die Verbindungen der Formel (XIII) mit für die Einführung von R⁶ geeigneten Aldehyden oder Ketonen sowie einem Reduktionsmittel gemäß einer dem Fachmann bekannten reduktiven Aminierung umgesetzt werden. Hier ist beispielsweise die Verwendung eines Alkyl- oder Arylsilans, beispielsweise Phenylsilan, gegebenenfalls in Kombination mit Dibutylzinndichlorid, als Reduktionsmittel eine dem Fachmann bekannte Methode, die die Intermediate (XIV) in hinreichenden Ausbeuten liefert (Bioorg. Med. Chem. Lett. (2009), 19, S. 688ff; D. V. Smil et al.).

Die weitere Umsetzung zu den erfindungsgemäßen Verbindungen gemäß Formel (Ib) über die Zwischenstufe (XV), (XVI), und (XVII) kann unter vergleichbaren Bedingungen, wie in den Schemata 4 und 5 für die Umsetzung der Zwischenstufe der Formel (VII) zu den erfindungsgemäßen Verbindungen gemäß Formel (Ia) über die Zwischenstufen (VIII), (IX) und (X) beschrieben, durchgeführt werden.

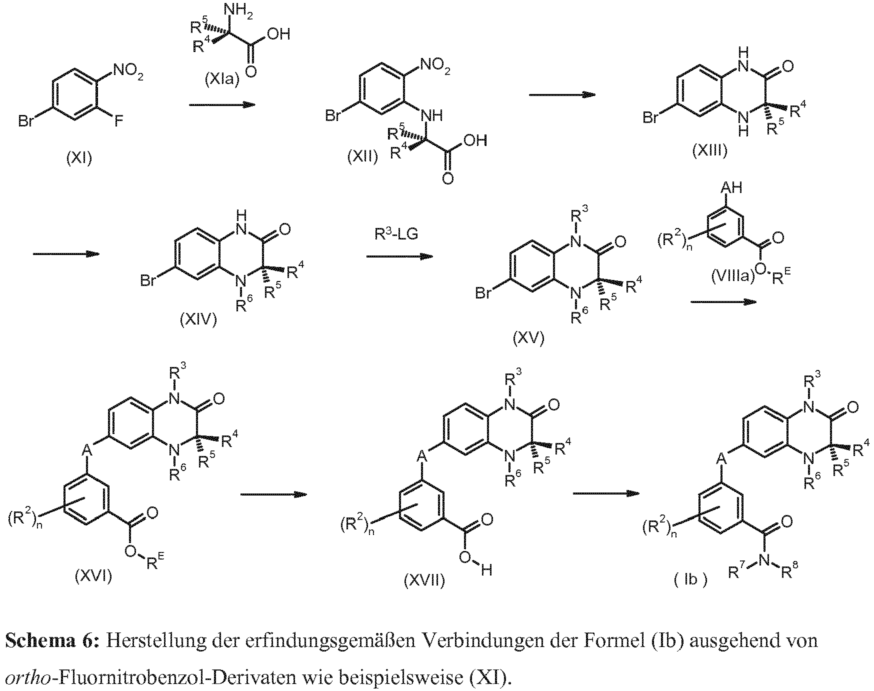

Alternativ können Strukturen der Formel (XIV) auch wie in Schema 7 beschrieben hergestellt werden. Dabei trägt der Aminosäureester (XVIII) bereits den Rest R⁶ gemäß Formel (I). Zur Herstellung der Aminosäureester (XVIII) wird der am Stickstoff unsubstituierte Aminosäureester (XIb) mit einem für die Einführung von R⁶ geeigneten Aldehyd oder Keton *in situ* zu einem Imin umgesetzt und dieses anschließend durch Zugabe eines geeigneten Reduktionsmittels wie Natriumtriacetoxyborhydrid zum sekundären Amin der Formel (XVIII) transformiert. Diese Umsetzung erfolgt unter den für den Fachmann bekannten Bedingungen der reduktiven Aminierung (für repräsentative Vorschriften siehe z.B. US2010/105906 A1).

Die weitere Umsetzung mit geeigneten ortho-Fluornitrobenzolderivaten, wie zum Beispiel 4-Brom-2-fluornitrobenzol ((XI); CAS-Nr. 321-23-3) durch nucleophile *ipso*-Substitution mit den R⁶-subtituierten Aminosäureestern der Formel (XVIII), in denen R⁴, R⁵ und R⁶ definiert sind wie für die allgemeine Formel (I), führt zu Verbindungen der Struktur (XIX), in denen R^{E} für C₁-C₆-Alkyl steht. Durch selektive Reduktion der Nitro-Gruppe mit einem geeigneten Reduktionsmittel und anschließender Aufarbeitung im sauren Milieu erhält man direkt die bicyclischen Verbindungen der Formel (XIV). Als geeignete Reduktionsmittel können verwendet werden z.B. Alkalidithionite (J Heterocyclic Chem. (1992), 29, P1859-61, Shafiee et al.), Zinn(II)chlorid (J. Org. Chem. (1983), 48, p2515ff, Xing et al.), oder Eisenpulver in Gegenwart einer geeigneten Säure, beispielsweise Salzsäure, Essigsäure oder wässriger Ammoniumchlorid-Lösung. Die gesamte Reaktionsfolge von Reduktion und Cyclisierung erfolgt analog einer Literaturvorschrift (WO2010/116270 A1, L.1.b), gegebenenfalls unter Ersatz des als Reduktionsmittel beschriebenen Natriumdilhionits in wässriger Kaliumcarbonat-Lösung durch Eisenpulver in einem Gemisch aus Methanol und Eisessig.

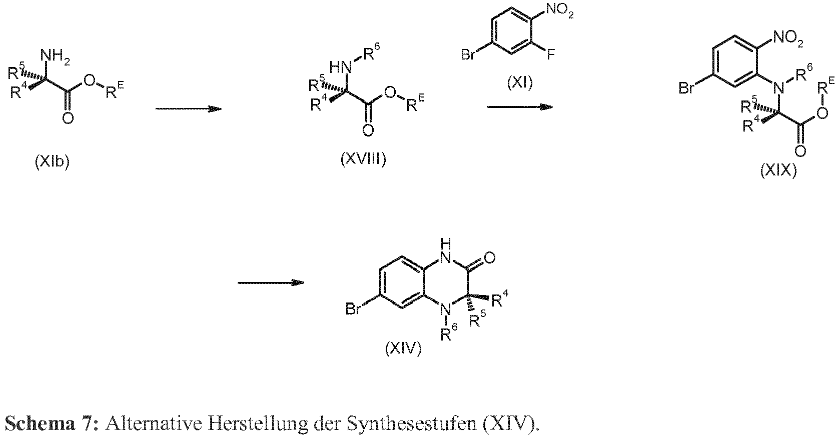

Die Herstellung von Zwischenstufen der Formel (VIIa), in denen R^{6'} für gegebenenfalls substituiertes Phenyl gemäß der Definition für R⁶ in der allgemeinen Formel (I) steht, ist in Schema 8 beschrieben.

3-Amino-2,6-dichlorpyridin ((IIc), CAS-Nr. 62476-56-6) wird umgesetzt mit Verbindungen der Formel (XX), in denen R⁴ und R⁵ definiert sind wie für die allgemeine Formel (I), und in denen LG und LG' unabhängig voneinander jeweils für eine Abgangsgruppe, bevorzugt Chlor oder Brom, stehen, wie z.B. 2-Brompropionylbromid (CAS 563-76-8). Dabei wird unter für den Fachmann bekannten Bedingungen mit einem geeineten Lösungsmittel wie Dichlormethan oder THF und unter Zusatz einer Base wie Triethylamin, Di-*iso*-propylethylamin oder Pyridin umgesetzt. Die Base kann auch als Lösungsmittel eingesetzt werden. Dabei werden Verbindungen der Formel (XXI) erhalten. Diese Intermediate (XXI) werden mit Anilinen der Formel R^{6'}-NH₂, in denen R^{6'} für gegebenenfalls substituiertes Phenyl gemäß der Definition für R⁶ in der allgemeinen Formel (I) steht, umgesetzt zu Verbindungen der Formel (XXII). Diese Reaktion kann durch Umsetzung in verschiedenen Lösungsmitteln wie Toluol oder Acetonitril und unter Zusatz einer Base wie beispielsweise Kaliumcarbonat, Di-*iso*-propylethylamin oder Triethylamin bei erhöhter Temperatur erfolgen (Org. Lett. (2008), 10, S. 2905 ff, S. P. Marsden et al.). Dihydropyridopyrazinone der Formel (VIIa), bei der R^{6'} für gegebenenfalls substituiertes Phenyl gemäß der Definition für R⁶ in der allgemeinen Formel (I) steht, werden erhalten durch Cyclisierung der Verbindungen der Formel (XXII) in Anwesenheit einer geeigneten Base wie zum Beispiel Triethylamin, Di-*iso*-propylethylamin oder Kaliumcarbonat unter erhöhter Temperatur in Lösungsmitteln wie zum Beispiel *N,N*-Dimethylformamid, *N,N*-Dimethylacetamid, *N*-Methylpyrrolidon oder auch Dimethylsulfoxid (siehe dazu auch WO2010/96426 A2, Example 16). Aus diesen Intermediaten der Formel (VIIa) lassen sich gemäß den Schemata 4 und 5 die entsprechenden erfindungsgemäßen Verbindungen der Formel (I) herstellen, in denen X für -N- und R^{6'} für gegebenenfalls substituiertes Phenyl gemäß der Definition für R⁶ in der allgemeinen Formel (I) steht. Dabei werden die genannten Verbindungen der Formel (I) als Racemate erhalten, sofern R⁴ und R⁵ voneinander verschieden sind. Diese können gegebenenfalls mit den für den Fachmann geläufigen Trennmethoden, beispielsweise präparativer HPLC an einer chiralen stationären Phase, in die Enantiomeren separiert werden.

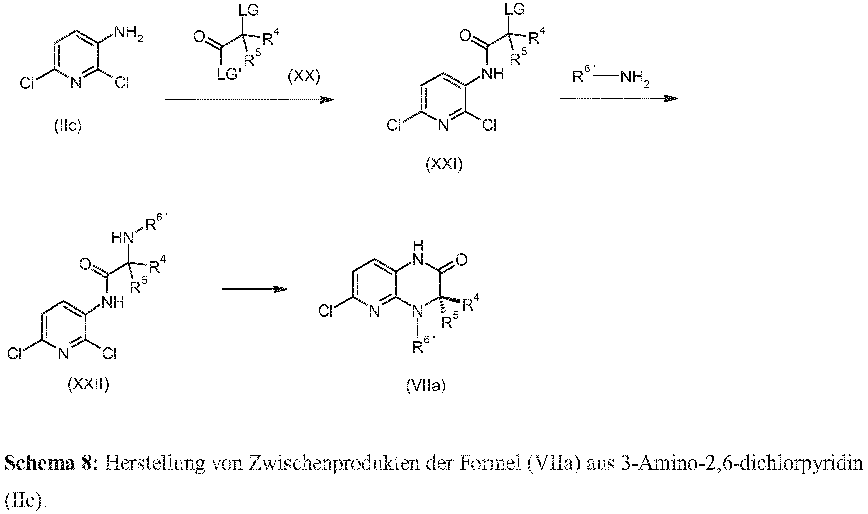

Die Herstellung der erfindungsgemäßen Verbindungen gemäß Formel (Ic) mit einer Sulfonamid-Gruppe an der der Stelle von R¹ kann erfolgen gemäß Schema 9. Hierbei können Verbindungen der Formel (VIII) (für Dihydropyridopyrazinon-Derivate) beziehungsweise Verbindungen der Formel (XV) (für Dihydrochinoxalinon-Derivate) in analoger Weise wie in Schema 4 für die Umsetzung von (VIII) zu (IX) diskutiert, mit Verbindungen der Formel (XXIII), in denen A, R², R⁷, R⁸ und n definiert sind wie in der allgemeinen Formel (I), in einer Palladium-katalysierten Kupplungsreaktion nach Buchwald und Hartwig direkt zu den erfindungsgemäßen Verbindungen der Formel (Ic) umgesetzt werden. Verbindungen der Formel (XXIII) sind kommerziell erhältlich oder können über für den Fachmann bekannte Methoden hergestellt werden (z.B. J. Med. Chem. (1996), 39, p904ff., T. R. Jones et al.).

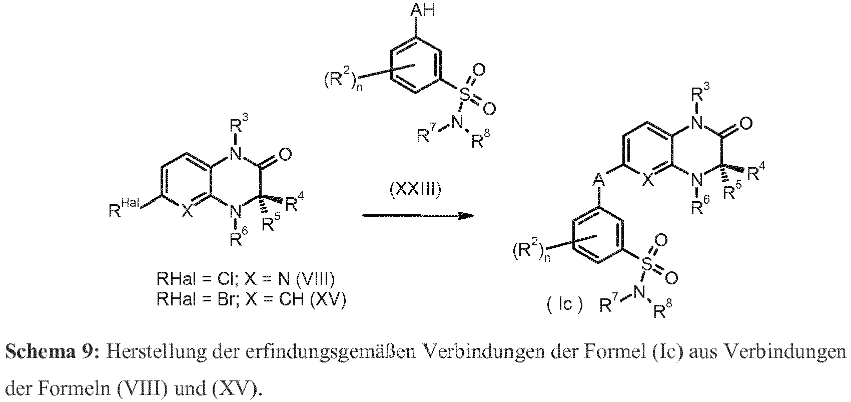

In analoger Weise kann diese Methode, wie in Schema 10 gezeigt, auch als alternative Methode für die Herstellung von Carboxamiden der allgemeinen Formeln (Ia) und (Ib) verwendet werden, indem man die Sulfonamid-Intermediate (XXIII) durch die analogen Carboxamide (XXIIIa) ersetzt, in denen A, R², R⁷, R⁸ und n definiert sind wie in der allgemeinen Formel (I).

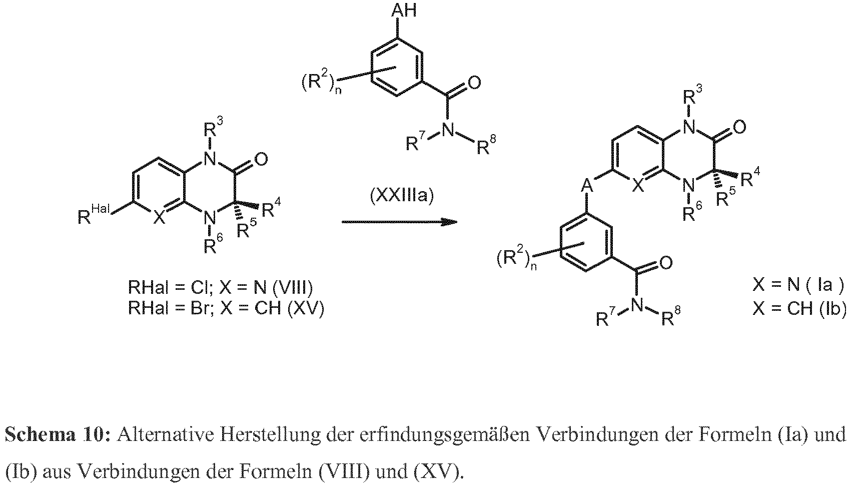

Weiterhin können in ebenfalls analoger Weise aus den halogenierten Zwischenprodukten (VIII) und (XV) durch Umsetzung mit Verbindungen der Formel (XXIIIb), in denen A, R² und n definiert sind wie in der allgemeinen Formel (I), und in denen HetAr für 5-gliedriges monocyclisches Heteroraryl-, wie in Formel (I) für R¹ definiert, steht, erfindungsgemäße Verbindungen der Formel (Id) erhalten werden, wie in Schema 11 gezeigt:

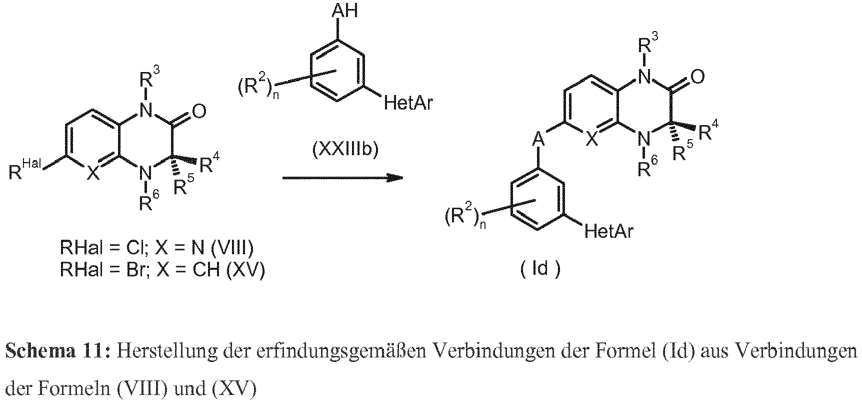

Verbindungen der Formel (XXIIIb) sind in vielen Fällen käuflich beziehungsweise dem Fachmann bekannt. Erfindungsgemäße Verbindungen der Formel (Id) sind weiterhin zugänglich, indem man, wie in Schema 12 gezeigt, Zwischenprodukte der Formel (XXIV), die mit den voranstehend beschriebenen Methoden hergestellt werden können und in denen A, X, R², R³, R⁴, R⁵, R⁶ und n definiert sind wie in der allgemeinen Formel (I), und in denen R^{Hal} für ein Halogen, vorzugsweise Brom oder Iod steht, in einer dem Fachmann geläufigen Suzuki-Kupplung mit einer heteroaromatischen Boronsäure oder einem entsprechendem Boronsäureester, worin HetAr für 5-gliedriges monocyclisches Heteroraryl-, wie in Formel (I) für R¹ definiert, steht, und R für Wasserstoff oder C₁-C₄-Alkyl- steht, oder -B(OR)₂ für einen Pinacolylboronsäureester steht, zu den erfindungsgemäßen Verbindungen der Formel (Id) umsetzt (siehe auch D.G. Hall, Boronic Acids, 2005 WILEY-VCH Verlag GmbH & Co. KGaA, Weinheim, ISBN 3-527-30991-8, und darin zitierte Literatur).

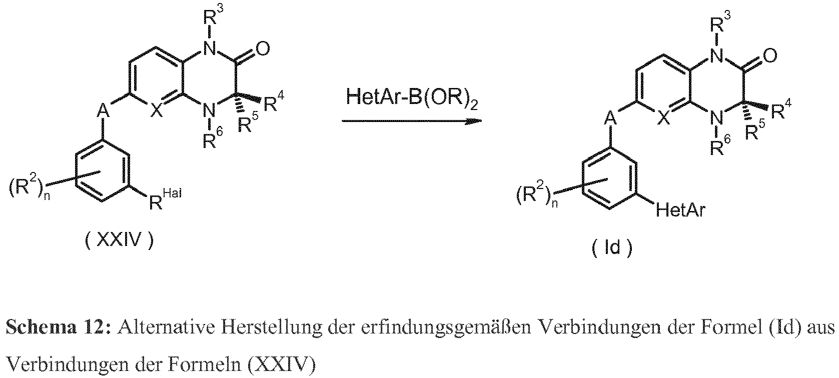

Ferner können die erfindungsgemäßen Verbindungen der Formel (Id) auch aus den in Schemata 5 und 6 gezeigten Ester-Intermediaten der Formeln (IX) und (XVI) sowie Carbonsäuren der Formeln (X) und (XVII) in dem Fachmann bekannter Weise aufgebaut werden, beispielsweise *via* direkte Umsetzung eines Esters mit Hydroxyamidinen wie in der Literatur beschrieben (Tetrahedron Lett. (2006), 47, p4271-4, W. Du et al.). Nach diesem Verfahren können sowohl aliphatisch substituiert Hydroxyamidine wie auch aromatisch substituierte Hydroxyamidine umgesetzt werden. Andere Heterocyclen lassen sich beispielhaft ausgehend von Carbonsäuren der Formel (X) und (XVII) herstellen, die zunächst mit Alkyl- oder Aryl-Hydraziden unter Verwendung von für den Fachmann bekannten Methoden (siehe auch Schema 5) zu Bis-Acyl-Hydraziden umgesetzt werden und anschließend unter Verwendung von für den Fachmann bekannten Reagenzien zur Abspaltung von Wasser, wie beispielsweise Phorphoroxychlorid, Thionylchlorid, p-Toluolsulfonsämechlorid oder Burgess-Reagenz umgesetzt werden. Auf diese Weise sind beispielweise 1,3,4-Oxadiazole (J. Med. Chem. (2005), 48, p4068ff Garcia et al.) zugänglich.

Die beschriebenen Reaktionswege erlauben, dass bei dem Einsatz einer enantiomerenreinen Azidocarbonsäure der Formel (IIa) beziehungsweise von enantiomerenreinen Aminosäuren der Formel (IIb) oder (XIa), beziehungsweise der entsprechenden Ester der Formel (XIb), zu Beginn der Sequenz eine Epimerisierung oder Racemisierung des stereogenen Zentrums am Kohlenstoffatom, welches an R⁴ und R⁵ gebunden ist, weitestgehend unterdrückt werden kann.

Ebenfalls Gegenstand der vorliegenden Erfindung sind die Intermediate der Verbindungen der allgemeinen Formeln (IX) und (XVI), 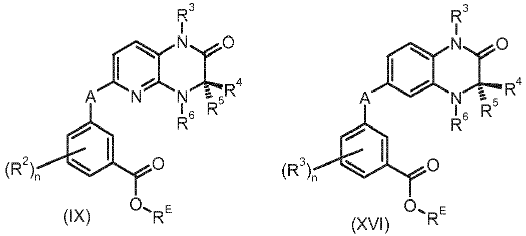 in denen A, R², R³, R⁴, R⁵, R⁶ und n die in der allgemeinen Formel (I) angegebenen Bedeutungen haben und R^{E} für C₁-C₆-Alkyl steht, die bevorzugt zur Herstellung der erfindungsgemäßen Verbindungen der allgemeinen Formel (I) verwendet werden können.

Bevorzugt sind solche Intermediate der allgemeinen Formeln (IX) und (XVI), in denen R^{E} für Methyl oder Ethyl steht.

Ein weiterer Gegenstand der vorliegenden Erfindung sind auch die Intermediate der Verbindungen der allgemeinen Formeln (X) und (XVII), 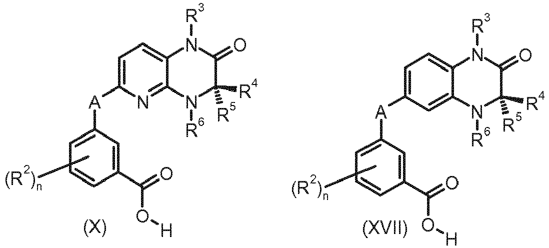 in denen A, R², R³, R⁴, R⁵, R⁶ und n die in der allgemeinen Formel (I) angegebenen Bedeutungen haben, die bevorzugt zur Herstellung der erfindungsgemäßen Verbindungen der allgemeinen Formel (I) verwendet werden können.

### Ausführungsbeispiele

Die nachfolgenden Beispiele erläutern die Herstellung der erfindungsgemäßen Verbindungen, ohne die Erfindung auf diese Beispiele zu beschränken.

Zunächst wird die Herstellung der Intermediate beschrieben, die schließlich zur Herstellung der erfindungsgemäßen Verbindungen bevorzugt zur Anwendung kommen.

IUPAC-Namen wurden erstellt mit Hilfe der Nomenklatursoftware ACD Name batch, Version 12.01, von Advanced Chemical Development, Inc., und bei Bedarf angepaßt, beispielsweise an die deutschsprachige Nomenklatur.

### Stöchiometrie von Salzformen

Wenn bei den im Folgenden beschriebenen Synthese-Intermediaten und Ausführungsbeispielen der Erfindung eine Verbindung in der Form eines Salzes der korrespondierenden Base bzw. Säure aufgeführt ist, so ist die exakte stöchiometrische Zusammensetzung eines solchen Salzes, wie es nach dem jeweiligen Herstell- und/oder Reinigungsverfahren erhalten wurde, in der Regel nicht bekannt. Sofern nicht genauer spezifiziert, sind daher Namens- und Strukturformel-Zusätze wie beispielsweise "Hydrochlorid", "Trifluoracetat", "Natrium-Salz" bzw. "x HCl", "x CF₃COOH", "x Na⁺" bei solchen Salzen nicht stöchiometrisch zu verstehen, sondern haben allein deskriptiven Charakter bezüglich der enthaltenen salzbildenden Komponenten.
Sinngemäß gleiches gilt für den Fall, dass Synthese-Intermediate oder Ausführungsbeispiele oder Salze hiervon nach den beschriebenen Herstell- und/oder Reinigungsverfahren in Form von Solvaten, wie beispielsweise Hydraten, erhalten wurden, deren stöchiometrische Zusammensetzung (sofern definierter Art) nicht bekannt ist.

### Herstellung der Intermediate

### Intermediat 1:

### (2R)-2-Azido-N-(2,6-dichlorpyridin-3-yl)propanamid

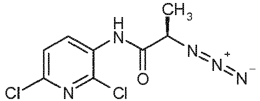

Eine Lösung von 6.6 g (2*R*)-2-Azidopropansäure (Chem. Eur. J. (2010), 16, p. 7572 - 7578) in 250 ml Dimethylacetamid wurde bei -10°C mit 5.02 ml Thionylchlorid tropfenweise versetzt. Es wurde 30 Minuten bei -10°C gerührt und dann 10.6 g 3-Amino-2,6-dichlorpyridin (kommerziell erhältlich; CAS-Nr. 62476-56-6) zugegeben. Es wurde langsam auf RT erwärmt und noch weitere 3 Stunden gerührt. Die Reaktionslösung wurde mit Wasser versetzt und dreimal mit Ethylacetat extrahiert. Die vereinten organischen Phasen wurden mit Wasser und gesättigter Kochsalzlösung gewaschen, über Natriumsulfat getrocknet und im Vakuum eingeengt. Der Rückstand wurde durch Chromatographie an Kieselgel (Hexan / Ethylacetat Gradient) gereinigt. Man erhielt 10.6 g (2R)-2-Azido-*N*-(2,6-dichlorpyridin-3-yl)propanamid.
¹H-NMR (400 MHz, DMSO-d6): δ = 1.47 (d, 3H); 4.27 (q, 1H); 7.61 (d, 1H); 8.22 (d, 1H); 10.08 (bs, 1H).

### Intermediat 2:

### N-(2,6-Dichlorpyridin-3-yl)-D-alaninamid hydrochlorid

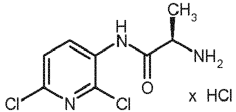

Unter Argon wurde eine Lösung von 10.0 g Intermediat 1 in 150 ml THF bei RT langsam mit 50 ml einer Lösung von Trimethylphosphin (1M in THF) versetzt. Es wurde 14 Stunden bei RT gerührt und dann mit Wasser versetzt. Der Ansatz wurde vollständig im Vakuum eingeengt und der Rückstand in Wasser aufgenommen. Die wäßrige Lösung wurde zweimal mit Dichlormethan extrahiert, die vereinten organischen Phasen wurden über Natriumsulfat getrocknet und im Vakuum vollständig eingeengt. Der Rückstand wurde in Diethylether aufgenommen und mit einer Lösung von Chlorwasserstoff in Diethylether (1M) versetzt. Das dabei entstandene Kristallisat wurde abgesaugt und im Trockenschrank unter Vakuum getrocknet. Man erhielt 11.4 g *N*-(2,6-Dichlorpyridin-3-yl)-D-alaninamid hydrochlorid. Das Produkt wurde ohne weitere Aufreinigung weiter umgesetzt.
¹H-NMR (400 MHz, DMSO-d6): δ = 1.50 (d, 3H); 4.23 (bq, 1H); 7.63 (d, 1H); 8.15 (d, 1H); 8.42 bs, 3H); 10.58 (s, 1H).

### Intermediat 3:

### N²-Cyclopentyl-N-(2,6-dichlorpyridin-3-yl)-D-alaninamid

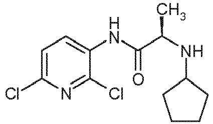

Unter Argon wurde eine Lösung von 10 g Intermediat 2, 4.04 g Cyclopentanon und 6.06 g Natriumacetat in 400 ml Dichlormethan bei 0°C mit 23.5 g Natriumtriacetoxyborhydrid versetzt. Nach 24 Stunden wurde der Ansatz vorsichtig auf gesättigte Natriumhydrogencarbonatlösung gegossen, die Phasen wurden getrennt und die wäßrige Phase mit Dichlormethan extrahiert. Die vereinten organischen Phasen wurden über Natriumsulfat getrocknet und im Vakuum eingeengt. Der Rückstand wurde durch Chromatographie an Kieselgel (Hexan / Ethylacetat Gradient) gereinigt. Man erhielt 8.4 g *N²*-Cyclopentyl-*N*-(2,6-dichlorpyridin-3-yl)-D-alaninamid.
¹H-NMR (400 MHz, DMSO-d6): δ = 1.27 (d, 3H); 1.31-1.41 (m, 2H); 1.42-1.55 (m, 2H); 1.59-1.73 (m, 3H); 1.73-1.83 (m, 1H); 3.06 (qi, 1H); 3.27 (q, 1H); 7.58 (d, 1H); 8.67 (d, 1H).

### Intermediat 4:

### (3R)-6-Chlor-4-cyclopentyl-3-methyl-3,4-dihydropyrido[2,3-b]pyrazin-2(1H)-on

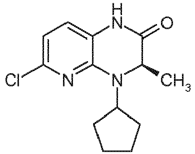

Eine Lösung von 8.4 g Intermediat 3 und 37.8 ml *N,N*-Diisopropylethylamin in 200 ml DMF wurde. 96 Stunden bei 170°C Badtemperatur gerührt. Nach dem Erkalten wurde der Ansatz mit Wasser verdünnt und dreimal mit Dichlormethan extrahiert. Die vereinten organischen Phasen wurden im Vakuum eingeengt. Es wurde mit Toluol versetzt und nochmals im Vakuum vollständig eingeengt. Der Rückstand wurde durch Chromatographie an Kieselgel (Hexan / Ethylacetat Gradient) gereinigt. Man erhielt 6.7 g (3*R*)-6-Chlor-4-cyclopentyl-3-methyl-3,4-dihydropyrido[2,3-*b*]pyrazin-2(1*H*)-on.
¹H-NMR (400 MHz, DMSO-d6): δ = 1.15 (d, 3H); 1.47-1.83 (sm, 6H); 1.84-1.98 (m, 2H); 4.12 (q, 1H); 4.19 (qi, 1H); 6.67 (d, 1H); 7.00 (d, 1H); 10.61 (s, 1H).

### Intermediat 5:

### (3R)-6-Chlor-4-cyclopentyl-1,3-dimethyl-3,4-dihydropyrido[2,3-b]pyrazin-2(1H)-on

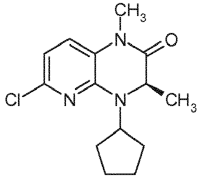

Eine Lösung von 6.7 g Intermediat 4 und 2.35 ml Methyljodid in 180 ml DMF wurde bei 0°C portionsweise mit 1.51 g Natriumhydrid (60% in Weißöl) versetzt. Nach einer Stunde Rühren bei 0°C wurde der Ansatz auf Eiswasser gegossen und mit gesättigter wäßriger Ammoniumchlorid-lösung neutralisiert. Der Ansatz wurde dreimal mit Ethylacetat extrahiert, die vereinten organischen Phasen wurden mit Wasser gewaschen, über Natriumsulfat getrocknet und im Vakuum vollständig eingeengt. Der Rückstand wurde durch Chromatographie an Kieselgel (Hexan / Ethylacetat 2:1) gereinigt. Man erhielt 7.1 g (3*R*)-6-Chlor-4-cyclopentyl-1,3-dimethyl-3,4-dihydro-pyrido[2,3-*b*]pyrazin-2(1*H*)-on.
¹H-NMR (400 MHz, DMSO-d6): δ = 1.11 (d, 3H); 1.48-1.62 (m, 2H); 1.63-1.82 (m, 4H); 1.87-1.98 (m, 2H); 3.23 (s, 3H); 4.21 (qi, 1H); 4.27 (q, 1H); 6.78 (d, 1H); 7.31 (d, 1H).

### Intermediat 6:

### 3-{[(3R)-4-Cyclopentyl-1,3-dimethyl-2-oxo-1,2,3,4-tetrahydropyrido[2,3-b]pyrazin-6-yl]-amino}benzoesäuremethylester

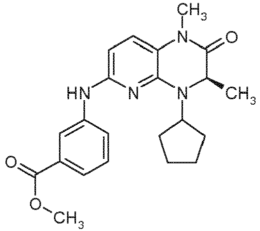

Eine Suspension von 900 mg Intermediat 5, 923 mg 3-Aminobenzoesäuremethylester (CAS 4518-10-9), 137 mg Palladiumacetat, 4.98 g Caesiumcarbonat und 380 mg (+)-BINAP in 68 ml Toluol wurde 3.5 Stunden unter Argon bei 110°C gerührt. Die Reaktionslösung wurde ab filtriert, der Rückstand mit Ethylacetat nachgewaschen und die vereinten organischen Phase vollständig im Vakuum eingeengt. Der Rückstand wurde durch Chromatographie an Kieselgel (Hexan / Ethyl acetat Gradient) gereinigt. Man erhielt 850 mg 3-{[(3R)-4-Cyclopentyl-1,3-dimethyl-2-oxo-1,2,3,4-tetrahydropyrido[2,3-*b*]pyrazin-6-yl]amino}benzoesäuremethylester.
¹H-NMR (400 MHz, CDCl₃): δ = 1.22 (d, 3H); 1.57-1.85 (m, 6H); 1.99-2.14 (m, 2H); 3.31 (s, 3H); 3.92 (s, 3H); 4.29 (q, 1H); 4.51 (qi, 1H); 6.25 (d, 1H); 6.32 (s, 1H); 7.01 (d, 1H); 7.35 (t, 1H); 7.54 (d, 1H); 7.63 (d, 1H); 8.09 (s, 1H).

### Intermediat 7:

### 3-{[(3R)-4-Cyclopentyl-1,3-dimethyl-2-oxo-1,2,3,4-tetrahydropyrido[2,3-b]pyrazin-6-yl]-amino}benzoesäure

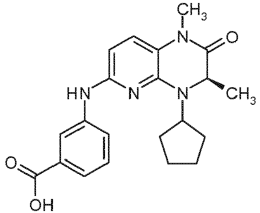

Eine Lösung von 820 mg Intermediat 6 in 6.5 ml THF und 49 ml Methanol wurde bei RT mit 21 ml 1N Lithiumhydroxidlösung versetzt und bei 60°C für 5.5 Stunden gerührt. Es wurde auf Wasser gegeben und mit Ethylacetat extrahiert. Die wäßrige Phase wurde mit Salzsäure auf pH<3 eingestellt und mit Ethylacetat dreimal extrahiert. Die vereinten organischen Phasen wurden mit Natriumsulfat getrocknet und das Lösungsmittel vollständig im Vakuum entfernt. Man erhielt 820 mg 3-{[(3*R*)-4-Cyclopentyl-1,3-dimethyl-2-oxo-1,2,3,4-tetrahydropyrido[2,3-*b*]pyrazin-6-yl]-amino}benzoesäure.
¹H-NMR (400 MHz, DMSO-d6): δ = 1.06 (d, 3H); 1.45-1.75 (m, 6H); 1.92-2.09 (m, 2H); 3.20 (s, 3H); 4.19 (q, 1H); 4.54 (qi, 1H); 6.24 (d, 1H); 7.25 (d, 1H); 7.31 (t, 1H); 7.39 (d, 1H); 7.64 (dd, 1H); 8.47 (s, 1H); 8.98 (s, 1H); 12.65 (bs, 1H).

### Intermediat 8:

### N-(2,6-Dichlorpyridin-3-yl)-N²-(tetrahydro-2H-pyran-4-yl)-D-alaninamid

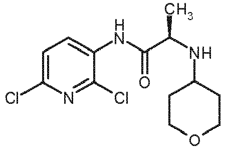

In Analogie zur Herstellung von Intermediat 3 wurde *N*-(2,6-Dichloropyridin-3-yl)-*N²*-(tetrahydro-2*H*-pyran-4-yl)-D-alaninamid ausgehend von 8 g Intermediat 2, 3.85 mg Tetrahydro-2*H-*pyran-4-on, 4.81 g Natriumacetat und 18.8 g Natriumtriacetoxyborhydrid in 426 ml Dichlormethan bei 0°C hergestellt. Man erhielt 8.7 g *N*-(2,6-Dichloropyridin-3-yl)-*N²*-(tetrahydro-2*H-*pyran-4-yl)-D-alaninamid. Dieses wurde als Rohprodukt in die Synthese von Intermediat 9 eingesetzt.

Vergrösserter Ansatz:
Eine Suspension von 20 g Intermediat 2 und 9.6 g Tetrahydro-4H-pyran-4-on in 1.07 L Dichlormethan wurde bei 0°C mit 12.1 g Natriumacetat und 47 g Natriumtriacetoxyborhydrid versetzt. Es wurde 16 Stunden unter Erwärmung auf RT gerührt. Der Ansatz wurde vorsichtig in gesättigte Natriumhydrogencarbonat Lösung gegossen und gerührt. Die Phasen wurden getrennt und die wässrige Phase einmal mit Dichlormethan extrahiert. Die vereinten organischen Phasen wurden über Natriumsulfat getrocknet und das Lösungsmittel vollständig im Vakuum entfernt. Der Rückstand wurde durch Chromatographie an Kieselgel (Hexan / Ethylacetat Gradient) gereinigt. Man erhielt 15 g *N*-(2,6-Dichlorpyridin-3-yl)-*N*²-(tetrahydro-2H-pyran-4-yl)-D-alaninamid.
   ¹H-NMR (400 MHz, CDCl₃): δ = 1.37-1.55 (m+d, 5H); 1.81-1.89 (m, 1H); 1.91-1.99 (m, 1H); 2.67-2.76 (m, 1H); 3.38 (dt, 2H); 3.45 (q, 1H); 3.95-4.05 (m, 2H); 7.29 (d, 1H); 8.85 (d, 1H); 10.33 (s, 1H).

### Intermediat 9:

### (3R)-6-Chlor-3-methyl-4-(tetrahydro-2H-pyran-4-yl)-3,4-dihydropyrido[2,3-b]pyrazin-2(1H)-on

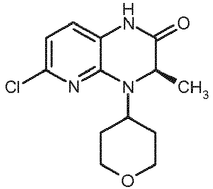

In Analogie zur Synthese von Intermediat 4 wurden ausgehend von 5 g Intermediat 8 und 40 ml *N,N*-Diisopropylethylamin in 242 ml DMF nach 45 Stunden bei 170°C Badtemperatur (3R)-6-Chlor-3-methyl-4-(tetrahydro-2*H-*pyran-4-yl)-3,4-dihydropyrido[2,3-*b*]pyrazin-2(1*H*-on dargestellt. Man erhielt 2.33 g.

Vergrösserter Ansatz:
Eine Lösung von 7.8 g Intermediat 8 und 31.7 ml *N,N*-Diisopropylethylamin in 170 ml DMF wurde in 4 einzelne, verschlossene Druckgefässe verteilt und 10 Stunden bei 175°C Badtemperatur geheizt. Die Lösungen wurde nach Abkühlen auf RT wieder vereint, mit Ethylacetat verdünnt und dreimal mit halbgesättigter Natriumchlorid Lösung extrahiert. Die organsiche Phase wurde über Natriumsulfat getrocknet und das Lösungsmittel vollständig im Vakuum entfernt. Der Rückstand wurde durch Chromatographie an Kieselgel (Dichlormethan / Methanol Gradient) gereinigt. Man erhielt 4.1 g (3*R*)-6-Chlor-3-methyl-4-(tetrahydro-2H-pyran-4-yl)-3,4-dihydropyrido[2,3-b]pyrazin-2(1H)-on.
   ¹H-NMR (300 MHz, CDCl₃): δ = 1.32 (d, 3H); 1.60-1.70 (m, 1H); 1.74-1.90 (m, 1H); 1.90-2.02 (m, 1H); 2.12-2.22 (m, 1H); 3.50-3.65 (m, 2H); 4.02-4.14 (m, 2H); 4.25 (q, 1H); 4.56 (tt, 1H); 6.65 (d, 1H); 6.91 (d, 1H); 8.68 (s, 1H).

### Intermediat 10:

### (3R)-6-Chlor-1,3-dimethyl-4-(tetrahydro-2H-pyran-4-yl)-3,4-dihydropyrido[2,3-b]pyrazin-2(1H)-on

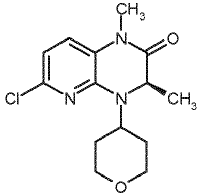

In Analogie zur Herstellung von Intermediat 5 wurde (3*R*)-6-Chlor-1,3-dimethyl-4-(tetrahydro-2*H-*pyran-4-yl)-3,4-dihydropyrido[2,3-*b*]pyrazin-2(1*H*)-on ausgehend von 2.3 g Intermediat 9, 465 mg Natriumhydrid (60% in Weissöl) und 0.73 ml Methyljodid in 98 ml DMF hergestellt. Nach Chromatographie an Kieselgel (Dichlormethan /Methanol Gradient) erhielt man 2.3 g (3*R*)-6-Chlor-1,3-dimethyl-4-(tetrahydro-2*H*-pyran-4-yl)-3,4-dihydropyrido[2,3-*b*]pyrazin-2(1*H*)-on.

Vergrößerter Ansatz:
Eine Lösung von 3.2 g Intermediat 9, 647 mg Natriumhydrid (60% in Weissöl) und 1.01 ml Methyljodid in 137 ml DMF wurde 16 Stunden bei RT gerührt. Der Ansatz wurde auf Wasser gegossen und dreimal mit Ethylacetat extrahiert. Die vereinten organischen Phasen wurden mit gesättigter Ammoniumchlorid-Lösung und halbgesättigter Natriumchlorid-Lösung gewaschen, über Natrimsulfat getrocknet und das Lösungsmittel vollständig im Vakuum entfernt. Man erhielt 2.8 g (³*R*)-6-Chlor-1,3-dimethyl-4-(tetrahydro-2H-pyran-4-yl)-3,4-dihydropyrido[2,3-b]pyrazin-2(1H)-on. Diese Verbindung wies in der Regel eine Enantiomerenreinheit von >90% ee auf, konnte aber durch chirale präparative HPLC noch weiter aufgereinigt werden.
   ¹H-NMR (400 MHz, CDCl₃): δ = 1.23 (d, 3H); 1.62-1.69 (m, 1H); 1.81 (dq, 1H); 1.96 (dq, 1H); 2.02-2.09 (m, 1H); 3.31 (s, 1H); 3.51-3.62 (m, 2H); 4.02-4.10 (m, 2H); 4.31 (q, 1H); 4.54 (tt, 1H); 6.70 (d, 1H); 7.00 (d, 1H).

Instrument: Agilent Prep 1200; Säule: Chiralpak IC 5 µm 250x30 mm; Eluent Hexan / 2-Propanol 70:30 (v/v); Flussrate 35 ml/min; Temperatur: 25 °C; Detektor: DAD 996 scan: 280 nm. Rt = 12.3-13.8 min

### Intermediat 11:

### N-(5-Brom-2-nitrophenyl)-D-alanin

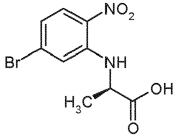

Eine Lösung von 13.57 g 4-Brom-2-Fluomitrobenzol, 5.49 g D-Alanin und 10.66 g Kaliumcarbonat in 150 ml Ethanol und 60 ml Wasser wurde für 6 Stunden unter Rückfluss erhitzt. Nach Abkühlen auf Raumtemperatur wurde das Reaktionsgemisch durch Zugabe von 1M Salzsäure sauer gestellt und das entstandenen Produkt als Niederschlag abfiltriert. Man erhielt 17.36 g *N-*(5-Brom-2-nitrophenyl)-D-alanin.
¹H-NMR (400 MHz, CDCl₃): δ = 1.46 (d, 3H); 4.52-4.62 (m, 1H); 6.89 (dd, 1H); 7.22 (d, 1H); 8.01 (d, 1H); 8.38 (d, 1H).

### Intermediat 12:

### (3R)-6-Brom-3-methyl-3,4-dihydrochinoxalin-2(1H)-on

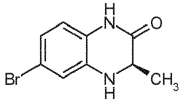

Eine Lösung von 5.19g Intermediat 11 und 4.96 g Kaliumcarbonat in 150 ml Wasser wurde mit einer Lösung von 9.37 g Natriumdithionit in 50 ml Wasser bei RT über 30 Minuten tropfenweise versetzt. Nach weiteren 30 Minuten bei RT wurde das Reaktionsgemisch durch Zugabe von 2M Salzsäure sauer gestellt und noch kurz gerührt. Es wurde mit Kaliumcarbonat neutralisiert und mit Dichlormethan extrahiert. Die organische Phase wurde über Natriumsulfat getrocknet und vollständig im Vakuum eingeengt. Man erhielt 1.88 g (3*R*)-6-Brom-3-methyl-3,4-dihydrochinoxalin-2(1*H*)-on.
¹H-NMR (400 MHz, CDCl₃): δ = 1.47 (d, 3H); 3.90 (bs, 1H); 4.03 (q, 1H); 6.62 (d, 1H); 6.82 (d, 1H); 6.87 (dd, 1H); 8.68 (bs, 1H).

### Intermediat 13:

### (3R)-6-Brom-3-methyl-4-(tetrahydro-2H-pyran-4-yl)-3,4-dihydrochinoxalin-2(1H)-on

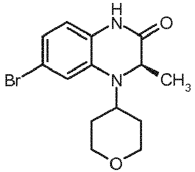

Eine Lösung von 1.54 g Intermediat 12, 1.9 g Tetrahydro-4*H*-pyran-4-on, 2.1 g Phenylsilan und 1.94 g Dibutylzinndichlorid in 40 ml THF wurde 96 Stunden bei RT gerührt. Die Lösung wurde im Vakuum vollständig eingeengt. Der Rückstand wurde durch Chromatographie an Kieselgel (Hexan / Ethylacetat Gradient) gereinigt. Man erhielt 1.97 g (3*R*)-6-Brom-3-methyl-4-(tetrahydro-2*H-*pyran-4-yl)-3,4-dihydrochinoxalin-2(1*H*-on.
¹H-NMR (400 MHz, CDCl₃): δ = 1.18 (d, 3H); 1.62-1.71 (m, 1H); 1.78-2.00 (m, 3H); 3.41-3.56 (m, 2H); 3.62 (tt, 1H); 4.00-4.17 (m, 3H); 6.71 (d, 1H); 6.94 (dd, 1H); 6.98 (d, 1H); 9.50 (s, 1H).

### Intermediat 14:

### (3R)-6-Brom-1,3-dimethyl-4-(tetrahydro-2H-pyran-4-yl)-3,4-dihydrochinoxalin-2(1H)-on

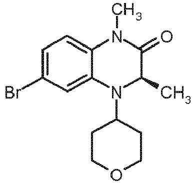

In Analogie zur Herstellung von Intermediat 5 wurde (3*R*)-6-Brom-1,3-dimethyl-4-(tetrahydro-2*H-*pyran-4-yl)-3,4-dihydrochinoxalin-2(1*H*-on ausgehend von 1.97 g Intermediat 13, 363 mg Natriumhydrid (60% in Weissöl) und 0.57 ml Methyljodid in 35 ml DMF hergestellt. Nach Chromatographie an Kieselgel (Hexan / Ethylacetat Gradient) erhielt man 1.54 g (3*R*)-6-Brom-1,3-dimethyl-4-(tetrahydro-2*H-*pyran-4-yl)-3,4-dihydrochinoxalin-2(1*H*-on.
¹H-NMR (400 MHz, CDCl₃): δ = 1.10 (d, 3H); 1.62-1.73 (m, 1H); 1.74-2.00 (m, 3H); 3.35 (s, 3H); 3.41-3.57 (m, 2H); 3.61 (tt, 1H); 4.00-4.20 (m, 3H); 6.81 (d, 1H); 6.97 (d, 1H); 7.01 (dd, 1H).

### Intermediat 15:

### (3R)-6-Brom-4-(4-methoxybenzyl)-3-methyl-3,4-dihydrochinoxalin-2(1H-on

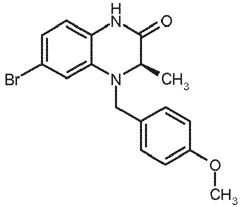

Eine Lösung von 1.54 g Intermediat 12, 2.59 g 4-Methoxybenzaldehyd, 2.06 g Phenylsilan und 1.93 g Dibutylzinndichlorid in 40 ml THF wurde 96 Stunden bei RT gerührt. Die Lösung wurde im Vakuum vollständig eingeengt. Der Rückstand wurde durch Chromatographie an Kieselgel (Hexan / Ethylacetat Gradient) gereinigt. Man erhielt 2.06 g (3R)-6-Brom-4-(4-methoxybenzyl)-3-methyl-3,4-dihydrochinoxalin-2(1*H*)-on.
¹H-NMR (400 MHz, CDCl₃): δ = 1.18 (d, 3H); 3.82 (s, 3H); 3.90 (q, 1H); 4.09 (d, 1H); 4.51 (d, 1H); 6.65 (d, 1H); 6.85-6.95 (m, 4H); 7.24 (d, 2H); 9.00 (bs, 1H).

### Intermediat 16:

### (3R)-6-Brom-4-(4-methoxybenzyl)-1,3-dimethyl-3,4-dihydrochinoxalin-2(1H)-on

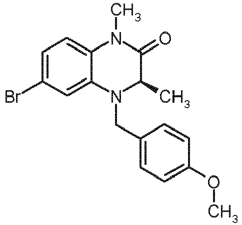

In Analogie zur Herstellung von Intermediate 5 wurde (3R)-6-Brom-4-(4-methoxybenzyl)-1,3-dimethyl-3,4-dihydrochinoxalin-2(1*H*)-on ausgehend von 2.03 g Intermediat 15, 337 mg Natriumhydrid (60% in Weissöl) und 0.52 ml Methyljodid in 35 ml DMF hergestellt. Nach Chromatographie an Kieselgel (Hexan / Ethylacetat Gradient) erhielt man 1.34 g (3*R*)-6-Brom-4-(4-methoxybenzoyl)-1,3-dimethyl-3,4-dihydrochinoxalin-2(1*H*)-on.
¹H-NMR (400 MHz, CDCl₃): δ = 0.99 (d, 3H); 3.26 (s, 3H); 3.74 (s, 3H); 3.90 (q, 1H); 4.15 (d, 1H); 4.50 (d, 1H); 6.88 (bs, 1H); 6.91 (d, 2H); 6.96-7.01 (m, 2H); 7.27 (d, 2H).

### Intermediat 17:

### 3-{[(3R)-1,3-Dimethyl-2-oxo-4-(tetrahydro-2H-pyran-4-yl)-1,2,3,4-tetrahydropyrido[2,3-b]-pyrazin-6-yl]amino}benzoesäuremethylester

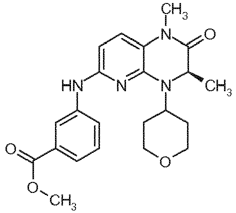

In Analogie zur Herstellung von Intermediat 6 wurde 3-{[(3*R*)-1,3-Dimethyl-2-oxo-4-(tetrahydro-2*H*-pyran-4-yl)-1,2,3,4-tetrahydropyrido[2,3-*b*]pyrazin-6-yl]amino}benzoesäuremethylester ausgehend von 1 g Intermediat 10 und 971 mg 3-Aminobenzoesäuremethylester hergestellt. Nach Chromatographie an Kieselgel (Hexan / Ethylacetat Gradient) erhielt man 280 mg 3-{[(3*R*)-1,3-Dimethyl-2-oxo-4-(tetrahydro-2*H*-pyran-4-yl)-1,2,3,4-tetrahydropyrido[2,3-*b*]pyrazin-6-yl]amino}-benzoesäuremethylester. Dieser Ansatz wurde zweimal durchgeführt.
¹H-NMR (400 MHz, CDCl₃): δ = 1.25 (d, 3H); 1.68 (bd, 1H); 1.82 (dq, 1H); 1.99 (dq, 1H); 2.05-2.15 (m, 1H); 3.32 (s, 3H); 3.50-3.65 (m, 2H); 3.92 (s, 3H); 4.01-4.13 (m, 2H); 4.31 (q, 1H); 4.59 (bs, 1H); 6.26 (d, 1H); 6.32 (bs, 1H); 7.06 (bd, 1H); 7.37 (t, 1H); 7.58 (d, 1H); 7.66 (bs, 1H); 8.05 (s, 1H).

### Intermediat 18:

### 3-{[(3R)-1,3-Dimethyl-2-oxo-4-(tetrahydro-2H-pyran-4-yl)-1,2,3,4-tetrahydropyrido[2,3-b]-pyrazin-6-yl]amino}benzoesäure

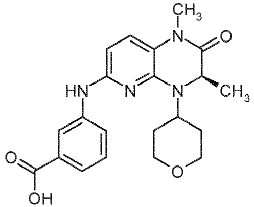

In Analogie zur Herstellung von Intermediat 7 wurde 3-{[(3*R*)-1,3-Dimethyl-2-oxo-4-(tetrahydro-2*H*-pyran-4-yl)-1,2,3,4-tetrahydropyrido[2,3-*b*]pyrazin-6-yl]amino}benzoesäure ausgehend von 500 mg 3-{[(3*R*)-1,3-Dimethyl-2-oxo-4-(tetrahydro-2*H-*pyran-4-yl)-1,2,3,4-tetrahydropyrido[2,3-*b*]pyrazin-6-yl]amino}benzoesäuremethylester (hergestellt wie unter Intermediat 17 beschrieben) und 277 mg Lithiumhydroxid hergestellt. Man erhielt 370 mg 3-{[(3*R*)-1,3-Dimethyl-2-oxo-4-(tetrahydro-2*H*-pyran-4-yl)-1,2,3,4-tetrahydropyrido[2,3-*b*]pyrazin-6-yl]amino}benzoesäure.
¹H-NMR (400 MHz, DMSO-d6): δ = 1.08 (d, 3H); 1.53-1.63 (m, 1H); 1.72 (dq, 1H); 1.87 (dq, 1H); 1.89-1.99 (m, 1H); 3.21 (s, 3H); 3.44 (dt, 1H); 3.52 (dt, 1H); 3.86-3.97 (m, 2H); 4.23 (q, 1H); 4.51 (tt, 1H); 6.25 (d, 1H); 7.27 (d, 1H); 7.33 (t, 1H); 7.41 (d, 1H); 7.74 (bd, 1H); 8.29 (t, 1H); 9.01 (s, 1H); 12.79 (bs, 1H).

### Intermediat 19:

### 3-{[(3R)-1,3-Dimethyl-2-oxo-4-(tetrahydro-2H-pyran-4-yl)-1,2,3,4-tetrahydropyrido[2,3-b]-pyrazin-6-yl]amino}-4-methoxybenzoesäuremethylester

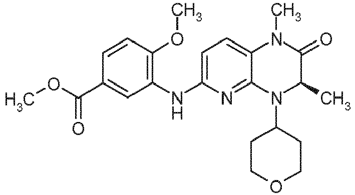

In Analogie zur Herstellung von Intermediat 6 wurde 3-{[(3*R*)-1,3-Dimethyl-2-oxo-4-(tetrahydro-2*H*-pyran-4-yl)-1,2,3,4-tetrahydropyrido[2,3-*b*]pyrazin-6-yl]amino}-4-methoxybenzoesäuremethylester ausgehend von 1 g Intermediat 10 und 1.16 g 3-Amino-4-methoxybenzoesäuremethylester (CAS 24812-90-6) hergestellt. Nach Chromatographie an Kieselgel (Hexan / Ethylacetat Gradient) erhielt man 950 mg 3-{[(3*R*)-1,3-Dimethyl-2-oxo-4-(tetrahydro-2H-pyran-4-yl)-1,2,3,4-tetrahydropyrido[2,3-*b*]pyrazin-6-yl]amino}-4-methoxybenzoesäuremethylester.
¹H-NMR (400 MHz, CDCl₃): δ = 1.23 (d, 3H); 1.62-1.72 (m, 1H); 1.79 (dq, 1H); 1.94 (dq, 1H); 2.07-2.14 (m, 1H); 3.32 (s, 3H); 3.57 (dt, 1H); 3.63 (dt, 1H); 3.89 (s, 3H); 3.97 (s, 3H); 3.97-4.06 (m, 2H); 4.31 (q, 1H); 4.71 (tt, 1H); 6.26 (d, 1H); 6.76 (s, 1H); 6.90 (d, 1H); 7.06 (d, 1H); 7.63 (dd, 1H); 8.74 (d, 1H).

### Intermediat 20:

### 3-{[(3R)-1,3-Dimethyl-2-oxo-4-(tetrahydro-2H-pyran-4-yl)-1,2,3,4-tetrahydropyrido[2,3-b]-pyrazin-6-yl]amino}-4-methoxybenzoesäure

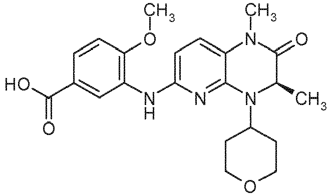

In Analogie zur Herstellung von Intermediat 7 wurde 3-{[(3*R*)-1,3-Dimethyl-2-oxo-4-(tetrahydro-2*H*-pyran-4-yl)-1,2,3,4-tetrahydropyrido[2,3-*b*]pyrazin-6-yl]amino}-4-methoxybenzoesäure ausgehend von 930 mg Intermediat 19 und 480 mg Lithiumhydroxid hergestellt. Man erhielt 520 mg 3-{[(3*R*)-1,3-Dimethyl-2-oxo-4-(tetrahydro-2*H*-pyran-4-yl)-1,2,3,4-tetrahydropyrido[2,3-*b*]-pyrazin-6-yl]amino}-4-methoxybenzoesäure.
¹H-NMR (400 MHz, CDCl₃): δ = 1.31 (d, 3H); 1.64-1.72 (m, 1H); 1.84 (dq, 1H); 1.98 (dq, 1H); 2.25-2.33 (m, 1H); 3.34 (s, 3H); 3.69 (dt, 1H); 3.88 (dt, 1H); 3.99 (s, 3H); 3.99-4.14 (m, 2H); 4.36 (q, 1H); 4.94-5.05 (m, 1H); 6.33 (d, 1H); 7.01 (d, 1H); 7.24 (d, 1H); 7.92 (d, 1H); 8.26 (bs, 1H).

### Intermediat 21:

### N-(2,6-Difluorobenzyl)alaninmethylester

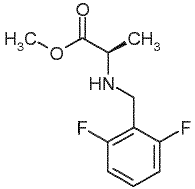

Eine Lösung von 2.9 g 2,6-Difluorbenzaldehyd, 3.35 g D-Alaninmethylester Hydrochlorid und 3.3 ml Triethylamin in 100 ml Dichlormethan wurde bei RT mit 8.5 g Natriumtriacetoxyborhydrid versetzt. Es wurde 30 Minuten gerührt und dann langsam mit 2.3 ml Eisessig versetzt. Es wurde über Nacht gerührt und dann mit Natriumhydrogencarbonatlösung versetzt. Die organische Phase wurde abgetrennt, über Natriumsulfat getrocknet und das Lösungsmittel im Vakuum entfernt. Man erhielt 4.7 g *N*-(2,6-Difluorobenzyl)alaninmethylester.

UPLC-MS: Instrument: Waters Acquity UPLC-MS SQD; Säule: Acquity UPLC BEH C18 1.7 50x2.1mm; Eluent A: Wasser + 0.1% Vol. Ameisensäure (99%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm
Rt = 0.53 min.

### Intermediat 22:

### 6-Brom-4-(2,6-difluorbenzyl)-3-methyl-3,4-dihydrochinoxalin-2(1H)-on

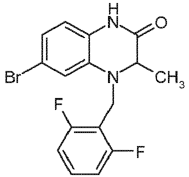

Eine Mischung aus 2.3 g *N*-(2,6-Difluorobenzyl)alaninmethylester (Intemediat 21), 2 g 4-Brom-2-fluornitrobenzol und 1.53 g Kaliumcarbonat in 20 ml Ethanol und 8 ml Wasser wurde 6 Stunden bei 100°C gerührt. Es wurde weitere 72 Stunden bei RT gerührt und dann mit Wasser verdünnt. Es wurde 1N Salzsäure zugegeben, bis der pH der Mischung < 7 war. Der entstehende Niederschlag wurde abgesaugt. Der Ansatz wurde im gleichen Massstab wiederholt und man erhielt zusammen 4.7 g *N*-(5-Brom-2-nitrophenyl)-*N*-(2,6-difluorbenzyl)alanin. Davon wurden 2.2 g in 12 ml Methanol und 12 ml Eisessig mit 1.04 g Eisenpulver versetzt und 2 Stunden bei 105°C gerührt. Diese Reaktion wurde mit weiteren 2.4 g *N*-(5-Brom-2-nitrophenyl)-*N*-(2,6-difluorbenzyl)alanin und 1.13 g Eisenpulver wiederholt. Nach vollständiger Reaktion wurden beide Ansätze vereinigt. Es wurde filtriert, zum Filtrat gesättigte Natriumhydrogencarbonatlösung zugegeben und mit Dichlormethan extrahiert. Die organische Phase wurde im Vakuum eingeengt und der Rückstand durch Chromatographie an Kieselgel (Dichlormethan / Methanol Gradient) gereinigt. Man erhielt 970 mg 6-Brom-4-(2,6-difluorbenzyl)-3-methyl-3,4-dihydrochinoxalin-2(1*H*)-on.
¹H NMR (300 MHz, DMSO-*d₆*) δ = 1.07 (d, 3H); 3.73 (d, 1H); 4.31 (s, 1 H); 4.26 (s, 1 H); 4.69 (s, 1 H); 4.64 (s, 1 H); 6.72 (d, 1H); 6.88 (dd, 1H); 7.03 (d, 1H); 7.09 - 7.22 (m, 2H); 7.36 - 7.52 (m, 1H); 10.51 (s, 1H).

### Intermediat 23:

### 6-Brom-4-(2,6-difluorbenzyl)-1,3-dimethyl-3,4-dihydrochinoxalin-2(1H)-on

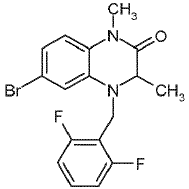

In Analogie zur Herstellung von Intermediat 5 wurde 6-Brom-4-(2,6-difluorbenzyl)-1,3-dimethyl-3,4-dihydrochinoxalin-2(1*H*)-on ausgehend von 970 mg Intermediat 22, 170 mg Natriumhydrid (60% in Weissöl) und 0.24 ml Methyljodid in 15 ml DMF hergestellt. Nach extraktiver Aufarbeitung erhielt man 1.15 g (3R)-6-Brom-4-(4-methoxybenzyl)-1,3-dimethyl-3,4-dihydrochinoxalin-2(1*H*)-on als Rohprodukt.

UPLC-MS: Instrument: Waters Acquity UPLC-MS SQD; Säule: Acquity UPLC BEH C18 1.7 50x2.1mm; Eluent A: Wasser + 0.1% Vol. Ameisensäure (99%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm
Rt = 1.36 min.

### Intermediat 24:

### 3-{[4-(2,6-Difluorbenzyl)-1,3-dimethyl-2-oxo-1,2,3,4-tetrahydrochinoxalin-6-yl]amino}-benzoesäureethylester

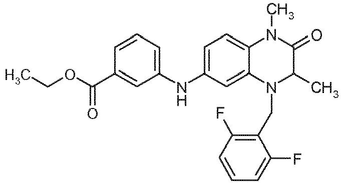

In Analogie zur Herstellung von Intermediat 6 wurde 3-{[4-(2,6-Difluorbenzyl)-1,3-dimethyl-2-oxo-1,2,3,4-tetrahydrochinoxalin-6-yl]amino}benzoesäureethylester ausgehend von 182 mg Intermediat 23 und 148 mg 3-Aminobenzoesäureethylester hergestellt. Nach Chromatographie an Kieselgel (Hexan / Ethylacetat Gradient) erhielt man 60 mg 3-{[4-(2,6-Difluorbenzyl)-1,3-dimethyl-2-oxo-1,2,3,4-tetrahydrochinoxalin-6-yl]amino}benzoesäureethylester.

UPLC-MS: Instrument: Waters Acquity UPLC-MS SQD; Säule: Acquity UPLC BEH C18 1.7 50x2.1mm; Eluent A: Wasser + 0.1% Vol. Ameisensäure (99%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm
Rt = 1.38 min.

### Intermediat 25:

### 3-{[4-(2,6-Difluorbenzyl)-1,3-dimethyl-2-oxo-1,2,3,4-tetrahydrochinoxalin-6-yl]amino}-benzoesäure

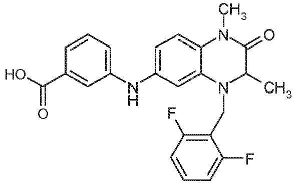

In Analogie zur Herstellung von Intermediat 7 wurde 3-{[4-(2,6-Difluorbenzyl)-1,3-dimethyl-2-oxo-1,2,3,4-tetrahydrochinoxalin-6-yl]amino}benzoesäure ausgehend von 60 mg Intermediat 24 und 26 mg Natriumhydroxid hergestellt. Man erhielt 40 mg 3-{[4-(2,6-Difluorbenzyl)-1,3-dimethyl-2-oxo-1,2,3,4-tetrahydrochinoxalin-6-yl]amino}benzoesäure.

UPLC-MS: Instrument: Waters Acquity UPLC-MS SQD; Säule: Acquity UPLC BEH C18 1.7 50x2.1mm; Eluent A: Wasser + 0.1% Vol. Ameisensäure (99%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm Rt = 1.12 min.

### Intermediat 26:

### N²-Cyclohexyl-N-(2,6-dichlorpyridin-3-yl)-D-alaninamid

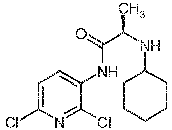

In Analogie zur Herstellung von Intermediat 3 wurde *N²*-Cyclohexyl-*N*-(2,6-dichlorpyridin-3-yl)-D-alaninamid ausgehend von 1.5 g Intermediat 2, 707 mg Cyclohexanon, 909 mg Natriumacetat und 3.5 g Natriumtriacetoxyborhydrid in 80 ml Dichlormethan bei 0°C hergestellt. Man erhielt 1.3 g *N*²-Cyclohexyl-*N*-(2,6-dichlorpyridin-3-yl)-D-alaninamid als Rohprodukt, welches ohne weitere Reinigung in der nächsten Stufe eingesetzt werden konnte.

UPLC-MS: Instrument: Waters Acquity UPLC-MS SQD; Säule: Acquity UPLC BEH C18 1.7 50x2.1mm; Eluent A: Wasser + 0.1% Vol. Ameisensäure (99%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm.
Rt = 1.49 min (M⁺+1 = 316, 318, 320)

### Intermediat 27:

### (3R)-6-Chlor-4-cyclohexyl-3-methyl-3,4-dihydropyrido[2,3-b]pyrazin-2(1H)-on

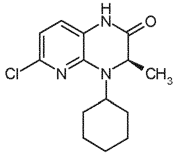

In Analogie zur Synthese von Intermediat 4 wurde (3R)-6-Chlor-4-cyclohexyl-3-methyl-3,4-dihydropyrido[2,3-b]pyrazin-2(1H)-on ausgehend von 1.3 g Intermediat 26 und 5.59 ml *N*,*N-*Diisopropylethylamin in 100 ml DMF durch 120 Stunden Erhitzen bei 170°C Badtemperatur hergestellt. Man erhielt 1.08 g (3*R*)-6-Chlor-4-cyclohexyl-3-methyl-3,4-dihydropyrido[2,3-b]pyrazin-2(1H)-on.
¹H-NMR (300 MHz, DMSO-d6): δ = 1.14 (d, 3H); 1.15-1.97 (5m, 10H); 4.03-4.13 (m, 1H); 4.15 (q, 1H); 6.65 (d, 1H); 7.00 (d, 1H); 10.58 (s, 1H).

### Intermediat 28:

### (3R)-6-Chlor-4-cyclohexyl-1,3-dimethyl-3,4-dihydropyrido[2,3-b]pyrazin-2(1H)-on

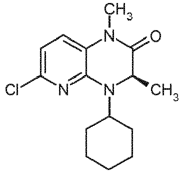

In Analogie zur Herstellung von Intermediat 5 wurde (3*R*)-6-Chlor-4-cyclohexyl-1,3-dimethyl-3,4-dihydropyrido[2,3-b]pyrazin-2(1H)-on ausgehend von 1.08 g Intermediat 27, 232 mg Natriumhydrid (60% in Weissöl) und 0.36 ml Methyljodid in 50 ml DMF hergestellt. Nach Reinigung durch Chromatographie an Kieselgel (Hexan / Ethylacetat 3:1) erhielt man 1.06 g (3*R*)-6-Chlor-4-cyclohexyl-1,3-dimethyl-3,4-dihydropyrido[2,3-b]pyrazin-2(1H)-on.
¹H-NMR (400 MHz, DMSO-d6): δ = 1.10 (d, 3H); 1.17 (tt, 1H); 1.24-1.43 (m, 2H); 1.45-1.85 (m, 6H); 1.94 (bd, 1H); 3.22 (s, 3H); 4.11 (tt, 1H); 4.31 (q, 1H); 6.76 (d, 1H); 7.31 (d, 1H).

### Intermediat 29:

### 3-{[(3R)-4-Cyclohexyl-1,3-dimethyl-2-oxo-1,2,3,4-tetrahydropyrido[2,3-b]pyrazin-6-yl]amino}benzoesäuremethylester

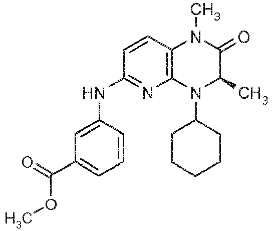

In Analogie zur Herstellung von Intermediat 6 wurde 3-{[(3*R*)-4-Cyclohexyl-1,3-dimethyl-2-oxo-1,2,3,4-tetrahydropyrido[2,3-b]pyrazin-6-yl]amino}benzoesäuremethylester ausgehend von 1.5 g Intermediat 28 und 1.57 g 3-Aminobenzoesäuremethylester hergestellt. Nach Chromatographie an Kieselgel (Hexan / Ethylacetat Gradient bis 50% Ethylacetat Anteil) erhielt man 2 g 3-{[(3*R*)-4-Cyclohexyl-1,3-dimethyl-2-oxo-1,2,3,4-tetrahydropyrido [2,3-b]pyrazin-6-yl]amino} benzoesäuremethylester.

UPLC-MS: Instrument: Waters Acquity UPLC-MS SQD; Säule: Acquity UPLC BEH C18 1.7 50x2.1mm; Eluent A: Wasser + 0.1% Vol. Ameisensäure (99%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm
Rt = 1.40 min.

### Intermediat 30:

### 3-{[(3R)-4-Cyclohexyl-1,3-dimethyl-2-oxo-1,2,3,4-tetrahydropyrido[2,3-b]pyrazin-6-yl]amino}benzoesäure

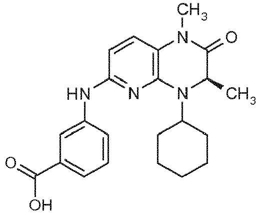

In Analogie zur Herstellung von Intermediat 7 wurde 3-{[(3*R*)-4-Cyclohexyl-1,3-dimethyl-2-oxo-1,2,3,4-tetrahydropyrido[2,3-b]pyrazin-6-yl]amino}benzoesäure ausgehend von 2.0 g Intermediat 29 und 0.98 g Natriumhydroxid hergestellt. Man erhielt 1.78 g 3-{[(3*R*)-4-Cyclohexyl-1,3-dimethyl-2-oxo-1,2,3,4-tetrahydropyrido[2,3-b]pyrazin-6-yl]amino}benzoesäure.

UPLC-MS: Instrument: Waters Acquity UPLC-MS SQD; Säule: Acquity UPLC BEH C18 1.7 50x2.1mm; Eluent A: Wasser + 0.1% Vol. Ameisensäure (99%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm
Rt = 1.18 min.

### Intermediat 31:

### N²-(1-Methylethyl)-N-(2,6-dichlorpyridin-3-yl)-D-alaninamid

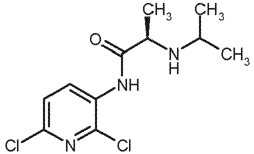

In Analogie zur Herstellung von Intermediat 3 wurde *N2*-(1-Methylethyl)-*N1*-(2,6-dichlorpyridin-3-yl)-D-alaninamid ausgehend von 0.5 g Intermediat 2, 0.27 ml Aceton, 303 mg Natriumacetat und 1.18 g Natriumtriacetoxyborhydrid in 40 ml Dichlormethan bei 0°C hergestellt. Man erhielt 420 mg *N²*-(1-Methylethyl)-*N*-(2,6-dichlorpyridin-3-yl)-D-alaninamid. Dieses wurde direkt in die Synthese der nächsten Stufe eingesetzt.
¹H-NMR (400 MHz, DMSO-d6): δ = 1.02 (d, 3H); 1.05 (d, 3H); 1.27 (d, 3H); 2.77 (sp, 1H); 3.30 (q, 1H); 7.58 (d, 1H); 8.67 (d, 1H).

### Intermediat 32:

### (3R)-6-Chlor-3-methyl-4-(propan-2-yl)-3,4-dihydropyrido[2,3-b]pyrazin-2(1H)-on

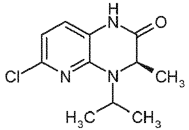

In Analogie zur Synthese von Intermediat 4 wurde (3*R*)-6-Chlor-3-methyl-4-(propan-2-yl)-3,4-dihydropyrido[2,3-b]pyrazin-2(1H)-on ausgehend von 420 mg Intermediat 31 und 2.1 ml *N*,*N-*Diisopropylethylamin in 40 ml DMF durch 72 Stunden Erhitzen bei 170°C Badtemperatur hergestellt. Man erhielt 320 mg (3*R*)-6-Chlor-3-methyl-4-(propan-2-yl)-3,4-dihydropyrido[2,3-b]pyrazin-2(1H)-on.
¹H-NMR (300 MHz, DMSO-d6): δ = 1.16 (d, 3H); 1.24 (d, 3H); 1.27 (d, 3H); 4.16 (q, 1H); 4.43 (sp, 1H); 6.65 (d, 1H); 7.00 (d, 1H); 10.56 (s, 1H).

### Intermediat 33:

### (3R)-6-Chlor-1,3-dimethyl-4-(propan-2-yl)-3,4-dihydropyrido[2,3-b]pyrazin-2(1H)-on

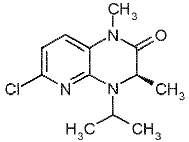

In Analogie zur Herstellung von Intermediat 5 wurde (3*R*)-6-Chlor-1,3-dimethyl-4-(propan-2-yl)-3,4-dihydropyrido[2,3-b]pyrazin-2(1H)-on ausgehend von 320 mg Intermediat 32, 80 mg Natriumhydrid (60% in Weissöl) und 0.13 ml Methyljodid in 20 ml DMF hergestellt. Nach Reinigung durch Chromatographie an Kieselgel (Hexan / Ethylacetat 2:1) erhielt man 280 mg (3*R*)-6-Chlor-1,3-dimethyl-4-(propan-2-yl)-3,4-dihydropyrido[2,3-b]pyrazin-2(1H)-on.
¹H-NMR (400 MHz, DMSO-d6): δ = 1.12 (d, 3H); 1.23 (d, 3H); 1.27 (d, 3H); 3.22 (s, 3H); 4.32 (q, 1H); 4.47 (sp, 1H); 6.76 (d, 1H); 7.31 (d, 1H).

### Intermediat 34:

### 3-{[(3R)-1,3-Dimethyl-2-oxo-4-(propan-2-yl)-1,2,3,4-tetrahydropyrido[2,3-b]pyrazin-6-yl]amino}benzoesäuremethylester

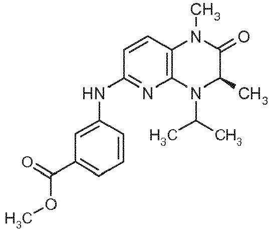

In Analogie zur Herstellung von Intermediat 6 wurde 3-{[(3*R*)-1,3-Dimethyl-2-oxo-4-(propan-2-yl)-1,2,3,4-tetrahydropyrido[2,3-b]pyrazin-6-yl]amino}benzoesäuremethylester ausgehend von 1.1 g Intermediat 33 und 1.24 g 3-Aminobenzoesäuremethylester hergestellt. Nach Chromatographie an Kieselgel (Hexan / Ethylacetat Gradient bis 50% Ethylacetat Anteil) erhielt man 1.1 g 3-{[(3*R*)-1,3-Dimethyl-2-oxo-4-(propan-2-yl)-1,2,3,4-tetrahydropyrido[2,3-b]pyrazin-6-yl]amino}benzoesäuremethylester ¹H-NMR (400 MHz, CDCl₃): δ = 1.25 (d, 3H); 1.30 (d, 3H); 1.36 (d, 3H); 3.31 (s, 3H); 3.92 (s, 3H); 4.32 (q, 1H); 4.77 (sept, 1H); 6.22 (d, 1H); 6.33 (bs, 1H); 7.02 (d, 1H); 7.35 (t, 1H); 7.50-7.56 (m, 1H); 7.64 (bd, 1H); 8.17 (bs, 1H).

### Intermediat 35:

### 3-{[(3R)-1,3-Dimethyl-2-oxo-4-(propan-2-yl)-1,2,3,4-tetrahydropyrido[2,3-b]pyrazin-6-yl]amino}benzoesäure

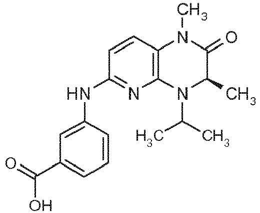

Eine Lösung von 1.1 g Intermediat 34 in 9 ml THF und 67 ml Methanol wurde bei RT mit 281 ml 1N Lithiumhydroxidlösung versetzt und bei 60°C für 4 Stunden gerührt. Es wurde mit 1N Salzsäure auf pH = 7 eingestellt und zweimal mit Ethylacetat extrahiert. Die vereinten organischen Phasen wurden über Natriumsulfat getrocknet und das Lösungsmittel vollständig im Vakuum entfernt. Man erhielt 1.78 g 3-{[(3*R*)-1,3-Dimethyl-2-oxo-4-(propan-2-yl)-1,2,3,4-tetrahydropyrido[2,3-b]pyrazin-6-yl]amino}benzoesäure.
¹H-NMR (400 MHz, DMSO-d6): δ = 1.09 (d, 3H); 1.23 (d, 3H); 1.30 (d, 3H); 3.20 (s, 3H); 4.24 (q, 1H)M; 4.75 (sept, 1H); 6.22 (d, 1H); 7.24 (d, 1H); 7.31 8t, 1H); 7.38 (bd, 1H); 7.64 (bd, 1H); 8.58 (t, 1H); 8.99 (s, 1H); 12.73 (bs, 1H).

### Intermediat 36:

### N²-Cycloheptyl-N-(2,6-dichlorpyridin-3-yl)-D-alaninamid

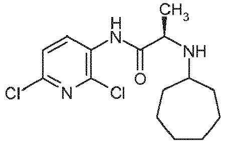

In Analogie zur Herstellung von Intermediat 3 wurde *N*²-Cycloheptyl-*N*-(2,6-dichlorpyridin-3-yl)-D-alaninamid ausgehend von 1.5 g Intermediat 2, 809 mg Cycloheptanon, 909 mg Natriumacetat und 3.5 g Natriumtriacetoxyborhydrid in 80 ml Dichlormethan bei 0°C hergestellt. Man erhielt 1.4 g *N²*-Cycloheptyl-*N*-(2,6-dichlorpyridin-3-yl)-D-alaninamid.
¹H-NMR (400 MHz, DMSO-d6): δ = 1.26 (d, 3H); 1.29-1.42 (m, 4H); 1.42-1.55 (m, 4H); 1.55-1.69 (m, 3H); 1.75-1.88 (m, 2H); 2.56-2.67 (m, 1H); 3.30 (m, 1H); 7.58 (d, 1H); 8.68 (d, 1H).

### Intermediat 37:

### (3R)-6-Chlor-4-cycloheptyl-3-methyl-3,4-dihydropyrido[2,3-b]pyrazin-2(1H)-on

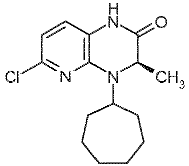

In Analogie zur Synthese von Intermediat 4 wurde (3*R*)-6-Chlor-4-cycloheptyl-3-methyl-3,4-dihydropyrido[2,3-b]pyrazin-2(1H)-on ausgehend von 1.4 g Intermediat 36 und 5.77 ml *N,N-*Diisopropylethylamin in 70 ml DMF durch 72 Stunden Erhitzen bei 170°C Badtemperatur hergestellt. Man erhielt 1.18 g (3*R*)-6-Chlor-4-cycloheptyl-3-methyl-3,4-dihydropyrido[2,3-b]pyrazin-2(1H)-on.
¹H-NMR (300 MHz, DMSO-d6): δ = 1.16 (d, 3H); 1.37-1.63 (m, 6H); 1.63-2.00 (m, 6H); 3.96-4.09 (m, 1H); 4.17 (q, 1H); 6.64 (d, 1H); 6.98 (d, 1H); 10.57 (s, 1H).

### Intermediat 38:

### (3R)-6-Chlor-4-cyclohepty-1-1,3-dimethyl-3,4-dihydropyrido[2,3-b]pyrazin-2(1H)-on

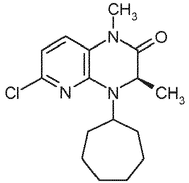

In Analogie zur Herstellung von Intermediat 5 wurde (3*R*)-6-Chlor-4-cycloheptyl-1,3-dimethyl-3,4-dihydropyrido[2,3-b]pyrazin-2(1H)-on ausgehend von 1.18 g Intermediate 37, 241 mg Natriumhydrid (60% in Weissöl) und 0.38 ml Methyljodid in 50 ml DMF hergestellt. Nach Reinigung durch Chromatographie an Kieselgel (Hexan / Ethylacetat 3:1) erhielt man 1.11 g (3*R*)-6-Chlor-4-cycloheptyl-1,3-dimethyl-3,4-dihydropyrido[2,3-b]pyrazin-2(1H)-on.
¹H-NMR (300 MHz, DMSO-d6): δ = 1.13 (d, 3H); 1.38-1.63 (m, 6H); 1.63-1.84 (m, 4H); 1.83-2.03 (m, 2H); 3.21 (s, 3H); 4.00-4.14 (m, 1H); 4.32 (q, 1H); 6.75 (d, 1H); 7.29 (d, 1H).

### Intermediat 39:

### N²-Benzyl-N-(2,6-dichlorpyridin-3-yl)-D-alaninamid

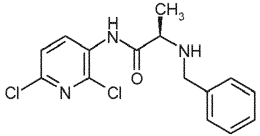

In Analogie zur Herstellung von Intermediat 3 wurde *N²*-Benzyl-*N*-(2,6-dichlorpyridin-3-yl)-D-alaninamid ausgehend von 1.5 g Intermediat 2, 765 mg Benzaldehyd, 909 mg Natriumacetat und 3.5 g Natriumtriacetoxyborhydrid in 80 ml Dichlormethan bei 0°C hergestellt. Man erhielt 1.5 g *N²*-Benzyl-*N*-(2,6-dichlorpyridin-3-yl)-D-alaninamid.
¹H-NMR (400 MHz, DMSO-d6): δ = 1.29 (d, 3H); 3.29 (q, 1H); 3.76 (s, 2H); 7.23 (t, 1H); 7.32 (t, 2H); 7.39 (d, 2H); 7.58 (d, 1H); 8.59 (d, 1H).

### Intermediat 40:

### (3R)-4-Benzyl-6-chlor-3-methyl-3,4-dihydropyrido[2,3-b]pyrazin-2(1H)-on

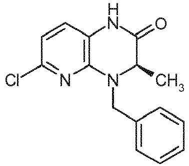

In Analogie zur Synthese von Intermediat 4 wurde (3*R*)-4-Benzyl-6-chlor-3-methyl-3,4-dihydropyrido[2,3-b]pyrazin-2(1H)-on ausgehend von 1.4 g Intermediat 39 und 5.88 ml *N*,*N-*Diisopropylethylamin in 100 ml DMF durch 72 Stunden Erhitzen bei 170°C Badtemperatur hergestellt. Man erhielt 1.14 g (3*R*)-4-Benzyl-6-chlor-3-methyl-3,4-dihydropyrido[2,3-b]pyrazin-2(1H)-on.
¹H-NMR (300 MHz, DMSO-d6): δ = 1.18 (d, 3H); 3.95 (q, 1H); 4.29 (d, 1H); 5.10 (d, 1H); 6.71 (d, 1H); 7.04 (d, 1H); 7.23-7.33 (m, 1H); 7.33-7.41 (m, 4H); 10.70 (s, 1H).

### Intermediat 41:

### (3R)-4-Benzyl-6-chlor-1,3-dimethyl-3,4-dihydropyrido[2,3-b]pyrazin-2(1H)-on

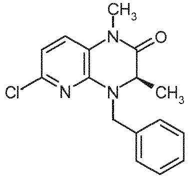

In Analogie zur Herstellung von Intermediat 5 wurde (3*R*)-4-Benzyl-6-chlor-1,3-dimethyl-3,4-dihydropyrido[2,3-b]pyrazin-2(1H)-on ausgehend von 1.14 g Intermediat 40, 238 mg Natriumhydrid (60% in Weissöl) und 0.37 ml Methyljodid in 50 ml DMF hergestellt. Nach Reinigung durch Chromatographie an Kieselgel (Hexan / Ethylacetat 3:1) erhielt man 1.15 g (3R)-4-Benzyl-6-chlor-1,3-dimethyl-3,4-dihydropyrido[2,3-b]pyrazin-2(1H)-on.
1H-NMR (300 MHz, DMSO-d6): δ = 1.15 (d, 3H); 3.24 (s, 3H); 4.08 (q, 1H); 4.28 (d, 1H); 5.11 (d, 1H); 6.82 (d, 1H); 7.22-7.42 (m, 6H).

### Intermediat 42:

### 3-{[(3R)-4-Benzyl-1,3-dimethyl-2-oxo-1,2,3,4-tetrahydropyrido[2,3-b]pyrazin-6-yl]amino}benzoesäuremethylester

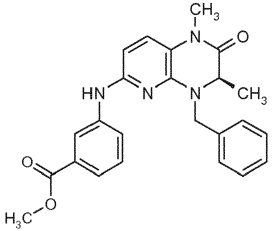

Eine Suspension von 900 mg Intermediat 41, 857 mg 3-Aminobenzoesäuremethylester (CAS 4518-10-9), 64 mg Palladium(II)acetat, 2.77 g Caesiumcarbonat und 176 mg (+)-BINAP in 63.4 ml Toluol wurde 14 Stunden unter einer Argonatmosphäre bei 120°C gerührt. Die Reaktionslösung wurde auf Wasser gegeben, zweimal mit Ethylacetat extrahiert, die vereinten organischen Phasen über Natriumsulfat getrocknet und das Lösungsmittel im Vakuum entfernt. Der Rückstand wurde durch Chromatographie an Kieselgel (Dichlormethan / Methanol Gradient bis 1% Methanol Anteil) gereinigt. Man erhielt 920 mg 3-{[(3*R*)-4-Benzyl-1,3-dimethyl-2-oxo-1,2,3,4-tetrahydropyrido[2,3-b]pyrazin-6-yl]amino}benzoesäuremethylester.
¹H-NMR: (300 MHz, 25°C, DMSO-d6): δ = 1.12 (d, 3H); 3.23 (s, 3H); 3.67 (s, 3H); 3.95 (q, 1H); 4.28 (d, 1H); 5.33 (d, 1H); 6.27 (d, 1H); 7.22-7.42 (m, 8H); 7.63 (bd, 1H); 8.50 (t, 1H); 9.09 (s, 1H).

### Intermediat 43:

### 3-{[(3R)-4-Benzyl-1,3-dimethyl-2-oxo-1,2,3,4-tetrahydropyrido[2,3-b]pyrazin-6-yl]amino}benzoesäure

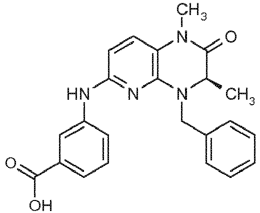

Eine Lösung von 900 mg Intermediat 42 in 6.8 ml THF und 51 ml Methanol wurde bei RT mit 22 ml 1N Lithiumhydroxidlösung versetzt und bei 60°C für 2 Stunden gerührt. Es wurde mit 1N Salzsäure auf pH = 7 eingestellt und zweimal mit Ethylacetat extrahiert. Die vereinten organischen Phasen wurden über Natriumsulfat getrocknet und das Lösungsmittel vollständig im Vakuum entfernt. Man erhielt 770 mg 3-{[(3*R*)-4-Benzyl-1,3-dimethyl-2-oxo-1,2,3,4-tetrahydropyrido[2,3-b]pyrazin-6-yl]amino}benzoesäure.
¹H-NMR (400 MHz, DMSO-d6): δ = 1.12 (d, 3H); 3.23 (s, 3H); 3.98 (q, 1H); 4.27 (d, 1H); 5.31 (d, 1H); 6.27 (d, 1H); 7.18-7.40 (m, 8H); 7.67 (bd, 1H); 8.34 (t, 1H); 9.01 (s, 1H); 12.50 (bs, 1H).

### Intermediat 44:

### N-(2,6-Dichlorpyridin-3-yl)-2-oxopropanamid

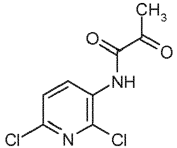

Eine Lösung von 17.6 g Brenztraubensäure in 150 ml DMF wurde langsam bei 0°C mit 14.6 ml Thionylchlorid versetzt. Es wurde 15 Minuten gerührt und dann 16.3 g 2,6-Dichlorpyridin-3-amin (CAS 62476-56-6) zugegeben. Man ließ 16 Stunden bei RT rühren und goss auf 300 ml Eiswasser. Der Niederschlag wurde abfiltriert und mit Wasser gewaschen. Man erhielt 9.8 g *N*-(2,6-Dichlorpyridin-3-yl)-2-oxopropanamid.
¹H-NMR (300 MHz, DMSO-d6): δ = 2.44 (s, 3H); 7.65 (d, 1H); 8.28 (d, 1H); 10.03 (bs, 1H).

### Intermediat 45:

### N-(2,6-Dichlorpyridin-3-yl)-N²-(2-methoxyethyl)alaninamid

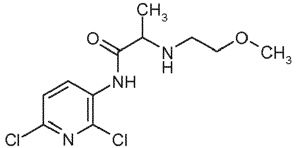

Eine Lösung von 1.7 g Intermediat 44 und 603 mg 2-Methoxyethylamin in 52 ml 1,2-Dichlorethan und 0.42 ml Essigsäure wurde bei RT mit 2.16 g Natriumtriacetoxyborhydrid versetzt. Es wurde 16 Stunden gerührt. Der Ansatz wurde in Wasser eingerührt und mit Dichlormethan extrahiert. Die organische Phase wurde mit Natriumhydrogencarbonat-Lösung und Wasser gewaschen, über Natriumsulfat getrocknet und das Lösungsmittel im Vakuum entfernt. Man erhielt 2.13 g *N*-(2,6-Dichlorpyridin-3-yl)-*N*²-(2-methoxyethyl)alaninamid.

UPLC-MS: Instrument: Waters Acquity UPLC-MS SQD; Säule: Acquity UPLC BEH C18 1.7 50x2.1mm; Eluent A: Wasser + 0.1% Vol. Ameisensäure (99%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm.
Rt = 0.62 min (M⁺+1 = 292/294/296)

### Intermediat 46:

### 6-Chlor-4-(2-methoxyethyl)-3-methyl-3,4-dihydropyrido[2,3-b]pyrazin-2(1H)-on

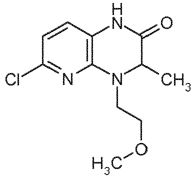

In Analogie zur Synthese von Intermediat 4 wurde 6-Chlor-4-(2-methoxyethyl)-3-methyl-3,4-dihydropyrido[2,3-b]pyrazin-2(1H)-on ausgehend von 2.9 g Intermediat 45 und 13.8 ml *N,N-*Diisopropylethylamin in 5 ml DMF durch 72 Stunden Erhitzen bei 170°C Badtemperatur hergestellt. Man erhielt 1.0 g 6-Chlor-4-(2-methoxyethyl)-3-methyl-3,4-dihydropyrido[2,3-b]pyrazin-2(1H)-on.
¹H-NMR (300 MHz, DMSO-d6): δ = 1.21 (d, 3H); 3.19-3.31 (m+s, 4H); 3.45-3.59 (m, 2H); 3.99 (dt, 1H); 4.14 (q, 1H); 6.65 (d, 1H); 6.97 (d, 1H); 10.62 (bs, 1H).

### Intermediat 47:

### 6-Chlor-4-(2-methoxyethyl)-1,3-dimethyl-3,4-dihydropyrido[2,3-b]pyrazin-2(1H)-on

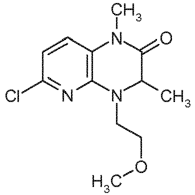

In Analogie zur Herstellung von Intermediat 5 wurde 6-Chlor-4-(2-methoxyethyl)-1,3-dimethyl-3,4-dihydropyrido[2,3-b]pyrazin-2(1H)-on ausgehend von 1.0 g Intermediat 46, 256 mg Natriumhydrid (60% in Weissöl) und 0.37 ml Methyljodid in 9 ml DMF hergestellt. Nach Reinigung durch Chromatographie an Kieselgel (Hexan / Ethylacetat Gradient) erhielt man 730 mg 6-Chlor-4-(2-methoxyethyl)-1,3-dimethyl-3,4-dihydropyrido[2,3-b]pyrazin-2(1H)-on.
¹H-NMR (300 MHz, DMSO-d6): δ = 1.17 (d, 3H); 3.19-3.31 (m+2s, 7H); 3.45-3.60 (m, 2H); 4.02 (dt, 1H); 4.28 (q, 1H); 6.77 (d, 1H); 7.29 (d, 1H).

### Intermediat 48:

### 3-{[4-(2-Methoxyethyl)-1,3-dimethyl-2-oxo-1,2,3,4-tetrahydropyrido[2,3-b]pyrazin-6-yl]amino}benzoesäuremethylester

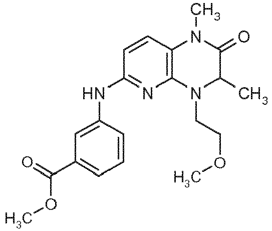

Eine Suspension von 1.6 g Intermediat 47, 1.7 g 3-Aminobenzoesäuremethylester (CAS 4518-10-9), 127 mg Palladium(II)acetat, 5.5 g Caesiumcarbonat und 351 mg (+)-BINAP in 126 ml Toluol wurde 14 Stunden unter einer Argonatmosphäre bei 120°C gerührt. Die Reaktionslösung wurde auf Wasser gegeben, zweimal mit Ethylacetat extrahiert, die vereinten organischen Phasen über Natriumsulfat getrocknet und das Lösungsmittel im Vakuum entfernt. Der Rückstand wurde durch Chromatographie an Kieselgel (Hexan / Ethylacetat Gradient bis 100% Ethylacetat Anteil) gereinigt. Man erhielt 1.5 g 3-{[4-(2-Methoxyethyl)-1,3-dimethyl-2-oxo-1,2,3,4-tetrahydropyrido[2,3-b]pyrazin-6-yl]amino}benzoesäuremethylester.
¹H-NMR: (300 MHz, 25°C, DMSO-d6): δ = 1.13 (d, 3H); 3.21 (s, 3H); 3.24 (s, 3H); 3.59 (t, 2H); 3.84 (s, 3H); 4.11-4.24 (m, 2H); 6.22 (d, 1H); 7.23 (d, 1H); 7.34 8t, 1H); 7.40 (d, 1H); 7.71 (d, 1H); 8.46 (t, 1H); 9.05 (s, 1H).

### Intermediat 49:

### 3-{[4-(2-Methoxyethyl)-1,3-dimethyl-2-oxo-1,2,3,4-tetrahydropyrido[2,3-b]pyrazin-6-yl]amino}benzoesäure

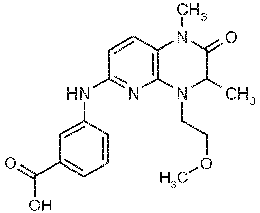

Eine Lösung von 1.5 g Intermediat 48 in 12 ml THF und 92 ml Methanol wurde bei RT mit 39 ml 1N Lithiumhydroxidlösung versetzt und bei 60°C für 2 Stunden gerührt. Es wurde mit 1N Salzsäure auf pH = 7 eingestellt und zweimal mit Ethylacetat extrahiert. Die vereinten organischen Phasen wurden über Natriumsulfat getrocknet und das Lösungsmittel vollständig im Vakuum entfernt. Man erhielt 1.3 mg 3-{[4-(2-Methoxyethyl)-1,3-dimethyl-2-oxo-1,2,3,4-tetrahydropyrido[2,3-b]pyrazin-6-yl]amino}benzoesäure.
¹H-NMR (300 MHz, DMSO-d6): δ = 1.13 (d, 3H); 3.21 (s, 3H); 3.24 (s, 3H); 3.24-3.31 (m, 1H); 3.54-3.64 (m, 2H); 4.11-4.22 (m, 2H); 6.21 (d, 1H); 7.23 (d, 1H); 7.31 (t, 1H); 7.39 (d, 1H); 7.71 (d, 1H); 8.38 (t, 1H); 8.99 (s, 1H); 12.61 (bs, 1H).

### Intermediat 50:

### 4-({(2R)-1-[(2,6-Dichlorpyridin-3-yl)amino]-1-oxopropan-2-yl}amino)piperidin-1-kohlensäure-tert-butylester

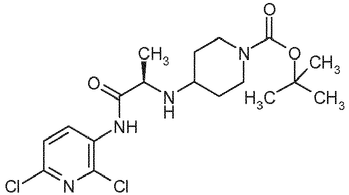

In Analogie zur Herstellung von Intermediat 3 wurde 4-({(2*R*)-1-[(2,6-Dichlorpyridin-3-yl)amino]-1-oxopropan-2-yl}amino)piperidin-1-kohlensäure-*tert*-butylester ausgehend von 2 g Intermediat 2, 2.02 g 1-Boc-4-piperidin-1-on (CAS 79099-07-3), 1.21 g Natriumacetat und 4.7 g Natriumtriacetoxyborhydrid in 60 ml Dichlormethan bei 0°C hergestellt. Man erhielt 4.1 g 4-({(2*R*)-1-[(2,6-Dichlorpyridin-3-yl)amino]-1-oxopropan-2-yl}amino)piperidin-1-kohlensäure-*tert-*butylester als Rohprodukt, welches ohne weitere Reinigung in der nächsten Stufe eingesetzt wurde.
¹H-NMR (400 MHz, DMSO-d6): δ = 1.10.1.25 (m, 2H); 1.27 (d, 3H); 1.38 (s, 9H); 1.74 (bd, 1H); 1.89 (bd, 1H); 2.67-2.83 (bs, 2H); 3.39 (q, 1H); 3.80-3.90 (m, 2H); 7.58 (d, 1H); 8.66 (d, 1H).

### Intermediat 51:

### 4-[(3R)-6-Chlor-3-methyl-2-oxo-2,3-dihydropyrido[2,3-b]pyrazin-4(1H)-yl]piperidin-1-kohlensäure-tert-butylester

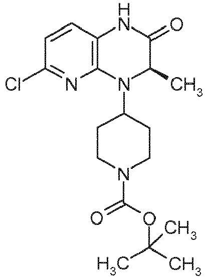

In Analogie zur Synthese von Intermediat 4 wurde 4-[(3*R*)-6-Chlor-3-methyl-2-oxo-2,3-dihydropyrido[2,3-b]pyrazin-4(1H)-yl]piperidin-1-kohlensäure-*tert*-butylester ausgehend von 1.02 g Intermediat 50 und 3.4 ml *N,N*-Diisopropylethylamin in 5 ml DMF durch 18 Stunden Erhitzen bei 170°C Badtemperatur hergestellt. Man erhielt 577 mg 4-[(3*R*)-6-Chlor-3-methyl-2-oxo-2,3-dihydropyrido[2,3-b]pyrazin-4(1H)-yl]piperidin-1-kohlensäure-*tert*-butylester.
¹H-NMR (300 MHz, DMSO-d6): δ = 1.14 (d, 3H); 1.41 (s, 9H); 1.53-1.62 (m, 1H); 1.65-1.77 (m, 1H); 1.82-1.93 (m, 2H); 2.68-2.90 (bs, 2H); 3.98-4.10 (m, 2H); 4.10-4.20 (m, 2H); 6.69 (d, 1H); 7.02 (d, 1H); 10.58 (s, 1H).

### Intermediat 52:

### 4-[(3R)-6-Chlor-1,3-dimethyl-2-oxo-2,3-dihydropyrido[2,3-b]pyrazin-4(1H)-yl]piperidin-1-kohlensäure-tert-butylester

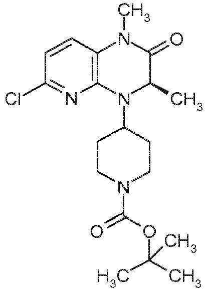

In Analogie zur Herstellung von Intermediat 5 wurde 4-[(3*R*)-6-Chlor-1,3-dimethyl-2-oxo-2,3-dihydropyrido[2,3-b]pyrazin-4(1H)-yl]piperidin-1-kohlensäure-*tert*-butylester ausgehend von 573 mg Intermediat 51, 98 mg Natriumhydrid (60% in Weissöl) und 0.14 ml Methyljodid in 6.6 ml DMF hergestellt. Nach Reinigung durch Chromatographie an Kieselgel (Hexan / Ethylacetat Gradient) erhielt man 460 mg 4-[(3*R*)-6-Chlor-1,3-dimethyl-2-oxo-2,3-dihydropyrido[2,3-b]pyrazin-4(1H)-yl]piperidin-1-kohlensäre-*tert*-butylester.
¹H-NMR (300 MHz, DMSO-d6): δ = 1.11 (d, 3H); 1.41 (s, 9H); 1.55-1.63 (m, 1H); 1.70 (qd, 1H); 1.81-1.93 (m, 2H); 2.71-2.91 (bs, 2H); 3.22 (s, 3H); 3.99-4.11 (m, 2H); 4.19 (tt, 1H); 4.30 (q, 1H); 6.80 (d, 1H); 7.33 (d, 1H).

### Intermediat 53

### tert-Butyl 4-[(3R)-6-{[3-(ethoxycarbonyl)phenyl]amino}-1,3-dimethyl-2-oxo-2,3-dihydropyrido[2,3-b]pyrazin-4(1H)-yl]piperidin-1-carboxylat

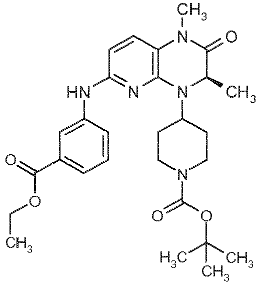

Eine Suspension von 209 mg Intermediat 52, 255 g 3-Aminobenzoesäureethylester (CAS 582-33-2), 33 mg Palladium(II)acetat, 1.2 g Caesiumcarbonat und 91 mg (+)-BINAP in 6.5 ml Toluol wurde 2 Stunden unter einer Argonatmosphäre bei 120°C und 14 Stunden bei RT gerührt. Die Reaktionslösung wurde auf Wasser gegeben, zweimal mit Ethylacetat extrahiert, die vereinten organischen Phasen über Natriumsulfat getrocknet und das Lösungsmittel im Vakuum entfernt. Der Rückstand wurde durch Chromatographie an Kieselgel (Hexan / Ethylacetat Gradient bis 60% Ethylacetat Anteil) gereinigt. Man erhielt 338 mg *tert*-Butyl 4-[(3*R*)-6-{[3-(ethoxycarbonyl)phenyl]amino}-1,3-dimethyl-2-oxo-2,3-dihydropyrido[2,3-b]pyrazin-4(1H)-yl]piperidin-1-carboxylat.
¹H-NMR: (300 MHz, 25°C, DMSO-d6): δ = 1,07 (d, 3H); 1.31 (t, 3H); 1.41 (s, 9H); 1.56 (qd, 1H); 1.62 (bd, 1H); 1.74 (qd, 1H); 2.00 (bd, 1H); 2.68-2.93 (m, 2H); 3.20 (s, 3H); 3.99-4.10 (m, 2H); 4.21 (q, 1H); 4.29 (q, 1H); 4.36 (tt, 1H); 6.25 /d, 1H); 7.28 (d, 1H); 7.36 (t, 1H); 7.41 (d, 1H); 7.85 (d, 1H); 8.18 (t, 1H); 9.04 (s, 1H).

### Intermediat 54:

### 3-({(3R)-4-[1-(tert-Butoxycarbonyl)piperidin-4-yl]-1,3-dimethyl-2-oxo-1,2,3,4-tetrahydropyrido[2,3-b]pyrazin-6-yl}amino)benzoesäure

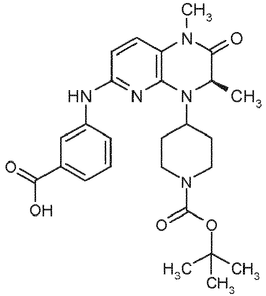

Eine Lösung von 334 mg Intermediat 53 in 5 ml Methanol wurde bei RT mit 128 mg Natriumhydroxyd versetzt und bei 50°C für 2 Stunden gerührt. Es wurde mit 1N Salzsäure auf pH = 7 eingestellt und zweimal mit Ethylacetat extrahiert. Die vereinten organischen Phasen wurden über Natriumsulfat getrocknet und das Lösungsmittel vollständig im Vakuum entfernt. Man erhielt 270 mg 3-({(3*R*)-4-[1-(*tert*-Butoxycarbonyl)piperidin-4-yl]-1,3-dimethyl-2-oxo-1,2,3,4-tetrahydropyrido[2,3-b]pyrazin-6-yl}amino)benzoesäure.
¹H-NMR (400 MHz, DMSO-d6): δ = 1.06 (d, 3H); 1.41 (s, 9H); 1.48-1.78 (m, 3H); 1.97 (bd, 1H); 2.73-3.00 (m, 2H); 3.20 (s, 3H); 4.05 (bs, 2H); 4.20 (q, 1H); 4.46 (tt, 1H); 6.26 (d, 1H); 7.27 (d, 1H); 7.33 (t, 1H); 7.41 (d, 1H); 7.68 (d, 1H); 8.35 (bs, 1H); 9.00 (s, 1H); 12.83 (bs, 1H).

### Intermediat 55:

### N-(2,6-Dichloropyridin-3-yl)-N²-(4,4-dimethylcyclohexyl)-D-alaninamid

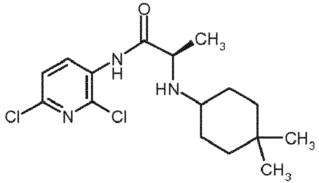

In Analogie zur Herstellung von Intermediat 3 wurde *N*-(2,6-Dichloropyridin-3-yl)-*N*²-(4,4-dimethylcyclohexyl)-D-alaninamid ausgehend von 2.85 g Intermediat 2, 1.76 g 4,4-Dimethylcyclohexanon (CAS 4255-62-3), 1.73 g Natriumacetat und 6.7 g Natriumtriacetoxyborhydrid in 100 ml Dichlormethan bei 0°C hergestellt. Man erhielt 4.0 g *N*-(*2*,*6-*Dichloropyridin-3-yl)-N²-(4,4-dimethylcyclohexyl)-D-alaninamid.
¹H-NMR (400 MHz, DMSO-d6): δ = 0.82-0.89 (m, 8H); 1.09-1.18 (m, 3H); 1.20-1.39 (m, 9H); 1.52-1.63 (m, 2H); 1.69-1.78 (m, 1H); 2.30-2.41 (m, 1H); 3.33 (q, 1H); 7.57 (d, 1H); 8.68 (d, 1H).

### Intermediat 56:

### (3R)-6-Chloro-4-(4,4-dimethylcyclohexyl)-3-methyl-3,4-dihydropyrido[2,3-b]pyrazin-2(1H)-on

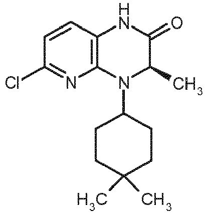

In Analogie zur Synthese von Intermediat 4 wurde (3*R*)-6-Chloro-4-(4,4-dimethylcyclohexyl)-3-methyl-3,4-dihydropyrido[2,3-b]pyrazin-2(1H)-on ausgehend von 3.96 g Intermediat 55 und 16 ml *N,N-*Diisopropylethylamin in 20 ml DMF durch 16 Stunden Erhitzen bei 170°C Badtemperatur hergestellt. Man erhielt 2.49 mg wurde (3*R*)-6-Chloro-4-(4,4-dimethylcyclohexyl)-3-methyl-3,4-dihydropyrido[2,3-b]pyrazin-2(1H)-on.
¹H-NMR (400 MHz, DMSO-d6): δ = 0.86 (d, 1H); 0.91 (s, 3H); 0.98 (s, 3H); 1.16 (d, 3H); 1.24 - 1.35 (m, 3H); 1.36 - 1.47 (m, 3H); 1.83 (dd, 1H); 1.97 - 2.11 (m, 1H); 3.81 - 3.93 (m, 1H); 6.63 (d, 1H); 6.98 (d, 1H); 10.54 (s, 1H).

### Intermediat 57:

### (3R)-6-Chlor-4-(4,4-dimethylcyclohexyl)-1,3-dimethyl-3,4-dihydropyrido[2,3-b]pyrazin-2(1H)-on

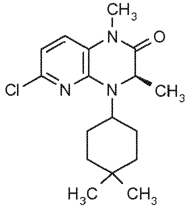

In Analogie zur Herstellung von Intermediat 5 wurde (3R)-6-Chlor-4-(4,4-dimethylcyclohexyl)-1,3-dimethyl-3,4-dihydropyrido[2,3-b]pyrazin-2(1H)-on ausgehend von 2.49 g Intermediat 56, 529 mg Natriumhydrid (60% in Weissöl) und 0.76 ml Methyljodid in 36 ml DMF hergestellt. Nach Reinigung durch Chromatographie an Kieselgel (Hexan / Ethylacetat Gradient bis 30% Ethylacetat Anteil) erhielt man 1.3 g (3*R*)-6-Chlor-4-(4,4-dimethylcyclohexyl)-1,3-dimethyl-3,4-dihydropyrido[2,3-b]pyrazin-2(1H)-on.
¹H-NMR (300 MHz, DMSO-d6): δ = 0.87 - 0.94 (m, 3H); 0.98 (s, 3H); 1.12 (d, 3H); 1.20 - 1.50 (m, 6H); 1.64 - 1.73 (m, 1H); 1.79 (td, 1H); 3.21 (s, 3H); 3.85 - 3.97 (m, 1H); 4.34 (q, 1H); 6.74 (d, 1H); 7.29 (d, 1H).

### Intermediat 58:

### N-(2,6-Dichlorpyridin-3-yl)-N²-(2-methylpropyl)-D-alaninamid

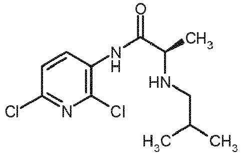

In Analogie zur Herstellung von Intermediat 3 wurde *N*-(2,6-Dichlorpyridin-3-yl)-*N²*-(2-methylpropyl)-D-alaninamid ausgehend von 2.92 g Intermediat 2, 1.24 g Isobutyraldehyd, 0.67 ml Essigsäure und 7.3 g Natriumtriacetoxyborhydrid in 34 ml Dichlormethan bei 0°C hergestellt. Man erhielt 1.22 g *N*-(2,6-Dichlorpyridin-3-yl)-*N*²-(2-methylpropyl)-D-alaninamid.
¹H-NMR (400 MHz, DMSO-d6): δ = 0.83-0.98 (m, 6H); 1.27 (d, 3H); 1.63-1.80 (m, 1H); 2.26 (dd, 1H); 2.46 (dd, 1H); 3.23 (q, 1H); 7.59 (d, 1H); 8.66 (d, 1H).

### Intermediat 59:

### (3R)-6-Chlor-3-methyl-4-(2-methylpropyl)-3,4-dihydropyrido[2,3-b]pyrazin-2(1H)-on

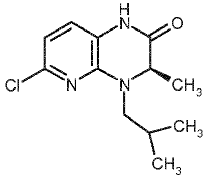

In Analogie zur Synthese von Intermediat 4 wurde (3*R*)-6-Chlor-3-methyl-4-(2-methylpropyl)-3,4-dihydropyrido[2,3-b]pyrazin-2(1H)-on ausgehend von 800 mg Intermediat 58 und 3.8 ml *N,N-*Diisopropylethylamin in 10 ml DMA durch 14 Stunden Erhitzen bei 165°C Badtemperatur hergestellt. Man erhielt 1.05 g (3*R*)-6-Chlor-3-methyl-4-(2-methylpropyl)-3,4-dihydropyrido[2,3-b]pyrazin-2(1H)-on als Rohprodukt, welches ohne weitere Reinigung in die nächste Stufe eingesetzt wurde.

UPLC-MS: Instrument: Waters Acquity UPLC-MS SQD; Säule: Acquity UPLC BEH C18 1.7 50x2.1mm; Eluent A: Wasser + 0.1% Vol. Ameisensäure (99%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm
Rt = 1.19 min.

### Intermediat 60:

### (3R)-6-Chlor-1,3-dimethyl-4-(2-methylpropyl)-3,4-dihydropyrido[2,3-b]pyrazin-2(1H)-on

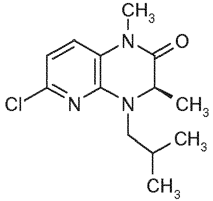

In Analogie zur Herstellung von Intermediat 5 wurde (3*R*)-6-Chlor-1,3-dimethyl-4-(2-methylpropyl)-3,4-dihydropyrido[2,3-b]pyrazin-2(1H)-on ausgehend von 1.05 g Intermediat 59 (Rohprodukt), 181 mg Natriumhydrid (60% in Weissöl) und 0.26 ml Methyljodid in 10 ml DMF hergestellt. Nach Reinigung durch Chromatographie an Kieselgel (Dichlormethan / Methanol Gradient bis 5% Methanol Anteil) erhielt man 390 mg wurde (3*R*)-6-Chlor-1,3-dimethyl-4-(2-methylpropyl)-3,4-dihydropyrido[2,3-b]pyrazin-2(1H)-on.
¹H-NMR (300 MHz, DMSO-d6): δ = 0.82 (d, 3H); 0.88 (d, 3H); 1.12 (d, 3H); 1.89 - 2.05 (m, 1H); 2.66 - 2.77 (m, 1H); 3.23 (s, 3H); 3.79 (dd, 1H); 4.15 (q, 1H); 6.74 (d, 1H); 7.28 (d, 1H).

### Intermediat 61:

### N²-(1-Benzylpiperidin-4-yl)-N-(2,6-dichlorpyridin-3-yl)-D-alaninamid

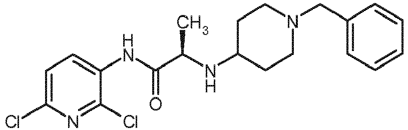

Eine Lösung von 10 g Intermediat 2 und 8.89 g 1-Benzylpiperidon (CAS 3612-20-2) in 100 ml Dichlormethan wurde bei RT mit 18.2 g Natriumtriacetoxyborhydrid versetzt. Nach 16 Stunden wurde der Ansatz vorsichtig auf gesättigte Natriumhydrogencarbonatlösung gegossen, die Phasen wurden getrennt und die wäßrige Phase mit Dichlormethan extrahiert. Die vereinten organischen Phasen wurden über Natriumsulfat getrocknet und im Vakuum eingeengt. Der Rückstand wurde durch Chromatographie an Kieselgel (Heptan / Ethylacetat Gradient) gereinigt. Man erhielt 15.1 g *N*²-(1-Benzylpiperidin-4-yl)-*N*-(2,6-dichlorpyridin-3-yl)-D-alaninamid.
¹H NMR (400 MHz, 25°C, CDCl₃): δ = 1.17 (bs, 1H), 1.37-1.52 (m, 5H), 1.86 (d, 1H), 1.91-2.04 (m, 3H), 2.48 (bs, 1H), 2.83-2.88 (m, 2H), 3.38 (q, 1H), 3.51 (s, 2H), 7.22-7.33 (m, 6H), 8.82 (d, 1H), 10.4 (bs, 1H).

### Intermediat 62

### (3R)-4-(1-Benzylpiperidin-4-yl)-6-chlor-3-methyl-3,4-dihydropyrido[2,3-b]pyrazin-2(1H)-on

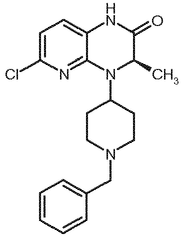

Eine Lösung von 15.1 g Intermediat 61 und 32.3 ml *N,N*-Diisopropylethylamin in 277 ml DMA wurde 48 Stunden bei 170°C Badtemperatur in einem dicht verschlossenen Gefäß gerührt. Nach dem Erkalten wurde der Ansatz mit Wasser verdünnt und dreimal mit Ethylacetat extrahiert. Die vereinten organischen Phasen wurden im Vakuum eingeengt. Es wurde mit Toluol versetzt und nochmals im Vakuum vollständig eingeengt. Der Rückstand wurde in einer Heptan / Wasser Mischung gerührt, der Niederschlag abgesaugt und anschließend mit Toluol destillativ getrocknet. Man erhielt 13.8 g (3*R*)-4-(1-Benzylpiperidin-4-yl)-6-chlor-3-methyl-3,4-dihydropyrido[2,3-b]pyrazin-2(1H)-on.

¹H NMR (400 MHz, 25°C, CDCl₃): δ = 1.27 (d, 3H), 1.54-1.81 (m, 3H), 1.86-2.26 (m, 3H), 2.90-3.05 (m, 2H), 3.54 (s, 2H), 4.22-4.39 (m, 2H), 6.60 (d, 1H), 6.87 (d, 1H), 7.25-7.32 (m, 5H), 8.72 (bs, 1H).

### Intermediat 63

### (R)-4-(1-Benzylpiperidin-4-yl)-6-chlor-1,3-dimethyl-3,4-dihydropyrido[2,3-b]pyrazin-2(1H)-on

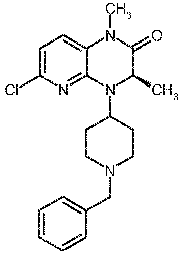

Eine Lösung von 13.1 g Intemediat 62 in 131 ml DMF wurde bei 0°C portionsweise mit 2.08 mg Natriumhydrid (60% in Weissöl) versetzt. Es wurde noch 30 min. bei RT gerührt, anschließend wieder auf 0°C gekühlt und 2.28 ml Methyljodid zugegeben. Nach ca. 10 min. wurde schnell unter einer Argonatmosphäre auf Eiswasser gegeben und der Niederschlag wurde abgesaugt und mit Heptan nachgewaschen. Man erhielt 12.7 g (*R*)-4-(1-Benzylpiperidin-4-yl)-6-chlor-1,3-dimethyl-3,4-dihydropyrido[2,3-b]pyrazin-2(1H)-on.
¹H NMR (400 MHz, 25°C, CDCl₃): δ = 1.19 (d, 3H), 1.57-1.79 (m, 2H +H₂O), 1.92 (bq, 1H), 2.04-2.22 (m, 3H), 2.96 (bs, 2H), 3.28 (s, 3H), 3.54 (s, 2H), 4.30-4.35 (m, 2H), 6.65 (d, 1H), 6.96 (d, 1H), 7.31-7.37 (m, 5H).

### Intermediat 64:

### (3R)-6-Chlor-1,3-dimethyl-4-(piperidin-4-yl)-3,4-dihydropyrido[2,3-b]pyrazin-2(1H)-on Hydrochlorid

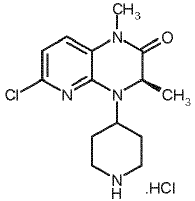

Eine Lösung von 12.2 g Intermediat 63 und 4.46 ml 1-Chlorethylcarbonochloridat (CAS 50893-53-3) in 131 ml 1,2-Dichlorethan wurde 4 Stunden unter Rückfluss erhitzt. Der Ansatz wurde vollständig eingedampft und in Ethylacetat / Heptan (1:1) aufgenommen. Diese Lösung wurde durch Kieselgel filtriert und zunächst mit Heptan, dann mit Ethylacetat nachgewaschen. Der eluierte Rückstand wurde in Methanol erhitzt und anschließend wieder eingedampft. Man erhielt 8.2 g (3*R*)-6-Chlor-1,3-dimethyl-4-(piperidin-4-yl)-3,4-dihydropyrido[2,3-b]pyrazin-2(1H)-on Hydrochlorid.
¹H NMR (400 MHz, 25°C, DMSO-d6₃): δ = 1.22 (d, 3H), 1.94-2.01 (m, 1H), 2.13 (dq, 1H), 2.23-2.37 (m, 2H), 3.16 (tt, 2H), 3.30 (s, 3H), 3.43-3.53 (m, 2H), 4.28 (q, 1H), 4.39 (tt, 1H), 6.80 (d, 1H), 7.07-7.21 (m, 1H), 7.32 (a, 1H).

### Intermediat 65

### (3R)-6-Chlor-1,3-dimethyl-4-(1-methylpiperidin-4-yl)-3,4-dihydropyrido[2,3-b]pyrazin-2(1H)-on

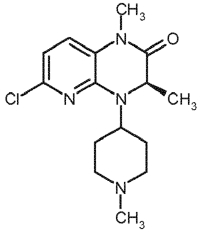

Eine Lösung von 8.2 g Intermediat 64 in 77.1 ml Methanol wurde zunächstbei RT mit 77.1 ml Formaldehyd-Lösung (37% in Wasser) und anschließend mit 2.19 g Natriumcyanoborhydrid und 3.49 g Essigsäure versetzt. Er wurde 16 Stunden gerührt und dann mit 2 N Natronlauge versetzt. Die Reaktionslösung wurde mit Ethylacetat extrahiert, die organische Phase über Natrimsulfat getrocknet und das Lösungsmittel im Vakuum entfernt. Der Rückstand wurde durch Chromatographie an Kieselgel (Start mit Heptan / Ethylacetat 1: 1 Gradient zu Ethylacetat / Triethylamin / Methanol 92:5:3) gereinigt. Man erhielt 6.7 g (3*R*)-6-Chlor-1,3-dimethyl-4-(1-methylpiperidin-4-yl)-3,4-dihydropyrido[2,3-b]pyrazin-2(1H)-on.
¹H NMR (400 MHz, 25°C, CDCl₃): δ = 1.20 (d, 3H), 1.62-1.68 (m, 1H), 1.75 (dq, 1H), 1.95 (dq, 1H), 2.07-2.21 (m, 3H), 2.31 (s, 3H), 2.94 (d, 2H), 3.29 (s, 3H), 4.2.5-4.35 (m, 2H), 6.66 (d, 1H), 6.97 (d, 1H).

### Intermediat 66:

### 2-Brom-N-(2,6-dichlorpyridin-3-yl)propanamid

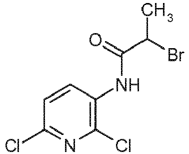

Eine Lösung von 8.5 g 3-Amino-2,6-dichlorpyridin (CAS 62476-59-9) in 200 ml THF und 12.7 ml Pyridin wurde bei RT langsam mit 20.3 g 2-Brompropionsäurebromid (CAS 563-76-8) versetzt. Man ließ 72 Stunden bei RT rühren. Dann wurde Wasser zugegeben und mit Ethylacetat extrahiert. Die organische Phase wurde über Natriumsulfat getrocknet und im Vakuum vollständig eingeengt. Der Rückstand wurde durch Chromatographie an Kieselgel (Dichlormethan) gereinigt. Man erhielt 8.2 g 2-Brom-*N*-(2,6-dichlorpyridin-3-yl)propanamid.
¹H-NMR (300 MHz, DMSO-d6): δ = 1.76 (d, 3H); 4.94 (q, 1H); 7.60 (d, 1H); 8.22 (d, 1H); 10.17 (s, 1H).

### Intermediat 67:

### N-(2,6-Dichlorpyridin-3-yl)-N²-phenylalaninamid

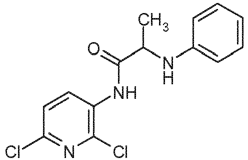

Eine Lösung von 2.7 g Intermediat 66 und 759 mg Anilin in 27 ml Toluol und 2.7 ml Di-*iso-*propylethylamin wurde 3 Stunden bei 140 °C gerührt. Nach Abkühlen auf RT wurde Wasser zugegeben und mit Ethylacetat extrahiert. Die organische Phase wurde über Natriumsulfat getrocknet und vollständig im Vakuum eingeengt. Der Rückstand wurde durch Chromatographie an Kieselgel (Dichlormethan) gereinigt. Man erhielt 3.1 g *N*-(2,6-Dichlorpyridin-3-yl)-*N2-*phenylalaninamid, welches eine ausreichende Reinheit für die weitere Umsetzung besaß.
¹H-NMR (300 MHz, DMSO-d6): δ = 1.44 (d, 3H); 4.12 (qi, 1H); 6.11 (d, 1H); 6.64 (d, 2H); 6.99 (t, 1H); 7.10 (t, 2H); 7.56 (d, 1H); 8.29 (d, 1H); 9.79 (s, 1H).

### Intermediat 68:

### 6-Chlor-3-methyl-4-phenyl-3,4-dihydropyrido[2,3-b]pyrazin-2(1H)-on

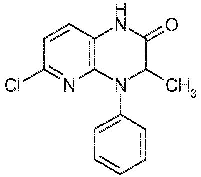

In Analogie zur Synthese von Intermediat 4 wurde 6-Chlor-3-methyl-4-phenyl-3,4-dihydropyrido[2,3-b]pyrazin-2(1H)-on ausgehend von 1.8 g Intermediat 67 und 12.3 ml *N*,*N-*Dicyclohexylmethylamin in 10 ml DMF durch 18 Stunden Erhitzen bei 170°C Badtemperatur hergestellt. Man erhielt 350 mg 6-Chlor-3-methyl-4-phenyl-3,4-dihydropyrido[2,3-b]pyrazin-2(1H)-on.
¹H-NMR (300 MHz, DMSO-d6): δ = 1.29 (d, 3H); 4.48 (q, 1H); 6.84 (d, 1H); 7.17 (d, 1H); 7.22 (t, 1H); 7.33 (d, 2H); 7.41 (t, 2H); 10.82 (s, 1H).

### Intermediat 69:

### 6-Chlor-1,3-dimethyl-4-phenyl-3,4-dihydropyrido[2,3-b]pyrazin-2(1H)-on

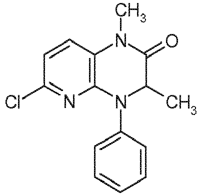

In Analogie zur Herstellung von Intermediat 5 wurde 6-Chlor-1,3-dimethyl-4-phenyl-3,4-dihydropyrido[2,3-b]pyrazin-2(1H)-on ausgehend von 500 mg Intermediat 68 (aus zwei Ansätzen erhalten), 120 mg Natriumhydrid (60% in Weissöl) und 0.171 ml Methyljodid in 9 ml DMF hergestellt. Nach Chromatographie an Kieselgel (Hexan / Ethylacetat Gradient) erhielt man 380 mg 6-Chlor-1,3-dimethyl-4-phenyl-3,4-dihydropyrido[2,3-b]pyrazin-2(1H)-on.
¹H-NMR (300 MHz, DMSO-d6): δ = 1.29 (d, 3H); 3.32 (s, 3H); 4.60 (q, 1H); 6.96 (d, 1H); 7.21 (t, 1H); 7.33 (d, 2H); 7.41 /t, 2H); 7.50 (d, 1H).

### Intermediat 70:

### N-(2,6-Dichlorpyridin-3-yl)-N²-(4-fluorphenyl)alaninamid

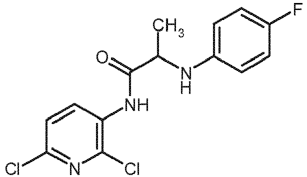

Eine Lösung von 2.0 g Intermediat 66 und 746 mg 4-Fluoranilin in 25 ml Toluol und 2.0 ml Di-*iso-*propylethylamin wurde 5 Stunden bei 130 °C gerührt. Nach Abkühlen auf RT wurde Wasser zugegeben und mit Ethylacetat extrahiert. Die organische Phase wurde über Natriumsulfat getrocknet und vollständig im Vakuum eingeengt. Der Rückstand wurde durch Chromatographie an Kieselgel (Hexan / Ethylacetat Gradient bis 50% Ethylacetat Anteil) gereinigt. Man erhielt 1.8 g *N*-(2,6-Dichlorpyridin-3-yl)-*N*²-(4-fluorphenyl)alaninamid.
¹H-NMR (300 MHz, DMSO-d6): δ = 1.43 (d, 3H); 4.04-4.12 (m, 1H); 6.08 (d, 1H); 6.63 (dd, 2H); 6.95 (t, 2H); 7.57 (d, 1H); 8.28 (d, 1H); 9.80 (bs, 1H).

### Intermediat 71:

### 6-Chlor-4-(4-fluorphenyl)-3-methyl-3,4-dihydropyrido[2,3-b]pyrazin-2(1H)-on

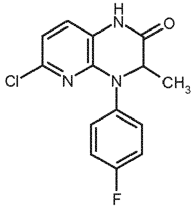

In Analogie zur Synthese von Intermediat 4 wurde 6-Chlor-4-(4-fluorphenyl)-3-methyl-3,4-dihydropyrido[2,3-b]pyrazin-2(1H)-on ausgehend von 1.8 g Intermediat 70 und 7.6 ml *N,N*-Di-iso-propylethylamin in 18 ml DMA durch 48 Stunden Erhitzen bei 175°C Badtemperatur hergestellt. Man erhielt 1.0 mg 6-Chlor-4-(4-fluorphenyl)-3-methyl-3,4-dihydropyrido[2,3-b]pyrazin-2(1H)-on.
¹H-NMR (300 MHz, DMSO-d6): δ = 1.27 (d, 3H); 4.45 (q, 1H); 6.81 (d, 1H); 7.14 (d, 1H); 7.25 /t, 2H); 7.38 (dd, 2H); 10.8 (bs, 1H).

### Intermediat 72:

### 6-Chlor-4-(4-fluorphenyl)-1,3-dimethyl-3,4-dihydropyrido[2,3-b]pyrazin-2(1H)-on

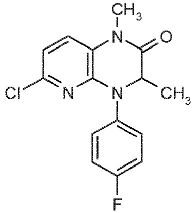

In Analogie zur Herstellung von Intermediat 5 wurde 6-Chlor-4-(4-fluorphenyl)-1,3-dimethyl-3,4-dihydropyrido[2,3-b]pyrazin-2(1H)-on ausgehend von 1.0 mg Intermediat 71, 224 mg Natriumhydrid (60% in Weissöl) und 0.32 ml Methyljodid in 20 ml DMF hergestellt. Nach Chromatographie an Kieselgel (Dichlormethan / Methanol Gradient bis 1% Methanol Anteil) erhielt man 870 mg 6-Chlor-4-(4-fluorphenyl)-1,3-dimethyl-3,4-dihydropyrido[2,3-b]pyrazin-2(1H)-on.
¹H-NMR (300 MHz, DMSO-d6): δ = 1.27 (d, 3H); 3.31 (s, 3H); 4.56 (q, 1H); 6.93 (d, 1H); 7.25 (t, 2H); 7.39 (dd, 1H); 7.47 (d, 1H).

### Intermediat 73:

### 3-{[4-(4-Fluorphenyl)-1,3-dimethyl-2-oxo-1,2,3,4-tetrahydropyrido[2,3-b]pyrazin-6-yl]amino}benzoesäuremethylester

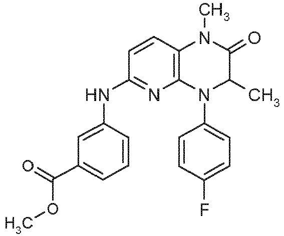

Eine Suspension von 225 mg Intermediat 73, 227 g 3-Aminobenzoesäuremethylester (CAS 4518-10-9), 33 mg Palladium(II)acetat, 1.2 g Caesiumcarbonat und 92 mg (+)-BINAP in 3 ml Toluol wurde 3 Stunden unter einer Argonatmosphäre bei 120°C und 56 Stunden bei RT gerührt. Die Reaktionslösung wurde auf Wasser gegeben, zweimal mit Ethylacetat extrahiert, die vereinten organischen Phasen über Natriumsulfat getrocknet und das Lösungsmittel im Vakuum entfernt. Der Rückstand wurde durch RP-HPLC (Waters Autopurificationsystem SQD; Säule: Waters XBrigde C18 5µ 100x30mm; Eluent A: Wasser + 0.1% Vol. Ameisensäure (99%), Eluent B: Acetonitril; Gradient: 0-8.0 min 1-100% B, 8.0-10.0 min 100% B; Fluss 50.0 m1/min; Temperatur: RT; Injektion: 2500 µl; DAD scan: 210-400 nm) gereinigt. Man erhielt 163 mg 3-{[4-(4-Fluorphenyl)-1,3-dimethyl-2-oxo-1,2,3,4-tetrahydropyrido[2,3-b]pyrazin-6-yl]amino}benzoesäuremethylester.

UPLC-MS: Instrument: Waters Acquity UPLC-MS SQD; Säule: Acquity UPLC BEH C18 1.7 50x2.1mm; Eluent A: Wasser + 0.1% Vol. Ameisensäure (99%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm
Rt = 1.27 min.

### Intermediat 74:

### 3-{[4-(4-Fluorphenyl)-1,3-dimethyl-2-oxo-1,2,3,4-tetrahydropyrido[2,3-b]pyrazin-6-yl] amino}benzoesäure

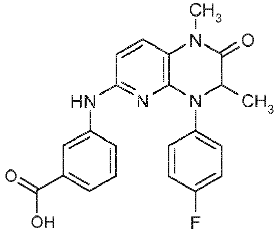

Eine Lösung von 150 mg Intermediat 73 in 4 ml THF wurde bei RT mit 0.89 ml Natronlauge (2 N) versetzt und bei 50°C für 3 Stunden und bei 90°C für 30 min. gerührt. Das THF wurde vollständig im Vakkum entfernt und die Lösung mit Wasser und 1N Salzsäure sauer gestellt. Der dabei gebildete Niederschlag wurde affiltriert und im Vakkum getrocknet. Man erhielt 135 mg 3-{[4-(4-Fluorphenyl)-1,3-dimethyl-2-oxo-1,2,3,4-tetrahydropyrido[2,3-b]pyrazin-6-yl]amino}benzoesäure.
¹H-NMR (300 MHz, DMSO-d6): δ = 1.27 (d, 3H); 3.28 (s, 3H); 4.50 (q, 1H); 6.35 (d, 1H); 6.94 (t, 1H); 7.21 - 7.30 (m, 3H); 7.34 - 7.43 (m, 4H); 7.57 - 7.63 (m, 1H); 7.72 (dd, 1H); 9.01 (s, 1H).

### Intermediat 75

### N-(2,6-Dichlorpyridin-3-yl)-N²-(3-methylphenyl)-D-alaninamid

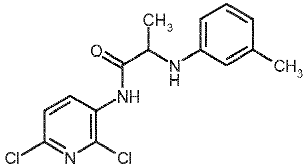

Eine Lösung von 1.5 g Intermediat 66 und 539 mg 3-Methylanilin in 20 ml Toluol und 1.5 ml Di*iso*-propylethylamin wurde 5 Stunden bei 130 °C gerührt. Nach Abkühlen auf RT wurde Wasser zugegeben und mit Ethylacetat extrahiert. Die organische Phase wurde über Natriumsulfat getrocknet und vollständig im Vakuum eingeengt. Der Rückstand wurde durch Chromatographie an Kieselgel (Hexan / Ethylacetat Gradient bis 50% Ethylacetat Anteil) gereinigt. Man erhielt 1.6 g *N*-(2,6-Dichlorpyridin-3-yl)-N²-(3-methylphenyl)-D-alaninamid.
¹H-NMR (300 MHz, DMSO-d6): δ = 1.43 (d, 3H); 2.18 (s, 3H); 4.03-4.16 (m, 1H); 6.01 (d, 1H); 6.40-6.50 (m, 3H); 6.98 (t, 1H); 7.57 (d, 1H); 8.28 (d, 1H), 9.80 (bs, 1H).

### Intermediat 76:

### 6-Chlor-3-methyl-4-(3-methylphenyl)-3,4-dihydropyrido[2,3-b]pyrazin-2(1H)-on

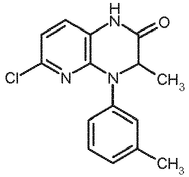

Eine Lösung von 1.6 g Intermediat 75 und 5.5 ml *N,N*-Diisopropylethylamin in 16 ml DMA wurde 72 Stunden bei 175°C Badtemperatur gerührt. Nach dem Erkalten wurde der Ansatz mit Wasser verdünnt und mit Ethylacetat extrahiert. Die vereinten organischen Phasen wurden im Vakuum eingeengt. Der Rückstand wurde in Dichlormethan verrührt und abgesaugt. Man erhielt 830 mg 6-Chlor-3-methyl-4-(3-methylphenyl)-3,4-dihydropyrido[2,3-b]pyrazin-2(1H)-on. ¹H-NMR (400 MHz, DMSO-d6): δ = 1.29 (d, 3H); 2.32 (s, 3H); 4.44 (q, 1H); 6.83 (d, 1H); 7.04 (d, 1H); 7.10-7.18 (m, 3H); 7.28 (t, 1H); 10.8 (bs, 1H).

### Intermediat 77:

### 6-Chlor-1,3-dimethyl-4-(3-methylphenyl)-3,4-dihydropyrido[2,3-b]pyrazin-2(1H)-on

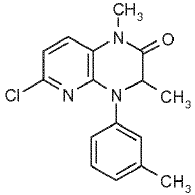

Eine Lösung von 830 mg Intermediat 76 und 0.27 ml Methyljodid in 20 ml DMF wurde bei 0°C mit 0.17 g Natriumhydrid (60% in Weißöl) versetzt. Nach einer Stunde Rühren bei 0°C und 14 Stunden bei RT wurde mit Dichlormethan verdünnt und dreimal mit halbgesättigter wässriger Natriumchloridlösung gewaschen. Die vereinten organischen Phasen wurden über Natriumsulfat getrocknet und im Vakuum vollständig eingeengt. Der Rückstand wurde durch Chromatographie an Kieselgel (Dichlormethan / Methanol Gradient bis 2% Methanol Anteil) gereinigt. Man erhielt 850 mg 6-Chlor-1,3-dimethyl-4-(3-methylphenyl)-3,4-dihydropyrido[2,3-b]pyrazin-2(1H)-on.
¹H-NMR (400 MHz, DMSO-d6): δ = 1.28 (d, 3H); 2.32 (s, 3H); 3.31 (s, 3H); 4.56 (q, 1H); 6.95 (d, 1H); 7.03 (bd, 1H); 7.10-7.17 (m, 2H); 7.28 (t, 1H); 7.49 (d, 1H).

### Intermediat 78:

### 5-{[(3R)-1,3-Dimethyl-2-oxo-4-(tetrahydro-2H-pyran-4-yl)-1,2,3,4-tetrahydropyrido[2,3-b]pyrazin-6-yl]amino}-2-methoxybenzoesäuremethylester

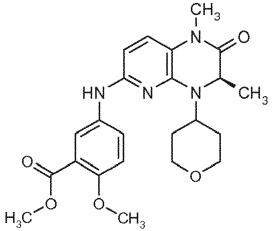

Eine Suspension von 800 mg Intermediat 10, 931 mg 5-Amino-2-methoxybenzoesäuremethylester (CAS 22802-67-1), 115 mg Palladium(II)acetat, 4.19 g Caesiumcarbonat und 320 mg (+)-BINAP in 57.5 ml Toluol wurde 5 Stunden unter einer Argonatmosphäre bei 120°C gerührt. Die Reaktionslösung wurde auf Wasser gegeben, zweimal mit Ethylacetat extrahiert, die vereinten organischen Phasen über Natriumsulfat getrocknet und das Lösungsmittel im Vakuum entfernt. Der Rückstand wurde durch Chromatographie an Kieselgel (Hexan / Ethylacetat Gradient bis 100% Ethylacetat Anteil) gereinigt. Man erhielt 810 mg 5-{[(3*R*)-1,3-Dimethyl-2-oxo-4-(tetrahydro-2H-pyran-4-yl)-1,2,3,4-tetrahydropyrido[2,3-b]pyrazin-6-yl]amino}-2-methoxybenzoesäuremethylester.
1H-NMR: (300 MHz, 25°C, DMSO-d6): δ = 1.07 (d, 3H); 1.57 (bd, 1H); 1.72 (qd, 1H); 1.79-1.96 (m, 2H); 3.19 (s, 3H); 3.31-3.49 (m, 2H); 3.77 (s, 3H); 3.78 (s, 3H); 3.88-3.99 (m, 2H); 4.20 (q, 1H); 4.38 (tt, 1H); 6.16 (d, 1H); 7.04 (d, 1H); 7.23 (d, 1H); 7.69 (dd, 1H); 7.87 (d, 1H); 8.72 (s, 1H).

### Intermediat 79:

### 5-{[(3R)-1,3-Dimethyl-2-oxo-4-(tetrahydro-2H-pyran-4-yl)-1,2,3,4-tetrahydropyrido[2,3-b]pyrazin-6-yl]amino}-2-methoxybenzoesäure

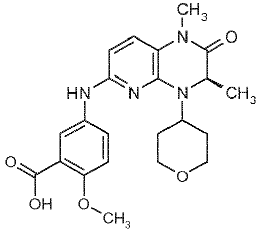

Eine Lösung von 780 mg Intermediat 78 in 6 ml THF und 40 ml Methanol wurde bei RT mit 17.7 ml 1N Lithiumhydroxidlösung versetzt und bei 60°C für 7 Stunden gerührt. Es wurde mit 1N Salzsäure auf pH = 7 eingestellt und zweimal mit Ethylacetat extrahiert. Die vereinten organischen Phasen wurden über Natriumsulfat getrocknet und das Lösungsmittel vollständig im Vakuum entfernt. Man erhielt 680 mg 5-{[(3*R*)-1,3-Dimethyl-2-oxo-4-(tetrahydro-2H-pyran-4-yl)-1,2,3,4-tetrahydropyrido[2,3-b]pyrazin-6-yl]amino}-2-methoxybenzoesäure.
¹H-NMR: (400 MHz, 25°C, DMSO-d6): δ = 1.06 (d, 3H); 1.55 (bd, 1H); 1.71 (qd, 1H); 1.79-1.95 (m, 2H); 3.19 (s, 3H); 3.38-3.52 (m, 2H); 3.47 (s, 3H); 3.87-3.97 (m, 2H); 4.20 (q, 1H); 4.44 (tt, 1H); 6.17 (d, 1H); 7.02 (d, 1H); 7.23 (d, 1H); 7.60 (dd, 1H); 7.96 (d, 1H); 8.70 (s, 1H); 12.29 (bs, 1H).

### Intermediat 80:

### 3-{[(3R)-1,3-Dimethyl-2-oxo-4-(tetrahydro-2H-pyran-4-yl)-1,2,3,4-tetrahydropyrido[2,3-b]pyrazin-6-yl]amino}-2-methoxybenzoesäuremethylester

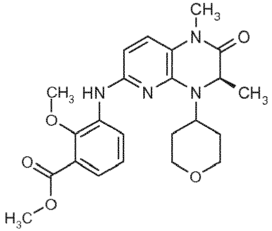

Eine Suspension von 600 mg Intermediat 10, 698 mg 3-Amino-2-methoxybenzoesäuremethylester (CAS 5129-25-9), 87 mg Palladium(II)acetat, 2.5 g Caesiumcarbonat und 240 mg (+)-BINAP in 43 ml Toluol wurde 7.5 Stunden unter einer Argonatmosphäre bei 120°C gerührt. Die Reaktionslösung wurde auf Wasser gegeben, zweimal mit Ethylacetat extrahiert, die vereinten organischen Phasen über Natriumsulfat getrocknet und das Lösungsmittel im Vakuum entfernt. Der Rückstand wurde durch Chromatographie an Kieselgel (Hexan / Ethylacetat Gradient bis 100% Ethylacetat Anteil) gereinigt. Man erhielt 325 mg 3-{[(3*R*)-1,3-Dimethyl-2-oxo-4-(tetrahydro-2H-pyran-4-yl)-1,2,3,4-tetrahydropyrido[2,3-b]pyrazin-6-yl]amino}-2-methoxybenzoesäuremethylester.
1H-NMR: (400 MHz, 25°C, CDCl₃): δ = 1.25 (d, 3H); 1.71 (bd, 1H); 1.85 (qd, 1H); 2.01 (qd, 1H); 2.12 (bd, 1H); 3.32 (s, 3H); 3.57 (t, 2H); 3.91 (s, 3H); 3.94 (s, 3H); 4.07-4.18 (m, 2H); 4.32 (q, 1H); 4.56 (tt, 1H); 6.25 (d, 1H); 6.95 (bs, 1H); 7.06 (d, 1H); 7.09 (t, 1H); 7.38 (dd, 1H); 8.43 (dd, 1H).

### Intermediat 81:

### 3-{[(3R)-1,3-Dimethyl-2-oxo-4-(tetrahydro-2H-pyran-4-yl)-1,2,3,4-tetrahydropyrido[2,3-b]pyrazin-6-yl]amino}-2-methoxybenzoesäure

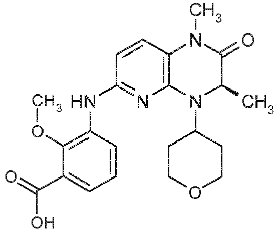

Eine Lösung von 300 mg Intermediat 80 in 2 ml THF und 16 ml Methanol wurde bei RT mit 6.8 ml 1N Lithiumhydroxidlösung versetzt und bei 60°C für 6 Stunden gerührt. Es wurde mit 1N Salzsäure auf pH = 7 eingestellt und zweimal mit Ethylacetat extrahiert. Die vereinten organischen Phasen wurden über Natriumsulfat getrocknet und das Lösungsmittel vollständig im Vakuum entfernt. Man erhielt 275 mg 5-{[(3*R*)-1,3-Dimethyl-2-oxo-4-(tetrahydro-2H-pyran-4-yl)-1,2,3,4-tetrahydropyrido[2,3-b]pyrazin-6-yl]amino}-2-methoxybenzoesäure.
¹H-NMR: (400 MHz, 25°C, DMSO-d6): δ = 1.09 (d, 3H); 1.60 (bd, 1H); 1.76 (qd, 1H); 1.86-2.00 (m, 2H); 3.21 (s, 3H); 3.34-3.48 (m, 2H); 3.76 (s, 3H); 3.92-4.03 (m, 2H); 4.23 (q, 1H); 4.35 (tt, 1H); 6.59 (d, 1H); 7.08 (t, 1H); 7.18 (dd, 1H); 7.27 (d, 1H); 8.14 (s, 1H); 8.49 (dd, 1H); 12.51 (bs, 1H).

### Intermediat 82:

### tert-Butyl-4-[4-({1-[(2,6-dichlorpyridin-3-yl)amino]-1-oxopropan-2-yl}amino)phenyl]piperazin-1-carboxylat

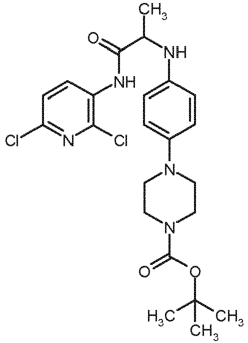

Eine Lösung von 5.1 g Intermediat 66 und 4.75 g tert-Butyl-4-(4-aminophenyl)piperazin-1-carboxylat (CAS 170911-92-9) in 63.75 ml Toluol und 5.96 ml Di-*iso*-propylethylamin wurde 14 Stunden bei 140 °C gerührt. Nach Abkühlen auf RT wurde Wasser zugegeben und mit Ethylacetat extrahiert. Die organische Phase wurde über Natriumsulfat getrocknet und vollständig im Vakuum eingeengt. Der Rückstand wurde durch Chromatographie an Kieselgel (Dichlormethan / Methanol Gradient bis 2% Methanol Anteil) gereinigt. Man erhielt 7.7 g *tert*-Butyl-4-[4-({1-[(2,6-dichlorpyridin-3-yl)amino]-1-oxopropan-2-yl}amino)phenyl]piperazin-1-carboxylat.
¹H-NMR: (400 MHz, 25°C, DMSO-d6): δ = 1.37-1.45 (m, 12H); 2.82-2.90 (m, 4H); 3.37-3.46 (m, 4H); 3.95-4.05 (m, 1H); 5.75 (d, 1H); 6.59 (d, 2H); 6.79 (d, 2H); 7.56 (d, 1H); 8.35 (d, 1H); 9.76 (s, 1H).

### Intermediat 83:

### tert-Butyl-4-{4-[6-chlor-3-methyl-2-oxo-2,3-dihydropyrido[2,3-b]pyrazin-4(1H)-yl]phenyl}piperazin-1-carboxylat

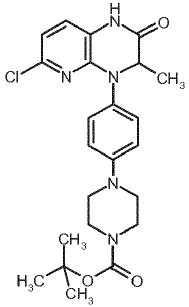

Eine Lösung von 7.7 g Intermediat 82 und 5.3 ml *N*,*N*-Diisopropylethylamin in 40 ml DMA wurde verteilt auf 4 dicht verschlossene Glasgefäße 48 Stunden bei 165°C Badtemperatur gerührt. Nach dem Erkalten wurden die vereinten Lösungen mit Wasser verdünnt und mit Ethylacetat extrahiert. Die vereinten organischen Phasen wurden im Vakuum eingeengt. Der Rückstand wurde durch Chromatographie an Kieselgel (Dichlormethan / Methanol Gradient bis 3% Methanol Anteil) und einer weiteren Chromatographie an Kieselgel (Hexan / Ethylacetat Gradient bis 25% Ethylacetat Anteil) gereinigt. Man erhielt 5.2 g *tert*-Butyl-4-{4-[6-chlor-3-methyl-2-oxo-2,3-dihydropyrido[2,3-b]pyrazin-4(1H)-yl]phenyl}piperazin-1-carboxylat.
¹H-NMR (400 MHz, 25°C, DMSO-d6): δ = 1.25 (d, 3H); 1.42 (s, 9H); 3.08-3.17 (m, 4H); 3.41-3.51 (m, 4H); 4.35 (d, 1H); 6.72 (d, 1H); 6.98 (d, 2H); 7.08 (d, 1H); 7.18 (d, 2H); 10.73 (s, 1H).

### Intermediat 84:

### tert-Butyl 4-[4-(6-chlor-1,3-dimethyl-2-oxo-2,3-dihydropyrido[2,3-b]pyrazin-4(1H)-yl)phenyl]piperazin-1-carboxylat

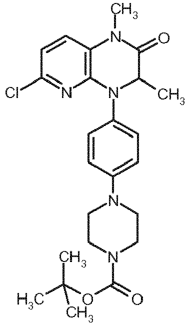

Eine Lösung von 750 mg Intermediat 83 und 0.145 ml Methyljodid in 30 ml DMF wurde bei 0°C mit 0.93 g Natriumhydrid (60% in Weißöl) versetzt. Nach einer Stunde Rühren bei 0°C und 14 Stunden bei RT wurde mit Ethylacetat verdünnt und dreimal mit halbgesättigter wässriger Natriumchloridlösung gewaschen. Die vereinten organischen Phasen wurden über Natriumsulfat getrocknet und im Vakuum vollständig eingeengt. Der Rückstand wurde durch Chromatographie an Kieselgel (Dichlormethan / Methanol Gradient bis 2% Methanol Anteil) gereinigt. Man erhielt 630 mg *tert*-Butyl 4-[4-(6-chlor-1,3-dimethyl-2-oxo-2,3-dihydropyrido[2,3-b]pyrazin-4(1H)-yl)phenyl]piperazin-1-carboxylat.
¹H-NMR (400 MHz, 25°C, DMSO-d6): δ = 1.25 (d, 3H); 1.43 (s, 9H); 3.10-3.16 (m, 4H); 3.30 (s, 3H); 3.43-3.50 (m, 4H); 4.45 (q, 1H); 6.85 (d, 1H); 6.99 (d, 2H); 7.19 (d, 2H); 7.41 (d, 1H).

### Intermediat 85:

### (3R)-4-Benzyl-6-brom-3-methyl-3,4-dihydrochinoxalin-2(1H)-on

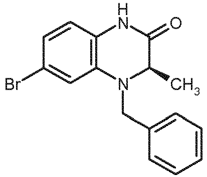

Eine Lösung von 5 g Intermediat 12, 6.6 g Benzaldehyd, 6.7 g Phenylsilan und 6.3 g Dibutylzinndichlorid in 70 ml THF wurde 85 Stunden bei RT gerührt. Die Lösung wurde im Vakuum vollständig eingeengt. Der Rückstand wurde durch Chromatographie an Kieselgel (Hexan / Ethylacetat Gradient bis 30% Ethylacetat Anteil) gereinigt. Man erhielt 6.14 g (3R)-4-Benzyl-6-brom-3-methyl-3,4-dihydrochinoxalin-2(1H)-on.
¹H-NMR (400 MHz, 25°C, CDCl₃): δ = 1.25 (d, 3H); 3.97 (q, 1H); 4.21 (d, 1H); 4.62 (d, 1H); 6.70 (d, 1H); 6.89 (d, 1H); 6.94 (dd, 1H); 7.32-7.44 (m, 5H); 8.98 (bs, 1H).

### Intermediat 86:

### (3R)-4-Benzyl-6-brom-1,3-dimethyl-3,4-dihydrochinoxalin-2(1H)-on

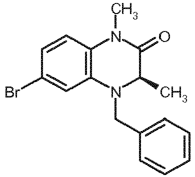

Eine Lösung von 6.14 g Intermediate 85 und 1.73 ml Methyljodid in 80 ml DMF wurde bei 0°C mit 1.11 g Natriumhydrid (60% in Weißöl) versetzt. Nach einer Stunde Rühren bei 0°C und 30 Minuten bei RT wurde mit gesättigter Ammoniumchlorid Lösung versetzt und mit Ethylacetat extrahiert. Die vereinten organischen Phasen wurden über Natriumsulfat getrocknet und im Vakuum vollständig eingeengt. Der Rückstand wurde durch Chromatographie an Kieselgel (Hexan / Ethylacetat Gradient bis 35% Ethylacetat Anteil) gereinigt. Man erhielt 5.9 g (3R)-4-Benzyl-6-brom-1,3-dimethyl-3,4-dihydrochinoxalin-2(1 H)-on.
¹H-NMR (400 MHz, 25°C, CDCl₃): δ = 1.11 (d, 3H); 3.37 (s, 3H); 3.96 (q, 1H); 4.12 (d, 1H); 4.54 (d, 1H); 6.81 (d, 1H); 6.85 (d, 1H); 6.99 (dd, 1H); 7.28-7.40 (m, 5H).

### Intermediat 87:

### tert-Butyl 4-[(2R)-7-brom-2-methyl-3-oxo-3,4-dihydrochinoxalin-1(2H)-yl]piperidin-1-carboxylat

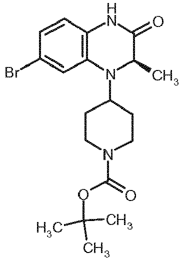

Eine Lösung von 3 g Intermediat 12, 7.4 g tert-Butyl-4-oxopiperidin-1-carboxylat (CAS 79099-07-3), 4.2 g Phenylsilan und 3.78 g Dibutylzinndichlorid in 100 ml THF wurde 76 Stunden bei RT gerührt. Nach Zugabe von Diatemeenerde wurde vollständig im Vakuum eingeengt. Der Rückstand wurde durch Chromatographie an Kieselgel (Hexan / Etylacetat Gradient bis 100% Ethylacetat Anteil) und RP-HPLC (Waters Autopurificationsystem SQD; Säule: Waters XBrigde C18 5µ 100x30mm; Eluent A: Wasser + 0.2% Vol. Ammoniak (32%), Eluent B: Acetonitril; Gradient: 0-8.0 min 1-100% B, 8.0-10.0 min 100% B; Fluss 50.0 ml/min; Temperatur: Rt; Injektion: 2500 µl; DAD scan: 210-400 nm)gereinigt. Man erhielt 2.4 g *tert*-Butyl 4-[(2*R*)-7-brom-2-methyl-3-oxo-3,4-dihydrochinoxalin-1(2H)-yl]piperidin-1-carboxylat.
¹H-NMR (400 MHz, DMSO-d6): δ = 0.97 (d, 3H); 1.41 (s, 9H); 1.46-1.65 (m, 3H); 1.88 (bd, 1H); 2.70-2.98 (m, 2H); 3.67 (tt, 1H); 3.91 (q, 1H); 3.95-4.08 (m, 2H); 6.74 (d, 1H); 6.89 (dd, 1H); 7.07 (d, 1H); 10.43 (s, 1H).

### Intermediat 88:

### tert-Butyl 4-[(2R)-7-brom-2,4-dimethyl-3-oxo-3,4-dihydrochinoxalin-1(2H)-yl]piperidin-1-carboxylat

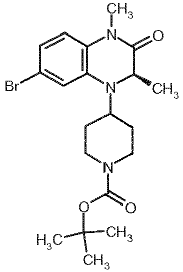

Eine Lösung von 2.4 g Intermediat 87 und 0.52 ml Methyljodid in 40 ml DMF wurde bei 0°C mit 520 mg Natriumhydrid (60% in Weißöl) versetzt. Nach einer Stunde Rühren bei 0°C wurde mit halb-gesättigter Natriumhydrogencarbonat-Lösung versetzt und dreimal mit Dichlormethan extrahiert. Die vereinten organischen Phasen wurden über Natriumsulfat getrocknet und im Vakuum vollständig eingeengt. Der Rückstand wurde durch Chromatographie an modifiziertem Kieselgel (Säule: Biotage KP-NH, Hexan / Ethylacetat Gradient bis 30% Ethylacetat Anteil) gereinigt. Man erhielt 2.47 g *tert*-Butyl 4-[(2*R*)-7-brom-2,4-dimethyl-3-oxo-3,4-dihydrochinoxalin-1(2H)-yl]piperidin-1-carboxylat.
¹H-NMR (400 MHz, CDCl₃): δ = 1.11 (d, 3H); 1.48 (s, 9H); 1.57-1.74 (m, 3H); 1.98 (bd, 1H); 2.70-2.97 (m, 2H); 3.35 (s, 3H); 3.46-3.56 (m, 1H); 4.10 (q, 1H); 4.15-4.34 (m, 2H); 6.83 (d, 1H); 7.01-7.08 (m, 2H).

### Intermediat 89:

### 3-{[(3R)-1,3-Dimethyl-2-oxo-4-(tetrahydro-2H-pyran-4-yl)-1,2,3,4-tetrahydrochinoxalin-6-yl]amino}benzoesäuremethylester

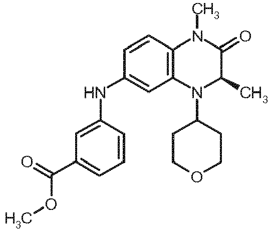

Eine Suspension von 2.0 g Intermediat 14, 1.69 g 3-Amino-2-methoxybenzoesäuremethylester (CAS 5129-25-9), 126 mg Palladium(II)acetat, .5.48 g Caesiumcarbonat und 349 mg (+)-BINAP in 125 ml Toluol wurde 14 Stunden unter einer Argonatmosphäre bei 120°C gerührt. Die Reaktionslösung wurde durch Kieselgur filtriert und das Lösungsmittel im Vakuum entfernt. Der Rückstand wurde durch Chromatographie an Kieselgel (Hexan / Ethylacetat Gradient bis 100% Ethylacetat Anteil) gereinigt. Man erhielt 1.5 g 3-{[(3*R*)-1,3-Dimethyl-2-oxo-4-(tetrahydro-2H-pyran-4-yl)-1,2,3,4-tetrahydrochinoxalin-6-yl]amino}benzoesäuremethylester.
1H-NMR: (400 MHz, 25°C, CDCl₃): δ = 1.13 (d, 3H); 1.68-1.97 (m, 4H); 3.37 (s, 3H); 3.43 (dt, 2H); 3.56 (tt, 1H); 3.90 (s, 3H); 3.99-4.10 (m, 2H); 4.10-4.19 (m, 1H); 5.74 (bs, 1H); 6.63-6.71 (m, 2H); 6.90 (d, 1H); 7.16 (dd, 1H); 7.32 /t, 1H); 7.55 (d, 1H); 7.73 (bs, 1H).

### Intermediat 90:

### 3-{[(3R)-1,3-Dimethyl-2-oxo-4-(tetrahydro-2H-pyran-4-yl)-1,2,3,4-tetrahydrochinoxalin-6-yl]amino}benzoesäure

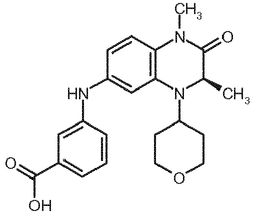

Eine Lösung von 300 mg Intermediat 89 in 2 ml THF und 16 ml Methanol wurde bei RT mit 6.9 ml 1N Lithiumhydroxidlösung versetzt und bei 60°C für 3 Stunden gerührt. Es wurde mit 1N Salzsäure auf pH = 7 eingestellt und zweimal mit Ethylacetat extrahiert. Die vereinten organischen Phasen wurden über Natriumsulfat getrocknet und das Lösungsmittel vollständig im Vakuum entfernt. Man erhielt 270 mg 3-{[(3*R*)-1,3-Dimethyl-2-oxo-4-(tetrahydro-2H-pyran-4-yl)-1,2,3,4-tetrahydrochinoxalin-6-yl]amino}benzoesäure.
¹H-NMR: (300 MHz, 25°C, DMSO-d6): δ = 0.98 (d, 3H); 1.58-1.93 (m, 4H); 3.25 (s, 3H); 3.36-3.45 (m, 2H); 3.59 (tt, 1H); 3.85-3.98 (m, 2H); 4.06 (q, 1H); 6.64 (dd, 1H); 6.72 (d, 1H); 6.99 (d, 1H); 7.15-7.23 (m, 1H); 7.27-7.36 (m, 2H); 7.67 (bs, 1H); 8.25 (s, 1H); 12.70 (bs, 1H).

### Intermediat 91:

### N-(1-Methylpiperidin-4-yl)-3-nitrobenzolsulfonamid

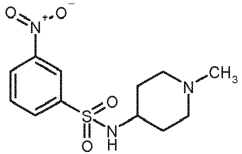

Eine Lösung von 3.0 g 3-Nitrobenzolsulfonsäurechlorid (CAS 121-51-7) und 1.62 g 1-Methylpiperidin-4-amin (CAS 41838-46-4) in 75 ml Dichlormethan wurden bei 0 °C mit 7.2 ml Triethylamin versetzt und 18 Stunden gerührt, wobei die Temperatur langsam auf RT angehoben wurde. Der Ansatz wurde mit Dichlormethan verdünnt und mit Wasser und gesättigter Natriumchlorid-Lösung gewaschen, über Natriumsulfat getrocknet und im Vakuum vollständig eingeengt. Der verbliebene Rückstand wurde durch Chromatographie an Kieselgel (Dichlormethan / Methanol Gradient) gereinigt. Man erhielt 3.13 g der Titelverbindung.
¹H-NMR (400MHz, 25°C, CDCl₃): δ = 1.55 - 1.67 (m, 2H); 1.86 (dd, 2H); 2.11 (t, 2H); 2.30 (s, 3H); 2.79 (d, 2H); 3.25 - 3.34 (m, 1H); 7.77 (t, 1H); 8.25 (dt, 1H); 8.46 (ddd, 1H); 8.76 (t, 1H).

### Intermediat 92:

### 3-Amino-N-(1-methylpiperidin-4-yl)benzenesulfonamid

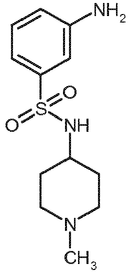

Eine Suspension von 3.5 g Intermediat 91 und 350 mg Palladium (10% auf Aktivkohle) in 100 ml Methanol wurde unter einer Wasserstoffatmosphäre bei RT für 8 Stunden geschüttelt. Es wurde durch Kieselgur abfiltriert und die Lösung im Vakuum vollständig eingeengt. Man erhielt 2.8 g der Titelverbindung.
¹H-NMR (300MHz, 25°C, DMSO-d6): δ = 1.26 - 1.42 (m, 2H); 1.45 - 1.57 (m, 2H); 1.70 - 1.83 (m, 2H); 2.05 (s, 3H); 2.58 (bd, 2H); 2.75 - 2.89 (m, 1H); 5.54 (bs, 2H); 6.71 (bdd, 1H); 6.88 (bd, 1H); 6.97 (t, 1H); 7.16 (t, 1H); 7.45 (d, 1H).

### Intermediat 93:

### N-[2-(Dimethylamino)ethyl]-3-nitrobenzolsulfonamid

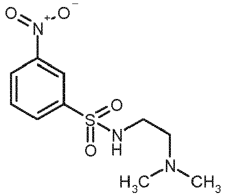

Eine Lösung von 3.0 g 3-Nitrobenzolsulfonsäurechlorid (CAS 121-51-7) und 1.245 g *N*,*N-*Dimethylethan-1,2-diamin (CAS 108-00-9) in 75 ml Dichlormethan wurden bei 0 °C mit 7.2 ml Triethylamin versetzt und 19 Stunden gerührt, wobei die Temperatur langsam auf RT angehoben wurde. Der Ansatz wurde mit Dichlormethan verdünnt und mit Wasser und gesättigter Natriumchlorid-Lösung gewaschen, über Natriumsulfat getrocknet und im Vakuum vollständig eingeengt. Der verbliebene Rückstand wurde durch Chromatographie an Kieselgel (Dichlormethan / Methanol Gradient) gereinigt. Man erhielt 3.30 g der Titelverbindung.
¹H-NMR (300MHz, 25°C, CDCl₃): δ = 2.15 (s, 6H); 2.38 - 2.46 (m, 2H); 3.04 - 3.11 (m, 2H); 7.77 (t, 1H); 8.25 (d, 1H); 8.46 (ddd, 1H); 8.75 (bt, 1H).

### Intermediat 94:

### 3-Amino-N-[2-(dimethylamino)ethyl]benzenesulfonamid

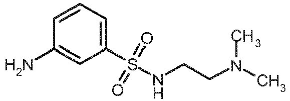

Eine Suspension von 3.3 g Intermediat 93 und 330 mg Palladium (10% auf Aktivkohle) in 200 ml Methanol wurde unter einer Wasserstoffatmosphäre bei RT für 7 Stunden geschüttelt. Es wurde durch Kieselgur abfiltriert und die Lösung im Vakuum vollständig eingeengt. Man erhielt 2.8 g der Titelverbindung als gelben Schaum.
¹H-NMR (400MHz, 25°C, DMSO-d6): δ = 2.05 (s, 6H); 2.22 (t, 2H); 2.78 (t, 2H); 5.53 (bs, 2H); 6.73 (ddd, 1H); 6.86 (dd, 1H); 6.97 (t, 1H); 7.17 (t, 1H); 7.20 - 7.27 (m, 1H).

### Intermediat 95:

### 4-[(4-Chlor-3-nitrophenyl)sulfonyl] morpholin

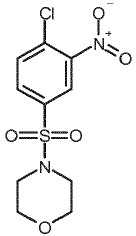

Eine Lösung von 22 g 4-Chlor-3-nitrobenzolsulfonsäurechlorid (CAS 97-08-5) in 330 ml Dichlormethan wurde bei -40 °C mit 18 ml Triethylamin sowie 7.49 g Morpholin (CAS 110-91-8) tropfenweise versetzt und 1 Stunde bei -40 °C gerührt, die Temperatur wurde langsam auf RT angehoben. Der Ansatz wurde mit Wasser und gesättigter Natriumchlorid-Lösung gewaschen, über Magnesiumsulfat getrocknet und im Vakuum vollständig eingeengt. Man erhielt 24 g der Titelverbindung als gelben Feststoff.
¹H NMR (400 MHz, 25°C, CDCl₃): δ = 3.05-3.07 (m, 4H); 3.76-3.78 (m, 4H); 7.77 (d, 1H); 7.87 (dd, 1H); 8.23 (d, 1H).

### Intermediat 96:

### 4-[(4-Methoxy-3-nitrophenyl)sulfonyl]morpholin

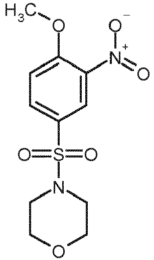

Eine Lösung von 28 g 4-[(4-Chlor-3-nitrophenyl)sulfonyl]morpholin (Intermediat 95) in 250 ml Methanol wurde bei 25 °C mit 84 ml Natriummethoxid (30%ige Lösung in Methanol) versetzt und 2 Stunden gerührt. Der Ansatz wurde mit Eis versetzt, nach dem Auftauen bei RT filtriert, mit Wasser gewaschen und getrocknet. Man erhielt 26 g der Titelverbindung als gelben Feststoff.
¹H NMR (400 MHz, 25°C, CDCl₃): δ = 3.02 (dd, 4H); 3.76 (dd, 4H); 4.06 (s, 3H); 7.23-7.25 (m, 1H); 7.91 (dd, 1H); 8.21 (d, 1H).

### Intermediat 97:

### 2-Methoxy-5-(morpholin-4-ylsulfonyl)anilin

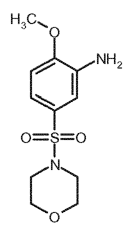

Eine Suspension von 1.0 g Intermediat 96 in 45.8 ml Ethanol wurde mit 3.73 g Zinn(II)chlorid Dihydrat 1.5 Stunden unter Rückfluß gerührt. Dann wurde mit gesättigter Natriumcarbonatlösung auf pH 8 eingestellt, 1 Stunde gerührt, dreimal mit Ethylacetat extrahiert, zweimal mit gesättigter Kochsalzlösung gewaschen, über Magnesiumsulfat getrocknet und im Vakuum vollständig eingeengt. Man erhielt 790 mg der Titelverbindung als beigen Feststoff.
¹H NMR (400 MHz, 25°C, DMSO-d6): δ = 2.78-2.80 (m, 4H); 3.60-3.63(m, 4H); 3.85 (s, 3H); 5.27 (s, 2H); 6.88 (dd, 1H); 6.96-7.00 (m, 2H).

### Intermediat 98:

### 3,3-Difluor-1-[(3-nitrophenyl)sulfonyl]azetidin

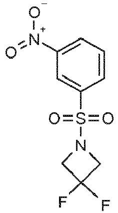

Eine Lösung von 10.27 g 3-Nitrobenzolsulfonsäurechlorid (CAS 121-51-7) und 4.0 g 3,3-Difluorazetidin Hydrochlorid (CAS 288315-03-7) in 120 ml Dichlormethan wurden bei 0 °C mit 17.2 ml Triethylamin versetzt und 1 Stunde gerührt, die Temperatur wurde langsam auf RT angehoben. Der Ansatz wurde mit Wasser und gesättigter Natriumchlorid-Lösung gewaschen, über Magnesiumsulfat getrocknet, im Vakuum vollständig eingeengt und aus warmen Ethylacetat kristallisiert. Man erhielt 7.28 g der Titelverbindung als Feststoff.
¹H-NMR (400 MHz, 25°C, CDC13): δ = 4.27 (t, 4H); 7.83 (t, 1H); 8.19 (dt, 1H); 8.53 (ddd, 1H); 8.70 (d, 1H).

### Intermediat 99:

### 3-[(3,3-Difluoroazetidin-1-yl)sulfonyl]anilin

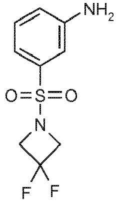

Eine Suspension von 114 mg Intermediat 98 und 14 mg Palladium (5% auf Aktivkohle) in 1 ml Ethylacetat wurde unter einer Wasserstoffatmosphäre bei RT für 2 Stunden gerührt. Es wurde durch Celite abfiltriert und die Lösung im Vakuum vollständig eingeengt. Man erhielt 97 mg rohe Titelverbindung, die mit Diethylether zum beigen Feststoff ausgerührt wurde.
¹H NMR (400 MHz, 25°C, DMSO-d6): δ = 4.15 (t, 4H), 5.72 (s, 2H), 6.88 (bs, 2H), 6.97 (s, 1H), 7.28 (t, 1H).

### Intermediat 100:

### 6-[(4-Methoxy-3-nitrophenyl)sulfonyl]-2-oxa-6-azaspiro [3.3] heptan

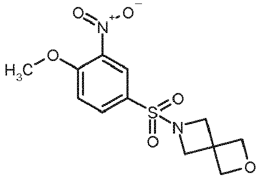

Eine Lösung von 3.0 g 4-Methoxy-3-nitrobenzolsulfonsäurechlorid (CAS 22117-79-9) in 60 ml Dichlormethan wurde mit 2.11 g 2-Oxa-6-azaspiro[3.3]heptan Oxalat (1:2) (CAS 1045709-32-7, 1159599-99-1) versetzt, bei 0 °C Badtemperatur wurden 6.45 ml Triethylamin dazugegeben und 18 Stunden gerührt, wobei die Temperatur langsam auf RT angehoben wurde. Der Ansatz wurde mit Dichlormethan verdünnt und mit Wasser und gesättigter Natriumchlorid-Lösung gewaschen, über Natriumsulfat getrocknet und im Vakuum vollständig eingeengt. Der verbliebene Rückstand wurde durch Chromatographie an Kieselgel (Dichlormethan / Methanol Gradient bis 1% Methanol Anteil) gereinigt. Man erhielt 1.5 g 6-[(4-Methoxy-3-nitrophenyl)sulfonyl]-2-oxa-6-azaspiro[3.3]heptan.
¹H-NMR (400MHz, 25°C, DMSO-d6): δ = 3.92 (s, 4H); 4.03 (s, 3H); 4.45 (s, 4H); 7.59 (d, 1H); 8.05 (dd, 1H); 8.25 (d, 1H).

### Intermediat 101:

### 2-Methoxy-5-(2-oxa-6-azaspiro[3.3]hept-6-ylsulfonyl)anilin

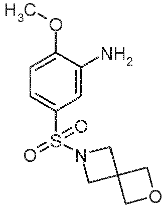

Eine Suspension von 1.50 g Intermediat 100 und 0.15 g Palladium (10% auf Aktivkohle) in 300 ml Methanol wurde unter einer Wasserstoffatmosphäre bei RT für 8 Stunden geschüttelt. Es wurde durch Kieselgur abfiltriert und die Lösung im Vakuum vollständig eingeengt. Man erhielt 1.20 g 2-Methoxy-5-(2-oxa-6-azaspiro[3.3]hept-6-ylsulfonyl)anilin als beigen Feststoff.
¹H-NMR (400MHz, 25°C, DMSO-d6): δ = 3.81 (s, 4H); 3.86 (s, 3H); 4.46 (s, 4H); 5.27 (bs, 2H); 6.94 (dd, 1H); 7.00 (d, 1H); 7.04 (d, 1H).

### Intermediat 102:

### 2-[(3-Nitrophenyl)sulfonyl]-2-azaspiro[3.3]heptan

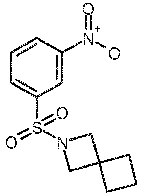

Eine Lösung von 0.27 g 3-Nitrobenzolsulfonsäurechlorid (CAS 121-51-7) und 0.17 g 2-Azaspiro[3.3]heptan Hydrochlorid (1:1) (CAS 665-04-3) in 27 ml Dichlormethan wurden bei 0 °C Badtemperatur mit 0.65 ml Triethylamin versetzt und 18 Stunden gerührt, wobei die Temperatur langsam auf RT angehoben wurde. Der Ansatz wurde mit Dichlormethan verdünnt und mit Wasser und gesättigter Natriumchlorid-Lösung gewaschen, über Natriumsulfat getrocknet und im Vakuum vollständig eingeengt. Man erhielt 310 mg 2-[(3-Nitrophenyl)sulfonyl]-2-azaspiro[3.3]heptan.
¹H-NMR (400MHz, 25°C, DMSO-d6): δ = 1.58 - 1.67 (m, 2H); 1.85 - 1.90 (m, 4H); 3.73 (s, 4H); 7.97 (t, 1H); 8.23 (dt, 1H); 8.40 (t, 1H); 8.57 (ddd, 1H).

### Intermediat 103:

### 3-(2-Azaspiro[3.3]hept-2-ylsulfonyl)anilin

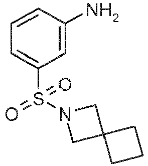

Eine Suspension von 0.30 g Intermediat 102 und 37.5 mg Palladium (10% auf Aktivkohle) in 24 ml Methanol wurde unter einer Wasserstoffatmosphäre bei RT für 7 Stunden geschüttelt. Es wurde durch Kieselgur abfiltriert und die Lösung im Vakuum vollständig eingeengt. Man erhielt 0.23 g 3-(2-Azaspiro[3.3]hept-2-ylsulfonyl)anilin als gelbes Öl.
¹H-NMR (400MHz, 25°C, DMSO-d6): δ = 1.59 - 1.70 (m, 2H); 1.88 (t, 4H); 3.61 (s, 4H); 5.63 (bs, 2H); 6.83 (t, 2H); 6.95 (bs, 1H); 7.26 (t, 1H).

### Intermediat 104:

### N-{4-[4-(Cyclopropylmethyl)piperazin-1-yl]cyclohexyl}-3-nitrobenzolsulfonamid

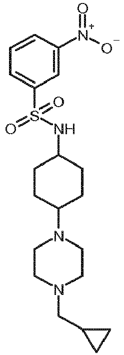

Eine Lösung von 3.0 g 3-Nitrobenzolsulfonsäurechlorid (CAS 121-51-7) und 3.36 g 4-[4-(cyclopropylmethyl)piperazin-1-yl]cyclohexanamin (CAS 876461-31-3, hergestellt analog WO2012049153) in 75 ml Dichlormethan wurden bei 0 °C mit 7.17 ml Triethylamin versetzt und 16 Stunden gerührt, wobei die Temperatur langsam auf RT angehoben wurde. Der Ansatz wurde mit Dichlormethan verdünnt und mit Wasser und gesättigter Natriumchlorid-Lösung gewaschen, über Natriumsulfat getrocknet und im Vakuum vollständig eingeengt. Der verbliebene Rückstand wurde durch Chromatographie an Kieselgel (Dichlormethan / Methanol Gradient bis 1% Methanol Anteil) gereinigt. Man erhielt 1.3 mg *N*-{-[4-(Cyclopropylmethyl)piperazin-1-yl]cyclohexyl}-3-nitrobenzolsulfonamid.
¹H-NMR (300MHz, 25°C, DMSO-d6): δ = 0.01 (q, 2H); 0.35 - 0.46 (m, 2H); 0.75 (t, 1H); 1.13 (t, 4H); 1.65 (d, 4H); 2.05 - 2.13 (m, 4H); 2.38 (bs, 8H); 7.88 (t, 1H); 8.03 (d, 1H); 8.22 (bd, 1H); 8.45 (ddd, 1H); 8.53 (t, 1H).

### Intermediat 105:

### 3-Amino-N-{cis-4-[4-(cyclopropylmethyl)piperazin-1-yl]cyclohexyl}benzolsulfonamid

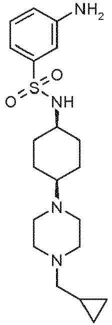

Eine Suspension von 1.30 g Intermediat 104 und 0.19 g Palladium (10% auf Aktivkohle) in 100 ml Methanol wurde unter einer Wasserstoffatmosphäre bei RT für 4 Stunden geschüttelt. Es wurde durch Kieselgur abfiltriert, die Lösung im Vakuum vollständig eingeengt und chromatographisch gereinigt. Man erhielt 1.1 g 3-Amino-N-{4-[4-(cyclopropylmethyl)piperazin-1-yl]cyclohexyl}benzolsulfonamid. Dieses wurde durch RP-HPLC (Waters Autopurificationsystem SQD; Säule: Waters XBrigde C18 5µm 100x30mm; Eluent A: Wasser + 0.1% Vol. Ameisensäure (99%), Eluent B: Acetonitril; Gradient: 0-8.0 min 1-100% B, 8.0-10.0 min 100% B; Fluss 50.0 ml/min; Temperatur: Rt; Injektion: 2500 µl; DAD scan: 210-400 nm) in die *cis*/*trans-*Isomere getrennt. Man erhielt 255 mg 3-Amino-*N*-{cis-4-[4-(cyclopropylmethyl)piperazin-1-yl]cyclohexyl}benzolsulfonamid.
¹H-NMR (500MHz, 25°C, DMSO-d6): δ = 0.00 - 0.04 (m, 2H); 0.39 - 0.44 (m, 2H); 0.73 - 0.80 (m, 1H); 1.06 - 1.18 (m, 6H); 1.68 (d, 4H); 2.03 - 2.11 (m, 3H); 2.32 - 2.44 (m, 6H); 2.76 - 2.87 (m, 1H); 5.51 (s, 2H); 6.72 (ddd, 1H); 6.89 (bd, 1H); 6.99 (t, 1H); 7.16 (t, 1H); 7.39 (d, 1H).

UPLC-MS: Instrument: Waters Acquity UPLC-MS SQD; Säule: Acquity UPLC BEH C18 1.7 50x2.1mm; Eluent A: Wasser + 0.2% Vol. Ammonik (32%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm
Rt = 0.95 min.

### Intermediat 106:

### 3-Amino-N-{trans-4-[4-(cyclopropylmethyl)piperazin-1-yl]cyclohexyl}benzolsulfonamid

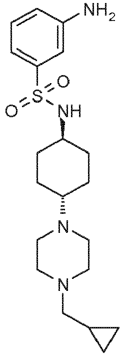

Eine Suspension von 1.30 g Intermediat 104 und 0.19 g Palladium (10% auf Aktivkohle) in 100 ml Methanol wurde unter einer Wasserstoffatmosphäre bei RT für 4 Stunden geschüttelt. Es wurde durch Kieselgur abfiltriert, die Lösung im Vakuum vollständig eingeengt und chromatographisch gereinigt. Man erhielt 1.1 g 3-Amino-*N*-{4-[4-(cyclopropylmethyl)piperazin-1-yl]cyclohexyl}benzolsulfonamid. Dieses wurde durch RP-HPLC (Waters Autopurificationsystem SQD; Säule: Waters XBrigde C18 5µm 100x30mm; Eluent A: Wasser + 0.1% Vol. Ameisensäure (99%), Eluent B: Acetonitril; Gradient: 0-8.0 min 1-100% B, 8.0-10.0 min 100% B; Fluss 50.0 ml/min; Temperatur: Rt; Injektion: 2500 µl; DAD scan: 210-400 nm) in die *cis*/*trans-*Isomere getrennt. Man erhielt 40 mg 3-Amino-*N*-{*trans*-4-[4-(cyclopropylmethyl)piperazin-1-yl]cyclohexyl}benzolsulfonamid.
¹H-NMR (500MHz, 25°C, DMSO-d6): δ = 0.00 - 0.04 (m, 2H); 0.39 - 0.44 (m, 2H); 0.73 - 0.80 (m, 1H); 1.06 - 1.18 (m, 6H); 1.68 (d, 4H); 2.03 - 2.11 (m, 3H); 2.32 - 2.44 (m, 6H); 2.76 - 2.87 (m, 1H); 5.51 (s, 2H); 6.72 (ddd, 1H); 6.89 (bd, 1H); 6.99 (t, 1H); 7.16 (t, 1H); 7.39 (d, 1H).

UPLC-MS: Instrument: Waters Acquity UPLC-MS SQD; Säule: Acquity UPLC BEH C18 1.7 50x2.1mm; Eluent A: Wasser + 0.2% Vol. Ammonik (32%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm
Rt = 0.92 min.

### Intermediat 107:

### 1-[(3-Nitrophenyl)sulfonyl]-4-(2,2,2-trifluorethyl)piperazin

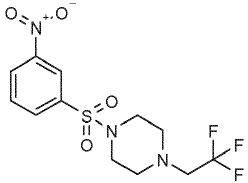

Eine Lösung von 2.0 g 3-Nitrobenzolsulfonsäurechlorid (CAS 121-51-7) und 1.59 g 1-(2,2,2-trifluoroethyl)piperazine (CAS 13349-90-1) in 50 ml Dichlormethan wurden bei 0 °C mit 4.78 ml Triethylamin versetzt und 18 Stunden gerührt, wobei die Temperatur langsam auf RT angehoben wurde. Der Ansatz wurde mit Dichlormethan verdünnt und mit Wasser und gesättigter Natriumchlorid-Lösung gewaschen, über Natriumsulfat getrocknet und im Vakuum vollständig eingeengt. Man erhielt 3.1 g 1-[(3-Nitrophenyl)sulfonyl]-4-(2,2,2-trifluorethyl)piperazin als gelben Feststoff.
¹H-NMR (400MHz, 25°C, DMSO-d6): δ = 2.70 (bt, 4H); 2.96 - 3.03 (m, 4H); 3.20 (q, 2H); 7.97 (t, 1H); 8.18 (dt, 1H); 8.37 (t, 1H); 8.57 (ddd, 1H).

### Intermediat 108:

### 3-{[4-(2,2,2-Trifluorethyl)piperazin-1-yl]sulfonyl}anilin

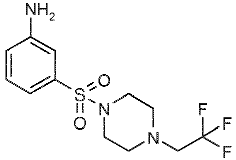

Eine Suspension von 3.1 g Intermediat 107 und 0.31 g Palladium (10% auf Aktivkohle) in 200 ml Methanol wurde unter einer Wasserstoffatmosphäre bei RT für 7 Stunden geschüttelt. Es wurde durch Kieselgur abfiltriert und die Lösung im Vakuum vollständig eingeengt. Man erhielt 2.8 g 3-{[4-(2,2,2-Trifluorethyl)piperazin-1-yl]sulfonyl}anilin als gelben Feststoff.
¹H-NMR (400MHz, 25°C, DMSO-d6): δ = 2.64 - 2.69 (m, 4H); 2.82 - 2.88 (m, 4H); 3.17 (q, 2H); 5.63 (s, 2H); 6.77 (ddd, 1H); 6.83 (ddd, 1H); 6.88 (t, 1H); 7.24 (t, 1H).

### Intermediat 109:

### 1-Methyl-4-[(3-nitrophenyl)sulfonyl]piperazin

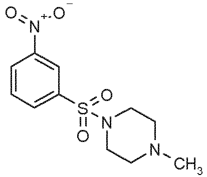

Eine Lösung von 3.0 g 3-Nitrobenzolsulfonsäurechlorid (CAS 121-51-7) und 1.42 g 1-Methylpiperazin (CAS 109-01-3) in 75 ml Dichlormethan wurden bei 0 °C mit 7.2 ml Triethylamin versetzt und 19 Stunden gerührt, wobei die Temperatur langsam auf RT angehoben wurde. Der Ansatz wurde mit Dichlormethan verdünnt und mit Wasser und gesättigter Natriumchlorid-Lösung gewaschen, über Natriumsulfat getrocknet und im Vakuum vollständig eingeengt. Der verbliebene Rückstand wurde durch Chromatographie an Kieselgel (Dichlormethan / Methanol Gradient bis 1 % Methanol Anteil) gereinigt. Man erhielt 3.55 g 1-Methyl-4-[(3-nitrophenyl)sulfonyl]piperazin als beigen Feststoff.
¹H-NMR (400MHz, 25°C, DMSO-d6): δ = 2.13 (s, 3H); 2.32 - 2.38 (m, 4H); 2.94 - 3.00 (m, 4H); 7.95 (t, 1H); 8.17 (bd, 1H); 8.36 (t, 1H); 8.54 (ddd, 1H).

### Intermediat 110:

### 3-[(4-Methylpiperazin-1-yl)sulfonyl]anilin

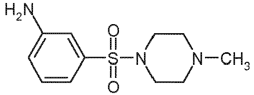

Eine Suspension von 3.55 g Intermediat 109 und 0.35 g Palladium (10% auf Aktivkohle) in 100 ml Methanol wurde unter einer Wasserstoffatmosphäre bei RT für 7 Stunden geschüttelt. Es wurde durch Kieselgur abfiltriert und die Lösung im Vakuum vollständig eingeengt. Man erhielt 3.18 g 3-[(4-Methylpiperazin-1-yl)sulfonyl]anilin als beigen Feststoff.
¹H-NMR (300MHz, 25°C, DMSO-d6): δ = 2.12 (s, 3H); 2.33 (t, 4H); 2.84 (bt, 4H); 5.63 (s, 2H); 6.76 (ddd, 1H); 6.81 (ddd, 1H); 6.88 (bt, 1H); 7.23 (t, 1H).

### Intermediat 111:

### N-[(1-Methylpiperidin-4-yl)methyl]-3-nitrobenzolsulfonamid

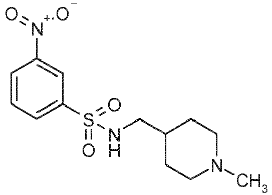

Eine Lösung von 1.50 g 3-Nitrobenzolsulfonsäurechlorid (CAS 121-51-7) und 0.91 g 1-(1-Methylpiperidin-4-yl)methanamin (CAS 7149-42-0) in 37.5 ml Dichlormethan wurden bei 0 °C mit 3.59 ml Triethylamin versetzt und 19 Stunden gerührt, wobei die Temperatur langsam auf RT angehoben wurde. Der Ansatz wurde mit Dichlormethan verdünnt und mit Wasser und gesättigter Natriumchlorid-Lösung gewaschen, über Natriumsulfat getrocknet und im Vakuum vollständig eingeengt. Der verbliebene Rückstand wurde durch Chromatographie an Kieselgel (Dichlormethan / Methanol Gradient) gereinigt. Man erhielt 1.6 g *N*-[(1-Methylpiperidin-4-yl)methyl]-3-nitrobenzolsulfonamid als gelben Schaum.
¹H-NMR (300MHz, 25°C, DMSO-d6): δ = 0.96 - 1.11 (m, 2H); 1.18 - 1.32 (m, 1H); 1.55 (bd, 2H); 1.72 (td, 2H); 2.09 (s, 3H); 2.66 (d, 4H); 7.89 (t, 1H); 7.98 (bs, 1H); 8.20 (dt, 1H); 8.47 (ddd, 1H); 8.49 - 8.53 (m, 1H).

### Intermediat 112:

### 3-Amino-N-[(1-methylpiperidin-4-yl)methyl]benzenesulfonamid

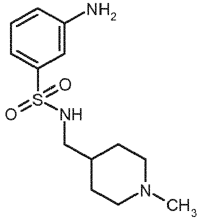

Eine Suspension von 1.60 g Intermediat 111 und 0.16 g Palladium (10% auf Aktivkohle) in 50 ml Methanol wurde unter einer Wasserstoffatmosphäre bei RT für 7 Stunden geschüttelt. Es wurde durch Kieselgur abfiltriert und die Lösung im Vakuum vollständig eingeengt. Man erhielt 1.40 g 3-Amino-*N*-[(1-methylpiperidin-4-yl)methyl]benzenesulfonamid als beigen Schaum.
¹H-NMR (400MHz, 25°C, DMSO-d6): δ = 0.96 - 1.09 (m, 2H); 1.26 (td, 1H); 1.56 (d, 2H); 1.68 - 1.77 (m, 2H); 2.09 (s, 3H); 2.57 (t, 2H); 2.63 - 2.72 (m, 2H); 5.52 (s, 2H); 6.72 (ddd, 1H); 6.83 - 6.86 (m, 1H); 6.95 (t, 1H); 7.16 (t, 1H); 7.36 (t, 1H).

### Intermediat 113:

### (3S)-6-Chlor-1,3-dimethyl-4-(tetrahydro-2H-pyran-4-yl)-3,4-dihydropyrido[2,3-b]pyrazin-2(1H)-on

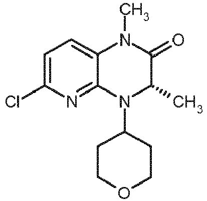

Bei der Enantiomerentrennung von Intermediat 10 wurden kleinere Mengen (3*S*)-6-Chlor-1,3-dimethyl-4-(tetrahydro-2*H-*pyran-4-yl)-3,4-dihydropyrido[2,3-*b*]pyrazin-2(1*H*)-on isoliert.

Instrument: Waters Alliance 2695; Säule: Chiralpak IC 5 m 250x30 mm, Eluent Hexan / 2-Propanol 70:30 (v/v); Flussrate 35 ml/min Temperatur: 25 °C; DAD 996 scan: 280 nm. Rt = 15.1-16.6 min
¹H-NMR (400 MHz, CDCl₃): δ = 1.23 (d, 3H); 1.62-1.69 (m, 1H); 1.81 (dq, 1H); 1.96 (dq, 1H); 2.02-2.09 (m, 1H); 3.31 (s, 1H); 3.51-3.62 (m, 2H); 4.02-4.10 (m, 2H); 4.31 (q, 1H); 4.54 (tt, 1H); 6.70 (d, 1H); 7.00 (d, 1H).

### Intermediat 114:

### 1-[(4-Chlor-3-nitrophenyl)sulfonyl]-4-methylpiperazin

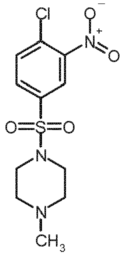

Eine Lösung von 24 g 4-Chlor-3-nitrobenzolsulfonsäurechlorid (CAS 109-01-3) in 360 ml Dichlormethan wurde bei -40 °C mit 19.6 ml Triethylamin sowie 10.4 ml 1-Methylpiperazin (CAS 110-91-8) tropfenweise versetzt und 1 Stunde bei -40 °C gerührt, die Temperatur wurde langsam auf RT angehoben. Der Ansatz wurde mit Wasser und gesättigter Natriumchlorid-Lösung gewaschen, über Magnesiumsulfat getrocknet und im Vakuum vollständig eingeengt. Man erhielt 28 g 1-[(4-Chlor-3-nitrophenyl)sulfonyl]-4-methylpiperazin als gelben Feststoff.
¹H NMR (400 MHz, 25°C, CDCl₃) δ = 2.27 (s, 3H); 2.49 (t, 4H); 3.08 (bs, 4H); 7.73 (d, 1H); 7.86 (dd, 1H); 8.21 (d, 1H).

### Intermediat 115:

### 1-[(4-Methoxy-3-nitrophenyl)sulfonyl]-4-methylpiperazin

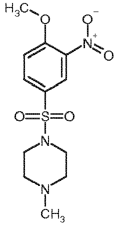

Eine Lösung von 28 g 1-[(4-Chlor-3-nitrophenyl)sulfonyl]-4-methylpiperazin (Intermediat 114) in 250 ml Methanol wurde bei 25 °C mit 84 ml Natriummethoxid (30% in Methanol) versetzt und 2 Stunden gerührt. Der Ansatz wurde mit Eis versetzt, nach dem Auftauen bei RT filtriert, mit Wasser gewaschen und getrocknet. Man erhielt 26 g 1-[(4-Methoxy-3-nitrophenyl)sulfonyl]-4-methylpiperazin als gelben Feststoff.
¹H NMR (400 MHz, 25°C, CDCl₃) δ = 2.27 (s, 3H); 2.46-2.50 (bs, 4H); 3.05 (bs, 4H); 4.04 (s, 3H); 7.21 (d, 1H); 7.91 (dd, 1H); 8.21 (d, 1H).

### Intermediat 116:

### 2-Methoxy-5-[(4-methylpiperazin-1-yl)sulfonyl]anilin

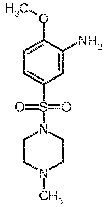

Eine Suspension von 12.0 g 1-[(4-Methoxy-3-nitrophenyl)sulfonyl]-4-methylpiperazin (Intermediat 115) in 100 ml Ethanol und 24 ml wässriger gesättigter Ammoniumchlorid Lösung wurde mit 10.6 g Eisenpulver versetzt und 2 Stunden unter Rückfluss gerührt. Dann wurden weitere 12.6 ml wässrige gesättigte Ammoniumchlorid Lösung zugegeben und weitere 4 Stunden unter Rückfluss gerührt. Die Reaktionslösung wurde durch Kieslgur filtriert und mit Ethanol und Ethylacetat nachgewaschen. Die Lösung wurde im vakuum eingeengt und der Rückstand mit Wasser suspendiert und dreimal mit Ethylacetat extrahiert. Die vereinten organsichen Phasen wurden über Magnesiumsulfat getrocknet und im Vakuum vollständig eingeengt. Deer Rückstand wurde mit Methanol ausgerührt. Man erhielt 4.7 g 2-Methoxy-5-[(4-methylpiperazin-1-yl)sulfonyl]anilin als gelben Feststoff.
¹H NMR (400 MHz, 25°C, DMSO-d6) δ = 2.14 (s, 3H); 2.36 (bs, 4H); 2.81 (bs, 4H); 3.84 (s, 3H); 5.25 (bs, 2H); 6.87 (dd, 1H); 6.95-6.98 (m, 2H).

### Intermediat 117:

### N-{4-[(1-Methylpiperidin-4-yl)amino]cyclohexyl}acetamid, cis-/trans- Isomerenmischung

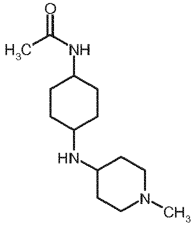

Eine Lösung von 772 mg 4-Amino-1-methylpiperin (CAS 41838-46-4) und 1 g *N*-(4-Oxocyclohexyl)acetamid (CAS 27514-08-5) in 10 ml 1,2-Dichlorethan und 0.37 ml Essigsäure wurde bei RT portionsweise mit 2.05 g Natriumtriacetoxyborhydrid versetzt. Es wurde 14 Stunden gerührt und danach auf 1 N Natronlauge gegeben. Der Ansatz wurde zweimal mit Ethylacetat extrahiert, die vereinten organischen Phasen mit gesättigter Natriumchlorid Lösung gewaschen, über Natriumsulfat getrocknet und das Lösungsmittel vollständig im Vakuum entfernt. Der Rückstand wurde erneut in Ethylacetat aufgenommen und der gebildetet Niederschlag wiurde abgesaugt. Man erhielt 3.8 g *N*-{4-[(1-Methylpiperidin-4-yl)amino]cyclohexyl}acetamid *cis-*/*trans-*Isomerengemisch als Rohprodukt, welches ohne weitere Reinigung in die folgende Reaktion eingesetzt wurde.

UPLC-MS: Instrument: Waters Acquity UPLC-MS SQD; Säule: Acquity UPLC BEH C18 1.7 50x2.1mm; Eluent A: Wasser + 0.2% Vol. Ammoniak (32%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm.
Rt = 0.66 min (M⁺+1 = 254)

### Intermediat 118:

### N-(1-Methylpiperidin-4-yl)cyclohexan-1,4-diamin Hydrochlorid, cis-/trans-Isomerenmischung

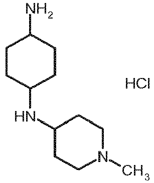

3.8 g Intermediat 117 in 50 ml Salzsäure (24%ig in Wasser) wurden 16 Stunden bei 115°C gerührt. Der Ansatz wurde danach vollständig im Vakuum eingeengt und der Rückstand in 2-Propanol aufgenommen. Diese Lösung wurde erhitzt und langsam wieder abgekühlt. Der entstandene Niederschlag wurde abgesaugt. Man erhielt 1.9 g *N*-(1-Methylpiperidin-4-yl)cyclohexan-1,4-diamin Hydrochlorid, *cis-*/*trans*-Isomerenmischung als Rohprodukt, welches ohne weitere Reinigung in die folgende Reaktion eingesetzt wurde.

UPLC-MS: Instrument: Waters Acquity UPLC-MS SQD; Säule: Acquity UPLC BEH C18 1.7 50x2.1mm; Eluent A: Wasser + 0.2% Vol. Ammoniak (32%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm.
Rt = 0.66 und 0.77 min (M⁺+1 = 212): cis und trans Isomer

### Intermediat 119:

### tert-Butyl-[4-(4,4-difluorpiperidin-1-yl)cyclohexyl]carbamat, cis-/trans- Isomerenmischung

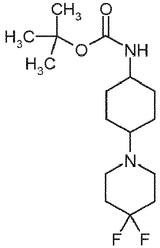

Eine Lösung von 2.26 g 4,4-Difluorpiperidin Hydrochlorid (CAS 144230-52-4) und 2 g *tert*-Butyl-(4-oxocyclohexyl)carbamat (CAS 179321-49-4) in 50 ml Dichlormethan und 1.77 ml Triethylamin wurde bei RT portionsweise mit 4.48 g Natriumtriacetoxyborhydrid und wenig Essigsäure versetzt.

Es wurde 14 Stunden gerührt und danach mit 50 ml Methanol versetzt. Es wurde 1 Stunde gerührt und mit Dichlormethan verdünnt. Der Ansatz wurde mit 1N Natronlauge, Wasser und gesättigter Natriumchlorid Lösung gewaschen, über Natriumsulfat getrocknet und das Lösungsmittel vollständig im Vakuum entfernt. Man erhielt 3.1 g *tert*-Butyl-[4-(4,4-difluorpiperidin-1-yl)cyclohexyl]carbamat als *cis-*/*trans-* Isomerengemisch.

UPLC-MS: Instrument: Waters Acquity UPLC-MS SQD; Säule: Acquity UPLC BEH C18 1.7 50x2.1mm; Eluent A: Wasser + 0.1% Vol. Ameisensäure (99%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm.
Rt = 0.68 min (M⁺+1 = 319)

### Intermediat 120:

### 4-(4,4-Difluorpiperidin-1-yl)cyclohexanamin, cis-/trans-Isomerenmischung

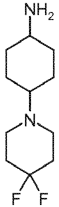

3.1 g Intermediat 119 in 90 ml Dichlormethan wurden mit 11.3 ml Trifluoressigsäure versetzt und 5 Stunden bei Siedehitze gerührt. Der Ansatz wurde danach vollständig im Vakuum eingeengt und der Rückstand in Ethylacetat aufgenommen. Es wurde mit gesättigter Natriumhydrogencarbonat Lösung extrahiert. Die wässrige Phase wurde dann dreimal mit Dichlormethan extrahiert. Die vereinten Dichlormethan-Phasen wurden über Natriumsulfat getrocknet und das Lösungsmittel vollständig im Vakuum entfernt. Man erhielt 920 mg 4-(4,4-Difluorpiperidin-1-yl)cyclohexanamin als *cis-*/*trans-* Isomerengemisch.

UPLC-MS: Instrument: Waters Acquity UPLC-MS SQD; Säule: Acquity UPLC BEH C18 1.7 50x2.1mm; Eluent A: Wasser + 0.2% Vol. Ammoniak (32%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm.
Rt = 0.91+0.87 min (M⁺+1 = 219): cis und trans Isomer

### Intermediat 121:

### N-[4-(4-Cyclopropylpiperazin-1-yl)cyclohexyl]acetamid, cis-/trans- Isomerenmischung

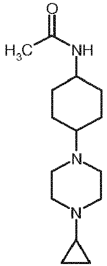

Eine Lösung von 1.25 g 1-Cyclopropylpiperazin Dihydrochlorid (CAS 1392.56-79-4) und 928 mg *N*-(4-Oxocyclohexyl)acetamid (CAS 27514-08-5) in 9.3 ml 1,2-Dichlorethan und 0.34 ml Essigsäure wurde bei RT portionsweise mit 1.9 g Natriumtriacetoxyborhydrid versetzt. Es wurde 14 Stunden gerührt und danach auf gesättigte Natriumhydrogencarbonat Lösung gegeben. Der Ansatz wurde zweimal mit Ethylacetat extrahiert, die vereinten organischen Phasen mit gesättigter Natriumchlorid Lösung gewaschen, über Natriumsulfat getrocknet und das Lösungsmittel vollständig im Vakuum entfernt.. Man erhielt 600 g *N*-[4-(4-Cyclopropylpiperazin-1-yl)cyclohexyl]acetamid, *cis-*/*trans-* Isomerenmischung als Rohprodukt, welches ohne weitere Reinigung in die folgende Reaktion eingesetzt wurde.

UPLC-MS: Instrument: Waters Acquity UPLC-MS SQD; Säule: Acquity UPLC BEH C18 1.7 50x2.1mm; Eluent A: Wasser + 0.2% Vol. Ammoniak (32%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm.
Rt = 0.71 und 0.74 min (M⁺+1 = 266)

### Intermediat 122:

### 4-(4-Cyclopropylpiperazin-1-yl)cyclohexanamin Hydrochlorid, cis-/trans-Isomerenmischung

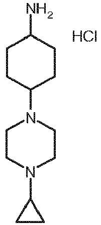

600 mg Intermediat 121 in 10 ml Salzsäure (24%ig in Wasser) wurden 13 Stunden bei 115°C gerührt. Der Ansatz wurde danach vollständig im Vakuum eingeengt und der Rückstand in 2-Propanol aufgenommen. Diese Lösung wurde erhitzt und langsam wieder abgekühlt. Der entstandene Niederschlag wurde abgesaugt. Man erhielt 170 mg 4-(4-Cyclopropylpiperazin-1-yl)cyclohexanamin Hydrochlorid, *cis-*/*trans*-Isomerenmischung als Rohprodukt, welches ohne weitere Reinigung in die folgende Reaktion eingesetzt wurde.

UPLC-MS: Instrument: Waters Acquity UPLC-MS SQD; Säule: Acquity UPLC BEH C18 1.7 50x2.1mm; Eluent A: Wasser + 0.2% Vol. Ammoniak (32%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm.
Rt = 0.78 min (M⁺+1 = 224): cis und trans Isomer

### Intermediat 123:

### N-(1-Methylpiperidin-4-yl)-3-nitrobenzamid

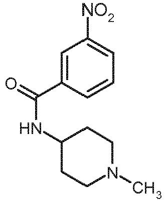

Eine Lösung von 16.25 g 3-Nitrobenzoylchlorid (CAS 121-90-4) in 175 ml Pyridin wurde bei RT mit 10 g 4-Amino-1-methylpiperin (CAS 41838-46-4) versetzt. Es wurde 1 Stunde gerührt, dann auf Eiswasser gegossen und 14 Stunden bei RT gerührt. Die Reaktionslösung wurde mit Ethylacetat extrahiert, die organsiche Phase mit 2 M Natronlauge und Wasser gewaschen, über Natriumsulfat getrocknet und im Vakuum eingeengt. Der Rückstand wurde erneut in Ethylacetat aufgenommen und ausgerührt. Der Rückstand wurde abgesaugt. Man erhielt 19.5 g *N*-(1-Methylpiperidin-4-yl)-3-nitrobenzamid.

UPLC-MS: Instrument: Waters Acquity UPLC-MS SQD; Säule: Acquity UPLC BEH C18 1.7 50x2.1mm; Eluent A: Wasser + 0.2% Vol. Ammoniak (32%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm.
Rt = 0.84 min (M⁺+1 = 264)

### Intermediat 124:

### 3-Amino-N-(1-methylpiperidin-4-yl)benzamid

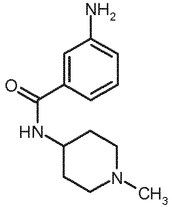

Eine Suspension von 19.5 g Intermediat 123 und 1.85 g Palladium (10% auf Aktivkohle) in 740 ml Ethanol wurde unter einer Wasserstoffatmosphäre bei RT für 6 Stunden gerührt. Es wurde durch Kieselgur abfiltriert und die Lösung im Vakuum vollständig eingeengt. Man erhielt 15.5 g 3-Amino-*N-*(1-methylpiperidin-4-yl)benzamid.
¹H NMR (400 MHz, 25°C, DMSO-d6) δ = 1.55 (dq, 2H); 1.71 (bd, 2H); 1.91 (dt, 2H); 2.15 (s, 3); 2.74 (bd, 2H); 3.61-3.74 (m, 1H); 5.18 (bs, 2H); 6.66 (ddd, 1H); 6.93 (bd, 1H); 7.00 (t, 1H); 7.05 (t, 1H); 7.97 (d, 1H).

Zur Herstellung der nachstehend genannten Ausführungsbeispiele wurden weiterhin die in der nachfolgenden Tabelle 1 gezeigten Amine verwendet, die entweder kommerziell verfügbar sind oder gegebenenfalls nach oder in Analogie zu den zitierten Vorschriften herstellen lassen.

**Tabelle 1:**

| Amine | Name | CAS No | Struktur | Herstellung beschrieben: |
|---|---|---|---|---|
| **1** | 3-(Pyrrolidin-1-ylsulfonyl)anilin | 91619-38-4 | 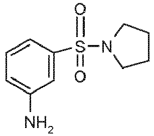 | |
| **2** | 3-Amino-*N*,*N-*dimethylbenzolsulf onamid | 6274-18-6 | 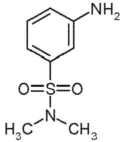 | |
| **3** | *N*,*N-*Dimethyl-3-(methylamino)benz olsulfonamid | 86317-09-1 | 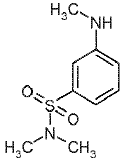 | |
| **4** | 2,3-Dihydro-1,2-benzothiazol-6-amin-1,1-dioxid | 916438-46-5 | 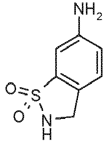 | |
| **5** | 3-Aminobenzolsulfo namid | 98-18-0 | 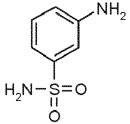 | |
| **6** | 3-(Morpholin-4-ylsulfonyl)anilin | 22184-97-0 | 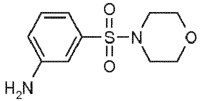 | |
| **7** | 3-Amino-*N-*ethylbenzolsulfona mid | 56445-08-0 | 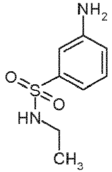 | |
| **8** | 3-Amino-*N-*cyclopropylbenzols ulfonamid | 459434-39-0 | 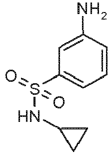 | |
| **9** | 3-Amino-*N*,*N-*diethylbenzolsulfo namid | 10372-41-5 | 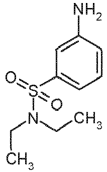 | |
| **10** | 3-Amino-*N* methylbenzolsulfo namid | 459434-40-3 | 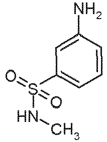 | |
| **11** | 3-Amino-*N-*isopropylbenzolsul fonamid | 118837-66-4 | 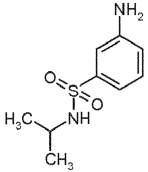 | |
| **12** | 5-Amino-2-methylbenzolsulfo namid | 6973-09-7 | 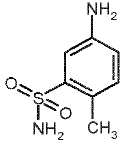 | |
| **13** | *trans*-4-[4-(Cyclopropylmethy 1)piperazin-1-yl]cyclohexanamin | 876461-31-3 | 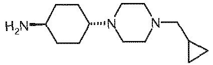 | analog WO2012049153 |
| **14** | 4-Amino-1-methylpiperidin | 41838-46-4 | 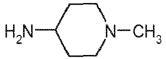 | |
| **15** | 2-(4-Methylpiperazin-1-yl)ethanamin | 934-98-5 | 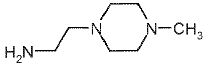 | |
| **16** | 1-Methylpiperazin | 109-01-3 | 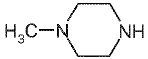 | |
| **17** | 1-Isopropylpiperazin | 4318-42-7 | 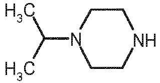 | |
| **18** | 1-Cyclopropylpipera zin | 20327-23-5 | 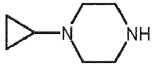 | |
| **19** | *N*,*N-*Dimethylethan-1,2-diamin | 108-00-9 | 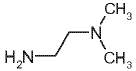 | |
| **20** | 1-Methylazetidin-3-amin Dihydrochloride | 1139634-75-5 | 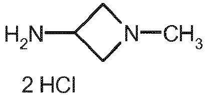 | |
| **21** | 3-Amino-*N*,*N-*dimethylbenzamid | 33322-60-0 | 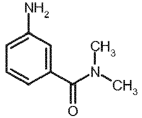 | |
| **22** | 1-(Cyclopropylmethy 1)piperazin | 57184-25-5 | 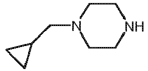 | |

Intermediate der allgemeinen Formeln (IX) und (XVI), die bevorzugt zur Herstellung der Verbindungen der allgemeinen Formel (I) zur Anwendung kommen, sind zum Beispiel:
3-{[(3*R*)-4-Cyclopentyl-1,3-dimethyl-2-oxo-1,2,3,4-tetrahydropyrido[2,3-*b*]pyrazin-6-yl]-amino}benzoesäuremethylester;
3-{[(3*R*)-1,3-Dimethyl-2-oxo-4-(tetrahydro-2*H*-pyran-4-yl)-1,2,3,4-tetrahydropyrido[2,3-*b*]-pyrazin-6-yl]amino}benzoesäuremethylester;
3-{[(3*R*)-1,3-Dimethyl-2-oxo-4-(tetrahydro-2*H*-pyran-4-yl)-1,2,3,4-tetrahydropyrido[2,3-*b*]-pyrazin-6-yl]amino}-4-methoxybenzoesäuremethylester;
3-{[4-(2,6-Difluorbenzyl)-1,3-dimethyl-2-oxo-1,2,3,4-tetrahydrochinoxalin-6-yl]amino}benzoesäureethylester;
3-{[(3*R*)-4-Cyclohexyl-1,3-dimethyl-2-oxo-1,2,3,4-tetrahydropyrido[2,3-b]pyrazin-6-yl]amino}benzoesäuremethylester;
3-{[(3*R*)-1,3-Dimethyl-2-oxo-4-(propan-2-yl)-1,2,3,4-tetrahydropyrido[2,3-b]pyrazin-6-yl]amino}benzoesäuremethylester;
3-{[(3*R*)-4-Benzyl-1,3-dimethyl-2-oxo-1,2,3,4-tetrahydropyrido[2,3-b]pyrazin-6-yl]amino}benzoesäuremethylester;
3-{[4-(2-Methoxyethyl)-1,3-dimethyl-2-oxo-1,2,3,4-tetrahydropyrido[2,3-b]pyrazin-6-yl]amino }benzoesäuremethylester;
*tert*-Butyl 4-[(3*R*)-6-{[3-(ethoxycarbonyl)phenyl]amino}-1,3-dimethyl-2-oxo-2,3-dihydropyrido[2,3-b]pyrazin-4(1H)-yl]piperidin-1-carboxylat;
3-{[4-(4-Fluorphenyl)-1,3-dimethyl-2-oxo-1,2,3,4-tetrahydropyrido[2,3-b]pyrazin-6-yl]amino}benzoesäuremethylester;
5-{[(3*R*)-1,3-Dimethyl-2-oxo-4-(tetrahydro-2H-pyran-4-yl)-1,2,3,4-tetrahydropyrido[2,3-b]pyrazin-6-yl]amino}-2-methoxybenzoesäuremethylester;
3-{[(3*R*)-1,3-Dimethyl-2-oxo-4-(tetrahydro-2H-pyran-4-yl)-1,2,3,4-tetrahydropyrido[2,3-b]pyrazin-6-yl]amino}-2-methoxybenzoesäuremethylester
   und
3-{[(3*R*)-1,3-Dimethyl-2-oxo-4-(tetrahydro-2H-pyran-4-yl)-1,2,3,4-tetrahydrochinoxalin-6-yl]amino}benzoesäuremethylester.

Diese Intermediate der allgemeinen Formeln (IX) und (XVI) sind ebenfalls Gegenstand der vorliegenden Erfindung.

Intermediate der allgemeinen Formeln (X) und (XVII), die bevorzugt zur Herstellung der Verbindungen der allgemeinen Formel (I) zur Anwendung kommen, sind zum Beispiel:
3-{[(3*R*)-4-Cyclopentyl-1,3-dimethyl-2-oxo-1,2,3,4-tetrahydropyrido[2,3-*b*]pyrazin-6-yl]-amino}benzoesäure;
3-{[(3*R*)-1,3-Dimethyl-2-oxo-4-(tetrahydro-2*H*-pyran-4-yl)-1,2,3,4-tetrahydropyrido[2,3-*b*]-pyrazin-6-yl]amino}benzoesäure;
3-{[(3*R*)-1,3-Dimethyl-2-oxo-4-(tetrahydro-2*H*-pyran-4-yl)-1,2,3,4-tetrahydropyrido[2,3-*b*]-pyrazin-6-yl]amino}-4-methoxybenzoesäure;
3-{[4-(2,6-Difluorbenzyl)-1,3-dimethyl-2-oxo-1,2,3,4-tetrahydrochinoxalin-6-yl]amino}-benzoesäure;
3-1[(3*R*)-4-Cyclohexyl-1,3-dimethyl-2-oxo-1,2,3,4-tetrahydropyrido[2,3-b]pyraz-in-6-yl]amino}benzoesäure;
3-1[(3*R*)-1,3-Dimethyl-2-oxo-4-(propan-2-yl)-1,2,3,4-tetrahydropyrido[2,3-b]pyrazin-6-yl]amino}benzoesäure;
3-{[(3*R*)-4-Benzyl-1,3-dimethyl-2-oxo-1,2,3,4-tetrahydropyrido[2,3-b]pyrazin-6-yl]amino}benzoesäure ;
3-{[4-(2-Methoxyethyl)-1,3-dimethyl-2-oxo-1,2,3,4-tetrahydropyrido[2,3-b]pyrazin-6-yl]amino}benzoesäure;
3-({(3*R*)-4-[1-(*tert*-Butoxycarbonyl)piperidin-4-yl]-1,3-dimethyl-2-oxo-1,2,3,4-tetrahydropyrido[2,3-b]pyrazin-6-yl}amino)benzoesäure;
3-{[4-(4-Fluorphenyl)-1,3-dimethyl-2-oxo-1,2,3,4-tetrahydropyrido[2,3-b]pyrazin-6-yl]amino}benzoesäure;
5-{[(3*R*)-1,3-Dimethyl-2-oxo-4-(tetrahydro-2H-pyran-4-yl)-1,2,3,4-tetrahydropyrido[2,3-b]pyrazin-6-yl]amino}-2-methoxybenzoesäure;
3-{[(3*R*)-1,3 -Dimethyl-2-oxo-4-(tetrahydro-2H-pyran-4-yl)-1,2,3,4-tetrahydropyrido[2,3-b]pyrazin-6-yl]amino}-2-methoxybenzoesäure
   und
3-{[(3*R*)-1,3-Dimethyl-2-oxo-4-(tetrahydro-2H-pyran-4-yl)-1,2,3,4-tetrahydrochinoxalin-6-yl]amino}benzoesäure.

Diese Intermediate der allgemeinen Formeln (X) und (XVII) sind ebenfalls Gegenstand der vorliegenden Erfindung.

### Herstellung der erfindungsgemäßen Verbindungen

### Beispiel 1, Allgemeine Synthesevorschrift A

### (3R)-4-Cyclopentyl-1,3-dimethyl-6-({3-[(4-methylpiperazin-1-yl)carbonyl]phenyl}amino)-3,4-dihydropyrido[2,3-b]pyrazin-2(1H)-on

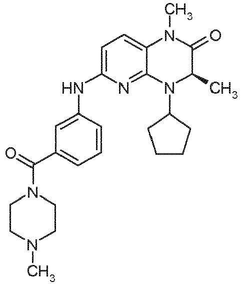

Eine Lösung von 150 mg Intermediat 7, 79 mg *N*-Methylpiperazin (Amin 16, Tabelle 1), 0.22 ml Triethylamin und 224 mg HATU in 16 ml DMF wurde 16 Stunden bei RT gerührt. Der Ansatz wurde auf halbgesättigte Kochsalzlösung gegeben und dreimal mit Etylacetat extrahiert. Die vereinten organischen Phasen wurden mit gesättigter Kochsalzlösung gewaschen, über Natriumsulfat getrocknet und das Lösungsmittel im Vakuum entfernt. Der Rückstand wurde durch RP-HPLC (Waters Autopurificationsystem SQD; Säule: Waters XBrigde C18 5µm 100x30mm; Eluent A: Wasser + 0.1% Vol. Ameisensäure (99%), Eluent B: Acetonitril; Gradient: 0-8.0 min 1-100% B, 8.0-10.0 min 100% B; Fluss 50.0 ml/min; Temperatur: RT; Injektion: 2500 µl; DAD scan: 210-400 nm) gereinigt. Man erhielt 70 mg (3*R*)-4-Cyclopentyl-1,3-dimethyl-6-({3-[(4-methylpiperazin-1-yl)carbonyl]phenyl}amino)-3,4-dihydropyrido[2,3-*b*]pyrazin-2(1*H*)-on.
¹H-NMR (300 MHz, DMSO-d6, ausgewählte Signale): δ = 1.06 (d, 3H);1.48-1.77 (m, 6H); 1.89-2.02 (m, 2H); 2.19 (s, 3H); 2.22-2.42 (m, 4H); 3.20 (s, 3H); 4.29 (q, 1H); 4.42 (qi, 1H); 6.25 (d, 1H); 6.76 (d, 1H); 7.21-7.29 (m, 2H); 7.44 (d, 1H); 7.94 (s, 1H); 8.93 (s, 1H).

### Beispiel 2

### 3-{[(3R)-4-Cyclopentyl-1,3-dimethyl-2-oxo-1,2,3,4-tetrahydropyrido[2,3-b]pyrazin-6-yl]-amino}-N-(1-methylpiperidin-4-yl)benzamid

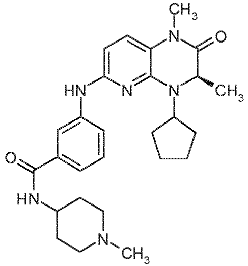

In Analogie zu Beispiel 1 wurde 3-{[(3*R*)-4-Cyclopentyl-1,3-dimethyl-2-oxo-1,2,3,4-tetrahydro-pyrido[2,3-*b*]pyrazin-6-yl]amino}-*N*-(1-methylpiperidin-4-yl)benzamid ausgehend von 150 mg Intermediat 7, 90 mg 1-Methylpiperidin-4-amin (Amin 14, Tabelle 1), 0.22 ml Triethylamin und 224 mg HATU in 16 ml DMF hergestellt. Nach Reinigung an RP-HPLC (Waters Autopurificationsystem SQD; Säule: Waters XBrigde C18 5µm 100x30mm; Eluent A: Wasser + 0.1% Vol. Ameisensäure (99%), Eluent B: Acetonitril; Gradient: 0-8.0 min 1-100% B, 8.0-10.0 min 100% B; Fluss 50.0 ml/min; Temperatur: RT; Injektion: 2500 µl; DAD scan: 210-400 nm) erhielt man 100 mg 3-{[(3*R*)-4-Cyclopentyl-1,3-dimethyl-2-oxo-1,2,3,4-tetrahydropyrido[2,3-*b*]pyrazin-6-yl]amino}-*N*-(1-methylpiperidin-4-yl)benzamid.
¹H-NMR (400 MHz, DMSO-d6): δ = 1.07 (d, 3H); 1.47-1.72 (m, 8H); 1.72-1.85 (m, 2H); 1.88-2.05 (m, 2H); 2.13 (t, 2H); 2.25 (s, 3H); 2.86 (d, 2H); 3.20 (s, 3H); 3.67-3.83 (m, 1H); 4.18 (q, 1H); 4.34-4.48 (m, 1H); 6.24 (m, 1H); 7.20-7.32 (m, 3H); 7.62-7.73 (m, 1H); 8.06 (s, 1H); 8.13 (d, 1H); 8.87 (s, 1H).

### Beispiel 3

### 3-{[(3R)]-4-Cyclopentyl-1,3-dimethyl-2-oxo-1,2,3,4-tetrahydropyrido[2.3-b]pyrazin-6-yl-amino}-N-(1-methylazetidin-3-yl)benzamid

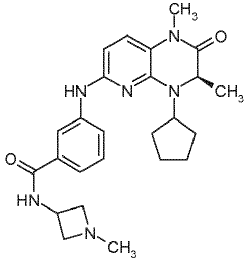

In Analogie zu Beispiel 1 wurde 3-{[(3*R*)-4-Cyclopentyl-1,3-dimethyl-2-oxo-1,2,3,4-tetrahydro-pyrido[2,3-*b*]pyrazin-6-yl]amino}-*N-*(1-methylazetidin-3-yl)benzamid ausgehend von 150 mg Intermediat 7, 125 mg 1-Methylazetidin-3-amin dihydrochlorid (Amin 20, Tabelle 1), 0.22 ml Triethylamin und 224 mg HATU in 16 ml DMF hergestellt. Nach Reinigung an RP-HPLC (Waters Autopurificationsystem SQD; Säule: Waters XBrigde C18 5µm 100x30mm; Eluent A: Wasser + 0.1% Vol. Ameisensäure (99%), Eluent B: Acetonitril; Gradient: 0-8.0 min 1-100% B, 8.0-10.0 min 100% B; Fluss 50.0 ml/min; Temperatur: RT; Injektion: 2500 µl; DAD scan: 210-400 nm) erhielt man 40 mg 3-{[(3*R*)-4-Cyclopentyl-1,3-dimethyl-2-oxo-1,2,3,4-tetrahydropyrido[2,3-*b*]pyrazin-6-yl]amino}-*N-*(1-methylazetidin-3-yl)benzamid.
¹H-NMR (400 MHz, DMSO-d6): δ = 1.07 (d, 3H); 1.47-1.73 (m, 6H); 1.88-2.92 (m, 2H); 2.29 (s, 3H); 3.04 (t, 2H); 3.61 (t, 2H); 4.18 (q, 1H); 4.36-4.51 (m, 2H); 6.24 (d, 1H); 7.20-7.33 (m, 3H); 7.57-7.66 (m, 1H); 8.16 (s, 1H); 8.65 (d, 1H); 8.89 (s, 1H).

### Beispiel 4

### 3-{[(3R)-4-Cyclopentyl-1,3-dimethyl-2-oxo-1,2,3,4-tetrahydropyrido[2,3-b]pyrazin-6-yl]-amino}-N-[2-(dimethylamino)ethyl]benzamid

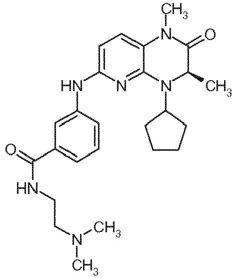

In Analogie zu Beispiel 1 wurde 3-{[(3*R*)-4-Cyclopentyl-1,3-dimethyl-2-oxo-1,2,3,4-tetrahydro-pyrido[2,3-*b*]pyrazin-6-yl]amino}-*N*-[2-(dimethylamino)ethyl]benzamid ausgehend von 150 mg Intermediat 7, 69 mg *N*,*N-*Dimethylethan-1,2-diamin (Amin 19, Tabelle 1), 0.22 ml Triethylamin und 224 mg HATU in 16 ml DMF hergestellt. Nach Reinigung an RP-HPLC (Waters Autopurificationsystem SQD; Säule: Waters XBrigde C18 5µm 100x30mm; Eluent A: Wasser + 0.1% Vol. Ameisensäure (99%), Eluent B: Acetonitril; Gradient: 0-8.0 min 1-100% B, 8.0-10.0 min 100% B; Fluss 50.0 ml/min; Temperatur: RT; Injektion: 2500 µl; DAD scan: 210-400 nm) erhielt man 40 mg 3-{[(3*R*)-4-Cyclopentyl-1,3-dimethyl-2-oxo-1,2,3,4-tetrahydropyrido[2,3-*b*]pyrazin-6-yl]amino}-*N*-[2-(dimethylamino)ethyl]benzamid.
¹H-NMR (400 MHz, DMSO-d6, ausgewählte Signale): δ = 1.07 (d, 3H); 1.47-1.73 (m, 6H); 1.89-2.06 (m, 2H); 2.23 (s, 6H); 3.2 (s, 3H); 3.35 (q, 2H); 4.18 (q, 1H); 4.46 (qi, 1H); 6.25 (d, 1H); 7.20-7.31 (m, 3H); 7.64 (d, 1H); 8.14 (s, 1H); 8.24 (t, 1H); 8.88 (s, 1H).

### Beispiel 5

### 3-{[(3R)-4-Cyclopentyl-1,3-dimethyl-2-oxo-1,2,3,4-tetrahydropyrido[2,3-b]pyrazin-6-yl]-amino}-N-{trans-4-[4-(cyclopropylmethyl)piperazin-1-yl]cyclohexyl}benzamid

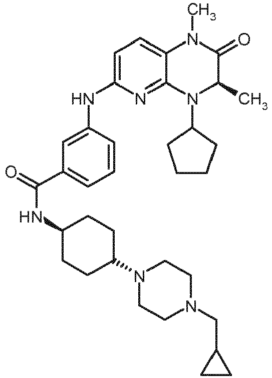

In Analogie zu Beispiel 1 wurde 3-{[(3*R*)-4-Cyclopentyl-1,3-dimethyl-2-oxo-1,2,3,4-tetrahydro-pyrido[2,3-*b*]pyrazin-6-yl]amino}-*N*-{*trans*-4-[4-(cyclopropylmethyl)piperazin-1-yl]cyclohexyl}benzamid ausgehend von 150 mg Intermediat 7, 187 mg *trans*-4-[4-(Cyclopropylmethyl) piperazin-1-yl]cyclohexanamin Amin 13, Tabelle 1), 0.22 ml Triethylamin und 224 mg HATU in 16 ml DMF hergestellt. Nach Reinigung an RP-HPLC (Waters Autopurificationsystem SQD; Säule: Waters XBrigde C18 5µm 100x30mm; Eluent A: Wasser + 0.1% Vol. Ameisensäure (99%), Eluent B: Acetonitril; Gradient: 0-8.0 min 1-100% B, 8.0-10.0 min 100% B; Fluss 50.0 ml/min; Temperatur: RT; Injektion: 2500 µl; DAD scan: 210-400 nm) erhielt man 95 mg 3-{[(3*R*)-4-Cyclopentyl-1,3-dimethyl-2-oxo-1,2,3,4-tetrahydropyrido[2,3-*b*]pyrazin-6-yl]amino}-*N*-{*trans*-4-[4-(cyclopropylmethyl)piperazin-1-yl]cyclohexyl}benzamid.
¹H-NMR (400 MHz, CDCl₃): δ = 0.02-0.10 (m, 2H); 0.40-0.49 (m, 2H); 0.73-0.88 (m, 1H); 1.06 (d, 3H); 1.19-1.43 (m, 4H); 1.46-1.73 (m, 6H); 1.76-2.04 (m, 6H); 2.12-2.30 (d+m, 3H); 4.18 (q, 1H); 4.32-4.48 (m, 1H); 6.24 (d, 1H); 7.18-7.30 (m, 3H); 7.67 (d, 1H); 8.04 (s, 1H); 8.08 (d, 1H); 8.89 (s, 1H).

### Beispiel 6

### 3-{[(3R)-1,3-Dimethyl-2-oxo-4-(tetrahydro-2H-pyran-4-yl)-1,2,3,4-tetrahydropyrido[2,3-b]-pyrazin-6-yl]amino}-N,N-dimethylbenzolsulfonamid

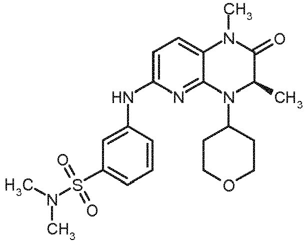

Eine Suspension von 150 mg Intermediat 10, 192 mg 3-Amino-*N*,*N-*dimethylbenzolsulfonamid (Amin 2, Tabelle 1), 21 mg Palladiumacetat, 784 mg Caesiumcarbonat und 60 mg (+)-BINAP in 10.8 ml Toluol wurde 5 Stunden unter Argon bei 120°C Badtemperatur gerührt. Die Reaktionslösung wurde abfiltriert, der Rückstand mit Ethylacetat nachgewaschen und die vereinten organischen Phase vollständig im Vakuum eingeengt. Der Rückstand wurde durch RP-HPLC (Waters Autopurificationsystem SQD; Säule: Waters XBrigde C18 5µm 100x30mm; Eluent A: Wasser + 0.1% Vol. Ameisensäure (99%), Eluent B: Acetonitril; Gradient: 0-8.0 min 1-100% B, 8.0-10.0 min 100% B; Fluss 50.0 ml/min; Temperatur: RT; Injektion: 2500 µl; DAD scan: 210-400 nm) gereinigt. Man erhielt 110 mg 3-{[(3*R*)-1,3-Dimethyl-2-oxo-4-(tetrahydro-2*H*-pyran-4-yl)-1,2,3,4-tetrahydropyrido[2,3-*b*]pyrazin-6-yl]amino}-*N*,*N*-dimethylbenzolsulfonamid.
¹H-NMR (400 MHz, DMSO-d6): δ = 1.08 (d, 3H); 1.59 (d, 1H); 1.77 (dq, 1H); 1.83-1.98 (m, 2H); 2.61 (s, 6H); 3.21 (s, 3H); 3.51 (t, 2H); 3.94 (t, 2H); 4.24 (q, 1H); 4.41-4.52 (m, 1H); 6.28 (d, 1H); 7.15 (d, 1H); 7.30 (d, 1H); 7.48 (t, 1H); 7.81 (t, 1H); 8.10 d, 1H); 9.21 (s, 1H).

### Beispiel 7

### 3-{[(3R)-1,3-Dimethyl-2-oxo-4-(tetrahydro-2H-pyran-4-vl)-1,2,3,4-tetrahydrochinoxalin-6-yl]amino}-N,N-dimethylbenzolsulfonamid

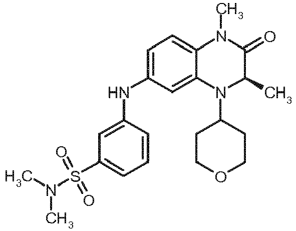

In Analogie zur Herstellung von Beispiel 6 wurde 3-{[(3*R*)-1,3-Dimethyl-2-oxo-4-(tetrahydro-2*H-*pyran-4-yl)-1,2,3,4-tetrahydrochinoxalin-6-yl]amino}-*N*,*N*-dimethylbenzolsulfonamid (Amin 2, Tabelle 1) ausgehend von 47 mg Intermediat 14 und 55 mg 3-Amino-*N*,*N-*dimethylbenzolsulfonamid hergestellt. Man erhielt 37 mg der Titelverbindung.
¹H-NMR (400 MHz, CDCl₃): δ = 1.13 (d, 3H); 1.70 (d, 1H); 1.75-1.96 (m, 3H); 2.74 (s, 6H); 3.38 (s, 3H); 3.46 (ddt, 2H); 3.58 (tt, 1H); 4.05 (dt, 2H); 4.15 (q, 1H); 5.85 (s, 1H); 6.67-6.73 (m, 2H); 6.91 (d, 1H); 7.14 (dd, 1H); 7.23 (dd, 1H); 7.39 (t, 1H); 7.42 (t, 1H).

### Beispiel 8

### (3R)-1,3-Dimethyl-6-{[3-(morpholin-4-ylsulfonyl)phenyl]amino}-4-(tetrahydro-2H-pyran-4-yl)-3,4-dihydrochinoxalin-2(1H)-on

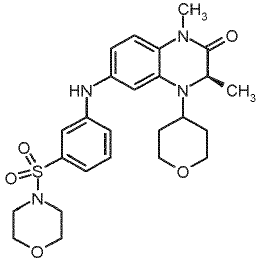

In Analogie zur Herstellung von Beispiel 6 wurde (3*R*)-1,3-Dimethyl-6-{[3-(morpholin-4-yl-sulfonyl)phenyl]amino}-4-(tetrahydro-2*H*-pyran-4-yl)-3,4-dihydrochinoxalin-2(1*H*)-on_ausgehend von 47 mg Intermediat 14 und 67 mg 3-(Morpholin-4-ylsulfonyl)anilin (Amin 6, Tabelle 1) hergestellt. Man erhielt 37 mg der Titelverbindung.
¹H-NMR (400 MHz, CDCl₃): δ = 1.14 (d, 3H); 1.67-1.75 (m, 1H); 1.76-1.97 (m, 3H); 3.00-3.10 (m, 4H); 3.38 (s, 3H); 3.46 (ddt, 2H); 3.58 (tt, 1H); 3.72-3.81 (m, 4H); 4.05 (dt, 2H); 4.15 (q, 1H); 5.85 (s, 1H); 6.67-6.74 (m, 2H); 6.92 (d, 1H); 7.15 (dd, 1H); 7.20 (d, 1H); 7.36-7.44 (m, 2H).

### Beispiel 9

### (3R)-1,3-Dimethyl-6-{[3-(morpholin-4-ylsulfonyl)phenyl]amino}-4-(tetrahydro-2H-pyran-4-yl)-3,4-dihydropyrido[2,3-b]pyrazin-2(1H)-on

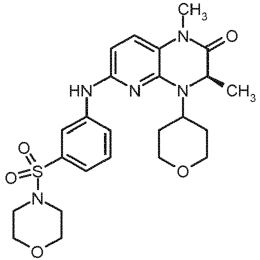

In Analogie zur Herstellung von Beispiel 6 wurde (3*R*)-1,3-Dimethyl-6-{[3-(morpholin-4-yl-sulfönyl)phenyl]amino}-4-(tetrahydro-2*H*-pyran-4-yl)-3,4-dihydropyrido[2,3-*b*]pyrazin-2(1*H*)-on ausgehend von 150 mg Intermediat 10 und 233 mg 3-(Morpholin-4-ylsulfonyl)anilin (Amin 6, Tabelle 1) hergestellt. Man erhielt 24 mg der Titelverbindung.
¹H-NMR (400 MHz, DMSO-d6): δ = 1.08 (t, 3H); 1.60 (bd, 1H); 1.71-1.83 (m, 1H); 1.83-1.98 (m, 2H); 2.81-2.91 (m, 4H); 3.21 (s, 3H); 3.46-3.57 (m, 2H); 3.60-3.67 (m, 4H); 3.89-3.99 (m, 2H); 4.24 (q, 1H); 4.46 (tt, 1H); 6.28 (d, 1H); 7.14 (d, 1H); 7.30 (d, 1H); 7.50 (t, 1H); 7.80 t, 1H); 8.11 (d, 1H); 9.24 (s, 1H).

### Beispiel 10

### 3-{[(3R)-4-(4-Methoxybenzyl)-1,3-dimethyl-2-oxo-1,2,3,4-tetrahydrochinoxalin-6-yl]amino}-N,N-dimethylbenzolsulfonamid

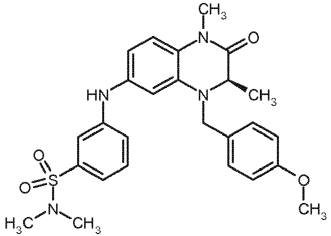

In Analogie zur Herstellung von Beispiel 6 wurde 3-{[(3*R*)-4-(4-Methoxybenzyl)-1,3-dimethyl-2-oxo-1,2,3,4-tetrahydrochinoxalin-6-yl]amino}-*N*,*N*-dimethylbenzolsulfonamid ausgehend von 50 mg Intermediat 16 und 53 mg 3-Amino-*N*,*N*-dimethylbenzolsulfonamid (Amin 2, Tabelle 1) hergestellt. Man erhielt 22 mg der Titelverbindung.
¹H-NMR (400 MHz, CDCl₃): δ = 1.15 (d, 3H); 2.73 (s, 6H); 3.41 (s, 3H); 3.82 (s, 3H); 4.01 (q, 1H); 4.09 (d, 1H); 4.43 (d, 1H); 5.95 (s, 1H); 6.48 (d, 1H); 6.64 (dd, 1H); 6.86-6.94 (m, 3H); 6.97 (dd, 1H); 7.19 (d, 1H); 7.22-7.28 (m, 3H); 7.32 (t, 1H).

### Beispiel 11

### (3R)-4-(4-Methoxybenzyl)-1,3-dimethyl-6-1{[3-(morpholin-4-ylsulfonyl)phenyl]amino}-3,4-dihydrochinoxalin-2(1H)-on

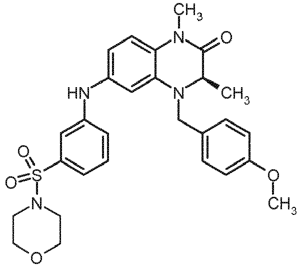

In Analogie zur Herstellung von Beispiel 6 wurde(3*R*)-4-(4-Methoxybenzyl)-1,3-dimethyl-6-{[3-(morpholin-4-ylsulfonyl)phenyl]amino}-3,4-dihydrochinoxalin-2(1*H*)-on ausgehend von 50 mg Intermediat 16 und 65 mg 3-(Morpholin-4-ylsulfonyl)anilin (Amin 6, Tabelle 1) hergestellt. Man erhielt 38 mg der Titelverbindung.
¹H-NMR (400 MHz, DMSO-d6): δ = 1.00 (d, 3H); 2.83-2.90 (m, 4H); 3.28 (s, 3H); 3.59-3.65 (m, 4H); 3.75 (s, 3H); 3.91 (q, 1H); 4.17 (d, 1H); 4.39 (d, 1H); 6.50 (d, 1H); 6.59 (dd, 1H); 6.92 (d, 2H); 6.98-7.03 (m, 3H); 7.23-7.28 (m, 3H); 7.30 (t, 1H); 8.44 (s, 1H).

### Beispiel 12

### (3R)-1,3-Dimethyl-6-({3-[(4-methylpiperazin-1-yl)sulfonyl]phenyl}amino)-4-(tetrahydro-2H-pyran-4-yl)-3,4-dihydropyrido[2,3-b]pyrazin-2(1H-on

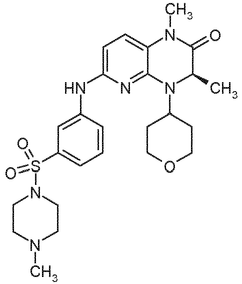

In Analogie zur Herstellung von Beispiel 6 wurde (3*R*)-1,3-Dimethyl-6-({3-[(4-methylpiperazin-1-yl)sulfonyl]phenyl}amino)-4-(tetrahydro-2*H-*pyran-4-yl)-3,4-dihydropyrido[2,3-*b*]pyrazin-2(1*H*)-on ausgehend von 150 mg Intermediat 10 und 246 mg 3-[(4-Methylpiperazin-1-yl)sulfonyl]anilin (Intermediat 110, US20030225106) hergestellt. Man erhielt 95 mg der Titelverbindung.
¹H-NMR (400 MHz, DMSO-d6): δ = 1.08 (d, 3H); 2.59 (bd, 1H); 1.76 (dq, 1H); 1.89 (dq, 1H); 1.93 (bd, 1H); 2.13 (s, 3H); 2.30-2.40 (m, 4H); 2.82-2.94 (m, 4H); 3.21 (s, 1H); 3.45-3.56 (m, 2H); 3.88-3.99 (m, 2H); 4.24 (q, 1H); 4.45 (tt, 1H); 6.28 (d, 1H); 7.13 (dd, 1H); 7.30 (d, 1H); 7.49 (t, 1H); 7.80 (t, 1H); 8.09 (dd, 1H); 9.22 (s, 1H).

### Beispiel 13

### 3-{[(3R)-1,3-Dimethyl-2-oxo-4-(tetrahydro-2H-pyran-4-yl)-1,2,3,4-tetrahydropyrido[2,3-b]-pyrazin-6-yl]amino}-N-(1-methylpiperidin-4-yl)benzamid

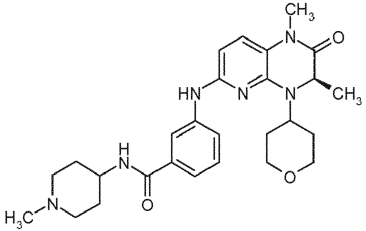

In Analogie zur Herstellung von Beispiel 1 wurde 3-{[(3*R*)-1,3-Dimethyl-2-oxo-4-(tetrahydro-2*H-*pyran-4-yl)-1,2,3,4-tetrahydropyrido[2,3-*b*]pyrazin-6-yl]amino}-*N*-(1-methylpiperidin-4-yl)benzamid ausgehend von 115 mg Intermediat 18 und 63 mg 1-Methylpiperidin-4-amin(Amin 14, Tabelle 1) hergestellt. Man erhielt 80 mg der Titelverbindung.
¹H-NMR (400 MHz, DMSO-d6): δ = 1.08 (d, 3H); 1.53-1.80 (m, 6H); 1.89 (dq, 1H); 1.94 (bd, 1H); 2.04 (t, 2H); 2.21 (s, 3H); 2.77-2.86 (m, 2H); 3.20 (s, 3H); 3.40 (dt, 1H); 3.48 (dt, 1H); 3.67-3.81 (m, 2H); 3.87-3.95 (m, 2H); 4.22 (q, 1H); 4.44 (tt, 1H); 6.24 (d, 1H); 7.23-7.31 (m, 3H); 7.71-7.78 (m, 1H); 7.99 (bs, 1H); 8.14 (d, 1H); 8.92 (s, 1H).

### Beispiel 14

### N-{trans-4-[4-(Cyclopropylmethyl)piperazin-1-yl]cyclohexyl}-3-{[(3R)-1,3-dimethyl-2-oxo-4-(tetrahydro-2H-pyran-4-yl)-1,2,3,4-tetrahydropyrido[2,3-b]pyrazin-6-yl]amino}benzamid

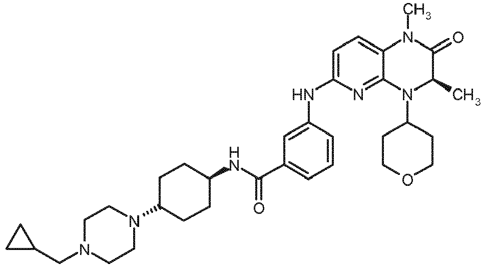

In Analogie zur Herstellung von Beispiel 1 wurde *N*-{*trans*-4-[4-(Cyclopropylmethyl)piperazin-1-yl]cyclohexyl}-3-{[(3*R*)-1,3-dimethyl-2-oxo-4-(tetrahydro-2*H-*pyran-4-yl)-1,2,3,4-tetrahydropyrido[2,3-*b*]pyrazin-6-yl]amino}benzamid ausgehend von 120 mg Intermediat 18 und 136 mg *trans*-4-[4-(Cyclopropylmethyl)piperazin-1-yl]cyclohexanamin (Amin 13, Tabelle 1) hergestellt. Man erhielt 65 mg der Titelverbindung.
¹H-NMR (400 MHz, DMSO-d6): δ = 0.03-0.09 (m, 2H); 0.41-0.48 (m, 2H); 0.75-0.86 (m, 1H); 1.08 (d, 3H); 1.22-1.42 (m, 4H); 1.54-1.63 (m, 1H); 1.71 (dq, 1H); 1.79-1.99 (m, 6H); 2.16 (d, 2H); 2.17-2.27 (m, 1H); 3.20 (s, 3H); 3.40 (dt, 1H); 3.48 (dt, 1H); 3.60-3.76 (m); 3.87-3.96 (m, 2H); 4.22 (q, 1H); 4.44 (tt, 1H); 6.24 (d, 1H); 7.22-7.31 (m, 3H); 7.75 (dt, 1H); 7.97 (bs, 1H); 8.08 (d, 1H); 8.92 (s, 1H).

### Beispiel 15

### (3R)-1,3-Dimethyl-6-({3-[(4-methylpiperazin-1-yl)carbonyl]phenyl}amino)-4-(tetrahydro-2H-pyran-4-yl)-3,4-dihydropyrido[2,3-b]pyrazin-2(1H)-on

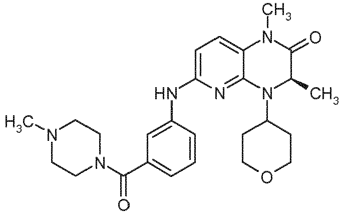

In Analogie zur Herstellung von Beispiel 1 wurde (3*R*)-1,3-Dimethyl-6-({3-[(4-methylpiperazin-1-yl)carbonyl]phenyl}amino)-4-(tetrahydro-2*H*-pyran-4-yl)-3,4-dihydropyrido[2,3-*b*]pyrazin-2(1*H*)-on ausgehend von 120 mg Intemediat 18 und 57 mg 1-Methylpiperazin (Amin 16, Tabelle 1) hergestellt. Man erhielt 53 mg der Titelverbindung.
¹H-NMR (400 MHz, DMSO-d6): δ = 1.08 (d, 3H); 1.53-1.62 (m, 1H); 1.76 (dq, 1H); 1.82-1.96 (m, 2H); 2.20 (s, 3H); 2.23-2.44 (m, 4H); 3.20 (s, 3H); 3.34-3.49 (m, 2H); 3.89-3.99 (m, 2H); 4.23 (q, 1H); 4.43 (tt, 1H); 6.26 (d, 1H); 6.78 (d, 1H); 7.22-7.30 (m, 3H); 7.53 (d, 1H); 7.84 (s, 1H); 8.97 (s, 1H).

### Beispiel 16

### 3-{[(3R)-1,3-Dimethyl-2-oxo-4-(tetrahydro-2H-pyran-4-yl)-1,2,3,4-tetrahydropyrido[2,3-b]-pyrazin-6-yl]amino}-4-methoxy-N-(1-methylpiperidin-4-yl)benzamid

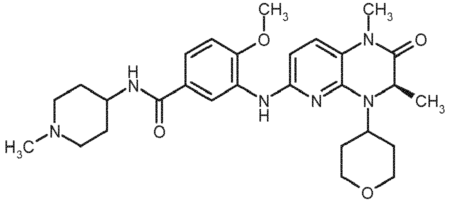

In Analogie zur Herstellung von Beispiel 1 wurde 3-{[(3*R*)-1,3-Dimethyl-2-oxo-4-(tetrahydro-2*H-*pyran-4-yl)-1,2,3,4-tetrahydropyrido[2,3-*b*]pyrazin-6-yl]amino}-4-methoxy-*N*-(1-methylpiperidin-4-yl)benzamid ausgehend von 120 mg Intermediat 20 und 61 mg 4-Amino-1-methylpiperidin (Amin 14, Tabelle 1) hergestellt. Man erhielt 68 mg der Titelverbindung.
¹H-NMR (400 MHz, DMSO-d6): δ = 1.06 (d, 3H); 1.46-1.68 (m, 4H); 1.68-1.92 (m, 4H); 2.01 (t, 2H); 2.19 (s, 3H); 2.75-2.86 (m, 2H); 3.25 (dt, 1H); 3.45 (dt, 1H); 3.67-3.87 (m); 3.89 (s, 3H); 4.29 (q, 1H); 4.44 (tt, 1H); 6.45 (d, 1H); 6.98 (d, 3H); 7.24 (d, 1H); 7.38 (dd, 1H); 7.91 (s, 1H); 8.03 (d, 1H); 8.54 (d, 1H).

### Beispiel 17

### (3R)-6-({2-Methoxy-5-[(4-methylpiperazin-1-yl)carbonyl]phenyl]amino)-1,3-dimethyl-4-(tetrahydro-2H-pyran-4-yl)-3,4-dihydropyrido[2,3-b]pyrazin-2(1H)-on

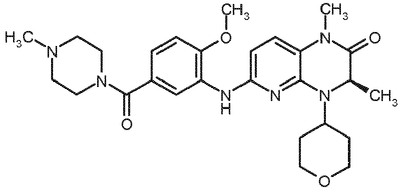

In Analogie zur Herstellung von Beispiel 1 wurde (3*R*)-6-({2-Methoxy-5-[(4-methylpiperazin-1-yl)carbonyl]phenyl}amino)-1,3-dimethyl-4-(tetrahydro-2*H-*pyran-4-yl)-3,4-dihydropyrido[2,3-*b*]-pyrazin-2(1*H*)-on ausgehend von 120 mg Intermediat 20 und 54 mg 1-Methylpiperazin (Amin 16, Tabelle 1) hergestellt. Man erhielt 59 mg der Titelverbindung.
¹H-NMR (400 MHz, DMSO-d6): δ = 1.07 (d, 3H); 1.87-1.58 (m, 1H); 1.63-1.90 (m, 3H); 2.18 (s, 3H); 2.23-2.39 (m, 4H); 3.20 (s, 3H); 3.28-3.47 (m, 2H); 3.82-3.95 (m+s, 5H); 4.22 (q, 1H); 4.43 (tt, 1H); 6.55 (d, 1H); 6.85 (dd, 1H); 6.88 (d, 1H); 7.26 (d, 1H); 7.97 (s, 1H); 8.44 (d, 1H).

### Beispiel 18

### N-{trans-4-[4-(Cyclopropylmethyl)piperazin-1-yl]cyclohexyl}-3-{[(3R)-1,3-dimethyl-2-oxo-4-(tetrahydro-2H-pyran-4-yl)-1,2,3,4-tetrahydropyrido[2,3-b]pyrazin-6-yl]amino}-4-methoxybenzamid

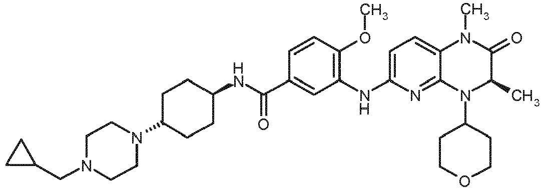

In Analogie zur Herstellung von Beispiel 1 wurde *N*-{*trans*-4-[4-(Cyclopropylmethyl)piperazin-1-yl]cyclohexyl}-3-{[(3*R*)-1,3-dimethyl-2-oxo-4-(tetrahydro-2*H*-pyran-4-yl)-1,2,3,4-tetrahydropyrido[2,3-*b*]pyrazin-6-yl]amino}-4-methoxybenzamid ausgehend von 120 mg Intermediat 20 und 127 mg *trans*-4-[4-(Cyclopropylmethyl)piperazin-1-yl]cyclohexanamin (Amin 13, Tabelle 1) hergestellt. Man erhielt 66 mg der Titelverbindung.
¹H-NMR (400 MHz, DMSO-d6): δ = 0.02-0.10 (m, 2H); 0.40-0.49 (m, 2H); 0.74-0.88 (m, 1H); 1.06 (d, 3H); 1.19-1.43 (m, 4H); 1.46-1.68 (m, 2H); 1.71-1.94 (m, 6H); 2.12-2.29 (m+d, 3H); 3.20 (s, 3H); 3.25 (r, 1H); 3.45 (t, 1H); 3.89 (s, 3H); 4.19 (q, 1H); 4.43 (tt, 1H); 6.44 (d, 1H); 6.98 (d, 1H); 7.24 (d, 1H); 7.37 (dd, 1H); 7.90 (s, 1H); 7.98 (d, 1H); 8.53 (d, 1H).

### Beispiel 19

### N-[2-(Dimethylamino)ethyl]-3-{[(3R)-1,3-dimethyl-2-oxo-4-(tetrahydro-2H-pyran-4-yl)-1,2,3,4-tetrahydropyrido[2,3-b]pyrazin-6-yl]amino}-4-methoxybenzamid

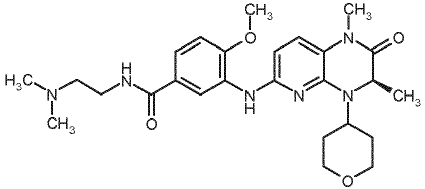

In Analogie zur Herstellung von Beispiel 1 wurde *N*-[2-(Dimethylamino)ethyl]-3-{[(3*R*)-1,3-di-methyl-2-oxo-4-(tetrahydro-2*H*-pyran-4-yl)-1,2,3,4-tetrahydropyrido[2,3-*b*]pyrazin-6-yl]amino}-4-methoxybenzamid ausgehend von 120 mg Intermediat 20 und 47 mg *N,N*-Dimethylethan-1,2-di-amin (Amin 19, Tabelle 1) hergestellt. Man erhielt 50 mg der Titelverbindung.
¹H-NMR (400 MHz, CD₃OH): δ = 1.16 (d, 3H); 1.66 (bd, 1H); 1.76 (dq, 1H); 1.89 (dq, 1H); 2.02 (bd, 1H); 2.59 (s, 6H); 2.80 (t, 2H); 3.30 (s, 3H); 3.50-3.61 (m, 3H); 3.65 (dt, 1H); 3.92 (dd, 1H); 3.96 (s, 3H); 4.27 (q, 1H); 4.67 (tt, 1H); 6.38 (d, 1H); 7.00 (d, 1H); 7.25 (d, 1H); 7.37 (dd, 1H); 8.73 (d, 1H).

### Beispiel 20

### 3-{[(3R)-1,3-Dimethyl-2-oxo-4-(tetrahydro-2H-pyran-4-yl)-1,2,3,4-tetrahydropyrido[2,3-b-pyrazin-6-yl]amino}-N-(1-methylpiperidin-4-yl)benzolsulfonamid

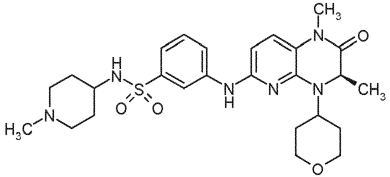

In Analogie zur Herstellung von Beispiel 6 wurde 3-{[(3*R*)-1,3-Dimethyl-2-oxo-4-(tetrahydro-2*H-*pyran-4-yl)-1,2,3,4-tetrahydropyrido[2,3-*b*]pyrazin-6-yl]amino}-*N*-(1-methylpiperidin-4-yl)benzol-sulfonamid ausgehend von 200 mg Intermediat 10 und 346 mg 3-Amino-*N*-(1-methylpiperidin-4-yl)benzolsulfonamid (Intermediat 92) hergestellt. Man erhielt 75 mg der Titelverbindung.
¹H-NMR (400 MHz, DMSO-d6): δ = 1.08 (d, 3H); 1.34-1.47 (m, 2H); 1.51-1.62 (m, 3H); 1.75 (dq, 1H); 1.82-1.92 (m, 4H); 2.12 (s, 3H); 2.62-2.70 (m, 2H); 2.87-2.98 (m, 1H); 3.21 (s, 3H); 3.87-3.97 (m, 2H); 4.23 (q, 1H); 4.47 (tt, 1H); 6.28 (d, 1H); 7.24 (d, 1H); 7.29 (d, 1H); 7.42 (t, 1H); 7.62 (d, 1H); 7.88 (dd, 1H); 7.98 (t, 1H); 9.15 (s, 1H).

### Beispiel 21

### N-[2-(Dimethylamino)ethyl]-3-{[(3R)-1,3-dimethyl-2-oxo-4-(tetrahydro-2H-pyran-4-yl)-1,2,3,4-tetrahydropyrido[2,3-b]pyrazin-6-yl]amino}benzolsulfonamid

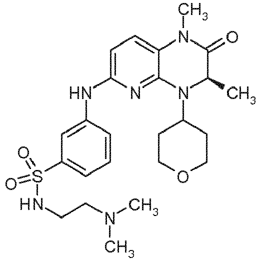

In Analogie zur Herstellung von Beispiel 6 wurde *N*-[2-(Dimethylamino)ethyl]-3-{[(3*R*)-1,3-di-methyl-2-oxo-4-(tetrahydro-2*H*-pyran-4-yl)-1,2,3,4-tetrahydropyrido [2,3 -*b*]pyrazin-6-yl]amino}-benzolsulfonamid ausgehend von 200 mg Intermediat 10 und 312 mg 3-Amino-*N*-[2-(dimethyl-amino)ethyl]benzolsulfonamid (Intermediat 94) hergestellt. Man erhielt 20 mg der Titelverbindung.
¹H-NMR (400 MHz, DMSO-d6): δ = 1.08 (d, 3H); 1.52-1.63 (m, 1H); 1.75 (dq, 1H); 1.81-1.99 (m, 2H); 2.00-2.09 (m+s, 7H); 2.20-2.30 (m, 2H); 2.82 (t, 2H); 3.52 (t); 3.86-3.98 (m, 2H); 4.24 (q, 1H); 4.48 (tt, 1H); 6.27 (d, 1H); 7.22 (d, 1H); 7.29 (d, 1H); 7.43 (t, 1H); 7.91 (dd, 1H); 7.96 (t, 1H); 9.17 (s,1H).

### Beispiel 22

### N-{trans-4-[4-(Cyclopropylmethyl)piperazin-1-yl]cyclohexyl}-3-{[4-(2,6-difluorbenzyl)-1,3-dimethyl-2-oxo-1,2,3,4-tetrahydrochinoxalin-6-yl]amino}benzamid

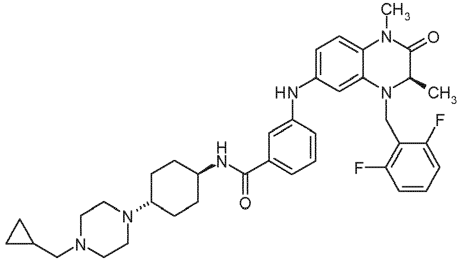

In Analogie zur Herstellung von Beispiel 1 wurde *N*-{*trans*-4-[4-(Cyclopropylmethyl)piperazin-1-yl]cyclohexyl}-3-{[4-(2,6-difluorbenzyl)-1,3-dimethyl-2-oxo-1,2,3,4-tetrahydrochinoxalin-6-yl]-amino}benzamid ausgehend von 40 mg Intermediat 25 und 44 mg *trans*-4-[4-(Cyclopropylmethyl)-piperazin-1-yl]cyclohexanamin (Amin 13, Tabelle 1) hergestellt. Man erhielt 33 mg der Titelverbindung.
¹H-NMR (400 MHz, DMSO-d6): δ = 0.01-0.09 (m, 2H); 0.40-0.48 (m, 2H); 0.72-0.87 (m, 1H); 1.03 (d, 3H); 1.18-1.40 (m, 5H); 1.73-1.90 (m, 4H); 2.08-2.20 (m+d, 3H); 3.22 (s, 3H); 3.60-3.74 (m, 1H); 3.79 (q, 1H); 4.24 (d, 1H); 4.55 (d, 1H); 6.60 (dd, 1H); 6.71 (d, 1H); 6.97 (d, 1H); 7.05 (bd, 1H); 7.08-7.28 (m, 4H); 7.39-7.52 (m, 2H); 8.09 (d, 1H); 8.17 (s, 1H).

### Beispiel 23, allgemeine Synthesevorschrift B

### 3-{[(3R)-1,3-Dimethyl-2-oxo-4-(tetrahydro-2H-pyran-4-yl)-1,2,3,4-tetrahydropyrido[2,3-b]pyrazin-6-yl] amino}-N-ethylbenzolsulfonamid

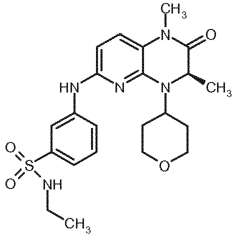

Eine Mischung von 150 mg Intermediat 10, 144 mg 3-Amino-*N*-ethylbenzolsulfonamid (Amin 7, Tabelle 1), 6.6 mg Tris(dibenzylidenaceton)dipalladium(0) (CAS 51364-51-3), 235 mg Caesiumcarbonat und 12.3 mg Xanthphos (CAS 161265-03-8) in 15 ml Dioxan wurde 20 Stunden unter einer Argonatmosphäre bei 120°C gerührt. Der Ansatz wurde auf Wasser gegeben und zweimal mit Ethylacetat extrahiert. Die organische Phase wurde mit gesättigter Natriumchlorid Lösung gewaschen, über Natriumsulfat getrocknet und das Lösungsmittel im Vakuum entfernt. Der Rückstand wurde durch Chromatographie an Kieselgel (Dichlormethan / Methanol Gradient bis 3% Methanol Anteil) gereinigt. Man erhielt 50 mg 3-{[(3*R*)-1,3-Dimethyl-2-oxo-4-(tetrahydro-2H-pyran-4-yl)-1,2,3,4-tetrahydropyrido[2,3-b]pyrazin-6-yl]amino}-*N*-ethylbenzolsulfonamid.
¹H-NMR (300 MHz, 25°C, DMSO-d6): δ = 0.97 (t, 3H); 1.07 (d, 3H); 1.57 (br. d, 1H); 1.90 (bs, 3H); 2.77 (ddd, 2H); 3.20 (s, 3H); 3.51 (t, 2H); 3.85 - 3.98 (m, 2H); 4.23 (q, 1H); 4.47 (tt, 1H); 6.27 (d, 1H); 7.20 (br. d, 1H); 7.28 (d, 1H); 7.38 - 7.49 (m, 2H); 7.88 - 7.97 (m, 2H); 9.16 (s, 1H).

### Beispiel 24

### (3R)-1,3-Dimethyl-4-(1-methylpiperidin-4-yl)-6-{[3-(pyrrolidin-1-ylsulfonyl)phenyl]amino}-3,4-dihydropyrido[2,3-b]pyrazin-2(1H)-on

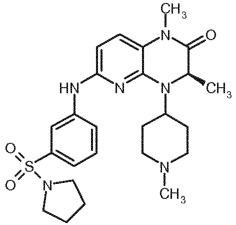

Eine Mischung von 200 mg Intermediat 65, 146 mg 3-(Pyrrolidin-1-ylsulfonyl)anilin (Amin 1, Tabelle 1), 10.6 mg 2-Dicyclohexylphosphino-2*'*,6*'*-diisopropoxybiphenyl (CAS: 787618-22-8), 17.6 mg Chlor-(2-dicyclohexylphosphino-2*'*,6*'*-diisopropoxy-1,1*'*-biphenyl)[2-(2*'*-amino-1,1*'-*biphenyl)]palladium (II) (CAS: 1375325-68-0) und 253 mg Caesiumcarbonat in 2 ml Dioxan wurde 2 Stunden unter einer Argonatmosphäre bei 130°C gerührt. Der Ansatz wurde mit Wasser und Dichlormethan verdünnt und durch eine Phasen-Trennungs-Kartusche (Biotage Isolute^{®} Phasen- Separator, Teilenummer 120-1903-B) filtriert. Die organische Phase wurde im Vakuum eingeengt. Der Rückstand wurde durch Chromatographie an Kieselgel (Dichlormethan / Methanol Gradient bis 10% Methanol Anteil) gereinigt. Man erhielt 180 mg (3*R*)-1,3-Dimethyl-4-(1-methylpiperidin-4-yl)-6-{[3-(pyrrolidin-1-ylsulfonyl)phenyl]amino-3,4-dihydropyrido[2,3-b]pyrazin-2(1H)-on.
¹H-NMR (300 MHz, 25°C, CDCl₃): δ = 1.22 (d, 3H); 1.67-1.88 (m, 6H); 2.01-2.21 (m, 4H); 2.33 (s, 3H); 2.97 (d, 2H); 3.25-3.30 (m, 7H); 4.23-4.36 (m, 2H); 6.25 (d, 1H); 6.45 (s, 1H); 7.04 (d, 1H); 7.33-7.45 (m, 2H); 7.67 (s, 1H); 7.80 (d, 1H).

### Beispiel 25, allgemeine Synthesevorschrift C

### (3R)-6-{[2-Methoxy-5-(morpholin-4-ylsulfonyl)phenyl]amino}-1,3-dimethyl-4-(1-methylpiperidin-4-yl)-3,4-dihydropyrido[2,3-b]pyrazin-2(1H)-on

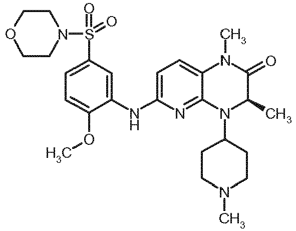

Eine Mischung von 200 mg Intermediat 65, 176 mg Intermediat 97, 10.6 mg 2-Dicyclohexylphosphino-2*'*,6*'*-diisopropoxybiphenyl (CAS: 787618-22-8), 17.6 mg Chlor-(2-dicyclohexylphosphino-2*'*,6*'*-diisopropoxy-1,1*'*-biphenyl)[2-(2*'*-amino-1,1*'*-biphenyl)]palladium (II) (CAS: 1375325-68-0) und 253 mg Caesiumcarbonat in 2 ml Dioxan wurde 3 Stunden unter einer Argonatmosphäre bei 130°C gerührt. Der Ansatz wurde mit Wasser und Dichlormethan verdünnt und durch eine Phasen-Trennungs-Kartusche (Biotage Isolute^{®} Phasen- Separator, Teilenummer 120-1903-B) filtriert. Die organische Phase wurde im Vakuum eingeengt. Der Rückstand wurde durch Chromatographie an Kieselgel (Dichlormethan / Methanol Gradient bis 10% Methanol Anteil) gereinigt. Man erhielt 85 mg (3*R*)-6-{[2-Methoxy-5-(morpholin-4-ylsulfonyl)phenyl]amino} -1,3-dimethyl-4-(1-methylpiperidin-4-yl)-3,4-dihydropyrido[2,3-b]pyrazin-2(1H)-on.
¹H-NMR (400 MHz, 25°C, CDCl₃): δ = 1.19 (d, 3H); 1.60-1.79 (m, 3H); 1.93 (dq, 1H); 2.08 (d, 1H); 2.26-2.34 (m, 4H); 2.86-3.00 (m, 6H); 3.30 (s, 3H); 3.70-3.73 (m, 4H); 3.99 (s, 3H); 4.31 (q, 1H); 4.40 (tt, 1H); 6.28 (d, 1H); 6.81 (s, 1H); 6.96 (d, 1H); 7.04 (d, 1H); 7.24-7.28 (m, 1H); 8.32 (d, 1H).

### Beispiel 26

### N-Cyclopropyl-3-{[(3R)-1,3-dimethyl-2-oxo-4-(tetrahydro-2H-pyran-4-yl)-1.2.3.4-tetrahydropyrido[2,3-b]pyrazin-6-yl]amino}benzolsulfonamid

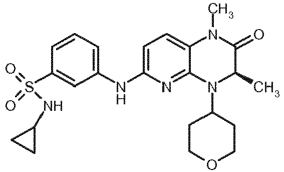

Eine Mischung von 150 mg Intermediat 10, 153 mg 3-Amino-*N*-cyclopropylbenzolsulfonamid (Amin 8, Tabelle 1), 6.6 mg Tris(dibenzylidenaceton)dipalladium(0) (CAS 51364-51-3), 235 mg Caesiumcarbonat und 12.3 mg Xanthphos (CAS 161265-03-8) in 15 ml Dioxan wurde 20 Stunden unter einer Argonatmosphäre bei 120°C gerührt. Der Ansatz wurde auf Wasser gegeben und zweimal mit Ethylacetat extrahiert. Die organische Phase wurde mit gesättigter Natriumchlorid Lösung gewaschen, über Natriumsulfat getrocknet und das Lösungsmittel im Vakuum entfernt. Der Rückstand wurde durch Chromatographie an Kieselgel (Dichlormethan / Methanol Gradient bis 2% Methanol Anteil) gereinigt. Man erhielt 20 mg *N*-Cyclopropyl-3-{[(3*R*)-1,3-dimethyl-2-oxo-4-(tetrahydro-2H-pyran-4-yl)-1,2,3,4-tetrahydropyrido[2,3-b]pyrazin-6-yl]amino}benzolsulfonamid.
¹H-NMR (400 MHz, 25°C; DMSO-d6): δ = 0.40 (d, 2H); 0.43 - 0.50 (m, 2H); 1.08 (d, 3H); 1.54 - 1.61 (m, 1H); 1.68 - 1.80 (m, 1H); 1.81 - 1.90 (m, 1H); 1.90 - 1.98 (m, 1H); 2.06 - 2.13 (m, 1H); 3.20 (s, 3H); 3.46 - 3.57 (m, 2H); 3.87 - 3.9.5 (m, 2H); 4.23 (q, 1H); 4.47 (tt, 1H); 6.27 (d, 1H); 7.23 (dt, 1H); 7.28 (d, 1H); 7.41 - 7.47 (m, 1H); 7.80 (d, 1H); 7.96 (d, 2H); 9.16 (s, 1H).

### Beispiel 27

### (3R)-1,3-Dimethyl-6-{[3-(pyrrolidin-1-ylsulfonyl)phenyl]amino}-4-(tetrahydro-2H-pyran-4-yl)-3,4-dihydropyrido[2,3-b]pyrazin-2(1H)-on

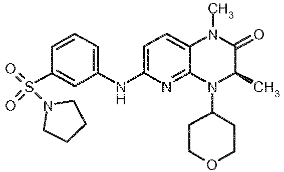

Eine Mischung von 150 mg Intermediat 10, 163 mg 3-(Pyrrolidin-1-ylsulfonyl)anilin (Amin 1, Tabelle 1), 6.6 mg Tris(dibenzylidenaceton)dipalladium(0) (CAS 51364-51-3), 235 mg Caesiumcarbonat und 12.3 mg Xanthphos (CAS 161265-03-8) in 15 ml Dioxan wurde 20 Stunden unter einer Argonatmosphäre bei 120°C gerührt. Der Ansatz wurde auf Wasser gegeben und zweimal mit Ethylacetat extrahiert. Die organische Phase wurde mit gesättigter Natriumchlorid Lösung gewaschen, über Natriumsulfat getrocknet und das Lösungsmittel im Vakuum entfernt. Der Rückstand wurde durch Chromatographie an Kieselgel (Dichlormethan / Methanol Gradient bis 2% Methanol Anteil) gereinigt. Man erhielt 150 mg (3*R*)-1,3-Dimethyl-6-{[3-(pyrrolidin-1-ylsulfonyl)phenyl]amino-4-(tetrahydro-2H-pyran-4-yl)-3,4-dihydropyrido[2,3-b]pyrazin-2(1H)-on.
¹H-NMR (400 MHz, 25°C; DMSO-d6): δ = 1.08 (d, 3H); 1.58 (bd, 1H); 1.62-1.69 (m, 4H); 1.77 (qd, 1H); 1.83-1.98 (m, 2H); 3.10-3.18 (m, 4H); 3.21 (s, 3H); 3.47-3.57 (m, 2H); 3.89-3.98 (m, 2H); 4.24 (q, 1H); 4.47 (tt, 1H); 6.28 (d, 1H); 7.20 (bd, 1H); 7.30 (d, 1H); 7.47 (t, 1H); 7.89 (t, 1H); 8.08 (dd, 1H); 9.21 (s, 1H).

### Beispiel 28

### (3R)-6-{[2-Methoxy-5-(morpholin-4-ylsulfonyl)phenyl]amino}-1,3-dimethyl-4-(tetrahydro-2H-pyran-4-yl)-3,4-dihydropyrido[2,3-b]pyrazin-2(1H)-on

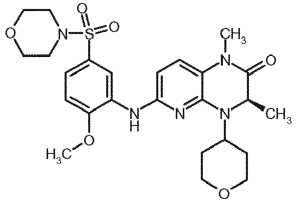

Eine Mischung von 200 mg Intermediat 10, 202 mg Intermediat 97, 11 mg 2-Dicyclohexylphosphino-2',6'-diisopropoxybiphenyl (CAS: 787618-22-8), 18.3 mg Chlor-(2-dicyclohexylphosphino-2*'*,6*'*-diisopropoxy-1,1*'*-biphenyl)[2-(2*'*-amino-1,1*'*-biphenyl)]palladium (II) (CAS: 1375325-68-0) und 264 mg Caesiumcarbonat in 2.3 ml Dioxan wurde 1.5 Stunden unter einer Argonatmosphäre bei 130°C gerührt. Der Ansatz wurde mit Wasser verdünnt und dreimal mit Dichlormethan extrahiert. Die vereinten organischen Phasen wurde über Magnesiumsulfat getrocknet und das Lösungsmittel im Vakuum entfernt. Der Rückstand wurde durch Chromatographie an Kieselgel (Dichlormethan / Methanol Gradient bis 2% Methanol Anteil) gereinigt. Man erhielt 40 mg (3*R*)-6-{[2-Methoxy-5-(morpholin-4-ylsulfonyl)phenyl]amino}-1,3-dimethyl-4-(tetrahydro-2H-pyran-4-yl)-3,4-dihydropyrido[2,3-b]pyrazin-2(1H)-on.
¹H-NMR (400 MHz, CDCl₃): δ =1.21 (d, 3H); 1.60-1.67 (m, 1H); 1.77 (dq, 1H); 1.91 (dq, 1H); 1.99-2.06 (m, 1H); 2.94-2.97 (m, 4H); 3.31 (s, 3H); 3.63-3.75 (m, 6H); 3.96-4.02 (m, 5H); 4.30 (q, 1H); 4.65-4.73 (m, 1H); 6.29 (d, 1H); 6.85 (s, 1H); 6.97 (d, 1H); 7.07 (d, 1H); 8.42 (s, 1H).

### Beispiel 29

### (3R)-6-({3-[(3,3-Difluorazetidin-1-yl)sulfonyl]phenyl}amino)-1,3-dimethyl-4-(1-methylpiperidin-4-yl)-3,4-dihydropyrido[2,3-b]pyrazin-2(1H)-on

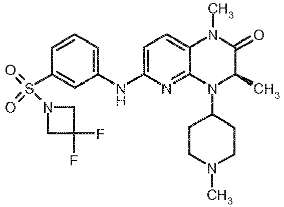

Eine Mischung von 200 mg Intermediat 65, 161 mg 3-[(3,3-Difluoroazetidin-1-yl)sulfonyl]anilin (siehe Intermediat 99), 10.6 mg 2-Dicyclohexylphosphino-2',6'-diisopropoxybiphenyl (CAS: 787618-22-8), 17.6 mg Chlor-(2-dicyclohexylphosphino-2*'*,6*'*-diisopropoxy-1,1*'*-biphenyl)[2-(2*'-*amino-1,1*'*-biphenyl)]palladium (II) (CAS: 1375325-68-0) und 253 mg Caesiumcarbonat in 2 ml Dioxan wurde 2 Stunden unter einer Argonatmosphäre bei 130°C gerührt. Der Ansatz wurde mit Wasser und Dichlormethan verdünnt und durch eine Phasen-Trennungs-Kartusche (Biotage Isolute^{®} Phasen- Separator, Teilenummer 120-1903-B) filtriert. Die organische Phase wurde im Vakuum eingeengt. Der Rückstand wurde durch Chromatographie an Kieselgel (Dichlormethan / Methanol Gradient bis 10% Methanol Anteil) gereinigt. Man erhielt 115 mg (3*R*)-6-({3-[(3,3-Difluorazetidin-1-yl)sulfonyl]phenyl}amino)-1,3-dimethyl-4-(1-methylpiperidin-4-yl)-3,4-dihydropyrido[2,3-b]pyrazin-2(1H)-on.
¹H-NMR (300 MHz, CDCl₃): δ = 1.23 (d, 3H); 1.58-1.90 (m, 2H); 2.01-2.23 (m, 4H); 2.32 (s, 3H); 2.96 (d, 2H); 3.31 (s, 3H); 4.14-4.21 (t, 4H); 4.24-4.35 (m, 2H); 6.24 (d, 1H); 6.48 (s, 1H); 7.04 (d, 1H); 7.37 (d, 1H); 7.49 (t, 1H); 7.75 (s, 1H); 7.82 (d, 1H).

### Beispiel 30

### (3R)-6-{[2-Methoxy-5-(2-oxa-6-azaspiro[3.3]hept-6-ylsulfonyl)phenyl]amino-1,3-dimethyl-4-(tetrahydro-2H-pyran-4-yl)-3,4-dihydropyrido[2,3-b]pyrazin-2(1H)-on

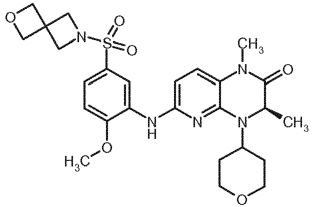

Eine Mischung von 150 mg Intermediat 10, 205 mg Intermediat 101, 22 mg Tris(dibenzylidenaceton)dipalladium(0) (CAS 51364-51-3), 235 mg Caesiumcarbonat und 28 mg Xanthphos (CAS 161265-03-8) in 15 ml Dioxan wurde 6 Stunden unter einer Argonatmosphäre bei 120°C gerührt. Dann wurden erneut 22 mg Tris(dibenzylidenaceton)dipalladium(0) (CAS 51364-51-3) und 28 mg Xanthphos (CAS 16126.5-03-8) zugegeben und weitere 8 Stunden bei 120°C gerührt. Der Ansatz wurde auf Wasser gegeben und zweimal mit Ethylacetat extrahiert. Die organische Phase wurde mit gesättigter Natriumchlorid Lösung gewaschen, über Natriumsulfat getrocknet und das Lösungsmittel im Vakuum entfernt. Der Rückstand wurde durch RP-HPLC (Waters Autopurificationsystem SQD; Säule: Waters XBrigde C18 5µm 100x30mm; Eluent A: Wasser + 0.1 % Vol. Ameisensäure (99%), Eluent B: Acetonitril; Gradient: 0-8.0 min 1-100% B, 8.0-10.0 min 100% B; Fluss 50.0 ml/min; Temperatur: RT; Injektion: 2500 µl; DAD scan: 210-400 nm) gereinigt. Man erhielt 105 mg (3*R*)-6-{[2-Methoxy-5-(2-oxa-6-azaspiro[3.3]hept-6-ylsulfonyl)phenyl]amino}-1,3-dimethyl-4-(tetrahydro-2H-pyran-4-yl)-3,4-dihydropyrido[2,3-b]pyrazin-2(1H)-on.
¹H-NMR (400 MHz, DMSO-d6): δ = 1.06 (d, 3H); 1.51 (bd, 1H); 1.68 (qd, 1H); 1.76 (qd, 1); 1.84 (bd, 1H); 3.21 (s, 3H); 3.47-3.59 (m, 2H); 3.77-3.87 (m, 6H); 3.97 (s, 3H); 4.21 (q, 1H); 4.44 (s, 4H); 4.53 (tt, 1H); 6.58 (d, 1H); 7.21 (d, 1H); 7.27-7.33 (m, 2H); 8.14 (s, 1H); 8.56 (d, 1H).

### Beispiel 31

### (3R)-6-({3-[(3,3-Difluorazetidin-1-yl)sulfonyl]phenyl}amino)-1,3-dimethyl-4-(tetrahydro-2H-pyran-4-yl)-3,4-dihydropyrido[2,3-b]pyrazin-2(1H)-on

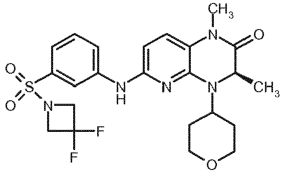

Eine Mischung von 100 mg Intermediat 10, 119 mg Intermediat 99, 14.7 mg Tris(dibenzylidenaceton)dipalladium(0) (CAS 51364-51-3), 157 mg Caesiumcarbonat und 18.6 mg Xanthphos (CAS 161265-03-8) in 15 ml Dioxan wurde 8 Stunden unter einer Argonatmosphäre bei 120°C gerührt. Dann wurden erneut 14.7 mg Tris(dibenzylidenaceton)dipalladium(0) (CAS 51364-51-3) und 18.6 mg Xanthphos (CAS 161265-03-8) zugegeben und weitere 7 Stunden bei 120°C gerührt. Der Ansatz wurde auf Wasser gegeben und zweimal mit Ethylacetat extrahiert. Die organische Phase wurde mit gesättigter wässriger Natriumchlorid-Lösung gewaschen, über Natriumsulfat getrocknet und das Lösungsmittel im Vakuum entfernt. Der Rückstand wurde durch RP-HPLC (Waters Autopurificationsystem SQD; Säule: Waters XBrigde C18 5µm 100x30mm; Eluent A: Wasser + 0.1% Vol. Ameisensäure (99%), Eluent B: Acetonitril; Gradient: 0-8.0 min 1-100% B, 8.0-10.0 min 100% B; Fluss 50.0 ml/min; Temperatur: RT; Injektion: 2500 µl; DAD scan: 210-400 nm) gereinigt. Man erhielt 19 mg (3*R*)-6-({3-[(3,3-Difluorazetidin-1-yl)sulfonyl]phenyl}amino)-1,3-dimethyl-4-(tetrahydro-2H-pyran-4-yl)-3,4-dihydropyrido[2,3-b]pyrazin-2(1H)-on.
¹H-NMR (400 MHz, DMSO-d6): δ = 1.08 (d, 3H); 1.58 (bd, 1H); 1.69-1.99 (m, 3H); 3.21 (s, 3H); 3.43-3.58 (m, 2H); 3.86-4.00 (m, 2H); 4.17-4.29 (m, 5H); 4.46 (tt, 1H); 6.29 (d, 1H); 7.26-7.35 (m, 2H); 7.56 (t, 1H); 7.99 (t, 1H); 8.10 (dd, 1H); 9.30 (s, 1H).

**Tabelle 2: Folgende Beispiele wurden entsprechend benannten allgemeinen Synthesevorschrift aus jeweiligen Intermediaten/Aminen (aus Tabelle 1) hergestellt:**

| Bsp | Struktur | Name | Intermediat/ Amin | Analytische Daten: ¹H-NMR |
|---|---|---|---|---|
| **32** | 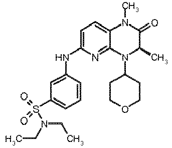 | 3-{[(3*R*)-1,3-Dimethyl-2-oxo-4-(tetrahydro-2H-pyran-4-yl)-1,2,3,4-tetrahydropyrido[2,3-b]pyrazin-6-yl]amino}-*N,N*-diethylbenzol sulfonamid | Intermediat 10, Amin 9, Allgemeine Synthesevor schrift B | (400 MHz, DMSO-d6): δ = 0.99-1.12 (m, 9H); 1.58 (bd, 1H); 1.68-2.00 (m, 3H); 3.15 (q, 4H); 3.21 (s, 3H); 3.46-3.57 (m, 2H); 3.87-3.99 (m, 2H); 4.24 (q, 1H); 4.47 (tt, 1H); 6.27 (d, 1H); 7.17 (bd, 1H); 7.29 (d, 1H); 7.43 (t, 1H); 7.89 (t, 1H); 8.03 (bd, 1H); 9.19 (s, 1H). |
| **33** | 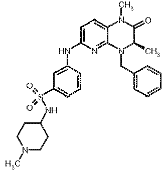 | 3-{[(3*R*)-4-Benzyl-1,3-dimethyl-2-oxo-1,2,3,4-tetrahydropyrido[2,3-b]pyrazin-6-yl]amino}-*N-*(1-methylpiperidin-4-yl)benzolsulfonamid | Intermediat 41, Intermediat 92, Allgemeine Synthesevor schrift B | (400 MHz, DMSO-d6): δ = 1.11 (d, 3H); 1.24 - 1.37 (m, 2H); 1.45 (d, 2H); 1.64 (d, 2H); 2.00 (s, 3H); 2.50 - 2.56 (m, 2H); 2.82 (d, 1H); 3.23 (s, 3H); 3.95 (q, 1H); 4.24 (d, 1H); 5.30 (d, 1H); 6.28 (d, 1H); 7.18 (d, 1H); 7.23 - 7.37 (m, 7H); 7.48 (d,1H); 7.60 (dd,1H); 8.23 (t, 1H); 9.15 (s, 1H). |
| **34** | 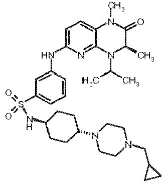 | *N-*{*trans*-4-[4-(Cyclopropylmethyl)pipera zin-1-yl]cyclohexyl}-3-{[(3*R*)-4-isopropyl-1,3-dimethyl-2-oxo-1,2,3,4-tetrahydropyrido[2,3-b]pyrazin-6-yl]amino} benzolsulfonamid | Intermediat 33, Intermediat 106, Allgemeine Synthesevor schrift B | (300 MHz, DMSO-d6): δ = 0.02 (q, 2H); 0.33 - 0.46 (m, 2H); 0.69 - 0.82 (m, 1H); 0.97 - 1.06 (m, 1H); 1.09 (d, 3H); 1.11 - 1.19 (m, 3H); 1.23 (d, 3H); 1.31 (d, 3H); 1.67 (d, 4H); 2.03 - 2.12 (m, 1H); 2.15 (d, 2H); 2.42 (bs, 8H); 2.87 (bs, 1H); 3.20 (s, 3H); 4.24 (q, 1H); 4.72 (spt, 1H); 6.24 (d, 1H); 7.21 (d, 1H); 7.25 (d, 1H); 7.39 (t, 1H); 7.47 (d, 1H); 7.66 (br. d, 1H); 8.31 (br.s, 1H); 9.12 (s, 1H). |
| **35** | 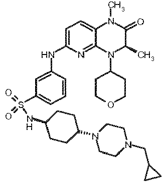 | *N*-{*trans*-4-[4-(Cyclopropylmethyl)pipera zin-1-yl]cyclohexyl}-3-{[(3*R*)-1,3-dimethyl-2-oxo-4-(tetrahydro-2H-pyran-4-yl)-1,2,3,4-tetrahydropyrido[2,3-b]pyrazin-6-yl]amino}benzolsulfonami d | Intermediat 10, Intermediat 106, Allgemeine Synthesevor schrift B | (400 MHz, DMSO-d6): δ = 0.01 - 0.07 (m, 2H); 0.39 - 0.45 (m, 2H); 0.77 (bs, 1H); 1.03 - 1.20 (m, 7H); 1.57 (d, 1H); 1.63 - 1.79 (m, 5H); 1.81 - 1.98 (m, 2H); 2.08 - 2.19 (m, 3H); 2.43 (bs, 6H); 2.52 - 2.54 (m, 1H); 2.80 - 2.91 (m, 1H); 3.20 (s, 3H); 3.51 (ddd, 2H); 3.91 (s, 2H); 4.23 (q, 1H); 4.47 (tt, 1H); 6.27 (a, 1H); 7.23 (br. d, 1H); 7.28 (d, 1H); 7.41 (t, 1H); 7.54 (d, 1H); 7.87 (dd, 1H); 7.97 (t, 1H); 8.19 (s,1H); 9.13 (s,1H). |
| **36** | 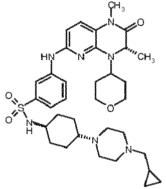 | *N*-{*trans*-4-[4-(Cyclopropylmethyl)pipera zin-1-yl]cyclohexyl}-3-{[(*3S*)-1,3-dimethyl-2-oxo-4-(tetrahydro-2H-pyran-4-yl)-1,2,3,4-tetrahydropyrido[2,3-b]pyrazin-6-yl]amino}benzolsulfonami d | Intermediat 113, Intermediat 106, Allgemeine Synthesevor schrift B | (400 MHz, DMSO-d6): δ = 0.00 - 0.06 (m, 2H); 0.39 - 0.45 (m, 2H); 0.72-0.82 (m, 1H); 1.03 - 1.20 (m, 7H); 1.58 (bd, 1H); 1.63 - 1.79 (m, 5H); 1.81 - 1.98 (m, 2H); 2.08 - 2.19 (m, 3H); 2.42 (bs, 6H); 2.52 - 2.54 (m, 1H); 2.80 - 2.91 (m, 1H); 3.21 (s, 3H); 3.51 (ddd, 2H); 3.91 (s, 2H); 4.24 (q, 1H); 4.48 (tt, 1H); 6.28 (d, 1H); 7.24 (br. d, 1H); 7.29 (d, 1H); 7.42 (t, 1H); 7.54 (d, 1H); 7.88 (dd, 1H); 7.97 (t, 1H); 8.18 (s, 1H); 9.14 (s, 1H). |
| **37** | 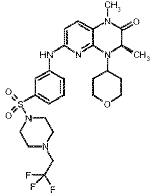 | (3R)-1,3-Dimethyl-4-(tetrahydro-2H-pyran-4-yl)-6-[(3-{[4-(2,2,2-trifluorethyl)piperazin-1-yl]sulfonyl}phenyl)amino] -3,4-dihydropyrido[2,3-b]pyrazin-2(1H)-on | Intermediat 10, Intermediat 108, Allgemeine Synthesevor schrift B | (300 MHz, DMSO-d6): δ =0.96 - 1.13 (m, 3H); 1.58 (br. d, 1H); 1.91 (bs, 3H); 2.67 (bs, 5H); 2.87 (bs, 4H); 3.10 - 3.26 (m, 5H); 3.50 (br. t, 2H); 3.92 (bs, 2H); 4.23 (q, 1H); 4.44 (br. t, 1H); 6.27 (d, 1H); 7.12 (br. d, 1H); 7.29 (d, 1H); 7.49 (t, 1H); 7.78 (br. s, 1H); 8.10 (br. d, |
| | | | | 1H); 9.24 (s, 1H). |
| **38** | 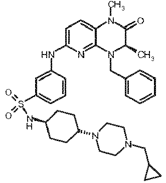 | 3-{[(3*R*)-4-Benzyl-1,3-dimethyl-2-oxo-1,2,3,4-tetrahydropyrido[2,3-b]pyrazin-6-yl]amino}-*N-*{*trans*-4-[4-(cyclopropyl-methyl)piperazin-1-yl]cyclohexyl}benzolsulfo namid | Intermediat 41, Intermediat 106, Allgemeine Synthesevor schrift B | (400 MHz, DMSO-d6): δ = - 0.02 - 0.04 (m, 2H); 0.36 - 0.43 (m, 2H); 0.75 (bs, 1H); 0.88 - 1.09 (m, 4H); 1.11 (d, 3H); 1.61 (d, 4H); 2.01 (bs, 1H); 2.07 (d, 2H); 2.34 (bs, 8H); 2.73 - 2.85 (m, 1H); 3.23 (s, 3H); 3.96 (q, 1H); 4.25 (d, 1H); 5.30 (d, 1H); 6.28 (d, 1H); 7.17 (d, 1H); 7.22 - 7.37 (m, 7H); 7.43 (d, 1H); 7.61 (dd, 1H); 8.20 (t, 1H); 9.14 (s,1H). |
| **39** | 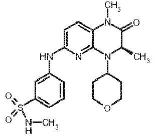 | 3-{[(3*R*)-1,3-Dimethyl-2-oxo-4-(tetrahydro-2H-pyran-4-yl)-1,2,3,4-tetrahydropyrido[2,3-b]pyrazin-6-yl]amino}-*N-*methylbenzolsulfonamid | Intermediat 10, Amin 10, Allgemeine Synthesevor schrift B | (400 MHz, DMSO-d6): δ = 1.07 (d, 3H); 1.53 - 1.60 (m, 1H); 1.68 - 1.80 (m, 1H); 1.80 - 1.90 (m, 1H); 1.90 - 1.97 (m, 1H); 2.41 (d, 3H); 3.20 (s, 3H); 3.48 - 3.56 (m, 2H); 3.86 - 3.96 (m, 2H); 4.23 (q, 1H); 4.47 (tt, 1H); 6.27 (d, 1H); 7.19 (br. d, 1H); 7.26 - 7.33 (m, 2H); 7.43 (t, 1H); 7.90 - 7.95 (m, 2H); 9.16 (s, 1H). |
| **40** | 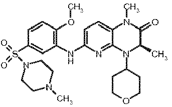 | (3*R*)-6-({2-Methoxy-5-[(4-methylpiperazin-1-yl)sulfonyl]phenyl}amino) -1,3-dimethyl-4-(tetrahydro-2H-pyran-4-yl)-3,4-dihydropyrido[2,3-b]pyrazin-2(1H)-on | Intermediat 10, Intermediat 116, Allgemeine Synthesevor schrift B | (400 MHz, CDC13): δ = 1.20 (d, 3H); 1.62 (d, 1H); 1.77 (dq, 1H); 1.90 (dq, 1H); 2.02 (d, 1H); 2.25 (bs, 3H); 2.45 (bs, 4H); 2.99 (bs, 4H); 3.31 (s, 3H); 3.64-3.75 (m, 2H); 3.97-4.02 (m, 5H); 4.30 (q, 1H); 4.69 (tt, 1H); 6.28 (d, 1H); 6.82 (s, 1H); 6.93 (d, 1H); 7.06 (d, 1H); 7.25-7.27 (m, 1H); 8.41 (d, 1H). |
| **41** | 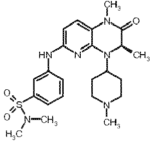 | 3-{[(3*R*)-1,3-Dimethyl-4-(1-methylpiperidin-4-yl)-2-oxo-1,2,3,4-tetra-hydropyrido[2,3-b]pyrazin-6-yl]amino}-*N*,*N-*dimethylbenzolsulfonamid | Intermediat 65, Amin 2, Allgemeine Synthesevor schrift C | (400 MHz, CDC13): δ = 1.22 (d, 3H); 1.65-1.70 (m, 1H); 1.84 (q, 1H); 2.00-2.18 (m, 4H); 2.32 (s, 3H); 2.72 (s, 6H); 2.97 (d, 2H); 3.30 (s, 3H); 4.21-4.33 (m, 2H); 6.25 (d, 1H); 6.51 (s, 1H); 7.03 (d, 1H); 7.79 (ddd, 1H); 7.43 (t, 1H); 7.59 (s, 1H); 7.81 (d, 1H). |
| **42** | 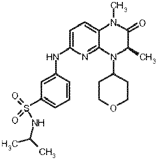 | 3-{[(3*R*)-1,3-Dimethyl-2-oxo-4-(tetrahydro-2H-pyran-4-yl)-1,2,3,4-tetrahydropyrido[2,3-b]pyrazin-6-yl]amino}*-N-*isopropylbenzolsulfonamid | Intermediat 10, Amin 11, Allgemeine Synthesevor schrift B | (400 MHz, CDC13): δ = 0.95 (d, 6H); 1.08 (d, 3H); 1.57 (bd, 1H); 1.66-2.00 (m, 3H); 3.16-3.29 (m+s, 4H); 3.45-3.59 (m, 2H); 3.85-3.99 (m, 2H); 4.24 (q, 1H); 4.47 8tt, 1H); 6.27 (d, 1H); 7.23 (d, 1H); 7.29 (d, 1H); 7.43 (t, 1H); 7.48 (d, 1H); 7.91 (d, 1H); 7.95 (t, 1H); 9.16(s, 1H). |
| **43** | 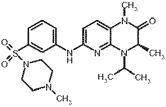 | (3*R*)-4-Isopropyl-1,3-dimethyl-6-({3-[(4-methylpiperazin-1-yl)sulfonyl]phenyl}amino) -3,4-dihydropyrido[2,3-b]pyrazin-2(1H)-on | Intermediat 33, Intermediat 110, Allgemeine Synthesevor schrift B | (400 MHz, DMSO-d6): δ =1.08 (d, 3H); 1.21 (d, 3H); 1.30 (d, 3H); 1.99 - 2.23 (m, 3H); 2.34 (t, 4H); 2.87 (bs, 4H); 3.19 (s, 3H); 4.24 (q, 1H); 4.74 (spt, 1H); 6.25 (d, 1H); 7.11 (d, 1H); 7.27 (d, 1H); 7.45 (dd, 1H); 7.71 (dd, 1H); 8.30 (br. t, 1H); 9.21 (s, 1H). |
| **44** | 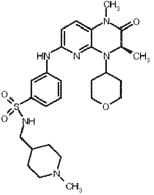 | 3-{[(3*R*)-1,3-Dimethyl-2-oxo-4-(tetrahydro-2H-pyran-4-yl)-1,2,3,4-tetrahydropyrido[2,3-b]pyrazin-6-yl]amino}-*N-*[(1-methylpiperidin-4-yl)methyl]benzolsulfonami d | Intermediat 10, Intermediat 112, Allgemeine Synthesevor schrift B | (400 MHz, DMSO-d6): δ = 0.96 - 1.05 (m, 2H); 1.07 (d, 3H); 1.21 - 1.35 (m, 1H); 1.53 - 1.61 (m, 3H); 1.70 - 1.82 (m, 3H); 1.82 - 1.97 (m, 2H); 2.11 (s, 3H); 2.60 (s, 2H); 2.65 - 2.74 (m, 2H); 3.20 (s, 3H); 3.46 - 3.56 (m, 3H); 3.86 - 3.95 (m, 2H); 4.19 - 4.27 (m, 1H); 4.41 - 4.51 (m, 1H); 6.26 (d, 1H); 7.19 (d, 1H); 7.28 (d, 1H); 7.41 (s, 1H); 7.52 (t, 1H); 7.89 (br. d, 1H); 7.93 (s, 1H); 9.16 (s, 1H). |
| **45** | 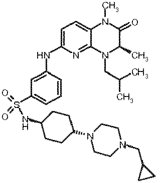 | *N*-{*trans*-4-[4-(Cyclopropylmethyl)pipera zin-1-yl]cyclohexyl}-3-{[(3*R*)-4-isobutyl-1,3-dimethyl-2-oxo-1,2,3,4-tetrahydropyrido[2,3-b]pyrazin-6-yl]amino} benzolsulfonamid | Intermediat 60, Intermediat 106, Allgemeine Synthesevor schrift B | (400 MHz, DMSO-d6): δ = - 0.03 - 0.05 (m, 2H); 0.37 - 0.43 (m, 2H); 0.69 - 0.79 (m, 1H); 0.87 (d, 3H); 0.91 (d, 3H); 1.02 - 1.19 (m, 7H); 1.67 (d, 4H); 2.07 (d, 4H); 2.27 - 2.43 (m, 7H); 2.66 (s, 2H); 2.80 - 2.92 (m, 1H); 3.18 - 3.27 (m, 3H); 3.93 - 4.01 (m, 1H); 4.07 (q, 1H); 6.21 (d, 1H); 7.19 - 7.25 (m, 2H); 7.38 (t, 1H); 7.50 (d, 1H); 7.89 (dd, 1H); 8.02 (t, 1H); 9.10 (s, 1H). |
| **46** | 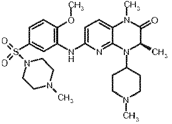 | (3*R*)-6-({2-Methoxy-5-[(4-methylpiperazin-1-yl)sulfonyl]phenyl}amino) -1,3-dimethyl-4-(1-methylpiperidin-4-yl)-3,4-dihydropyrido[2,3-b]pyrazin-2(1H)-on | Intermediat 65, Intermediat 116, Allgemeine Synthesevor schrift C | (400 MHz, CDC13): δ = 1.20 (d, 3H); 1.63-1.80 (m, 3H); 1.94 (q, 1H); 2.08 (d, 1H); 2.21-2.49 (m, 11H); 2.41-3.09 (m, 6H); 3.29 (s, 3H); 3.97 (s, 3H); 4.30 (q, 1H); 4.41 (tt, 1H); 6.28 (d, 1H); 6.78 (s, 1H); 6.93 (s, 1H); 7.03 (d, 1H); 7.24-7.28 (m, 1H); 8.32 (s, 1H). |
| **47** | 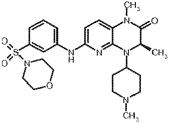 | (3R)-1,3-Dimethyl-4-(1-methylpiperidin-4-yl)-6-{[3-(morpholin-4-ylsulfonyl)phenyl]amino}-3,4-dihydropyrido[2,3-b]pyrazin-2(1H)-on | Intermediat 65, Intermediat 97, Allgemeine Synthesevor schrift C | (300 MHz, CDC13): δ = 1.23 (d, 3H); 1.71 (d, 1H); 1.91 (br.s, 1H); 2.06-2.29 (m, 4H); 2.36 (s, 3H); 2.97-3.04 (m, 6H); 3.30 (s, 3H); 3.73-3.76 (m, 4H); 4.20-4.34 (m, 2H); 6.25 (d, 1H); 6.61 (br.s, 1H); 7.03 (d, 1H); 7.25-7.28 (m, 1H); 7.44 (t, 1H); 7.63 (s, 1H); 7.80 (d, 1H). |
| **48** | 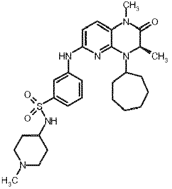 | 3-{[(3*R*)-4-Cycloheptyl-1,3-dimethyl-2-oxo-1,2,3,4-tetrahydropyrido [2,3-b]pyrazin-6-yl] amino}-*N*-(1-methyl-piperidin-4-yl)benzolsulfonamid | Intermediat 38, Intermediat 92, Allgemeine Synthesevor schrift B | (400 MHz, DMSO-d6): δ =1.07 (d, 3H); 1.30 - 1.45 (m, 2H); 1.48 - 1.73 (m, 12H); 1.84 (t, 3H); 2.03 (d, 1H); 2.06 - 2.11 (m, 3H); 2.62 (d, 2H); 2.83 - 2.97 (m, 1H); 3.19 (s, 3H); 4.23 (q, 1H); 4.33 (br. t, 1H); 6.23 (d, 1H); 7.19 - 7.28 (m, 2H); 7.38 (t, 1H); 7.61 (d, 1H); 7.77 (t, 1H); 8.09 (dd, 1H); 9.12 (s, 1H). |
| **49** | 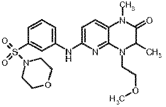 | 4-(2-Methoxyethyl)-1,3-dimethyl-6-{[3-(morpholin-4-ylsulfonyl) phenyl]amino}-3,4-dihydropyrido[2,3-b]pyrazin-2(1H)-on | Intermediat 47, Amin 6, Allgemeine Synthesevor schrift B | (400 MHz, DMSO-d6): δ = 1.12 (d, 3H); 2.81-2.93 (m, 4H); 3.21 (s, 3H); 3.24 (s, 3H); 3.50-3.69 (m, 6H); 4.13-4.26 (m, 2H); 6.24 (d, 1H); 7.12 (d, 1H); 7.26 (d, 1H); 7.48 8t, 1H); 7.77 (dd, 1H); 8.23 (t, 1H); 9.26 (s, 1H). |
| **50** | 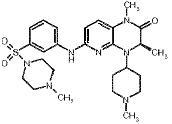 | (3*R*)-1,3-Dimethyl-6-({3-[(4-methylpiperazin-1-yl)sulfonyl]phenyl}amino) -4-(1-methylpiperidin-4-yl)-3,4-dihydropyrido[2,3-b]pyrazin-2(1H)-on | Intermediat 65, Intermediat 110, Allgemeine Synthesevor schrift C | (300 MHz, CDC13): δ = 1.21 (d, 3H); 1.70 (d, 1H); 1.88 (s, 1H); 2.06-2.32 (m, 7H); 2.36 (s, 3H); 2.46-2.49 (m, 4H); 2.95-3.07 (m, 6H); 3.30 (s, 3H); 4.20-4.34 (m, 2H); 6.23 (d, 1H); 6.52 (s, 1H); 7.02 (d, 1H); 7.26-7.28 (m, 1H); 7.41 (t, 1H); 7.62 (s, 1H); 7.72 (s, 1H). |
| **51** | 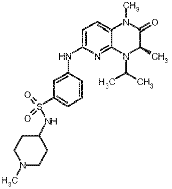 | 3-{[(3*R*)-4-Isopropyl-1,3-dimethyl-2-oxo-1,2,3,4-tetrahydropyrido[2,3-b]pyrazin-6-yl]amino}-*N-*(1-methylpiperidin-4-yl)benzolsulfonamid | Intermediat 33, Intermediat 92, Allgemeine Synthesevor schrift B | (300 MHz, DMSO-d6): δ = 1.08 (d, 3H); 1.22 (d, 3H); 1.30 (d, 3H); 1.33 - 1.44 (m, 2H); 1.45 - 1.58 (m, 2H); 1.68 - 1.80 (m, 2H); 2.04 (s, 3H); 2.54 - 2.62 (m, 2H); 2.80 - 2.94 (m, 1H); 3.20 (s, 3H); 4.24 (q, 1H); 4.65 - 4.78 (m, 1H); 6.24 (d, 1H); 7.21 (br. d, 1H); 7.26 (d, 1H); 7.39 (t, 1H); 7.52 (d, 1H); 7.65 (br. d, 1H); 8.33 (br. s, 1H); 9.13 (s, 1H). |
| **52** | 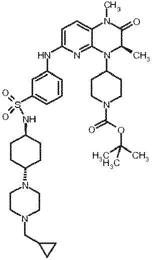 | *tert*-Butyl-4-[(3*R*)-6-{[3-({*trans*-4-[4-(cyclopropyl-methyl)piperazin-1-yl]cyclohexyl}sulfamoyl) phenyl] amino}-1,3-dimethyl-2-oxo-2,3-dihydropyrido[2,3-b]pyrazin-4(1H)-yl]piperidin-1-carboxylat | Intermediat 52, Intermediat 106, Allgemeine Synthesevor schrift B | (400 MHz, DMSO-d6): δ = - 0.02 - 0.05 (m, 2H); 0.37 - 0.46 (m, 2H); 0.71 - 0.82 (m, 1H); 1.06 (d, 3H); 1.08 - 1.21 (m, 5H); 1.42 (s, 9H); 1.61 - 1.74 (m, 6H); 1.93 - 2.06 (m, 2H); 2.09 (d, 3H); 2.39 (bs, 6H); 2.78 - 3.06 (m, 4H); 3.21 (s, 3H); 4.05 (br. s, 2H); 4.22 (q, 1H); 4.43 (br. t, 1H); 6.28 (d, 1H); 7.24 (br. d, 1H); 7.30 (d, 1H); 7.43 (t, 1H); 7.57 (d, 1H); 7.78 (dd, 1H); 8.07 (br. t, 1H); 9.16 (s, 1H). |
| **53** | 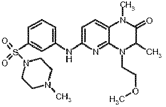 | 4-(2-Methoxyethyl)-1,3-dimethyl-6-({3-[(4-methylpiperazin-1-yl)sulfonyl]phenyl}amino) -3,4-dihydropyrido[2,3-b]pyrazin-2(1H)-on | Intermediat 47, Intermediat 110, Allgemeine Synthesevor schrift B | (400 MHz, DMSO-d6): δ = 1.10 (s, 3H); 2.12 (s, 3H); 2.32 - 2.38 (m, 4H); 2.85 - 2.92 (m, 4H); 3.20 (s, 3H); 3.22 - 3.25 (m, 3H); 3.50 - 3.64 (m, 2H); 4.13 - 4.24 (m, 3H); 6.24 (d, 1H); 7.11 (d, 1H); 7.25 (d, 1H); 7.45 (t, 1H); 7.75 (br. d, 1H); 8.20 (br. s, 1H); 9.21 (s, 1H). |
| **54** | 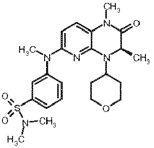 | 3- {[(3*R*)-1,3-Dimethyl-2-oxo-4-(tetrahydro-2H-pyran-4-yl)-1,2,3,4-tetrahydropyrido[2,3-b]pyrazin-6-yl](methyl) amino}-*N,N*-dimethyl-benzolsulfonamid | Intermediat 10, Amin 3, Allgemeine Synthesevor schrift C | (400 MHz, CDC13): δ = 1.26 (d, 3H); 1.51 - 1.74 (m, 2H); 1.80 (dd, 1H); 1.91 - 2.08 (m, 2H); 2.75 (s, 5H); 3.26 - 3.43 (m, 4H); 3.44 - 3.68 (m, 4H); 3.94 - 4.13 (m, 2H); 4.24 - 4.41 (m, 2H); 6.18 (d, 1H); 6.98 (d, 1H); 7.37 - 7.55 (m, 3H); 7.66 (s, 1H). |
| **55** | 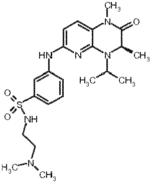 | *N*-[2-(Dimethylamino) ethyl]-3-{[(3*R*)-4-isopropyl-1,3-dimethyl-2-oxo-1,2,3,4-tetrahydro-pyrido[2,3-b]pyrazin-6-yl]amino}benzolsulfonamid | Intermediat 33, Intermediat 94, Allgemeine Synthesevor schrift B | (300 MHz, DMSO-d6): δ =1.08 (d, 3H); 1.22 (d, 3H); 1.30 (d, 3H); 2.08 (s, 6H); 2.28 (t, 2H); 2.83 (t, 2H); 3.20 (s, 3H); 4.24 (q, 1H); 4.72 (spt, 1H); 6.24 (d, 1H); 7.20 (d, 1H); 7.25 (d, 1H); 7.40 (t, 1H); 7.65 (br. d, 1H); 8.16 (s, 1H); 8.34 (br. s, 1H); 9.13 (s, 1H). |
| **56** | 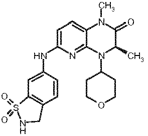 | (3R)-6-[(1,1-Dioxido-2,3-dihydro-1,2-benzothiazol-6-yl)amino]-1,3-dimethyl-4-(tetrahydro-2H-pyran-4-yl)-3,4-dihydropyrido[2,3-b]pyrazin-2(1H)-on | Intermediat 10, Amin 4, Allgemeine Synthesevor schrift B | (400 MHz, DMSO-d6): δ = 1.10 (d, 3H); 1.58 (br. d, 1H); 1.71 - 1.96 (m, 3H); 3.20 - 3.24 (m, 3H); 3.47 - 3.56 (m, 1H); 3.60 - 3.68 (m, 1H); 3.89 - 3.96 (m, 2H); 4.26 (q, 1H); 4.30 (d, 2H); 4.52 (tt, 1H); 6.30 (d, 1H); 7.32 (d, 1H); 7.39 (d, 1H); 7.57 (dd, 1H); 7.70 (br. t, 1H); 8.34 (d, 1H); 9.29 (s, 1H). |
| **57** | 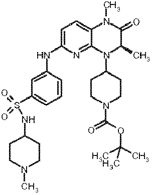 | *tert*-Butyl-4-[(3*R*)-1,3-dimethyl-6-({3-[(1-methylpiperidin-4-yl)sulfamoyl]phenyl}amin o)-2-oxo-2,3-dihydro-pyrido[2,3-b]pyrazin-4(1H)-yl]pipendin-1-carboxylat | Intermediat 52, Intermediat 92, Allgemeine Synthesevor schrift B | (400 MHz, DMSO-d6): δ = 1.05 (d, 3H); 1.38 (d, 2H); 1.41 (s, 9H); 1.51 (d, 3H); 1.55 - 1.64 (m, 2H); 1.75 (bs, 4H); 1.93 - 2.01 (m, 1H); 2.04 (s, 3H); 2.57 (d, 2H); 2.80 - 2.98 (m, 3H); 3.20 (s, 3H); 3.96 - 4.10 (m, 2H); 4.20 (d, 1H); 4.41 (tt, 1H); 6.27 (d, 1H); 7.23 (br. d, 1H); 7.28 (d, 1H); 7.42 (t, 1H); 7.59 (d, 1H); 7.79 (dd, 1H); 8.04 (t, 1H); 9.13 (s, 1H). |
| **58** | 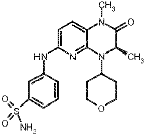 | 3-{[(3*R*)-1,3-Dimethyl-2-oxo-4-(tetrahydro-2H-pyran-4-yl)-1,2,3,4-tetrahydropyrido[2,3-b]pyrazin-6-yl]amino} benzolsulfonamid | Intermediat 10, Amin 5, Allgemeine Synthesevor schrift B | (400 MHz, DMSO-d6): δ = 1.07 (d, 3H); 1.53 - 1.60 (m, 1H); 1.67 - 1.79 (m, 1H); 1.80 - 1.97 (m, 2H); 3.20 (s, 3H); 3.48 - 3.56 (m, 2H); 3.86 - 3.94 (m, 2H); 4.22 (q, 1H); 4.47 (tt, 1H); 6.26 (d, 1H); 7.23 - 7.30 (m, 4H); 7.37 - 7.43 (m, 1H); 7.86 (dd, 1H); 7.98 (t, 1H); 9.15(s, 1H). |
| **59** | 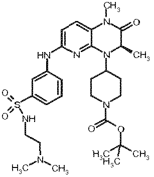 | *tert*-Butyl-4-[(3*R*)-6-[(3-{[2-(dimethylamino)ethyl] sulfamoyl}phenyl)amino]-1,3-dimethyl-2-oxo-2,3-dihydropyrido[2,3-b]pyrazin-4(1H)-yl] piperidin-1-carboxylat | Intermediat 52, Intermediat 94, Allgemeine Synthesevor schrift B | (400 MHz, DMSO-d6): δ = 1.05 (d, 3H); 1.21 - 1.32 (m, 1H); 1.41 (s, 9H); 1.47 - 1.63 (m, 2H); 1.63 - 1.75 (m, 1H); 1.93 - 2.00 (m, 1H); 2.04 (s, 7H); 2.22 (t, 2H); 2.77 - 2.84 (m, 2H); 3.20 (s, 3H); 3.97 - 4.10 (m, 2H); 4.20 (q, 1H); 4.42 (tt, 1H); 6.27 (d, 1H); 7.21(d, 1H); 7.28 (d, 1H); 7.34 - 7.40 (m, 1H); 7.43 (t, 1H); 7.81 (br. d, 1H); 8.02 (br. s, 1H); 9.15 (s, 1H). |
| **60** | 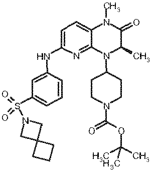 | *tert*-Butyl-4-[(3*R*)-6-{[3-(2-azaspiro[3.3]hept-2-ylsulfonyl)phenyl]amino}-1,3-dimethyl-2-oxo-2,3-dihydropyrido[2,3-b]pyrazin-4(1H)-yl] piperidin-1-carboxylat | Intermediat 52, Intermediat 103, Allgemeine Synthesevor schrift B | (300 MHz, DMSO-d6): δ =1.05 (d, 3H); 1.41 (s, 9H); 1.51 - 1.73 (m, 5H); 1.82 - 1.90 (m, 4H); 1.91 - 2.00 (m, 1H); 2.80 - 3.00 (m, 2H); 3.20 (s, 3H); 3.64 (s, 4H); 3.96 - 4.11 (m, 2H); 4.20 (q, 1H); 4.40 (br. t, 1H); 6.28 (d, 1H); 7.18 (br. d, 1H); 7.30 (d, 1H); 7.51 (t, 1H); 7.94 - 8.00 (m, 2H); 9.22 - 9.27 (m, 1H). |
| **61** | 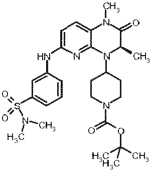 | *tert*-Butyl-4-[(3*R*)-6-{[3-(dimethylsulfamoyl)phenyl ]amino}-1,3-dimethyl-2-oxo-2,3-dihydropyrido [2,3-b]pyrazin-4(1H)-yl]piperidin-1-carboxylat | Intermediat 52, Amin 2, Allgemeine Synthesevor schrift B | (300 MHz, DMSO-d6): δ =1.04 (d, 3H); 1.41 (s, 9H); 1.52 - 1.75 (m, 3H); 1.97 (d, 1H); 2.60 (s, 6H); 2.78 - 3.01 (m, 2H); 3.20 (s, 3H); 4.05 (bs, 2H); 4.20 (q, J=6.53Hz, 1H); 4.39 (t, 1H); 6.27 (d, 1H); 7.13 (d, 1H); 7.29 (d, 1H); 7.49 (t, 1H); 7.85 (s, 1H); 8.03 (d, 1H); 9.23 (s, 1H). |
| **62** | 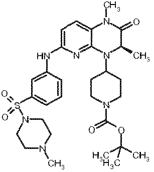 | *tert*-Butyl-4-[(3*R*)-1,3-dimethyl-6-({3-[(4-methylpiperazin-1-yl)sulfonyl]phenyl}amino) -2-oxo-2,3-dihydropyrido | Intermediat 52, Intermediat 110, Allgemeine | (300 MHz, DMSO-d6): δ = 1.05 (d, 3H); 1.41 (s, 9H); 1.49 - 1.80 (m, 3H); 1.90 - 2.05 (m, 1H); 2.12 (s, 3H); 2.34 (bs, 4H); 2.86 (bs, 6H); 3.20 (s, |
| | | [2,3-b]pyrazin-4(1H)-yl]piperidin-1-carboxylat | Synthesevor schrift B | 3H); 3.96 - 4.13 (m, 2H); 4.20 (q, 1H); 4.38 (br. t, 1H); 6.27 (d, 1H); 7.12 (d, 1H); 7.29 (d, 1H); 7.49 (t, 1H); 7.84 (br. s, 1H); 8.01 (br. d, 1H); 9.23 (s, 1H). |
| **63** | 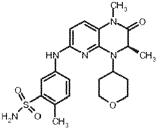 | 5-{[(3*R*)-1,3-Dimethyl-2-oxo-4-(tetrahydro-2H-pyran-4-yl)-1,2,3,4-tetrahydropyrido[2,3-b]pyrazin-6-yl]amino}-2-methylbenzolsulfonamid | Intermediat 10, Amin 12, Allgemeine Synthesevor schrift B | (400 MHz, DMSO-d6): δ = 1.07 (d, 3H); 1.53 - 1.61 (m, 1H); 1.67 - 1.79 (m, 1H); 1.81 - 1.90 (m, 1H); 1.90 - 1.98 (m, 1H); 2.50 - 2.54 (m, 2H); 3.19 (s, 3H); 3.46 - 3.56 (m, 2H); 3.87 - 3.98 (m, 2H); 4.21 (q, 1H); 4.46 (tt, 1H); 6.24 (d, 1H); 7.18 - 7.22 (m, 3H); 7.25 (d, 1H); 7.89 (dd, 1H); 7.95 (d, 1H); 9.00 (s, 1H). |
| **64** | 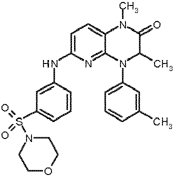 | 1,3-Dimethyl-4-(3-methylphenyl)-6-{[3-(morpholin-4-ylsulfonyl)phenyl]amino}-3,4-dihydropyrido[2,3-b]pyrazin-2(1H)-on | Intermediat 77, Amin 6, Allgemeine Synthesevor schrift B | (400 MHz, DMSO-d6): δ = 1.30 (d, 3H); 2.33 (s, 3H); 2.63 - 2.87 (m, 4H); 3.33 (s, 3H); 3.60 (t, 4H); 4.55 (q, 1H); 6.42 (d, 1H); 7.01 - 7.11 (m, 3H); 7.15 (d, 1H); 7.19 (s,1H); 7.33 (t, 1H); 7.37 (br. s, 1H); 7.45 (d, 1H); 7.99 (d, 1H); 9.30 (s, 1H). |
| **65** | 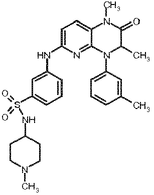 | 3-{[1,3-Dimethyl-4-(3-methylphenyl)-2-oxo-1,2,3,4-tetrahydro-pyrido[2,3-b]pyrazin-6-yl]amino}-*N*-(1-methylpiperidin-4-yl)benzolsulfonamid | Intermediat 77, Intermediat 92, Allgemeine Synthesevor schrift B | (300 MHz, DMSO-d6): δ = 1.28 (d, 3H); 2.11 (s, 3H); 2.31 (s, 7H); 2.79 (d, 4H); 3.29 (s, 3H); 4.54 (d, 1H); 6.40 (d, 1H); 6.97 - 7.08 (m, 3H); 7.10 - 7.20 (m, 2H); 7.28 - 7.37 (m, 2H); 7.43 (d, 1H); 7.96 (d, 1H); 9.27 (s, 1H). |
| **66** | 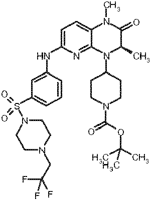 | *tert*-Butyl-4-[(3*R*)-1,3-dimethyl-2-oxo-6-[(3- {[4-(2,2,2-trifluorethyl) piperazin-1-yl]sulfonyl} phenyl)amino]-2,3-dihydropyrido[2,3-b]pyrazin-4(1H)-yl]piperidin-1-carboxylat | Intermediat 52, Intermediat 108, Allgemeine Synthesevor schrift B | (400 MHz, DMSO-d6): δ = 1.06 (d, 3H); 1.42 (s, 9H); 1.51 - 1.66 (m, 2H); 1.67 - 1.80 (m, 1H); 1.92 - 2.06 (m, 1H); 2.61 - 2.77 (m, 4H); 2.80 - 3.02 (m, 6H); 3.14 - 3.26 (m, 5H); 3.99 - 4.14 (m, 2H); 4.22 (q, 1H); 4.40 (br. t, 1H); 6.29 (d, 1H); 7.13 (d, 1H); 7.31 (d, 1H); 7.51 (s, 1H); 7.85 (br. s, 1H); 8.05 (br. d, 1H); 9.25 (s, 1H). |
| **67** | 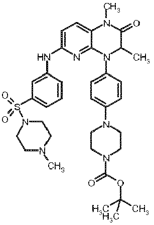 | *tert*-Butyl-4-{4-[1,3-dimethyl-6-({3-[(4-methylpiperazin-1-yl)sulfonyl]phenyl}amino) -2-oxo-2,3-dihydropyrido[2,3-b]pyrazin-4(1H)-yl]phenyl}piperazin-1-carboxylat | Intermediat 84, Intermediat 110, Allgemeine Synthesevor schrift B | (400 MHz, DMSO-d6): δ = 1.28 (d, 3H); 1.44 (s, 10H); 2.13 (d, 6H); 3.13 - 3.19 (m, 4H); 3.29 (s, 3H); 3.48 - 3.54 (m, 4H); 4.45 (q, 1H); 6.30 (d, 1H); 6.78 (ddd, 1H); 6.82 (ddd, 1H); 6.89 (t, 1H); 6.99 - 7.03 (m, 2H); 7.03 - 7.08 (m, 2H); 7.19 - 7.27 (m, 3H); 7.29 (br. s, 1H); 7.37 (d, 1H); 7.93 - 7.98 (m, 1H); 9.22 (s, 1H). |
| **68** | 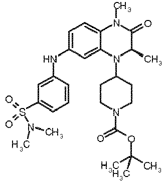 | *tert*-Butyl-4-[(2*R*)-7-{[3-(dimethylsulfamoyl)phenyl ]amino}-2,4-dimethyl-3-oxo-3,4-dihydro-chinoxalin-1(2H)-yl]piperidin-1-carboxylat | Intermediat 52, Amin 2, Allgemeine Synthesevor schrift B | (300 MHz, CDC13): δ = 1.20 (d, 3H); 1.49 (s, 9H); 1.53 - 1.83 (m, 5H); 1.94 - 2.03 (m, 1H); 2.76 (s, 9H); 3.45 - 3.58 (m, 1H); 4.14 (q, 1H); 4.18 - 4.34 (m, 3H); 6.81 (d, 2H); 6.95 (d, 1H); 7.19 (s, 1H); 7.26 (d, 1H); 7.41 (d, 1H); 7.44 (s, 1H). |
| **69** | 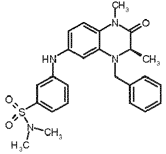 | 3-{[(3*R*)-4-Benzyl-1,3-dimethyl-2-oxo-1,2,3,4-tetrahydrochinoxalin-6-yl]amino}-*N,N-*dimethylbenzolsulfonamid | Intermediat 86, Amin 2, Allgemeine Synthesevor schrift B | (400 MHz, CDC13): δ = 1.19 (d, 3H); 2.72 - 2.76 (m, 6H); 3.43 (s, 3H); 4.06 (q, 1H); 4.18 (d, 1H); 4.50 (d, 1H); 5.80 (bs, 1H); 6.45 (d, 1H); 6.64 (dd, 1H); 6.92 (d, 2H); 7.17 - 7.27 (m, 2H); 7.30 - 7.40 (m, 6H). |
| **70** | 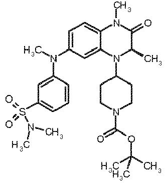 | *tert*-Butyl-4-[(2*R*)-7-{[3-(dimethylsulfamoyl)phenyl ](methyl)amino}-2,4-dimethyl-3-oxo-3,4-dihydrochinoxalin-1(2H)-yl]piperidin-1-carboxylat | Intermediat 88, Amin 3, Allgemeine Synthesevor schrift B | (300 MHz, CDC13): δ = 1.14 (d, 3H); 1.48 (s, 9H); 1.58 - 1.75 (m, 4H); 1.87 - 1.98 (m, 1H); 2.71 - 2.77 (m, 7H); 3.37 (s, 3H); 3.40 (s, 3H); 3.41 - 3.49 (m, 1H); 4.07 - 4.15 (m, 1H); 4.15 - 4.32 (m, 2H); 6.70 (s, 1H); 6.71 - 6.76 (m, 1H); 6.97 (d, 1H); 7.02 (dd, 1H); 7.18 (d, 1H); 7.22 (d, 1H); 7.32 - 7.40 (m, 1H). |
| **71** | 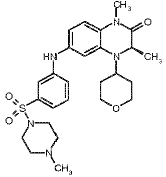 | (3*R)-*1,3-Dimethyl-6-({3-[(4-methylpiperazin-1-yl)sulfonyl]phenyl}amino) -4-(tetrahydro-2H-pyran-4-yl)-3,4-dihydrochinoxalin-2(1H)-on | Intermediat 14, Intermediat 110, Allgemeine Synthesevor schrift B | (400 MHz, DMSO-d6): δ = 0.97 (d, 3H); 1.54 - 1.61 (m, 1H); 1.63 - 1.80 (m, 2H); 1.81 - 1.88 (m, 1H); 2.13 (s, 3H); 2.35 (t, 4H); 2.88 (bs, 4H); 3.24 (s, 3H); 3.36 - 3.43 (m, 2H); 3.60 (tt, 1H); 3.85 - 3.96 (m, 2H); 4.06 (q, 1H); 6.67 (dd, 1H); 6.72 (d, 1H); 7.00 - 7.04 (m, 2H); 7.24 (dd, 1H); 7.29 (t, 1H); 7.43 (t, 1H); 8.46 (s, 1H). |

**Tabelle 3: Folgende Beispiele wurden entsprechend der in Beispiel 1 beschriebenen allgemeinen Synthesevorschrift A aus den jeweiligen Intermediaten und Aminen (Tabelle 1) hergestellt:**

| Bsp | Struktur | Name | Intermediat/ Amin | Analytische Daten: ¹H-NMR |
|---|---|---|---|---|
| **72** | 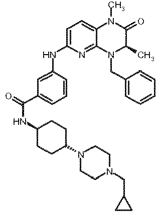 | 3-{[(3R)-4-Benzyl-1,3 - dimethyl-2-oxo-1,2,3,4-tetrahydropyrido[2,3-b]pyrazin-6-yl]amino}-*N-*{trans-4- [4-(cyclopropylmethyl)piperazin-1-yl]cyclohexyl}benzamid | Intermediat 43, Amin 13 | (400 MHz, DMSO-d6): δ = 0.01 - 0.06 (m, 2H); 0.40 - 0.46 (m, 2H); 0.72 - 0.84 (m, 1H); 1.10 (d, 3H); 1.15 - 1.29 (m, 4H); 1.69 - 1.82 (m, 4H); 2.03 - 2.09 (m, 1H); 2.12 (d, 2H); 2.40 (bs, 4H); 2.47 (bs, 4H); 3.22 (s, 3H); 3.55 - 3.67 (m, 1H); 3.93 (q, 1H); 4.23 (d, 1H); 5.26 (d, 1H); 6.26 (d, 1H); 7.14 - 7.21 (m, 2H); 7.23 - 7.37 (m, 6H); 7.56 (dt, 1H); 8.03 (d, 1H); 8.08 (s, 1H); 8.91 (s, 1H). |
| **73** | 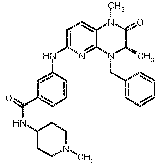 | 3-{[(3R)-4-Benzyl-1,3-dimethyl-2-oxo-1,2,3,4-tetrahydropyrido[2,3-b]pyrazin-6-yl]amino}*-N-*(1-methylpiperidin-4-yl)benzamid | Intermediat 43, Amin 14 | (400 MHz, DMSO-d6): δ = 1.11 (d, 3H); 1.42 - 1.57 (m, 2H); 1.58 - 1.70 (m, 2H); 1.83 - 1.94 (m, 2H); 2.13 (s, 3H); 2.65 - 2.74 (m, 2H); 3.22 (s, 3H); 3.60 - 3.69 (m, 1H); 3.97 (q, 1H); 4.24 (d, 1H); 5.24 (d, 1H); 6.26 (d, 1H); 7.12 - 7.36 (m, 8H); 7.61 (dt, 1H); 8.03 (br. s, 1H); 8.08 (d, 1H); 8.90 (s, 1H). |
| **74** | 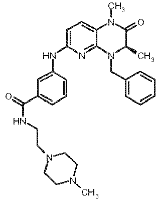 | 3-{[(3*R*)-4-Benzyl-1,3-dimethyl-2-oxo-1,2,3,4-tetrahydropyrido[2,3-b]pyrazin-6-yl]amino}-*N-*[2-(4-methylpiperazin-1-yl)ethyl]benzamid | Intermediat 43, Amin 15 | (400 MHz, DMSO-d6): δ = 1.11 (d, 3H); 2.12 (s, 3H); 2.19 - 2.32 (m, 4H); 2.37 (t, 5H); 3.22 (s, 3H); 3.25 - 3.31 (m, 3H); 3.98 (q, 1H); 4.25 (d, 1H); 5.26 (d, 1H); 6.26 (d, 1H); 7.14 - 7.21 (m, 2H); 7.21 - 7.37 (m, 6H); 7.62 (dt, 1H); 8.02 - 8.05 (m, 1H); 8.18 (t, 1H); 8.91 (s, 1H). |
| **75** | 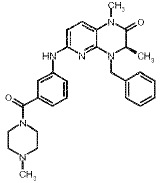 | (3*R*)-4-Benzyl-1,3-dimethyl-6-({3-[(4-methylpiperazin-1-yl)carbonyl]phenyl}amino )-3,4-dihydropyrido[2,3-b]pyrazin-2(1H)-on | Intermediat 43, Amin 16 | (400 MHz, DMSO-d6): δ = 1.08 - 1.13 (m, 3H); 2.09 - 2.12 (m, 3H); 2.14 - 2.28 (m, 4H); 3.22 (s, 3H); 3.24 - 3.31 (m, 2H); 3.40 - 3.55 (m, 2H); 3.97 (q, 1H); 4.25 (d, 1H); 5.20 (d, 1H); 6.25 (d, 1H); 6.72 (br. d, 1H); 7.16 (t, 1H); 7.22 - 7.29 (m, 2H); 7.31- 7.3 (m, 4H); 7.46 (dd, 1H); 7.66 (t, 1H); 8.92 (s, 1H). |
| **76** | 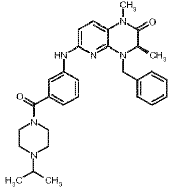 | (3*R*)-4-Benzyl-6-({3-[(4-isopropylpiperazin-1-yl)carbonyl]phenyl}amino )-1,3-dimethyl-3,4-dihydropyrido[2,3-b]pyrazin-2(1H)-on | Intermediat 43, Amin 17 | (400 MHz, DMSO-d6): δ = 0.92 (d, 6H); 1.11 (d, 3H); 2.25 - 2.43 (m, 4H); 2.50 - 2.54 (m, 1H); 2.55 - 2.63 (m, 1H); 3.22 (s, 3H); 3.24 - 3.29 (m, 1H); 3.36 - 3.59 (m, 2H); 3.99 (q, 1H); 4.26 (d, 1H); 5.20 (d, 1H); 6.25 (d, 1H); 6.72 (br. d, 1H); 7.15 (t, 1H); 7.20 - 7.36 (m, 6H); 7.49 (dd, 1H); 7.60 (t, 1H); 8.91 (s, 1H). |
| **77** | 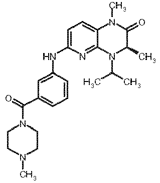 | (3*R*)-4-Isopropyl-1,3-dimethyl-6-({3-[(4-methylpiperazin-1-yl)earbonyl]phenyl} amino )-3,4-dihydropyrido[2,3-b]pyrazin-2(1H)-on | Intermediat 35, Amin 17 | (400 MHz, DMSO-d6): δ = 1.08 (d, 3H); 1.23 (d, 3H); 1.29 (d, 3H); 2.17 (s, 3H); 2.19 - 2.39 (m, 4H); 3.19 (s, 3H); 3.34 (d, 2H); 3.49 - 3.66 (m, 2H); 4.23 (q, 1H); 4.57 (spt, 1H); 6.22 (d, 1H); 6.75 (d, 1H); 7.25 (t, 2H); 7.46 (dd, 1H); 7.92 (t, 1H); 8.92 (s, 1H). |
| **78** | 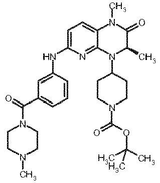 | *tert*-Butyl-4-[(3R)-1,3-dimethyl-6-({3-[(4-methylpiperazin-1-yl)carbonyl]phenyl}amino )-2-oxo-2,3-dihydro-pyrido[2,3-b]pyrazin-4(1H)-yl]piperidin-1-carboxylat | Intermediat 54, Amin 16 | (300 MHz, DMSO-d6): δ = 1.05 (d, 3H); 1.41 (s, 9H); 1.45 - 1.64 (m, 2H); 1.64 - 1.81 (m, 1H); 1.90 - 2.00 (m, 1H); 2.18 (s, 3H); 2.29 (bs, 4H); 2.66 - 2.90 (m, 2H); 3.19 (s, 3H); 3.35 (bs, 2H); 3.56 (bs, 2H); 4.06 (bs, 2H); 4.20 (q, 1H); 4.36 (br. t, 1H), 6.25 (d, 1H); 6.76 (d, 1H); 7.22 - 7.29 (m, 2H); 7.41 (br. d, 1H); 7.97 (br. s, 1H); 8.99 (s, 1H). |
| **79** | 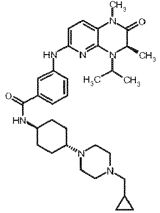 | *N*-{*trans*-4-[4-(Cyclopropylmethyl)pipera zin-1-yl]cyclohexyl}-3-{[(3*R*)-4-isopropyl-1,3-dimethyl-2-oxo-1,2,3,4-tetrahydropyrido[2,3-b]pyrazin-6-yl]amino} benzamid | Intermediat 35, Amin 13 | (400 MHz, DMSO-d6): δ = 0.00 - 0.07 (m, 2H); 0.40 - 0.45 (m, 2H); 0.72 - 0.84 (m, 1H); 1.08 (d, 3H); 1.22 (d, 3H); 1.26 - 1.39 (m, 6H); 2.12 (d, 2H); 2.14 - 2.22 (m, 1H); 2.33 - 2.45 (m, 3H); 3.19 (s, 3H); 3.62 - 3.73 (m, 1H); 4.22 (q, 1H); 4.61 (spt, 1H); 6.21 (d,1H); 7.20 - 7.27 (m, 3H); 7.72 (dt, 1H); 8.01 (br. s, 1H); 8.04 (d, 1H); 8.86 (s, 1H). |
| **80** | 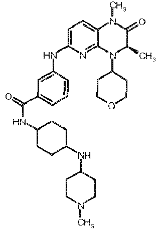 | 3-{[(3*R*)-1,3-Dimethyl-2-oxo-4-(tetrahydro-2H-pyran-4-yl)-1,2,3,4-tetrahydropyrido[2,3-b]pyrazin-6-yl]amino}-*N-*{4-[(1-methylpiperidin-4-yl)amino]cyclohexyl}benz amid | Intermediat 18, Intermediat 118 | (300 MHz, DMSO-d6): δ = 0.95 - 1.04 (m, 1H); 1.07 (d, 3H); 1.11 - 1.41 (m, 4H); 1.42-1.64 (m, 3H); 1.71 (d, 3H); 1.84 (t, 8H); 2.10 (s, 3H); 2.67 (d, 2H); 3.19 (s, 3H); 3.38 - 3.53 (m, 4H); 3.60 - 3.77 (m, 1H); 3.90 (br. d, 2H); 4.21 (q, 1H); 4.43 (br. t, 1H); 6.23 (d, 1H); 7.25 (d, 3H); 7.71 - 7.78 (m, 1H); 7.96 (br. s, 1H); 8.08 (br. d, 1H); 8.93 (s, 1H). |
| **81** | 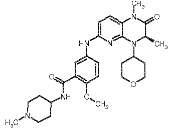 | 5-{[(3*R*)-1,3-Dimethyl-2-oxo-4-(tetrahydro-2H-pyran-4-yl)-1,2,3,4-tetrahydropyrido[2,3-b]pyrazin-6-yl]amino}-2-methoxy-*N*-(1-methylpiperidin-4-yl)benzamid | Intermediat 79, Amin 14 | (400 MHz, DMSO-d6): δ = 1.06 (d, 3H); 1.55 (d, 2H); 1.62 - 1.75 (m, 1H); 1.83 (d, 3H); 1.87 - 1.94 (m, 1H); 2.08 - 2.17 (m, 2H); 2.20 (s, 3H); 2.63 - 2.74 (m, 2H); 3.18 (s, 3H); 3.37 - 3.53 (m, 4H); 3.84 (s, 3H); 3.88 (bs, 2H); 4.19 (q, 1H); 4.44 (tt, 1H); 6.16 (d, 1H); 7.02 (d, 1H); 7.21 (d, 1H); 7.63 (dd, 1H); 7.99 - 8.04 (m, 2H); 8.71 (s, 1H). |
| **82** | 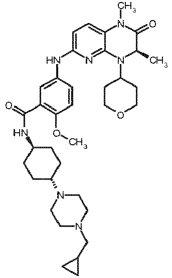 | *N-{trans-*4-[4-(Cyclopropylmethyl)pipera zin-1-yl]cyclohexyl}-5-{[(3*R*)-1,3-dimethyl-2-oxo-4-(tetrahydro-2H-pyran-4-yl)-1,2,3,4-tetrahydropyrido[2,3-b]pyrazin-6-yl]amino}-2-methoxybenzamid | Intermediat 79, Amin 13 | (400 MHz, DMSO-d6): δ = 0.01 - 0.08 (m, 2H); 0.40 - 0.48 (m, 2H); 0.80 (bs, 1H); 1.06 (d, 3H); 1.23 - 1.35 (m, 4H); 1.52 - 1.59 (m, 1H); 1.62 - 1.75 (m, 1H); 1.79 - 1.90 (m, 3H); 1.93 (d, 2H); 2.16 (d, 2H); 2.19 - 2.28 (m, 1H); 3.18 (s, 3H); 3.35 - 3.53 (m, 4H); 3.54 - 3.74 (m, 8H); 3.83 (s, 3H); 3.89 (dd, 2H); 4.19 (q, 1H); 4.43 (tt, 1H); 6.15 (d, 1H); 7.00 (d, 1H); 7.21 (d, 1H); 7.64 (dd, 1H); 7.92 (d, 1H); 7.98 (d, 1H); 8.70 (s, 1H). |
| **83** | 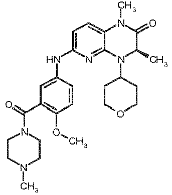 | (3*R*)-6-({4-Methoxy-3-[(4-methylpiperazin-1-yl)carbonyl]phenyl} amino )-1,3-dimethyl-4-(tetrahydro-2H-pyran-4-yl)-3,4-dihydropyrido[2,3-b]pyrazin-2(1H)-on | Intermediat 79, Amin 16 | (400 MHz, DMSO-d6): δ = 1.06 (d, 3H); 1.51 - 1.59 (m, 1H); 1.67 - 1.80 (m, 1H); 1.81 - 1.92 (m, 2H); 2.15 - 2.19 (m, 4H); 2.23 - 2.36 (m, 3H); 3.11 - 3.15 (m, 1H); 3.18 (s, 3H); 3.41 - 3.48 (m, 3H); 3.54 - 3.63 (m, 2H); 3.73 (s, 3H); 3.88 - 3.98 (m, 2H); 4.20 (q, 1H); 4.41 (tt, 1H); 6.17 (d, 1H); 6.96 (d, 1H); 7.22 (d, 1H); 7.38 - 7.48 (m, 1H); 7.64 - 7.70 (m, 1H); 8.69 (s, 1H). |
| **84** | 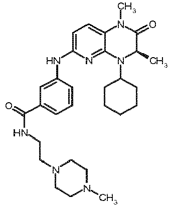 | 3-{[(3*R*)-4-Cyclohexyl-1,3-dimethyl-2-oxo-1,2,3,4-tetrahydropyrido[2,3-b]pyrazin-6-yl]amino}-*N*-[2-(4-methylpiperazin-1-yl)ethyl]benzamid | Intermediat 30, Amin 15 | (300 MHz, DMSO-d6): δ = 1.06 (d, 3H); 1.09 - 1.51 (m, 5H); 1.54 - 1.68 (m, 3H); 1.75 (d, 2H); 1.99 (bs, 1H); 2.12 (s, 3H); 2.29 (bs, 4H); 2.38 - 2.46 (m, 5H); 3.19 (s, 3H); 3.34 (bs, 2H); 4.15 - 4.29 (m, 2H); 6.20 (d, 1H); 7.18 - 7.29 (m, 3H); |
| | | | | 7.82 (br. d, 1H); 7.94 (s, 1H); 8.23 (t, 1H); 8.91 (s, 1H). |
| **85** | 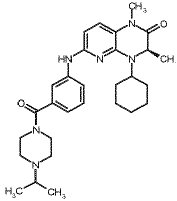 | (3*R*)-4-Cyclohexyl-6-({3-[(4-isopropylpiperazin-1-yl)carbonyl]phenyl}amino )-1,3-dimethyl-3,4-dihydropyrido[2,3-b]pyrazin-2(1H)-on | Intermediat 30, Amin 17 | (400 MHz, DMSO-d6): δ = 0.95 (d, 6H); 1.06 (d, 3H); 1.10 - 1.24 (m, 1H); 1.27 - 1.51 (m, 3H); 1.60 (d, 3H); 1.77 (bs, 2H); 1.96 - 2.04 (m, 1H); 2.33 - 2.47 (m, 4H); 2.66 (spt, 1H); 3.19 (s, 3H); 3.31-3.41 (m, 2H); 3.48 - 3.64 (m, 2H); 4.17 - 4.26 (m, 2H); 6.21 (d, 1H); 6.76 (br. d, 1H); 7.21 - 7.26 (m, 2H); 7.57 (dd, 1H); 7.80 (t, 1H); 8.92 (s, 1H). |
| **86** | 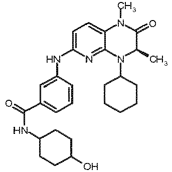 | 3-{[(3*R*)-4-Cyclohexyl-1,3-dimethyl-2-oxo-1,2,3,4-tetrahydropyrido[2,3-b]pyrazin-6-yl]amino}-*N*-(4-hydroxycyclohexyl)benza mid | Intermediat 30, Amin 14 | (400 MHz, DMSO-d6): δ = 1.12 - 1.28 (m, 3H); 1.28 - 1.46 (m, 5H); 1.54 - 1.70 (m, 3H); 1.72 - 1.87 (m, 6H); 1.97 - 2.05 (m, 1H); 3.18 (s, 3H); 3.32 - 3.41 (m, 1H); 3.61 - 3.74 (m, 1H); 4.16 - 4.24 (m, 2H); 4.52 (d, 1H); 6.20 (d, 1H); 7.19 - 7.26 (m, 3H); 7.84 - 7.90 (m, 2H); 8.02 (d, 1H); 8.88 (s, 1H). |
| **87** | 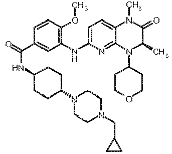 | N-{trans-4-[4-(Cyclopropylmethyl)pipera zin-1-yl]cyclohexyl}-3-{[(3R)-1,3-dimethyl-2-oxo-4-(tetrahydro-2H-pyran-4-yl)-1,2,3,4-tetrahydropyrido[2,3-b]pyrazin-6-yl]amino} -4-methoxybenzamid | Intermediat 20, Amin 13 | (400 MHz, DMSO-d6): δ = 0.22 - 0.27 (m, 2H); 0.34 - 0.39 (m, 2H); 1.06 (d, 3H); 1.21 - 1.39 (m, 5H); 1.47 - 1.64 (m, 4H); 1.73 - 1.89 (m, 7H); 2.12 - 2.22 (m, 1H); 2.43 (bs, 4H); 3.19 (s, 3H); 3.21 - 3.27 (m, 2H); 3.39 - 3.47 (m, 1H); 3.62 - 3.71 (m, 1H); 3.75 (d, 1H); 3.79 - 3.85 (m, 1H); 3.88 (s, 3H); 4.18 (q, 1H); 4.42 (t, 1H); 6.43 (d, 1H); 6.97 (d, 1H); 7.23 (d, 1H); 7.36 (dd, 1H); 7.86 (s, 1H); 7.94 (d, 1H); 8.51 (d, 1H). |
| **88** | 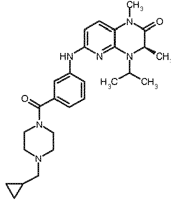 | (3*R*)-6-[(3-{[4-(Cyclopropylmethyl) piperazin-1-yl]carbonyl}phenyl) amino]-4-isopropyl-1,3-dimethyl-3,4-dihydropyrido[2,3-b]pyrazin-2(1H)-on | Intermediat 35, Amin 22 | (400 MHz, DMSO-d6): δ = 0.05 (d, 2H); 0.41 - 0.47 (m, 2H); 0.75 - 0.87 (m, 1H); 1.08 (d, 3H); 1.23 (d, 3H); 1.29 (d, 3H); 1.43 - 1.54 (m, 1H); 2.19 (d, 2H); 2.28 - 2.45 (m, 4H); 3.19 (s, 3H); 3.49 - 3.69 (m, 3H); 4.23 (q, 1H); 4.57 (spt, 1H); 6.22 (d, 1H); 6.75 (br. d, 1H); 7.22 - 7.28 (m, 2H); 7.47 (dd, 1H); 7.91 (br. s, 1H); 8.92 (s, 1H). |
| **89** | 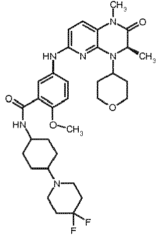 | N-[4-(4,4-Difluorpiperidin-1-yl)cyclohexyl]-5-{[(3*R*)-1,3-dimethyl-2-oxo-4-(tetrahydro-2H-pyran-4-yl)-1,2,3,4-tetrahydropyrido[2,3-b]pyrazin-6-yl]amino}-2-methoxybenzamid | Intermediat 79, Intermediat 120 | (400 MHz, DMSO-d6): δ = 1.22 - 1.44 (m, 2H); 1.48 - 1.63 (m, 5H); 1.65 - 1.84 (m, 4H); 1.86 - 1.99 (m, 6H); 2.38 - 2.45 (m, 1H); 2.56 - 2.64 (m, 4H); 3.18 (s, 3H); 3.36 - 3.57 (m, 2H); 3.65 - 3.74 (m, 1H*); 3.83 (s, 1H); 3.85 - 3.92 (m, 4H); 4.01-4.10 (m, 1H); 4.19 (q, 1H); 4.38-4.50 (m, 1H); 6.15 (d,1H*); 6.16 (d,1H); 7.00 (d, 1H*); 7.05 (d, 1H); 7.21 (d, 1H*); 7.22 (d, 1H); 7.62 (dd, 1H); 7.63 (dd, 1H*); 7.92 (d, 1H*); 7.98 (d, 1H*); 8.08 (d, 1H); 8.17 (d, 1H); 8.70 (s, 1H*); 8.72 (s, 1H); Diastereomere (2:1), *) Nebendiastereomer. |
| **90** | 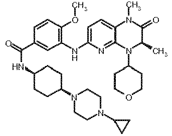 | *N-*[*cis-*4-(4-Cyclopropylpiperazin-1-yl)cyclohexyl]-3-1[(3*R*)-1,3-dimethyl-2-oxo-4-(tetrahydro-2H-pyran-4-yl)-1,2,3,4-tetrahydropyrido[2,3-b]pyrazin-6-yl]amino}-4-methoxybenzamid | Intermediat 20, Intermediat 122 | (400 MHz, DMSO-d6): δ = 0.23 - 0.28 (m, 2H); 0.34 - 0.41 (m, 2H); 1.04 - 1.08 (m, 3H); 1.10 (s, 2H); 1.12 - 1.66 (m, 9H); 1.68 - 1.89 (m, 7H); 2.06 - 2.12 (m, 1H); 2.35 - 2.43 (m, 3H); 3.19 (s, 3H); 3.22 - 3.28 (m, 2H); 3.39 - 3.50 (m, 1H); 3.72 - 3.78 (m, 1H); 3.79 - 3.85 (m, 1H); 3.88 (s, 3H); 4.18 (q, 1H); 4.43 (tt, 1H); 6.44 (d, 1H); 6.97 (d, 1H); 7.23 (d, 1H); 7.40 (dd, 1H); 7.84 - 7.87 (m, 1H); 7.90 (d, 1H); 8.53 (d, 1H). |
| **91** | 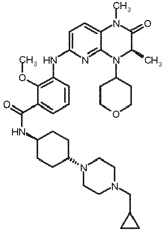 | *N-*{*trans-*4-[4-(Cyclopropylmethyl)pipera zin-1-yl]cyclohexyl}-3-{[(3*R*)-1,3-dimethyl-2-oxo-4-(tetrahydro-2H-pyran-4-yl)-1,2,3,4-tetrahydropyrido[2,3-b]pyrazin-6-yl]amino}-2-methoxybenzamid | Intermediat 81, Amin 13 | (400 MHz, DMSO-d6): δ = 0.05 (q, 2H); 0.41 - 0.46 (m, 2H); 0.75 - 0.84 (m, 1H); 1.08 (d, 3H); 1.23 - 1.36 (m, 4H); 1.59 (br. d, 1H); 1.69 - 1.98 (m, 7H); 2.15 (d, 2H); 2.19 (d, 1H); 2.44 (bs, 3H); 2.53 (bs, 1H); 3.19 (s, 3H); 3.32 - 3.46 (m, 5H); 3.52 (bs, 2H); 3.71 (s, 3H); 3.91 - 4.01 (m, 2H); 4.22 (q, 1H); 4.33 (tt, 1H); 6.55 (d, 1H); 6.93 (dd, 1H); 7.04 (t, 1H); 7.25 (d, 1H); 8.02 (d, 1H); 8.08 (s, 1H); 8.35 (dd, 1H). |
| **92** | 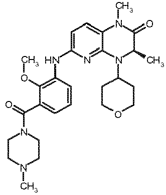 | (3*R*)-6-({2-Methoxy-3-[(4-methylpiperazin-1-yl)carbonyl]phenyl} amino)-1,3-dimethyl-4-(tetrahydro-2H-pyran-4-yl)-3,4-dihydropyrido[2,3-b]pyrazin-2(1H)-on | Intermediat 81, Amin 16 | (400 MHz, DMSO-d6): δ = 1.08 (d, 3H); 1.60 (d, 1H); 1.70 - 1.83 (m, 1H); 1.88 - 2.00 (m, 2H); 2.40 (s, 3H); 2.52 - 2.59 (m, 2H); 2.60 - 2.72 (m, 2H); 3.20 (s, 3H); 3.27 (bs, 2H); 3.33 - 3.49 (m, 4H); 3.67 - 3.76 (m, 5H); 3.97 (bs, 2H); 4.23 (q, 1H); 4.35 (bs, 1H); 6.57 (d, 1H); 6.71 (dd, 1H); 7.06 (t, 1H); 7.23 - 7.29 (m, 1H); 8.11 (s, 1H); 8.39 (dd, 1H). |
| **93** | 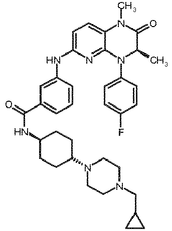 | *N*-{*trans*-4-[4-(Cyclopropylmethyl)pipera zin-1-yl]cyclohexyl}-3-{[(3*R*)-4-(4-fluorphenyl)-1,3-dimethyl-2-oxo-1,2,3,4-tetrahydropyrido [2,3-b]pyrazin-6-yl]amino}benzamid | Intermediat 74, Amin 13 | (400 MHz, DMSO-d6): δ = 0.01 - 0.06 (m, 2H); 0.40 - 0.46 (m, 2H); 0.73 - 0.86 (m, 2H); 0.87 - 1.17 (m, 2H); 1.18 - 1.38 (m, 10H); 1.83 (bs, 4H); 2.12 (d, 2H); 2.13 - 2.22 (m, 1H); 2.33 - 2.47 (m, 6H); 3.60 - 3.73 (m, 1H); 4.49 (d, 1H); 6.36 (d, 1H); 6.89 (t, 1H); 7.14 (br. d, 1H); 7.22 (t, 2H); 7.34 - 7.40 (m, 3H); 7.43 (s, 1H); 7.58 (dd, 1H); 8.91 (s, 1H). |
| **94** | 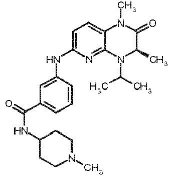 | 3-{[(3R)-4-Isopropyl-1,3-dimethyl-2-oxo-1,2,3,4-tetrahydropyrido[2,3-b]pyrazin-6-yl]amino}-*N-*(1-methylpiperidin-4-yl)benzamid | Intermediat 35, Amin 14 | (400 MHz, DMSO-d6): δ = 1.08 (d, 3H); 1.22 (d, 3H); 1.28 (d, 3H); 1.51 - 1.63 (m, 2H); 1.75 (d, 2H); 2.02 (t, 2H); 2.19 (s, 3H); 2.80 (d, 2H); 3.19 (s, 3H); 3.66 - 3.76 (m, 1H); 4.22 (q, 1H); 4.62 (spt, 1H); 6.22 (d, 1H); 7.20 - 7.28 (m, 3H); 7.72 (dt, 1H); 8.03 (br. s, 1H); 8.10 (d, 1H); 8.87 (s, 1H). |
| **95** | 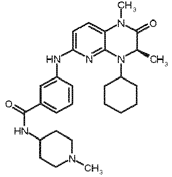 | 3-{[(3*R*)-4-Cyclohexyl-1,3-dimethyl-2-oxo-1,2,3,4-tetrahydropyrido [2,3-b]pyrazin-6-yl]amino}-*N*-(1-methylpiperidin-4-yl)benzamid | Intermediat 30, Amin 14 | (400 MHz, DMSO-d6): δ = 1.06 (d, 3H); 1.09 - 1.23 (m, 1H); 1.28 - 1.48 (m, 3H); 1.50 - 1.66 (m, 5H); 1.67 - 1.81 (m, 4H); 1.86 - 1.96 (m, 2H); 2.01 (d, 1H); 2.14 (s, 3H); 2.76 (s, 1H); 2.73 (s, 1H); 3.18 (s, 3H); 3.63 - 3.75 (m, 1H); 4.15 - 4.26 (m, 2H); 6.20 (d, 1H); 7. 18 - 7.28 (m, 3H); 7.82 - 7.92 (m, 2H); 8.10 (d, 1H); 8.89 (s, 1H). |
| **96** | 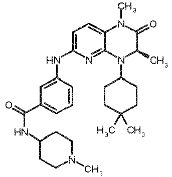 | 3-{[(3R)-4-(4,4-Dimethylcyclohexyl)-1,3-dimethyl-2-oxo-1,2,3,4-tetrahydropyrido[2,3-b]pyrazin-6-yl]amino}-*N-*(1-methylpiperidin-4-yl)benzamid | Intermediat 57, Intermediat 124, Allgemeine Synthesevor schrift B | (400 MHz, DMSO-d6): δ = 0.93 (d, 6H); 1.07 (d, 3H); 1.35 (bs, 1H); 1.40 (bs, 4H); 1.45 (bs, 1H); 1.49 - 1.65 (m, 3H); 1.68 - 1.85 (m, 4H); 1.90 (t, 2H); 2.14 (s, 3H); 2.76 (s, 1H); 2.73 (s, 1H); 3.19 (s, 3H); 3.65 - 3.78 (m, 1H); 4.15 - 4.29 (m, 2H); 6.20 (d, 1H); 7.18 - 7.28 (m, 3H); 7.78 (dt, 1H); 7.92 - 7.98 (m, 1H); 8.08 (d, 1H); 8.88 (s, 1H). |
| **97** | 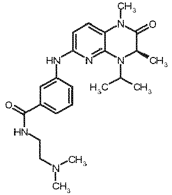 | *N*-[2-(Dimethylamino)ethyl]-3-{[(3R)-4-isopropyl-1,3-dimethyl-2-oxo-1,2,3,4-tetrahydropyrido[2,3-b]pyrazin-6-yl]amino}benzamid | Intermediat 35, Amin 19 | (400 MHz, DMSO-d6): δ = 1.08 (d, 3H); 1.22 (d, 3H); 1.29 (d, 3H); 2.20 (s, 6H); 2.43 (t, 2H); 3.19 (s, 3H); 3.30 - 3.37 (m, 2H); 4.22 (d, 1H); 4.65 (spt, 1H); 6.22 (d, 1H); 7.19 - 7.29 (m, 3H); 7.71 (d, 1H); 8.09 (br. s, 1H); 8.15 - 8.23 (m, 1H); 8.88 (s, 1H). |
| **98** | 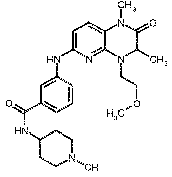 | 3-{[4-(2-Methoxyethyl)-1,3-dimethyl-2-oxo-1,2,3,4-tetrahydropyrido [2,3-b]pyrazin-6-yl]amino}-*N*-(1-methylpiperidin-4-yl)benzamid | Intermediat 49, Amin 14 | (300 MHz, DMSO-d6): δ = 1.13 (d, 3H); 1.49 - 1.64 (m, 2H); 1.67 - 1.80 (m, 2H); 1.86 - 2.00 (m, 2H); 2.15 (s, 3H); 2.75 (d, 2H); 3.20 (s, 3H); 3.21 (s, 3H); 3.23 - 3.28 (m, 1H); 3.50 - 3.60 (m, 2H); 3.62 - 3.78 (m, 1H); 4.04 - 4.20 (m, 2H); 6.21 (d, 1H); 7.18-7.28 (m, 3H); 7.65 - 7.72 (m, 1H); 8.04 (s, 1H); 8.09 (d, 1H); 8.88 (s, 1H). |
| **99** | 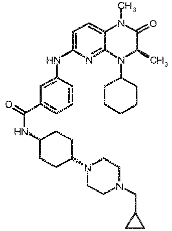 | 3-{[(3*R*)-4-Cyclohexyl-1,3-dimethyl-2-oxo-1,2,3,4-tetrahydropyrido [2,3-b]pyrazin-6-yl]amino}-*N*-{trans-4-[4-(cyclopropylmethyl)pipera zin-1-yl]cyclohexyl} benzamid | Intermediat 30, Amin 13 | (300 MHz, DMSO-d6): δ = - 0.01 - 0.08 (m, 2H); 0.37 - 0.47 (m, 2H); 0.71 - 0.84 (m, 1H); 1.06 (d, 3H); 1.19 - 1.38 (m, 8H); 1.52 - 1.68 (m, 3H); 1.69 - 1.92 (m, 6H); 2.00 (t, 2H); 2.11 (d, 2H); 2.39 (bs, 4H); 2.53 (s, 4H); 3.18 (s, 3H); 3.67 (d, 1H); 4.20 (q, 2H); 6.20 (d, 1H); 7.18 - 7.26 (m, 3H); 7.82 - 7.92 (m, 2H); 8.07 (d, 1H); 8.90 (s, 1H). |
| **100** | 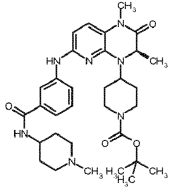 | *tert*-Butyl-4-[(3*R*)-1,3-dimethyl-6-({3-[(1-methylpiperidin-4-yl)carbamoyl]phenyl} amin o)-2-oxo-2,3-dihydropyrido[2,3-b]pyrazin-4(1H)-yl]piperidin-1-carboxylat | Intermediat 54, Amin 14 | (300 MHz, DMSO-d6): δ = 1.04 (d, 3H); 1.40 (s, 9H); 1.46 - 1.63 (m, 4H); 1.65 - 1.78 (m, 3H); 1.83 - 2.01 (m, 3H); 2.13 (s, 3H); 2.75 (s, 2H); 2.71 (s, 2H); 2.88 (bs, 1H); 3.19 (s, 3H); 3.59 - 3.76 (m, 1H); 4.00 (bs, 2H); 4.19 (q, 1H); 4.38 (t, 1H); 6.23 (d, 1H); 7.19 - 7.29 (m, 3H); 7.57 (d, 1H); 8.09 - 8.19 (m, 2H); 8.94 (s, 1H). |
| **101** | 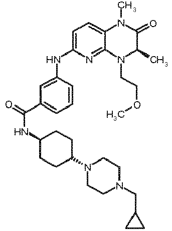 | *N-*{*trans-*4-[4-(Cyclopropylmethyl)pipera zin-1-yl]cyclohexyl}-3-{[(3*R*)-4-(2-methoxyethyl) -1,3-dimethyl-2-oxo-1,2,3,4-tetrahydropyrido [2,3-b]pyrazin-6-yl]amino}benzamid | Intermediat 49, Amin 13 | (300 MHz, DMSO-d6): δ = 0.04 (q, 2H); 0.38 - 0.48 (m, 2H); 0.72 - 0.87 (m, 2H); 1.12 (d, 3H); 1.20 - 1.43 (m, 5H); 1.74 - 1.96 (m, 4H); 2.12 (d, 2H); 2.18 (bs, 2H); 2.40 (bs, 4H); 3.17 - 3.23 (m, 6H); 3.23 - 3.30 (m, 2H); 3.55 (t, 2H); 3.69 (bs, 1H); 4.03 - 4.11 (m, 1H); 4.16 (q, 1H); 6.21 (d,1H); 7.17 - 7.29 (m, 3H); 7.69 dt, 1H); 8.03 (br. s, 1H); 8.07 (d, 1H); 8.90 (s, 1H). |
| **102** | 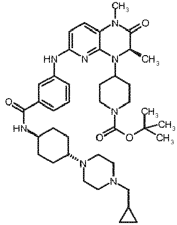 | *tert*-Butyl-4-[(3*R*)-6-{[3-({*trans*-4-[4-(cyclopropylmethyl)piperazin-1-yl]cyclohexyl}carbamoyl) phenyl]amino}-1,3-dimethyl-2-oxo-2,3-dihydropyrido[2,3-b]pyrazin-4(1H)-yl]piperidin-1-carboxylat | Intermediat 54, Amin 13 | (300 MHz, DMSO-d6): δ = - 0.01 - 0.07 (m, 3H); 0.44 (bs, 3H); 0.71 - 0.86 (m, 2H); 1.04 (d, 3H); 1.13 - 1.36 (m, 7H); 1.40 (s, 9H); 1.45 - 1.70 (m, 4H); 1.76 - 1.89 (m, 6H); 1.90 - 2.01 (m, 2H); 2.11 (d, 3H); 3.19 (s, 3H); 3.58 - 3.73 (m, 1H); 3.94 - 4.10 (m, 2H); 4.19 (q, 1H); 4.38 (br. t, 1H); 6.22 (d, 1H); 7.20 - 7.30 (m, 3H); 7.53 - 7.60 (m, 1H); 8.10(d, 1H); 8.14 (br. s, 1H); 8.90 - 8.97 (m, 1H). |
| **103** | 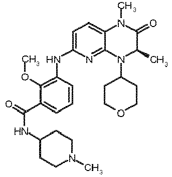 | 3-{[(3*R*)-1,3-Dimethyl-2-oxo-4-(tetrahydro-2H-pyran-4-yl)-1,2,3,4-tetrahydropyrido[2,3-b]pyrazin-6-yl]amino}-2-methoxy-*N*-(1-methylpiperidin-4-yl)benzamid | Intermediat 81, Amin 14 | (400 MHz, DMSO-d6): δ = 1.08 (d, 3H); 1.60 (d, 1H); 1.65 - 1.80 (m, 3H); 1.87 - 2.02 (m, 4H); 2.63 (s, 3H); 2.89 (bs, 2H); 3.20 (s, 3H); 3.24 (d, 2H); 3.34 - 3.46 (m, 4H); 3.72 (s, 3H); 3.92 - 4.01 (m, 3H); 4.22 (q, 1H); 4.34 (tt, 1H); 6.56 (d, 1H); 6.91 (dd, 1H); 7.06 (t, 1H); 7.26 (d, 1H); 8.08 (s, 1H); 8.27 (d, 1H); 8.38 (dd, 1H). |
| **104** | 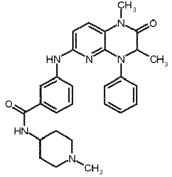 | 3-[(1,3-Dimethyl-2-oxo-4-phenyl-1,2,3,4-tetrahydropyrido[2,3-b]pyrazin-6-yl)amino]-*N-*(1-methylpiperidin-4-yl)benzamid | Intermediat 69, Intermediat 124, Allgemeine Synthesevor schrift B | (300 MHz, DMSO-d6): δ = 1.27 (d, 3H); 1.42 - 1.64 (m, 2H); 1.71 (d, 2H); 1.91 (t, 2H); 2.14 (s, 3H); 2.74 (d, 2H); 3.34 (bs, 3H); 3.61 - 3.74 (m, 1H); 4.54 (q, 1H); 6.40 (d, 1H); 6.88 (t, 1H); 7.10 - 7.22 (m, 2H); 7.30 - 7.45 (m, 6H); 7.71 (dd, 1H); 8.07 (d, 1H); 8.96 (s, 1H). |

**Tabelle 4: Folgende Beispiele wurden entsprechend der genannten allgemeinen Synthesevorschriften aus den jeweiligen Intermediaten und Aminen (Tabelle 1) hergestellt:**

| Bsp | Struktur | Name | Intermediat/ Amin, Allgemeine Synthesevor schrift | Analytische Daten: ¹H-NMR |
|---|---|---|---|---|
| **105** | 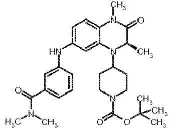 | tert-Butyl-4-[(2R)-7-{[3-(dimethylcarbamoyl)pheny 1]amino}-2,4-dimethyl-3-oxo-3,4-dihydrochinoxalin-1(2H)-yl]piperidin-1-carboxylat | Intermediat 88, Amin 21, Allgemeine Synthesevor schrift B | (400 MHz, DMSO-d6): δ = 0.97 (d, 3H); 1.41 (s, 9H); 1.61 (bs, 3H); 1.85 - 1.94 (m, 1H); 2.71 - 2.88 (m, 2H); 2.94 (bs, 6H); 3.24 (s, 3H); 3.47 - 3.58 (m, 1H); 3.95 - 4.11 (m, 3H); 6.65 (dd, 1H); 6.68 (d, 1H); 6.75 (br. d, 1H); 6.97 - 7.01 (m, 2H); 7.03 (dd, 1H); 7.25 (t, 1H); 8.17 (s, 1H). |
| **106** | 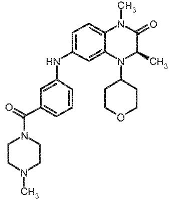 | (3*R*)-1,3-Dimethyl-6-({3-[(4-methylpiperazin-1-yl)carbonyl]phenyl} amino)-4-(tetrahydro-2H-pyran-4-yl)-3,4-dihydrochinoxalin-2(1H)-on | Intermediat 90, Amin 16, Allgemeine Synthesevor schrift A | (300 MHz, DMSO-d6): δ = 0.97 (d, 3H); 1.54 - 1.88 (m, 5H); 2.18 (s, 3H); 2.29 (bs, 4H); 3.23 (s, 3H); 3.31 - 3.44 (m, 5H); 3.83 - 3.96 (m, 3H); 4.05 (q, 1H); 6.61 - 6.66 (m, 1H); 6.67 (s, 1H); 6.71 (d, 1H); 6.94 - 7.00 (m, 2H); 7.01 - 7.07 (m, 1H); 7.19 - 7.28 (m, 1H); 8.18 (s, 1H). |
| **107** | 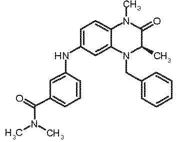 | 3-{[(3*R*)-4-Benzyl-1,3-dimethyl-2-oxo-1,2,3,4-tetrahydrochinoxalin-6-yl]amino}-*N,N-*dimethylbenzamid | Intermediat 86, Amin 21, Allgemeine Synthesevor schrift C | (400 MHz, CDC13): δ = 1.17 (d, 3H); 1.64 (bs, 1H); 3.00 (bs, 3H); 3.11 (bs, 3H); 3.42 (s, 3H); 4.04 (q, 1H); 4.16 (d, 1H); 4.49 (d, 1H); 6.43 (d, 1H); 6.61 (dd, 1H); 6.79 (dd, 1H); 6.86 (d, 1H); 6.89 (d, 1H); 6.95 (s, 1H); 7.13 (t, 1H); 7.30 - 7.39 (m, 5H). |
| **108** | 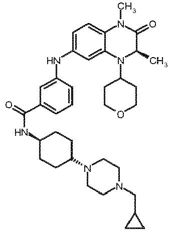 | *N*-{*trans*-4-[4-(Cyclopropylmethyl)pipera zin-1-yl]cyclohexyl}-3-{[(3*R*)-1,3-dimethyl-2-oxo-4-(tetrahydro-2H-pyran-4-yl)-1,2,3,4-tetrahydrochinoxalin-6-yl]amino}benzamid | Intermediat 90, Amin 13, Allgemeine Synthesevor schrift A | (400 MHz, DMSO-d6): δ = 0.01 - 0.06 (m, 2H); 0.39 - 0.46 (m, 2H); 0.72 - 0.83 (m, 1H); 0.97 (d, 3H); 1.19 - 1.41 (m, 4H); 1.57 - 1.64 (m, 1H); 1.64 - 1.77 (m, 2H); 1.77 - 1.90 (m, 5H); 2.12 (d, 2H); 2.14 - 2.21 (m, 1H); 2.29 - 2.45 (m, 4H); 3.23 (s, 3H); 3.32 - 3.41 (m, 4H); 3.56 (br. t, 1H); 3.62 - 3.72 (m, 1H); 3.85 - 3.95 (m, 2H); 4.05 (q, 1H); 6.63 (dd, 1H); 6.68 (d, 1H); 6.96 (d, 1H); 7.09 (br. d, 1H); 7.20 (br. d, 1H); 7.24 (t, 1H); 7.50 (br. s, 1H); 8.06 (d, 1H); 8.14 (s, 1H). |
| **109** | 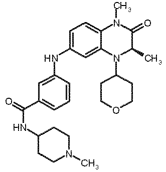 | 3-{[(3R)-1,3-Dimethyl-2-oxo-4-(tetrahydro-2H-pyran-4-yl)-1,2,3,4-tetrahydrochinoxalin-6-yl]amino}-*N*-(1-methylpiperidin-4-yl)benzamid | Intermediat 90, Amin 14, Allgemeine Synthesevor schrift A | (300 MHz, DMSO-d6): δ = 0.97 (d, 3H); 1.60 (d, 4H); 1.67 - 1.90 (m, 5H); 1.90 - 2.02 (m, 2H); 2.16 (s, 3H); 2.76 (d, 2H); 3.23 (s, 3H); 3.30 - 3.42 (m, 2H); 3.65 - 3.76 (m, 1H); 3.90 (bs, 2H); 4.05 (q, 1H); 6.63 (dd, 1H); 6.68 (d, 1H); 6.96 (d, 1H); 7.10 (dt, 1H); 7.19 - 7.29 (m, 2H); 7.51 (br. s, 1H); 8.11 (d, 1H); 8.15 (s, 1H). |
| **110** | 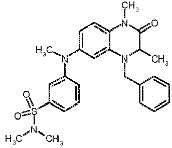 | 3-[(4-Benzyl-1,3-dimethyl-2-oxo-1,2,3,4-tetrahydrochinoxalin-6-yl)(methyl)amino]-*N,N-*dimethylbenzolsulfonamid | Intermediat 86, Amin 3, Allgemeine Synthesevor schrift C | (400 MHz, CHCl3): δ = 1.19 (d, 3H); 2.71 (s, 6H); 3.25 (s, 3H); 3.44 (s, 3H); 4.04 (q, 1H); 4.13 (d, 1H); 4.47 (d, 1H); 6.49 (d, 1H); 6.68 (dd, 1H); 6.87 (ddd, 1H); 6.95 (d, 1H); 7.14 (br. d, 1H); 7.17 (t, 1H); 7.22 (t, 1H); 7.29 - 7.36 (m, 5H). |

**Tabelle 5: Folgende Beispiele wurden durch Trennung der entsprechenden racemischen Beispielverbindungen erhalten: Säule; Eluent; Flussrate; Temperatur: 25 °C; DAD 996 scan: 280 nm.**

| Bsp | Struktur | Name | Racemisches Beispiel Nr | Säule; Eluent; Flussrate; Analytik |
|---|---|---|---|---|
| **111** | 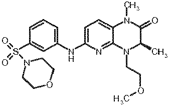 | (3*R*)-4-(2-Methoxyethyl)-1,3-dimethyl-6-{[3-(morpholin-4-ylsulfonyl) phenyl]amino}-3,4-dihydropyrido[2,3-b]pyrazin-2(1H)-on | Beispiel 49 | - Chiralpak IA 5µm 250x20 mm; -Hexan / Ethanol / Diethylamin 70:30:0.1 -Fluss 20 ml/min; -Rt = 8.8-9.9 min Ausbeute: 14 mg |
| **112** | 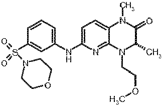 | (3*S*)-4-(2-Methoxyethyl)-1,3-dimethyl-6-{[3-(morpholin-4-ylsulfonyl) phenyl]amino}-3,4-dihydropyrido[2,3-b]pyrazin-2(1H)-on | Beispiel 49 | - Chiralpak IA 5µm 250x20 mm; -Hexan / Ethanol / Diethylamin 70:30:0.1 -Fluss 20 ml/min; -Rt = 5.2-6.7 min Ausbeute: 10 mg |
| **113** | 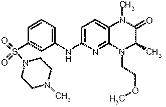 | (3*R*)-4-(2-Methoxyethyl)-1,3-dimethyl-6-({3-[(4-methylpiperazin-1-yl)sulfonyl]phenyl}amino) -3,4-dihydropyrido[2,3-b]pyrazin-2(1H)-on | Beispiel 53 | - Chiralpak IA 5µm 250x30 mm; -Hexan / 2-Propanol / Diethylamin 70:30:0.1 -Fluss 50 ml/min; -Rt = 7.8-11.0 min Ausbeute: 55 mg |
| **114** | 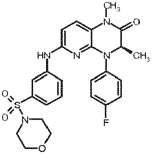 | (3*R*)-4-(4-Fluorphenyl)-1,3-dimethyl-6-{[3-(morpholin-4-ylsulfonyl)phenyl]amino}-3,4-dihydropyrido[2,3-b]pyrazin-2(1H)-on | Beispiel 135 | - Chiralpak IA 5µm 250x30 mm; -Hexan / 2-Propanol / Diethylamin 70:30:0.1 -Fluss 40 ml/min; -Rt = 15.2-23.5 min (400 MHz, 25°C, DMSO-d6): δ = 1.29 (d, 3H); 2.75-2.84 (m, 4H); 3.30 (s, 3H); 3.57.3.65 (m, 4H); 4.52 (q, 1H); 6.38 (d, 1H); |
| | | | | 7.01-7.10 (m, 2H); 7.26-7.32 (m, 2H); 7.36 (t, 1H); 7.38-7.44 (m, 3H); 7.84-7.88 (m, 1H); 9.26 (s, 1H). Ausbeute: 88 mg |
| **115** | 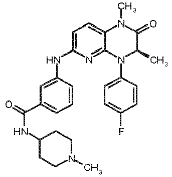 | 3-{[(3*R*)-4-(4-Fluorphenyl)-1,3-dimethyl-2-oxo-1,2,3,4-tetrahydropyrido[2,3-b]pyrazin-6-yl]amino}-*N-*(1-methylpiperidin-4-yl)benzamid | Beispiel 136 | Chiralpak IC 5µm 250x30 mm; -Hexan / Ethanol / Diethylamin 70:30:0.1 -Fluss 50 ml/min; -Rt = 13.7-15.3 min (400 MHz, DMSO-d6): δ = 1.26 (d, 3H); 1.49 - 1.61 (m, 2H); 1.68 - 1.77 (m, 2H); 1.91 - 2.02 (m, 2H); 2.17 (s, 3H); 2.73 - 2.81 (m, 2H); 3.28 (s, 3H); 3.64 - 3.76 (m, 1H); 4.50 (q, 1H); 6.36 (d, 1H); 6.90 (t, 1H); 7.15 (br. d, 1H); 7.22 (t, 2H); 7.35 - 7.41 (m, 3H); 7.44 (t, 1H); 7.58 (dd, 1H); 8.05 (d, 1H); 8.92 (s, 1H). Ausbeute: 26 mg |
| **116** | 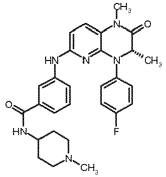 | 3-{[(3*S*-4-(4-Fluorphenyl)-1,3-dimethyl-2-oxo-1,2,3,4-tetrahydropyrido[2,3-b]pyrazin-6-yl]amino}-*N-*(1-methylpiperidin-4-yl)benzamid | Beispiel 136 | - Chiralpak IC 5µm 250x30 mm; -Hexan / Ethanol / Diethylamin 70:30:0.1 -Fluss 50 ml/min; -Rt = 21.2-23.4 min (400 MHz, DMSO-d6): δ = 1.26 (d, 3H); 1.49 - 1.61 (m, 2H); 1.68 - 1.77 (m, 2H); 1.91 - 2.02 (m, 2H); 2.17 (s, 3H); 2.73 - 2.81 (m, 2H); 3.28 (s, 3H); 3.64 - 3.76 (m, 1H); 4.50 (q, 1H); 6.36 (d, 1H); 6.90 (t, 1H); 7.15 (br. d, 1H); 7.22 (t, 2H); 7.35 - 7.41 (m, 3H); 7.44 (t, |
| | | | | 1H); 7.58 (dd, 1H); 8.05 (d, 1H); 8.92 (s, 1H). Ausbeute: 32 mg |
| **117** | 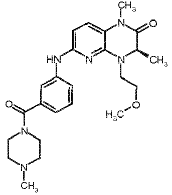 | (3*R*)-4-(2-Methoxyethyl)-1,3-dimethyl-6-({3-[(4-methylpiperazin-1-yl)carbonyl]phenyl} amino )-3,4-dihydropyrido[2,3-b]pyrazin-2(1H)-on | Beispiel 137 | Chiralpak AS-H 5µm 250x20 mm; -Methanol/ Ethanol / Diethylamin 50:50:0.1 -Fluss 31 ml/min; -Rt = 2.5-4.25 min (400 MHz, DMSO-d6): δ = 1.12 (d, 3H); 2.18 (s, 3H); 2.21 - 2.37 (m, 4H); 3.19 (s, 3H); 3.22-3.26 (m, 4H); 3.33-3.41 (m, 2H); 3.52 - 3.63 (m, 4H); 3.97 - 4.08 (m, 1H); 4.12 - 4.19 (m, 2H); 6.21 (d, 1H); 7.22 (d, 1H); 7.24 - 7.27 (m, 1H); 7.54 (dd, 1H); 7.69 (s, 1H); 8.91 (s, 1H). Ausbeute: 6 mg |
| **118** | 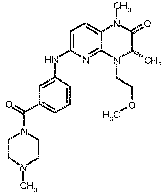 | (3*S*)-4-(2-Methoxyethyl)-1,3-dimethyl-6-({3-[(4-methylpiperazin-1-yl)carbonyl]phenyl}amino )-3,4-dihydropyrido[2,3-b]pyrazin-2(1H)-on | Beispiel 137 | - Chiralpak AS-H 5µm 250x20 mm; -Methanol/ Ethanol / Diethylamin 50:50:0.1 -Fluss 31 ml/min; -Rt = 5.2-9.0 min (400 MHz, DMSO-d6): δ = 1.12 (d, 3H); 2.18 (s, 3H); 2.21 - 2.37 (m, 4H); 3.19 (s, 3H); 3.22 - 3.26 (m, 4H); 3.33 - 3.41 (m, 2H); 3.52 - 3.63 (m, 4H); 3.97 - 4.08 (m, 1H); 4.12 - 4.19 (m, 2H); 6.21 (d, 1H); 7.22 (d, 1H); 7.24 - 7.27 (m, 1H); 7.54 (dd, 1H); 7.69(s, 1H);8.91 (s, 1H). Ausbeute: 2 mg |
| **119** | 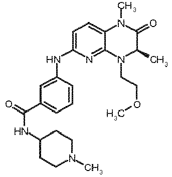 | 3-{[(3*R*)-4-(2-Methoxyethyl)-1,3-dimethyl-2-oxo-1,2,3,4-tetrahydropyrido [2,3-b]pyrazin-6-yl] amino}-*N*-(1-methyl piperidin-4-yl)benzamid | Beispiel 98 | - Chiralpak AD-H 5µm 250x30 mm; -Hexan / 2-Propanol / Diethylamin 70:30:0.1 -Fluss 240 ml/min; -Rt = 14.5-18.0 min (300 MHz, DMSO-d6): δ = 1.12 (d, 3H); 1.23 (d, 1H); 1.56 (d, 2H); 1.73 (d, 2H); 1.93 (t, 2H); 2.15 (s, 3H); 2.75 (d, 2H); 3.19 (s, 3H); 3.20 - 3.22 (m, 3H); 3.55 (t, 2H); 3.63 - 3.78 (m, 1H); 4.02 - 4.21 (m, 3H); 6.21 (d, 1H); 7.18 - 7.27 (m, 3H); 7.65 - 7.72 (m, 1H); 8.04 (br. s, 1H); 8.11 (d, 1H); 8.90 (s, 1H). Ausbeute: 60 mg |
| **120** | 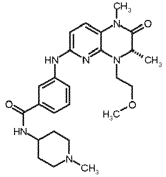 | 3-{[(3*S*)-4-(2-Methoxyethyl)-1,3-dimethyl-2-oxo-1,2,3,4-tetrahydropyrido [2,3-b]pyrazin-6-yl] amino}*-N-*(1*-*methyl piperidin-4-yl)benzamid | Beispiel 98 | - Chiralpak AD-H 5µm 250x30 mm; -Hexan / 2-Propanol / Diethylamin 70:30:0.1 -Fluss 240 ml/min; -Rt = 19.0-23.0 min (300 MHz, DMSO-d6): δ = 1.12 (d, 3H); 1.23 (d, 1H); 1.56 (d, 2H); 1.73 (d, 2H); 1.93 (t, 2H); 2.15 (s, 3H); 2.75 (d, 2H); 3.19 (s, 3H); 3.20 - 3.22 (m, 3H); 3.55 (t, 2H); 3.63 - 3.78 (m, 1H); 4.02 - 4.21 (m, 3H); 6.21 (d, 1H); 7.18 - 7.27 (m, 3H); 7.65 - 7.72 (m, 1H); 8.04 (br. s, 1H); 8.11 (d, 1H); 8.90 (s, 1H). Ausbeute: 45 mg |
| **121** | 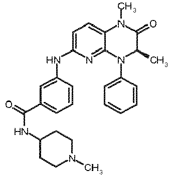 | 3-{[(3*R*)-1,3-Dimethyl-2-oxo-4-phenyl-1,2,3,4-tetrahydropyrido[2,3-b]pyrazin-6-yl]amino}-*N-*(1-methylpiperidin-4-yl)benzamid | Beispiel 104 | - Chiralpak IC 5µm 250x20 mm; -Hexan / Ethanol / Diethylamin 75:25:0.1 -Fluss 31 ml/min; -Rt = 8.8-10.2 min (300 MHz, DMSO-d6): δ = 1.10(s, 1H); 1.28 (d, 3H); 1.31 - 1.62 (m, 3H); 1.66 - 1.77 (m, 2H); 1.84 - 2.04 (m, 3H); 2.14 (s, 3H); 2.69 - 2.78 (m, 2H); 3.59 - 3.76 (m, 1H); 4.54 (q, 1H); 6.40 (d, 1H); 6.88 (t, 1H); 7.11-7.23 (m, 2H); 7.31-7.45 (m, 6H); 7.70 (dd, 1H); 8.04 (d, 1H); 8.93 (s, 1H). Ausbeute: 30 mg |

### Beispiel 122, Allgemeine Synthesevorschrift D

### (3R)-6-{[3-(2-Azaspiro[3.3]hept-2-ylsulfonyl)phenyl]amino}-1,3-dimethyl-4-(piperidin-4-yl)-3,4-dihydropyrido[2,3-b]pyrazin-2(1H)-on

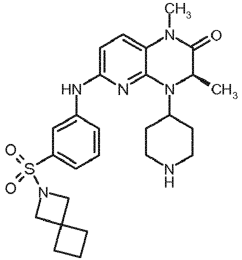

Eine Lösung von 150 mg Beispiel 60 in 17 ml Dichlormethan und 0.25 ml Trifluoressigsäure wurde 14 Stunden bei RT gerührt. Unter Zugabe von Toluol wurde das Lösungsmittel im Vakuum entfernt und der Rückstand durch RP-HPLC (Waters Autopurificationsystem SQD; Säule: Waters XBrigde C18 5µm 100x30mm; Eluent A: Wasser + 0.1% Vol. Ameisensäure (99%), Eluent B: Acetonitril; Gradient: 0-8.0 min 1-100% B, 8.0-10.0 min 100% B; Fluss 50.0 ml/min; Temperatur: RT; Injektion: 2500 µl; DAD scan: 210-400 nm) gereinigt. Man erhielt 85 mg (3*R*)-6-{[3-(2-Azaspiro[3.3]hept-2-ylsulfonyl)phenyl]amino}-1,3-dimethyl-4-(piperidin-4-yl)-3,4-dihydropyrido[2,3-b]pyrazin-2(1H)-on.
¹H-NMR (300 MHz, DMSO-d6): δ = 1.08 (d, 3H); 1.63 (q, 2H); 1.74 - 2.00 (m, 6H); 2.12 (d, 1H); 3.01 (t, 3H); 3.21 (s, 3H); 3.26 - 3.40 (m, 3H); 3.57 - 3.70 (m, 4H); 4.16 (q, 1H); 4.52 (br. t, 1H); 6.32 (d, 1H); 7.20 (d, 1H); 7.34 (d, 1H); 7.51 (t, 1H); 7.76 (dd, 1H); 8.25 (br. s, 1H); 9.32 (s, 1H).

**Tabelle 6: Folgende Beispiele wurden entsprechend der allgemeinen Synthesevorschrift D aus den genannten Herstellungsbeispielen erhalten :**

| Bsp | Struktur | Name | Aus Beispiel | Analytische Daten: ¹H-NMR |
|---|---|---|---|---|
| **123** | 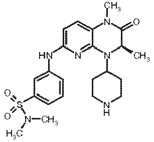 | 3-{[(3*R*)-1,3-Dimethyl-2-oxo-4-(piperidin-4-yl)-1,2,3,4-tetrahydropyrido [2,3-b]pyrazin-6-yl]amino}-*N,N*-dimethyl benzolsulfonamid | Beispiel 61 | (300 MHz, DMSO-d6): δ = 1.07 (d, 3H); 1.48 - 1.60 (m, 2H); 1.60 - 1.77 (m, 1H); 1.95 (d, 1H); 2.56 - 2.70 (m, 9H); 3.03 (t, 2H); 3.20 (s, 3H); 4.20 (q, 1H); 4.32 (t, 1H); 6.25 (d, 1H); 7.13 (d, 1H); 7.28 (d, 1H); 7.45 (t, 1H); 7.73 (s, 1H); 8.22 (d, 1H); 9.22 (s, 1H). |
| **124** | 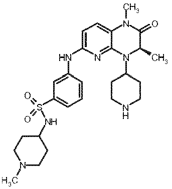 | 3-{[(3*R*)-1,3-Dimethyl-2-oxo-4-(piperidin-4-yl)-1,2,3,4-tetrahydropyrido [2,3-b]pyrazin-6-yl]amino}-*N*(1-methyl piperidin-4-yl)benzol sulfonamid | Beispiel 57 | (400 MHz, DMSO-d6): δ = 1.07 (d, 3H); 1.30 - 1.42 (m, 2H); 1.47 - 1.64 (m, 5H); 1.74 (d, 3H); 1.94 (br. d, 1H); 2.04 (s, 3H); 2.54 - 2.68 (m, 3H); 2.89 (s, 1H); 2.96 - 3.07 (m, 3H); 3.20 (s, 3H); 4.19 (q, 1H); 4.30 (br. t, 1H); 6.25 (d, 1H); 7.23 (br. d, 1H); 7.26 (d, 1H); 7.39 (t, 1H); 7.59 - 7.70 (m, 1H); 7.86 (s, 1H); 8.02 (d, 1H); 9.13 (s, 1H). |
| **125** | 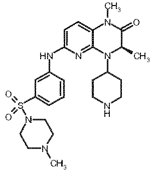 | (3*R*)-1,3-Dimethyl-6-({3-[(4-methylpiperazin-1-yl)sulfonyl]phenyl}amino) -4-(piperidin-4-yl)-3,4-dihydropyrido[2,3-b]pyrazin-2(1H)-on | Beispiel 62 | (400 MHz, DMSO-d6): δ = 1.09 (d, 3H); 1.55 (bs, 3H); 1.92 - 2.00 (m, 1H); 2.14 (s, 4H); 2.34 - 2.39 (m, 4H); 2.63 (d, 2H); 2.89 (bs, 4H); 3.03 (bs, 2H); 3.21 (s, 3H); 4.21 (q, 1H); 4.33 (tt, 1H); 6.26 (d, 1H); 7.13 (br. d, 1H); 7.29 (d, 1H); 7.47 (t, 1H); 7.73 (t, 1H); 8.22 (dd, 1H); 9.23 (s, 1H). |
| **126** | 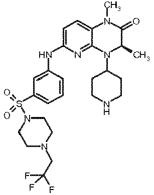 | (3R)-1,3-Dimethyl-4-(piperidin-4-yl)-6-[(3-{[4-(2,2,2-trifluorethyl) piperazin-1-yl]sulfonyl} phenyl)amino]-3,4-dihydropyrido[2,3-b]pyrazin-2(1H)-on | Beispiel 66 | (400 MHz, DMSO-d6): δ = 1.10 (d, 3H); 1.85 (d, 1H); 1.90 - 2.04 (m, 1H); 2.18 (br. d, 1H); 2.68 (br. t, 4H); 2.89 (bs, 4H); 2.98 - 3.12 (m, 2H); 3.13 - 3.21 (m, 2H); 3.22 - 3.24 (m, 3H); 3.33 - 3.42 (m, 4H); 4.17 (q, 1H); 4.53 (tt, 1H); 6.33 (d, 1H); 7.16 (br. d, 1H); 7.35 (d, 1H); 7.51 (t, 1H); 7.80 (dd, 1H); 8.12 (t, 1H); 9.33 (s, 1H). |
| **127** | 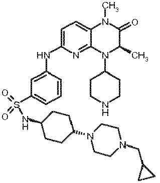 | *N*-{*trans*-4-[4-(Cyclopropylmethyl)pipera zin-1-yl]cyclohexyl}-3-{[(3*R*)-1,3-dimethyl-2-oxo-4-(piperidin-4-yl)-1,2,3,4-tetra-hydropyrido[2,3-b]pyrazin-6-yl]amino} benzolsulfonamid | Beispiel 52 | (400 MHz, DMSO-d6): δ = 0.25 (bs, 2H); 0.56 (d, 2H); 0.83 - 1.05 (m, 2H); 1.09 (d, 3H); 1.19 (bs, 4H); 1.62 - 1.90 (m, 6H); 1.90 - 2.08 (m, 2H); 2.09 - 2.29 (m, 2H); 2.72 (bs, 2H); 2.88 (bs, 3H); 2.99 - 3.30 (m, 10H); 4.15 (q, 1H); 4.57 (br. t, 1H); 6.31 (d, 1H); 7.25 (d, 1H); 7.33 (d, 1H); 7.38 - 7.46 (m, 1H); 7.50 (d, 1H); 7.66 (br. d, 1H); 8.26 - 8.42 (m, 2H); 8.76 (br. d, 1H); 9.25 (s, 1H). |
| **128** | 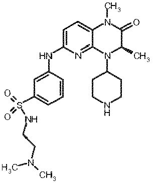 | *N*-[2-(Dimethylamino) ethyl]-3-{[(3*R*)-1,3-dimethyl-2-oxo-4-(piperidin-4-yl)-1,2,3,4-tetrahydropyrido[2,3-b]pyrazin-6-yl]amino} benzolsulfonamid | Beispiel 59 | (400 MHz, DMSO-d6): δ = 1.09 (d, 3H); 1.50 - 1.64 (m, 2H); 1.65 - 1.78 (m, 1H); 1.92 - 1.99 (m, 1H); 2.05 (s, 7H); 2.24 (t, 2H); 2.56 - 2.70 (m, 3H); 2.82 (d, 2H); 2.97 - 3.07 (m, 2H); 3.21 (s, 3H); 4.21 (q, 1H); 4.32 (br. t, 1H); 6.26 (d, 1H); 7.23 (br. d, 1H); 7.28 (d, 1H); 7.41 (t, 1H); 7.86 (s, 1H); 8.06 (br. d, 1H); 9.16 (s, 1H). |
| **129** | 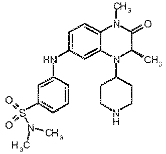 | 3-{[(3*R*)-1,3-Dimethyl-2-oxo-4-(piperidin-4-yl)-1,2,3,4-tetrahydro-chinoxalin-6-yl]amino}-*N,N*-dimethylbenzol-sulfonamid | Beispiel 68 | (600 MHz, DMSO-d6): δ = 0.98 (d, 3H); 1.47 - 1.63 (m, 3H); 1.82 - 1.88 (m, 1H); 2.52 (d, 2H); 2.62 (s, 6H); 2.95 - 3.03 (m, 2H); 3.25 (s, 3H); 3.37 - 3.45 (m, 1H); 4.04 (q, 1H); 5.75 (s, 1H); 6.67 (dd, 1H); 6.68 (br. s, 1H); 7.01 (d, 1H); 7.05 (br. d, 1H); 7.24 (dd, 1H); 7.32 (t, 1H); 7.43 (dd, 1H); 8.46 (s, 1H). |
| **130** | 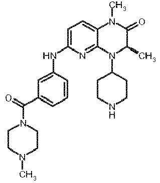 | (3*R*)-1,3-Dimethyl-6-({3-[(4-methylpiperazin-1-yl)carbonyl]phenyl}amino )-4-(piperidin-4-yl)-3,4-dihydropyrido[2,3-b]pyrazin-2(1H)-on | Beispiel 78 | (400 MHz, DMSO-d6): δ = 1.09 (d, 3H); 1.48 - 1.60 (m, 2H); 1.61 - 1.74 (m, 1H); 1.89 - 1.96 (m, 1H); 2.19 (s, 3H); 2.23 - 2.38 (m, 4H); 2.52 - 2.60 (m, 4H); 2.98 - 3.07 (m, 2H); 3.21 (s, 3H); 3.52 - 3.69 (m, 2H); 4.16 - 4.23 (m, 2H); 4.30 (tt, 1H); 6.24 (d, 1H); 6.78 (d, 1H); 7.26 (d, 2H); 7.62 (dd, 1H); 7.77 (br. s, 1H); 8.96 (s, 1H). |
| **131** | 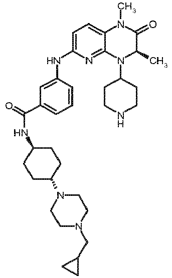 | *N*-{*trans*-*4*-[4-(Cyclopropylmethyl)pipera zin-1-yl]cyclohexyl}-3-{[(3*R*)-1,3-dimethyl-2-oxo-4-(piperidin-4-yl)-1,2,3,4-tetrahydro-pyrido[2,3-b]pyrazin-6-yl]amino}benzamid | Beispiel 102 | (400 MHz, DMSO-d6): δ = - 0.01 - 0.07 (m, 2H); 0.39 - 0.46 (m, 2H); 0.73 - 0.83 (m, 1H); 1.07 (d, 3H); 1.19 - 1.40 (m, 4H); 1.45 - 1.57 (m, 2H); 1.68 (dd, 1H); 1.78 - 1.91 (m, 4H); 1.95 (d, 1H); 2.12 (d, 2H); 2.17 (t, 1H); 2.39 (bs, 4H); 2.51 - 2.64 (m, 3H); 3.00 (d, 2H); 3.19 (s, 3H); 3.21 - 3.24 (m, 2H); 3.68 (bs, 1H); 4.17 (q, 1H); 4.29 (t, 1H); 6.21 (d, 1H); 7.20 - 7.28 (m, 3H); 7.82 (s, 1H); 7.90 (d, 1H); 8.06 (d, 1H); 8.90 (s, 1H). |
| **132** | 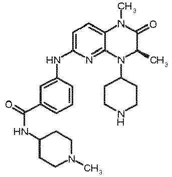 | 3-{[(3*R*)-1,3-Dimethyl-2-oxo-4-(piperidin-4-yl)-1,2,3,4-tetrahydro-pyrido[2,3-b]pyrazin-6-yl]amino}-*N*-(1-methylpiperidin-4-yl)benzamid | Beispiel 100 | (300 MHz, DMSO-d6): δ = 1.07 (d, 3H); 1.45 - 1.64 (m, 4H); 1.65 - 1.79 (m, 3H); 1.83 - 2.00 (m, 3H); 2.14 (s, 3H); 2.56 (d, 4H); 2.74 (d, 2H); 3.00 (d, 2H); 3.19 (s, 3H); 4.17 (q, 1H); 4.29 (br. t, 1H); 6.22 (d, 1H); 7.20 - 7.28 (m, 3H); 7.84 (br. s, 1H); 7.86 - 7.94 (m, 1H); 8.12 (d, 1H); 8.93 (s, 1H). |
| **133** | 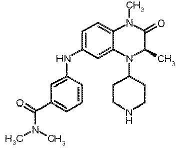 | 3-{[(3*R*)-1,3-Dimethyl-2-oxo-4-(piperidin-4-yl)-1,2,3,4-tetrahydro-chinoxalin-6-yl]amino}-*N,N*-dimethylbenzamid | Beispiel 105 | (400 MHz, DMSO-d6): δ = 0.98 (d, 3H); 1.51 - 1.70 (m, 3H); 1.89 (d, 1H); 2.53 - 2.63 (m, 2H); 2.94 (bs, 6H); 3.01 - 3.12 (m, 2H); 3.24 (s, 3H); 3.37 - 3.51 (m, 2H); 4.02 (q, 1H); 6.62 - 6.68 (m, 2H); 6.75 (br. d, 1H); 6.96 - 7.01 (m, 2H); 7.04 (dd, 1H 7.25 (t, 1H); 8.16 (s, 1H). |

### Beispiel 134:

### (3R)-1,3-Dimethyl-6-{[3-(5-methy-1,3,4-oxadiazol-2-yl)phenyl]amino}-4-(tetrahydro-2H-pyran-4-yl)-3,4-dihydropyrido[2,3-b]pyrazin-2(1H)-on

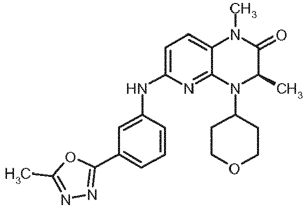

Eine Mischung von 200 mg Intermediat 10, 225 mg 3-(5-Methyl-1,3,4-oxadiazol-2-yl)-phenylanilin (CAS 122733-40-8), 29 mg Palladium(II)acetat (CAS 3375-31-3), 1.05 g Caesiumcarbonat und 80 mg (+)-BINAP in 14.3 ml Toluol wurde 5 Stunden unter einer Argonatmosphäre bei 120°C gerührt. Der Ansatz wurde auf Wasser gegeben und zweimal mit Ethylacetat extrahiert. Die organische Phase wurde mit gesättigter Natriumchlorid Lösung gewaschen, über Natriumsulfat getrocknet und das Lösungsmittel im Vakuum entfernt. Der Rückstand wurde durch RP-HPLC Chromatographie gereinigt (Säule: X-Bridge C18 5µm 100x30mm, Mobile Phase: Acetonitril / Wasser (0.1 Vol% Ameisensäure)-Gradient). Man erhielt 31 mg (3*R*)-1,3-Dimethyl-6-{[3-(5-methyl-1,3,4-oxadiazol-2-yl)phenyl]amino}-4-(tetrahydro-2H-pyran-4-yl)-3,4-dihydropyrido[2,3-b]pyrazin-2(1H)-on.
¹H-NMR: (400 MHz, 25°C, DMSO-d6): δ = 1.09 (d, 3H); 1.56-1-64 (m, 1H); 1.75 (qd, 1H); 1.89 (qd, 1H); 1.93-2.01 (m, 1H); 2.58 (s, 3H); 3.22 (s, 3H); 3.35 (dt, Signal z.T. unter Wasser Peak, 1H); 3.46 (dt, 1H); 3.83-3.94 (m, 2H); 4.24 (q, 1H); 4.49 (tt, 1H); 6.28 (d, 1H); 7.30 (d, 1H); 7.38 (td, 1H); 7.43 (t, 1H); 7.80 (td, 1H); 8.27 (t, 1H); 9.11 (s, 1H).

### Beispiel 135:

### 4-(4-Fluorphenyl)-1,3-dimethyl-6-{[3-(morpholin-4-ylsulfonyl)phenyl]amino}-3,4-dihydropyrido[2,3-b]pyrazin-2(1H)-on

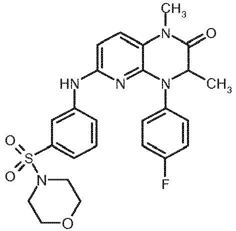

Eine Mischung von 400 mg Intermediat 72, 451 mg 3-(Morpholin-4-ylsulfonyl)anilin (Amin 6), 17.1 mg Tris(dibenzylidenaceton)dipalladium(0) (CAS 51364-51-3), 607 mg Caesiumcarbonat und 41.6 mg Xanthphos (CAS 161265-03-8) in 3.5 ml Dioxan wurde 20 Stunden unter einer Argonatmosphäre bei 120°C gerührt. Der Ansatz wurde auf Wasser gegeben und zweimal mit Ethylacetat extrahiert. Die organische Phase wurde mit gesättigter Natriumchlorid Lösung gewaschen, über Natriumsulfat getrocknet und das Lösungsmittel im Vakuum entfernt. Der Rückstand wurde durch RP-HPLC Chromatographie gereinigt (Säule: X-Bridge C18 5µm 100x30mm, Mobile Phase: Acetonitril / Wasser (0.1 Vol% Ameisensäure)-Gradient). Man erhielt 900 mg 3-{[4-(4-Fluorphenyl)-1,3-dimethyl-2-oxo-1,2,3,4-tetrahydropyrido[2,3-b]pyrazin-6-yl]amino}-*N*-(tetrahydro-2H-pyran-4-yl)benzolsulfonamid als Rohprodukt.

UPLC-MS: Instrument: Waters Acquity UPLC-MS SQD; Säule: Acquity UPLC BEH C18 1.7 50x2.1mm; Eluent A: Wasser + 0.1% Vol. Ameisensäure (99%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm.
Rt = 1.22 min (M⁺+1 = 511)

### Beispiel 136:

### 3-{[4-(4-Fluorphenyl)1,3-dimethyl-2-oxo-1,2,3,4-tetrahydropyrido[2,3-b]pyrazin-6-yl]amino}-N-(1-methylpiperidin-4-yl)benzamid

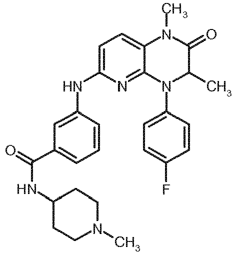

Eine Lösung von 78 mg Intennediat 74, 46 mg 4-Amino-1-methylpiperidin, 0.11 ml Triethylamin und 109 mg HATU in 3 ml DMF wurde 16 Stunden bei RT gerührt. Der Ansatz wurde auf gesättigte Kochsalzlösung gegeben und dreimal mit Etylacetat extrahiert. Die vereinten organischen Phasen wurden mit gesättigter Kochsalzlösung gewaschen, über Natriumsulfat getrocknet und das Lösungsmittel im Vakuum entfernt. Man erhielt 200 mg 3-{[4-(4-Fluorphenyl)-1,3-dimethyl-2-oxo-1,2,3,4-tetrahydropyrido[2,3-b]pyrazin-6-yl]amino}-*N*-(1-methylpiperidin-4-yl)benzamid als Rohprodukt.

UPLC-MS: Instrument: Waters Acquity UPLC-MS SQD; Säule: Acquity UPLC BEH C18 1.7 50x2.1mm; Eluent A: Wasser + 0.1% Vol. Ameisensäure (99%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm.
Rt = 0.86 min (M⁺+1 = 503)

### Beispiel 137:

### 4-(2-Methoxyethyl)-1,3-dimethyl-6-({3-[(4-methylpiperazin-1-yl)carbonyl]phenyl}amino)-3,4-dihydropyrido[2,3-b]pyrazin-2(1H)-on

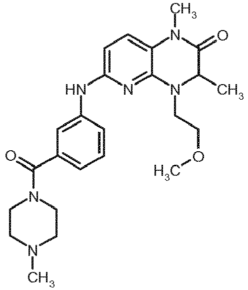

Eine Lösung von 100 mg Intemediat 49, 46 mg 1-Methylpiperazin, 0.30 ml Triethylamin und 307 mg HATU in 22 ml DMF wurde 48 Stunden bei RT gerührt. Der Ansatz wurde auf halbgesättigte Kochsalzlösung gegeben und dreimal mit Etylacetat extrahiert. Die vereinten organischen Phasen wurden mit gesättigter Kochsalzlösung gewaschen, über Natriumsulfat getrocknet und das Lösungsmittel im Vakuum entfernt. Man erhielt 100 mg 4-(2-Methoxyethyl)-1,3-dimethyl-6-({3-[(4-methylpiperazin-1-yl)carbonyl]phenyl}amino)-3,4-dihydropyrido[2,3-b]pyrazin-2(1H)-on als Rohprodukt.

UPLC-MS: Instrument: Waters Acquity UPLC-MS SQD; Säule: Acquity UPLC BEH C18 1.7 50x2.1mm; Eluent A: Wasser + 0.1% Vol. Ameisensäure (99%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm.
Rt = 0.95 min (M⁺+1 = 453)

### Biologische Wirksamkeit der erfindungsgemäßen Verbindungen

### Protein-Protein Wechselwirkungsassay: Bindungsassay BRD4 / acetyliertes Peptid H4

### 1. Assay-Beschreibung BRD4-Bromodomäne 1 [BRD4(1)]

Zur Beurteilung der BRD4(1)-Bindungsstärke der in dieser Anmeldung beschriebenen Substanzen wurde deren Fähigkeit quantifiziert, die Wechselwirkung zwischen BRD4(1) und acetyliertem Histon H4 dosisabhängig zu hemmen.

Zu diesem Zweck wurde ein zeitaufgelöster Fluoreszenz-Resonanz-Energie-Transfer (TR-FRET) Assay verwendet, der die Bindung zwischen N-terminal His6-getaggtem BRD4(1) (Aminosäuren 67-152) und einem synthetischen acetylierten Histon H4 (Ac-H4) Peptid mit Sequenz GRGK(Ac)GGK(Ac)GLGK(Ac)GGAK(Ac)RHGSGSK-Biotin misst. Das nach Filippakopoulos et al., Nature, 2010, 468:1119-1123 und Cell, 2012, 149:214-231 im Haus produzierte rekombinante BRD4(1) Protein wurde in E. coli exprimiert und mittels (Ni-NTA) Affinitäts- und (Sephadex G-75) Größenausschlusschromatografie gereinigt. Das Ac-H4 Peptid kann von z.B. Biosyntan (Berlin, Deutschland) gekauft werden.

Im Assay wurden typischerweise 11 verschiedene Konzentrationen von jeder Substanz (0,1 nM, 0,33 nM, 1,1 nM, 3,8 nM, 13 nM, 44 nM, 0,1.5 µM, 0,51 µM, 1,7 µM, 5,9 µM und 20 µM) als Duplikate auf derselben Mikrotiter-Platte gemessen. Dafür wurden 100-fach konzentrierte Lösungen in DMSO vorbereitet durch serielle Verdünnungen (1:3,4) einer 2 mM Stammlösung in eine klare, 384-Well Mikrotiter-Platte (Greiner Bio-One, Frickenhausen, Germany). Daraus wurden 50 nl in eine schwarze Testplatte (Greiner Bio-One, Frickenhausen, Germany) überführt. Der Test wurde gestartet durch die Zufuhr von 2 µl einer 2,5-fach konzentrierten BRD4(1)-Lösung (üblicherweise 10 nM Endkonzentration in den 5 µl des Reaktionsvolums) in wässrigem Assaypuffer [50 mM HEPES pH 7.5, 50 mM Natriumchlorid (NaCl), 0,25 mM CHAPS und 0,05% Serumalbumin (BSA)] zu den Substanzen in der Testplatte. Darauf folgte ein 10-minütiger Inkubationsschritt bei 22°C für die Voräquilibrierung von putativen Komplexen zwischen BRD4(1) und den Substanzen. Anschließend wurden 3 µl einer 1,67-fach konzentrierten Lösung (im Assaypuffer) bestehend aus Ac-H4 Peptid (83,5 nM) und TR-FRET Detektionsreagenzien [16,7 nM Anti-6His-XL665 und 3,34 nM Streptavidin-Kryptat (beide von Cisbio Bioassays, Codolet, France), so wie 668 mM Kaliumfluorid (KF)] zugegeben.

Die Mischung wurde dann im Dunkeln für eine Stunde bei 22°C und anschließend für mindestens 3 Stunden und maximal über Nacht bei 4°C inkubiert. Die Bildung von BRD4(1) /Ac-H4 Komplexen wurde bestimmt durch die Messung des Resonanzenergietransfers von dem Streptavidin-Eu-Kryptat zum anti-6His-XL665 Antikörper der sich in der Reaktion befindet. Dafür wurden die Fluoreszenzemission bei 620 nm und 665 nm nach Anregung bei 330-350 nm in einem TR-FRET Messgerät, z.B. ein Rubystar oder Pherastar (beide von BMG Lab Technologies, Offenburg, Germany) oder ein Viewlux (Perkin-Elmer), gemessen. Das Verhältnis der Emission bei 665 nm und bei 622 nm (Ratio) wurde als Indikator für die Menge der gebildeten BRD4(1)/Ac-H4 Komplexe genommen.

Die erhaltenen Daten (Ratio) wurden normalisiert, wobei 0% Inhibition dem Mittelwert aus den Messwerten eines Satzes von Kontrollen (üblicherweise 32 Datenpunkte) entsprach, bei denen alle Reagenzien enthalten waren. Dabei wurden anstatt von Testsubstanzen 50 nl DMSO (100%) eingesetzt. Inhibition von 100% entsprach dem Mittelwert aus den Messwerten eines Satzes von Kontrollen (üblicherweise 32 Datenpunkte), bei denen alle Reagenzien außer BRD4(1) enthalten waren. Die Bestimmung des IC₅₀ Wertes erfolgte durch Regressionsanalyse auf Basis einer 4-Parameter Gleichung (Minimum, Maximum, IC₅₀, Hill; Y = Max + (Min - Max) / (1 + (X/IC₅₀)Hill).

### 2. Assay-Beschreibung BRD4-Bromodomäne 2 [BRD4(2)]

Zur Beurteilung der BRD4(2)-Bindungsstärke der in dieser Anmeldung beschriebenen Substanzen wurde deren Fähigkeit quantifiziert, die Wechselwirkung zwischen BRD4(2) und acetyliertem Histon H4 dosisabhängig zu hemmen.

Zu diesem Zweck wurde ein zeitaufgelöster Fluoreszenz-Resonanz-Energie-Transfer (TR-FRET) Assay verwendet, der die Bindung zwischen N-terminal His6-getaggtem BRD4(2) (Aminosäuren 357-445) und einem synthetischen acetylierten Histon H4 (Ac-H4) Peptid mit Sequenz SGRGK(Ac)GGK(Ac)GLGK(Ac)GGAK(Ac)RHRKVLRDNGSGSK-Biotin misst. Das nach Filippakopoulos et al., Nature, 2010, 468:1119-1123 und Cell, 2012, 149:214-231 im Haus produzierte rekombinante BRD4(2) Protein wurde in E. coli exprimiert und mittels (Ni-NTA) Affinitäts- und (Sephadex G-75) Größenausschlusschromatografie gereinigt. Das Ac-H4 Peptid kann von z.B. Biosyntan (Berlin, Deutschland) gekauft werden.

Im Assay wurden typischerweise 11 verschiedene Konzentrationen von jeder Substanz (0,1 nM, 0,33 nM, 1,1 nM, 3,8 nM, 13 nM, 44 nM, 0,15 µM, 0,51 µM, 1,7 µM, 5,9 µM und 20 µM) als Duplikate auf derselben Mikrotiter-Platte gemessen. Dafür wurden 100-fach konzentrierte Lösungen in DMSO vorbereitet durch serielle Verdünnungen (1:3,4) einer 2 mM Stammlösung in eine klare, 384-Well Mikrotiter-Platte (Greiner Bio-One, Frickenhausen, Germany). Daraus wurden 50 nl in eine schwarze Testplatte (Greiner Bio-One, Frickenhausen, Germany) überführt. Der Test wurde gestartet durch die Zufuhr von 2 µl einer 2,5-fach konzentrierten BRD4(2)-Lösung (üblicherweise 100 nM Endkonzentration in den 5 µl des Reaktionsvolums) in wässrigem Assaypuffer [50 mM HEPES pH 7.5, 50 mM Natriumchlorid (NaCl); 50 mM Kaliumfluorid (KF); 0,25 mM CHAPS und 0,05% Serumalbumin (BSA)] zu den Substanzen in der Testplatte. Darauf folgte ein 10-minütiger Inkubationsschritt bei 22°C für die Voräquilibrierung von putativen Komplexen zwischen BRD4(2) und den Substanzen. Anschließend wurden 3 µl einer 1,67-fach konzentrierten Lösung (im Assaypuffer) bestehend aus Ac-H4 Peptid (83,5 nM) und TR-FRET Detektionsreagenzien [83,5 nM Anti-6His-XL665 (Cisbio Bioassays, Codolet, France) und 12, 52 nM Streptavidin-Eu), (Perkin Elmer, # W1024)] im Assaypuffer zugegeben.

Die Mischung wurde dann im Dunkeln für eine Stunde bei 22°C und anschließend für mindestens 3 Stunden und maximal über Nacht bei 4°C inkubiert. Die Bildung von BRD4(2)/Ac-H4 Komplexe wurde bestimmt durch die Messung des Resonanzenergietransfers von dem Streptavidin-Eu-Chelat zum anti-6His-XL665 Antikörper der sich in der Reaktion befindet. Dafür wurden die Fluoreszenzemission bei 620 nm und 665 nm nach Anregung bei 330-350 nm in einem TR-FRET Messgerät, z.B. ein Rubystar oder Pherastar (beide von BMG Lab Technologies, Offenburg, Germany) oder ein Viewlux (Perkin-Elmer), gemessen. Das Verhältnis der Emission bei 665 nm und bei 622 nm (Ratio) wurde als Indikator für die Menge der gebildeten BRD4(2)/Ac-H4 Komplexe genommen.

Die erhaltenen Daten (Ratio) wurden normalisiert, wobei 0% Inhibition dem Mittelwert aus den Messwerten eines Satzes von Kontrollen (üblicherweise 32 Datenpunkte) entsprach, bei denen alle Reagenzien enthalten waren. Dabei wurden anstatt von Testsubstanzen 50 nl DMSO (100%) eingesetzt. Inhibition von 100% entsprach dem Mittelwert aus den Messwerten eines Satzes von Kontrollen (üblicherweise 32 Datenpunkte), bei denen alle Reagenzien außer BRD4(2) enthalten waren. Die Bestimmung des IC₅₀ Wertes erfolgte durch Regressionsanalyse auf Basis einer 4-Parameter Gleichung (Minimum, Maximum, IC₅₀, Hill; Y = Max + (Min - Max) / (1 + (X/IC₅₀)Hill)).

### 3. Zell-Assay

### Zellproliferationsassay

In Übereinstimmung mit der Erfindung, wurde die Fähigkeit der Substanzen die Zellproliferation zu hemmen bestimmt. Die Zellviabilität wurde mittels des alamarBlue® Reagenz (Invitrogen) in einem Victor X3 Multilabel Reader (Perkin Elmer) bestimmt. Die Anregungswellenlänge war 530 nm und die Emissionswellenlänge 590 nM.
Die MOLM-13-Zellen (DSMZ, ACC 554) wurden zu einer Konzentration von 4000 Zellen/Well in 100µl Wachstumsmedium (RPMI1640, 10% FCS) auf 96well Microtiterplatten ausgesät.
Die MOLP-8-Zellen (DSMZ, ACC 569) wurden zu einer Konzentration von 4000 Zellen/Well in 100µl Wachstumsmedium (RPMI1640, 20% FCS) auf 96well Microtiterplatten ausgesät.
Die B16F10-Zellen (ATCC, CRL-6475) wurden zu einer Konzentration von 300-500 Zellen/Well in 100µl Wachstumsmedium (DMEM mit Phenolrot, 10% FCS) auf 96well Microtiterplatten ausgesät.
Die CHL-1-Zellen (ATCC, CRL-9446) wurden zu einer Konzentration von 1000 Zellen/Well in 100µl Wachstumsmedium (DMEM mit Glutamin, 10% FCS) auf 96well Microtiterplatten ausgesät.
Nach einer Übernachtinkubation bei 37°C wurden die Fluoreszenzwerte bestimmt (CI Werte). Dann wurden die Platten mit verschiedenen Substanzverdünnungen behandelt (1E-5 M, 3E-6 M, 1E-6M, 3E-7 M, 1E-7 M, 3E-8 M, 1E-8 M) und während 96 (MOLM-13-, B16F10-, CHL-1-Zellen) oder 120 (MOLP-8-Zellen) Stunden bei 37°C inkubiert. Anschließend wurden die Fluoreszenzwerte bestimmt (CO Werte). Für die Datenanalyse wurden die CI Werte von den CO Werten abgezogen und die Ergebnisse verglichen zwischen Zellen, die mit verschiedenen Verdünnungen der Substanz oder nur mit Pufferlösung behandelt wurden. Die IC50-Werte (Substanzkonzentration, die für eine 50%ige Hemmung der Zellproliferation notwendig ist) wurden daraus berechnet.

Die Substanzen wurden in den Zelllinien der Tabelle 7 untersucht, die beispielhaft die angegebenen Indikationen vertreten:

**Tabelle 7:**

| **Zelllinie** | **Quelle** | **Indikation** |
|---|---|---|
| MOLM-13 | DSMZ | Akute myeloische Leukämie |
| MOLP-8 | DSMZ | Multiples Myelom |
| B16F10 | ATCC | Melanom (BRAF Wild-Typ) |
| CHL-1 | ATCC | Melanom (BRAF Wild-Typ) |

### 4. Ergebnisse:

### 4.1 Bindungsassay

Die Tabelle 8 zeigt die Ergebnisse aus dem BRD4(1) Bindungsassay.

**Tabelle 8:**

| Beispiel | IC₅₀ [BRD4(1)] (nmol/l) |
|---|---|
| 1 | 67 |
| 2 | 94 |
| 3 | 91 |
| 4 | 144 |
| 5 | 99 |
| 6 | 27 |
| 7 | 46 |
| 8 | 79 |
| 9 | 28 |
| 10 | 159 |
| 11 | 208 |
| 12 | 56 |
| 13 | 322 |
| 14 | 144 |
| 15 | 108 |
| 16 | 234 |
| 17 | 129 |
| 18 | 149 |
| 19 | 234 |
| 20 | 58 |
| 21 | 48 |
| 22 | 708 |
| 23 | 15 |
| 24 | 16 |
| 25 | 16 |
| 26 | 17 |
| 27 | 17 |
| 28 | 26 |
| 29 | 29 |
| 30 | 44 |
| 31 | 40 |
| 32 | 22 |
| 33 | 22 |
| 34 | 23 |
| 35 | 26 |
| 36 | 727 |
| 37 | 28 |
| 38 | 19 |
| 39 | 38 |
| 40 | 43 |
| 41 | 20 |
| 42 | 36 |
| 43 | 49 |
| 44 | 50 |
| 45 | 51 |
| 46 | 51 |
| 47 | 21 |
| 48 | 75 |
| 49 | 112 |
| 50 | 76 |
| 51 | 77 |
| 52 | 80 |
| 53 | 151 |
| 54 | 106 |
| 55 | 112 |
| 56 | 112 |
| 57 | 114 |
| 58 | 139 |
| 59 | 156 |
| 60 | 200 |
| 61 | 208 |
| 62 | 256 |
| 63 | 163 |
| 64 | 271 |
| 65 | 386 |
| 66 | 1110 |
| 67 | 4380 |
| 68 | 433 |
| 69 | 496 |
| 70 | 749 |
| 71 | 232 |
| 72 | 62 |
| 73 | 77 |
| 74 | 87 |
| 75 | 118 |
| 76 | 143 |
| 77 | 144 |
| 78 | 151 |
| 79 | 193 |
| 80 | 196 |
| 81 | 210 |
| 82 | 218 |
| 83 | 218 |
| 84 | 268 |
| 85 | 314 |
| 86 | 322 |
| 87 | 351 |
| 88 | 382 |
| 89 | 399 |
| 90 | 430 |
| 91 | 439 |
| 92 | 452 |
| 93 | 466 |
| 94 | 467 |
| 95 | 475 |
| 96 | 501 |
| 97 | 503 |
| 98 | 531 |
| 99 | 551 |
| 100 | 555 |
| 101 | 617 |
| 102 | 788 |
| 103 | 810 |
| 104 | 956 |
| 105 | 208 |
| 106 | 357 |
| 107 | 385 |
| 108 | 575 |
| 109 | 521 |
| 110 | 1290 |
| 111 | 75 |
| 112 | 5300 |
| 113 | 84 |
| 114 | 286 |
| 115 | 379 |
| 116 | 6750 |
| 117 | 489 |
| 118 | 18200 |
| 119 | 742 |
| 120 | 6870 |
| 121 | 447 |
| 122 | 21 |
| 123 | 18 |
| 124 | 30 |
| 125 | 37 |
| 126 | 38 |
| 127 | 47 |
| 128 | 66 |
| 129 | 45 |
| 130 | 206 |
| 131 | 251 |
| 132 | 306 |
| 133 | 124 |
| 134 | 179 |

Die Tabelle 9 zeigt die Ergebnisse aus dem BRD4(2) Bindungsassay.

**Tabelle 9:**

| Beispiel | IC₅₀ [BRD4(2)] (nmol/l) |
|---|---|
| 1 | 55 |
| 2 | 89 |
| 3 | 91 |
| 4 | 101 |
| 5 | 59 |
| 6 | 32 |
| 7 | 71 |
| 8 | 89 |
| 9 | 54 |
| 10 | 62 |
| 11 | 48 |
| 12 | 68 |
| 13 | 218 |
| 14 | 170 |
| 15 | 196 |
| 16 | 174 |
| 17 | 60 |
| 18 | 66 |
| 19 | 132 |
| 20 | 52 |
| 21 | 53 |
| 22 | 157 |
| 23 | 39 |
| 24 | 55 |
| 25 | 56 |
| 26 | 31 |
| 27 | 40 |
| 28 | 53 |
| 29 | 64 |
| 30 | 58 |
| 32 | 39 |
| 33 | 76 |
| 34 | 125 |
| 35 | 69 |
| 36 | 4230 |
| 37 | 16 |
| 38 | 48 |
| 39 | 135 |
| 40 | 52 |
| 41 | 43 |
| 43 | 96 |
| 44 | 88 |
| 45 | 148 |
| 46 | 69 |
| 47 | 59 |
| 48 | 101 |
| 49 | 159 |
| 50 | 121 |
| 51 | 99 |
| 52 | 86 |
| 53 | 186 |
| 54 | 62 |
| 55 | 149 |
| 56 | 77 |
| 57 | 177 |
| 58 | 103 |
| 59 | 77 |
| 60 | 265 |
| 61 | 166 |
| 62 | 133 |
| 63 | 158 |
| 64 | 197 |
| 65 | 589 |
| 66 | 308 |
| 67 | 5190 |
| 68 | 76 |
| 69 | 63 |
| 70 | 384 |
| 71 | 31.5 |
| 72 | 256 |
| 73 | 86 |
| 74 | 91 |
| 75 | 79 |
| 76 | 108 |
| 77 | 118 |
| 78 | 157 |
| 79 | 116 |
| 80 | 247 |
| 81 | 227 |
| 82 | 337 |
| 83 | 227 |
| 84 | 155 |
| 85 | 189 |
| 86 | 127 |
| 87 | 339 |
| 88 | 177 |
| 89 | 497 |
| 90 | 332 |
| 91 | 325 |
| 92 | 579 |
| 93 | 2450 |
| 94 | 115 |
| 95 | 277 |
| 96 | 573 |
| 97 | 179 |
| 98 | 216 |
| 99 | 274 |
| 100 | 462 |
| 101 | 195 |
| 102 | 687 |
| 103 | 518 |
| 104 | 471 |
| 105 | 598 |
| 106 | 817 |
| 107 | 117 |
| 108 | 520 |
| 109 | 1120 |
| 110 | 320 |
| 111 | 115 |
| 112 | 57 |
| 113 | 189 |
| 114 | 143 |
| 115 | 706 |
| 116 | >40 000 |
| 117 | 434 |
| 118 | >40 000 |
| 119 | 428 |
| 120 | 4460 |
| 121 | 430 |
| 122 | 94 |
| 123 | 62 |
| 124 | 46 |
| 125 | 44 |
| 126 | 86 |
| 127 | 90 |
| 128 | 137 |
| 129 | 97 |
| 130 | 178 |
| 131 | 188 |
| 132 | 252 |
| 133 | 990 |
| 134 | 191 |

### 4.2 Zell-Proliferationsassay

Die Tabelle 10 zeigt die Ergebnisse aus den Zellproliferationsassays.

**Tabelle 10:**

| Beispiel | IC₅₀ [MOLM-13] (mol/l) | IC₅₀ [MOLP-8] (nmol/l) | IC₅₀[B16F10] (nmol/l) | IC₅₀ [CHL-1] (nmol/l) |
|---|---|---|---|---|
| 1 | 613 | 337 | 661 | |
| 2 | 352 | 114 | 442 | |
| 3 | 296 | 187 | 695 | |
| 4 | 535 | 249 | 778 | |
| 5 | 326 | 136 | 506 | |
| 6 | 53 | 37 | 71 | |
| 7 | 149 | | 178 | |
| 8 | 271 | | 258 | |
| 9 | 66 | 40 | 78 | |
| 10 | 233 | 82 | 122 | |
| 12 | 195 | 126 | 544 | |
| 13 | 697 | 306 | 840 | |
| 14 | 343 | 240 | 809 | |
| 15 | 511 | 313 | 1080 | |
| 16 | 679 | 306 | 1140 | 346 |
| 20 | 175 | 190 | 556 | |
| 21 | 237 | 175 | 626 | |
| 23 | 47 | 37 | 44 | 50 |
| 24 | 20 | 11 | 112 | 13 |
| 25 | 18 | 11 | 43 | 16 |
| 26 | 32 | 24 | 25 | 31 |
| 27 | 41 | 30 | 60 | 23 |
| 28 | 21 | 18 | 30 | 14 |
| 29 | 64 | 41 | 230 | 36 |
| 30 | 152 | 96 | 165 | 80 |
| 33 | 16 | 12 | 157 | 13 |
| 34 | 55 | 20 | 202 | |
| 35 | 140 | 23 | 146 | |
| 37 | 74 | 65 | 93 | 53 |
| 38 | 20 | 12 | 190 | 19 |
| 39 | 90 | 91 | 149 | 63 |
| 40 | 98 | 73 | 314 | 56 |
| 41 | 22 | 17 | 52 | 21 |
| 43 | 196 | 144 | 537 | |
| 44 | 253 | 335 | 944 | 362 |
| 45 | 126 | 67 | 393 | 81 |
| 46 | 45 | 33 | 135 | 64 |
| 47 | 51 | 25 | 188 | 20 |
| 48 | 240 | 197 | 586 | 111 |
| 49 | 269 | 163 | 399 | 153 |
| 50 | 213 | 140 | 727 | 118 |
| 51 | 97 | 58 | 423 | |
| 52 | 180 | 133 | 569 | 147 |
| 53 | 565 | 365 | 1300 | 179 |
| 54 | 233 | 247 | 164 | 156 |
| 55 | 78 | 48 | 312 | |
| 56 | 239 | 214 | 488 | 181 |
| 57 | 193 | 151 | 531 | 175 |
| 58 | 378 | 268 | 613 | 299 |
| 59 | 516 | 297 | 1140 | 204 |
| 72 | 250 | 122 | 396 | 106 |
| 73 | 110 | 63 | 468 | 80 |
| 74 | 157 | 82 | 491 | 83 |
| 75 | 197 | 133 | 381 | 125 |
| 77 | 1010 | 494 | 973 | |
| 79 | 287 | 77 | 478 | |
| 88 | 2080 | 876 | 1620 | |
| 93 | 410 | 800 | 1180 | 539 |
| 104 | 1440 | 826 | 2430 | |
| 111 | 223 | 106 | 316 | 85 |
| 113 | 329 | 240 | 674 | 251 |
| 122 | 396 | 493 | 1840 | 712 |
| 123 | 140 | 169 | 2500 | 218 |
| 124 | >20 000 | >20 000 | >20 000 | >20 000 |
| 125 | 383 | 719 | >10 000 | 2260 |
| 126 | 84 | 130 | 1160 | 202 |
| 127 | >10 000 | >10 000 | >20 000 | >20 000 |
| 129 | 536 | 567 | 2160 | 945 |
| 131 | >10 000 | >20 000 | >20 000 | >20 000 |
| 132 | >20 000 | >20 000 | >20 000 | >20 000 |
| 133 | >10 000 | 2010 | >20 000 | >10 000 |
| 134 | 546 | 453 | 768 | |

## Patentansprüche

1. Verbindungen der allgemeinen Formel (I) 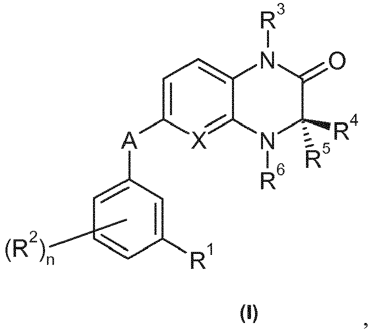 in der
A für -NH-, -N(C₁-C₃-Alkyl)- oder -O- steht,
X für -N- oder -CH- steht,
n für 0,1 oder 2 steht,
R¹ für eine Gruppe -C(=O)NR⁷R⁸ oder -S(=O)₂NR⁷R⁸ steht,
oder
für 5-gliedriges monocyclisches Heteroaryl- steht, das gegebenenfalls ein-, zwei- oder dreifach, gleich oder verschieden substituiert sein kann mit Halogen, Cyano, C₁-C₄-Alkyl-, C₂-C₄-Alkenyl-, C₂-C₄-Alkinyl-, Halogen-C₁-C₄-alkyl-, C₁-C₄-Alkoxy-, Halogen-C₁-C₄-alkoxy-, C₁-C₄-Alkylthio-, Halogen-C₁-C₄-Alkylthio-,
-NR⁹R¹⁰, -C(=O)OR¹¹, -C(=O)N⁹R¹⁰, -C(=O)R¹¹, -S(=O)₂R¹¹,-S(=O)₂NR⁹R¹⁰,
R² für Wasserstoff, Halogen, Cyano, C₁-C₃-Alkyl-, C₂-C₄-Alkenyl-, C₂-C₄-Alkinyl-, Halogen-C₁-C₄-alkyl-, C₁-C₄-Alkoxy-, Halogen-C₁-C₄-alkoxy-, C₁-C₄-Alkylthio- oder Halogen-C₁-C₄-Alkylthio- steht, und falls n für 2 steht, kann R² gleich oder verschieden sein, oder
R¹ und R² gemeinsam für eine Gruppe *-S(=O)₂-NR⁸-CH₂-**, *-S(=O)₂-NR⁸-CH₂-CH₂-**, *-C(=O)-NR⁸-CH₂-** oder *-C(=O)-NR⁸-CH₂-CH₂-** stehen, worin "*" den Anknüpfungspunkt von R¹ am in Formel (I) enthaltenen Phenylring bedeutet, und worin "**" ein diesem Anknüfungspunkt benachbartes Kohlenstoffatom dieses Phenylringes bedeutet,
R³ für Methyl- oder Ethyl- steht,
R⁴ für Wasserstoff oder C₁-C₃-Alkyl- steht,
R⁵ für Wasserstoff oder C₁-C₃-Alkyl- steht,
oder
R⁴ und R⁵ gemeinsam mit dem Kohlenstoffatom, an das sie gebunden sind, für C₃-C₆-Cycloalkylen stehen,
R⁶ für C₁-C₆-Alkyl- steht, das gegebenenfalls einfach substituiert sein kann mit C₁-C₃-Alkoxy-, Phenyl-, C₃-C₈-Cycloalkyl-, oder 4-bis 8-gliedrigem Heterocycloalkyl-,
worin Phenyl- seinerseits gegebenenfalls ein-, zwei- oder dreifach, gleich oder verschieden substituiert sein kann mit: Halogen, Cyano, C₁-C₄-Alkyl-, C₂-C₄-Alkenyl-, C₂-C₄-Alkinyl-, C₁-C₄-Alkoxy-, Halogen-C₁-C₄-Alkyl-, Halogen-C₁-C₄-Alkoxy-, und
worin C₃-C₈-Cycloalkyl- und 4-bis 8-gliedriges Heterocycloalkylihrerseits gegebenenfalls ein- oder zweifach, gleich oder verschieden substituiert sein können mit C₁-C₃-Alkyl-,
oder
für C₃-C₈-Cycloalkyl- oder 4-bis 8-gliedriges Heterocycloalkyl- steht, die gegebenenfalls ein- oder zweifach, gleich oder verschieden substituiert sein können mit C₁-C₃-Alkyl- oder C₁-C₄-Alkoxycarbonyl-,
oder
für Phenyl steht, das gegebenenfalls ein- oder zweifach, gleich oder verschieden substituiert sein kann mit Halogen, C₁-C₃-Alkyl- oder 4-8-gliedrigem Heterocycloalkyl-,
worin das 4-8-gliedrige Heterocycloalkyl- seinerseits gegebenenfalls ein- oder zweifach, gleich oder verschieden substituiert sein kann mit C₁-C₃-Alkyl- oder C₁-C₄-Alkoxycarbonyl-,
R⁷ für Wasserstoff steht,
oder
für C₁-C₆-Alkyl- steht, das gegebenenfalls ein-, zwei-, oder dreifach, gleich oder verschieden substituiert sein kann mit: Hydroxy, Oxo, Fluor, Cyano, C₁-C₄-Alkoxy-, Halogen-C₁-C₄-alkoxy-, -NR⁹R¹⁰, C₃-C₈-Cycloalkyl-, C₄-C₈-Cycloalkenyl-, 4- bis 8-gliedrigem Heterocycloalkyl-, 4- bis 8-gliedrigem Heterocycloalkeiryl-, C₅-C₁₁-Spirocycloalkyl-, C₅-C₁₁-Heterospirocycloalkyl-, verbrücktem C₆-C₁₂-Cycloalkyl-, verbrücktem C₆-C₁₂-Heterocycloalkyl-, C₆-C₁₂-Bicycloalkyl-, C₆-C₁₂-Heterobicycloalkyl-, Phenyl-, 5- bis 6-gliedrigem Heteroaryl-,
worin C₃-C₈-Cycloalkyl-, C₄-C₈-Cycloalkenyl-, 4- bis 8-gliedriges Heterocycloalkyl-, 4- bis 8-gliedriges Heterocycloalkenyl-, C₅-C₁₁-Spirocycloalkyl-, C₅-C₁₁-Heterospirocycloalkyl-, verbrücktes C₆-C₁₂-Cycloalkyl-, verbrücktes C₆-C₁₂-Heterocycloalkyl-, C₆-C₁₂-Bicycloalkyl-, C₆-C₁₂-Heterobicycloalkyl- ihrerseits gegebenenfalls ein- oder zweifach, gleich oder verschieden substituiert sein können mit: Hydroxy, Fluor, Oxo, Cyano, C₁-C₃-Alkyl-, Fluor-C₁-C₃-Alkyl-, C₃-C₆-Cycloalkyl-, Cyclopropylmethyl-, C₁-C₃-Alkylcarbonyl-, C₁-C₄-Alkoxycarbonyl- oder -NR⁹R¹⁰, und
worin Phenyl- und 5- bis 6-gliedriges Heteroaryl- gegebenenfalls ein- oder zweifach, gleich oder verschieden substituiert sein können mit: Halogen, Cyano, Trifluormethyl-, C₁-C₃-Alkyl-, C₁-C₃-Alkoxy-,
oder für C₃-C₆-Alkenyl- oder C₃-C₆-Alkinyl- steht,
oder
für C₃-C₈-Cycloalkyl-, C₄-C₈-Cycloalkenyl-, C₅-C₁₁-Spirocycloalkyl-, verbrücktes C₆-C₁₂-Cycloalkyl- oder C₆-C₁₂-Bicycloalkyl- steht, die gegebenenfalls ein- oder zweifach, gleich oder verschieden substituiert sein können mit: Hydroxy, Oxo, Cyano, Fluor, C₁-C₃-Alkyl, C₁-C₃-Alkoxy, Trifluormethyl, -NR⁹R¹⁰,
oder
für 4- bis 8-gliedriges Heterocycloalkyl-, 4- bis 8-gliedriges Heterocycloalkenyl-, C₅-C₁₁-Heterospirocycloalkyl-, verbrücktes C₆-C₁₂-Heterocycloalkyl- oder C₆-C₁₂-Heterobicycloalkyl- steht, die gegebenenfalls ein- oder zweifach, gleich oder verschieden substituiert sein können mit: Hydroxy, Fluor, Oxo, Cyano, C₁-C₃-Alkyl-, Fluor-C₁-C₃-Alkyl-, C₃-C₆-Cycloalkyl-, Cyclopropylmethyl-, C₁-C₃-Alkylcarbonyl-, C₁-C₄-Alkoxycarbonyl- oder -NR⁹R¹⁰,
R⁸ für Wasserstoff oder für gegebenenfalls ein- oder zweifach, gleich oder verschieden mit Hydroxy, Oxo oder C₁-C₃-Alkoxy- substituiertes C₁-C₃-Alkyl-, oder für Fluor-C₁-C₃-Alkyl steht,
oder
R⁷ und R⁸ gemeinsam mit dem Stickstoffatom, an welches sie gebunden sind, für 4-bis 8-gliedriges Heterocycloalkyl, 4-bis 8-gliedriges Heterocycloalkenyl-, C₅-C₁₁-Heterospirocycloalkyl-, verbrücktes C₆-C₁₂-Heterocycloalkyl- oder C₆-C₁₂-Heterobicycloalkyl- stehen, die gegebenenfalls ein- oder zweifach, gleich oder verschieden substituiert sein können mit: Hydroxy, Fluor, Oxo, Cyano, C₁-C₃-Alkyl-, Fluor-C₁-C₃-Alkyl-, C₃-C₆-Cycloalkyl-, Cyclopropylmethyl-, C₁-C₃-Alkylcarbonyl-, C₁-C₄-Alkoxycarbonyl- oder -NR⁹R¹⁰,
R⁹ und R¹⁰ unabhängig voneinander für Wasserstoff oder für gegebenenfalls ein- oder zweifach, gleich oder verschieden mit Hydroxy, Oxo, C₁-C₃-Alkoxysubstituiertes C₁-C₃-Alkyl, Fluor-C₁-C₃-Alkyl, oder für 4-bis 8-gliedriges Heterocycloalkyl- stehen, worin das 4-bis 8-gliedrige Heterocycloalkyl- seinerseits gegebenenfalls ein- oder zweifach, gleich oder verschieden substituiert sein kann mit C₁-C₃-Alkyl-,
oder
R⁹ und R¹⁰ gemeinsam mit dem Stickstoffatom, an das sie gebunden sind, für 4- bis 8-gliedriges Heterocycloalkyl- stehen, das gegebenenfalls ein- oder zweifach, gleich oder verschieden substituiert sein kann mit: Hydroxy, Fluor, Oxo, Cyano, C₁-C₃-Alkyl-, Fluor-C₁-C₃-Alkyl-, C₃-C₆-Cycloalkyl-, Cyclopropylmethyl-, C₁-C₃-Alkylcarbonyl-oder C₁-C₄-Alkoxycarbonyl-,
R¹¹ für C₁-C₆-Alkyl- oder Phenyl-C₁-C₃-Alkyl- steht,
sowie deren Diastereomere, Racemate, Polymorphe und physiologisch verträglichen Salze.

2. Verbindungen der allgemeinen Formel (I) gemäß Anspruch 1, in der
A für -NH- oder -N(C₁-C₃-Alkyl)- steht,
X für -N- oder -CH- steht,
n für 0,1 oder 2 steht,
R¹ für eine Gruppe -C(=O)NR⁷R⁸ oder -S(=O)₂NR⁷R⁸ steht,
oder
für Oxazolyl-, Thiazolyl-, Oxadiazolyl- oder Thiadiazolyl- steht, die gegebenenfalls ein- oder zweifach, gleich oder verschieden substituiert sein können mit Halogen, Cyano, C₁-C₃-Alkyl-, Trifluormethyl-, C₁-C₃-Alkoxy-, Trifluonnethoxy- oder -NR⁹R¹⁰,
R² für Wasserstoff, Fluor, Chlor, Cyano, Methyl-, Methoxy-, Ethyl- oder Ethoxy- steht, und falls n für 2 steht, kann R² gleich oder verschieden sein,
oder
R¹ und R² gemeinsam für eine Gruppe *-S(=O)₂-NR⁸-CH₂-** oder *-C(=O)-NR⁸-CH₂-** stehen, worin "*" den Anknüpfungspunkt von R¹ am in Formel (I) enthaltenen Phenylring bedeutet, und worin "**" ein diesem Anknüfungspunkt benachbartes Kohlenstoffatom dieses Phenylringes bedeutet,
R³ für Methyl- oder Ethyl- steht,
R⁴ für Wasserstoff, Methyl- oder Ethyl- steht,
R⁵ für Wasserstoff, Methyl- oder Ethyl- steht,
R⁶ für C₂-C₅-Alkyl- steht,
oder
für Methyl- oder Ethyl- steht, das einfach substituiert ist mit C₁-C₃-Alkoxy-, Phenyl- oder 4-bis 8-gliedrigem Heterocycloalkyl-, worin Phenyl- seinerseits gegebenenfalls ein-, zwei- oder dreifach, gleich oder verschieden substituiert sein kann mit: Fluor, Chlor, Brom, Cyano, C₁-C₃-Alkyl-, C₁-C₃-Alkoxy-, und
worin das 4-bis 8-gliedrige Heterocycloalkyl- seinerseits gegebenenfalls ein- oder zweifach, gleich oder verschieden substituiert sein kann mit Methyl-,
oder
für C₃-C₈-Cycloalkyl- oder 4-bis 8-gliedriges Heterocycloalkyl- steht, die gegebenenfalls ein- oder zweifach, gleich oder verschieden substituiert sein können mit C₁-C₃-Alkyl- oder C₁-C₄-Alkoxycarbonyl-,
oder
für Phenyl steht, das gegebenenfalls ein- oder zweifach, gleich oder verschieden substituiert sein kann mit Fluor, Chlor, Methyl- oder 6-gliedrigem Heterocycloalkyl-,
worin das 6-gliedrige Heterocycloalkyl- seinerseits gegebenenfalls einfach substituiert sein kann mit Methyl- oder *tert*-Butoxycarbonyl-,
R⁷ für Wasserstoff steht,
oder
für C₁-C₆-Alkyl- steht, das gegebenenfalls ein-, zwei-, oder dreifach, gleich oder verschieden substituiert sein kann mit: Hydroxy, Oxo, Fluor, Cyano, C₁-C₃-Alkoxy-, Fluor-C₁-C₃-alkoxy-, -NR⁹R¹⁰, 4- bis 8-gliedrigem Heterocycloalkyl-, Phenyl-, 5- bis 6-gliedrigem Heteroaryl-,
worin das 4- bis 8-gliedrige Heterocycloalkyl- seinerseits gegebenenfalls einfach substituiert sein kann mit: Hydroxy, Oxo, C₁-C₃-Alkyl-, Fluor-C₁-C₃-Alkyl-, Cyclopropyl-, Cyclopropylmethyl-, Acetyl- oder *tert-*Butoxycarbonyl-,
oder
für C₃-C₆-Cycloalkyl- steht, das gegebenenfalls ein- oder zweifach, gleich oder verschieden substituiert sein kann mit: Hydroxy, Oxo, Cyano, Fluor,-NR⁹R¹⁰,
oder
für 4- bis 8-gliedriges Heterocycloalkyl-, C₆-C₈-Heterospirocycloalkyl-, verbrücktes C₆-C₁₀-Heterocycloalkyl- oder C₆-C₁₀-Heterobicycloalkylsteht, die gegebenenfalls ein- oder zweifach, gleich oder verschieden substituiert sein können mit: Hydroxy, Oxo, C₁-C₃-Alkyl-, Fluor-C₁-C₃-Alkyl-, Cyclopropyl-, Cyclopropylmethyl-, Acetyl- oder *tert-*Butoxycarbonyl-,
R⁸ für Wasserstoff oder C₁-C₃-Alkyl- steht,
oder
R⁷ und R⁸ gemeinsam mit dem Stickstoffatom, an welches sie gebunden sind, für 4-bis 8-gliedriges Heterocycloalkyl-, C₆-C₈-Heterospirocycloalkyl-, verbrücktes C₆-C₁₀-Heterocycloalkyl- oder C₆-C₁₀-Heterobicycloalkylstehen,
die gegebenenfalls ein- oder zweifach, gleich oder verschieden substituiert sein können mit: Hydroxy, Fluor, Oxo, C₁-C₃-Alkyl-, Fluor-C₁-C₃-Alkyl-, Cyclopropyl-, Cyclopropylmethyl-, Acetyl- oder *tert*-Butoxycarbonyl-,
R⁹ und R¹⁰ unabhängig voneinander für Wasserstoff oder für gegebenenfalls einfach mit Hydroxy oder Oxo substituiertes C₁-C₃-Alkyl- oder für Trifluormethyl-, oder für 6-gliedriges Heterocycloalkyl- stehen,
worin das 6-gliedrige Heterocycloalkyl- seinerseits gegebenenfalls ein- oder zweifach, gleich oder verschieden substituiert sein kann mit C₁-C₃-Alkyl-,
oder
R⁹ und R¹⁰ gemeinsam mit dem Stickstoffatom, an das sie gebunden sind, für 4- bis 7-gliedriges Heterocycloalkyl- stehen, das gegebenenfalls ein- oder zweifach, gleich oder verschieden substituiert sein kann mit: Hydroxy, Fluor, Oxo, C₁-C₃-Alkyl-, Fluor-C₁-C₃-Alkyl-, Cyclopropyl-, Cyclopropylmethyl-, Acetyl- oder *tert*-Butoxycarbonyl-,
sowie deren Diastereomere, Racemate, Polymorphe und physiologisch verträglichen Salze.

3. Verbindungen der allgemeinen Formel (I) gemäß den Ansprüchen 1 und 2, in der
A für -NH- oder -N(Methyl)- steht,
X für -N- oder -CH- steht,
n für 0 oder 1 steht,
R¹ für eine Gruppe -C(=O)NR⁷R⁸ oder -S(=O)₂NR⁷R⁸ steht,
oder
für Oxazolyl- oder Oxadiazolyl-, die gegebenenfalls ein- oder zweifach, gleich oder verschieden substituiert sein können mit C₁-C₃-Alkyl-,
R² für Wasserstoff, Fluor, Chlor, Methyl- oder Methoxy- steht,
oder
R¹ und R² gemeinsam für eine Gruppe *-S(=O)₂-NR⁸-CH₂-** stehen, worin "*" den Anknüpfungspunkt von R¹ am in Formel (I) enthaltenen Phenylring bedeutet, und worin "**" ein diesem Anknüfungspunkt benachbartes Kohlenstoffatom dieses Phenylringes bedeutet,
R³ für Methyl- steht,
R⁴ für Methyl- oder Ethyl- steht,
R⁵ für Wasserstoff steht,
R⁶ für C₃-C₅-Alkyl- oder für 2-Methoxyethyl- steht,
oder
für Methyl- steht, das einfach substituiert ist mit Phenyl- oder 4-bis 6-gliedrigem Heterocycloalkyl-,
worin Phenyl- seinerseits gegebenenfalls ein-, oder zweifach, gleich oder verschieden substituiert sein kann mit: Fluor, Chlor, Cyano, Methyl-, Methoxy-, und
worin das 4-bis 6-gliedrige Heterocycloalkyl- seinerseits gegebenenfalls einfach substituiert sein kann mit Methyl-,
oder
für C₃-C₈-Cycloalkyl- oder für 4-bis 6-gliedriges Heterocycloalkyl- steht, die gegebenenfalls ein- oder zweifach, gleich oder verschieden substituiert sein können mit C₁-C₃-Alkyl- oder C₁-C₄-Alkoxycarbonyl-,
oder
für Phenyl steht, das gegebenenfalls ein- oder zweifach, gleich oder verschieden substituiert sein kann mit Fluor, Chlor, Methyl- oder *N-tert-*Butoxycarbonylpiperazinyl-,
R⁷ für Wasserstoff steht,
oder
für C₁-C₄-Alkyl- steht, das gegebenenfalls einfach substituiert sein kann mit -NR⁹R¹⁰ oder 4- bis 8-gliedrigem Heterocycloalkyl-,
worin das 4- bis 8-gliedrige Heterocycloalkyl- seinerseits gegebenenfalls einfach substituiert sein kann mit: Oxo, C₁-C₃-Alkyl-, Fluor-C₁-C₃-Alkyl-, Cyclopropyl- oder Cyclopropylmethyl-,
oder
für C₃-C₆-Cycloalkyl- steht, das gegebenenfalls einfach substituiert sein kann mit Hydroxy, Fluor oder -NR⁹R¹⁰,
oder
für 4- bis 8-gliedriges Heterocycloalkyl- steht, das gegebenenfalls ein- oder zweifach, gleich oder verschieden substituiert sein kann mit: Oxo, C₁-C₃-Alkyl-, Fluor-C₁-C₃-Alkyl-, Cyclopropyl- oder Cyclopropylmethyl-,
R⁸ für Wasserstoff, Methyl- oder Ethyl- steht,
oder
R⁷ und R⁸ gemeinsam mit dem Stickstoffatom, an welches sie gebunden sind, für 4-bis 6-gliedriges Heterocycloalkyl- oder C₆-C₈-Heterospirocycloalkylstehen, die gegebenenfalls ein- oder zweifach, gleich oder verschieden substituiert sein können mit: Fluor, Oxo, C₁-C₃-Alkyl-, Fluor-C₁-C₃-Alkyl-, Cyclopropyl- oder Cyclopropylmethyl-,
R⁹ und R¹⁰ unabhängig voneinander für Wasserstoff oder für gegebenenfalls einfach mit Hydroxy oder Oxo substituiertes C₁-C₃-Alkyl-, Trifluormethyl-,
oder
für *N*-Methylpiperidinyl- stehen,
oder
R⁹ und R¹⁰ gemeinsam mit dem Stickstoffatom, an das sie gebunden sind, für 4- bis 7gliedriges Heterocycloalkyl- stehen, das gegebenenfalls ein- oder zweifach, gleich oder verschieden substituiert sein kann mit: Fluor, Oxo, C₁-C₃-Alkyl-, Fluor-C₁-C₃-Alkyl-, Cyclopropyl- oder Cyclopropylmethyl-,
sowie deren Diastereomere, Racemate, Polymorphe und physiologisch verträglichen Salze.

4. Verbindungen der allgemeinen Formel (I) gemäß den Ansprüchen 1 bis 3, in der
A für -NH- oder -N(Methyl)- steht,
X für -N- steht,
n für 0 oder 1 steht,
R¹ für eine Gruppe -C(=O)NR⁷R⁸ oder -S(=O)₂NR⁷R⁸ steht,
oder
für Oxazolyl- oder Oxadiazolyl-, die gegebenenfalls ein- oder zweifach, gleich oder verschieden substituiert sein können mit C₁-C₃-Alkyl-,
R² für Wasserstoff, Fluor, Chlor, Methyl- oder Methoxy- steht,
oder
R¹ und R² gemeinsam für eine Gruppe *-S(=O)₂-NR⁸-CH₂-** stehen, worin "*" den Anknüpfungspunkt von R¹ am in Formel (I) enthaltenen Phenylring bedeutet, und worin "**" ein diesem Anknüfungspunkt benachbartes Kohlenstoffatom dieses Phenylringes bedeutet,
R³ für Methyl- steht,
R⁴ für Methyl- oder Ethyl- steht,
R⁵ für Wasserstoff steht,
R⁶ für C₃-C₅-Alkyl- oder für 2-Methoxyethyl- steht,
oder
für Methyl- steht, das einfach substituiert ist mit Phenyl- oder 4-bis 6-gliedrigem Heterocycloalkyl-,
worin Phenyl- seinerseits gegebenenfalls ein-, oder zweifach, gleich oder verschieden substituiert sein kann mit: Fluor, Chlor, Cyano, Methyl-, Methoxy-, und
worin das 4-bis 6-gliedrige Heterocycloalkyl- seinerseits gegebenenfalls einfach substituiert sein kann mit Methyl-,
oder
für C₃-C₈-Cycloalkyl- oder für 4-bis 6-gliedriges Heterocycloalkyl- steht, die gegebenenfalls ein- oder zweifach, gleich oder verschieden substituiert sein können mit C₁-C₃-Alkyl- oder C₁-C₄-Alkoxycarbonyl-,
oder
für Phenyl steht, das gegebenenfalls ein- oder zweifach, gleich oder verschieden substituiert sein kann mit Fluor, Chlor, Methyl- oder *N-tert-*Butoxycarbonylpiperazinyl-,
R⁷ für Wasserstoff steht,
oder
für C₁-C₄-Alkyl- steht, das gegebenenfalls einfach substituiert sein kann mit -NR⁹R¹⁰ oder 4- bis 8-gliedrigem Heterocycloalkyl-,
worin das 4- bis 8-gliedrige Heterocycloalkyl- seinerseits gegebenenfalls einfach substituiert sein kann mit: Oxo, C₁-C₃-Alkyl-, Fluor-C₁-C₃-Alkyl-, Cyclopropyl- oder Cyclopropylmethyl-,
oder
für C₃-C₆-Cycloalkyl- steht, das gegebenenfalls einfach substituiert sein kann mit Hydroxy, Fluor oder -NR⁹R¹⁰,
oder
für 4- bis 8-gliedriges Heterocycloalkyl-, das gegebenenfalls ein- oder zweifach, gleich oder verschieden substituiert sein kann mit: Oxo, C₁-C₃-Alkyl-, Fluor-C₁-C₃-Alkyl-, Cyclopropyl- oder Cyclopropylmethyl-,
R⁸ für Wasserstoff, Methyl- oder Ethyl- steht,
oder
R⁷ und R⁸ gemeinsam mit dem Stickstoffatom, an welches sie gebunden sind, für 4-bis 6-gliedriges Heterocycloalkyl- oder C₆-C₈-Heterospirocycloalkylstehen, die gegebenenfalls ein- oder zweifach, gleich oder verschieden substituiert sein können mit: Fluor, Oxo, C₁-C₃-Alkyl-, Fluor-C₁-C₃-Alkyl-, Cyclopropyl- oder Cyclopropylmethyl-,
R⁹ und R¹⁰ unabhängig voneinander für Wasserstoff oder für gegebenenfalls einfach mit Hydroxy oder Oxo substituiertes C₁-C₃-Alkyl-, Trifluormethyl-, oder für *N*-Methylpiperidinyl- stehen,
oder
R⁹ und R¹⁰ gemeinsam mit dem Stickstoffatom, an das sie gebunden sind, für 4- bis 7-gliedriges Heterocycloalkyl- stehen, das gegebenenfalls ein- oder zweifach, gleich oder verschieden substituiert sein kann mit: Fluor, Oxo, C₁-C₃-Alkyl-, Fluor-C₁-C₃-Alkyl-, Cyclopropyl- oder Cyclopropylmethyl-,
sowie deren Diastereomere, Racemate, Polymorphe und physiologisch verträglichen Salze.

5. Verbindungen der allgemeinen Formel (I) gemäß den Ansprüchen 1 bis 3, in der
A für -NH- oder -N(Methyl)- steht,
X für -CH- steht,
n für 0 oder 1 steht,
R¹ für eine Gruppe -C(=O)NR⁷R⁸ oder -S(=O)₂NR⁷R⁸ steht,
oder
für Oxazolyl- oder Oxadiazolyl-, die gegebenenfalls ein- oder zweifach, gleich oder verschieden substituiert sein können mit C₁-C₃-Alkyl-,
R² für Wasserstoff, Fluor, Chlor, Methyl- oder Methoxy- steht,
oder
R¹ und R² gemeinsam für eine Gruppe *-S(=O)₂-NR⁸-CH₂-** stehen, worin "*" den Anknüpfungspunkt von R¹ am in Formel (I) enthaltenen Phenylring bedeutet, und worin "**" ein diesem Anknüfungspunkt benachbartes Kohlenstoffatom dieses Phenylringes bedeutet,
R³ für Methyl- steht,
R⁴ für Methyl- oder Ethyl- steht,
R⁵ für Wasserstoff steht,
R⁶ für C₃-C₅-Alkyl- oder für 2-Methoxyethyl- steht,
oder
für Methyl- steht, das einfach substituiert ist mit Phenyl- oder 4-bis 6-gliedrigem Heterocycloalkyl-, worin Phenyl- seinerseits gegebenenfalls ein-, oder zweifach, gleich oder verschieden substituiert sein kann mit: Fluor, Chlor, Cyano, Methyl-, Methoxy-, und
worin das 4-bis 6-gliedrige Heterocycloalkyl- seinerseits gegebenenfalls einfach substituiert sein kann mit Methyl-,
oder
für C₃-C₈-Cycloalkyl- oder für 4-bis 6-gliedrige Heterocycloalkyl- steht, die gegebenenfalls ein- oder zweifach, gleich oder verschieden substituiert sein können mit C₁-C₃-Alkyl- oder C₁-C₄-Alkoxycarbonyl-,
oder
für Phenyl steht, das gegebenenfalls ein- oder zweifach, gleich oder verschieden substituiert sein kann mit Fluor, Chlor, Methyl- oder *N-tert-*Butoxycarbonylpiperazinyl-,
R⁷ für Wasserstoff steht,
oder
für C₁-C₄-Alkyl- steht, das gegebenenfalls einfach substituiert sein kann mit -NR⁹R¹⁰ oder 4- bis 8-gliedrigem Heterocycloalkyl-,
worin das 4- bis 8-gliedrige Heterocycloalkyl- seinerseits gegebenenfalls einfach substituiert sein kann mit: Oxo, C₁-C₃-Alkyl-, Fluor-C₁-C₃-Alkyl-, Cyclopropyl- oder Cyclopropylmethyl-,
oder
für C₃-C₆-Cycloalkyl- steht, das gegebenenfalls einfach substituiert sein kann mit Hydroxy, Fluor oder -NR⁹R¹⁰,
oder
für 4- bis 8-gliedriges Heterocycloalkyl-, das gegebenenfalls ein- oder zweifach, gleich oder verschieden substituiert sein kann mit: Oxo, C₁-C₃-Alkyl-, Fluor-C₁-C₃-Alkyl-, Cyclopropyl- oder Cyclopropylmethyl-,
R⁸ für Wasserstoff, Methyl- oder Ethyl- steht,
oder
R⁷ und R⁸ gemeinsam mit dem Stickstoffatom, an welches sie gebunden sind, für 4-bis 6-gliedriges Heterocycloalkyl- oder C₆-C₈-Heterospirocycloalkylstehen, die gegebenenfalls ein- oder zweifach, gleich oder verschieden substituiert sein können mit: Fluor, Oxo, C₁-C₃-Alkyl-, Fluor-C₁-C₃-Alkyl-, Cyclopropyl- oder Cyclopropylmethyl-,
R⁹ und R¹⁰ unabhängig voneinander für Wasserstoff oder für gegebenenfalls einfach mit Hydroxy oder Oxo substituiertes C₁-C₃-Alkyl-, Trifluormethyl-, oder für *N*-Methylpiperidinyl- stehen,
oder
R⁹ und R¹⁰ gemeinsam mit dem Stickstoffatom, an das sie gebunden sind, für 4- bis 7-gliedriges Heterocycloalkyl- stehen, das gegebenenfalls ein- oder zweifach, gleich oder verschieden substituiert sein kann mit: Fluor, Oxo, C₁-C₃-Alkyl-, Fluor-C₁-C₃-Alkyl-, Cyclopropyl- oder Cyclopropylmethyl-,
sowie deren Diastereomere, Racemate, Polymorphe und physiologisch verträglichen Salze.

6. Verbindungen der allgemeinen Formel (I) gemäß den Ansprüchen 1 bis 3, in der
A für -NH- oder -N(Methyl)- steht,
X für -N- oder -CH- steht,
n für 0 oder 1 steht,
R¹ für eine Gruppe -C(=O)NR⁷R⁸ oder -S(=O)₂NR⁷R⁸ steht, oder für Oxazolyl- oder Oxadiazolyl-, die gegebenenfalls ein- oder zweifach substituiert sein können mit Methyl-,
R² für Wasserstoff, Methyl- oder Methoxy- steht,
oder
R¹ und R² gemeinsam für eine Gruppe *-S(=O)₂-NH-CH₂-** stehen, worin "*" den Anknüpfungspunkt von R¹ am in Formel (I) enthaltenen Phenylring, bedeutet, und worin "**" ein diesem Anknüfungspunkt benachbartes Kohlenstoffatom dieses Phenylringes bedeutet,
R³ für Methyl- steht,
R⁴ für Methyl- steht,
R⁵ für Wasserstoff steht,
R⁶ für Isopropyl-, Isobutyl- oder 2-Methoxyethyl- steht,
oder
für Benzyl- steht, dessen Phenylteil gegebenenfalls ein- oder zweifach, gleich oder verschieden substituiert sein kann mit: Fluor, Methoxy-,
oder
für C₅-C₇-Cycloalkyl- steht, das gegebenenfalls ein- oder zweifach substituiert sein kann mit Methyl-,
oder
für Tetrahydrofuranyl-, Tetrahydropyranyl- oder Piperidinyl- steht,
worin Piperidinyl- gegebenenfalls einfach substitiert sein kann mit Methyl- oder *tert*-Butoxycarbonyl-,
oder
für Phenyl steht, dass gegebenenfalls einfach substituiert sein kann mit Fluor, Methyl- oder *N*-*tert*-Butoxycarbonylpiperazinyl-,
R⁷ für Wasserstoff steht,
oder
für C₁-C₃-Alkyl- steht, das gegebenenfalls einfach substituiert sein kann mit -NR⁹R¹⁰ oder *N*-Methylpiperidinyl-,
oder
für Cyclopropyl- steht, oder für Cyclohexyl-,
worin Cyclohexyl- gegebenenfalls einfach substituiert sein kann mit Hydroxy- oder -NR⁹R¹⁰,
oder
für 4- bis 6-gliedriges Heterocycloalkyl- steht, das gegebenenfalls einfach substituiert sein kann mit Methyl-,
R⁸ für Wasserstoff, Methyl- oder Ethyl- steht,
oder
R⁷ und R⁸ gemeinsam mit dem Stickstoffatom, an das sie gebunden sind, für 4- bis 6-gliedriges Heterocycloalkyl- stehen, das gegebenenfalls ein- oder zweifach substituiert sein kann mit Fluor, oder das gegebenenfalls einfach substituiert sein kann mit Methyl-, Isopropyl-, 2,2,2-Trifluoroethyl- oder Cyclopropylmethyl-,
oder
für 6-Azaspiro[3.3]heptyl-oder für 2-Oxa-6-azaspiro[3.3]heptyl- stehen,
R⁹ und R¹⁰ unabhängig voneinander für Wasserstoff, C₁-C₃-Alkyl- oder für *N*-Methylpiperidinyl- stehen,
oder
R⁹ und R¹⁰ gemeinsam mit dem Stickstoffatom, an das sie gebunden sind, für 6-gliedriges Heterocycloalkyl- stehen, das gegebenenfalls ein- oder zweifach substituiert sein kann mit Fluor, oder das gegebenenfalls einfach substituiert sein kann mit Methyl-, 2,2,2-Trifluorethyl-, Cyclopropyl- oder Cyclopropylmethyl-,
sowie deren Diastereomere, Racemate, Polymorphe und physiologisch verträglichen Salze.

7. Verbindungen der allgemeinen Formel (I) gemäß den Ansprüchen 1 bis 3 und 6, in der
A für -NH- oder -N(Methyl)- steht,
X für -N- oder -CH- steht,
n für 0 oder 1 steht,
R¹ für eine Gruppe -C(=O)NR⁷R⁸ oder -S(=O)₂NR⁷R⁸ steht,
oder
für 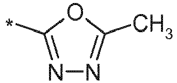 steht, worin "*" den Ankuüpfungspunkt an den Rest des Moleküls bedeutet,
R² für Wasserstoff, Methyl- oder Methoxy- steht, oder
R¹ und R² gemeinsam mit dem Phenylring, an den sie gebunden sind, für 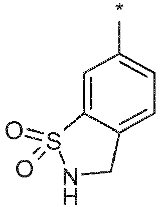 stehen, worin "*" den Anknüpfungspunkt an den Rest des Moleküls bedeutet,
R³ für Methyl- steht,
R⁴ für Methyl- steht,
R⁵ für Wasserstoff steht,
R⁶ für Isopropyl-, Isobutyl-, 2-Methoxyethyl-, Benzyl-, 4-Methoxybenzyl-, 2,6-Difluorbenzyl-, Cyclopentyl-, Cyclohexyl-, Cycloheptyl-, Tetrahydropyran-4-yl-, Phenyl-, 3-Methylphenyl- oder 4-Fluorphenylsteht,
oder
für 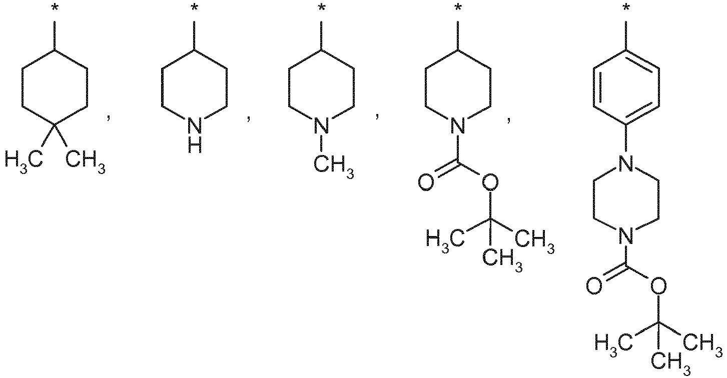 steht, worin "*" jeweils den Anknüpfungspunkt an den Rest des Moleküls bedeutet,
R⁷ für Wasserstoff, Methyl-, Ethyl-, Isopropyl- oder Cyclopropyl- steht,
oder
für 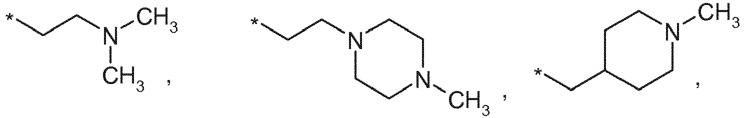 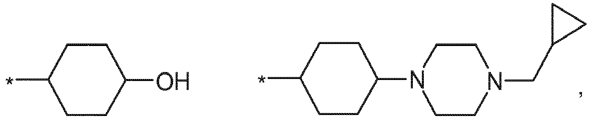 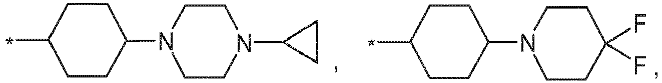 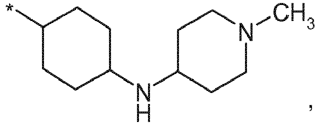 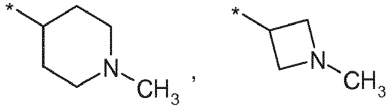 steht,
wobei "*" jeweils den Anknüpfungspunkt an den Rest des Moleküls bedeutet,
R⁸ für Wasserstoff, Methyl- oder Ethyl- steht,
oder
R⁷ und R⁸ gemeinsam mit dem Stickstoffatom, an welches sie gebunden sind, für 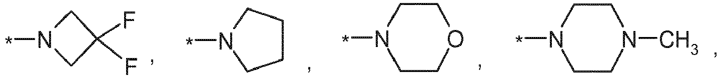 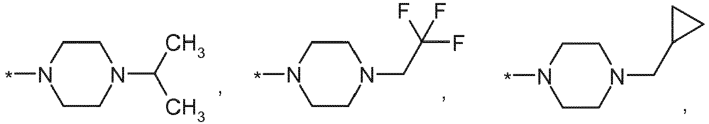 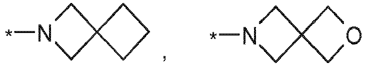 stehen,
wobei "*" jeweils den Anknüpfungspunkt an den Rest des Moleküls bedeutet,
sowie deren Diastereomere, Racemate, Polymorphe und physiologisch verträglichen Salze.

8. Verbindungen der allgemeinen Formel (I) gemäß den Ansprüchen 1 bis 7
(3*R*)-4-Cyclopentyl-1,3-dimethyl-6-({3-[(4-methylpiperazin-1-yl)carbonyl]phenyl}amino)-3,4-dihydropyrido[2,3-*b*]pyrazin-2(*H*)-on;
3-{[(3*R*)-4-Cyclopentyl-1,3-dimethyl-2-oxo-1,2,3,4-tetrahydropyrido[2,3-*b*]pyrazin-6-yl]amino}-*N*-(1-methylpiperidin-4-yl)benzamid;
3-{[(3*R*)-4-Cyclopentyl-1,3-dimethyl-2-oxo-1,2,3,4-tetrahydropyrido[2,3-*b*]pyrazin-6-yl]amino}-*N*-(1-methylazetidin-3-yl)benzamid;
3-{[(3*R*)-4-Cyclopentyl-1,3-dimethyl-2-oxo-1,2,3,4-tetrahydropyrido[2,3-*b*]pyrazin-6-yl]amino}-*N*-[2-(dimethylamino)ethyl]benzamid;
3-{[(3*R*)-4-Cyclopentyl-1,3-dimethyl-2-oxo-1,2,3,4-tetrahydropyrido[2,3-*b*]pyrazin-6-yl]amino}-*N*-{*trans*-4-[4-(cyclopropylmethyl)piperazin-1-yl]cyclohexyl}benzamid;
3-{[(3*R*)-1,3-Dimethyl-2-oxo-4-(tetrahydro-2*H*-pyran-4-yl)-1,2,3,4-tetrahydropyrido[2,3-*b*]pyrazin-6-yl]amino}-*N,N*-dimethylbenzolsulfonamid;
3-{[(3*R*)-1,3-Dimethyl-2-oxo-4-(tetrahydro-2*H*-pyran-4-yl)-1,2,3,4-tehahydrochinoxalin-6-yl]amino}-*N,N*-dimethylbenzolsulfonamid;
(3*R*)-1,3-Dimethyl-6-{[3-(morpholin-4-ylsulfonyl)phenyl]amino}-4-(tetrahydro-2*H*-pyran-4-yl)-3,4-dihydrochinoxalin-2(*H*)-on;
(3*R*)-1,3-Dimethyl-6-{[3-(morpholin-4-ylsulfonyl)phenyl]amino}-4-(tetrahydro-2*H*-pyran-4-yl)-3,4-dihydropyrido[2,3-*b*]pyrazin-2(1*H*)-on;
3-{[(3*R*)-4-(4-Methoxybenzyl)-1,3-dimethyl-2-oxo-1,2,3,4-tetrahydrochinoxalin-6-yl]amino}-*N,N*-dimethylbenzolsulfonamid;
(3*R*)-4-(4-Methoxybenzyl)-1,3-dimethyl-6-{[3-(morpholin-4-ylsulfonyl)phenyl]amino}-3,4-dihydrochinoxalin-2(1*H*)-on;
(3*R*)-1,3-Dimethyl-6-({3-[(4-methylpiperazin-1-yl)sulfonyl]phenyl}amino)-4-(tetrahydro-2*H*-pyran-4-yl)-3,4-dihydropyrido[2,3-*b*]pyrazin-2(1*H*)-on;
3-{[(3*R*)-1,3-Dimethyl-2-oxo-4-(tetrahydro-2*H*-pyran-4-yl)-1,2,3,4-tetrahydropyrido[2,3-*b*]pyrazin-6-yl]amino}-*N*-(1-methylpiperidin-4-yl)benzamid;
*N*-{*trans*-4-[4-(Cyclopropylmethyl)piperazin-1-yl]cyclohexyl}-3-{[(3*R*)-1,3-dimethyl-2-oxo-4-(tetrahydro-2*H*-pyran-4-yl)-1,2,3,4-tetrahydropyrido [2,3-*b*]pyrazin-6-yl]amino}benzamid;
(3*R*)-1,3-Dimethyl-6-({3-[(4-methylpiperazin-1-yl)carbonyl]phenyl}amino)-4-(tetrahydro-2*H*-pyran-4-yl)-3,4-dihydropyrido[2,3-*b*]pyrazin-2(1*H*)-on;
3-{[(3*R*)-1,3-Dimethyl-2-oxo-4-(tetrahydro-2*H*-pyran-4-yl)-1,2,3,4-tetrahydropyrido[2,3-*b*]pyrazin-6-yl]amino}-4-methoxy-*N*-(1-methylpiperidin-4-yl)benzamid;
(3*R*)-6-({2-Methoxy-5-[(4-methylpiperazin-1-yl)carbonyl]phenyl}amino)-1,3-dimethyl-4-(tetrahydro-2*H*-pyran-4-yl)-3,4-dihydropyrido[2,3-*b*]pyrazin-2(1*H*)-on;
*N*-{*trans*-4-[4-(Cyclopropylmethyl)piperazin-1-yl]cyclohexyl}-3-{[(3*R*)-1,3-dimethyl-2-oxo-4-(tetrahydro-2*H*-pyran-4-yl)-1,2,3,4-tetrahydropyrido[2,3-*b*]pyrazin-6-yl]amino}-4-methoxybenzamid;
*N*-[2-(Dimethylamino)ethyl]-3-{[(3*R*)-1,3-dimethyl-2-oxo-4-(tetrahydro-2*H*-pyran-4-yl)-1,2,3,4-tetrahydropyrido[2,3-*b*]pyrazin-6-yl]amino}-4-methoxybenzamid;
3-{[(3*R*)-1,3-Dimethyl-2-oxo-4-(tetrahydro-2*H*-pyran-4-yl)-1,2,3,4-tetrahydropyrido[2,3-*b*]pyrazin-6-yl]amino}-*N*-(1-methylpiperidin-4-yl)benzolsulfonamid;
*N*-[2-(Dimethylamino)ethyl]-3-{[(3*R*)-1,3-dimethyl-2-oxo-4-(tetrahydro-2*H*-pyran-4-yl)-1,2,3,4-tetrahydropyrido[2,3-*b*]pyrazin-6-yl]amino}benzolsulfonamid;
*N*-{*trans*-4-[4-(Cyclopropylmethyl)piperazin-1-yl]cyclohexyl}-3-{[4-(2,6-difluorbenzyl)-1,3-dimethyl-2-oxo-1,2,3,4-tetrahydrochinoxalin-6-yl]amino}benzamid;
3-{[(3*R*)-1,3-Dimethyl-2-oxo-4-(tetrahydro-2*H*-pyran-4-yl)-1,2,3,4-tetrahydropyrido[2,3-b]pyrazin-6-yl]amino}-*N*-ethylbenzolsulfonamid;
(3*R*)-1,3-Dimethyl-4-(1-methylpiperidin-4-yl)-6-{[3-(Pyrrolidin-1-ylsulfonyl)phenyl]amino}-3,4-dihydropyrido[2,3-b]pyrazin-2(1H)-on;
(3*R*)-6-{[2-Methoxy-5-(morpholin-4-ylsulfonyl)phenyl]amino}-1,3-dimethyl-4-(1-methylpiperidin-4-yl)-3,4-dihydropyrido[2,3-b]pyrazin-2(1H)-on;
*N-*Cyclopropyl-3-{[(3*R*)-1,3-dimethyl-2-oxo-4-(tetrahydro-2H-pyran-4-yl)-1,2,3,4-tetrahydropyrido[2,3-b]pyrazin-6-yl]amino}benzolsulfonamid;
(3*R*)-1,3-Dimethyl-6-{[3-(pyrrolidin-1-ylsulfonyl)phenyl]amino}-4-(tetrahydro-2H-pyran-4-yl)-3,4-dihydropyrido[2,3-b]pyrazin-2(1H)-on;
(3*R*)-6-{[2-Methoxy-5-(morpholin-4-ylsulfonyl)phenyl]amino}-1,3-dimethyl-4-(tetrahydro-2H-pyran-4-yl)-3,4-dihydropyrido[2,3-b]pyrazin-2(1H)-on;
(3*R*)-6-({3-[(3,3-Difluorazetidin-1-yl)sulfonyl]phenyl}amino)-1,3-dimethyl-4-(1-methylpiperidin-4-yl)-3,4-dihydropyrido[2,3-b]pyrazin-2(1H)-on;
(3*R*)-6-{[2-Methoxy-5-(2-oxa-6-azaspiro[3.3]hept-6-ylsulfonyl)phenyl]amino}-1,3-dimethyl-4-(tetrahydro-2H-pyran-4-yl)-3,4-dihydropyrido[2,3-b]pyrazin-2(1H)-on;
(3*R*)-6-({3-[(3,3-Difluorazetidin-1-yl)sulfonyl]phenyl}amino)-1,3-dimethyl-4-(tetrahydro-2H-pyran-4-yl)-3,4-dihydropyrido[2,3-b]pyrazin-2(1H)-on;
3-{[(3*R*)-1,3-Dimethyl-2-oxo-4-(tetrahydro-2H-pyran-4-yl)-1,2,3,4-tetrahydropyrido[2,3-b]pyrazin-6-yl]amino}-N,N-diethylbenzol sulfonamid;
3-{[(3*R*)-4-Benzyl-1,3-dimethyl-2-oxo-1,2,3,4-tetrahydropyrido[2,3-b]pyrazin-6-yl]amino}-*N*-(1-methylpiperidin-4-yl)benzolsulfonamid;
*N*-{*trans*-4-[4-(Cyclopropylmethyl)piperazin-1-yl]cyclohexyl}-3-{[(3*R*)-4-isopropyl-1,3-dimethyl-2-oxo-1,2,3,4-tetrahydropyrido[2,3-b]pyrazin-6-yl]amino}benzolsulfonamid;
*N-*{*trans*-4-[4-(Cyclopropylmethyl)piperazin-1-yl]cyclohexyl}-3-{[(3*R*)-1,3-dimethyl-2-oxo-4-(tetrahydro-2H-pyran-4-yl)-1,2,3,4-tetrahydropyrido[2,3-b]pyrazin-6-yl]amino}benzolsulfonamid;
*N*-{*trans*-4-[4-(Cyclopropylmethyl)piperazin-1-yl]cyclohexyl}-3-{[(3*S*)-1,3-dimethyl-2-oxo-4-(tetrahydro-2H-pyran-4-yl)-1,2,3,4-tetrahydropyrido[2,3-b]pyrazin-6-yl]amino}benzolsulfonamid;
(3*R*)-1,3-Dimethyl-4-(tetrahydro-2H-pyran-4-yl)-6-[(3-{[4-(2,2,2-trifluorethyl)piperazin-1-yl]sulfonyl}phenyl)amino]-3,4-dihydropyrido[2,3-b]pyrazin-2(1H)-on;
3-{[(3*R*)-4-Benzyl-1,3-dimethyl-2-oxo-1,2,3,4-tetrahydropyrido[2,3-b]pyrazin-6-yl]amino}-*N*-{*trans*-4-[4-(cyclopropyl-methyl)piperazin-1-yl] cyclohexyl} benzolsulfonamid;
3-{[(3*R*)-1,3-Dimethyl-2-oxo-4-(tetrahydro-2H-pyran-4-yl)-1,2,3,4-tetrahydropyrido[2,3-b]pyrazin-6-yl]amino}-*N-*methylbenzolsulfonamid;
(3*R*)-6-({2-Methoxy-5-[(4-methylpiperazin-1-yl)sulfonyl]phenyl}amino)-1,3-dimethyl-4-(tetrahydro-2H-pyran-4-yl)-3,4-dihydropyrido[2,3-b]pyrazin-2(1H)-on;
3-1[(3*R*)-1,3-Dimethyl-4-(1-methylpiperidin-4-yl)-2-oxo-1,2,3,4-tetra-hydropyrido[2,3-b]pyrazin-6-yl]amino}-*N,N*-dimethylbenzolsulfonamid;
3-{[(3*R*)-1,3-Dimethyl-2-oxo-4-(tetrahydro-2H-pyran-4-yl)-1,2,3,4-tetrahydropyrido[2,3-b]pyrazin-6-yl]amino}-*N*-isopropylbenzolsulfonamid;
(3*R*)-4-Isopropyl-1,3-dimethyl-6-({3-[(4-methylpiperazin-1-yl)sulfonyl]phenyl}amino)-3,4-dihydropyrido[2,3-b]pyrazin-2(1H)-on;
3-{[(3*R*)-1,3-Dimethyl-2-oxo-4-(tetrahydro-2H-pyran-4-yl)-1,2,3,4-tetrahydropyrido[2,3-b]pyrazin-6-yl]amino}-*N*-[(1-methylpiperidin-4-yl)methyl]benzolsulfonamid;
*N*-{*trans*-4-[4-(Cyclopropylmethyl)piperazin-1-yl]cyclohexyl}-3-{[(3*R*)-4-isobutyl-1,3-dimethyl-2-oxo-1,2,3,4-tetrahydropyrido[2,3-b]pyrazin-6-yl]amino}benzolsulfonamid;
(3*R*)-6-({2-Methoxy-5-[(4-methylpiperazin-1-yl)sulfonyl]phenyl}amino)-1,3-dimethyl-4-(1-methylpiperidin-4-yl)-3,4-dihydropyrido[2,3-b]pyrazin-2(1H)-on;
(3*R*)-1,3-Dimethyl-4-(1-methylpiperidin-4-yl)-6-{[3-(morpholin-4-ylsulfonyl)phenyl]amino}-3,4-dihydropyrido[2,3-b]pyrazin-2(1H)-on;
3-{[(3*R*)-4-Cycloheptyl-1,3-dimethyl-2-oxo-1,2,3,4-tetrahydropyrido[2,3-b]pyrazin-6-yl] amino}-*N*-(1-methyl-piperidin-4-yl)benzol-sulfonamid;
4-(2-Methoxyethyl)-1,3-dimethyl-6-{[3-(morpholin-4-ylsulfonyl)phenyl]amino}-3,4-dihydropyrido[2,3-b]pyrazin-2(1H)-on;
(3*R*)-1,3-Dimethyl-6-({3-[(4-methylpiperazin-1-yl)sulfonyl]phenyl}amino)-4-(1-methylpiperidin-4-yl)-3,4-dihydropyrido[2,3-b]pyrazin-2(1H)-on;
3-{[(3*R*)-4-Isopropyl-1,3-dimethyl-2-oxo-1,2,3,4-tetrahydropyrido[2,3-b]pyrazin-6-yl]amino}-*N*-(1-methylpiperidin-4-yl)benzolsulfonamid;
*tert*-Butyl-4-[(3*R*)-6-{[3-({*trans*-4-[4-(cyclopropyl-methyl)piperazin-1-yl]cyclohexyl}sulfamoyl)phenyl]amino}-1,3-dimethyl-2-oxo-2,3-dihydropyrido[2,3-b]pyrazin-4(1H)-yl]piperidin-1-carboxylat;
4-(2-Methoxyethyl)-1,3-dimethyl-6-({3-[(4-methylpiperazin-1-yl)sulfonyl]phenyl}amino)-3,4-dihydropyrido[2,3-b]pyrazin-2(1H)-on;
3-{[(3*R*)-1,3-Dimethyl-2-oxo-4-(tetrahydro-2H-pyran-4-yl)-1,2,3,4-tetrahydropyrido[2,3-b]pyrazin-6-yl](methyl)amino}-*N,N*-dimethyl-benzolsulfonamid;
*N*-[2-(Dimethylamino)ethyl]-3-{[(3*R*)-4-isopropyl-1,3-dimethyl-2-oxo-1,2,3,4-tetrahydro-pyrido[2,3-b]pyrazin-6-yl]amino}benzolsulfonamid;
(3*R*)-6-[(1,1-Dioxido-2,3-dihydro-1,2-benzothiazol-6-yl)amino]-1,3-dimethyl-4-(tetrahydro-2H-pyran-4-yl)-3,4-dihydropyrido[2,3-b]pyrazin-2(1H)-on;
*tert*-Butyl-4-[(3*R*)-1,3-dimethyl-6-({3-[(1-methylpiperidin-4-yl)sulfamoyl]phenyl}amino)-2-oxo-2,3-dihydro-pyrido[2,3-b]pyrazin-4(1H)-yl]piperidin-1-carboxylat;
3-{[(3*R*)-1,3-Dimethyl-2-oxo-4-(tetrahydro-2H-pyran-4-yl)-1,2,3,4-tetrahydropyrido[2,3-b]pyrazin-6-yl]amino} benzolsulfonamid;
*tert*-Butyl-4-[(3*R*)-6-[(3-{[2-(dimethylamino)ethyl]sulfamoyl}phenyl)amino]-1,3-dimethyl-2-oxo-2,3-dihydropyrido[2,3-b]pyrazin-4(1H)-yl]piperidin-1-carboxylat;
*tert*-Butyl-4-[(3*R*)-6-{[3-(2-azaspiro[3.3]hept-2-ylsulfonyl)phenyl]amino}-1,3-dimethyl-2-oxo-2,3-dihydropyrido[2,3-b]pyrazin-4(1H)-yl]piperidin-1-carboxylat;
*tert*-Butyl-4-[(3*R*)-6-{[3-(dimethylsulfamoyl)phenyl]amino}-1,3-dimethyl-2-oxo-2,3-dihydropyrido [2,3-b]pyrazin-4(1H)-yl]piperidin-1-carboxylat;
*tert*-Butyl-4-[(3*R*)-1,3-dimethyl-6-({3-[(4-methylpiperazin-1-yl)sulfonyl]phenyl}amino)-2-oxo-2,3-dihydropyrido [2,3-b]pyrazin-4(1H)-yl]piperidin-1-carboxylat;
5-{[(3*R*)-1,3-Dimethyl-2-oxo-4-(tetrahydro-2H-pyran-4-yl)-1,2,3,4-tetrahydropyrido[2,3-b]pyrazin-6-yl]amino}-2-methylbenzolsulfonamid;
1,3-Dimethyl-4-(3-methylphenyl)-6-{[3-(morpholin-4-ylsulfonyl)phenyl]amino}-3,4-dihydropyrido[2,3-b]pyrazin-2(1H)-on;
3-{[1,3-Dimethyl-4-(3-methylphenyl)-2-oxo-1,2,3,4-tetrahydro-pyrido[2,3-b]pyrazin-6-yl]amino}-*N*-(1-methylpiperidin-4-yl)benzolsulfonamid;
*tert*-Butyl-4-[(3*R*)-1,3-dimethyl-2-oxo-6-[(3-{[4-(2,2,2-trifluorethyl)piperazin-1-yl]sulfonyl}phenyl)amino]-2,3-dihydropyrido[2,3-b]pyrazin-4(1H)-yl]piperidin-1-carboxylat;
*tert*-Butyl-4-{4-[1,3-dimethyl-6-({3-[(4-methylpiperazin-1-yl)sulfonyl]phenyl}amino)-2-oxo-2,3-dihydropyrido[2,3-b]pyrazin-4(1H)-yl]phenyl}piperazin-1-carboxylat;
*tert*-Butyl-4-[(2*R*)-1-{[3-(dimethylsulfamoyl)phenyl]amino}-2,4-dimethyl-3-oxo-3,4-dihydro-chinoxalin-1(2H)-yl]piperidin-1-carboxylat;
3-{[(3*R*)-4-Benzyl-1,3-dimethyl-2-oxo-1,2,3,4-tetrahydrochinoxalin-6-yl]amino}-*N,N-*dimethylbenzolsulfonamid;
*tert*-Butyl-4-[(2*R*)-7-{[3-(dimethylsulfamoyl)phenyl](methyl)amino}-2,4-dimethyl-3-oxo-3,4-dihydrochinoxalin-1(2H)-yl]piperidin-1-carboxylat;
(3*R*)-1,3-Dimethyl-6-({3-[(4-methylpiperazin-1-yl)sulfonyl]phenyl}amino)-4-(tetrahydro-2H-pyran-4-yl)-3,4-dihydrochinoxalin-2(1H)-on;
3-{[(3*R*)-4-Benzyl-1,3-dimethyl-2-oxo-1,2,3,4-tetrahydropyrido[2,3-b]pyrazin-6-yl]amino}-*N*-{trans-4-[4-(cyclopropyl-methyl)piperazin-1-yl]cyclohexyl}benzamid;
3-{[(3*R*)-4-Benzyl-1,3-dimethyl-2-oxo-1,2,3,4-tetrahydropyrido[2,3-b]pyrazin-6-yl]amino}-*N*-(1-methylpiperidin-4-yl)benzamid;
3-{[(3*R*)-4-Benzyl-1,3-dimethyl-2-oxo-1,2,3,4-tetrahydropyrido[2,3-b]pyrazin-6-yl]amino}-*N*-[2-(4-methylpiperazin-1-yl)ethyl]benzamid;
(3*R*)-4-Benzyl-1,3-dimethyl-6-({3-[(4-methylpiperazin-1-yl)carbonyl]phenyl}amino)-3,4-dihydropyrido[2,3-b]pyrazin-2(1H)-on;
(3*R*)-4-Benzyl-6-({3-[(4-isopropylpiperazin-1-yl)carbonyl]phenyl}amino)-1,3-dimethyl-3,4-dihydropyrido[2,3-b]pyrazin-2(1H)-on;
(3*R*)-4-Isopropyl-1,3-dimethyl-6-({3-[(4-methylpiperazin-1-yl)carbonyl]phenyl}amino)-3,4-dihydropyrido[2,3-b]pyrazin-2(1H)-on;
*tert*-Butyl-4-[(3*R*)-1,3-dimethyl-6-({3-[(4-methylpiperazin-1-yl)carbonyl]phenyl}amino)-2-oxo-2,3-dihydro-pyrido[2,3-b]pyrazin-4(1H)-yl]piperidin-1-carboxylat;
*N*-{*trans*-4-[4-(Cyclopropylmethyl)piperazin-1-yl]cyclohexyl}-3-{[(3*R*)-4-isopropyl-1,3-dimethyl-2-oxo-1,2,3,4-tetrahydropyrido[2,3-b]pyrazin-6-yl]amino} benzamid;
3-{[(3*R*)-1,3-Dimethyl-2-oxo-4-(tetrahydro-2H-pyran-4-yl)-1,2,3,4-tetrahydropyrido[2,3-b]pyrazin-6-yl]amino}-*N*-{4-[(1-methylpiperidin-4-yl)amino]cyclohexyl}benzamid;
5-{[(3*R*)-1,3-Dimethyl-2-oxo-4-(tetrahydro-2H-pyran-4-yl)-1,2,3,4-tetrahydropyrido[2,3-b]pyrazin-6-yl]amino}-2-methoxy-*N*-(1-methylpiperidin-4-yl)benzamid;
*N*-{*trans*-4-[4-(Cyclopropylmethyl)piperazin-1-yl]cyclohexyl}-5-{[(3*R*)-1,3-dimethyl-2-oxo-4-(tetrahydio-2H-pyran-4-yl)-1,2,3,4-tetrahydropyrido[2,3-b]pyrazin-6-yl]amino}-2-methoxybenzamid;
(3*R*)-6-({4-Methoxy-3-[(4-methylpiperazin-1-yl)carbonyl]phenyl}amino)-1,3-dimethyl-4-(tetrahydro-2H-pyran-4-yl)-3,4-dihydropyrido[2,3-b]pyrazin-2(1H)-on;
3-{[(3*R*)-4-Cyclohexyl-1,3-dimethyl-2-oxo-1,2,3,4-tetrahydro-pyrido[2,3-b]pyrazin-6-yl]amino}-*N*-[2-(4-methylpiperazin-1-yl)ethyl]benzamid;
(3*R*)-4-Cyclohexyl-6-({3-[(4-isopropylpiperazin-1-yl)carbonyl]phenyl}amino)-1,3-dimethyl-3,4-dihydropyrido[2,3-b]pyrazin-2(1H)-on;
3-{[(3*R*)-4-Cyclohexyl-1,3-dimethyl-2-oxo-1,2,3,4-tetrahydro-pyrido[2,3-b]pyrazin-6-yl]amino}-*N*-(4-hydroxycyclohexyl)benzamid;
*N*-{*trans*-4-[4-(Cyclopropylmethyl)piperazin-1-yl]cyclohexyl}-3-{[(3*R*)-1,3-dimethyl-2-oxo-4-(tetrahydro-2H-pyran-4-yl)-1,2,3,4-tetrahydropyrido[2,3-b]pyrazin-6-yl]amino}-4-methoxybenzamid;
(3*R*)-6-[(3-{[4-(Cyclopropylmethyl) piperazin-1-yl]carbonyl}phenyl) amino]-4-isopropyl-1,3-dimethyl-3,4-dihydropyrido[2,3-b]pyrazin-2(1H)-on;
*N*-[4-(4,4-Difluorpiperidin-1-yl)cyclohexyl]-5-{[(3*R*)-1,3-dimethyl-2-oxo-4-(tetrahydro-2H-pyran-4-yl)-1,2,3,4-tetrahydro-pyrido[2,3-b]pyrazin-6-yl]amino}-2-methoxy-benzamid;
*N*-[*cis*-4-(4-Cyclopropylpiperazin-1-yl)cyclohexyl]-3-{[(3*R*)-1,3-dimethyl-2-oxo-4-(tetrahydro-2H-pyran-4-yl)-1,2,3,4-tetrahydro-pyrido[2,3-b]pyrazin-6-yl]amino}-4-methoxy-benzamid;
*N*-{*trans*-4-[4-(Cyclopropylmethyl)piperazin-1-yl]cyclohexyl}-3-{[(3*R*)-1,3-dimethyl-2-oxo-4-(tetrahydro-2H-pyran-4-yl)-1,2,3,4-tetrahydropyrido[2,3-b]pyrazin-6-yl]amino}-2-methoxybenzamid;
(3*R*)-6-({2-Methoxy-3-[(4-methylpiperazin-1-yl)carbonyl]phenyl}amino)-1,3-dimethyl-4-(tetrahydro-2H-pyran-4-yl)-3,4-dihydropyrido[2,3-b]pyrazin-2(1H)-on;
*N*-{*trans*-4-[4-(Cyclopropylmethyl)piperazin-1-yl]cyclohexyl}-3-{[(3*R*)-4-(4-fluorphenyl)-1,3-dimethyl-2-oxo-1,2,3,4-tetrahydropyrido [2,3-b]pyrazin-6-yl]amino}benzamid;
3-{[(3*R*)-4-Isopropyl-1,3-dimethyl-2-oxo-1,2,3,4-tetrahydropyrido[2,3-b]pyrazin-6-yl]amino}-*N*-(1-methylpiperidin-4-yl)benzamid;
3-{[(3*R*)-4-Cyclohexyl-1,3-dimethyl-2-oxo-1,2,3,4-tetrahydropyrido[2,3-b]pyrazin-6-yl]amino}-*N*-(1-methylpiperidin-4-yl)benzamid;
3-{[(3*R*)-4-(4,4-Dimethylcyclohexyl)-1,3-dimethyl-2-oxo-1,2,3,4-tetrahydropyrido[2,3-b]pyrazin-6-yl]amino}-*N*-(1-methylpiperidin-4-yl)benzamid;
*N*-[2-(Dimethylamino)ethyl]-3-{[(3R)-4-isopropyl-1,3-dimethyl-2-oxo-1,2,3,4-tetrahydropyrido[2,3-b]pyrazin-6-yl]amino}benzamid;
3-{[4-(2-Methoxyethyl)-1,3-dimethyl-2-oxo-1,2,3,4-tetrahydropyrido [2,3-b]pyrazin-6-yl]amino}-*N*-(1-methylpiperidm-4-yl)benzamid;
3-{[(3*R*)-4-Cyclohexyl-1,3-dimethyl-2-oxo-1,2,3,4-tetrahydropyrido[2,3-b]pyrazin-6-yl]amino}-*N*-{*trans*-4-[4-(cyclopropylmethyl)piperazin-1-yl]cyclohexyl}benzamid;
*tert*-Butyl-4-[(3*R*)-1,3-dimethyl-6-({3-[(1-methylpiperidin-4-yl)carbamoyl]phenyl}amino)-2-oxo-2,3-dihydropyrido[2,3-b]pyrazin-4(1H)-yl]piperidin-1-carboxylat;
*N*-{*trans*-4-[4-(Cyclopropylmethyl)piperazin-1-yl]cyclohexyl}-3-{[(3*R*)-4-(2-methoxyethyl)-1,3-dimethyl-2-oxo-1,2,3,4-tetrahydropyrido[2,3-b]pyrazin-6-yl]amino}benzamid;
*tert*-Butyl-4-[(3*R*)-6-{[3-({*trans*-4-[4-(cyclopropyl-methyl)piperazin-1-yl]cyclohexyl}carbamoyl)phenyl]amino}-1,3-dimethyl-2-oxo-2,3-dihydropyrido[2,3-b]pyrazin-4(1H)-yl]piperidin-1-carboxylat;
3-{[(3*R*)-1,3-Dimethyl-2-oxo-4-(tetrahydro-2H-pyran-4-yl)-1,2,3,4-tetrahydropyrido[2,3-b]pyrazin-6-yl]amino}-2-methoxy-*N*-(1-methyl-piperidin-4-yl)benzamid;
3-[(1,3-Dimethyl-2-oxo-4-phenyl-1,2,3,4-tetrahydropyrido[2,3-b]pyrazin-6-yl)amino]-*N-*(1-methylpiperidin-4-yl)benzamid;
*tert*-Butyl-4-[(2*R*)-7-{[3-(dimethylcarbamoyl)phenyl]amino}-2,4-dimethyl-3-oxo-3,4-dihydrochinoxalin-1(2H)-yl]piperidin-1-carboxylat;
(3*R*)-1,3-Dimethyl-6-({3-[(4-methylpiperazin-1-yl)carbonyl]phenyl}amino)-4-(tetrahydro-2H-pyran-4-yl)-3,4-dihydrochinoxalin-2(1H)-on;
3-{[(3*R*)-4-Benzyl-1,3-dimethyl-2-oxo-1,2,3,4-tetrahydrochinoxalin-6-yl]amino}-*N,N-*dimethylbenzamid;
*N*-{*trans*-4-[4-(Cyclopropylmethyl)piperazin-1-yl]cyclohexyl}-3-{[(3*R*)-1,3-dimethyl-2-oxo-4-(tetrahydro-2H-pyran-4-yl)-1,2,3,4-tetrahydrochinoxalin-6-yl]amino}benzamid;
3-{[(3*R*)-1,3-Dimethyl-2-oxo-4-(tetrahydro-2H-pyran-4-yl)-1,2,3,4-tetrahydrochinoxalin-6-yl]amino}-*N*-(1-methylpiperidin-4-yl)benzamid;
3-[(4-Benzyl-1,3-dimethyl-2-oxo-1,2,3,4-tetrahydrochinoxalin-6-yl)(methyl)amino]-*N,N-*dimethylbenzamid;
(3*R*)-4-(2-Methoxyethyl)-1,3-dimethyl-6-{[3-(morphohn-4-ylsulfonyl)phenyl]amino}-3,4-dihydropyrido[2,3-b]pyrazin-2(1H)-on;
(3*S*)-4-(2-Methoxyethyl)-1,3-dimethyl-6-{[3-(morpholin-4-ylsulfonyl)phenyl]amino}-3,4-dihydropyrido[2,3-b]pyrazin-2(1H)-on;
(3*R*)-4-(2-Methoxyethyl)-1,3-dimethyl-6-({3-[(4-methylpiperazin-1-yl)sulfonyl]phenyl}amino)-3,4-dihydropyrido[2,3-b]pyrazin-2(1H)-on;
(3*R*)-4-(4-Fluorphenyl)-1,3-dimethyl-6-{[3-(morpholin-4-ylsulfonyl)phenyl]amino}-3,4-dihydropyrido[2,3-b]pyrazin-2(1H)-on;
3-{[(3*R*)-4-(4-Fluorphenyl)-1,3-dimethyl-2-oxo-1,2,3,4-tetrahydropyrido[2,3-b]pyrazin-6-yl]amino}-*N*-(1-methylpiperidin-4-yl)benzamid;
3-{[(3*S*)-4-(4-Fluorphenyl)-1,3-dimethyl-2-oxo-1,2,3,4-tetrahydropyrido[2,3-b]pyrazin-6-yl]amino}-*N*-1-methylpiperidin-4-yl)benzamid;
(3*R*)-4-(2-Methoxyethyl)-1,3-dimethyl-6-({3-[(4-methylpiperazin-1-yl)carbonyl]-phenyl}amino)-3,4-dihydropyrido[2,3-b]pyrazin-2(1H)-on;
(3*S*)-4-(2-Methoxyethyl)-1,3-dimethyl-6-({3-[(4-methylpiperazin-1-yl)carbonyl]-phenyl}amino)-3,4-dihydropyrido[2,3-b]pyrazin-2(1H)-on;
3-{[(3*R*)-4-(2-Methoxy-ethyl)-1,3-dimethyl-2-oxo-1,2,3,4-tetrahydropyrido [2,3-b]pyrazin-6-yl] amino}-*N*-(1-methyl piperidin-4-yl)benzamid;
3-{[(3*S*)-4-(2-Methoxy-ethyl)-1,3-dimethyl-2-oxo-1,2,3,4-tetrahydropyrido [2,3-b]pyrazin-6-yl]amino}-*N*-(1-methyl piperidin-4-yl)benzamid;
3-{[(3*R*)-1,3-Dimethyl-2-oxo-4-phenyl-1,2,3,4-tetrahydropyrido[2,3-b]pyrazin-6-yl]amino}-*N*-(1-methylpiperidin-4-yl)benzamid;
(3*R*)-6-{[3-(2-Azaspiro[3.3]hept-2-ylsulfonyl)phenyl]amino}-1,3-dimethyl-4-(piperidin-4-yl)-3,4-dihydropyrido[2,3-b]pyrazin-2(1H)-on;
3-{[(3*R*)-1,3-Dimethyl-2-oxo-4-(piperidin-4-yl)-1,2,3,4-tetrahydropyrido [2,3-b]pyrazin-6-yl]amino} -*N,N*-dimethyl benzolsulfonamid;
3-{[(3*R*)-1,3-Dimethyl-2-oxo-4-(piperidin-4-yl)-1,2,3,4-tetrahydropyrido [2,3-b]pyrazin-6-yl]amino}-*N*-(1-methyl piperidin-4-yl)benzol sulfonamid;
(3*R*)-1,3-Dimethyl-6-({3-[(4-methylpiperazin-1-yl)sulfonyl]phenyl}amino)-4-(piperidin-4-yl)-3,4-dihydropyrido [2,3-b]pyrazin-2(1H)-on;
(3*R*)-1,3-Dimethyl-4-(piperidin-4-yl)-6-[(3-{[4-(2,2,2-trifluorethyl)piperazin-1-yl]sulfonyl}phenyl)amino]-3,4-dihydropyrido[2,3-b]pyrazin-2(1H)-on;
*N*-{*trans*-4-[4-(Cyclopropylmethyl)piperazin-1-yl]cyclohexyl}-3-{[(3*R*)-1,3-dimethyl-2-oxo-4-(piperidin-4-yl)-1,2,3,4-tetra-hydropyrido[2,3-b]pyrazin-6-yl]amino} benzolsulfonamid;
*N*-[2-(Dimethylamino)ethyl]-3-{[(3*R*)-1,3-dimethyl-2-oxo-4-(piperidin-4-yl)-1,2,3,4-tetrahydropyrido[2,3-b]pyrazin-6-yl]amino} benzolsulfonamid;
3-{[(3*R*)-1,3-Dimethyl-2-oxo-4-(piperidin-4-yl)-1,2,3,4-tetrahydro-chinoxalin-6-yl]amino}-*N,N*-dimethylbenzol-sulfonamid;
(3*R*)-1,3-Dimethyl-6-({3-[(4-methylpiperazin-1-yl)carbonyl]phenyl}amino)-4-(piperidin-4-yl)-3,4-dihydropyrido[2,3-b]pyrazin-2(1H)-on;
*N-*{*trans*-4-[4-(Cyclopropylmethyl)piperazin-1-yl]cyclohexyl}-3-{[(3*R*)-1,3-dimethyl-2-oxo-4-(piperidin-4-yl)-1,2,3,4-tetrahydro-pyrido[2,3-b]pyrazin-6-yl]amino}benzamid;
3-{[(3*R*)-1,3-Dimethyl-2-oxo-4-(piperidin-4-yl)-1,2,3,4-tetrahydro-pyrido[2,3-b]pyrazin-6-yl]amino}-*N*-(1-methylpiperidin-4-yl)benzamid;
3-{[(3*R*)-1,3-Dimethyl-2-oxo-4-(piperidin-4-yl)-1,2,3,4-tetrahydro-chinoxalin-6-yl]amino}-*N,N*-dimethylbenzamid;
(3*R*)-1,3-Dimethyl-6-{[3-(5-methyl-1,3,4-oxadiazol-2-yl)phenyl]amino}-4-(tetrahydro-2H-pyran-4-yl)-3,4-dihydropyrido[2,3-b]pyrazin-2(1H)-on;
4-(4-Fluorphenyl)-1,3-dimethyl-6-{[3-(morpholin-4-ylsulfonyl)phenyl]amino}-3,4-dihydropyrido[2,3-b]pyrazin-2(1H)-on;
3-{[4-(4-Fluorphenyl)-1,3-dimethyl-2-oxo-1,2,3,4-tetrahydropyrido[2,3-b]pyrazin-6-yl]amino}-*N*-(1-methylpiperidin-4-yl)benzamid
und
4-(2-Methoxyethyl)-1,3-dimethyl-6-({3-[(4-methylpiperazin-1-yl)carbonyl]phenyl}amino)-3,4-dihydropyrido[2,3-b]pyrazin-2(1H)-on.

9. Verbindungen gemäß den Ansprüchen 1 bis 8 zur Verwendung als Arzneimittel.

10. Verbindungen gemäß den Ansprüchen 1 bis 8, zur Verwendung in der Prophylaxe und/oder Therapie von Tumorerkrankungen.

11. Verwendung der Verbindungen gemäß den Ansprüchen 1 bis 8, zur Herstellung eines Arzneimittels.

12. Verwendung der Verbindungen gemäß den Ansprüchen 1 bis 8, zur Herstellung eines Arzneimittels zur Prophylaxe und/oder Therapie von Tumorerkrankungen.

13. Verbindungen gemäß den Ansprüchen 1 bis 8, zur Verwendung in der Prophylaxe und/oder Therapie von hyperproliferativen Erkrankungen.

14. Verbindungen gemäß den Ansprüchen 1 bis 8 zur Verwendung in der Prophylaxe und/ oder Therapie von viralen Infektionen, neurodegenerativen Erkrankungen, inflammatorischen Erkrankungen, atherosklerotischen Erkrankungen und in der männlichen Fertilitätskontrolle.

15. Verwendung der Verbindungen gemäß den Ansprüchen 1 bis 8 zur Herstellung eines Arzneimittels zur Prophylaxe und/ oder Therapie von viralen Infektionen, neurodegenerativen Erkrankungen, inflammatorischen Erkrankungen, atherosklerotischen Erkrankungen und in der männlichen Fertilitätskontrolle.

16. Verbindungen gemäß den Ansprüchen 1 bis 8 in Kombination mit ein oder mehreren weiteren pharmakologisch wirksamen Substanzen.

17. Verbindungen gemäß den Ansprüchen 1 bis 8 in Kombination mit ein oder mehreren weiteren pharmakologisch wirksamen Substanzen, zur Verwendung in der Prophylaxe und/oder Therapie von hyperproliferativen Erkrankungen.

18. Verbindungen gemäß den Ansprüchen 1 bis 8 in Kombination mit ein oder mehreren weiteren pharmakologisch wirksamen Substanzen, zur Verwendung in der Prophylaxe und/oder Therapie von Tumorerkrankungen.

19. Verbindungen gemäß den Ansprüchen 1 bis 8 in Kombination mit ein oder mehreren weiteren pharmakologisch wirksamen Substanzen zur Verwendung in der Prophylaxe und/oder Therapie von viralen Infektionen, neurodegenerativen Erkrankungen, inflammatorischen Erkrankungen, atherosklerotischen Erkrankungen und in der männlichen Fertilitätskontrolle.

20. Verbindungen der allgemeinen Formeln (IX) und (XVI), 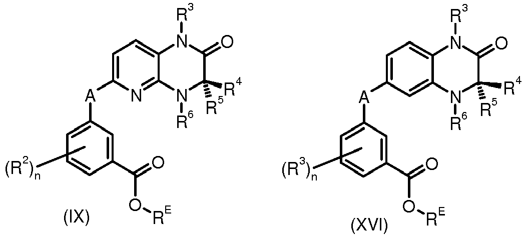 in denen A, R², R³, R⁴, R⁵, R⁶ und n die in der allgemeinen Formel (I) gemäß Anspruch 1 angegebenen Bedeutungen haben und R^{E} für C₁-C₆-Alkyl steht.

21. Verbindungen der allgemeinen Formeln (IX) und (XVI), gemäß Anspruch 20, in denen R^{E} für Methyl oder Ethyl steht.

22. Verbindungen der allgemeinen Formeln (IX) und (XVI), gemäß den Ansprüchen 20 und 21:
3-{[(3*R*)-4-Cyclopentyl-1,3-dimethyl-2-oxo-1,2,3,4-tetrahydropyrido[2,3-*b*]pyrazin-6-yl]-amino}benzoesäuremethylester;
3-{[(3*R*)-1,3-Dimethyl-2-oxo-4-(tetrahydro-2*H*-pyran-4-yl)-1,2,3,4-tetrahydropyrido[2,3-*b*]pyrazin-6-yl]amino}benzoesäuremethylester;
3-{[(3*R*)-1,3-Dimethyl-2-oxo-4-(tetrahydro-2*H*-pyran-4-yl)-1,2,3,4-tetrahydropyrido[2,3-*b*]pyrazin-6-yl]amino}-4-methoxybenzoesäuremethylester;
3-{[4-(2,6-Difluorbenzyl)-1,3-dimethyl-2-oxo-1,2,3,4-tetrahydrochinoxalin-6-yl]amino}-benzoesäureethylester;
3-{[(3*R*)-4-Cyclohexyl-1,3-dimethyl-2-oxo-1,2,3,4-tetrahydropyrido[2,3-b]pyrazin-6-yl]amino}benzoesäuremethylester;
3-{[(3*R*)-1,3-Dimethyl-2-oxo-4-(propan-2-yl)-1,2,3,4-tetrahydropyrido[2,3-b]pyrazin-6-yl]amino}benzoesäuremethylester;
3-{[(3*R*)-4-Benzyl-1,3-dimethyl-2-oxo-1,2,3,4-tetrahydropyrido[2,3-b]pyrazin-6-yl]amino}benzoesäuremethylester;
3-{[4-(2-Methoxyethyl)-1,3-dimethyl-2-oxo-1,2,3,4-tetrahydropyrido[2,3-b]pyrazin-6-yl]amino}benzoesäuremethylester;
*tert*-Butyl 4-[(3*R*)-6-{[3-(ethoxycarbonyl)phenyl]amino}-1,3-dimethyl-2-oxo-2,3-dihydropyrido[2,3-b]pyrazin-4(1H)-yl]piperidin-1-carboxylat;
3-{[4-(4-Fluorphenyl)-1,3-dimethyl-2-oxo-1,2,3,4-tetrahydropyrido[2,3-b]pyrazin-6-yl]amino}benzoesäuremethylester;
5-{[(3*R*)-1,3-Dimethyl-2-oxo-4-(tetrahydro-2H-pyran-4-yl)-1,2,3,4-tetrahydropyrido[2,3-b]pyrazin-6-yl]amino}-2-methoxybenzoesäuremethylester;
3-{[(3*R*)-1,3-Dimethyl-2-oxo-4-(tetrahydro-2H-pyran-4-yl)-1,2,3,4-tetrahydropyrido[2,3-b]pyrazin-6-yl]amino}-2-methoxybenzoesäuremethylester
und
3-{[(3*R*)-1,3-Dimethyl-2-oxo-4-(tetrahydro-2H-pyran-4-yl)-1,2,3,4-tetrahydrochinoxalin-6-yl]amino}benzoesäuremethylester.

23. Verbindungen der allgemeinen Formeln (X) und (XVII) 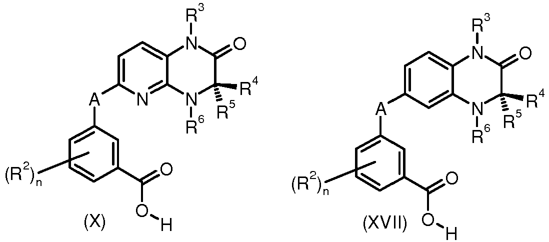 in denen A, R², R³, R⁴, R⁵, R⁶ und n die in der allgemeinen Formel (I) gemäß Anspruch 1 angegebenen Bedeutungen haben.

24. Verbindungen der allgemeinen Formeln (X) und (XVII), gemäß Anspruch 23:
3-{[(3*R*)-4-Cyclopentyl-1,3-dimethyl-2-oxo-1,2,3,4-tetrahydropyrido[2,3-*b*]pyrazin-6-yl]-amino}benzoesäure;
3-{[(3*R*)-1,3-Dimethyl-2-oxo-4-(tetrahydro-2*H*-pyran-4-yl)-1,2,3,4-tetrahydropyrido[2,3-*b*]pyrazin-6-yl]amino}benzoesäure;
3-{[(3*R*)-1,3-Dimethyl-2-oxo-4-(tetrahydro-2*H*-pyran-4-yl)-1,2,3,4-tetrahydropyrido[2,3-*b*]pyrazin-6-yl]amino}-4-methoxybenzoesäure;
3-{[4-(2,6-Difluorbenzyl)-1,3-dimethyl-2-oxo-1,2,3,4-tetrahydrochinoxalin-6-yl]amino}-benzoesäure;
3-{[(3*R*)-4-Cyclohexyl-1,3-dimethyl-2-oxo-1,2,3,4-tetrahydropyrido[2,3-b]pyrazin-6-yl]amino}benzoesäure;
3-{[(3*R*)-1,3-Dimethyl-2-oxo-4-(propan-2-yl)-1,2,3,4-tetrahydropyrido[2,3-b]pyrazin-6-yl]amino}benzoesäure;
3-{[(3*R*)-4-Benzyl-1,3-dimethyl-2-oxo-1,2,3,4-tetrahydropyrido[2,3-b]pyrazin-6-yl]amino}benzoesäure ;
3-{[4-(2-Methoxyethyl)-1,3-dimethyl-2-oxo-1,2,3,4-tetrahydropyrido[2,3-b]pyrazin-6-yl]amino}benzoesäure;
3-({(3*R*)-4-[1-(*tert*-Butoxycarbonyl)piperidin-4-yl]-1,3-dimethyl-2-oxo-1,2,3,4-tetrahydropyrido[2,3-b]pyrazin-6-yl}amino)benzoesäure;
3-{[4-(4-Fluorphenyl)-1,3-dimethyl-2-oxo-1,2,3,4-tetrahydropyrido[2,3-b]pyrazin-6-yl]amino}benzoesäure;
5-{[(3*R*)-1,3-Dimethyl-2-oxo-4-(tetrahydro-2H-pyran-4-yl)-1,2,3,4-tetrahydropyrido[2,3-b]pyrazin-6-yl]amino} -2-methoxybenzoesäure;
3-{[(3*R*)-1,3-Dimethyl-2-oxo-4-(tetrahydro-2H-pyran-4-yl)-1,2,3,4-tetrahydropyrido[2,3-b]pyrazin-6-yl]amino}-2-methoxybenzoesäure
und
3-{[(3*R*)-1,3-Dimethyl-2-oxo-4-(tetrahydro-2H-pyran-4-yl)-1,2,3,4-tetrahydrochinoxalin-6-yl]amino}benzoesäure.

25. Verwendung der Verbindungen gemäß den Ansprüchen 20 bis 24 zur Herstellung der erfindungsgemäßen Verbindungen der allgemeinen Formel (I) gemäß Anspruch 1.

## Claims

1. Compounds of the general formula (I) 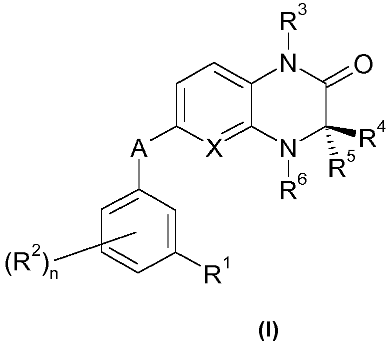 in which
A is -NH-, -N(C₁-C₃-alkyl)- or -O-,
X is -N- or -CH-,
n is 0, 1 or 2,
R¹ is a -C(=O)NR⁷R⁸ or -S(=O)₂NR⁷R⁸ group,
or
is 5-membered monocyclic heteroarylwhich may optionally be mono-, di- or trisubstituted identically or differently by halogen, cyano, C₁-C₄-alkyl-, C₂-C₄-alkenyl-, C₂-C₄-alkynyl-, halo-C₁-C₄-alkyl-, C₁-C₄-alkoxy-, halo-C₁-C₄-alkoxy-, C₁-C₄-alkylthio-, halo-C₁-C₄-alkylthio-,
-NR⁹R¹⁰, -C(=O)OR¹¹, -C(=O)N⁹R¹⁰, -C(=O)R¹¹, -S(=O)₂R¹¹, -S(=O)₂NR⁹R¹⁰,
R² is hydrogen, halogen, cyano, C₁-C₃-alkyl-, C₂-C₄-alkenyl-, C₂-C₄-alkynyl-, halo-C₁-C₄-alkyl-, C₁-C₄-alkoxy-, halo-C₁-C₄-alkoxy-, C₁-C₄-alkylthio- or halo-C₁-C₄-alkylthio-, and, if n is 2, R² may be the same or different, or
R¹ and R² together are a *-S(=O)₂-NR⁸-CH₂-**, *-S(=O)₂-NR⁸-CH₂-CH₂-**, *-C (=O)-NR⁸-CH₂-** or *-C(=O)-NR⁸-CH₂-CH₂-** group in which "*" denotes the attachment point of R¹ to the phenyl ring in formula (I), and in which "**" denotes a carbon atom of this phenyl ring adjacent to this attachment point,
R³ is methyl- or ethyl-,
R⁴ is hydrogen or C₁-C₃-alkyl-,
R⁵ is hydrogen or C₁-C₃-alkyl-,
or
R⁴ and R⁵ together with the carbon atom to which they are bonded are C₃-C₆-cycloalkylene,
R⁶ is C₁-C₆-alkyl- which may optionally be monosubstituted by C₁-C₃-alkoxy-, phenyl-, C₃-C₈-cycloalkyl-, or 4- to 8-membered heterocycloalkyl-,
in which phenyl- may itself optionally be mono-, di- or trisubstituted identically or differently by: halogen, cyano, C₁-C₄-alkyl-, C₂-C₄-alkenyl-, C₂-C₄-alkynyl-, C₁-C₄-alkoxy-, halo-C₁-C₄-alkyl-, halo-C₁-C₄-alkoxy-, and
in which C₃-C₈-cycloalkyl- and 4- to 8-membered heterocycloalkyl- may themselves optionally be mono- or disubstituted identically or differently by C₁-C₃-alkyl-,
or
is C₃-C₈-cycloalkyl- or 4- to 8-membered heterocycloalkyl-, which may optionally be mono- or disubstituted identically or differently by C₁-C₃-alkyl- or C₁-C₄-alkoxycarbonyl-,
or
is phenyl which may optionally be mono-or disubstituted identically or differently by halogen, C₁-C₃-alkyl- or 4-8-membered heterocycloalkyl-, in which the 4-8-membered heterocycloalkyl- may itself optionally be mono- or disubstituted identically or differently by C₁-C₃-alkyl or C₁-C₄-alkoxycarbonyl-,
R⁷ is hydrogen,
or
is C₁-C₆-alkyl- which may optionally be mono-, di- or trisubstituted identically or differently by: hydroxyl, oxo, fluorine, cyano, C₁-C₄-alkoxy-, halo-C₁-C₄-alkoxy-, -NR⁹R¹⁰, C₃-C₈-cycloalkyl-, C₄-C₈-cycloalkenyl-, 4- to 8-membered heterocycloalkyl-, 4- bis 8-membered heterocycloalkenyl-, C₅-C₁₁-spirocycloalkyl-, C₅-C₁₁-heterospirocycloalkyl-, bridged C₆-C₁₂-cycloalkyl-, bridged C₆-C₁₂-heterocycloalkyl-, C₆-C₁₂-bicycloalkyl-, C₆-C₁₂-heterobicycloalkyl-, phenyl-, 5-to 6-membered heteroaryl-,
in which C₃-C₈-cycloalkyl-, C₄-C₈-cycloalkenyl-, 4- to 8-membered heterocycloalkyl-, 4- to 8-membered heterocycloalkenyl-, C₅-C₁₁-spirocycloalkyl-, C₅-C₁₁-heterospirocycloalkyl-, bridged C₆-C₁₂-cycloalkyl-, bridged C₆-C₁₂-heterocycloalkyl-, C₆-C₁₂-bicycloalkyl-, C₆-C₁₂-heterobicycloalkyl- may itself optionally be mono- or disubstituted identically or differently by: hydroxyl, fluorine, oxo, cyano, C₁-C₃-alkyl-, fluoro-C₁-C₃-alkyl-, C₃-C₆-cycloalkyl-, cyclopropylmethyl-, C₁-C₃-alkylcarbonyl-, C₁-C₄-alkoxycarbonyl- or -NR⁹R¹⁰, and in which phenyl and 5- to 6-membered heteroaryl may optionally be mono- or disubstituted identically or differently by: halogen, cyano, trifluoromethyl-, C₁-C₃-alkyl-, C₁-C₃-alkoxy-,
or
is C₃-C₆-alkenyl- or C₃-C₆-alkynyl-,
or
is C₃-C₈-cycloalkyl-, C₄-C₈-cycloalkenyl-, C₅-C₁₁-spirocycloalkyl-, bridged C₆-C₁₂-cycloalkyl- or C₆-C₁₂-bicycloalkyl-, which may optionally be mono- or disubstituted identically or differently by: hydroxyl, oxo, cyano, fluorine, C₁-C₃-alkyl, C₁-C₃-alkoxy, trifluoromethyl, -NR⁹R¹⁰,
or
is 4- to 8-membered heterocycloalkyl-, 4- to 8-membered heterocycloalkenyl-, C₅-C₁₁-heterospirocycloalkyl-, bridged C₆-C₁₂-heterocycloalkyl- or C₆-C₁₂-heterobicycloalkyl-, which may optionally be mono- or disubstituted identically or differently by: hydroxyl, fluorine, oxo, cyano, C₁-C₃-alkyl-, fluoro-C₁-C₃-alkyl-, C₃-C₆-cycloalkyl-, cyclopropylmethyl-, C₁-C₃-alkylcarbonyl-, C₁-C₄-alkoxycarbonyl- or -NR⁹R¹⁰,
R⁸ is hydrogen or optionally singly or doubly, identically or differently hydroxyl-, oxo- or C₁-C₃-alkoxy-substituted C₁-C₃-alkyl-, or fluoro-C₁-C₃-alkyl, or
R⁷ and R⁸ together with the nitrogen atom to which they are bonded are 4- to 8-membered heterocycloalkyl, 4- to 8-membered heterocycloalkenyl-, C₅-C₁₁-heterospirocycloalkyl-, bridged C₆-C₁₂-heterocycloalkyl- or C₆-C₁₂-heterobicycloalkyl-, which may optionally be mono- or disubstituted identically or differently by: hydroxyl, fluorine, oxo, cyano, C₁-C₃-alkyl-, fluoro-C₁-C₃-alkyl-, C₃-C₆-cycloalkyl-, cyclopropylmethyl-, C₁-C₃-alkylcarbonyl-, C₁-C₄-alkoxycarbonyl- or -NR⁹R¹⁰,
R⁹ and R¹⁰ are each independently hydrogen or optionally singly or doubly, identically or differently hydroxyl-, oxo- or C₁-C₃-alkoxy-substituted C₁-C₃-alkyl, or fluoro-C₁-C₃-alkyl, or 4- to 8-membered heterocycloalkyl,
in which the 4- to 8-membered heterocycloalkyl- may itself optionally be mono- or disubstituted identically or differently by C₁-C₃-alkyl,
or
R⁹ and R¹⁰ together with the nitrogen atom to which they are attached are 4- to 8-membered heterocycloalkyl- which may optionally be mono- or disubstituted identically or differently by: hydroxyl, fluorine, oxo, cyano, C₁-C₃-alkyl-, fluoro-C₁-C₃-alkyl-, C₃-C₆-cycloalkyl-, cyclopropylmethyl-, C₁-C₃-alkylcarbonyl- or C₁-C₄-alkoxycarbonyl-,
R¹¹ is C₁-C₆-alkyl or phenyl-C₁-C₃-alkyl,
and the diastereomers, racemates, polymorphs and physiologically acceptable salts thereof.

2. Compounds of the general formula (I) according to Claim 1, in which
A is -NH- or -N(C₁-C₃-alkyl)-,
X is -N- or -CH-,
n is 0, 1 or 2,
R¹ is a -C(=O)NR⁷R⁸ or -S(=O)₂NR⁷R⁸ group,
or
is oxazolyl-, thiazolyl-, oxadiazolyl- or thiadiazolyl-, which may optionally be mono- or disubstituted identically or differently by halogen, cyano, C₁-C₃-alkyl-, trifluoromethyl-,
C₁-C₃-alkoxy-, trifluoromethoxy- or-NR⁹R¹⁰,
R² is hydrogen, fluorine, chlorine, cyano, methyl-, methoxy-, ethyl- or ethoxy-, and if n is 2, R² may be the same or different,
R¹ and R² together are a *-S(=O)₂-NR⁸-CH₂-** or *-C(=O)-NR⁸-CH₂-** group in which "*" denotes the attachment point of R¹ to the phenyl ring in formula (I), and in which "**" denotes a carbon atom of this phenyl ring adjacent to this attachment point,
R³ is methyl- or ethyl-,
R⁴ is hydrogen, methyl- or ethyl-,
R⁵ is hydrogen, methyl- or ethyl-,
R⁶ is C₂-C₅-alkyl-,
or
is methyl- or ethyl- monosubstituted by C₁-C₃-alkoxy-, phenyl- or 4- to 8-membered heterocycloalkyl-,
in which phenyl- may itself optionally be mono-, di- or trisubstituted identically or differently by: fluorine, chlorine, bromine, cyano, C₁-C₃-alkyl-, C₁-C₃-alkoxy-, and
in which the 4- to 8-membered heterocycloalkyl- may itself optionally be mono- or disubstituted identically or differently by methyl-,
or
is C₃-C₈-cycloalkyl- or 4- to 8-membered heterocycloalkyl-, which may optionally be mono- or disubstituted identically or differently by C₁-C₃-alkyl- or C₁-C₄-alkoxycarbonyl-,
or
is phenyl which may optionally be mono- or disubstituted identically or differently by fluorine, chlorine, methyl- or 6-membered heterocycloalkyl-, in which the 6-membered heterocycloalkyl- may itself optionally be monosubstituted by methyl- or *tert-*butoxycarbonyl-,
R⁷ is hydrogen,
or
is C₁-C₆-alkyl- which may optionally be mono-, di- or trisubstituted identically or differently by: hydroxyl, oxo, fluorine, cyano, C₁-C₃-alkoxy-, fluoro-C₁-C₃-alkoxy-, -NR⁹R¹⁰, 4- to 8-membered heterocycloalkyl-, phenyl-, 5- to 6-membered heteroaryl-,
in which the 4- to 8-membered heterocycloalkyl- may itself optionally be monosubstituted by: hydroxyl, oxo, C₁-C₃-alkyl-, fluoro-C₁-C₃-alkyl-, cyclopropyl-, cyclopropylmethyl-, acetyl- or *tert*-butoxycarbonyl-,
or is C₃-C₆-cycloalkyl- which may optionally be mono- or disubstituted identically or differently by: hydroxyl, oxo, cyano, fluorine, -NR⁹R¹⁰,
or
is 4- to 8-membered heterocycloalkyl-, C₆-C₈-heterospirocycloalkyl-, bridged C₆-C₁₀-heterocycloalkyl- or C₆-C₁₀-heterobicycloalkyl-, which may optionally be mono- or disubstituted identically or differently by: hydroxyl, oxo, C₁-C₃-alkyl-, fluoro-C₁-C₃-alkyl-, cyclopropyl-, cyclopropylmethyl-, acetyl- or *tert*-butoxycarbonyl-,
R⁸ is hydrogen or C₁-C₃-alkyl-,
or
R⁷ and R⁸ together with the nitrogen atom to which they are bonded are 4- to 8-membered heterocycloalkyl-, C₆-C₈-heterospirocycloalkyl-, bridged C₆-C₁₀-heterocycloalkyl- or C₆-C₁₀-heterobicycloalkyl-,
which may optionally be mono- or disubstituted identically or differently by: hydroxyl, fluorine, oxo, C₁-C₃-alkyl-, fluoro-C₁-C₃-alkyl-, cyclopropyl-, cyclopropylmethyl-, acetyl- or *tert-*butoxycarbonyl-,
R⁹ and R¹⁰ are each independently hydrogen or optionally mono-hydroxyl- or -oxo-substituted C₁-C₃-alkyl- or trifluoromethyl-, or 6-membered heterocycloalkyl-,
in which the 6-membered heterocycloalkyl- may itself optionally be mono- or disubstituted identically or differently by C₁-C₃-alkyl,
or
R⁹ and R¹⁰ together with the nitrogen atom to which they are bonded are 4- to 7-membered heterocycloalkyl- which may optionally be mono- or disubstituted identically or differently by: hydroxyl, fluorine, oxo, C₁-C₃-alkyl-, fluoro-C₁-C₃-alkyl-, cyclopropyl-, cyclopropylmethyl-, acetyl- or *tert*-butoxycarbonyl-,
and the diastereomers, racemates, polymorphs and physiologically acceptable salts thereof.

3. Compounds of the general formula (I) according to Claims 1 and 2, in which
A is -NH- or -N(methyl)-,
X is -N- or -CH-,
n is 0 or 1,
R¹ is a -C(=O)NR⁷R⁸ or -S(=O)₂NR⁷R⁸ group,
or
is oxazolyl- or oxadiazolyl- which may optionally be mono- or disubstituted identically or differently by C₁-C₃-alkyl-,
R² is hydrogen, fluorine, chlorine, methyl- or methoxy-,
or
R¹ and R² together are a *-S(=O)₂-NR⁸-CH₂-** group in which "*" denotes the attachment point of R¹ to the phenyl ring in formula (I), and in which "**" denotes a carbon atom of this phenyl ring adjacent to this attachment point,
R³ is methyl-,
R⁴ is methyl- or ethyl-,
R⁵ is hydrogen,
R⁶ is C₃-C₅-alkyl- or 2-methoxyethyl-,
or
is methyl- monosubstituted by phenyl- or 4- to 6-membered heterocycloalkyl-, in which phenyl- may itself optionally be mono- or disubstituted identically or differently by: fluorine, chlorine, cyano, methyl-, methoxy-, and in which the 4- to 6-membered heterocycloalkyl- may itself optionally be monosubstituted by methyl-,
or
is C₃-C₈-cycloalkyl- or is 4- to 6-membered heterocycloalkyl-, which may optionally be mono- or disubstituted identically or differently by C₁-C₃-alkyl- or C₁-C₄-alkoxycarbonyl-,
or
is phenyl which may optionally be mono- or disubstituted identically or differently by fluorine, chlorine, methyl- or *N-tert-*butoxycarbonylpiperazinyl-,
R⁷ is hydrogen,
or
is C₁-C₄-alkyl which may optionally be monosubstituted by -NR⁹R¹⁰ or 4- to 8-membered heterocycloalkyl,
in which the 4- to 8-membered heterocycloalkyl- may itself optionally be monosubstituted by: oxo, C₁-C₃-alkyl-, fluoro-C₁-C₃-alkyl-, cyclopropyl- or cyclopropylmethyl-,
or
is C₃-C₆-cycloalkyl- which may optionally be monosubstituted by hydroxyl, fluorine or -NR⁹R¹⁰,
or
is 4- to 8-membered heterocycloalkyl-which may optionally be mono- or disubstituted identically or differently by: oxo, C₁-C₃-alkyl-, fluoro-C₁-C₃-alkyl-, cyclopropyl- or cyclopropylmethyl-,
R⁸ is hydrogen, methyl- or ethyl-,
or
R⁷ and R⁸ together with the nitrogen atom to which they are bonded are 4- to 6-membered heterocycloalkyl- or C₆-C₈-heterospirocycloalkyl-, which may optionally be mono- or disubstituted identically or differently by: fluorine, oxo, C₁-C₃-alkyl-, fluoro-C₁-C₃-alkyl-, cyclopropyl- or cyclopropylmethyl-,
R⁹ and R¹⁰ are each independently hydrogen or optionally mono-hydroxyl- or -oxo-substituted C₁-C₃-alkyl-, trifluoromethyl-, or is *N*-methylpiperidinyl-, or
R⁹ and R¹⁰ together with the nitrogen atom to which they are bonded are 4- to 7-membered heterocycloalkyl- which may optionally be mono- or disubstituted identically or differently by: fluorine, oxo, C₁-C₃-alkyl-, fluoro-C₁-C₃-alkyl-, cyclopropyl- or cyclopropylmethyl-,
and the diastereomers, racemates, polymorphs and physiologically acceptable salts thereof.

4. Compounds of the general formula (I) according to Claims 1 to 3, in which
A is -NH- or -N(methyl)-,
X is -N-,
n is 0 or 1,
R¹ is a -C(=O)NR⁷R⁸ or -S(=O)₂NR⁷R⁸ group,
or
is oxazolyl- or oxadiazolyl- which may optionally be mono- or disubstituted identically or differently by C₁-C₃-alkyl-,
R² is hydrogen, fluorine, chlorine, methyl- or methoxy-,
or
R¹ and R² together are a *-S(=O)₂-NR⁸-CH₂-** group in which "*" denotes the attachment point of R¹ to the phenyl ring in formula (I), and in which "**" denotes a carbon atom of this phenyl ring adjacent to this attachment point,
R³ is methyl-,
R⁴ is methyl- or ethyl-,
R⁵ is hydrogen,
R⁶ is C₃-C₅-alkyl- or 2-methoxyethyl-,
or
is methyl- monosubstituted by phenyl- or 4- to 6-membered heterocycloalkyl-, in which phenyl- may itself optionally be mono- or disubstituted identically or differently by: fluorine, chlorine, cyano, methyl-, methoxy-, and
in which the 4- to 6-membered heterocycloalkyl- may itself optionally be monosubstituted by methyl-,
or
is C₃-C₈-cycloalkyl- or is 4- to 6-membered heterocycloalkyl-, which may optionally be mono- or disubstituted identically or differently by C₁-C₃-alkyl- or C₁-C₄-alkoxycarbonyl-,
or
is phenyl which may optionally be mono- or disubstituted identically or differently by fluorine, chlorine, methyl- or *N-tert-*butoxycarbonylpiperazinyl-,
R⁷ is hydrogen,
or
is C₁-C₄-alkyl which may optionally be monosubstituted by -NR⁹R¹⁰ or 4- to 8-membered heterocycloalkyl,
in which the 4- to 8-membered heterocycloalkyl- may itself optionally be monosubstituted by: oxo, C₁-C₃-alkyl-, fluoro-C₁-C₃-alkyl-, cyclopropyl- or cyclopropylmethyl-,
or
is C₃-C₆-cycloalkyl- which may optionally be monosubstituted by hydroxyl, fluorine or -NR⁹R¹⁰,
or
is 4- to 8-membered heterocycloalkyl-which may optionally be mono- or disubstituted identically or differently by: oxo, C₁-C₃-alkyl-, fluoro-C₁-C₃-alkyl-, cyclopropyl- or cyclopropylmethyl-,
R⁸ is hydrogen, methyl- or ethyl-,
or
R⁷ and R⁸ together with the nitrogen atom to which they are bonded are 4- to 6-membered heterocycloalkyl- or C₆-C₈-heterospirocycloalkyl-, which may optionally be mono- or disubstituted identically or differently by: fluorine, oxo, C₁-C₃-alkyl-, fluoro-C₁-C₃-alkyl-, cyclopropyl- or cyclopropylmethyl-,
R⁹ and R¹⁰ are each independently hydrogen or optionally mono-hydroxyl- or -oxo-substituted C₁-C₃-alkyl-, trifluoromethyl-, or *N-*methylpiperidinyl-,
or
R⁹ and R¹⁰ together with the nitrogen atom to which they are bonded are 4- to 7-membered heterocycloalkyl- which may optionally be mono- or disubstituted identically or differently by: fluorine, oxo, C₁-C₃-alkyl-, fluoro-C₁-C₃-alkyl-, cyclopropyl- or cyclopropylmethyl-,
and the diastereomers, racemates, polymorphs and physiologically acceptable salts thereof.

5. Compounds of the general formula (I) according to Claims 1 to 3, in which
A is -NH- or -N(methyl)-,
X is -CH-,
n is 0 or 1,
R¹ is a -C(=O)NR⁷R⁸ or -S(=O)₂NR⁷R⁸ group,
or
is oxazolyl- or oxadiazolyl- which may optionally be mono- or disubstituted identically or differently by C₁-C₃-alkyl-,
R² is hydrogen, fluorine, chlorine, methyl- or methoxy-,
or
R¹ and R² together are a *-S(=O)₂-NR⁸-CH₂-** group in which "*" denotes the attachment point of R¹ to the phenyl ring in formula (I), and in which "**" denotes a carbon atom of this phenyl ring adjacent to this attachment point,
R³ is methyl-,
R⁴ is methyl- or ethyl-,
R⁵ is hydrogen,
R⁶ is C₃-C₅-alkyl- or 2-methoxyethyl-,
or
is methyl- monosubstituted by phenyl- or 4- to 6-membered heterocycloalkyl-, in which phenyl- may itself optionally be mono- or disubstituted identically or differently by: fluorine, chlorine, cyano, methyl-, methoxy-, and in which the 4- to 6-membered heterocycloalkyl- may itself optionally be monosubstituted by methyl-,
or
is C₃-C₈-cycloalkyl- or is 4- to 6-membered heterocycloalkyl-, which may optionally be mono- or disubstituted identically or differently by C₁-C₃-alkyl- or C₁-C₄-alkoxycarbonyl-,
or
is phenyl which may optionally be mono- or disubstituted identically or differently by fluorine, chlorine, methyl- or *N-tert-*butoxycarbonylpiperazinyl-,
R⁷ is hydrogen,
or
is C₁-C₄-alkyl which may optionally be monosubstituted by -NR⁹R¹⁰ or 4- to 8-membered heterocycloalkyl, in which the 4- to 8-membered heterocycloalkyl- may itself optionally be monosubstituted by: oxo, C₁-C₃-alkyl-, fluoro-C₁-C₃-alkyl-, cyclopropyl- or cyclopropylmethyl-,
or
is C₃-C₆-cycloalkyl- which may optionally be monosubstituted by hydroxyl, fluorine or -NR⁹R¹⁰,
or
is 4- to 8-membered heterocycloalkyl-which may optionally be mono- or disubstituted identically or differently by: oxo, C₁-C₃-alkyl-, fluoro-C₁-C₃-alkyl-, cyclopropyl- or cyclopropylmethyl-,
R⁸ is hydrogen, methyl- or ethyl-,
or
R⁷ and R⁸ together with the nitrogen atom to which they are bonded are 4- to 6-membered heterocycloalkyl- or C₆-C₈-heterospirocycloalkyl-, which may optionally be mono- or disubstituted identically or differently by: fluorine, oxo, C₁-C₃-alkyl-, fluoro-C₁-C₃-alkyl-, cyclopropyl- or cyclopropylmethyl-,
R⁹ and R¹⁰ are each independently hydrogen or optionally mono-hydroxyl- or -oxo-substituted C₁-C₃-alkyl-, trifluoromethyl-, or *N-*methylpiperidinyl-,
or
R⁹ and R¹⁰ together with the nitrogen atom to which they are bonded are 4- to 7-membered heterocycloalkyl- which may optionally be mono- or disubstituted identically or differently by: fluorine, oxo, C₁-C₃-alkyl-, fluoro-C₁-C₃-alkyl-, cyclopropyl- or cyclopropylmethyl-,
and the diastereomers, racemates, polymorphs and physiologically acceptable salts thereof.

6. Compounds of the general formula (I) according to Claims 1 to 3, in which
A is -NH- or -N(methyl)-,
X is -N- or -CH-,
n is 0 or 1,
R¹ is a -C(=O)NR⁷R⁸ or -S(=O)₂NR⁷R⁸ group, or is oxazolyl- or oxadiazolyl- which may optionally be mono- or disubstituted by methyl-,
R² is hydrogen, methyl- or methoxy-,
or
R¹ and R² together are a *-S(=O)₂-NH-CH₂-** group in which "*" denotes the attachment point of R¹ to the phenyl ring in formula (I), and in which "**" denotes a carbon atom of this phenyl ring adjacent to this attachment point,
R³ is methyl-,
R⁴ is methyl-,
R⁵ is hydrogen,
R⁶ is isopropyl-, isobutyl- or 2-methoxyethyl-,
or
is benzyl wherein the phenyl moiety may optionally be mono- or disubstituted identically or differently by: fluorine, methoxy-,
or
is C₅-C₇-cycloalkyl- which may optionally be mono- or disubstituted by methyl-,
or
is tetrahydrofuranyl-, tetrahydropyranyl- or piperidinyl-, in which piperidinyl- may optionally be monosubstituted by methyl- or *tert-*butoxycarbonyl-,
or
is phenyl which may optionally be monosubstituted by fluorine, methyl- or *N-tert*-butoxycarbonylpiperazinyl-,
R⁷ is hydrogen,
or
is C₁-C₃-alkyl which may optionally be monosubstituted by -NR⁹R¹⁰ or *N-*methylpiperidinyl-,
or
is cyclopropyl-, or is cyclohexyl-, in which cyclohexyl- may optionally be monosubstituted by hydroxyl- or -NR⁹R¹⁰,
or is 4- to 6-membered heterocycloalkyl which may optionally be monosubstituted by methyl-,
R⁸ is hydrogen, methyl- or ethyl-,
or
R⁷ and R⁸ together with the nitrogen atom to which they are bonded are 4- to 6-membered heterocycloalkyl- which may optionally be mono- or disubstituted by fluorine, or which may optionally be monosubstituted by methyl-, isopropyl-, 2,2,2-trifluoroethyl- or cyclopropylmethyl-,
or
are 6-azaspiro[3.3]heptyl- or are 2-oxa-6-azaspiro[3.3]heptyl-,
R⁹ and R¹⁰ are each independently hydrogen, C₁-C₃-alkyl- or *N*-methylpiperidinyl-,
or
R⁹ and R¹⁰ together with the nitrogen atom to which they are bonded are 6-membered heterocycloalkyl- which may optionally be mono- or disubstituted by fluorine, or which may optionally be monosubstituted by methyl-, 2,2,2-trifluoroethyl-, cyclopropyl- or cyclopropylmethyl-,
and the diastereomers, racemates, polymorphs and physiologically acceptable salts thereof.

7. Compounds of the general formula (I) according to Claims 1 to 3 and 6, in which
A is -NH- or -N(methyl)-,
X is -N- or -CH-,
n is 0 or 1,
R¹ is a -C(=O) NR⁷R⁸ or -S(=O)₂NR⁷R⁸ group,
or
is 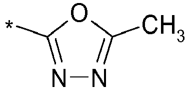 in which "*" denotes the attachment point to the rest of the molecule,
R² is hydrogen, methyl- or methoxy-,
or
R¹ and R² together with the phenyl ring to which they are bonded are 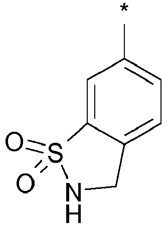 in which "*" denotes the attachment point to the rest of the molecule,
R³ is methyl-,
R⁴ is methyl-,
R⁵ is hydrogen,
R⁶ is isopropyl-, isobutyl-, 2-methoxyethyl-, benzyl-, 4-methoxybenzyl-, 2,6-difluorobenzyl-, cyclopentyl-, cyclohexyl-, cycloheptyl-, tetrahydropyran-4-yl-, phenyl-, 3-methylphenyl- or 4-fluorophenyl-,
or
is 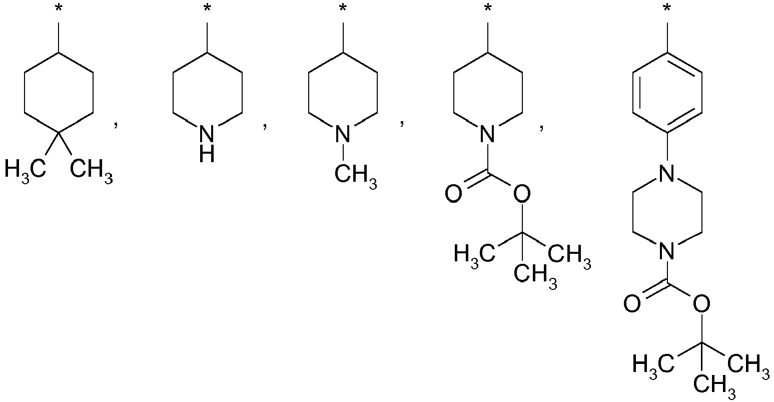 in which "*" denotes the attachment point to the rest of the molecule,
R⁷ is hydrogen, methyl-, ethyl-, isopropyl-or cyclopropyl-,
or
is 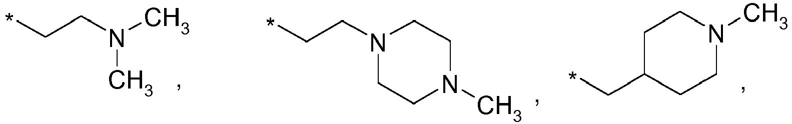 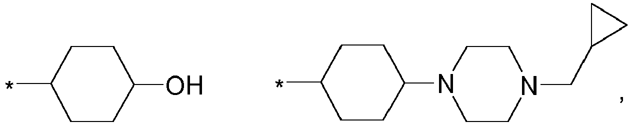 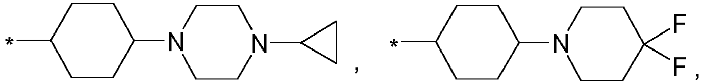 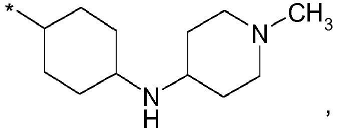 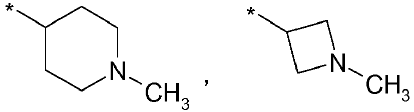 where "*" in each case denotes the attachment point to the rest of the molecule,
R⁸ is hydrogen, methyl- or ethyl-, or
R⁷ and R⁸ together with the nitrogen atom to which they are bonded are 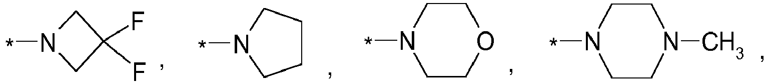 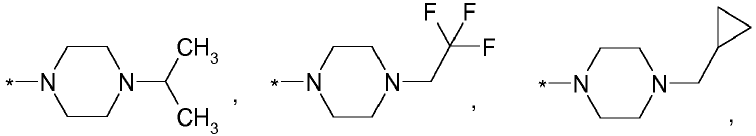 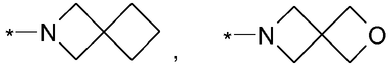 where "*" in each case denotes the attachment point to the rest of the molecule,
and the diastereomers, racemates, polymorphs and physiologically acceptable salts thereof.

8. Compounds of the general formula I according to any of Claims 1 to 7:
(3*R*)-4-cyclopentyl-1,3-dimethyl-6-({3-[(4-methylpiperazin-1-yl)carbonyl]phenyl}amino)-3,4-dihydropyrido[2,3-*b*]pyrazin-2(1*H*)-one;
3-{[(3*R*)-4-cyclopentyl-1,3-dimethyl-2-oxo-1,2,3,4-tetrahydropyrido[2,3-*b*]pyrazin-6-yl]amino}-*N*-(1-methylpiperidin-4-yl)benzamide;
3-{[(3*R*)-4-cyclopentyl-1,3-dimethyl-2-oxo-1,2,3,4-tetrahydropyrido[2,3-*b*]pyrazin-6-yl]amino}-*N*-(1-methylazetidin-3-yl)benzamide;
3-{[(3*R*)-4-cyclopentyl-1,3-dimethyl-2-oxo-1,2,3,4-tetrahydropyrido[2,3-*b*]pyrazin-6-yl]amino}-*N*-[2-(dimethylamino)ethyl]benzamide;
3-{[(3*R*)-4-cyclopentyl-1,3-dimethyl-2-oxo-1,2,3,4-tetrahydropyrido[2,3-*b*]pyrazin-6-yl]amino}-*N*-{ *trans*-4-[4-(cyclopropylmethyl)piperazin-1-yl]cyclohexyl}benzamide;
3-{[(3*R*)-1,3-dimethyl-2-oxo-4-(tetrahydro-2*H-*pyran-4-yl)-1,2,3,4-tetrahydropyrido[2,3-*b*]pyrazin-6-yl]amino}-*N,N-*dimethylbenzenesulphonamide;
3-{[(3*R*)-1,3-dimethyl-2-oxo-4-(tetrahydro-2*H-*pyran-4-yl)-1,2,3,4-tetrahydroquinoxalin-6-yl]amino}-*N,N*-dimethylbenzenesulphonamide;
(3*R*)-1,3-dimethyl-6-{[3-(morpholin-4-ylsulphonyl)phenyl]amino}-4-(tetrahydro-2*H*-pyran-4-yl)-3,4-dihydroquinoxalin-2(1*H*)-one;
(3*R*)-1,3-dimethyl-6-{[3-(morpholin-4-ylsulphonyl)phenyl]amino}-4-(tetrahydro-2*H*-pyran-4-yl)-3,4-dihydropyrido[2,3-*b*]pyrazin-2(1*H*)-one;
3-{[(3*R*)-4-(4-methoxybenzyl)-1,3-dimethyl-2-oxo-1,2,3,4-tetrahydroquinoxalin-6-yl]amino}-*N,N-*dimethylbenzenesulphonamide;
(3*R*)-4-(4-methoxybenzyl)-1,3-dimethyl-6-{[3-(morpholin-4-ylsulphonyl)phenyl]amino}-3,4-dihydroquinoxalin-2(1*H*)-one;
(3*R*)-1,3-dimethyl-6-({3-[(4-methylpiperazin-1-yl)sulphonyl]phenyl}amino)-4-(tetrahydro-2*H*-pyran-4-yl)-3,4-dihydropyrido[2,3-*b*]pyrazin-2(1*H*)-one;
3-{[(3*R*)-1,3-dimethyl-2-oxo-4-(tetrahydro-2*H-*pyran-4-yl)-1,2,3,4-tetrahydropyrido[2,3-*b*]pyrazin-6-yl]amino}-*N*-(1-methylpiperidin-4-yl)benzamide;
*N*-{*trans*-4-[4-(cyclopropylmethyl)piperazin-1-yl]cyclohexyl}-3-{[(3*R*)-1,3-dimethyl-2-oxo-4-(tetrahydro-2H-pyran-4-yl)-1,2,3,4-tetrahydropyrido[2,3-*b*]pyrazin-6-yl]amino}benzamide;
(3*R*)-1,3-dimethyl-6-({3-[(4-methylpiperazin-1-yl)carbonyl]phenyl}amino)-4-(tetrahydro-2*H*-pyran-4-yl)-3,4-dihydropyrido[2,3-*b*]pyrazin-2(1*H*)-one;
3-{[(3*R*)-1,3-dimethyl-2-oxo-4-(tetrahydro-2*H-*pyran-4-yl)-1,2,3,4-tetrahydropyrido[2,3-*b*]pyrazin-6-yl]amino}-4-methoxy-*N*-(1-methylpiperidin-4-yl)benzamide;
(3*R*)-6-({2-methoxy-5-[(4-methylpiperazin-1-yl)carbonyl]phenyl}amino)-1,3-dimethyl-4-(tetrahydro-2*H*-pyran-4-yl)-3,4-dihydropyrido[2,3-*b*]pyrazin-2(1*H*)-one;
*N*-{*trans*-4-[4-(cyclopropylmethyl)piperazin-1-yl]cyclohexyl}-3-{[(3*R*)-1,3-dimethyl-2-oxo-4-(tetrahydro-2*H*-pyran-4-yl)-1,2,3,4-tetrahydropyrido[2,3-*b*]pyrazin-6-yl]amino}-4-methoxybenzamide;
*N*-[2-(dimethylamino)ethyl]-3-{[(3*R*)-1,3-dimethyl-2-oxo-4-(tetrahydro-2*H*-pyran-4-yl)-1,2,3,4-tetrahydropyrido[2,3-*b*]pyrazin-6-yl]amino}-4-methoxybenzamide;
3-{[(3*R*)-1,3-dimethyl-2-oxo-4-(tetrahydro-2*H-*pyran-4-yl)-1,2,3,4-tetrahydropyrido[2,3-*b*]pyrazin-6-yl]amino}-*N*-(1-methylpiperidin-4-yl)benzenesulphonamide;
*N*-[2-(dimethylamino)ethyl]-3-{[(3*R*)-1,3-dimethyl-2-oxo-4-(tetrahydro-2H-pyran-4-yl)-1,2,3,4-tetrahydropyrido[2,3-*b*]pyrazin-6-yl]amino}benzenesulphonamide;
*N*-{*trans*-4-[4-(cyclopropylmethyl)piperazin-1-yl]cyclohexyl}-3-{[4-(2,6-difluorobenzyl)-1,3-dimethyl-2-oxo-1,2,3,4-tetrahydroquinoxalin-6-yl]amino}benzamide;
3-{[(3*R*)-1,3-dimethyl-2-oxo-4-(tetrahydro-2H-pyran-4-yl)-1,2,3,4-tetrahydropyrido[2,3-b]pyrazin-6-yl]amino}-*N*-ethylbenzenesulphonamide;
(3*R*)-1,3-dimethyl-4-(1-methylpiperidin-4-yl)-6-{[3-(pyrrolidin-1-ylsulphonyl)phenyl]amino}-3,4-dihydropyrido[2,3-b]pyrazin-2(1H)-one;
(3*R*)-6-{[2-methoxy-5-(morpholin-4-ylsulphonyl)phenyl]amino}-1,3-dimethyl-4-(1-methylpiperidin-4-yl)-3,4-dihydropyrido[2,3-b]pyrazin-2(1H)-one;
*N*-cyclopropyl-3-{[(3*R*)-1,3-dimethyl-2-oxo-4-(tetrahydro-2H-pyran-4-yl)-1,2,3,4-tetrahydropyrido[2,3-b]pyrazin-6-yl]amino}benzenesulphonamide;
(3*R*)-1,3-dimethyl-6-{[3-(pyrrolidin-1-ylsulphonyl)phenyl]amino}-4-(tetrahydro-2H-pyran-4-yl)-3,4-dihydropyrido[2,3-b]pyrazin-2(1H)-one;
(3*R*)-6-{[2-methoxy-5-(morpholin-4-ylsulphonyl)phenyl]amino}-1,3-dimethyl-4-(tetrahydro-2H-pyran-4-yl)-3,4-dihydropyrido[2,3-b]pyrazin-2(1H)-one;
(3*R*)-6-({3-[(3,3-difluoroazetidin-1-yl)sulphonyl]phenyl}amino)-1,3-dimethyl-4-(1-methylpiperidin-4-yl)-3,4-dihydropyrido[2,3-b]pyrazin-2(1H)-one;
(3*R*)-6-{[2-methoxy-5-(2-oxa-6-azaspiro[3.3]hept-6-ylsulphonyl)phenyl]amino}-1,3-dimethyl-4-(tetrahydro-2H-pyran-4-yl)-3,4-dihydropyrido[2,3-b]pyrazin-2(1H)-one;
(3*R*)-6-({3-[(3,3-difluoroazetidin-1-yl)sulphonyl]phenyl}amino)-1,3-dimethyl-4-(tetrahydro-2H-pyran-4-yl)-3,4-dihydropyrido[2,3-b]pyrazin-2(1H)-one;
3-{[(3*R*)-1,3-dimethyl-2-oxo-4-(tetrahydro-2H-pyran-4-yl)-1,2,3,4-tetrahydropyrido[2,3-b]pyrazin-6-yl]amino}-N,N-diethylbenzenesulphonamide;
3-{[(3*R*)-4-benzyl-1,3-dimethyl-2-oxo-1,2,3,4-tetrahydropyrido[2,3-b]pyrazin-6-yl]amino}-*N*-(1-methylpiperidin-4-yl)benzenesulphonamide;
*N*-{*trans*-4-[4-(cyclopropylmethyl)piperazin-1-yl]cyclohexyl}-3-{[(3*R*)-4-isopropyl-1,3-dimethyl-2-oxo-1,2,3,4-tetrahydropyrido[2,3-b]pyrazin-6-yl]amino}benzenesulphonamide;
*N*-{*trans*-4-[4-(cyclopropylmethyl)piperazin-1-yl]cyclohexyl}-3-{[(3*R*)-1,3-dimethyl-2-oxo-4-(tetrahydro-2H-pyran-4-yl)-1,2,3,4-tetrahydropyrido[2,3-b]pyrazin-6-yl]amino}benzenesulphonamide;
*N*-{*trans*-4-[4-(cyclopropylmethyl)piperazin-1-yl]cyclohexyl}-3-{[(*3S*)-1,3-dimethyl-2-oxo-4-(tetrahydro-2H-pyran-4-yl)-1,2,3,4-tetrahydropyrido[2,3-b]pyrazin-6-yl]amino}benzenesulphonamide;
(3*R*)-1,3-dimethyl-4-(tetrahydro-2H-pyran-4-yl)-6-[(3-{[4-(2,2,2-trifluoroethyl)piperazin-1-yl]sulphonyl}phenyl)amino]-3,4-dihydropyrido[2,3-b]pyrazin-2(1H)-one;
3-{[(3*R*)-4-benzyl-1,3-dimethyl-2-oxo-1,2,3,4-tetrahydropyrido[2,3-b]pyrazin-6-yl]amino}-*N-*{*trans*-4-[4-(cyclopropylmethyl)piperazin-1-yl]cyclohexyl}benzenesulphonamide;
3-{[(3*R*)-1,3-dimethyl-2-oxo-4-(tetrahydro-2H-pyran-4-yl)-1,2,3,4-tetrahydropyrido[2,3-b]pyrazin-6-yl]amino}-*N*-methylbenzenesulphonamide;
(3*R*)-6-({2-methoxy-5-[(4-methylpiperazin-1-yl)sulphonyl]phenyl}amino)-1,3-dimethyl-4-(tetrahydro-2H-pyran-4-yl)-3,4-dihydropyrido[2,3-b]pyrazin-2(1H)-one;
3-{[(3*R*)-1,3-dimethyl-4-(1-methylpiperidin-4-yl)-2-oxo-1,2,3,4-tetrahydropyrido[2,3-b]pyrazin-6-yl]amino}-*N,N*-dimethylbenzenesulphonamide;
3-{[(3*R*)-1,3-dimethyl-2-oxo-4-(tetrahydro-2H-pyran-4-yl)-1,2,3,4-tetrahydropyrido[2,3-b]pyrazin-6-yl]amino}-*N-*isopropylbenzenesulphonamide;
(3*R*)-4-isopropyl-1,3-dimethyl-6-({3-[(4-methylpiperazin-1-yl)sulphonyl]phenyl}amino)-3,4-dihydropyrido[2,3-b]pyrazin-2(1H)-one;
3-{[(3*R*)-1,3-dimethyl-2-oxo-4-(tetrahydro-2H-pyran-4-yl)-1,2,3,4-tetrahydropyrido[2,3-b]pyrazin-6-yl]amino}-*N*-[(1-methylpiperidin-4-yl)methyl]benzenesulphonamide;
*N*-{*trans*-4-[4-(cyclopropylmethyl)piperazin-1-yl]cyclohexyl}-3-{[(3*R*)-4-isobutyl-1,3-dimethyl-2-oxo-1,2,3,4-tetrahydropyrido[2,3-b]pyrazin-6-yl]amino}benzenesulphonamide;
(3*R*)-6-({2-methoxy-5-[(4-methylpiperazin-1-yl)sulphonyl]phenyl}amino)-1,3-dimethyl-4-(1-methylpiperidin-4-yl)-3,4-dihydropyrido[2,3-b]pyrazin-2(1H)-one;
(3*R*)-1,3-dimethyl-4-(1-methylpiperidin-4-yl)-6-{[3-(morpholin-4-ylsulphonyl)phenyl]amino}-3,4-dihydropyrido[2,3-b]pyrazin-2(1H)-one;
3-{[(3*R*)-4-cycloheptyl-1,3-dimethyl-2-oxo-1,2,3,4-tetrahydropyrido[2,3-b]pyrazin-6-yl]amino}-*N*-(1-methylpiperidin-4-yl)benzenesulphonamide;
4-(2-methoxyethyl)-1,3-dimethyl-6-{[3-(morpholin-4-ylsulphonyl) phenyl]amino}-3,4-dihydropyrido[2,3-b]pyrazin-2(1H)-one;
(3*R*)-1,3-dimethyl-6-({3-[(4-methylpiperazin-1-yl)sulphonyl]phenyl}amino)-4-(1-methylpiperidin-4-yl)-3,4-dihydropyrido[2,3-b]pyrazin-2(1H)-one;
3-{[(3*R*)-4-isopropyl-1,3-dimethyl-2-oxo-1,2,3,4-tetrahydropyrido[2,3-b]pyrazin-6-yl]amino}-N-(1-methylpiperidin-4-yl)benzenesulphonamide;
*tert*-butyl 4*-*[(3*R*)-6-{[3-({*tr*ans-4-[4-(cyclopropylmethyl)piperazin-1-yl]cyclohexyl}sulphamoyl)phenyl]amino}-1,3-dimethyl-2-oxo-2,3-dihydropyrido[2,3-b]pyrazin-4(1H)-yl]piperidine-1-carboxylate;
4-(2-methoxyethyl)-1,3-dimethyl-6-({3-[(4-methylpiperazin-1-yl)sulphonyl]phenyl}amino)-3,4-dihydropyrido[2,3-b]pyrazin-2(1H)-one;
3-{[(3*R*)-1,3-dimethyl-2-oxo-4-(tetrahydro-2H-pyran-4-yl)-1,2,3,4-tetrahydropyrido[2,3-b]pyrazin-6-yl](methyl)amino}-*N*,*N-*dimethylbenzenesulphonamide;
*N*-[2-(dimethylamino)ethyl]-3-{[(3*R*)-4-isopropyl-1,3-dimethyl-2-oxo-1,2,3,4-tetrahydro-pyrido[2,3-b]pyrazin-6-yl]amino}benzenesulphonamide;
(3*R*)-6-[(1,1-dioxido-2,3-dihydro-1,2-benzothiazol-6-yl)amino]-1,3-dimethyl-4-(tetrahydro-2H-pyran-4-yl)-3,4-dihydropyrido[2,3-b]pyrazin-2(1H)-one;
*tert*-butyl 4-[(3*R*)-1,3-dimethyl-6-({3-[(1-methylpiperidin-4-yl)sulphamoyl]phenyl}amino)-2-oxo-2,3-dihydro-pyrido[2,3-b]pyrazin-4(1H)-yl]piperidine-1-carboxylate;
3-{[(3*R*)-1,3-dimethyl-2-oxo-4-(tetrahydro-2H-pyran-4-yl)-1,2,3,4-tetrahydropyrido[2,3-b]pyrazin-6-yl]amino}benzenesulphonamide;
*tert*-butyl 4-[(3*R*)-6-[(3-{[2-(dimethylamino)ethyl]sulphamoyl}phenyl)amino]-1,3-dimethyl-2-oxo-2,3-dihydropyrido[2,3-b]pyrazin-4(1H)-yl]piperidine-1-carboxylate;
*tert*-butyl 4-[(3*R*)-6-{[3-(2-azaspiro[3.3]hept-2-ylsulphonyl)phenyl]amino}-1,3-dimethyl-2-oxo-2,3-dihydropyrido[2,3-b]pyrazin-4(1H)-yl]piperidine-1-carboxylate;
*tert*-butyl 4-[(3*R*)-6-{[3-(dimethylsulphamoyl)phenyl]amino}-1,3-dimethyl-2-oxo-2,3-dihydropyrido[2,3-b]pyrazin-4(1H)-yl]piperidine-1-carboxylate;
*tert*-butyl 4-[(3*R*)-1,3-dimethyl-6-({3-[(4-methylpiperazin-1-yl)sulphonyl]phenyl}amino)-2-oxo-2,3-dihydropyrido[2,3-b]pyrazin-4(1H)-yl]piperidine-1-carboxylate;
5-{[(3*R*)-1,3-dimethyl-2-oxo-4-(tetrahydro-2H-pyran-4-yl)-1,2,3,4-tetrahydropyrido[2,3-b]pyrazin-6-yl]amino}-2-methylbenzenesulphonamide;
1,3-dimethyl-4-(3-methylphenyl)-6-{[3-(morpholin-4-ylsulphonyl)phenyl]amino}-3,4-dihydropyrido[2,3-b]pyrazin-2(1H)-one;
3-{[1,3-dimethyl-4-(3-methylphenyl)-2-oxo-1,2,3,4-tetrahydropyrido[2,3-b]pyrazin-6-yl]amino}-*N*-(1-methylpiperidin-4-yl)benzenesulphonamide;
*tert*-butyl 4-[(3*R*)-1,3-dimethyl-2-oxo-6-[(3-{[4-(2,2,2-trifluoroethyl)piperazin-1-yl]sulphonyl}phenyl)amino]-2,3-dihydropyrido[2,3-b]pyrazin-4(1H)-yl]piperidine-1-carboxylate;
*tert*-butyl 4-{4-[1,3-dimethyl-6-({3-[(4-methylpiperazin-1-yl)sulphonyl]phenyl}amino)-2-oxo-2,3-dihydropyrido[2,3-b]pyrazin-4(1H)-yl]phenyl}piperazien-1-carboxylate;
*tert*-butyl 4-[(2*R*)-7-{[3-(dimethylsulphamoyl)phenyl]amino}-2,4-dimethyl-3-oxo-3,4-dihydroquinoxalin-1(2H)-yl]piperidine-1-carboxylate;
3-{[(3R)-4-benzyl-1,3-dimethyl-2-oxo-1,2,3,4-tetrahydroquinoxalin-6-yl]amino}-*N,N-*dimethylbenzenesulphonamide;
*tert*-butyl 4-[(2*R*)-7-{[3-(dimethylsulphamoyl)phenyl](methyl)amino}-2,4-dimethyl-3-oxo-3,4-dihydroquinoxalin-1(2H)-yl]piperidine-1-carboxylate;
(3*R*)-1,3-dimethyl-6-({3-[(4-methylpiperazin-1-yl)sulphonyl]phenyl}amino)-4-(tetrahydro-2H-pyran-4-yl)-3,4-dihydroquinoxalin-2(1H)-one;
3-{[(3*R*)-4-benzyl-1,3-dimethyl-2-oxo-1,2,3,4-tetrahydropyrido[2,3-b]pyrazin-6-yl]amino}-*N-*{trans-4-[4-(cyclopropylmethyl)piperazin-1-yl]cyclohexyl}benzamide;
3-{[(3*R*)-4-benzyl-1,3-dimethyl-2-oxo-1,2,3,4-tetrahydropyrido[2,3-b]pyrazin-6-yl]amino}-*N*-(1-methylpiperidin-4-yl)benzamide;
3-{[(3*R*)-4-benzyl-1,3-dimethyl-2-oxo-1,2,3,4-tetrahydropyrido[2,3-b]pyrazin-6-yl]amino}-*N*-[2-(4-methylpiperazin-1-yl)ethyl]benzamide;
(3*R*)-4-benzyl-1,3-dimethyl-6-({3-[(4-methylpiperazin-1-yl)carbonyl]phenyl}amino)-3,4-dihydropyrido[2,3-b]pyrazin-2(1H)-one;
(3*R*)-4-benzyl-6-({3-[(4-isopropylpiperazin-1-yl)carbonyl]phenyl}amino)-1,3-dimethyl-3,4-dihydropyrido[2,3-b]pyrazin-2(1H)-one;
(3*R*)-4-isopropyl-1,3-dimethyl-6-({3-[(4-methylpiperazin-1-yl)carbonyl]phenyl}amino)-3,4-dihydropyrido[2,3-b]pyrazin-2(1H)-one;
*tert*-butyl 4-[(3*R*)-1,3-dimethyl-6-({3-[(4-methylpiperazin-1-yl)carbonyl]phenyl}amino)-2-oxo-2,3-dihydropyrido[2,3-b]pyrazin-4(1H)-yl]piperidine-1-carboxylate;
*N*-{*trans*-4-[4-(cyclopropylmethyl)piperazin-1-yl]cyclohexyl}-3-{[(3*R*)-4-isopropyl-1,3-dimethyl-2-oxo-1,2,3,4-tetrahydropyrido[2,3-b]pyrazin-6-yl]amino}benzamide;
3-{[(3*R*)-1,3-dimethyl-2-oxo-4-(tetrahydro-2H-pyran-4-yl)-1,2,3,4-tetrahydropyrido[2,3-b]pyrazin-6-yl]amino}-*N*-{4-[(1-methylpiperidin-4-yl)amino]cyclohexyl}benzamide;
5-{[(3*R*)-1,3-dimethyl-2-oxo-4-(tetrahydro-2H-pyran-4-yl)-1,2,3,4-tetrahydropyrido[2,3-b]pyrazin-6-yl]amino}-2-methoxy-*N*-(1-methylpiperidin-4-yl)benzamide;
*N*-{*trans*-4-[4-(cyclopropylmethyl)piperazin-1-yl]cyclohexyl}-5-{[(3*R*)-1,3-dimethyl-2-oxo-4-(tetrahydro-2H-pyran-4-yl)-1,2,3,4-tetrahydropyrido[2,3-b]pyrazin-6-yl]amino}-2-methoxybenzamide;
(3*R*)-6-({4-methoxy-3-[(4-methylpiperazin-1-yl)carbonyl]phenyl}amino)-1,3-dimethyl-4-(tetrahydro-2H-pyran-4-yl)-3,4-dihydropyrido[2,3-b]pyrazin-2(1H)-one;
3-{[(3*R*)-4-cyclohexyl-1,3-dimethyl-2-oxo-1,2,3,4-tetrahydropyrido[2,3-b]pyrazin-6-yl]amino}-N-[2-(4-methylpiperazin-1-yl)ethyl]benzamide;
(3*R*)-4-cyclohexyl-6-({3-[(4-isopropylpiperazin-1-yl)carbonyl]phenyl}amino)-1,3-dimethyl-3,4-dihydropyrido[2,3-b]pyrazin-2(1H)-one;
3-{[(3*R*)-4-cyclohexyl-1,3-dimethyl-2-oxo-1,2,3,4-tetrahydropyrido[2,3-b]pyrazin-6-yl]amino}-*N*-(4-hydroxycyclohexyl)benzamide;
*N*-{*trans*-4-[4-(cyclopropylmethyl)piperazin-1-yl]cyclohexyl}-3-{[(3*R*)-1,3-dimethyl-2-oxo-4-(tetrahydro-2H-pyran-4-yl)-1,2,3,4-tetrahydropyrido[2,3-b]pyrazin-6-yl]amino}-4-methoxybenzamide;
(3*R*)-6-[(3-{[4-(cyclopropylmethyl)piperazin-1-yl]carbonyl}phenyl) amino]-4-isopropyl-1,3-dimethyl-3,4-dihydropyrido[2,3-b]pyrazin-2(1H)-one;
*N*-[4-(4,4-difluoropiperidin-1-yl)cyclohexyl]-5-{[(3*R*)-1,3-dimethyl-2-oxo-4-(tetrahydro-2H-pyran-4-yl)-1,2,3,4-tetrahydropyrido[2,3-b]pyrazin-6-yl]amino}-2-methoxybenzamide;
*N*-[*cis*-4-(4-cyclopropylpiperazin-1-yl)cyclohexyl]-3-{[(3*R*)-1,3-dimethyl-2-oxo-4-(tetrahydro-2H-pyran-4-yl)-1,2,3,4-tetrahydropyrido[2,3-b]pyrazin-6-yl]amino}-4-methoxybenzamide;
*N*-{*trans*-4-[4-(cyclopropylmethyl)piperazin-1-yl]cyclohexyl}-3-{[(3*R*)-1,3-dimethyl-2-oxo-4-(tetrahydro-2H-pyran-4-yl)-1,2,3,4-tetrahydropyrido[2,3-b]pyrazin-6-yl]amino}-2-methoxybenzamide;
(3*R*)-6-({2-methoxy-3-[(4-methylpiperazin-1-yl)carbonyl]phenyl}amino)-1,3-dimethyl-4-(tetrahydro-2H-pyran-4-yl)-3,4-dihydropyrido[2,3-b]pyrazin-2(1H)-one;
*N*-{*trans*-4-[4-(cyclopropylmethyl)piperazin-1-yl]cyclohexyl}-3-{[(3*R*)-4-(4-fluorophenyl)-1,3-dimethyl-2-oxo-1,2,3,4-tetrahydropyrido[2,3-b]pyrazin-6-yl]amino}benzamide;
3-{[(3*R*)-4-isopropyl-1,3-dimethyl-2-oxo-1,2,3,4-tetrahydropyrido[2,3-b]pyrazin-6-yl]amino}-*N*-(1-methylpiperidin-4-yl)benzamide;
3-{[(3*R*)-4-cyclohexyl-1,3-dimethyl-2-oxo-1,2,3,4-tetrahydropyrido [2,3-b]pyrazin-6-yl]amino}-*N*-(1-methylpiperidin-4-yl)benzamide;
3-{[(3*R*)-4-(4,4-dimethylcyclohexyl)-1,3-dimethyl-2-oxo-1,2,3,4-tetrahydropyrido[2,3-b]pyrazin-6-yl]amino}-*N*-(1-methylpiperidin-4-yl)benzamide;
*N*-[2-(dimethylamino)ethyl]-3-{[(3R)-4-isopropyl-1,3-dimethyl-2-oxo-1,2,3,4-tetrahydropyrido[2,3-b]pyrazin-6-yl]amino}benzamide;
3-{[4-(2-methoxyethyl)-1,3-dimethyl-2-oxo-1,2,3,4-tetrahydropyrido [2,3-b]pyrazin-6-yl]amino}-*N*-(1-methylpiperidin-4-yl)benzamide;
3-{[(3*R*)-4-cyclohexyl-1,3-dimethyl-2-oxo-1,2,3,4-tetrahydropyrido[2,3-b]pyrazin-6-yl]amino}-*N-*{*trans*-4-[4-(cyclopropylmethyl)piperazin-1-yl]cyclohexyl}benzamide;
*tert*-butyl 4-[(3*R*)-1,3-dimethyl-6-({3-[(1-methylpiperidin-4-yl)carbamoyl]phenyl}amino)-2-oxo-2,3-dihydropyrido[2,3-b]pyrazin-4(1H)-yl]piperidine-1-carboxylate;
*N*-{*trans*-4-[4-(cyclopropylmethyl)piperazin-1-yl]cyclohexyl}-3-{[(3*R*)-4-(2-methoxyethyl) -1,3-dimethyl-2-oxo-1,2,3,4-tetrahydropyrido[2,3-b]pyrazin-6-yl]amino}benzamide;
*tert*-butyl 4-[(3*R*)-6-{[3-(*trans*-4-[4-(cyclopropylmethyl)piperazin-1-yl]cyclohexyl}carbamoyl)phenyl]amino}-1,3-dimethyl-2-oxo-2,3-dihydropyrido[2,3-b]pyrazin-4(1H)-yl]piperidine-1-carboxylate;
3-{[(3*R*)-1,3-dimethyl-2-oxo-4-(tetrahydro-2H-pyran-4-yl)-1,2,3,4-tetrahydropyrido[2,3-b]pyrazin-6-yl]amino}-2-methoxy-*N*-(1-methylpiperidin-4-yl)benzamide;
3-[(1,3-dimethyl-2-oxo-4-phenyl-1,2,3,4-tetrahydropyrido[2,3-b]pyrazin-6-yl)amino]-*N*-(1-methylpiperidin-4-yl)benzamide;
*tert*-butyl 4-[(2*R*)-7-{[3-(dimethylcarbamoyl)phenyl]amino}-2,4-dimethyl-3-oxo-3,4-dihydroquinoxalin-1(2H)-yl]piperidine-1-carboxylate;
(3*R*)-1,3-dimethyl-6-({3-[(4-methylpiperazin-1-yl)carbonyl]phenyl}amino)-4-(tetrahydro-2H-pyran-4-yl)-3,4-dihydroquinoxalin-2(1H)-one;
3-{[(3*R*)-4-benzyl-1,3-dimethyl-2-oxo-1,2,3,4-tetrahydroquinoxalin-6-yl]amino}-*N,N-*dimethylbenzamide;
*N*-{*trans*-4-[4-(cyclopropylmethyl)piperazin-1-yl]cyclohexyl}-3-{[(3*R*)-1,3-dimethyl-2-oxo-4-(tetrahydro-2H-pyran-4-yl)-1,2,3,4-tetrahydroquinoxalin-6-yl]amino}benzamide;
3-{[(3*R*)-1,3-dimethyl-2-oxo-4-(tetrahydro-2H-pyran-4-yl)-1,2,3,4-tetrahydroquinoxalin-6-yl]amino}-*N*-(1-methylpiperidin-4-yl)benzamide;
3-[(4-benzyl-1,3-dimethyl-2-oxo-1,2,3,4-tetrahydroquinoxalin-6-yl)(methyl)amino]-*N,N-*dimethylbenzamide;
(3*R*)-4-(2-methoxyethyl)-1,3-dimethyl-6-{[3-(morpholin-4-ylsulphonyl) phenyl]amino}-3,4-dihydropyrido[2,3-b]pyrazin-2(1H)-one;
(3*S*)-4-(2-methoxyethyl)-1,3-dimethyl-6-{[3-(morpholin-4-ylsulphonyl) phenyl]amino}-3,4-dihydropyrido[2,3-b]pyrazin-2(1H)-one;
(3*R*)-4-(2-methoxyethyl)-1,3-dimethyl-6-({3-[(4-methylpiperazin-1-yl)sulphonyl]phenyl}amino)-3,4-dihydropyrido[2,3-b]pyrazin-2(1H)-one;
(3*R*)-4-(4-fluorophenyl)-1,3-dimethyl-6-{[3-(morpholin-4-ylsulphonyl)phenyl]amino}-3,4-dihydropyrido[2,3-b]pyrazin-2(1H)-one;
3-{[(3*R*)-4-(4-fluorophenyl)-1,3-dimethyl-2-oxo-1,2,3,4-tetrahydropyrido[2,3-b]pyrazin-6-yl]amino}-*N*-(1-methylpiperidin-4-yl)benzamide;
3-{[(3*S*)-4-(4-fluorophenyl)-1,3-dimethyl-2-oxo-1,2,3,4-tetrahydropyrido[2,3-b]pyrazin-6-yl]amino}-*N*-(1-methylpiperidin-4-yl)benzamide;
(3*R*)-4-(2-methoxyethyl)-1,3-dimethyl-6-({3-[(4-methylpiperazin-1-yl)carbonyl]phenyl}amino)-3,4-dihydropyrido[2,3-b]pyrazin-2(1H)-one;
(3*S*)-4-(2-methoxyethyl)-1,3-dimethyl-6-({3-[(4-methylpiperazin-1-yl)carbonyl]phenyl}amino)-3,4-dihydropyrido[2,3-b]pyrazin-2(1H)-one;
3-{[(3*R*)-4-(2-methoxyethyl)-1,3-dimethyl-2-oxo-1,2,3,4-tetrahydropyrido[2,3-b]pyrazin-6-yl]amino}-*N*-(1-methylpiperidin-4-yl)benzamide;
3-{[(3*S*)-4-(2-methoxyethyl)-1,3-dimethyl-2-oxo-1,2,3,4-tetrahydropyrido[2,3-b]pyrazin-6-yl]amino}-*N*-(1-methylpiperidin-4-yl)benzamide;
3-{[(3*R*)-1,3-dimethyl-2-oxo-4-phenyl-1,2,3,4-tetrahydropyrido[2,3-b]pyrazin-6-yl]amino}-*N*-(1-methylpiperidin-4-yl)benzamide;
(3*R*)-6-{[3-(2-azaspiro[3.3]hept-2-ylsulphonyl)phenyl]amino}-1,3-dimethyl-4-(piperidin-4-yl)-3,4-dihydropyrido[2,3-b]pyrazin-2(1H)-one;
3-{[(3*R*)-1,3-dimethyl-2-oxo-4-(piperidin-4-yl)-1,2,3,4-tetrahydropyrido[2,3-b]pyrazin-6-yl]amino}-*N,N*-dimethylbenzenesulphonamide;
3-{[(3*R*)-1,3-dimethyl-2-oxo-4-(piperidin-4-yl)-1,2,3,4-tetrahydropyrido[2,3-b]pyrazin-6-yl]amino}-*N*-(1-methylpiperidin-4-yl)benzenesulphonamide;
(3*R*)-1,3-dimethyl-6-({3-[(4-methylpiperazin-1-yl)sulphonyl]phenyl}amino)-4-(piperidin-4-yl)-3,4-dihydropyrido[2,3-b]pyrazin-2(1H)-one;
(3*R*)-1,3-dimethyl-4-(piperidin-4-yl)-6-[(3-{[4-(2,2,2-trifluoroethyl)piperazin-1-yl]sulphonyl}phenyl)amino]-3,4-dihydropyrido[2,3-b]pyrazin-2(1H)-one;
*N*-{trans-4-[4-(cyclopropylmethyl)piperazin-1-yl]cyclohexyl}-3-{[(3*R*)-1,3-dimethyl-2-oxo-4-(piperidin-4-yl)-1,2,3,4-tetrahydropyrido[2,3-b]pyrazin-6-yl]amino}benzenesulphonamide;
*N*-[2-(dimethylamino)ethyl]-3-{[(3*R*)-1,3-dimethyl-2-oxo-4-(piperidin-4-yl)-1,2,3,4-tetrahydropyrido[2,3-b]pyrazin-6-yl]amino}benzenesulphonamide;
3-{[(3*R*)-1,3-dimethyl-2-oxo-4-(piperidin-4-yl)-1,2,3,4-tetrahydroquinoxalin-6-yl]amino}-*N*,*N-*dimethylbenzenesulphonamide;
(3*R*)-1,3-dimethyl-6-({3-[(4-methylpiperazin-1-yl)carbonyl]phenyl}amino)-4-(piperidin-4-yl)-3,4-dihydropyrido[2,3-b]pyrazin-2(1H)-one;
*N*-{*trans*-4-[4-(cyclopropylmethyl)piperazin-1-yl]cyclohexyl}-3-{[(3*R*)-1,3-dimethyl-2-oxo-4-(piperidin-4-yl)-1,2,3,4-tetrahydropyrido[2,3-b]pyrazin-6-yl]amino}benzamide;
3-{[(3*R*)-1,3-dimethyl-2-oxo-4-(piperidin-2-yl)-1,2,3,4-tetrahydropyrido[2,3-b]pyrazin-6-yl]amino}-*N*-(1-methylpiperidin-4-yl)benzamide;
3-{[(3*R*)-1,3-dimethyl-2-oxo-4-(piperidin-4-yl)-1,2,3,4-tetrahydroquinoxalin-6-yl]amino}-*N,N-*dimethylbenzamide;
(3*R*)-1,3-dimethyl-6-{[3-(5-methyl-1,3,4-oxadiazol-2-yl)phenyl]amino}-4-(tetrahydro-2H-pyran-4-yl)-3,4-dihydropyrido[2,3-b]pyrazin-2(1H)-one;
4-(4-fluorophenyl)-1,3-dimethyl-6-{[3-(morpholin-4-ylsulphonyl) phenyl]amino}-3,4-dihydropyrido[2,3-b]pyrazin-2(1H)-one;
3-{[4-(4-fluorophenyl)-1,3-dimethyl-2-oxo-1,2,3,4-tetrahydropyrido[2,3-b]pyrazin-6-yl]amino}-*N*-(1-methylpiperidin-4-yl)benzamide
and
4-(2-methoxyethyl)-1,3-dimethyl-6-({3-[(4-methylpiperazin-1-yl)carbonyl]phenyl}amino)-3,4-dihydropyrido[2,3-b]pyrazin-2(1H)-one.

9. Compounds according to Claims 1 to 8 for use as medicaments.

10. Compounds according to Claims 1 to 8 for use in prophylaxis and/or treatment of tumour diseases.

11. Use of the compounds according to Claims 1 to 8 for production of a medicament.

12. Use of the compounds according to Claims 1 to 8 for production of a medicament for prophylaxis and/or treatment of tumour diseases.

13. Compounds according to Claims 1 to 8 for use in prophylaxis and/or treatment of hyperproliferative disorders.

14. Compounds according to Claims 1 to 8 for use in prophylaxis and/or treatment of viral infections, neurodegenerative disorders, inflammation disorders, atherosclerotic disorders and in male fertility control.

15. Use of the compounds according to Claims 1 to 8 for production of a medicament for prophylaxis and/or treatment of viral infections, neurodegenerative disorders, inflammation disorders, atherosclerotic disorders and in male fertility control.

16. Compounds according to Claims 1 to 8 in combination with one or more further pharmacologically active substances.

17. Compounds according to Claims 1 to 8 in combination with one or more further pharmacologically active substances for use in prophylaxis and/or treatment of hyperproliferative disorders.

18. Compounds according to Claims 1 to 8 in combination with one or more further pharmacologically active substances for use in prophylaxis and/or treatment of tumour diseases.

19. Compounds according to Claims 1 to 8 in combination with one or more further pharmacologically active substances for use in prophylaxis and/or treatment of viral infections, neurodegenerative disorders, inflammation disorders, atherosclerotic disorders and in male fertility control.

20. Compounds of the general formulae (IX) and (XVI) 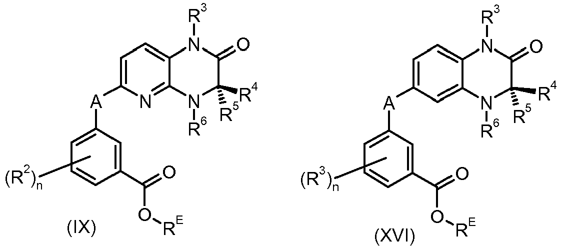 in which A, R², R³, R⁴, R⁵, R⁶ and n are each as defined in the general formula (I) according to Claim 1 and R^{E} is C₁-C₆-alkyl.

21. Compounds of the general formulae (IX) and (XVI) according to Claim 20, in which R^{E} is methyl or ethyl.

22. Compounds of the general formulae (IX) and (XVI) according to Claims 20 and 21:
methyl 3-{[(3*R*)-4-cyclopentyl-1,3-dimethyl-2-oxo-1,2,3,4-tetrahydropyrido[2,3-b]pyrazin-6-yl]amino}benzoate;
methyl 3-{[(3*R*)-1,3-dimethyl-2-oxo-4-(tetrahydro-2*H*-pyran-4-yl)-1,2,3,4-tetrahydropyrido[2,3-*b*]pyrazin-6-yl]amino}benzoate;
methyl 3-{[(3*R*)-1,3-dimethyl-2-oxo-4-(tetrahydro-2*H*-pyran-4-yl)-1,2,3,4-tetrahydropyrido[2,3-*b*]pyrazin-6-yl]amino}-4-methoxybenzoate;
ethyl 3-{[4-(2,6-difluorobenzyl)-1,3-dimethyl-2-oxo-1,2,3,4-tetrahydroquinoxalin-6-yl]amino}benzoate;
methyl 3-{[(3R)-4-cyclohexyl-1,3-dimethyl-2-oxo-1,2,3,4-tetrahydropyrido[2,3-b]pyrazin-6-yl]amino}benzoate;
methyl 3-{[(3*R*)-1,3-dimethyl-2-oxo-4-(propan-2-yl)-1,2,3,4-tetrahydropyrido[2,3-b]pyrazin-6-yl]amino}benzoate;
methyl 3-{[(3*R*)-4-benzyl-1,3-dimethyl-2-oxo-1,2,3,4-tetrahydropyrido[2,3-b]pyrazin-6-yl]amino}benzoate;
methyl 3-{[4-(2-methoxyethyl)-1,3-dimethyl-2-oxo-1,2,3,4-tetrahydropyrido[2,3-b]pyrazin-6-yl]amino}benzoate;
*tert*-butyl 4-[(3*R*)-6-{[3-(ethoxycarbonyl)phenyl]amino}-1,3-dimethyl-2-oxo-2,3-dihydropyrido[2,3-b]pyrazin-4(1H)-yl]piperidine-1-carboxylate;
methyl 3-{[4-(4-fluorophenyl)-1,3-dimethyl-2-oxo-1,2,3,4-tetrahydropyrido[2,3-b]pyrazin-6-yl]amino}benzoate;
methyl 5-{[(3*R*)-1,3-dimethyl-2-oxo-4-(tetrahydro-2H-pyran-4-yl)-1,2,3,4-tetrahydropyrido[2,3-b]pyrazin-6-yl]amino}-2-methoxybenzoate;
Methyl 3-{[(3*R*)-1,3-dimethyl-2-oxo-4-(tetrahydro-2H-pyran-4-yl)-1,2,3,4-tetrahydropyrido[2,3-b]pyrazin-6-yl]amino}-2-methoxybenzoate
and
methyl 3-{[(3*R*)-1,3-dimethyl-2-oxo-4-(tetrahydro-2H-pyran-4-yl)-1,2,3,4-tetrahydroquinoxalin-6-yl]amino}benzoate.

23. Compounds of the general formulae (X) and (XVII) 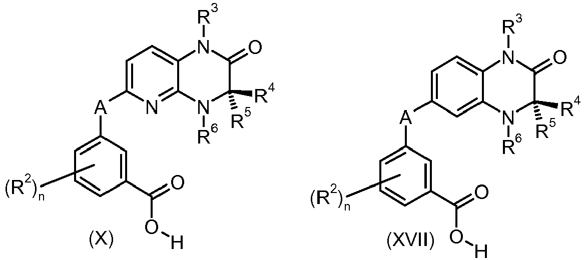 in which A, R², R³, R⁴, R⁵, R⁶ and n are each as defined in the general formula (I) according to Claim 1.

24. Compounds of the general formulae (X) and (XVII) according to Claim 23:
3-{[(3*R*)-4-cyclopentyl-1,3-dimethyl-2-oxo-1,2,3,4-tetrahydropyrido[2,3-*b*]pyrazin-6-yl]amino}benzoic acid;
3-{[(3*R*)-1,3-dimethyl-2-oxo-4-(tetrahydro-2*H-*pyran-4-yl)-1,2,3,4-tetrahydropyrido[2,3-*b*]pyrazin-6-yl]amino}benzoic acid;
3-{[(3*R*)-1,3-dimethyl-2-oxo-4-(tetrahydro-2*H-*pyran-4-yl)-1,2,3,4-tetrahydropyrido[2,3-*b*]pyrazin-6-yl]amino}-4-methoxybenzoic acid;
3-{[4-(2,6-difluorobenzyl)-1,3-dimethyl-2-oxo-1,2,3,4-tetrahydroquinoxalin-6-yl]amino}benzoic acid;
3-{[(3*R*)-4-cyclohexyl-1,3-dimethyl-2-oxo-1,2,3,4-tetrahydropyrido[2,3-b]pyrazin-6-yl]amino}benzoic acid;
3-{[(3*R*)-1,3-dimethyl-2-oxo-4-(propan-2-yl)-1,2,3,4-tetrahydropyrido[2,3-b]pyrazin-6-yl]amino}benzoic acid;
3-{[(3*R*)-4-benzyl-1,3-dimethyl-2-oxo-1,2,3,4-tetrahydropyrido[2,3-b]pyrazin-6-yl]amino}benzoic acid;
3-{[4-(2-methoxyethyl)-1,3-dimethyl-2-oxo-1,2,3,4-tetrahydropyrido[2,3-b]pyrazin-6-yl]amino}benzoic acid;
3-({(3*R*)-4-[1-(*tert*-butoxycarbonyl)piperidin-4-yl]-1,3-dimethyl-2-oxo-1,2,3,4-tetrahydropyrido[2,3-b]pyrazin-6-yl}amino)benzoic acid;
3-{[4-(4-fluorophenyl)-1,3-dimethyl-2-oxo-1,2,3,4-tetrahydropyrido[2,3-b]pyrazin-6-yl]amino}benzoic acid;
5-{[(3*R*)-1,3-dimethyl-2-oxo-4-(tetrahydro-2H-pyran-4-yl)-1,2,3,4-tetrahydropyrido[2,3-b]pyrazin-6-yl]amino}-2-methoxybenzoic acid;
3-{[(3*R*)-1,3-Dimethyl-2-oxo-4-(tetrahydro-2H-pyran-4-yl)-1,2,3,4-tetrahydropyrido[2,3-b]pyrazin-6-yl]amino}-2-methoxybenzoic acid
and
3-{[(3*R*)-1,3-dimethyl-2-oxo-4-(tetrahydro-2H-pyran-4-yl)-1,2,3,4-tetrahydroquinoxalin-6-yl]amino}benzoic acid.

25. Use of the compounds according to Claims 20 to 24 for preparation of the inventive compounds of the general formula (I) according to Claim 1.

## Revendications

1. Composés de formule générale (I) 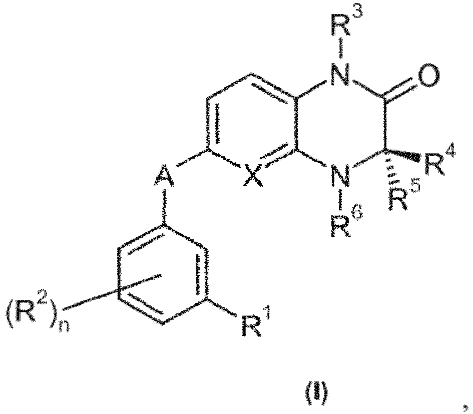 dans laquelle
A représente -NH-, -N(alkyle en C₁-C₃) - ou -O-,
X représente -N- ou -CH-,
n représente 0, 1 ou 2,
R¹ représente un groupe -C(=O)NR⁷R⁸ ou-S(=O)₂NR⁷R⁸,
ou
représente un hétéroaryle monocyclique à 5 éléments, qui peut éventuellement être substitué une, deux ou trois fois, de manière identique ou différente, avec halogène, cyano, alkyle en C₁-C₄, alcényle en C₂-C₄, alcynyle en C₂-C₄, halogène, alkyle en C₁-C₄, alcoxy en C₁-C₄, halogéno-alcoxy en C₁-C₄, alkylthio en C₁-C₄, halogéno-alkylthio en C₁-C₄, -NR⁹R¹⁰, -C(=O)OR¹¹, -C(=O)N⁹R¹⁰, -C(=O)R¹¹, -S(=O)²R¹¹_{,} -S(=O)₂NR⁹R¹⁰,
R² représente hydrogène, halogène, cyano, alkyle en C₁-C₃, alcényle en C₂-C₄, alcynyle en C₂-C₄, halogéno-alkyle en C₁-C₄, alcoxy en C₁-C₄, halogéno-alcoxy en C₁-C₄, alkylthio en C₁-C₄ ou halogéno-alkylthio en C₁-C₄, et si n représente 2, les R² peuvent être identiques ou différents, ou
R¹ et R² représentent ensemble un groupe *-S(=O)₂-NR⁸-CH₂-**, *-S(=O)₂-NR⁸-CH₂-CH₂-**, *-C(=O)-NR⁸-CH₂-** ou *-C(=O)-NR⁸-CH₂-CH₂-**, « * » signifiant le point de liaison de R¹ au cycle phényle contenu dans la formule (I) et « ** » signifiant un atome de carbone voisin de ce point de liaison de ce cycle phényle,
R³ représente méthyle ou éthyle,
R⁴ représente hydrogène ou alkyle en C₁-C₃,
R⁵ représente hydrogène ou alkyle en C₁-C₃, ou
R⁴ et R⁵ représentent ensemble avec l'atome de carbone auquel ils sont reliés un cycloalkylène en C₃-C₆,
R⁶ représente alkyle en C₁-C₆, qui peut éventuellement être substitué une fois avec alcoxy en C₁-C₃, phényle, cycloalkyle en C₃-C₈ ou hétérocycloalkyle de 4 à 8 éléments,
le phényle pouvant de son côté éventuellement être substitué une, deux ou trois fois, de manière identique ou différente, avec : halogène, cyano, alkyle en C₁-C₄, alcényle en C₂-C₄, alcynyle en C₂-C₄, alcoxy en C₁-C₄, halogéno-alkyle en C₁-C₄, halogéno-alcoxy en C₁-C₄, et
le cycloalkyle en C₃-C₈ et l'hétérocycloalkyle de 4 à 8 éléments pouvant de leur côté éventuellement être substitués une ou deux fois, de manière identique ou différente, avec alkyle en C₁-C₃, ou représente cycloalkyle en C₃-C₈ ou hétérocycloalkyle de 4 à 8 éléments, qui peuvent éventuellement être substitués une ou deux fois, de manière identique ou différente, avec alkyle en C₁-C₃ ou alcoxycarbonyle en C₁-C₄,
ou
représente phényle, qui peut éventuellement être substitué une ou deux fois, de manière identique ou différente, avec halogène, alkyle en C₁-C₃ ou hétérocycloalkyle de 4 à 8 éléments,
l'hétérocycloalkyle de 4 à 8 éléments pouvant de son côté éventuellement être substitué une ou deux fois, de manière identique ou différente, avec alkyle en C₁-C₃ ou alcoxycarbonyle en C₁-C₄,
R⁷ représente hydrogène,
ou
représente alkyle en C₁-C₆, qui peut éventuellement être substitué une, deux ou trois fois, de manière identique ou différente, avec : hydroxy, oxo, fluor, cyano, alcoxy en C₁-C₄, halogéno-alcoxy en C₁-C₄, -NR⁹R¹⁰, cycloalkyle en C₃-C₈, cycloalcényle en C₄-C₈, hétérocycloalkyle de 4 à 8 éléments, hétérocycloalcényle de 4 à 8 éléments, spirocycloalkyle en C₅-C₁₁, hétérospirocycloalkyle en C₅-C₁₁, cycloalkyle en C₆-C₁₂ ponté, hétérocycloalkyle en C₆-C₁₂ ponté, bicycloalkyle en C₆-C₁₂, hétérobicycloalkyle en C₆-C₁₂, phényle, hétéroaryle de 5 à 6 éléments,
le cycloalkyle en C₃-C₈, le cycloalcényle en C₄-C₈, l'hétérocycloalkyle de 4 à 8 éléments, l'hétérocycloalcényle de 4 à 8 éléments, le spirocycloalkyle en C₅-C₁₁, l'hétérospirocycloalkyle en C₅-C₁₁, le cycloalkyle en C₆-C₁₂ ponté, l'hétérocycloalkyle en C₆-C₁₂ ponté, le bicycloalkyle en C₆-C₁₂, l'hétérobicycloalkyle en C₆-C₁₂ pouvant de leur côté éventuellement être substitués une ou deux fois, de manière identique ou différente, avec : hydroxy, fluor, oxo, cyano, alkyle en C₁-C₃, fluoro-alkyle en C₁-C₃, cycloalkyle en C₃-C₆, cyclopropylméthyle, alkylcarbonyle en C₁-C₃, alcoxycarbonyle en C₁-C₄ ou-NR⁹R¹⁰, et
le phényle et l'hétéroaryle de 5 à 6 éléments pouvant éventuellement être substitués une ou deux fois, de manière identique ou différente, avec : halogène, cyano, trifluorométhyle, alkyle en C₁-C₃, alcoxy en C₁-C₃,
ou
représente alcényle en C₃-C₆ ou alcynyle en C₃-C₆,
ou
représente cycloalkyle en C₃-C₈, cycloalcényle en C₄-C₈, spirocycloalkyle en C₅-C₁₁, cycloalkyle en C₆-C₁₂ ponté ou bicycloalkyle en C₆-C₁₂, qui peuvent éventuellement être substitués une ou deux fois, de manière identique ou différente, avec : hydroxy, oxo, cyano, fluor, alkyle en C₁-C₃, alcoxy en C₁-C₃, trifluorométhyle, -NR⁹R¹⁰,
ou
représente hétérocycloalkyle de 4 à 8 éléments, hétérocycloalcényle de 4 à 8 éléments, hétérospirocycloalkyle en C₅-C₁₁, hétérocycloalkyle en C₆-C₁₂ ponté ou hétérobicycloalkyle en C₆-C₁₂, qui peuvent éventuellement être substitués une ou deux fois, de manière identique ou différente, avec : hydroxy, fluor, oxo, cyano, alkyle en C₁-C₃, fluoro-alkyle en C₁-C₃, cycloalkyle en C₃-C₆, cyclopropylméthyle, alkylcarbonyle en C₁-C₃, alcoxycarbonyle en C₁-C₄ ou -NR⁹R¹⁰,
R⁸ représente hydrogène, ou alkyle en C₁-C₃ éventuellement substitué une ou deux fois, de manière identique ou différente, avec hydroxy, oxo ou alcoxy en C₁-C₃ ; ou fluoro-alkyle en C₁-C₃,
ou
R⁷ et R⁸ représentent ensemble avec l'atome d'azote auquel ils sont reliés hétérocycloalkyle de 4 à 8 éléments, hétérocycloalcényle de 4 à 8 éléments, hétérospirocycloalkyle en C₅-C₁₁, hétérocycloalkyle en C₆-C₁₂ ponté ou hétérobicycloalkyle en C₆-C₁₂, qui peuvent éventuellement être substitués, une ou deux fois, de manière identique ou différente, avec : hydroxy, fluor, oxo, cyano, alkyle en C₁-C₃, fluoro-alkyle en C₁-C₃, cycloalkyle en C₃-C₆, cyclopropylméthyle, alkylcarbonyle en C₁-C₃, alcoxycarbonyle en C₁-C₄ ou -NR⁹R¹⁰,
R⁹ et R¹⁰ représentent indépendamment l'un de l'autre hydrogène ou alkyle en C₁-C₃ éventuellement substitué une ou deux fois, de manière identique ou différente, avec hydroxy, oxo, alcoxy en C₁-C₃ ; fluoroalkyle en C₁-C₃ ou hétérocycloalkyle de 4 à 8 éléments, l'hétérocycloalkyle de 4 à 8 éléments pouvant de son côté éventuellement être substitué une ou deux fois, de manière identique ou différente, avec alkyle en C₁-C₃,
ou
R⁹ et R¹⁰ représentent ensemble avec l'atome d'azote auquel ils sont reliés hétérocycloalkyle de 4 à 8 éléments, qui peut éventuellement être substitué une ou deux fois, de manière identique ou différente, avec : hydroxy, fluor, oxo, cyano, alkyle en C₁-C₃, fluoro-alkyle en C₁-C₃, cycloalkyle en C₃-C₆, cyclopropylméthyle, alkylcarbonyle en C₁-C₃ ou alcoxycarbonyle en C₁-C₄,
R¹¹ représente alkyle en C₁-C₆ ou phényl-alkyle en C₁-C₃,
ainsi que leurs diastéréomères, racémats, polymorphes et sels physiologiquement compatibles.

2. Composés de formule générale (I) selon la revendication 1, dans laquelle
A représente -NH- ou -N(alkyle en C₁-C₃) -,
X représente -N- ou -CH-,
n représente 0, 1 ou 2,
R¹ représente un groupe -C(=O)NR⁷R⁸ ou-S(=O)₂NR⁷R⁸,
ou
représente oxazolyle, thiazolyle, oxadiazolyle ou thiadiazolyle, qui peuvent éventuellement être substitués une ou deux fois, de manière identique ou différente, avec halogène, cyano, alkyle en C₁-C₃, trifluorométhyle, alcoxy en C₁-C₃, trifluorométhoxy ou -NR⁹R¹⁰,
R² représente hydrogène, fluor, chlore, cyano, méthyle, méthoxy, éthyle ou éthoxy, et si n représente 2, les R² peuvent être identiques ou différents,
ou
R¹ et R² représentent ensemble un groupe *-S(=O)₂-NR⁸-CH₂-** ou *-C(=O)-NR⁸-CH₂-**, « * » signifiant le point de liaison de R¹ au cycle phényle contenu dans la formule (I) et « ** » signifiant un atome de carbone voisin de ce point de liaison de ce cycle phényle,
R³ représente méthyle ou éthyle,
R⁴ représente hydrogène, méthyle ou éthyle,
R⁵ représente hydrogène, méthyle ou éthyle,
R⁶ représente alkyle en C₂-C₅,
ou
représente méthyle ou éthyle, qui peut est substitué une fois avec alcoxy en C₁-C₃, phényle ou hétérocycloalkyle de 4 à 8 éléments,
le phényle pouvant de son côté éventuellement être substitué une, deux ou trois fois, de manière identique ou différente, avec : fluor, chlore, brome, cyano, alkyle en C₁-C₃, alcoxy en C₁-C₃ et l'hétérocycloalkyle de 4 à 8 éléments pouvant de son côté éventuellement être substitué une ou deux fois, de manière identique ou différente, avec méthyle, ou représente cycloalkyle en C₃-C₈ ou hétérocycloalkyle de 4 à 8 éléments, qui peuvent éventuellement être substitués une ou deux fois, de manière identique ou différente, avec alkyle en C₁-C₃ ou alcoxycarbonyle en C₁-C₄,
ou
représente phényle, qui peut éventuellement être substitué une ou deux fois, de manière identique ou différente, avec fluor, chlore, méthyle ou hétérocycloalkyle à 6 éléments, l'hétérocycloalkyle à 6 éléments pouvant de son côté éventuellement être substitué une fois avec méthyle ou *tert.*-butoxycarbonyle,
R⁷ représente hydrogène,
ou
représente alkyle en C₁-C₆, qui peut éventuellement être substitué une, deux ou trois fois, de manière identique ou différente, avec : hydroxy, oxo, fluor, cyano, alcoxy en C₁-C₃, fluoro-alcoxy en C₁-C₃, -NR⁹R¹⁰, hétérocycloalkyle de 4 à 8 éléments, phényle, hétéroaryle de 5 à 6 éléments,
l'hétérocycloalkyle de 4 à 8 éléments pouvant de son côté éventuellement être substitué une fois avec : hydroxy, oxo, alkyle en C₁-C₃, fluoro-alkyle en C₁-C₃, cyclopropyle, cyclopropylméthyle, acétyle ou *tert*.-butoxycarbonyle,
ou
représente cycloalkyle en C₃-C₆, qui peut éventuellement être substitué une ou deux fois, de manière identique ou différente, avec : hydroxy, oxo, cyano, fluor, -NR⁹R¹⁰,
ou
représente hétérocycloalkyle de 4 à 8 éléments, hétérospirocycloalkyle en C₆-C₈, hétérocycloalkyle en C₆-C₁₀ ponté ou hétérobicycloalkyle en C₆-C₁₀, qui peuvent éventuellement être substitués une ou deux fois, de manière identique ou différente, avec : hydroxy, oxo, alkyle en C₁-C₃, fluoro-alkyle en C₁-C₃, cyclopropyle, cyclopropylméthyle, acétyle ou *tert*.-butoxycarbonyle,
R⁸ représente hydrogène ou alkyle en C₁-C₃, ou
R⁷ et R⁸ représentent ensemble avec l'atome d'azote auquel ils sont reliés hétérocycloalkyle de 4 à 8 éléments, hétérospirocycloalkyle en C₆-C₈, hétérocycloalkyle en C₆-C₁₀ ponté ou hétérobicycloalkyle en C₆-C₁₀,
qui peuvent éventuellement être substitués, une ou deux fois, de manière identique ou différente, avec : hydroxy, fluor, oxo, alkyle en C₁-C₃, fluoro-alkyle en C₁-C₃, cyclopropyle, cyclopropylméthyle, acétyle ou *tert.-*butoxycarbonyle,
R⁹ et R¹⁰ représentent indépendamment l'un de l'autre hydrogène ou alkyle en C₁-C₃ éventuellement substitué une fois avec hydroxy ou oxo ; ou trifluorométhyle ou hétérocycloalkyle à 6 éléments, l'hétérocycloalkyle à 6 éléments pouvant de son côté éventuellement être substitué une ou deux fois, de manière identique ou différente, avec alkyle en C₁-C₃,
ou
R⁹ et R¹⁰ représentent ensemble avec l'atome d'azote auquel ils sont reliés hétérocycloalkyle de 4 à 7 éléments, qui peut éventuellement être substitué une ou deux fois, de manière identique ou différente, avec : hydroxy, fluor, oxo, alkyle en C₁-C₃, fluoro-alkyle en C₁-C₃, cyclopropyle, cyclopropylméthyle, acétyle ou *tert*.-butoxycarbonyle,
ainsi que leurs diastéréomères, racémats, polymorphes et sels physiologiquement compatibles.

3. Composés de formule générale (I) selon les revendications 1 et 2, dans laquelle
A représente -NH- ou -N(méthyle)-,
X représente -N- ou -CH-,
n représente 0 ou 1,
R¹ représente un groupe -C(=O)NR⁷R⁸ ou-S(=O)₂NR⁷R⁸,
ou
oxazolyle ou oxadiazolyle, qui peuvent éventuellement être substitués une ou deux fois, de manière identique ou différente, avec alkyle en C₁-C₃,
R² représente hydrogène, fluor, chlore, méthyle ou méthoxy,
ou
R¹ et R² représentent ensemble un groupe *-S(=O)₂-NR⁸-CH₂-**, « * » signifiant le point de liaison de R¹ au cycle phényle contenu dans la formule (I) et « ** » signifiant un atome de carbone voisin de ce point de liaison de ce cycle phényle,
R³ représente méthyle,
R⁴ représente méthyle ou éthyle,
R⁵ représente hydrogène,
R⁶ représente alkyle en C₃-C₅ ou 2-méthoxyéthyle,
ou
représente méthyle, qui est substitué une fois avec phényle ou hétérocycloalkyle de 4 à 6 éléments, le phényle pouvant de son côté éventuellement être substitué une ou deux fois, de manière identique ou différente, avec : fluor, chlore, cyano, méthyle, méthoxy et
l'hétérocycloalkyle de 4 à 6 éléments pouvant de son côté éventuellement être substitué une fois avec méthyle,
ou
représente cycloalkyle en C₃-C₈ ou hétérocycloalkyle de 4 à 6 éléments, qui peuvent éventuellement être substitués une ou deux fois, de manière identique ou différente, avec alkyle en C₁-C₃ ou alcoxycarbonyle en C₁-C₄,
ou
représente phényle, qui peut éventuellement être substitué une ou deux fois, de manière identique ou différente, avec fluor, chlore, méthyle ou N-*tert*.-butoxycarbonylpipérazinyle,
R⁷ représente hydrogène,
ou
représente alkyle en C₁-C₄, qui peut éventuellement être substitué une fois avec -NR⁹R¹⁰ ou hétérocycloalkyle de 4 à 8 éléments,
l'hétérocycloalkyle de 4 à 8 éléments pouvant de son côté éventuellement être substitué une fois avec : oxo, alkyle en C₁-C₃, fluoro-alkyle en C₁-C₃, cyclopropyle ou cyclopropylméthyle,
ou
représente cycloalkyle en C₃-C₆, qui peut éventuellement être substitué une fois avec hydroxy, fluor ou -NR⁹R¹⁰,
ou
représente hétérocycloalkyle de 4 à 8 éléments qui peut éventuellement être substitué une ou deux fois, de manière identique ou différente, avec : oxo, alkyle en C₁-C₃, fluoro-alkyle en C₁-C₃, cyclopropyle ou cyclopropylméthyle,
R⁸ représente hydrogène, méthyle ou éthyle,
ou
R⁷ et R⁸ représentent ensemble avec l'atome d'azote auquel ils sont reliés hétérocycloalkyle de 4 à 6 éléments, ou hétérospirocycloalkyle en C₆-C₈, qui peuvent éventuellement être substitués, une ou deux fois, de manière identique ou différente, avec : fluor, oxo, alkyle en C₁-C₃, fluoro-alkyle en C₁-C₃, cyclopropyle ou cyclopropylméthyle,
R⁹ et R¹⁰ représentent indépendamment l'un de l'autre hydrogène ou alkyle en C₁-C₃ éventuellement substitué une fois avec hydroxy ou oxo ; trifluorométhyle,
ou
N-méthylpipéridinyle,
ou
R⁹ et R¹⁰ représentent ensemble avec l'atome d'azote auquel ils sont reliés hétérocycloalkyle de 4 à 7 éléments, qui peut éventuellement être substitué une ou deux fois, de manière identique ou différente, avec : fluor, oxo, alkyle en C₁-C₃, fluoro-alkyle en C₁-C₃, cyclopropyle ou cyclopropylméthyle,
ainsi que leurs diastéréomères, racémats, polymorphes et sels physiologiquement compatibles.

4. Composés de formule générale (I) selon les revendications 1 à 3, dans laquelle
A représente -NH- ou -N(méthyle)-,
X représente -N-,
n représente 0 ou 1,
R¹ représente un groupe -C(=O)NR⁷R⁸ ou-S(=O)₂NR⁷R⁸,
ou
oxazolyle ou oxadiazolyle, qui peuvent éventuellement être substitués une ou deux fois, de manière identique ou différente, avec alkyle en C₁-C₃,
R² représente hydrogène, fluor, chlore, méthyle, méthoxy, ou
R¹ et R² représentent ensemble un groupe *-S(=O)₂-NR⁸-CH₂-**, « * » signifiant le point de liaison de R¹ au cycle phényle contenu dans la formule (I) et « ** » signifiant un atome de carbone voisin de ce point de liaison de ce cycle phényle,
R³ représente méthyle,
R⁴ représente méthyle ou éthyle,
R⁵ représente hydrogène,
R⁶ représente alkyle en C₃-C₅ ou 2-méthoxyéthyle,
ou
représente méthyle, qui est substitué une fois avec phényle ou hétérocycloalkyle de 4 à 6 éléments,
le phényle pouvant de son côté éventuellement être substitué une ou deux fois, de manière identique ou différente, avec : fluor, chlore, cyano, méthyle, méthoxy et
l'hétérocycloalkyle de 4 à 6 éléments pouvant de son côté éventuellement être substitué une fois avec méthyle,
ou représente cycloalkyle en C₃-C₈ ou hétérocycloalkyle de 4 à 6 éléments, qui peuvent éventuellement être substitués une ou deux fois, de manière identique ou différente, avec alkyle en C₁-C₃ ou alcoxycarbonyle en C₁-C₄,
ou
représente phényle, qui peut éventuellement être substitué une ou deux fois, de manière identique ou différente, avec fluor, chlore, méthyle ou N-*tert*.-butoxycarbonylpipérazinyle,
R⁷ représente hydrogène,
ou
représente alkyle en C₁-C₄, qui peut éventuellement être substitué une fois avec -NR⁹R¹⁰ ou hétérocycloalkyle de 4 à 8 éléments,
l'hétérocycloalkyle de 4 à 8 éléments pouvant de son côté éventuellement être substitué une fois avec : oxo, alkyle en C₁-C₃, fluoro-alkyle en C₁-C₃, cyclopropyle ou cyclopropylméthyle,
ou
représente cycloalkyle en C₃-C₆, qui peut éventuellement être substitué une fois avec hydroxy, fluor ou -NR⁹R¹⁰,
ou
représente hétérocycloalkyle de 4 à 8 éléments qui peut éventuellement être substitué une ou deux fois, de manière identique ou différente, avec : oxo, alkyle en C₁-C₃, fluoro-alkyle en C₁-C₃, cyclopropyle ou cyclopropylméthyle,
R⁸ représente hydrogène, méthyle ou éthyle, ou
R⁷ et R⁸ représentent ensemble avec l'atome d'azote auquel ils sont reliés hétérocycloalkyle de 4 à 6 éléments, ou hétérospirocycloalkyle en C₆-C₈, qui peuvent éventuellement être substitués, une ou deux fois, de manière identique ou différente, avec : fluor, oxo, alkyle en C₁-C₃, fluoro-alkyle en C₁-C₃, cyclopropyle ou cyclopropylméthyle,
R⁹ et R¹⁰ représentent indépendamment l'un de l'autre hydrogène ou alkyle en C₁-C₃ éventuellement substitué une fois avec hydroxy ou oxo ; trifluorométhyle ou *N-*méthylpipéridinyle, ou
R⁹ et R¹⁰ représentent ensemble avec l'atome d'azote auquel ils sont reliés hétérocycloalkyle de 4 à 7 éléments, qui peut éventuellement être substitué une ou deux fois, de manière identique ou différente, avec : fluor, oxo, alkyle en C₁-C₃, fluoro-alkyle en C₁-C₃, cyclopropyle ou cyclopropylméthyle,
ainsi que leurs diastéréomères, racémats, polymorphes et sels physiologiquement compatibles.

5. Composés de formule générale (I) selon les revendications 1 à 3, dans laquelle
A représente -NH- ou -N(méthyle)-,
X représente -CH-,
n représente 0 ou 1,
R¹ représente un groupe -C(=O)NR⁷R⁸ ou-S(=O)₂NR⁷R⁸,
ou
oxazolyle ou oxadiazolyle, qui peuvent éventuellement être substitués une ou deux fois, de manière identique ou différente, avec alkyle en C₁-C₃,
R² représente hydrogène, fluor, chlore, méthyle, méthoxy, ou
R¹ et R² représentent ensemble un groupe *-S(=O)₂-NR⁸-CH₂-**, « * » signifiant le point de liaison de R¹ au cycle phényle contenu dans la formule (I) et « ** » signifiant un atome de carbone voisin de ce point de liaison de ce cycle phényle,
R³ représente méthyle,
R⁴ représente méthyle ou éthyle,
R⁵ représente hydrogène,
R⁶ représente alkyle en C₃-C₅ ou 2-méthoxyéthyle,
ou
représente méthyle, qui est substitué une fois avec phényle ou hétérocycloalkyle de 4 à 6 éléments, le phényle pouvant de son côté éventuellement être substitué une ou deux fois, de manière identique ou différente, avec : fluor, chlore, cyano, méthyle, méthoxy et l
'hétérocycloalkyle de 4 à 6 éléments pouvant de son côté éventuellement être substitué une fois avec méthyle,
ou représente cycloalkyle en C₃-C₈ ou hétérocycloalkyle de 4 à 6 éléments, qui peuvent éventuellement être substitués une ou deux fois, de manière identique ou différente, avec alkyle en C₁-C₃ ou alcoxycarbonyle en C₁-C₄,
ou
représente phényle, qui peut éventuellement être substitué une ou deux fois, de manière identique ou différente, avec fluor, chlore, méthyle ou N-*tert*.-butoxycarbonylpipérazinyle,
R⁷ représente hydrogène,
ou
représente alkyle en C₁-C₄, qui peut éventuellement être substitué une fois avec -NR⁹R¹⁰ ou hétérocycloalkyle de 4 à 8 éléments,
l'hétérocycloalkyle de 4 à 8 éléments pouvant de son côté éventuellement être substitué une fois avec : oxo, alkyle en C₁-C₃, fluoro-alkyle en C₁-C₃, cyclopropyle ou cyclopropylméthyle,
ou représente cycloalkyle en C₃-C₆, qui peut éventuellement être substitué une fois avec hydroxy, fluor ou -NR⁹R¹⁰,
ou
représente hétérocycloalkyle de 4 à 8 éléments qui peut éventuellement être substitué une ou deux fois, de manière identique ou différente, avec : oxo, alkyle en C₁-C₃, fluoro-alkyle en C₁-C₃, cyclopropyle ou cyclopropylméthyle,
R⁸ représente hydrogène, méthyle ou éthyle,
ou
R⁷ et R⁸ représentent ensemble avec l'atome d'azote auquel ils sont reliés hétérocycloalkyle de 4 à 6 éléments, ou hétérospirocycloalkyle en C₆-C₈, qui peuvent éventuellement être substitués, une ou deux fois, de manière identique ou différente, avec : fluor, oxo, alkyle en C₁-C₃, fluoro-alkyle en C₁-C₃, cyclopropyle ou cyclopropylméthyle,
R⁹ et R¹⁰ représentent indépendamment l'un de l'autre hydrogène ou alkyle en C₁-C₃ éventuellement substitué une fois avec hydroxy ou oxo ; trifluorométhyle ou *N-*méthylpipéridinyle,
ou
R⁹ et R¹⁰ représentent ensemble avec l'atome d'azote auquel ils sont reliés hétérocycloalkyle de 4 à 7 éléments, qui peut éventuellement être substitué une ou deux fois, de manière identique ou différente, avec : fluor, oxo, alkyle en C₁-C₃, fluoro-alkyle en C₁-C₃, cyclopropyle ou cyclopropylméthyle,
ainsi que leurs diastéréomères, racémats, polymorphes et sels physiologiquement compatibles.

6. Composés de formule générale (I) selon les revendications 1 à 3, dans laquelle
A représente -NH- ou -N(méthyle)-,
X représente -N- ou -CH-,
n représente 0 ou 1,
R¹ représente un groupe -C(=O)NR⁷R⁸ ou-S(=O)₂NR⁷R⁸,
ou
oxazolyle ou oxadiazolyle, qui peuvent éventuellement être substitués une ou deux fois avec méthyle,
R² représente hydrogène, méthyle ou méthoxy,
ou
R¹ et R² représentent ensemble un groupe *-S(=O)₂-NH-CH₂-**, « * » signifiant le point de liaison de R¹ au cycle phényle contenu dans la formule (I) et « ** » signifiant un atome de carbone voisin de ce point de liaison de ce cycle phényle,
R³ représente méthyle,
R⁴ représente méthyle,
R⁵ représente hydrogène,
R⁶ représente isopropyle, isobutyle ou 2-méthoxyéthyle,
ou
représente benzyle, dont la partie phényle est éventuellement substituée une ou deux fois, de manière identique ou différente, avec : fluor, méthoxy,
ou
représente cycloalkyle en C₅-C₇, qui peut éventuellement être substitué une ou deux fois avec méthyle,
ou
représente tétrahydrofuranyle, tétrahydropyranyle ou pipéridinyle ; le pipéridinyle pouvant éventuellement être substitué une fois avec méthyle ou *tert*.-butoxycarbonyle,
ou
représente phényle, qui peut éventuellement être substitué une fois avec fluor, méthyle ou N-*tert.-*butoxycarbonylpipérazinyle,
R⁷ représente hydrogène,
ou
représente alkyle en C₁-C₃, qui peut éventuellement être substitué une fois avec -NR⁹R¹⁰ ou *N*-méthylpipéridinyle,
ou
représente cyclopropyle ou cyclohexyle ; le cyclohexyle pouvant éventuellement être substitué une fois avec hydroxy ou -NR⁹R¹⁰,
ou
représente hétérocycloalkyle de 4 à 6 éléments qui peut éventuellement être substitué une fois avec méthyle,
R⁸ représente hydrogène, méthyle ou éthyle,
ou
R⁷ et R⁸ représentent ensemble avec l'atome d'azote auquel ils sont reliés hétérocycloalkyle de 4 à 6 éléments, qui peut éventuellement être substitué une ou deux fois avec fluor, ou qui peut éventuellement être substitué une fois avec méthyle, isopropyle, 2,2,2-trifluoroéthyle ou cyclopropylméthyle,
ou
représente 6-azaspiro[3.3]heptyle ou 2-oxa-6-azaspiro[3.3]heptyle,
R⁹ et R¹⁰ représentent indépendamment l'un de l'autre hydrogène, alkyle en C₁-C₃ ou *N-*méthylpipéridinyle,
ou
R⁹ et R¹⁰ représentent ensemble avec l'atome d'azote auquel ils sont reliés hétérocycloalkyle à 6 éléments, qui peut éventuellement être substitué une ou deux fois avec fluor, ou qui peut éventuellement être substitué une fois avec méthyle, 2,2,2-trifluoroéthyle, cyclopropyle ou cyclopropylméthyle,
ainsi que leurs diastéréomères, racémats, polymorphes et sels physiologiquement compatibles.

7. Composés de formule générale (I) selon les revendications 1 à 3 et 6, dans laquelle
A représente -NH- ou -N(méthyle)-,
X représente -N- ou -CH-,
n représente 0 ou 1,
R¹ représente un groupe -C(=O)NR⁷R⁸ ou-S(=O)₂NR⁷R⁸,
ou
représente 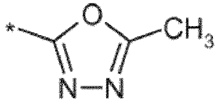 « * » signifiant le point de liaison au reste de la molécule,
R² représente hydrogène, méthyle ou méthoxy,
ou
R¹ et R² représentent ensemble avec le cycle phényle auquel ils sont reliés 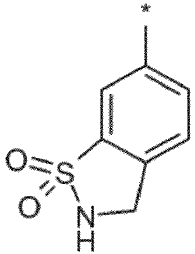
« * » signifiant le point de liaison au reste de la molécule,
R³ représente méthyle,
R⁴ représente méthyle,
R⁵ représente hydrogène,
R⁶ représente isopropyle, isobutyle, 2-méthoxyéthyle, benzyle, 4-méthoxybenzyle, 2,6-difluorobenzyle, cyclopentyle, cyclohexyle, cycloheptyle, tétrahydropyran-4-yle, phényle, 3-méthylphényle ou 4-fluorophényle,
ou représente 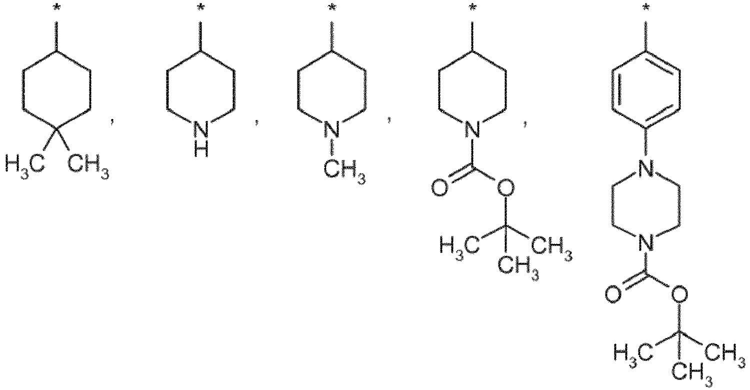 « * » signifiant à chaque fois le point de liaison au reste de la molécule,
R⁷ représente hydrogène, méthyle, éthyle, isopropyle ou cyclopropyle
ou représente 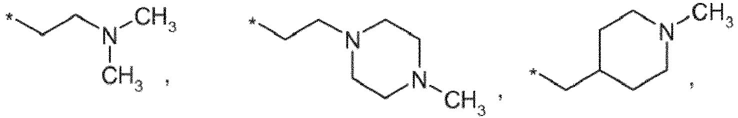 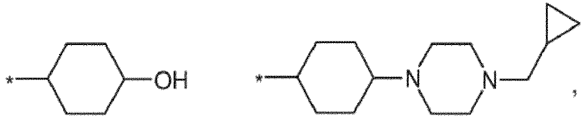 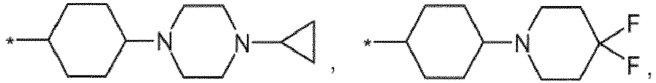 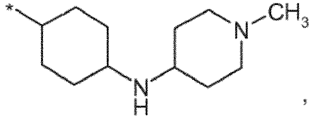 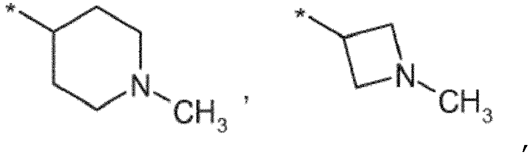 « * » signifiant à chaque fois le point de liaison au reste de la molécule,
R⁸ représente hydrogène, méthyle ou éthyle,
ou R⁷ et R⁸ représentent ensemble avec l'atome d'azote auquel ils sont reliés 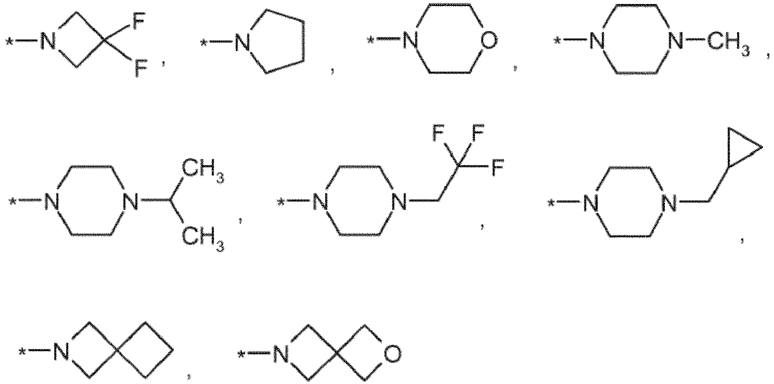
« * » signifiant à chaque fois le point de liaison au reste de la molécule, ainsi que leurs diastéréomères, racémats, polymorphes et sels physiologiquement compatibles.

8. Composés de formule générale (I) selon les revendications 1 à 7,
(3R)-4-cyclopentyl-1,3-diméthyl-6-({3-[(4-méthylpipérazin-1-yl)carbonyl]phényl}amino)-3,4-dihydropyrido[2,3-b]pyrazin-2(1H)-one ;
3-{[(3R)-4-cyclopentyl-1,3-diméthyl-2-oxo-1,2,3,4-tétrahydropyrido[2,3-b]pyrazin-6-yl]amino}-*N*-*(*1-méthylpipéridin-4-yl)benzamide ;
3-{[(3R)-4-cyclopentyl-1,3-diméthyl-2-oxo-1,2,3,4-tétrahydropyrido[2,3-b]pyrazin-6-yl]amino}-*N*-(1-méthylazétidin-3-yl)benzamide ;
3-{[(3R)-4-cyclopentyl-1,3-diméthyl-2-oxo-1,2,3,4-tétrahydropyrido[2,3-b]pyrazin-6-yl]amino}-*N*-[2-(diméthylamino)éthyl]benzamide ;
3-{[(3*R*)-4-cyclopentyl-1,3-diméthyl-2-oxo-1,2,3,4-tétrahydropyrido[2,3-b]pyrazin-6-yl]amino}-*N-*{*trans*-4-[4-(cyclopropylméthyl)pipérazin-1-yl]cyclohexyl}benzamide ;
3-{[(3R)-1,3-diméthyl-2-oxo-4-(tétrahydro-2H-pyran-4-yl)-1,2,3,4-tétrahydropyrido[2,3-b]pyrazin-6-yl]amino}-*N,N-*diméthylbenzènesulfonamide ;
3-{[(3R)-1,3-diméthyl-2-oxo-4-(tétrahydro-2H-pyran-4-yl)-1,2,3,4-tétrahydroquinoxalin-6-yl]amino}-*N,N*-diméthylbenzènesulfonamide ;
(3R)-1,3-diméthyl-6-{[3-(morpholin-4-ylsulfonyl)phényl]amino}-4-(tétrahydro-2H-pyran-4-yl)-3,4-dihydroquinoxalin-2(1*H)*-one ;
(3R)-1,3-diméthyl-6-{[3-(morpholin-4-ylsulfonyl)phényl]amino}-4-(tétrahydro-2H-pyran-yl)-3,4-dihydropyrido[2,3-b]pyrazin-2(1H)-one ;
3-{[(3R)-4-(4-méthoxybenzyl)-1,3-diméthyl-2-oxo-1,2,3,4-tétrahydroquinoxalin-6-yl]amino}-*N,N-*diméthylbenzènesulfonamide ;
(3R)-4-(4-méthoxybenzyl)-1,3-diméthyl-6-{[3-(morpholin-4-ylsulfonyl)phényl]amino}-3,4-dihydroquinoxalin-2(1H)-one ;
(3R)-1,3-diméthyl-6-({3-[(4-méthylpipérazin-1-yl)sulfonyl]phényl}amino)-4-(tétrahydro-2H-pyran-4-yl)-3,4-dihydropyrido[2,3-b]pyrazin-2(1H)-one ;
3-{[(3R)-1,3-diméthyl-2-oxo-4-(tétrahydro-2H-pyran-4-yl)-1,2,3,4-tétrahydropyrido[2,3-b]pyrazin-6-yl]amino}-*N*-(1-méthylpipéridin-4-yl)benzamide ;
*N*-{*trans*-4-[4-(cyclopropylméthyl)pipérazin-1-yl]cyclohexyl}-3-{[(3R)-1,3-diméthyl-2-oxo-4-(tétrahydro-2H-pyran-4-yl)-1,2,3,4-tétrahydropyrido[2,3-b]pyrazin-6-yl]amino}benzamide ;
(3*R*)-1,3-diméthyl-6-({3-[(4-méthylpipérazin-1-yl)carbonyl]phényl}amino)-4-(tétrahydro-2H-pyran-4-yl)-3,4-dihydropyrido[2,3-b]pyrazin-2(1H)-one ;
3-{[(3R)-1,3-diméthyl-2-oxo-4-(tétrahydro-2H-pyran-4-yl)-1,2,3,4-tétrahydropyrido[2,3-b]pyrazin-6-yl]amino}-4-méthoxy-*N*-(1-méthylpipéridin-4-yl)benzamide ;
(3R)-6-({2-méthoxy-5-[(4-méthylpipérazin-1-yl)carbonyl]phényl}amino)-1,3-diméthyl-4-(tétrahydro-2H-pyran-4-yl)-3,4-dihydropyrido[2,3-b]pyrazin-2(1H)-one ;
*N*-{trans-4-[4-(cyclopropylméthyl)pipérazin-1-yl]cyclohexyl}-3-{[(3R)-1,3-diméthyl-2-oxo-4-(tétrahydro-2H-pyran-4-yl)-1,2,3,4-tétrahydropyrido[2,3-b]pyrazin-6-yl]amino}-4-méthoxybenzamide ;
*N*-[2-(diméthylamino)éthyl]-3-{[(3R)-1,3-diméthyl-2-oxo-4-(tétrahydro-2H-pyran-4-yl)-1,2,3,4-tétrahydropyrido[2,3-b]pyrazin-6-yl]amino}-4-méthoxybenzamide ;
3-{[(3R)-1,3-diméthyl-2-oxo-4-(tétrahydro-2H-pyran-4-yl)-1,2,3,4-tétrahydropyrido[2,3-b]pyrazin-6-yl]amino}-*N*-(1-méthylpipéridin-4-yl)benzènesulfonamide ;
N-[2-(diméthylamino)éthyl]-3-{[(3R)-1,3-diméthyl-2-oxo-4-(tétrahydro-2H-pyran-4-yl)-1,2,3,4-tétrahydropyrido[2,3-b)]pyrazin-6-yl]amino}benzènesulfonamide ;
*N*-{trans-4-[4-(cyclopropylméthyl)pipérazin-1-yl]cyclohexyl}-3-{[4-(2,6-difluorobenzyl)-1,3-diméthyl-2-oxo-1,2,3,4-tétrahydroquinoxalin-6-yl]amino}benzamide ;
3-{[(3R)-1,3-diméthyl-2-oxo-4-(tétrahydro-2H-pyran-4-yl)-1,2,3,4-tétrahydropyrido[2,3-b]pyrazin-6-yl]amino}-*N*-éthylbenzènesulfonamide ;
(3*R*)-1,3-diméthyl-4-(1-méthylpipéridin-4-yl)-6-{[3-(pyrrolidin-1-ylsulfonyl)phényl]amino}-3,4-dihydropyrido[2,3-b]pyrazin-2(1H)-one ;
(3R)-6-{[2-méthoxy-5-(morpholin-4-ylsulfonyl)phényl]amino}-1,3-diméthyl-4-(1-méthylpipéridin-4-yl)-3,4-dihydropyrido[2,3-b]pyrazin-2(1H)-one ;
*N*-cyclopropyl-3-{[(3R)-1,3-diméthyl-2-oxo-4-(tétrahydro-2H-pyran-4-yl)-1,2,3,4-tétrahydropyrido[2,3-b]pyrazin-6-yl]amino}benzènesulfonamide ;
(3*R*)-1,3-diméthyl-6-{[3-(pyrrolidin-1-ylsulfonyl)phényl]amino}-4-(tétrahydro-2H-pyran-yl)-3,4-dihydropyrido[2,3-b]pyrazin-2(1H)-one ;
(3R)-6-{[2-méthoxy-5-(morpholin-4-ylsulfonyl)phényl]amino}-1,3-diméthyl-4-(tétrahydro-2H-pyran-4-yl)-3,4-dihydropyrido[2,3-b]pyrazin-2(1H)-one ;
(3R)-6-({3-[(3,3-difluoroazétidin-1-yl)sulfonyl]phényl}amino)-1,3-diméthyl-4-(1-méthylpipéridin-4-yl)-3,4-dihydropyrido[2,3-b]pyrazin-2(1H)-one ;
(3R)-6-{[2-méthoxy-5-(2-oxa-6-azaspiro[3,3]hept-6-ylsulfonyl)phényl]amino}-1,3-diméthyl-4-(tétrahydro-2H-pyran-4-yl)-3,4-dihydropyrido[2,3-b]pyrazin-2(1H)-one ;
(3R)-6-({3-[(3,3-difluoroazétidin-1-yl)sulfonyl]phényl}amino)-1,3-diméthyl-4-(tétrahydro-2H-pyran-4-yl)-3,4-dihydropyrido[2,3-b]pyrazin-2(1H)-one ;
3-{[(3R)-1,3-diméthyl-2-oxo-4-(tétrahydro-2H-pyran-4-yl)-1,2,3,4-tétrahydropyrido[2,3-b]pyrazin-6-yl]amino}-*N,N-*diéthylbenzènesulfonamide ;
3-{[(3R)-4-benzyl-1,3-diméthyl-2-oxo-1,2,3,4-tétrahydropyrido[2,3-b]pyrazin-6-yl]amino}-*N*-(1-méthylpipéridin-4-yl)benzènesulfonamide ;
*N*-{trans-4-[4-(cyclopropylméthyl)pipérazin-1-yl]cyclohexyl}-3-{[(3R)-4-isopropyl-1,3-diméthyl-2-oxo-1,2,3,4-tétrahydropyrido[2,3-b]pyrazin-6-yl]amino}benzènesulfonamide ;
*N*-{trans-4-[4-(cyclopropylméthyl)pipérazin-1-yl]cyclohexyl}-3-{[(3R)-1,3-diméthyl-2-oxo-4-(tétrahydro-2H-pyran-4-yl)-1,2,3,4-tétrahydropyrido[2,3-b]pyrazin-6-yl]amino}benzènesulfonamide ;
*N*-{trans-4-[4-(cyclopropylméthyl)pipérazin-1-yl]cyclohexyl}-3-{[(3S)-1,3-diméthyl-2-oxo-4-(tétrahydro-2H-pyran-4-yl)-1,2,3,4-tétrahydropyrido[2,3-b]pyrazin-6-yl]amino}benzènesulfonamide ;
(3R)-1,3-diméthyl-4-(tétrahydro-2H-pyran-4-yl)-6-[(3-{[4-(2,2,2-trifluoroéthyl)pipérazin-1-yl]sulfonyl}phényl)amino]-3,4-dihydropyrido[2,3-b]pyrazin-2(1H)-one ;
3-{[(3R)-4-benzyl-1,3-diméthyl-2-oxo-1,2,3,4-tétrahydropyrido[2,3-b]pyrazin-6-yl]amino}-*N-*{*trans*-4-[4-(cyclopropyl-méthyl)pipérazin-1-yl]cyclohexyl}benzènesulfonamide ;
3-{[(3R)-1,3-diméthyl-2-oxo-4-(tétrahydro-2H-pyran-4-yl)-1,2,3,4-tétrahydropyrido[2,3-b]pyrazin-6-yl]amino}- N-méthylbenzènesulfonamide ;
(3R)-6-({2-méthoxy-5-[(4-méthylpipérazin-1-yl)sulfonyl]phényl}amino)-1,3-diméthyl-4-(tétrahydro-2H-pyran-4-yl)-3,4-dihydropyrido[2,3-b]pyrazin-2(1H)-one ;
3-{[(3R)-1,3-diméthyl-4-(1-méthylpipéridin-4-yl)-2-oxo-1,2,3,4-tétra-hydropyrido[2,3-b]pyrazin-6-yl]amino}-*N,N*-diméthylbenzènesulfonamide ;
3-{[(3R)-1,3-diméthyl-2-oxo-4-(tétrahydro-2H-pyran-4-yl)-1,2,3,4-tétrahydropyrido[2,3-b]pyrazin-6-yl]amino}-*N-*isopropylbenzènesulfonamide ;
(3R)-4-isopropyl-1,3-diméthyl-6-({3-[(4-méthylpipérazin-1-yl)sulfonyl]phényl}amino)-3,4-dihydropyrido[2,3-b]pyrazin-2(1H)-one ;
3-{[(3R)-1,3-diméthyl-2-oxo-4-(tétrahydro-2H-pyran-4-yl)-1,2,3,4-tétrahydropyrido[2,3-b]pyrazin-6-yl]amino}-*N*-[(1-méthylpipéridin-4-yl)méthyl]benzènesulfonamide ;
*N*-{trans-4-[4-(cyclopropylméthyl)pipérazin-1-yl]cyclohexyl}-3-{[(3R)-4-isobutyl-1,3-diméthyl-2-oxo-1,2,3,4-tétrahydropyrido[2,3-b]pyrazin-6-yl]amino}benzènesulfonamide ;
(3R)-6-({2-méthoxy-5-[(4-méthylpipérazin-1-yl)sulfonyl]phényl}amino)-1,3-diméthyl-4-(1-méthylpipéridin-4-yl)-3,4-dihydropyrido[2,3-b]pyrazin-2(1H)-one ;
(3R)-1,3-diméthyl-4-(1-méthylpipéridin-4-yl)-6-{[3-(morpholin-4-ylsulfonyl)phényl]amino}-3,4-dihydropyrido[2,3-b]pyrazin-2(1H)-one ;
3-{[(3R)-4-cycloheptyl-1,3-diméthyl-2-oxo-1,2,3,4-tétrahydropyrido[2,3-b]pyrazin-6-yl]amino}-*N*-(1-méthyl-pipéridin-4-yl)benzène-sulfonamide ;
4-(2-méthoxyéthyl)-1,3-diméthyl-6-{[3-(morpholin-4-ylsulfonyl)phényl]amino}-3,4-dihydropyrido[2,3-b]pyrazin-2(1H)-one ;
(3*R*)-1,3-diméthyl-6-({3-[(4-méthylpipérazin-1-yl)sulfonyl]phényl}amino)-4-(1-méthylpipéridin-4-yl)-3,4-dihydropyrido[2,3-b]pyrazin-2(1H)-one ;
3-{[(3R)-4-isopropyl-1,3-diméthyl-2-oxo-1,2,3,4-tétrahydropyrido[2,3-b]pyrazin-6-yl]amino}-*N*-(1-méthylpipéridin-4-yl)benzènesulfonamide ;
4-[(3R)-6-{[3-({trans-4-[4-(cyclopropyl-méthyl)pipérazin-1-yl]cyclohexyl}sulfamoyl)phényl]amino}-1,3-diméthyl-2-oxo-2,3-dihydropyrido[2,3-b]pyrazin-4(1H)-yl]pipéridine-1-carboxylate de tert-butyle ;
(2-méthoxyéthyl)-1,3-diméthyl-6-({3-[(4-méthylpipérazin-1-yl)sulfonyl]phényl}amino)-3,4-dihydropyrido[2,3-b]pyrazin-2(1H)-one ;
3-{[(3)-1,3-diméthyl-2-oxo-4-(tétrahydro-2H-pyran-4-yl)-1,2,3,4-tétrahydropyrido[2,3-b]pyrazin-6-yl](méthyl)amino}-*N,N*-diméthylbenzènesulfonamide ;
*N*-[2-(diméthylamino)éthyl]-3-{[(3R)-4-isopropyl-1,3-diméthyl-2-oxo-1,2,3,4-tétrahydro-pyrido[2,3-b]pyrazin-6-yl]amino}benzènesulfonamide ;
(3R)-6-[(1,1-dioxido-2,3-dihydro-1,2-benzothiazol-6-yl)amino]-1,3-diméthyl-4-(tétrahydro-2H-pyran-4-yl)-3,4-dihydropyrido[2,3-b]pyrazin-2(1H)-one ;
4-[(3R)-1,3-diméthyl-6-({3-[(1-méthylpipéridin-4-yl)sulfamoyl]phényl}amino)-oxo-2,3-dihydro-pyrido[2,3-b]pyrazin-4(1H)-yl]pipéridine-1-carboxylate de tert-butyle ;
3-{[(3R)-1,3-diméthyl-2-oxo-4-(tétrahydro-2H-pyran-4-yl)-1,2,3,4-tétrahydropyrido[2,3-b]pyrazin-6-yl]amino}benzènesulfonamide ;
4-[(3R)-6-[(3-{[2-(diméthylamino)éthyl]sulfamoyl}phényl)amino]-1,3-diméthyl-2-oxo-2,3-dihydropyrido[2,3-b]pyrazin-4(1H)-yl]pipéridine-1-carboxylate de tert-butyle ;
4-[(3R)-6-{[3-(2-azaspiro[3.3]hept-2-ylsulfonyl)phényl]amino}-1,3-diméthyl-2-oxo-2,3-dihydropyrido[2,3-b]pyrazin-4(1H)-yl]pipéridine-1-carboxylate de tert-butyle ;
4-[(3R)-6-{[3-(diméthylsulfamoyl)phényl]amino}-1,3-diméthyl-2-oxo-2,3-dihydropyrido[2,3-b]pyrazin-4(1H)-yl]pipéridine-1-carboxylate de tert-butyle ;
4-[(3R)-1,3-diméthyl-6-({3-[(4-méthylpipérazin-1-yl)sulfonyl]phényl}amino)-2-oxo-2,3-dihydropyrido[2,3-b]pyrazin-4(1H)-yl]pipéridine-1-carboxylate de tert-butyle ;
5-{[(3R)-1,3-diméthyl-2-oxo-4-(tétrahydro-2H-pyran-4-yl)-1,2,3,4-tétrahydropyrido[2,3-b]pyrazin-6-yl]amino}-2-méthylbenzènesulfonamide ;
1,3-diméthyl-4-(3-méthylphényl)-6-{[3-(morpholin-4-ylsulfonyl)phényl]amino}-3,4-dihydropyrido[2,3-b]pyrazin-2(1H)-one ;
3-{[1,3-diméthyl-4-(3-méthylphényl)-2-oxo-1,2,3,4-tétrahydro-pyrido[2,3-b]pyrazin-6-yl]amino}-*N*-(1-méthylpipéridin-4-yl)benzènesulfonamide ;
4-[(3R)-1,3-diméthyl-2-oxo-6-[(3-{[4-(2,2,2-trifluoroéthyl)pipérazin-1-yl]sulfonyl}phényl)amino]-2,3-dihydropyrido[2,3-b]pyrazin-4(1H)-yl]pipéridine-1-carboxylate de tert-butyle ;
4-{4-[1,3-diméthyl-6-({3-[(4-méthylpipérazin-1-yl)sulfonyl]phényl}amino)-2-oxo-2,3-dihydropyrido[2,3-b]pyrazin-4(1H)-yl]phényl}pipérazine-1-carboxylate de tert-butyle ;
4-[(2R)-7-{[3-(diméthylsulfamoyl)phényl]amino}-2,4-diméthyl-3-oxo-3,4-dihydro-quinoxalin-1(2H)-yl]pipéridine-1-carboxylate de tert-butyle ;
3-{[(3R)-4-benzyl-1,3-diméthyl-2-oxo-1,2,3,4-tétrahydroquinoxalin-6-yl]amino}-*N,N-*diméthylbenzènesulfonamide ;
4-[(2R)-7-{[3-(diméthylsulfamoyl)phényl](méthyl)amino}-2,4-diméthyl-3-oxo-dihydroquinoxalin-1(2H)-yl]pipéridine-1-carboxylate de tert-butyle ;
(3R)-1,3-diméthyl-6-({3-[(4-méthylpipérazin-1-yl)sulfonyl]phényl}amino)-4-(tétrahydro-2H-pyran-4-yl)-3,4-dihydroquinoxalin-2(1H)-one ;
3-{[(3R)-4-benzyl-1,3-diméthyl-2-oxo-1,2,3,4-tétrahydropyrido[2,3-b]pyrazin-6-yl]amino}-*N-*{trans-4-[4-(cyclopropyl-méthyl)pipérazin-1-yl]cyclohexyl}benzamide ;
3-{[(3R)-4-benzyl-1,3-diméthyl-2-oxo-1,2,3,4-tétrahydropyrido[2,3-b]pyrazin-6-yl]amino}-*N*-(1-méthylpipéridin-4-yl)benzamide ;
3-{[(3R)-4-benzyl-1,3-diméthyl-2-oxo-1,2,3,4-tétrahydropyrido[2,3-b]pyrazin-6-yl]amino}-N-[2-(4-méthylpipérazin-1-yl)éthyl]benzamide ;
(3R)-4-benzyl-1,3-diméthyl-6-({3-[(4-méthylpipérazin-1-yl)carbonyl]phényl}amino)-3,4-dihydropyrido[2,3-b]pyrazin-2(1H)-one ;
(3R)-4-benzyl-6-({3-[(4-isopropylpipérazin-1-yl)carbonyl]phényl}amino)-1,3-diméthyl-dihydropyrido[2,3-b]pyrazin-2(1H)-one ;
(3R)-4-isopropyl-1,3-diméthyl-6-({3-[(4-méthylpipérazin-1-yl)carbonyl]phényl}amino)-3,4-dihydropyrido[2,3-b]pyrazin-2(1H)-one ;
4-[(3R)-1,3-diméthyl-6-({3-[(4-méthylpipérazin-1-yl)carbonyl]phényl}amino)-oxo-2,3-dihydro-pyrido[2,3-b]pyrazin-4(1H)-yl]pipéridine-1-carboxylate de tert-butyle ;
*N*-{trans-4-[4-(cyclopropylméthyl)pipérazin-1-yl]cyclohexyl}-3-{[(3R)-4-isopropyl-1,3-diméthyl-2-oxo-1,2,3,4-tétrahydropyrido[2,3-b]pyrazin-6-yl]amino}benzamide ;
3-{[(3R)-1,3-diméthyl-2-oxo-4-(tétrahydro-2H-pyran-4-yl)-1,2,3,4-tétrahydropyrido[2,3-b]pyrazin-6-yl]amino}-*N*-{4-[(1-méthylpipéridin-4-yl)amino]cyclohexyl}benzamide ;
5-{[(3R)-1,3-diméthyl-2-oxo-4-(tétrahydro-2H-pyran-4-yl)-1,2,3,4-tétrahydropyrido[2,3-b]pyrazin-6-yl]amino}-2-méthoxy-*N*-(1-méthylpipéridin-4-yl)benzamide ;
*N*-{trans-4-[4-(cyclopropylméthyl)pipérazin-1-yl]cyclohexyl}-5-{[(3R)-1,3-diméthyl-2-oxo-4-(tétrahydro-2H-pyran-4-yl)-1,2,3,4-tétrahydropyrido[2,3-b]pyrazin-6-yl]amino}-2-méthoxybenzamide ;
(3R)-6-({4-méthoxy-3-[(4-méthylpipérazin-1-yl)carbonyl]phényl}amino)-1,3-diméthyl-4-(tétrahydro-2H-pyran-4-yl)-3,4-dihydropyrido[2,3-b]pyrazin-2(1H)-one ;
3-{[(3R)-4-cyclohexyl-1,3-diméthyl-2-oxo-1,2,3,4-tétrahydro-pyrido[2,3-b]pyrazin-6-yl]amino}-*N*-[2-(4-méthylpipérazin-1-yl)éthyl]benzamide ;
(3R)-4-cyclohexyl-6-({3-[(4-isopropylpipérazin-1-yl)carbonyl]phényl}amino)-1,3-diméthyl-3,4-dihydropyrido[2,3-b]pyrazin-2(1H)-one ;
3-{[(3R)-4-cyclohexyl-1,3-diméthyl-2-oxo-1,2,3,4-tétrahydro-pyrido[2,3-b]pyrazin-6-yl]amino}-N-(4-hydroxycyclohexyl)benzamide ;
*N*-{trans-4-[4-(cyclopropylméthyl)pipérazin-1-yl]cyclohexyl}-3-{[(3R)-1,3-diméthyl-2-oxo-4-(tétrahydro-2H-pyran-4-yl)-1,2,3,4-tétrahydropyrido[2,3-b]pyrazin-6-yl]amino}-4-méthoxybenzamide ;
(3R)-6-[(3-{[4-(cyclopropylméthyl)pipérazin-1-yl]carbonyl}phényl)amino]-4-isopropyl-1,3-diméthyl-3,4-dihydropyrido[2,3-b]pyrazin-2(1H)-one ;
*N*-[4-(4,4-difluoropipéridin-1-yl)cyclohexyl]-5-{[(3R)-1,3-diméthyl-2-oxo-4-(tétrahydro-2H-pyran-4-yl)-1,2,3,4-tétrahydro-pyrido[2,3-b]pyrazin-6-yl]amino}-2-méthoxy-benzamide ;
*N*-[cis-4-(4-cyclopropylpipérazin-1-yl)cyclohexyl]-3-{[(3R)-1,3-diméthyl-2-oxo-4-(tétrahydro-2H-pyran-4-yl)-1,2,3,4-tétrahydro-pyrido[2,3-b]pyrazin-6-yl]amino}-4-méthoxy-benzamide ;
*N*-{trans-4-[4-(cyclopropylméthyl)pipérazin-1-yl]cyclohexyl}-3-{[(3R)-1,3-diméthyl-2-oxo-4-(tétrahydro-2H-pyran-4-yl)-1,2,3,4-tétrahydropyrido[2,3-b]pyrazin-6-yl]amino}-2-méthoxybenzamide ;
(3R)-6-({2-méthoxy-3-[(4-méthylpipérazin-1-yl)carbonyl]phényl}amino)-1,3-diméthyl-4-(tétrahydro-2H-pyran-4-yl)-3,4-dihydropyrido[2,3-b]pyrazin-2(1H)-one ;
*N*-{trans-4-[4-(cyclopropylméthyl)pipérazin-1-yl]cyclohexyl}-3-{[(3R)-4-(4-fluorophényl)-1,3-diméthyl-2-oxo-1,2,3,4-tétrahydropyrido[2,3-b]pyrazin-6-yl]amino}benzamide ;
3-{[(3R)-4-isopropyl-1,3-diméthyl-2-oxo-1,2,3,4-tétrahydropyrido[2,3-b]pyrazin-6-yl]amino}-*N*-(1-méthylpipéridin-4-yl)benzamide ;
3-{[(3R)-4-cyclohexyl-1,3-diméthyl-2-oxo-1,2,3,4-tétrahydropyrido[2,3-b]pyrazin-6-yl]amino}-*N*-(1-méthylpipéridin-4-yl)benzamide ;
3-{[(3R)-4-(4,4-diméthylcyclohexyl)-1,3-diméthyl-2-oxo-1,2,3,4-tétrahydropyrido[2,3-b]pyrazin-6-yl]amino}-*N*-(1-méthylpipéridin-4-yl)benzamide ;
*N*-[2-(diméthylamino)éthyl]-3-{[(3R)-4-isopropyl-1,3-diméthyl-2-oxo-1,2,3,4-tétrahydropyrido[2,3-b]pyrazin-6-yl]amino}benzamide ;
3-{[4-(2-méthoxyéthyl)-1,3-diméthyl-2-oxo-1,2,3,4-tétrahydropyrido[2,3-b]pyrazin-6-yl]amino}-*N*-(1-méthylpipéridin-4-yl)benzamide ;
3-{[(3R)-4-cyclohexyl-1,3-diméthyl-2-oxo-1,2,3,4-tétrahydropyrido[2,3-b]pyrazin-6-yl]amino}-*N-*{trans-4-[4-(cyclopropylméthyl)pipérazin-1-yl]cyclohexyl}benzamide ;
4-[(3R)-1,3-diméthyl-6-({3-[(1-méthylpipéridin-4-yl)carbamoyl]phényl}amino)-oxo-2,3-dihydropyrido[2,3-b]pyrazin-4(1H)-yl]pipéridine-1-carboxylate de tert-butyle ;
*N*-{trans-4-[4-(cyclopropylméthyl)pipérazin-1-yl]cyclohexyl}-3-{[(3R)-4-(2-méthoxyéthyl)-1,3-diméthyl-2-oxo-1,2,3,4-tétrahydropyrido[2,3-b]pyrazin-6-yl]amino}benzamide ;
4-[(3R)-6-{[3-({trans-4-[4-(cyclopropyl-méthyl)pipérazin-1-yl]cyclohexyl}carbamoyl)phényl]amino}-1,3-diméthyl-2-oxo-2,3-dihydropyrido[2,3-b]pyrazin-4(1H)-yl]pipéridine-1-carboxylate de tert-butyle ;
3-{[(3R)-1,3-diméthyl-2-oxo-4-(tétrahydro-2H-pyran-4-yl)-1,2,3,4-tétrahydropyrido[2,3-b]pyrazin-6-yl]amino}-2-méthoxy-*N*-(1-méthyl-pipéridin-4-yl)benzamide ;
3-[(1,3-diméthyl-2-oxo-4-phényl-1,2,3,4-tétrahydropyrido[2,3-b]pyrazin-6-yl)amino]-*N*-(1-méthylpipéridin-4-yl)benzamide ;
4-[(2R)-7-{[3-(diméthylcarbamoyl)phényl]amino}-2,4-diméthyl-3-oxo-3,4-dihydroquinoxalin-1(2H)-yl]pipéridine-1-carboxylate de tert-butyle ;
(3*R*)-1,3-diméthyl-6-({3-[(4-méthylpipérazin-1-yl)carbonyl]phényl}amino)-4-(tétrahydro-2H-pyran-4-yl)-3,4-dihydroquinoxalin-2(1H)-one ;
3-{[(3R)-4-benzyl-1,3-diméthyl-2-oxo-1,2,3,4-tétrahydroquinoxalin-6-yl]amino}-*N,N-*diméthylbenzamide ;
*N*-{*trans*-4-[4-(cyclopropylméthyl)pipérazin-1-yl]cyclohexyl}-3-{[(3*R*)-1,3-diméthyl-2-oxo-4-(tétrahydro-2H-pyran-4-yl)-1,2,3,4-tétrahydroquinoxalin-6-yl]amino}benzamide ;
3-{[(3*R*)-1,3-diméthyl-2-oxo-4-(tétrahydro-2H-pyran-4-yl)-1,2,3,4-tétrahydroquinoxalin-6-yl]amino}-*N*-(1-méthylpipéridin-4-yl)benzamide ;
3-[(4-benzyl-1,3-diméthyl-2-oxo-1,2,3,4-tétrahydroquinoxalin-6-yl)(méthyl)amino]-N,N-diméthylbenzamide ;
(3*R*)-4-(2-méthoxyéthyl)-1,3-diméthyl-6-{[3-(morpholin-4-ylsulfonyl)phényl]amino}-3,4-dihydropyrido[2,3-b]pyrazin-2(1H)-one ;
(3*S*)-4-(2-méthoxyéthyl)-1,3-diméthyl-6-{[3-(morpholin-4-ylsulfonyl)phényl]amino}-3,4-dihydropyrido[2,3-b]pyrazin-2(1H)-one ;
(3*R*)-4-(2-méthoxyéthyl)-1,3-diméthyl-6-({3-[(4-méthylpipérazin-1-yl)sulfonyl]phényl}amino)-3,4-dihydropyrido[2,3-b]pyrazin-2(1H)-one ;
(3*R*)-4-(4-fluorophényl)-1,3-diméthyl-6-{[3-(morpholin-4-ylsulfonyl)phényl]amino}-3,4-dihydropyrido[2,3-b]pyrazin-2(1H)-one ;
3-{[(3*R*)-4-(4-fluorophényl)-1,3-diméthyl-2-oxo-1,2,3,4-tétrahydropyrido[2,3-b]pyrazin-6-yl]amino}-*N*-(1-méthylpipéridin-4-yl)benzamide ;
3-{[(3*S*)-4-(4-fluorophényl)-1,3-diméthyl-2-oxo-1,2,3,4-tétrahydropyrido[2,3-b]pyrazin-6-yl]amino}-*N*-(1-méthylpipéridin-4-yl)benzamide ;
(3*R*)-4-(2-méthoxyéthyl)-1,3-diméthyl-6-({3-[(4-méthylpipérazin-1-yl)carbonyl]-phényl}amino)-3,4-dihydropyrido[2,3-b]pyrazin-2(1H)-one ;
(3*S*)-4-(2-méthoxyéthyl)-1,3-diméthyl-6-({3-[(4-méthylpipérazin-1-yl)carbonyl]-phényl}amino)-3,4-dihydropyrido[2,3-b]pyrazin-2(1H)-one ;
3-{[(3R)-4-(2-méthoxy-éthyl)-1,3-diméthyl-2-oxo-1,2,3,4-tétrahydropyrido[2,3-b]pyrazin-6-yl]amino}-*N*-(1-méthylpipéridin-4-yl)benzamide ;
3-{[(3*S*)-4-(2-méthoxy-éthyl)-1,3-diméthyl-2-oxo-1,2,3,4-tétrahydropyrido[2,3-b]pyrazin-6-yl]amino}-*N*-(1-méthylpipéridin-4-yl)benzamide ;
3-{[(3*R*)-1,3-diméthyl-2-oxo-4-phényl-1,2,3,4-tétrahydropyrido[2,3-b]pyrazin-6-yl]amino}-*N*-(1-méthylpipéridin-4-yl)benzamide ;
(3*R*)-6-{[3-(2-azaspiro[3.3]hept-2-ylsulfonyl)phényl]amino}-1,3-diméthyl-4-(pipéridin-4-yl)-3,4-dihydropyrido[2,3-b]pyrazin-2(1H)-one ;
3-{[(3*R*)-1,3-diméthyl-2-oxo-4-(pipéridin-4-yl)-1,2,3,4-tétrahydropyrido[2,3-b]pyrazin-6-yl]amino}-*N,N*-diméthylbenzènesulfonamide ;
3-{[(3*R*)-1,3-diméthyl-2-oxo-4-(pipéridin-4-yl)-1,2,3,4-tétrahydropyrido[2,3-b]pyrazin-6-yl]amino}-*N*-(1-méthylpipéridin-4-yl)benzènesulfonamide ;
(3*R*)-1,3-diméthyl-6-({3-[(4-méthylpipérazin-1-yl)sulfonyl]phényl}amino)-4-(pipéridin-4-yl)-3,4-dihydropyrido[2,3-b]pyrazin-2(1H)-one ;
(3*R*)-1,3-diméthyl-4-(pipéridin-4-yl)-6-[(3-{[4-(2,2,2-trifluoroéthyl)pipérazin-1-yl]sulfonyl}phényl)amino]-3,4-dihydropyrido[2,3-b]pyrazin-2(1H)-one ;
*N*-{*trans*-4-[4-(cyclopropylméthyl)pipérazin-1-yl]cyclohexyl}-3-{[(3*R*)-1,3-diméthyl-2-oxo-4-(pipéridin-4-yl)-1,2,3,4-tétra-hydropyrido[2,3-b]pyrazin-6-yl]amino}benzènesulfonamide ;
*N*-[2-(diméthylamino)éthyl]-3-{[(3*R*)-1,3-diméthyl-2-oxo-4-(pipéridin-4-yl)-1,2,3,4-tétrahydropyrido[2,3-b]pyrazin-6-yl]amino}benzènesulfonamide ;
3-{[(3*R*)-1,3-diméthyl-2-oxo-4-(pipéridin-4-yl)-1,2,3,4-tétrahydro-quinoxalin-6-yl]amino}-*N,N-*diméthylbenzène-sulfonamide ;
(3*R*)-1,3-diméthyl-6-({3-[(4-méthylpipérazin-1-yl)carbonyl]phényl}amino)-4-(pipéridin-4-yl)-3,4-dihydropyrido[2,3-b]pyrazin-2(1H)-one ;
*N*-{*trans*-4-[4-(cyclopropylméthyl)pipérazin-1-yl]cyclohexyl}-3-{[(3*R*)-1,3-diméthyl-2-oxo-4-(pipéridin-4-yl)-1,2,3,4-tétrahydro-pyrido[2,3-b]pyrazin-6-yl]amino}benzamide ;
3-{[(3*R*)-1,3-diméthyl-2-oxo-4-(pipéridin-4-yl)-1,2,3,4-tétrahydro-pyrido[2,3-b]pyrazin-6-yl]amino}-*N*-(1-méthylpipéridin-4-yl)benzamide ;
3-{[(3*R*)-1,3-diméthyl-2-oxo-4-(pipéridin-4-yl)-1,2,3,4-tétrahydro-quinoxalin-6-yl]amino}-*N,N-*diméthylbenzamide ;
(3*R*)-1,3-diméthyl-6-{[3-(5-méthyl-1,3,4-oxadiazol-2-yl)phényl]amino}-4-(tétrahydro-2H-pyran-4-yl)-3,4-dihydropyrido[2,3-b]pyrazin-2(1H)-one ;
4-(4-fluorophényl)-1,3-diméthyl-6-{[3-(morpholin-4-ylsulfonyl)phényl]amino}-3,4-dihydropyrido[2,3-b]pyrazin-2(1H)-one ;
3-{[4-(4-fluorophényl)-1,3-diméthyl-2-oxo-1,2,3,4-tétrahydropyrido[2,3-b]pyrazin-6-yl]amino}-*N*-(1-méthylpipéridin-4-yl)benzamide
et
4-(2-méthoxyéthyl)-1,3-diméthyl-6-({3-[(4-méthylpipérazin-1-yl)carbonyl]phényl}amino)-3,4-dihydropyrido[2,3-b]pyrazin-2(1H)-one.

9. Composés selon les revendications 1 à 8, destinés à une utilisation en tant que médicament.

10. Composés selon les revendications 1 à 8, destinés à une utilisation dans la prophylaxie et/ou le traitement de maladies tumorales.

11. Utilisation des composés selon les revendications 1 à 8 pour la fabrication d'un médicament.

12. Utilisation des composés selon les revendications 1 à 8 pour la fabrication d'un médicament pour la prophylaxie et/ou le traitement de maladies tumorales.

13. Composés selon les revendications 1 à 8, destinés à une utilisation dans la prophylaxie et/ou le traitement de maladies hyperprolifératives.

14. Composés selon les revendications 1 à 8, destinés à une utilisation dans la prophylaxie et/ou le traitement d'infections virales, de maladies neurodégénératives, de maladies inflammatoires, de maladies athérosclérotiques et/ou pour le contrôle de la fertilité masculine.

15. Utilisation des composés selon les revendications 1 à 8 pour la fabrication d'un médicament pour la prophylaxie et/ou le traitement d'infections virales, de maladies neurodégénératives, de maladies inflammatoires, de maladies athérosclérotiques et dans le contrôle de la fertilité masculine.

16. Composés selon les revendications 1 à 8 en combinaison avec une ou plusieurs autres substances pharmacologiquement actives.

17. Composés selon les revendications 1 à 8 en combinaison avec une ou plusieurs autres substances pharmacologiquement actives, destinés à une utilisation dans la prophylaxie et/ou le traitement de maladies hyperprolifératives.

18. Composés selon les revendications 1 à 8 en combinaison avec une ou plusieurs autres substances pharmacologiquement actives, destinés à une utilisation dans la prophylaxie et/ou le traitement de maladies tumorales.

19. Composés selon les revendications 1 à 8 en combinaison avec une ou plusieurs autres substances pharmacologiquement actives, destinés à une utilisation dans la prophylaxie et/ou le traitement d'infections virales, de maladies neurodégénératives, de maladies inflammatoires, de maladies athérosclérotiques et dans le contrôle de la fertilité masculine.

20. Composés de formules générales (IX) et (XVI) 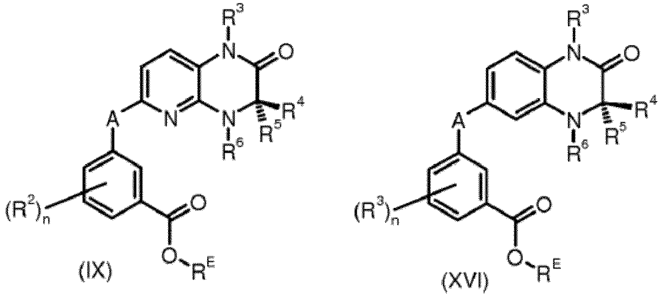 dans lesquelles A, *R*², *R*³, *R*⁴, *R*⁵, *R*⁶ et n ont les significations indiquées pour la formule générale (I) dans la revendicatin 1, et *R*^{E} représente alkyle en C₁-C₆.

21. Composés de formules générales (IX) et (XVI) selon la revendication 20, dans lesquelles *R*^{E} représente méthyle ou éthyle.

22. Composés de formules générales (IX) et (XVI) selon la revendication 20 et 21 :
ester méthylique de l'acide 3-{[(3*R*)-4-cyclopentyl-1,3-diméthyl-2-oxo-1,2,3,4-tétrahydropyrido[2,3-b]pyrazin-6-yl]-amino}benzoïque ;
ester méthylique de l'acide 3-{[(3*R*)-1,3-diméthyl-2-oxo-4-(tétrahydro-2H-pyran-4-yl)-1,2,3,4-tetrahydropyrido[2,3-b]pyrazin-6-yl]amino}benzoïque ;
ester méthylique de l'acide 3-{[(3*R*)-1,3-diméthyl-2-oxo-4-(tétrahydro-2H-pyran-4-yl)-1,2,3,4-tétrahydropyrido[2,3-b]pyrazin-6-yl]amino}-4-méthoxybenzoïque ;
ester éthylique de l'acide 3-{[4-(2,6-difluorobenzyl)-1,3-diméthyl-2-oxo-1,2,3,4-tétrahydroquinoxalin-6-yl]amino}-benzoïque ;
ester méthylique de l'acide 3-{[(3*R*)-4-cyclohexyl-1,3-diméthyl-2-oxo-1,2,3,4-tétrahydropyrido[2,3-b]pyrazin-6-yl]amino}benzoïque ;
ester méthylique de l'acide 3-{[(3*R*)-1,3-diméthyl-2-oxo-4-(propan-2-yl)-1,2,3,4-tétrahydropyrido[2,3-b]pyrazin-6-yl]amino}benzoïque ;
ester méthylique de l'acide 3-{[(3*R*)-4-benzyl-1,3-diméthyl-2-oxo-1,2,3,4-tétrahydropyrido[2,3-b]pyrazin-6-yl]amino}benzoïque ;
ester méthylique de l'acide 3-{[4-(2-méthoxyéthyl)-1,3-diméthyl-2-oxo-1,2,3,4-tétrahydropyrido[2,3-b]pyrazin-6-yl]amino}benzoïque ;
4-[(3*R*)-6-{[3-(éthoxycarbonyl)phényl]amino}-1,3-diméthyl-2-oxo-2,3-dihydropyrido[2,3-b]pyrazin-4(1H)-yl]pipéridine-1-carboxylate de tert-butyle;
ester méthylique de l'acide 3-{[4-(4-fluorophényl)-1,3-diméthyl-2-oxo-1,2,3,4-tétrahydropyrido[2,3-b]pyrazin-6-yl]amino}benzoïque ;
ester méthylique de l'acide 5-{[(3*R*)-1,3-diméthyl-2-oxo-4-(tétrahydro-2H-pyran-4-yl)-1,2,3,4-tétrahydropyrido[2,3-b]pyrazin-6-yl]amino}-2-méthoxybenzoïque ;
ester méthylique de l'acide 3-{[(3*R*)-1,3-diméthyl-2-oxo-4-(tétrahydro-2H-pyran-4-yl)-1,2,3,4-tétrahydropyrido[2,3-b]pyrazin-6-yl]amino}-2-méthoxybenzoïque
et
ester méthylique de l'acide 3-{[(3*R*)-1,3-diméthyl-2-oxo-4-(tétrahydro-2H-pyran-4-yl)-1,2,3,4-tétrahydroquinoxalin-6-yl]amino}benzoïque.

23. Composés de formules générales (X) et (XVII) 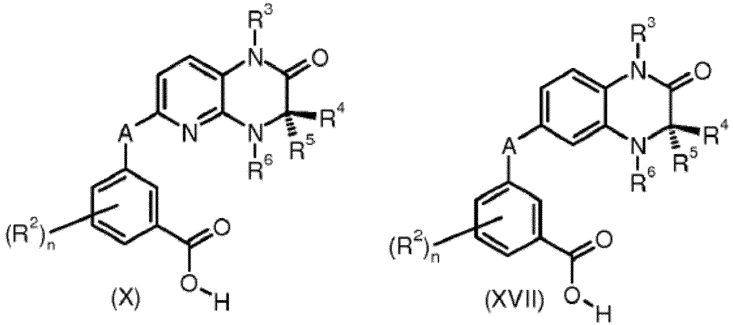 dans lesquelles A, *R*², *R*³, *R*⁴, *R*⁵, *R*⁶ et n ont les significations indiquées pour la formule générale (I) dans la revendication 1.

24. Composés de formules générales (X) et (XVII) selon la revendication 23 :
acide 3-{[(3*R*)-4-cyclopentyl-1,3-diméthyl-2-oxo-1,2,3,4-tétrahydropyrido[2,3-b]pyrazin-6-yl]-amino}benzoïque ;
acid 3-{[(3*R*)-1,3-diméthyl-2-oxo-4-(tétrahydro-2H-pyran-4-yl)-1,2,3,4-tétrahydropyrido[2,3-b]pyrazin-6-yl]amino}benzoïque ;
acide 3-{[(3*R*)-1,3-diméthyl-2-oxo-4-(tétrahydro-2H-pyran-4-yl)-1,2,3,4-tétrahydropyrido[2,3-b]pyrazin-6-yl]amino}-4-méthoxybenzoïque ;
acide 3-{[4-(2,6-difluorobenzyl)-1,3-diméthyl-2-oxo-1,2,3,4-tétrahydroquinoxalin-6-yl]amino}-benzoïque ;
acide 3-{[(3*R*)-4-cyclohexyl-1,3-diméthyl-2-oxo-1,2,3,4-tétrahydropyrido[2,3-b]pyrazin-6-yl]amino}benzoïque ;
acide 3-{[(3*R*)-1,3-diméthyl-2-oxo-4-(propan-2-yl)-1,2,3,4-tétrahydropyrido[2,3-b]pyrazin-6-yl]amino}benzoïque ;
acide 3-{[(3*R*)-4-benzyl-1,3-diméthyl-2-oxo-1,2,3,4-tétrahydropyrido[2,3-b]pyrazin-6-yl]amino}benzoïque ;
acide 3-{[4-(2-méthoxyéthyl)-1,3-diméthyl-2-oxo-1,2,3,4-tétrahydropyrido[2,3-b]pyrazin-6-yl]amino}benzoïque ;
acide 3-({(3*R*)-4-[1-tert-butoxycarbonyl)pipéridin-4-yl]-1,3-diméthyl-2-oxo-1,2,3,4-tétrahydropyrido[2,3-b]pyrazin-6-yl}amino)benzoïque ;
acide 3-{[4-(4-fluorophényl)-1,3-diméthyl-2-oxo-1,2,3,4-tétrahydropyrido[2,3-b]pyrazin-6-yl]amino}benzoïque ;
acide 5-{[(3*R*)-1,3-diméthyl-2-oxo-4-(tétrahydro-2H-pyran-4-yl)-1,2,3,4-tétrahydropyrido[2,3-b]pyrazin-6-yl]amino}-2-méthoxybenzoïque ;
acide 3-{[(3*R*)-1,3-diméthyl-2-oxo-4-(tétrahydro-2H-pyran-4-yl)-1,2,3,4-tétrahydropyrido[2,3-b]pyrazin-6-yl]amino}-2-méthoxybenzoïque
et
acide 3-{[(3*R*)-1,3-diméthyl-2-oxo-4-(tétrahydro-2H-pyran-4-yl)-1,2,3,4-tétrahydroquinoxalin-6-yl]amino}benzoïque.

25. Utilisation des composés selon les revendications 20 à 24 pour la fabrication des composés de formule générale (I) selon l'invention selon la revendication 1.


### SEQUENCE LISTING

<110> Bayer Pharma Aktiengesellschaft
<120> Modifizierte BET-proteininhibitorische Dihydrochinoxalinone und
   Dihydropyridopyrazinone
<130> BHC133024FC
<150> EP13175767.6
   <151> 2013-07-09
<160> 2
<170> BiSSAP 1.3
<210> 1
   <211> 22
   <212> PRT
   <213> synthetic organisms
<220>
   <223> Synthetisches acetyliertes Histon H4 (Ac-H4) Peptid; acetyliert am Lysin in Position 4, 7, 11 und 15
<400> 1
   Gly Arg Gly Lys Gly Gly Lys Gly Leu Gly Lys Gly Gly Ala Lys Arg
   1               5                   10                  15
   His Gly Ser Gly Ser Lys
               20
<210> 2
   <211> 30
   <212> PRT
   <213> synthetic organisms
<220>
   <223> Synthetisches acetyliertes Histon H4 (Ac-H4) Peptid; acetyliert am Lysin in Position 5, 8, 12 und 16
<400> 2
   Ser Gly Arg Gly Lys Gly Gly Lys Gly Leu Gly Lys Gly Gly Ala Lys
   1               5                   10                  15
   Arg His Arg Lys Val Leu Arg Asp Asn Gly Ser Gly Ser Lys
               20                  25                  30